# EUROPEAN PATENT SPECIFICATION

(11) **EP 2 949 328 B1**
(45) Date of publication and mention of the grant of the patent: **20.07.2022**
(21) Application number: 15165807.7
(22) Date of filing: 28.03.2006
(51) Int. Cl.: A61K 31/506, A61P 17/14, A61K 8/65, A61Q 5/00, A61Q 5/10, A61K 38/18, A61P 17/02, A61P 43/00

(54) **MINOXIDIL FOR GENERATING NEW HAIR FOLLICLES AND TREATING BALDNESS**
MINOXIDIL ZUR ERZEUGUNG NEUER HAARFOLLIKEL UND BEHANDLUNG
MINOXIDIL POUR GÉNÉRER DE NOUVEAUX FOLLICULES PILEUX ET POUR TRAITER LA CALVITIE

(30) Priority: 29.03.2005 US 665857 P; 23.05.2005 US 683293 P
(43) Date of publication of application: 02.12.2015
(62) Divisional of application: 06748823.9
(73) Proprietor: The Trustees of The University of Pennsylvania, Philadelphia, PA 19104 (US)
(72) Inventor: COTSARELIS, George, Berwyn, PA Pennsylvania 19312 (US); ITO, Mayumi, New York, NY New York 10016 (US)
(74) Representative: Pearl Cohen Zedek Latzer Baratz UK LLP

(56) References cited:
- US-A1- 2004 219 179
- MESSENGER A G ET AL: "Minoxidil: Mechanisms of action on hair growth", BRITISH JOURNAL OF DERMATOLOGY 200402 GB, vol. 150, no. 2, February 2004 (2004-02), pages 186-194, XP002745708, ISSN: 0007-0963
- ARGYRIS T S: "The growth-promoting effects of wounds on hair follicles already stimulated by plucking", THE ANATOMICAL RECORD JUL 1962, vol. 143, July 1962 (1962-07), pages 183-188, XP002745709, ISSN: 0003-276X
- ARGYRIS T S ET AL: "Factors affecting the stimulation of hair growth during wound healing", ANATOMICAL RECORD, vol. 142, no. 2, 1962, pages 139-145, XP002681426, ISSN: 0003-276X, DOI: 10.1002/AR.1091420206
- KLIGMAN A M: "Neogenesis of human hair follicles", ANNALS OF THE NEW YORK ACADEMY OF SCIENCES, vol. 83, 20 November 1959 (1959-11-20), pages 507-511, XP002681427, ISSN: 0077-8923
- KLIGMAN A M ET AL: "The formation of vellus hair follicles from human adult epidermis", THE JOURNAL OF INVESTIGATIVE DERMATOLOGY JUL 1956, vol. 27, no. 1, July 1956 (1956-07), pages 19-23, XP002745710, ISSN: 0022-202X
- BREEDIS C: "Regeneration of hair follicles and sebaceous glands from the epithelium of scars in the rabbit", CANCER RESEARCH, vol. 14, no. 8, September 1954 (1954-09), page 17PP, XP002681458, ISSN: 0008-5472

### SEQUENCE LISTING

<110> The Trustees of the University of Pennsylvania
<120> METHODS FOR GENERATING NEW HAIR FOLLICLES, TREATING BALDNESS, AND HAIR REMOVAL
<130> P-7628-EP1
<150> EP06748823.9
   <151> 2006-03-28
<150> PCT/US06/11319
   <151> 2006-03-28
<150> US60/683,293
   <151> 2005-05-23
<150> US60/665,857
   <151> 2005-03-29
<160> 280
<170> PatentIn version 3.5
<210> 1
   <211> 321
   <212> RNA
   <213> Homo sapiens
<400> 1
   uuuuuuuuuu uuuuuuucuu aaaaauauaa auguauuugu cugcaucaug acguucuucg 60
   ggcaccuagc uggccagacc acuggccaug ggacaaggag gaagucaggu guaagucuga 120
   gcaaggaaca ggacucugcc cuggcagggu ggagguggcc ucacaguguc ccaugcuggg 180
   ccugguagcg ugaaagcaca gcacgguagu gggacagcuc cugccgcaca gccaccaccu 240
   ccugccgcaa cagggcguuu uccuucucca ggaaggcagc ccgcacagau auccgguucu 300
   ccuugagucu ucuugcaucu c 321
<210> 2
   <211> 1580
   <212> RNA
   <213> Homo sapiens
<400> 2
   caguuccuca gcauggccac cugcagccgc caguucaccu ccuccagcuc caugaagggc 60
   uccuguggca ucgguggugg cucuagccgc auguccucca uccuggcugg aggauccugc 120
   cgggcuccca gcaccugcgg gggcauguca guuaccuccu cucgcuucuc cucuggggga 180
   gucuguggga uuggaggugg cuaugguggg agcuucagca gcagcaguuu ugguggagga 240
   cuugguagug gauuuggugg ucgauuugau ggauuuggug gugguuuugg ugcuggucuu 300
   gguggugguc uuggcggugg uauuggugau gggcuccugg ugggcaguga gaaagugacc 360
   augcagaacc ucaaugaccg ccuggccacc uaccuggaca aggugcgugc ccuggaagag 420
   gccaacagag accuggaggu gaagauccgg gacugguacc agaggcagcg gcccacugag      480
   aucaaagacu acagccccua cuucaagacc auugaggacc ugaagagcaa gaucauuauu      540
   gccacccagg agaaugcaca guucacuuug cagauugaca augccaggcu ggcagcugau      600
   gacuucagga ccaaguacga gaaugagcug uucuugcggc aguccgugga gggugacauc      660
   aauggccugc gcaaggugcu agaugagcug acccugucca gagcugaccu ggaaaugcag      720
   auugaaaacc ucagagaaga gcuggccuuc cugaagaaga accaugagga ggagaugcuu      780
   gccuugaggg gucagacugg uggggacguc aauguggaga uggacgcagc ccccggugug      840
   gaccucagcc gcauucugaa ugagaugagg gaccaguaug agcagauggc agagaagaac      900
   cgcagagaug uggaggccug guuccugaga aagacugagg agcugaacaa agagguggcc      960
   ucuaacagug aucuaaucca gagcaaccgc agcgaggugg cugagcuccg cagggguguc    1020
   cagggccugg agauugaacu gcagucccag cucagcauga aagcauccuu ggagaacagc     1080
   cuagaagaga ccaaaggcag auacuguaug cagcuguccc agauccaggg uuugaucagc     1140
   aguguggagg agcagcuggc ucagcuucgc ugcgagaugg agcagcagag ccaggaguac    1200
   aacaucuugu uggaugugaa gacaaggcug gagcaggaga ucgccaccua ccgccgucug     1260
   cuggauggcg agaauaucca cuccuccuca cagcacuccu cuggacaguc cuauucuucu     1320
   cgagaagucu ucuccucauc cucccgccag ccccggucca uccucaagga gcaagguuca     1380
   accagcuuca gccagagcca aagucagagu uccagggacu aauguuuugc cuagagccuc     1440
   cucacccaca acugccucuc aagcugaggg cuuggggcag gacccuguuu ucuuugcgca     1500
   uuccccaucu gucuccccua cccucucaug gugguaggcu aauaaagcuu uuugguugau     1560
   gcaaaaaaaa aaaaaaaaaa                                                  1580
<210> 3
   <211> 1357
   <212> RNA
   <213> Homo sapiens
<400> 3
   gcgccagucc uccgauagac ugagucgccc ggguacccgu guucucaaua aagccucuug      60
   cuguuugcau ccgaaucgug gucucgcugg uccuugagag ggucuccuca gauugauuga     120
   cuacccacgu cgggggucuu ucauuuggag gccccagcga gauuuggaga ccccugccca     180
   gggaccaccg accccccguc gggagguaag cuggccagcg gucguuucgu gucugucucu     240
   gucuucgugc guguuugugc cggcauuuaa uguuugcgcc ugcgucugua cuaguuagcu     300
   aacuagaucu guaucuggcg guuccgcgga agaacugacg aguucguauu cccggccgca     360
   gccccuggga gacgucccag cggccucggg ggcccguuuu guggcccauu cuguaucagu     420
   aaccacccga gucggacuuu uuggagcucc gccacuguac guggcuuugu ugggggacga     480
   gagacagaga cacuucccgc ccccgucuga auuuuugcuu ucgguuuuac gccgaaaccg     540
   cgccgcgcgu cugauuuguu uguuguucuu uuguucuucg uuaguuuucu ucugucuuua     600
   aguguuuuuc gagaucaugg gacagaccgu aacuaccccu cugaguuuaa ccuugcagca     660
   cuggggagau guccagcgca uugcauccaa ccagucugug gaugucaaga agaggcgcug     720
   gauuaccuuc uguuccgcug aauggccaac uuucaaugug ggauggccuc aggaugguac     780
   uuucaauuua aguauuaucu cucagguuaa gucuagagug uuuuguccug guccccacgg     840
   acacccggau caggucccau auaucgucac cugggaggca cuugccuaug accccccucc     900
   gugggucaaa ccguuugugu cuccuaaacu uccucccucg ccgacagcuc ccguccuccc     960
   gcccgguccu ucugcgcaac cuccgucccg aucugcccuu uacccugccc uuacccccuc    1020
   uauaaagucc aaaccuccua agccccaggu ucucccugau agcggcggac cccucauuga    1080
   ccuucucaca gaggaccucc cgccguacgg agcacaaccu uccuccucug ccagagagaa    1140
   cgauaaagaa gaggcggcca ccaccuccga gguuuccccc ccuucuccca uggugucucg    1200
   acugcgggga aggagagacc cucccgcagc ggacuccauc aucucccagg cauucccacu    1260
   ccgcaugggg ggagauggcc agcuucagua cuggccguuu uccuccucug auuuaacccu    1320
   uccuuuucug aagauccagg uaaauugacg gccuuga                             1357
<210> 4
   <211> 463
   <212> RNA
   <213> Homo sapiens
<400> 4
   gacguagagc cccuugcgcc cgguuuccug aucccgcuua cuccucugcg cgccggcagg      60
   auggcccaca agcagaucua cuacucagac aaguacuucg augagcacua cgaguaccgg     120
   caugucaugu uacccagaga acucucuaaa caaguaccca aaacucaucu gauguccgaa     180
   gaggagugga ggagacuugg uguccaacag agucuaggau ggguucauua caugauucau     240
   gagccagaac cgcauauucu ucucuuuaga cgaccucuuc caaaagaaca acaaaaauga     300
   agugcagcug ggaucaucua aucuuuuuca aauuuaaugu auauguguau auaagguagu     360
   auucagugaa uacuugaaaa guguacaaac cuuucaucca uaccugugca ugcgcuguau     420
   ucuucacagc aacagagcuc agucaaaugc aacugcaagu agg                       463
<210> 5
   <211> 1973
   <212> RNA
   <213> Homo sapiens
<220>
   <221> misc_feature
   <222> (558)..(558)
   <223> n is a, c, g, or u
<220>
   <221> misc_feature
   <222> (569)..(569)
   <223> n is a, c, g, or u
<400> 5
   auuaaaaagc cagcugccca augccugcac acagaaucca caccaacaga gaaccugcuc    60
   uucucugagu auuagguaag ucucugcuug caacugauuu gaaauucuug cuuauuuuuu   120
   uacuaugaaa aacuguucaa agccaacucu auuacagagu ugaugugggg ugcugccuac  180
   aguuagugaa caguagcauu guuugcuuaa auuauagcac auuuuggguc uaggaacuug  240
   agaggugauu caguugguca cuguaguaga cagccuugga aucagagauu aaggcaaaag   300
   gaaaccucca uucaaauauu cauggaaguu cacagcugga gacagguuaa ggcuucaguc   360
   cagauagcuu ucagaauuau ugcaguucuu acugauaggc auaucaauag cgaaauuuaa   420
   uuuauuagag gaaucuacua aaguaaauuu uuaggccaau auagaacaua ccauacuugu   480
   agucuggcag agaaggugac auaugaaauu gaaaugcgau ucauagacag ugguagugaa   540
   agaaauaaug guggggangg gcugugcang ggaggcaugg cucaaggaca gcacuuagug  600
   guagcacaca ccaugaacua ugauggaaaa gcauuugaua ggcagagaca gaaauguagg   660
   aaauguguag ggauccauga gagcauaaac uaaaagggca aaagcauaug agcauggacu   720
   aacaugcagc cacucugcaa guuauacuau gaucuauuuc acaaggaggu ugguaugcug   780
   cugucuuugg gugacaccgc uuucccagau guccuggugu guagguucac aagccucuca   840
   gaagccauac uuuauugcuu uuuaagacgu auuauuaaua uuuuggcuag cauaugguuu   900
   agugugagag uuuuauaugc auaugucauu auacuuugcu cauuuauguc uuggaacuuc   960
   uucaacuagg uagaaaacau gaccagggag aaugagagua aggaaagaac ccacugagac  1020
   agacaagagc aaaccauacu ucugcuaauc auguuuaaaa guccagaaau gaucauacca  1080
   uauuuuauuu ucaagugugg aagucagcau ggagaggggc ucuuuuucuc uaaaggggcc 1140
   ugaaauuaaa uugacuugau guugagguua ccuucucuuu caaugaauca cuaaauuguc  1200
   uuuucuguuu cccaggaccc aagugcuauc uaaccaugag uucccaccag cagaagcagc  1260
   ccugcacugu accuccucag cugcaccagc agcaggugaa gcagccuugc cagccaccac  1320
   cccaggaacc uugugccccc aaaaccaagg aucccugcca cccuguuccu gagcccugca  1380
   accccaaggg gccagagccc ugccacccca aggcacccga gcccugccac cccaaggcac  1440
   cugagcccug caaccccaag gugccagagc ccugccagcc uaaggugcca gagcccugcc  1500
   agccuaaggu gccagagccc ugcaacccca aggugccaga gcccugccaa ccuaaggcac  1560
   cagagccuug ccaccccaag gcgccugagc ccugccaccc uguuguuccc gagcccugcc  1620
   ccucaacugu cacuccauca ccauaccagc agaagacaaa gcagaaguaa uauuguccag 1680
   agccaugccu gaagaccuga ucaccagaug cugaggcugc ugucuauccu gcuuaugagu 1740
   cccauugccu ugugcuacca augcugugac cuucagucuu aaucccucuc uccuugcacc 1800
   accuaaaaag uugacucuca uccucaucuu caagggcucc ugagccucuu aacauugccc 1860
   aaagucauau ugaauggcua cacuuuucau ggcucaggau ucaucugaag ggggugagga 1920
   gugagacaag uguaugguca auauuuuccc cccauuaaau gccauuuaac ucc 1973
<210> 6
   <211> 254
   <212> RNA
   <213> Homo sapiens
<400> 6
   uuuuuuuuuu uuuuuuuagc caaaauaguu auuuauuaau aauuuaaggu uuuacauucu 60
   uauaauaaau uccagcucaa aacuuuacac cacgaacauc auggagcaag uuaauucucc 120
   cuuccucuca accuguugca uccaccaaau gggcgcucau acucgcacac auacacacac 180
   uuccaguuuc guauuuuuuu uuaaaaggaa agaaaccaac ccaaaguauu gcauuugagg 240
   ugacacuccc ugaa 254
<210> 7
   <211> 137
   <212> RNA
   <213> Homo sapiens
<400> 7
   aagaugcccu uuggauugga uuggauugau cauguuuaac ucagcguauu uuauggauga 60
   aagcuaaaua cagauauuug gcaucucuaa gguggaauga gcccacucca cacacugaua 120
   aaauucaugc auaguuu 137
<210> 8
   <211> 622
   <212> RNA
   <213> Homo sapiens
<220>
   <221> misc_feature
   <222> (27)..(27)
   <223> n is a, c, g, or u
<220>
   <221> misc_feature
   <222> (30)..(30)
   <223> n is a, c, g, or u
<220>
   <221> misc_feature
   <222> (133)..(133)
   <223> n is a, c, g, or u
<400> 8
   aaccaaaugg gaaugggguc cccaacnuun cugugguacc agccggguuu cucuugcauu      60
   ggaaacaaac accuuuguag gcauuugcgu auucgugaag agacuguuuu augaaucacc     120
   ucuuagauuu aunuaauuaa ccuaaguugu ugaaguuucu guuucuccuu aagagaaauu     180
   acaaaaauuc aacauugaag cauaguuucu uguuuucugu ugucaaauag uaauaaugug     240
   cugugauguu uaugcuuauu cauaaagaug uuuuacuuuu uaguguaaug uuaguucuuu     300
   uuaacauuau uuugcuuaaa uuugauaaug cccgacaaga auauauuuug cuuugauuua     360
   uacacugauu cuuugugaca aauaugaccc auuaaaaaug ccuuuuaaua gacuaacuua     420
   ccuuuuguag cuagguacuc auguucuuuu uuaaaagaug cccuuuggau uggauuggau     480
   ugaucauguu uaacucagcg uauuuuaugg augaaagcua aauacagaua uuuggcaucu     540
   cuaaggugga augagcccac uccacacacu gauaaaauuc augcauaguu uuaaaugaac     600
   auuaauaaac ucauguuguc uu                                              622
<210> 9
   <211> 613
   <212> RNA
   <213> Homo sapiens
<400> 9
   gaauucaagg aggcuuucca gcuguuugac cgaacaggug auggcaagau ccuguacagc      60
   cagugugggg augugaugcg ggcccugggc cagaacccua ccaacgccga ggugcucaag     120
   guucuaggaa cccccaagag ugaugagaug aaugugaagg uacuggacuu ugagcacuuc     180
   cugcccaugc ugcagaccgu ggcgaagaac aaggcccagg gaaccuacga ggauuauguu     240
   gaaggccuuc guguguuuga caaggaagga aauggcaccg ucaugggugc ugaaauccgu     300
   cauguccuag ucacacuggg cgagaagaug acagaggaag aaguagagau gcuaguggca     360
   gggcaugagg acagcaaugg uugcaucaac uaugaagcau uugugaggca uauccugucg     420
   gggugacggg cccgaugggg cggagcucgu ccggauggug cugaauggcu gagacauucu     480
   guaucccgag ucuguucccu gcccagugug auuucugugu ggcuccagac gcuccccugu     540
   cacagcaccu ugucccauuu gguuucuuug gaugauguuu gccuucacca aauaaaauuu     600
   gcucucuuug ccc                                                        613
<210> 10
   <211> 1508
   <212> RNA
   <213> Homo sapiens
<400> 10
   caaccaccuc cuaccugccu gcccaaagcu ccagggcugg agcacggaga ccugucaggg 60
   auggauuuug cccacaugua ccaaguguac aaguccaggc ggggaauaaa acggagcgaa 120
   gacagcaagg aaacuuacaa acugccgcac cggcugauug agaaaaagag acgugaccgg 180
   auuaacgagu gcauugccca gcugaaggau cuccuacccg aacaucucaa acuuacuacu 240
   uugggucacu uggaaaaagc agugguucug gagcuuacgu ugaagcacgu gaaagcauug 300
   acaaaucuaa uugaucagca gcagcagaaa aucauugccc ugcagagcgg uuuacaagcu 360
   ggugauuugu cgggaagaaa ucucgaggca gggcaagaaa uguucugcuc agguuuccag 420
   acuugugccc gugagguacu ucaguaccug gcgaagcaug agaacacucg ggaccugaaa 480
   ucuucccagc ucgucacuca ucuccaucgu guggucucgg agcugcugca ggguggugcu 540
   uccaggaaac cauuggacuc ggcucccaaa gccgucgacu ugaaagagaa gcccagcuuc 600
   cuagccaagg gaucagaagg cccagggaaa aacugugugc cagucaucca gcggacuuuu 660
   gcucccucgg guggggagca gagcggcagu gacacggaca cagacagugg cuauggaggu 720
   gaauuggaga aaggggacuu gcgcagugaa cagccguacu ucaaaagcga ccauggacgc 780
   agguucgccg ugggagaacg ugucagcaca auuaagcaag aauccgaaga gccccccacc 840
   acaaagagcc gaaugcagcu cucagaagag gaaggccacu ucgcgggcag ugaucugaug 900
   gguuccccau uucuugggcc acacccacau cagccuccuu uuugccuucc cuucuaucuc 960
   aucccaccau cggccacugc cuaccugccu augcuggaga aaugcuggua ccccaccucu 1020
   gugccagugu uauacccagg ccucaacacc ucagcugcag cccucuccag cuucaugaac 1080
   ccagacaaga uaccgacucc cuugcuucug ccccagagac ucccuucucc uuuggcacau 1140
   ucgucccuug acucuucggc cuugcuccag gcuuugaagc agaucccucc uuuaaacuua 1200
   gaaaccaaag acuaaacucu ggagggaucu ccugcugccu ugcuuucuuu ccucccuaau 1260
   uccaaaaacc acgaagguuu cccugagugc agagagauca gcccacccug cagacccaca 1320
   gagaagauuc agagugugug ugagagugag ugagugugcg ugcgugcgug cuuguaugua 1380
   uguuuguaua uguaggacaa uaaguuccuu cugacacaag ggagacacga gaaggauagc 1440
   cugacaucag augacagacu ggaggacugu agcacaucuc ugggcguuuc ccuacccaga 1500
   gaagagcc 1508
<210> 11
   <211> 3660
   <212> RNA
   <213> Homo sapiens
<400> 11
   uugcaggcga gggcuuccac aacuaccacc acaccuuccc cuucgacuac ucugccagug     60
   aguaccgcug gcacaucaac uucaccacgu ucuucaucga cugcauggcu gcccugggcc    120
   uggcuuacga ccggaagaaa guuucuaagg cuacugucuu agccaggauu aagagaacug    180
   gagacgggag ucacaagagu agcugagcuu ugggcuucug aguuccuguu ucaaacguuu    240
   ucuggcagag auuuaauauu cuguugauua acuaacaacu ggauauugcu aucggggugu 300
   uaaugaugca uuuaaccuau uccgguacag uauucuuaua aaaugagaaa gcuuugauca 360
   cguuuugagg uaauaaauau uuuauuuagc uaggauuaac caugccacaa gacauuauau 420
   auuucuaagc acacaugaua aaugcauaua caauuuugca caacagcuuu aaauaauaac 480
   aauaaauuug aacauucuau acagagagga ucaaagccaa ggaacaugcu guuuugaugc 540
   uagggugagc auggugcuca gucccuguuu guuugcaugg uguccagcuu uguuucuucu 600
   cugucaucac caccuucagg caaauaguug accaaccacu ggccuguguc uguccacccu 660
   ccaaagccca ggccaccuuu cuguuuucug aaauacugau ccuuccuccu gaauacaucc 720
   cuccuuguuc cuagcuucaa gacugcugcc ucaaauaggg auagagcaag uccccgcugc 780
   agguugugcu agaugggaug gagaaauuau cuucauuuga uacagagcaa guagauuguc 840
   ucgagagaaa aguuagcaug cgugguauga uuuguaagua aagauggaag agagagagag 900
   agagagagag agagagagag agagagagag agagguagcc auaucuaaca gccuacuuac 960
   caaagacccc aggccucucu gcuuggcaug ccuccuuucu guccauccuc ugaaccccag 1020
   agauuaguga gauuugaaua auuaaaucau uuucagagug aagggguuaa augcaggguc 1080
   ugugcuaggg gaggguuuua gcuuuuggua acugaagauu uuuucaugga aaaagucuuc 1140
   guguucaaug ugccuagaac ugauaacuaa acagcugaca uuugucgggg acagauaugg 1200
   ugugaaacua ugaaaauaua agcaaaaucu ucacuuggaa caugaaacua uuucacuuag 1260
   aaaauaaucg aaggacccga gguguugccu ggguugccag uuucuuucgu ggcugggcag 1320
   gaacuaguga gguugagggg cagugucugu aaguagcugc uaagaggugc auuuccagau 1380
   gaagcccuug gggaacaucu gccagggauc cgcauggugu uggcuccauc cauugcuuua 1440
   guuuccuccu uggauugugu agaaacuugg cuucccaugg uuuugaaccu uccaugccuu 1500
   cuuugcuuug uggccaccca gccugccuag ugcugccuag gaagcucuua cccaccugau 1560
   uucuucugac auuucuuucu uuggccuuuu uuucuuucuc cggacaugca gcuaguugcc 1620
   ugaguguauc aagagcaccc aggacuugcu gcuguccagg ccuguuccuc ccccaguauc 1680
   cgugggugug gaagagcugu guagcuucag gaagcagagc caggugccac cuuucugugg 1740
   cuuccagauc cucccuaccu ccaacucaug ugccucuguc acagugauuu caggaaagcu 1800
   ugguagaccc ucuagcaaca ucucgguuca gaaagucucu cugguuugug aguuaacagc 1860
   ucagcuaagu gcuguuuugu cucagugagu uaaccacuga augcgagggu ugguuguuga 1920
   ucugucucgg ugugugucgg aguagacagc auaugcacuu cucccugugc gcuuugcaag 1980
   guaauguggc uuuggcugau ccaugcaggc agguaguggu acagugcugc ugaaaggaag 2040
   aaguucccca uuuuaucugu uaaaacacca gagacauggg caagugcuaa uggaccucac 2100
   uucaggaaga gggucugcuu ccugaagcca gugugugaug aaaagugacu gagaccugau 2160
   aucuaaggug agaccugaua ccuaacacuc ugucacacag uccagggcca acagugcuau 2220
   aggaaagucu agaagaaaac aucacaucag uauuuuagaa ccaucaacca ucucuugucc 2280
   cuauagccca auccagaggc cugguuuuua gaacuggcug uguaaggugc caaacacuca 2340
   guucacuugu agaaucagag ccuuuuuucc ccccuauguu aauugaacac gcgcucugag 2400
   cuguuuuguu gaaguagaaa aucucauaga aaaaucacug uagaucuacu gaccuauagc 2460
   ccucuggaaa ugccuuugag augguuuuac uuuucuaggu cauagaugcc ugauuauaaa 2520
   gaugaacaau aaaaucagcu uucuuucuuu cucuucugau cuuauucccc agaucugauu 2580
   caggccaugu uccaaagcaa ggcuacauug agguccuggu gucuuuaagu aaaggacauc 2640
   uuucagaucc ucucaaagaa ggauuuauaa caguuuccag augaauguac uaauagcuuu 2700
   gggugccuua ucucuuuccu aaucuguagu gccugugagc ucagucucac uccuucccuu 2760
   agcccggaga ccccuuagau cgagugggaa uagucaagag gcuggcugga gagucaucag 2820
   uacauugguu ugcagaaauc uuuuacaggc uacauuuugg aauuuuuuuu uuuuuaguaa 2880
   gugaucaaau uuggugggaa guaauucgag uguauucgau uguauugucg uccucguuau 2940
   cauugucaaa cauguuauag acggcaguug gcacuggggc ugcuaaucuc uggguguagu 3000
   cucugaaacu guagcuccag ugagguggug ugaaagguua gcaaagccac caucugcugg 3060
   ugcuccagcc aaggugccuc uuagccacug aauugcuaug uuauccuuuc ucuuguaaca 3120
   aacccacccc agagauaaag ccuuuaauca acccaagaaa cuccugggcu aaguaucuga 3180
   cagucucaca ucucaacagu gugaauuaag uguccauagc aucagcucag gaggacacuc 3240
   ugggagagug cugacaaaaa aggguuauua auacugaccu acuacuucaa gggcaguucu 3300
   gaggugauua gagcuuuuuu uaaaaaccaa guauuugggg auccucagca gagguauuca 3360
   uacagacucc caaagaacua uauauguucc ugagaccauc guuuagucua cauugcucuu 3420
   cccagagacu gacagauaug accagucaaa gugcaagacu accuacccac ugccaugaaa 3480
   accauugcag gaaaccuuuc ccuuccugaa ugagauuuuu uuuuucccuu uuuauguggg 3540
   guaauuauuu gugacccaag uguaauuugg augauuucca uuaauaucaa cucuugaagc 3600
   cuacuuguac ugauugagau uguauuuguu ccuaauaaaa guggaucugg uuguacuguc 3660
<210> 12
   <211> 429
   <212> RNA
   <213> Homo sapiens
<400> 12
   accacuugca gacaaaugaa uuccuucgaa auguauuuga gguuggaccc ccugugaugc      60
   uugaugcugc aacacuuaaa accaugaaga uuucucguuu ugaaaggcau uuauauaacu     120
   cugcagcuuu caaagcucga acaaaagcuc gaagcaaaug ccgagauaag agagcagaug     180
   uuggagaauu cuuguagaug ucugaauuug auggcuguuu ucuaaucucu uccuuuauua     240
   uuauuuuugc uacuucuaau guauauaagc uuuuagagac aguuuuuuau cuuggucaac     300
   uuaaauaauu uuugauguag ggugugguug uauuuuaauu uaauguacag uguuacaaau     360
   uaaugaguuc uuuauucucu guaaaaauaa cugguaacca caaauaaagu guuugugaug     420
   uuuggucgu                                                             429
<210> 13
   <211> 1639
   <212> RNA
   <213> Homo sapiens
<220>
   <221> misc_feature
   <222> (1003)..(1003)
   <223> n is a, c, g, or u
<220>
   <221> misc_feature
   <222> (1098)..(1099)
   <223> n is a, c, g, or u
<220>
   <221> misc_feature
   <222> (1106)..(1106)
   <223> n is a, c, g, or u
<220>
   <221> misc_feature
   <222> (1112)..(1112)
   <223> n is a, c, g, or u
<220>
   <221> misc_feature
   <222> (1158)..(1158)
   <223> n is a, c, g, or u
<220>
   <221> misc_feature
   <222> (1161)..(1161)
   <223> n is a, c, g, or u
<220>
   <221> misc_feature
   <222> (1171)..(1171)
   <223> n is a, c, g, or u
<220>
   <221> misc_feature
   <222> (1182)..(1182)
   <223> n is a, c, g, or u
<220>
   <221> misc_feature
   <222> (1245)..(1245)
   <223> n is a, c, g, or u
<220>
   <221> misc_feature
   <222> (1436)..(1436)
   <223> n is a, c, g, or u
<220>
   <221> misc_feature
   <222> (1472)..(1473)
   <223> n is a, c, g, or u
<400> 13
   auguccgcgg ccgccuacau ggacuucgug gcugcccagu gucugguuuc caucuccaac      60
   cgcgccgccg ugccggagca cgggggcgcu ccggaagccg agcggcugcg acuaccugag     120
   cgcgagguga ccaaggaaca cggugacccg ggggacaccu ggaaggauua uugcacgcug     180
   gucacuaucg ccaagagcuu guuggaccuc aacaaauacc gacccaucca gacccccucg     240
   gugugcagcg acagucugga gagucccgau gaggauauag gauccgacag cgacgugacc     300
   accgaaucug ggucgagucc uucccacagc ccggaggaga gacaggauuc uggcagcgcg     360
   cccagcccac ucucccuccu ccacucugga guggcuucga aggggaaaca cgccuccgaa     420
   aagaggcaca agugccccua caguggcugu gggaaagucu auggaaaauc cucccaucuu     480
   aaagcccauu acagagugca uacaggugaa cggcccuuuc ccugcacgug gccagacugc     540
   cuuaaaaagu ucucgcgcuc ggaugagcug acccgccacu accggaccca cacuggggaa     600
   aagcaguucc guugcccacu gugugagaag agauucauga ggagugacca ucucaccaag     660
   caugcccggc gucacaccgu guuccauccc agcaugauca agagaucaaa aaaggcucuu     720
   gccugccccu ugugaggugc uccccauggc agccaggcag agaugggucc ccggaaggac     780
   agagcuccca ggaaacagac ugacacaugg aaaucugcca cagcagaggc gcgcuggcca     840
   caggagguca cugcuucuuu ggccaauauu cugauaucuc ccugcacugu uuccaaaaag     900
   cacaugguag cccuaagguc aaagucaaca uuuggucccc uugcagaggc aacucugaac     960
   cgucucugac ugaagauuca gacugguggu guacauacgu cunacugggu gaguugaccc    1020
   cuggccuccc acagugcaga accacucucu ugaaucacau uaacuuuuga gauuuaaaaa    1080
   aaacccaaac ccaacccnnc aaaacnccag anacaccgaa acucuggauc cucgaugcuu    1140
   gcugacucuc agaauugnuu nuucuucuca nuuaugcaag cnagagcaca ccuacuccag    1200
   caugauuugu caucuaaaga cuugaaaaca aaacaacaac aacanaaagu uacuuauagu    1260
   caauggauaa gcagaguccg aauuuacacu aaucaagaca gaccuucgag gggucacgau    1320
   aaguccggaa cuuucaaacc uugcuucgua ugaauuguac uaucugaaca uaaacugcac    1380
   uuuuauuuuc uaauaccgag ggugaauacg guaaauacau gcuuugaggg uagaanccga    1440
   cggucuguuu ggcaccacgu uauaaucugc unnuuuuaac gaguaccacc uuggagggca    1500
   ggcaaauaaa ugcuuuuggg uauuuucucc uuuguuuuug acaaaugcug cggauggggg    1560
   aucgggaucg gaggggagug cuuuuaaaga uaauaaaaaa ugagguaaau aauuuuaacu    1620
   uaugaauuug uuugaauuc                                                1639
<210> 14
   <211> 1929
   <212> RNA
   <213> Homo sapiens
<400> 14
   gagauugaaa caauggaaga acuuuccaug gcaaacacca uguuugcccu caaucuccuu      60
   aagcagauag aaaaaucaaa cucuacccag aacaucuuua ucucuccaug gagcaucuca     120
   ucaacauugg ccauaguucu ccucggugcu ggggguaaca cugaacagca gauggccaaa     180
   gugcugcagu uuaaugaaau uggcaguuau gguaucacca caagaaaccc agagaacuuc     240
   aguggcugug auuucgcaca acagauacag aaggaaaauu auccuagugc uauuuuacag     300
   gcacaagcag gagauaaaau ccauucagcc uucuccucuc ucagcucaac aaucaacaca     360
   ccacaggggg auuauuuguu agaaagugca aacaagcugu uuggagagaa gucugcaaga     420
   uucaaagaag aauacaucca acucucuaag aaauauuacu caacagaacc agaagcagug     480
   gacuuccuug aaugugcuga agaagcuagg gaaaagauua auucuugggu caagacucaa     540
   accaaaggug aaaucccaaa ccugcuaccc gaagguucug uagaugaaga caccaagaug     600
   gugcugguga augcugucua cuucaaagga aaguggaaaa cuccauuuga gaagaaacuu     660
   aaugggcuuu auccuuuccg ugugaacucg caugagagca uaccugucca gaugauguuc     720
   cuccaugcaa agcugaacau uggauacaua aaggaccuga agacucagau ccuagaacuu     780
   ccgcauacug gaaacaucag caugcuccug uugcuucccg augagauuga ggacgcaucc     840
   acuggcuugg aauugcugga aagugaaaua aacuuugcca acuucaacaa guggaucagc     900
   aaagacacac uggaugaaga ugauguugug gucuacauuc ccaaguucaa acuggcacaa     960
   agcuacgaac ucaaguccau ucuucaaagc augggcaugg aggaugccuu caacaagggc    1020
   aaggccaacu ucucaggaau gucugagagg aaugaccuuu uucuuucuga gguguuccau    1080
   caagccagcg uggaugucac cgaggagggc acuguggcag cuggugggac uggggcaguu    1140
   augacaggaa gaacuggcca ugguggccca caguuugugg ccgaucaucc cuuucuuuuc    1200
   uuuaucaugg acaaaauuac ccacacgaua cuauuuguug guagauucuc cucacccuaa    1260
   aaggggaaga ccuauuucca caugagguuu uguagcauga acuauaagcc ucagaauugc    1320
   aucuucaagu gccaaaaguu uaaauacuuu cuuacacauu uuuauacuuc ugcuauacac    1380
   uaaauauaac cuaaaagcaa uuguauagca gucuucagug cuuacaguau aacucuauua    1440
   augauuuugu uuccuaaagu cagaugaugu cuauuuaguu cauccuuauu acugcuuugu    1500
   cuuuauaacu uuaguuuuua caguguuauu uauuguuuau auaaugguug uuuuacaaau    1560
   uguuguccuc uguuuaauga aacuguaaca cuacagaagc agaaaauuag auaauuuucu 1620
   auuuaaagaa aaucagccau uuaauuuaau aaugaaggaa aaauaugagu cuuccauacu 1680
   uucccaugau auucacccag aaaaaaugua cuuaacaaaa gacauguuau aucucuauca 1740
   uuauauauca uauguacgua ucugcaacuc aucuauaauu aggacuacau cauaaguaag 1800
   caugcuuacu uacacacugc uaucuguugu auaaaacuua gcaauccuua uuuguuaguu 1860
   aucuuucuau cacuguaaca aaauaccuga gauaauaaag uucaaagauu uauuuugaaa 1920
   aaaaaaaaa 1929
<210> 15
   <211> 284
   <212> RNA
   <213> Homo sapiens
<220>
   <221> misc_feature
   <222> (16)..(16)
   <223> n is a, c, g, or u
<400> 15
   uuuuuuuuuu uuuuunuaag aauuaacuuu uauuuuugcu uaguuuuauu aaaaaaauaa 60
   auaugucaua aagcuuuugu uuccuuuagg gagaaaaaaa aggaacaagu uccauaaaau 120
   caaacaagca augguaacau gucuuaacuu gaaaacaacu ggggucacug guuuacaagu 180
   uauaacugaa ugaaugacug ccacaguugc ccauuuccuc cugccaaugg cagcaaacaa 240
   caggaucaac uagggcaaaa uaaauaauug uguggaagcc cuga 284
<210> 16
   <211> 219
   <212> RNA
   <213> Homo sapiens
<220>
   <221> misc_feature
   <222> (70)..(70)
   <223> n is a, c, g, or u
<220>
   <221> misc_feature
   <222> (88)..(88)
   <223> n is a, c, g, or u
<220>
   <221> misc_feature
   <222> (132)..(132)
   <223> n is a, c, g, or u
<220>
   <221> misc_feature
   <222> (205)..(205)
   <223> n is a, c, g, or u
<400> 16
   aacuaaacuu ccuuguaacu uuugagaacu cagcucuggu acuuuuucau gccuugcaaa    60
   auggcguuan ugcagcuagc uugcuaancc uuaugguggg gucuuucauu ccccccucuu    120
   ucuggaaacu gnauaaaauc auuuauucac gugauucuau uucuucugga ucuauugauu   180
   ugaguuggug auacuguugg gucanaacca gggccuguu                          219
<210> 17
   <211> 4179
   <212> RNA
   <213> Homo sapiens
<400> 17
   cagcuccgag cuaggugcua ucgcaaggcc agagcgcaca gcccggcgga gagagcagau      60
   ccuugcucag aucgagucaa aucgggccaa ggcggaggac gaagagucca ggcuccuauu     120
   cuggacuugu uccccagcuc cgggggcgcu ucuagguccu gcagcagcca gcagugcgga     180
   gccaccaacu cggugcugga augaaaaaau ucccgcgcgc cagugcagaa ucuuucuaag     240
   ugacccggag cuucgggugc uagcucugca cgaacuuucc caucaaagug aucgugaauu     300
   uuaagcauca ggagcaggcc agcgaagcuc uacgcgucua aacgucuauc cagaccaaga     360
   guucucugcg gugcagggug cggugccaug cagccaaaag ucccuuuugg ggucacgcaa     420
   gcagaagccc ugcuccgaca ugggggacgu ccagcgggca gcgagaucuc ggggcucucu     480
   guccgcacac augcuguugc ugcuccucgc uuccauaaca augcugcuau gugcgcgggg     540
   cgcacacggg cgccccacgg aggaagauga ggagcugguc cugcccucgc uggagcgcgc     600
   cccgggccac gauuccacca ccacacgccu ucgucuggac gccuuuggcc agcagcuaca     660
   ucugaaguug cagccggaca gcgguuucuu ggcgccuggc uucacccugc agacuguggg     720
   gcgcaguccc ggguccgagg cacaacaucu ggaccccacc ggggaccugg cucacugcuu     780
   cuacucuggc acggugaacg gugaucccgg cucugccgca gcccucagcc ucugugaagg     840
   ugugcguggu gccuucuacc uacaaggaga ggaguucuuc auucagccag cgccuggagu     900
   ggccaccgag cgccuggccc cugccgugcc cgaggaggag ucauccgcac ggccgcaguu     960
   ccacauccug aggcgaaggc ggcggggcag uggcggcgcc aagugcggcg ucauggacga    1020
   cgagacccug ccaaccagcg acucgcgacc cgagagccag aacacccgga accaguggcc    1080
   ugugcgggac cccacgccuc aggacgcggg aaagccauca ggaccaggaa gcauaaggaa    1140
   gaagcgauuu guguccagcc cccguuaugu ggaaaccaug cucguagcug accaguccau    1200
   ggccgacuuc cacggcagcg gucuaaagca uuaccuucua acccuguucu cgguggcagc    1260
   cagguuuuac aagcauccca gcauuaggaa uucaauuagc cugguggugg ugaagaucuu    1320
   ggucauauac gaggagcaga agggaccaga aguuaccucc aaugcagcuc ucacccuucg    1380
   gaauuucugc agcuggcaga aacaacacaa cagccccagu gaccgggauc cagagcacua 1440
   ugacacugca auucuguuca ccagacagga uuuauguggc ucccacacgu gugacacucu 1500
   cggaauggca gauguuggaa ccguauguga ccccagcagg agcugcucag ucauagaaga 1560
   ugaugguuug caagccgccu ucaccacagc ccaugaauug ggccaugugu uuaacaugcc 1620
   gcacgaugau gcuaagcacu gugccagcuu gaauggugug aguggcgauu cucaucugau 1680
   ggccucgaug cucuccagcu uagaccauag ccagcccugg ucaccuugca gugccuacau 1740
   ggucacgucc uuccuagaua auggacacgg ggaauguuug auggacaagc cccagaaucc 1800
   aaucaagcuc ccuucugauc uucccgguac cuuguacgau gccaaccgcc agugucaguu 1860
   uacauucgga gaggaaucca agcacugccc ugaugcagcc agcacaugua cuacccugug 1920
   gugcacuggc accuccggug gcuuacuggu gugccaaaca aaacacuucc cuugggcaga 1980
   uggcaccagc uguggagaag ggaaguggug ugucaguggc aagugcguga acaagacaga 2040
   caugaagcau uuugcuacuc cuguucaugg aagcugggga ccauggggac cguggggaga 2100
   cugcucaaga accuguggug guggaguuca auacacaaug agagaaugug acaacccagu 2160
   cccaaagaac ggagggaagu acugugaagg caaacgaguc cgcuacaggu ccuguaacau 2220
   cgaggacugu ccagacaaua acggaaaaac guucagagag gagcagugcg aggcgcacaa 2280
   ugaguuuucc aaagcuuccu uugggaauga gcccacugua gaguggacac ccaaguacgc 2340
   cggcgucucg ccaaaggaca ggugcaagcu caccugugaa gccaaaggca uuggcuacuu 2400
   uuucgucuua cagcccaagg uuguagaugg cacucccugu aguccagacu cuaccucugu 2460
   cugugugcaa gggcagugug ugaaagcugg cugugaucgc aucauagacu ccaaaaagaa 2520
   guuugauaag uguggcguuu guggaggaaa cgguuccaca ugcaagaaga ugucaggaau 2580
   agucacuagu acaagaccug gguaucauga cauugucaca auuccugcug gagccaccaa 2640
   cauugaagug aaacaucgga aucaaagggg guccagaaac aauggcagcu uucuggcuau 2700
   uagagccgcu gaugguaccu auauucugaa uggaaacuuc acucugucca cacuagagca 2760
   agaccucacc uacaaaggua cugucuuaag guacaguggu uccucggcug cgcuggaaag 2820
   aauccgcagc uuuaguccac ucaaagaacc cuuaaccauc cagguucuua ugguaggcca 2880
   ugcucuccga cccaaaauua aauucaccua cuuuaugaag aagaagacag agucauucaa 2940
   cgccauuccc acauuuucug agugggugau ugaagagugg ggggagugcu ccaagacaug 3000
   cggcucaggu uggcagagaa gaguagugca gugcagagac auuaacggac acccugcuuc 3060
   cgaaugugca aaggaaguga agccagccag uaccagaccu ugugcagacc uuccuugccc 3120
   acacuggcag gugggggauu ggucaccaug uuccaaaacu ugcgggaagg guuacaagaa 3180
   gagaaccuug aaaugugugu cccacgaugg gggcguguua ucaaaugaga gcugugaucc 3240
   uuugaagaag ccaaagcauu acauugacuu uugcacacug acacagugca guuaagaggc      3300
   guuagaggac aagguagcgu ggggaggggc ugauacacug agugcaagag uacuggaggg      3360
   auccagugag ucaaaccagu aagcagugag guguggcaag gaggugugug uaggggauac      3420
   auagcaaagg agguagauca ggacacuacc cugccaguua cauucugaua agguaguuaa      3480
   ugaggcacag uagcaucuga aagaccauac agagcacuaa ggagccccaa agcacuauua      3540
   guaucucuuu ucuuauaucu aucgcccaaa uaauuuucag agucuggcag aagcccuguu      3600
   gcacuguacu aacuagauac uucuuaucac aaagauuggg aaaggcaaag cagaaagaug      3660
   guaagacugg guuucaaaca aggcuugguu ucaaucacug gaggcaagga ggaggggaca      3720
   aacaagauca uuauucgaag ucgcugguug cugugguuuu acggaagguu gaugcaucau      3780
   uccuaucaac agugaaaagu ucagcuuguu caacgugaca gaaaggcuca ucuccgugaa      3840
   agagcuccug auuucuucuu acaccaucuc aguucuuaac uauaguucau guugagguag      3900
   aaacaauuca ucuauuuaua aaauguacau uggaaaaaaa aagugaaguu uaugagguac      3960
   acauaaaaac ugaaggaaac aaugagcaac augccuccug cuuugcuucc uccugaggua      4020
   aaccugccug gggauugagg uuguuuaaga uuauccaugg cucacaagag gcaguaaaau      4080
   aauacauguu gugccagagu uagaaugggg uauagagauc agggucccau gagaugggga      4140
   acauggugau cacucaucuc acaugggagg cugcugcag                             4179
<210> 18
   <211> 1416
   <212> RNA
   <213> Homo sapiens
<400> 18
   ccgcgggccc gcguucaugc accgccugcu ggccugggac gcagcaugcc ucccgccgcc      60
   gcccgccgcc uuuagaccca uggaaguggc caacuucuac uacgagcccg acugccuggc     120
   cuacggggcc aaggcggccc gcgccgcgcc gcgcgccccc gccgccgagc cggccauugg     180
   cgagcacgag cgcgccaucg acuucagccc cuaccuggag ccgcucgcgc ccgccgcgga     240
   cuucgccgcg cccgcgcccg cgcaccacga cuuccucucc gaccucuucg ccgacgacua     300
   cggcgccaag ccgagcaaga agccggccga cuacgguuac gugagccucg gccgcgcggg     360
   cgccaaggcc gcgccgcccg ccugcuuucc gccgccgccu cccgccgcgc ucaaggcgga     420
   gccgggcuuc gaacccgcgg acugcaagcg cgcggacgac gcgcccgcca uggcggccgg     480
   uuucccguuc gcccugcgcg ccuaccuggg cuaccaggcg acgccgagcg gcagcagcgg     540
   cagccugucc acgucgucgu cguccagccc gcccggcacg ccgagccccg ccgacgccaa     600
   ggccgcgccc gccgccugcu ucgcggggcc gccggccgcg cccgccaagg ccaaggccaa     660
   gaagacggug gacaagcuga gcgacgagua caagaugcgg cgcgagcgca acaacaucgc     720
   ggugcgcaag agccgcgaca aggccaagau gcgcaaccug gagacgcagc acaaggugcu     780
   ggagcugacg gcggagaacg agcggcugca gaagaaggug gagcagcugu cgcgagagcu     840
   cagcacccug cggaacuugu ucaagcagcu gcccgagccg cugcuggccu cggcgggcca     900
   cugcuagcgc ggcgcggugg cguggggggc gccgcggcca ccgugcgccc ugccccgcgc     960
   gcuccggccc cgcgcgcgcg cccggaccac cgugcgugcc cugcgcgcac cugcaccugc    1020
   accgagggga caccgcgggc acaccgcggg cacgcgcggc gcacgcaccu gcacagcgca    1080
   ccggguuucg ggacuugaug caauccggau caaacguggc ugagcgcgug uggacacggg    1140
   acuacgcaac acacguguaa cugucuagcc gggcccugag uaaucaccuu aaagauguuc    1200
   cugcgggguu guugauguuu uugguuuugu uuuuguuuuu uguuuuguuu uguuuuuuuu    1260
   uuuggucuua uuauuuuuuu uguauuauau aaaaaaguuc uauuucuaug agaaaagagg    1320
   cguauguaua uuuugagaac cuuuuccguu ucgagcauua aagugaagac auuuuaauaa    1380
   acuuuuuugg agaauguuua aaagccaaaa aaaaaa                              1416
<210> 19
   <211> 566
   <212> RNA
   <213> Homo sapiens
<220>
   <221> misc_feature
   <222> (154)..(154)
   <223> n is a, c, g, or u
<220>
   <221> misc_feature
   <222> (254)..(254)
   <223> n is a, c, g, or u
<220>
   <221> misc_feature
   <222> (262)..(262)
   <223> n is a, c, g, or u
<220>
   <221> misc_feature
   <222> (287)..(287)
   <223> n is a, c, g, or u
<220>
   <221> misc_feature
   <222> (305)..(305)
   <223> n is a, c, g, or u
<220>
   <221> misc_feature
   <222> (354)..(354)
   <223> n is a, c, g, or u
<220>
   <221> misc_feature
   <222> (378)..(378)
   <223> n is a, c, g, or u
<220>
   <221> misc_feature
   <222> (419)..(419)
   <223> n is a, c, g, or u
<220>
   <221> misc_feature
   <222> (449)..(449)
   <223> n is a, c, g, or u
<220>
   <221> misc_feature
   <222> (492)..(493)
   <223> n is a, c, g, or u
<220>
   <221> misc_feature
   <222> (521)..(521)
   <223> n is a, c, g, or u
<220>
   <221> misc_feature
   <222> (550)..(550)
   <223> n is a, c, g, or u
<400> 19
   aaaauguaga aggcaagauu uaauaaggca gcaacaugaa agcacacaga ccagagcucu 60
   ggggucgaaa uaaagcagca acauggaagc acacagagcu cuggggucga aacucauaca 120
   ccuuagcaca ggguagagga gucucgacgg ucanccagaa uuuuucacag gcuuauauag 180
   uaaaacucaa agggggagaa cugggcaggg aaaguacaag uuuacaucac uagggaguuc 240
   ugccaaagga caanggguuc unucagagga aucuacguaa cuaaggnguc auguccuauc 300
   aaggnaucua cguaacuaag gagucauguc cuaucauuug gcaauguacc cggnucuuuu 360
   gagguuguuc cggagggncu uauucucaaa auguuuuuca gauuggaagg gguggguunc 420
   cgguaaaaag uucuguuuuc aaagaggcng uaauuuucua uucuucauuc cccauucucc 480
   uguaaguauu cnnaucuuag aauuucagaa guccauauuc ncuauguggg gaauaacugg 540
   cuuuaacccn aucuuuaaaa uggggg 566
<210> 20
   <211> 358
   <212> RNA
   <213> Homo sapiens
<220>
   <221> misc_feature
   <222> (269)..(269)
   <223> n is a, c, g, or u
<400> 20
   uuuuuuuuuu uuuuuuuaug aaacaguuuu guucagccca ugacuuugug caugacuguc 60
   guccguucua gucaccugug cucuccaucu acugccuuuu aaagcugcgu caugagaagg 120
   aucuacacgu uccaccauga cucucguuuc uugccacaag uagaagaaau gguugauucu 180
   uugcuuuucg guuaggccgu uaaacaaaac ucagucacac cccugccuuc cacccucaaa 240
   cugugaucac guggcuguuc uuguaguuna accagggcaa cacuauauau uccaggucua 300
   uacauugcug aggucuuuuu cugguuguuc auguaucagu guuuucaacc ucgugccg 358
<210> 21
   <211> 2149
   <212> RNA
   <213> Homo sapiens
<400> 21
   gcggccgcgg cuccagaccc cggccuugcg cacccucccc ccaccuccag cccgcgccuc      60
   cccccccccc acgcauggcu caccaccccu cggguuuccc ugucaucccu cagguuccug     120
   cacuuggcaa ucauccacga agagaagccg cugaccaugg aagucauugg ucaggugaag     180
   ggagaccugg ccuuccucaa cuuccagaac aaccugcagc aggugcgcug cuugcuugcc     240
   ccgcggugcc ccucuuugac cccuuggugg agucagaugu agcacggucg cccccaaagc     300
   guuucuaaau uaauagucac uuaguucuua ucugccuugg cuuuuuugca uucaaaucag     360
   ccaacgucuu agaaccagaa gaaaaauccc uugguuuuag ugaggucuuu augacccacc     420
   uagggccucc uguuugccug aucuccuaag agacaaaugg gaggaucaga uuccauagcg     480
   ggaggugucu gggguucagg aaacucaaga cuauggguuu gcuccauggc uuaucccuuu     540
   cuguuuccuc uuccauuugu agacuccacu ccacuuggcu gugaucacca accagccagg     600
   aauugcugag gcacuucuga aagcuggcug ugauccugag cuccgagacu uucgaggaaa     660
   uaccccucua caucuugccu gugagcaggg cugccuggcc aguguagcag ucuugacgca     720
   gaccugcaca ccccagcauc uccacuccgu ccugcaggcc accaacuaca augguagguc     780
   ugccagucca uccaaggaug cagaggaggg agagagaugg ggccacuuga gucuuaaacu     840
   ccgaacguau acaaaguuca gacacuguga ucuuuuuaaa aaguuuucuc cucgaugccu     900
   auaugauauu cacucagaac ccagauucug aguucuucaa aacugaugau guuugugguu     960
   guccucaaga caauagacau gaguugugug aggauugaaa cacguaguac aguuuuuguc    1020
   uuccuccucc aggccacacg ugucugcacc uagccucuac ucacggcuac cuggccaucg    1080
   uggagcacuu ggugacuuug ggugcugaug ucaacgcuca ggugaguaca ucucccuucc    1140
   accuaaucuc uguugggcug gcucugaugg ugagcagguu uccagaugca gccguaaacu    1200
   aacgccugau ugcuuuuggu uucaggagcc cugcaauggc cggacagccc uccaccuugc    1260
   gguggaccug cagaauccug accugguuuc gcucuuguug aaaugugggg cugaugucaa    1320
   caggguaacc uaccaaggcu acucccccua ccagcuuacc uggggccgcc caaguacccg    1380
   gauacagcag cagcugggcc agcugacccu ggaaaaucuc cagaugcuac ccgagagcga 1440
   ggaugaggag agcuaugaca cggagucaga auucacagag gaugagguga guguuccucc 1500
   ccucagcacg cugacggcug uucuagggcu gcuuuggauc agagggauuu caguuguuua 1560
   acuucucaga cucggcuugc aaagcaggau cccaagaauu ugucucuggu uuguuuuaag 1620
   agcuuacccu uuugguugag gaaugaggga auucuagaaa uugaacccag gccuuagcac 1680
   auggugauaa gcacacguuc aaccauuaag cuccaccccc ucaauagcuu agacuuuuuu 1740
   uuuuuuuaag gaaagaauag guaagggaaa cuccuacagc cuggugcccu uguucuauuu 1800
   ggguuaagga gaaaaagagc ccaagaauag gaguuauuuc agcagcagcu cuccccuuau 1860
   cccaaugucu uggugaaguu cuaggaauuu aauaugucuu uuuccccucu cuuguuuuag 1920
   cugcccuaug augacugugu guuuggaggc cagcgucuga cauuauaagu ggaaaguggc 1980
   aaaaaagaau guggacuugu auauuuguac aaauagaguu uuauuuuucu aaaaaaaaaa 2040
   aaaaaaaaaa aaaaaaaaaa aaaaaguaua cuuagcacca caccacacag cgccuagacc     2100
   caggcauuuu acuggggaga uucggcuguu gucuuuguga aauccgggg                 2149
<210> 22
   <211> 2062
   <212> RNA
   <213> Homo sapiens
<400> 22
   cucucgguca cugccggucg cuuccugagc cgcugcuggc ucugugucuc uguccucagc        60
   guucucuucc ucguccucgu ccuaccacgc cauggaaggu uaccauaagc cagaucagca       120
   gaagcuccag gcccugaagg acacagccaa ucgccugcgc aucagcucca uccaggccac       180
   caccgcggca ggcucaggcc accccacauc augcugcagc gcugccgaga ucauggcugu       240
   ccuguuuuuc cauaccaugc gcuacaaggc ccuggauccc cgaaacccuc acaaugaucg       300
   cuuugugcuc ucuaagggcc augcagcucc cauuuuauau gcagucuggg cugaagcugg       360
   cuuccugccc gaggccgagc ugcugaaccu gaggaagauc agcucugacu uggacgggca       420
   uccugucccg aaacaagccu ucaccgaugu ggccacuggc ucccugggcc agggccuggg       480
   agcugcuugc gggauggcau acacaggcaa auacuuugac aaagccagcu accgagucua       540
   uugcaugcug ggagacgggg aggucuccga gggcuccguc ugggaggcca uggccuuugc       600
   uggaauuuac aagcuggaca accucguugc cauuuuugac aucaaccgcc ugggccagag       660
   cgacccagcc ccgcugcagc accaggugga caucuaccag aagcgcugug aggccuuugg       720
   cuggcacacc aucaucgugg acggacacag cguggaggag cugugcaagg ccuuugguca       780
   ggccaagcac caaccaacag ccaucaucgc caagaccuuc aagggccgag ggaucacagg       840
   gauugaagac aaggaggcgu ggcacgggaa gccccucccc aaaaacaugg ccgagcagau       900
   uauccaggag auuuacagcc agguucagag caaaaagaag auccuggcca cgcccccuca 960
   ggaggaugcc ccauccgugg acauugcuaa cauccgaaug ccuacgccac ccagcuacaa 1020
   agugggggac aagauagcca cccggaaggc cuauggacug gcccucgcua agcugggcca 1080
   cgccagugac cguaucauug cccuggaugg agacaccaag aauuccaccu ucucggagcu 1140
   cuucaaaaag gagcacccag accgguucau ugagugcuac auugccgagc aaaacauggu 1200
   gagcauugcc gugggcugug ccacacguga ccggacagug cccuucugca guacuuucgc 1260
   ggccuucuuc acacgggccu ucgaccagau ucgcauggcc gccaucucug agagcaacau 1320
   caaccucugu ggcucccacu gugguguguc cauuggggaa gacgggcccu cucagauggc 1380
   ccucgaagac cuggccaugu uccggucagu ccccaugucc accgucuuuu acccaagcga 1440
   uggaguugca acagagaagg caguggaguu agcagccaac acaaagggca uuugcuucau 1500
   ccggaccagc cgcccagaga augccauuau uuauagcaac aaugaggauu uccaggucgg 1560
   ccaagccaag gugguccuga agagcaagga ugaccaagug acagugaucg gggcuggugu 1620
   aacucugcau gaggccuugg cugcugcaga gagucuaaag aaagauaaga ucagcauccg 1680
   ggugcuggau cccuucacua ucaagccccu ggacaggaaa cucauccuag acucugcccg 1740
   agcaaccaaa ggcaggaucc ucaccgugga ggaccacuac uacgaaggug gcauaggaga 1800
   ggcagugucu gcugccguag ugggugaacc uggagugacg gucacucgcc uggcugucag 1860
   ccaaguacca cgaaguggca agccagcuga gcuacugaag auguucggua uugacaagga 1920
   cgccauugug caagcuguga aaggccuugu caccaagggc uagggagggc augggaugcu 1980
   ggguggguga acuacacauu ccagggaggu ucuggcagag guggcgaagg uguacugagu       2040
   ggggagguaa auauauguuu ug 2062
<210> 23
   <211> 230
   <212> RNA
   <213> Homo sapiens
<400> 23
   uuaacggugu cauaaauaag uaauauaacu uuauuaaaau gaaaagacaa uauucaaaau 60
   aaugcaacaa aaugaauaaa uccuuugucc aauacuguac acacagugcg gagaucagug 120
   cauuuuucua aagcauguuu uaaccuucau uuaguucaua cuaaaguaag cuuuaaauag 180
   cucaaauaau gucauucagc aguuuaaacu gaacagcuug uugggacaug 230
<210> 24
   <211> 1560
   <212> RNA
   <213> Homo sapiens
<400> 24
   ugaguucucg uaaacuccag agcagcgaua ggccguaaua ucggggaaag cacuauaggg 60
   acaugauguu ccacacguca caugggucgu ccuauccgag ccagucgugc caaaggggcg 120
   gucccgcugu gcacacuggc gcuccaggga gcucugcacu ccgcccgaaa agugcgcucg 180
   gcucugccag gacgcggggc gcgugacuau gcgugggcug gagcaaccgc cugcugggug 240
   caaacccuuu gcgcccggac ucguccaacg acuauaaaga gggcaggcug uccucuaagc 300
   gucaccacga cuucaacguc cugaguaccu ucuccucacu uacuccguag cuccagcuuc 360
   accagaucuc ggaauggacc ccaacugcuc cugcuccacc gguaagacuc ccgauccuug 420
   gucuuuagaa uaccaaguug ggaccgcaga gcggaauccc cgaguuguag aggcuuggcg 480
   ggaauaggca ccuuuaguug gcgauucauu ccgguucuuu cuagaauccg cucuugcaaa 540
   agccuucauu aguuacgagu auugucgaac ggguccuuug gcgggguugg ggcuaggauu 600
   uagacgcgca aauguccggu uccugaucac ccaguuagug gggacaucug gguugagucc 660
   caggcauuac uaaacuuacu gugaauugcu ugaauuaaga aagaggugaa ggaccuuuau 720
   gucuugggac ucaaagacau aaucccugac uuaaccugug aggagaaaag uggggcuagg 780
   cucccugcag cuccgaggag gacuuaguga acugagccgg gacucguggu guuggccacu 840
   gcuguaaugc ugccucccuc augcugucuu cuuucuccuc ccaggcggcu ccugcacuug 900
   caccagcucc ugcgccugca agaacugcaa gugcaccucc ugcaagaaga gugaguuggg 960
   acaccuuggg uggcggcuaa ggcuaggggc ggggaacucc uacaaaacug gcucugagaa 1020
   auguccuuug cuucccggag gccauuguau ugucucgggg acagaacuau acagagaacu 1080
   auuuaaaaaa accgaggucu ucucuguugg ggacaggaag cagaggucuu cagccaggcu 1140
   gccucuuccu ccuucuucua ggcugcugcu ccugcugucc cgugggcugc uccaaaugug 1200
   cccagggcug ugucugcaaa ggcgccgcgg acaagugcac gugcugugcc ugaugugacg 1260
   aacagcgcug ccaccacgug uaaauaguau cggaccaacc cagcgucuuc cuauacaguu 1320
   ccacccuguu uacuaaaccc ccguuuucua ccgaguacgu uaauaauaaa agccuguuug 1380
   agucuaacuc ugguuucuug gugugguuug gcaauaagaa acugggguga cuugauaguc 1440
   uggggaucug guuuuggacc cccucgugcc uuuaccuccg cccucuggcc cucacagagg 1500
   gguaaugucu uuggguaaag ccaagcuaua ucccauaagc uuccucaugg aaaacagcug 1560
<210> 25
   <211> 253
   <212> RNA
   <213> Homo sapiens
<400> 25
   gcggcacgau gugucuucgg guggcuuuuu uuuuuuuguu uugaauaaug uuuacaauuu 60
   cccucaauca cuuuuauaga aauccaccuc caggcccccc ccuuucccca cuuaggccuu 120
   cgaggcuguc ugaagaugcu ugagaaacuc aaccaaaucc caguucaauu cagacuuugc 180
   acauauauuu auauuuauaa ucagaaaaga aacauuucag uaauuuauaa uaaaagagca 240
   cuauuuuuua acg 253
<210> 26
   <211> 3660
   <212> RNA
   <213> Homo sapiens
<400> 26
   uugcaggcga gggcuuccac aacuaccacc acaccuuccc cuucgacuac ucugccagug    60
   aguaccgcug gcacaucaac uucaccacgu ucuucaucga cugcauggcu gcccugggcc   120
   uggcuuacga ccggaagaaa guuucuaagg cuacugucuu agccaggauu aagagaacug   180
   gagacgggag ucacaagagu agcugagcuu ugggcuucug aguuccuguu ucaaacguuu   240
   ucuggcagag auuuaauauu cuguugauua acuaacaacu ggauauugcu aucggggugu   300
   uaaugaugca uuuaaccuau uccgguacag uauucuuaua aaaugagaaa gcuuugauca   360
   cguuuugagg uaauaaauau uuuauuuagc uaggauuaac caugccacaa gacauuauau   420
   auuucuaagc acacaugaua aaugcauaua caauuuugca caacagcuuu aaauaauaac   480
   aauaaauuug aacauucuau acagagagga ucaaagccaa ggaacaugcu guuuugaugc   540
   uagggugagc auggugcuca gucccuguuu guuugcaugg uguccagcuu uguuucuucu   600
   cugucaucac caccuucagg caaauaguug accaaccacu ggccuguguc uguccacccu   660
   ccaaagccca ggccaccuuu cuguuuucug aaauacugau ccuuccuccu gaauacaucc   720
   cuccuuguuc cuagcuucaa gacugcugcc ucaaauaggg auagagcaag uccccgcugc   780
   agguugugcu agaugggaug gagaaauuau cuucauuuga uacagagcaa guagauuguc   840
   ucgagagaaa aguuagcaug cgugguauga uuuguaagua aagauggaag agagagagag   900
   agagagagag agagagagag agagagagag agagguagcc auaucuaaca gccuacuuac   960
   caaagacccc aggccucucu gcuuggcaug ccuccuuucu guccauccuc ugaaccccag  1020
   agauuaguga gauuugaaua auuaaaucau uuucagagug aaggggguua augcaggguc  1080
   ugugcuaggg gaggguuuua gcuuuuggua acugaagauu uuuucaugga aaaagucuuc  1140
   guguucaaug ugccuagaac ugauaacuaa acagcugaca uuugucgggg acagauaugg  1200
   ugugaaacua ugaaaauaua agcaaaaucu ucacuuggaa caugaaacua uuucacuuag  1260
   aaaauaaucg aaggacccga gguguugccu ggguugccag uuucuuucgu ggcugggcag  1320
   gaacuaguga gguugagggg cagugucugu aaguagcugc uaagaggugc auuuccagau  1380
   gaagcccuug gggaacaucu gccagggauc cgcauggugu uggcuccauc cauugcuuua  1440
   guuuccuccu uggauugugu agaaacuugg cuucccaugg uuuugaaccu uccaugccuu  1500
   cuuugcuuug uggccaccca gccugccuag ugcugccuag gaagcucuua cccaccugau 1560
   uucuucugac auuucuuucu uuggccuuuu uuucuuucuc cggacaugca gcuaguugcc 1620
   ugaguguauc aagagcaccc aggacuugcu gcuguccagg ccuguuccuc ccccaguauc 1680
   cgugggugug gaagagcugu guagcuucag gaagcagagc caggugccac cuuucugugg 1740
   cuuccagauc cucccuaccu ccaacucaug ugccucuguc acagugauuu caggaaagcu 1800
   ugguagaccc ucuagcaaca ucucgguuca gaaagucucu cugguuugug aguuaacagc 1860
   ucagcuaagu gcuguuuugu cucagugagu uaaccacuga augcgagggu ugguuguuga 1920
   ucugucucgg ugugugucgg aguagacagc auaugcacuu cucccugugc gcuuugcaag 1980
   guaauguggc uuuggcugau ccaugcaggc agguaguggu acagugcugc ugaaaggaag 2040
   aaguucccca uuuuaucugu uaaaacacca gagacauggg caagugcuaa uggaccucac 2100
   uucaggaaga gggucugcuu ccugaagcca gugugugaug aaaagugacu gagaccugau 2160
   aucuaaggug agaccugaua ccuaacacuc ugucacacag uccagggcca acagugcuau 2220
   aggaaagucu agaagaaaac aucacaucag uauuuuagaa ccaucaacca ucucuugucc 2280
   cuauagccca auccagaggc cugguuuuua gaacuggcug uguaaggugc caaacacuca 2340
   guucacuugu agaaucagag ccuuuuuucc ccccuauguu aauugaacac gcgcucugag 2400
   cuguuuuguu gaaguagaaa aucucauaga aaaaucacug uagaucuacu gaccuauagc 2460
   ccucuggaaa ugccuuugag augguuuuac uuuucuaggu cauagaugcc ugauuauaaa 2520
   gaugaacaau aaaaucagcu uucuuucuuu cucuucugau cuuauucccc agaucugauu 2580
   caggccaugu uccaaagcaa ggcuacauug agguccuggu gucuuuaagu aaaggacauc 2640
   uuucagaucc ucucaaagaa ggauuuauaa caguuuccag augaauguac uaauagcuuu 2700
   gggugccuua ucucuuuccu aaucuguagu gccugugagc ucagucucac uccuucccuu 2760
   agcccggaga ccccuuagau cgagugggaa uagucaagag gcuggcugga gagucaucag 2820
   uacauugguu ugcagaaauc uuuuacaggc uacauuuugg aauuuuuuuu uuuuuaguaa 2880
   gugaucaaau uuggugggaa guaauucgag uguauucgau uguauugucg uccucguuau 2940
   cauugucaaa cauguuauag acggcaguug gcacuggggc ugcuaaucuc uggguguagu 3000
   cucugaaacu guagcuccag ugagguggug ugaaagguua gcaaagccac caucugcugg 3060
   ugcuccagcc aaggugccuc uuagccacug aauugcuaug uuauccuuuc ucuuguaaca 3120
   aacccacccc agagauaaag ccuuuaauca acccaagaaa cuccugggcu aaguaucuga 3180
   cagucucaca ucucaacagu gugaauuaag uguccauagc aucagcucag gaggacacuc 3240
   ugggagagug cugacaaaaa aggguuauua auacugaccu acuacuucaa gggcaguucu 3300
   gaggugauua gagcuuuuuu uaaaaaccaa guauuugggg auccucagca gagguauuca 3360
   uacagacucc caaagaacua uauauguucc ugagaccauc guuuagucua cauugcucuu 3420
   cccagagacu gacagauaug accagucaaa gugcaagacu accuacccac ugccaugaaa 3480
   accauugcag gaaaccuuuc ccuuccugaa ugagauuuuu uuuuucccuu uuuauguggg 3540
   guaauuauuu gugacccaag uguaauuugg augauuucca uuaauaucaa cucuugaagc 3600
   cuacuuguac ugauugagau uguauuuguu ccuaauaaaa guggaucugg uuguacuguc 3660
<210> 27
   <211> 1969
   <212> RNA
   <213> Homo sapiens
<400> 27
   acugccaucg aguggguguc caguugaacu ugcucucucu gccaucugcu ccgcgggcgc      60
   cgucagcaug ggucccugga cccacuccuu gcgcgccguc cugcugcugg ugcuuuuggg     120
   agucugcacc gugcgcuccg acacuccugc caacugcacc uacccugauc ugcugggcac     180
   cuggguguuc caggugggcc cuagaaguuc ccgaagcgac auuaacugcu cggugaugga     240
   agcaacagaa gaaaagguag ugguacaccu uaagaaguug gauacugccu acgacgagcu     300
   gggcaauucc gggcauuuua cccucauuua caaccaaggc uucgagauug uguugaauga     360
   cuacaaaugg uuugcguuuu ucaaguauga agucagaggc cacacagcua ucaguuacug     420
   ccaugagacc augacugggu ggguccauga ugugcugggc cggaacuggg cuugcuuugu     480
   uggcaagaag guggaaaguc acauugagaa gguuaauaug aaugcagcac aucuuggagg     540
   ucuccaggaa agauauucug aaagacucua cacucacaac cacaacuuug ugaaggccau     600
   caauaccguu cagaagucuu ggacugcaac ugcauauaag gaauaugaga aaaugagccu     660
   gcgagaucug auaaggagaa guggccacag ccaaaggauc ccaaggccca aaccugcccc     720
   gaugacugau gaaauacagc aacaaauuuu aaauuugcca gaaucuuggg acuggagaaa     780
   cguccaaggc gucaauuaug uuagcccugu ucgaaaccaa gaaucuugug gaagcugcua     840
   cucauuugcc ucuaugggua ugcuagaagc aagaauucgu auauuaacca acaauucuca     900
   gacaccaauc cugaguccuc aggagguugu aucuugcagc cccuaugccc aagguuguga     960
   ugguggauuc ccauaccuca uugcagggaa guaugcccaa gauuuugggg ugguggaaga    1020
   aagcugcuuu cccuacacag ccaaagauuc uccaugcaaa ccaagggaga auugccuccg    1080
   uuacuauucu ucugacuacu acuauguggg ugguuucuau gguggcugca augaagcccu    1140
   gaugaagcuu gagcugguca aacauggacc cauggcaguu gccuuugaag uccacgauga    1200
   cuuccuacac uaccacagug gaaucuauca ccacacuggg cugagugacc cuuucaaccc    1260
   cuucgagcug acaaaucaug cuguuuugcu ugugggcuau ggaagagauc caguuacugg    1320
   gauagaauac uggauuauaa agaacagcug gggcucuaac uggggggaga guggcuacuu    1380
   ccguauccgc agaggaacug augaaugugc aauugagagu auagccgugg cggccauacc    1440
   gauuccuaaa uuauaggaca uagcucccag uguuacauac gggucuuuau cacucacaga 1500
   gugauuuagu cacaugcuga agacuuuuuc agagcaauau cagaagcuua ccacuaagca 1560
   ucuuuaaaga auuuugucuu ugaacuuaaa accauccuug auuuuuuucu uuuaauaucu 1620
   uccccaucaa cuacugaacu acuuuucuuu uuaaaguacu ugguuaagua auacuuuuau 1680
   gagcaguggu ucaguugucc aauauuuuuu gcaggucauc uacaaugcaa ccagauguuu 1740
   caguucuaaa aaucuaugua aaaguacaag cucguuuuua aauuauguaa gucacaugaa 1800
   aacauggcaa aaaaauuagu uaaauuuuuu acaaagaguu uuaaauaaau guuuauguaa 1860
   ucaguaccau agucuuucua ugugugugua caagaauuuu ugucaccuac uucuucccuu 1920
   agaagcauuu augcuccaug gacguacuuc uuuauggaga aaaaaaaaa 1969
<210> 28
   <211> 1478
   <212> RNA
   <213> Homo sapiens
<400> 28
   gagcucgauc ccuguuccgc cuuugcuaug ucugaaggcg uccugcuuug cgcgugucgg 60
   ggccaaaucc agauuuucau uucgcuccag gcuuggacgg cuaaguaggu ccaaaccgca 120
   caaacaggaa ggagggaagg caaggagugc gggcagaggg cgggucguuc ccagcagcac 180
   cccagucccu ccccgcuccg ucuccgaccc acuggggccg gggcgggcgu gcgcgucagc 240
   uggggcuaga aaaggcggcg guccggcccg gcgaggugac agccaacuug gacgccaggu 300
   ccggccgacg ccgccaugag cgccgcgcuu uucagccugg acagcccggu gcgcggcaca 360
   cccuggccca cagaacccgc ggccuucuac gagccaggca ggguggacaa gcccggccga 420
   gggcccgagc caggggaacu gggggagcug ggcuccacga cuccugccau guacgacgac 480
   gagagcgcca ucgacuucag cgccuacauu gacuccaugg ccgccgugcc cacccuagag 540
   cugugccacg acgaacucuu cgccgaccuc uucaacagca accacaaagc ggccggcgcg 600
   ggcggccugg agcugcugca gggcggcccu acgcgacccc cggguguggg gucugucgcu 660
   agggggccgc ucaagcgcga acccgacugg ggcgacggcg acgcgccggg cucccugcug 720
   ccggcgcaag uggcggugug cgcgcagaca guggugagcu uggcggccgc ggcucagccc 780
   acuccaccca cuucgccgga gccuccucga ggcagcccgg ggccgagccu cgcgcccggc 840
   acaguccgag aaaagggcgc gggcaagagg gguccggacc gcggcagccc ggaguaccgg 900
   cagcggcgcg agcgcaacaa caucgcugug cgcaagagcc gcgacaaggc caagcgccgc 960
   aaccaggaga ugcagcagaa gcugguggag uugucggccg agaacgagaa gcugcaucag 1020
   cgcguggagc agcucacccg ggaccuggcu ggccuccggc aguucuucaa aaaacugccc 1080
   agcccgccuu uccugccgcc caccggcgcc gacugccggu aacgcgcggc gugggccuuu 1140
   gagacucuga acgaccuaua ccucagaccc cgacagcggg gagcagacgc cgcccgaauc 1200
   gcuaguuucu uugggaccug cgagcgacag gaagcugcag cuugggcacu ggacugcgag 1260
   agaagcuaua uuaaucuuuc cccuuaaauu auuuuuuaua augguagcau uuucuacguc 1320
   uuauuaccau ugcagcuaag guacauuugu agaaaagaca uuuccgacag acuuuuguag 1380
   auaagaggaa gagacugcgc augcuuuuua uauucauuuu uacaguauuu guaagaauaa 1440
   gaauaagaau aaagaagcau uuaaaucgca aaaaaaaa 1478
<210> 29
   <211> 1786
   <212> RNA
   <213> Homo sapiens
<400> 29
   uugagggugg ccaggccagc guaggaggcc agcguaggau ccugcuggga gcggggaacu 60
   gagggaagcg acgccgagaa agcaggcgua ccacggaggg agagaaaagc uccggaagcc 120
   cagcagcgcc uuuacgcaca gcugccaacu ggccgcugcc gaccgucucc agcucccgag 180
   gacgcgcgac cggacaccgg guccugccac agccgaggac agcucgccgc ucgccgcagc 240
   gagcccgggg cggcccuuca gggggaccuu ucccagaucg cccaggccgc ccggaugugc 300
   acgaaaaugg aacagccuuu cuaucacgac gacucuuacg cagcggcggg auacggucgg 360
   agcccuggca gccugucucu acacgacuac aaacuccuga aacccaccuu ggcgcucaac 420
   cuggcggauc ccuaucgggg ucucaagggu ccuggggcgc gggguccagg cccggagggc 480
   aguggggcag gcagcuacuu uucgggucag ggaucagaca caggcgcauc ucugaagcua 540
   gccuccacgg aacuggagcg cuugaucguc cccaacagca acggcgugau cacgacgacg 600
   cccacgccuc cgggacagua cuuuuacccc cgugggggug gcagcggugg agguacaggg 660
   ggcggcguca ccgaggagca ggagggcuuu gcggacgguu uugucaaagc ccuggacgac 720
   cugcacaaga ugaaccacgu gacgcccccc aacguguccc ugggcgccag cggggguccc 780
   caggccggcc cagggggcgu cuaugcuggu ccggagccgc cucccgucua caccaaccuc 840
   agcaguuacu cuccagccuc ugcacccucu ggaggcuccg ggaccgccgu gcggacuggg 900
   agcucauacc cgacggccac caucagcuac cucccacaug caccacccuu ugcgggcggc 960
   cacccggcac agcuggguuu gagucguggc gcuuccgccu uuaaagagga accgcagacc 1020
   guaccggagg cacgcagccg cgacgccacg ccgccugugu cccccaucaa cauggaagac 1080
   caggagcgca ucaaagugga gcgaaagcgg cugcggaaca ggcuggcggc caccaagugc 1140
   cggaagcgga agcuggagcg caucgcgcgc cuggaggaca aggugaagac acucaaggcu 1200
   gagaacgcgg ggcugucgag ugcugccggu cuccuaaggg agcaaguggc gcagcucaag 1260
   cagaagguca ugacccaugu cagcaacggc ugccaguugc ugcuaggggu caagggacac 1320
   gccuucugag agccucccuu gccccauacg gacaccccca gccuugaagg cugggcgccu 1380
   gccccccacu ggggugaggg gggcaggcga ugggcacccg ccaaaaggcc uggggcgcag 1440
   cucacacacu ggacuccggc ccgcccgccu gcgcccaguc cuuccaccuc gagguuuaca 1500
   uggcccccuu ccagcguauu uuguauguuu uuuuuuucug caaagagacu gaauucauau 1560
   ugaauauaau auauuugugu auuuaacagg agggagaagg gggcugucgc ggcggagcug 1620
   gccgccgcuu gguacucagc ugcggggaua cuagggaggg accuccgccc ccugcccucc 1680
   cccucugcau aguacugugg agaagaaaca cgacuucgug ucuaaagucu auuuuaagau 1740
   guguuugugu guguguguuu gacuuuuuau ugaaucuauu uaagua 1786
<210> 30
   <211> 1897
   <212> RNA
   <213> Homo sapiens
<400> 30
   gaauucggca cgagcagcga gacgccgcgc acggugcuuc cccaguggag ccaaucggcu      60
   aacccgcgcu ccggcagagu ccuuggcgcu cgcccgccgg cgggacagac cacccgccuc     120
   uggccgcucu cuggacccug gccgccccga gcgaagacug gagcaaaaug augcuucaac     180
   auccaggcca ggucucugcc ucagaaguca gugcgaccgc cauugucccc ugccucucac     240
   cuccuggguc acugguauuu gaggauuuug cuaaccugac acccuuuguc aaggaagagc     300
   ugagauucgc cauccagaau aaacaccucu gccaucggau guccucugcg cuggagucag     360
   uuaccgucaa caacagaccc cuggagaugu cagucaccaa gucugaggcg gccccugaag     420
   aagaugagag gaaaaggagg cggcgagaaa gaaauaaaau ugcugcugcc aagugucgaa     480
   acaagaaaaa ggagaagaca gagugccugc agaaagaguc agagaaacug gagaguguga     540
   augcugagcu gaaggcccag auugaggagc ugaagaauga gaaacagcau uugauauaca     600
   ugcucaaccu gcaccggccc accuguaucg uccgggcuca gaauggacgg acaccggaag     660
   acgagaggaa ccucuuuauc caacagauaa aagaaggaac auugcagagc uaagcagagg     720
   uggcacggag gcaauugggg aguucuuacu gaauccuccu uuuccacccc acacccugaa     780
   gccauuggaa aacuggcuuc cugugcacuu cuagaauccc agcagccaag agccguuggg     840
   gcaggagggc cuguggugac cuacugcauu gacccacucu gcccccgagu gaaccgugga     900
   gcaggcagga gcauccuuug ucucaccaau uccaggauuu aggccuuauc aucccggcca     960
   gucucagaug accuagcugg ccccaggcug ggguccuaug caaagcagga ucccacuaau    1020
   gggauucagg cagaaguguc uaccuugaua gguggggugg gaccacaucc uccacugugg    1080
   cugacaacgc ccuuccaagg gaauauggaa ugagaacauu cauuauugag guuguccaau    1140
   ggccagggua ugcuuucuag aaaauaugcu guucuguccc agaaugacug ugcauagggu    1200
   auccguuuca gagccuggug uugugcuauu uagauguuug ucuugcacaa cauuggcaug 1260
   auuuuuccgg gaguuucauc agaucugauu ucugagaguc uggggaucug ccauggugga 1320
   aagugccccu caaaagcauu ugugugccca caugaacugg cuggcaccag gggagugaaa 1380
   cuggcugaug accagcugag ccacuuugug ccaacagagg auggacgaca ccuuucccug 1440
   uacccacugc agaggaagaa cccugggcac agcagcuuug uccuuggcua caaacuguua 1500
   caacgucaca caaugaaggc acaaagucca acuuucaaag gguguaggac uccauacuca 1560
   gugacagggc aggaagagcc aaagauaacc acagccacag ccuguggaga ccaggguugg 1620
   aagccaggug cagggccagg caucugcauu gugggauguu aauggcacuu uugucuugua 1680
   gcuauuuuga gauguggucc agagcauuuc agcugggaga ucucccucug gccaccagga 1740
   cucuggcuac uguuaaaauc cugauguuuc uguggaaucc ucaguguuua aucccacuca 1800
   auaguaucau uacaguuuuc uguaagagaa aauauuacuu auuuauccca guauuccuag 1860
   ccugucaaca uaauaaauau cggaacaaaa ccuggua 1897
<210> 31
   <211> 473
   <212> RNA
   <213> Homo sapiens
<400> 31
   uuuuuuuuuu uuuuuuugcu gauaagaauu cuuuuuaugu uauucgaaua aaaaauacau 60
   ucauacagaa auauaacaau cucgcaaaaa acaauuucaa auaaaaucuu guaaaacaaa 120
   auuuuacaaa aaucuuacaa agauucuuua gauaacaggg ugcuucaaaa aaaaagaaau 180
   aaagaaauuu cacuaauaga aauuuuuuuu uuuaauuuca agcaaaaguu ccugcuugau 240
   ugaggcucag uugucaccug accagaaugg acugcuuagu auuaaaguua cagcaucgac 300
   acggacggca cccagcccca gccaguccag caacgucgcu guguuucaua agugagacgc 360
   gccagcacaa guuuccucuc ucuucuguuu accuucuuac uuaauggaau ugcuauggau 420
   aagcacacag cagggccaaa aaaggaguuu uccaaaaucc agcaaaucaa gug 473
<210> 32
   <211> 1680
   <212> RNA
   <213> Homo sapiens
<400> 32
   uaguggugau cuggaccgug cggacuugcu cgucccucag cucuccuguu aggcgucucu      60
   uuucuccagg aggaaaaaau ggcagcagca guaguggauc cgcaacagag cguggugaug    120
   agaguggcca accugcccuu ggugagcucu accuacgacc uuguguccuc cgcuuauguc    180
   aguacaaagg aucaguaccc guauuugaga uccgugugug agauggccga gaagggcgug    240
   aagaccguga ccucugcggc caugacaagu gcccugccca ucauccagaa gcuggagcca    300
   caaauugcgg uugccaauac cuaugccugc aaggggcuag acaggaugga ggaaagacug    360
   ccuauucuga accagccaac guccgagauu guugccagug ccagaggugc cguaacuggg    420
   gcgaaggaug uggugacgac uaccauggcu ggagccaagg auucuguagc cagcacaguc    480
   ucaggggugg uggauaagac caaaggagca gugacuggca gcguggaaag gaccaagucu    540
   guggucaaug gcagcaucaa uacaguuuug ggaugguggc aguucaugaa caguggagua    600
   gauaaugcca ucaccaaguc ggagaugcug guagaccagu acuucccucu cacucaggag    660
   gagcuggaga uggaagcaaa aaagguggaa ggauuugaua ugguucagaa gccgagcaac    720
   uaugaacggc uggagucccu gucuaccaag cucugcucuc gggcuuauca ccaggcucuc    780
   agcaggguua aagaggccaa acaaaagagc caggagacca uuucucagcu ccacuccacu    840
   guccaccuga uugaauucgc caggaagaau augcacagug ccaaccagaa aauucagggu    900
   gcucaggaua agcucuaugu cucgugggug gaguggaaga gaagcaucgg cuacgacgac    960
   accgaugagu cccacugugu ugagcacauc gagucacgua cucuggcuau cgcccgcaac   1020
   cugacccagc agcuccagac uacaugccag acuguccugg ucaacgccca aggguuacca   1080
   cagaacauuc aagaucaggc caaacacuug ggggugaugg caggcgacau cuacuccgua   1140
   uuccgcaaug cugccuccuu uaaggaagug uccgauggcg uccucacauc uagcaagggg   1200
   cagcugcaga aaaugaagga auccuuagau gaaguuaugg auuacuuugu uaacaacacg   1260
   ccucucaacu ggcugguagg ucccuuuuau ccucagucua ccgaggugaa caaggccagc   1320
   cugaaggucc agcagucuga ggucaaagcu caguaaaccc cuccuuguca ccagagcaug   1380
   auguugcugg ccagaugacc ccuuuugcug uauugaaauu aacuugguag auggcuuuag   1440
   cuuagaaaag cagcuucuua gaaccaaggg ccucauuaug gucacucaca gcucaguuau   1500
   ggucuugccc cagcuggccc uggcacagga guucucuuac cuggcuggug aguggccugu   1560
   guuagucuug ugaggaccug gaggaaccua aaagcucaga ugcacuuaca gucuugucug   1620
   uggccuuugu auuguuauug gcuguaaacg ucugucugga ccgaauaaag auucacguga   1680
<210> 33
   <211> 1783
   <212> RNA
   <213> Homo sapiens
<400> 33
   ggcacgaggg agcugcagug uucgcgcuug guagcuggug caucggacuc agcuggcuuu 60
   gugucccuga ggcucaccga aaaacacuuu cucagcccuc ugacuccaga gagagagaga 120
   gagagguacu uuuugugguc accgacuuug accccugcag aggcugagcg auggcgucua 180
   ugggacuaca gguccuggga aucuccuugg caguccuggg cuggcugggg aucauccuga 240
   guugugcgcu ccccaugugg cgggugaccg ccuucaucgg cagcaacauc gucacggcac 300
   agaccagcug ggagggccuc uggaugaacu gcguggugca gagcacaggu cagaugcagu 360
   gcaagaugua cgacucgaug cucgcccugc cgcaggaccu gcaggccgcc cgagcccuua 420
   uggucaucag caucaucgug ggugcucugg ggaugcuucu cucaguggua gggggcaagu 480
   gcaccaacug cauggaggac gagaccguca aggccaagau caugaucacc gccggagccg 540
   uguucaucgu ggcaagcaug cugauuaugg ugcccguguc cuggaccgcu cacaacguca 600
   uccgcgacuu cuacaacccu augguggcuu ccgggcagaa gagggaaaug ggggccucgc 660
   uuuacgucgg cugggcggcc uccgggcugc ugcuccuggg aggaggccuc cucugcugca 720
   guugcccacc ucguagcaac gacaagcccu acucggccaa guacuccgcc gcccgcucug 780
   uccccgccag caacuaugug uaaggugggc cacucugucc acauugccuu uguuauuuuu 840
   uuucggauug agcucauaac agccuguggc cccucacauu cuccaggacc ugcccugcua 900
   ugggccacua acugcuugcu ggggacaggc aaacccggac ugugcaaagu uacuagcccg 960
   uagcucuugg gcugcuccac auggcuccuu acggccggca agaauggaug uaaaaauauc 1020
   uugcugcuua cauccaaauu gcgguggaua ugggcugaag gcagaagcag cugggaaggg 1080
   caguagaggc gcaagcuggg uccugcuggc cgggguagcu cagcugugac uuuggacucg 1140
   gaguggaugu ccucauguua gcaaacgucc acuguccuuu cucuaucccc cucacucagc 1200
   cuacacguua cuccagcgcu acucuugcca uuacgccccg uguuuccgag cacagcuggu 1260
   ccuaccccaa gucauggugu gcugagugac ugaugagggg ccauugagag ccggugggcu 1320
   cugccaugga acccuuccgu ugauuagcaa ugacugugcu ugacccaccc accuacccua 1380
   cuaaugaauu ucuguagagu ggauggacgg guuugaggga agaagggugg agguggauua 1440
   aacugguuug gggagggcug gggaccuaga agcagcccag ugugucccca ccccuuuucc 1500
   gcacugucuu gcuaauguuc ugaucacugu gcgcccccuc ccucuucaga aggacccugg 1560
   gccucuugag uuggccccuc ugaguucccu cccuuugccc auuucaagga caccggccag 1620
   ucugcggaag gaagguacgg gggggggggg ggggggugau ggcauuguac cagggagucu 1680
   ccuggacucc ccugccuucu cugugguuuc uuguuuugua auuaaggucu guucacagcu 1740
   guaauuauua uuauuuucua caauaaaugg caccugcaua cag 1783
<210> 34
   <211> 1786
   <212> RNA
   <213> Homo sapiens
<400> 34
   uugagggugg ccaggccagc guaggaggcc agcguaggau ccugcuggga gcggggaacu 60
   gagggaagcg acgccgagaa agcaggcgua ccacggaggg agagaaaagc uccggaagcc 120
   cagcagcgcc uuuacgcaca gcugccaacu ggccgcugcc gaccgucucc agcucccgag 180
   gacgcgcgac cggacaccgg guccugccac agccgaggac agcucgccgc ucgccgcagc 240
   gagcccgggg cggcccuuca gggggaccuu ucccagaucg cccaggccgc ccggaugugc 300
   acgaaaaugg aacagccuuu cuaucacgac gacucuuacg cagcggcggg auacggucgg 360
   agcccuggca gccugucucu acacgacuac aaacuccuga aacccaccuu ggcgcucaac 420
   cuggcggauc ccuaucgggg ucucaagggu ccuggggcgc gggguccagg cccggagggc 480
   aguggggcag gcagcuacuu uucgggucag ggaucagaca caggcgcauc ucugaagcua 540
   gccuccacgg aacuggagcg cuugaucguc cccaacagca acggcgugau cacgacgacg 600
   cccacgccuc cgggacagua cuuuuacccc cgugggggug gcagcggugg agguacaggg 660
   ggcggcguca ccgaggagca ggagggcuuu gcggacgguu uugucaaagc ccuggacgac 720
   cugcacaaga ugaaccacgu gacgcccccc aacguguccc ugggcgccag cgggggaccc 780
   caggccggcc cagggggcgu cuaugcuggu ccggagccgc cucccgucua caccaaccuc 840
   agcaguuacu cuccagccuc ugcacccucu ggaggcuccg ggaccgccgu gcggacuggg 900
   agcucauacc cgacggccac caucagcuac cucccacaug caccacccuu ugcgggcggc 960
   cacccggcac agcuggguuu gagucguggc gcuuccgccu uuaaagagga accgcagacc 1020
   guaccggagg cacgcagccg cgacgccacg ccgccugugu cccccaucaa cauggaagac 1080
   caggagcgca ucaaagugga gcgaaagcgg cugcggaaca ggcuggcggc caccaagugc 1140
   cggaagcgga agcuggagcg caucgcgcgc cuggaggaca aggugaagac acucaaggcu 1200
   gagaacgcgg ggcugucgag ugcugccggu cuccuaaggg agcaaguggc gcagcucaag 1260
   cagaagguca ugacccaugu cagcaacggc ugccaguugc ugcuaggggu caagggacac 1320
   gccuucugag agccucccuu gccccauacg gacaccccca gccuugaagg cugggcgccu 1380
   gccccccacu ggggugaggg gggcaggcga ugggcacccg ccaaaaggcc uggggcgcag 1440
   cucacacacu ggacuccggc ccgcccgccu gcgcccaguc cuuccaccuc gagguuuaca 1500
   uggcccccuu ccagcguauu uuguauguuu uuuuuuucug caaagagacu gaauucauau 1560
   ugaauauaau auauuugugu auuuaacagg agggagaagg gggcugucgc ggcggagcug 1620
   gccgccgcuu gguacucagc ugcggggaua cuagggaggg accuccgccc ccugcccucc 1680
   cccucugcau aguacugugg agaagaaaca cgacuucgug ucuaaagucu auuuuaagau 1740
   guguuugugu gugugugguu gacuuuuuau ugaaucuauu uaagua 1786
<210> 35
   <211> 1389
   <212> RNA
   <213> Homo sapiens
<400> 35
   augcagcuga gaaaaaugca gaccaucaaa aaggagcccg caccccuaga uccuaccagc      60
   agcucagaca agaugcugcu gcugaacucu gccuuagcug agguggccga ggaccuagcc     120
   ucaggugaag auuugcuccu gaacgaaggg agcaugggga aaaacaaauc cucggcgugu     180
   cggagaaaac gggaauucau uccggacgag aagaaagacg ccauguauug ggagaaacgg     240
   cggaaaaaca acgaagcugc caaaagaucu cgggagaagc gccgccucaa ugaccugguu     300
   uuggagaaca agcugauugc ccugggagaa gaaaaugcca cuuuaaaagc ugagcugcuc     360
   ucccugaaau uaaaguuugg uuuaauuagc uccacggcgu augcccaaga aauccagaaa     420
   cucaguaauu ccacagcugu cuacuuucag gacuaccaga cauccaaggc ugccgugagc     480
   ucuuuugugg acgagcauga gccugcgaug guagccggaa guugcaucuc agucaucaag     540
   cacucucccc agagcucgcu cuccgaugug ucagaggugu ccucggugga gcacacucag     600
   gaaagccccg cacagggagg cugccggagc ccugagaaca aguucccugu gaucaagcag     660
   gagcccgugg aguuggagag cuuugccagg gaggccaggg aggagcgggg cacguauucc     720
   accuccaucu accagagcua caugggaagc ucuuucucca cuuacuccca cuccccaccc     780
   cucuugcagg uccauggguc cacuagcaac uccccaagaa ccucagaggc cgaugagggu    840
   guagugggca agucuucuga uggggaagac gaacaacagg ucccuaaggg ccccauccau     900
   ucuccagugg agcugcaacg gguucacgcc acggugguga agguuccgga agugaacccu     960
   ucugccuuac cgcacaagcu ucggauuaaa gccaaggcca ugcaggucaa aguggaggcu    1020
   uuggacagcg aguuugaagg caugcagaaa cucucuucac ccgccgaugc gaucgccaaa    1080
   agacauuuug accuggagaa acauggaacc ucggguaugg cccauuccuc ccucccuccu    1140
   uucucagugc aggugacgaa cauucaagau ugguccucca aaucggaaca cuggcaucac    1200
   aaagaacuga gcagcaaaac ucagaguagc uucaaaacag guguggugga agucaaagac    1260
   gguggcuaua agguuuccga agcugagaau uuguauuuga agcagggaau agcaaacuua    1320
   ucugcagagg uggucucgcu caagagauuc auagccacac aaccgaucuc ggcuucggac    1380
   uccagguaa                                                             1389
<210> 36
   <211> 961
   <212> RNA
   <213> Homo sapiens
<400> 36
   gcucccggga aacgaaugag gaaccaccuc cuccugcugu ucaaguacag gggccuggug      60
   cgcaaaggga agaaaagcaa aagacgaaaa uggcuaaauu uaagauccgu ccagccacug     120
   ccucugacug cagugacauc cugcgacuga ucaaggaacu ggcuaaauau gaauacaugg     180
   aagaucaagu cauuuuaacu gagaaagauc uccaagagga uggcuuugga gaacaccccu     240
   ucuaccacug ccugguugca gaagugccua aagagcacug gaccccugaa ggacauagca     300
   uuguuggguu cgccauguac uauuuuaccu augacccaug gauuggcaag uugcuguauc     360
   uugaagacuu cuucgugaug agugauuaca gaggcuuugg uauaggauca gaaauuuuga     420
   agaaucuaag ccagguugcc augaaguguc gcugcagcag uaugcacuuc uugguagcag     480
   aauggaauga accaucuauc aacuucuaca aaagaagagg ugcuucggau cuguccagug     540
   aagagggaug gaggcucuuc aagauugaca aagaguacuu gcuaaaaaug gcagcagagg     600
   agugaggcgu gccgguguag acaaugacaa ccuccauugu gcuuuagaau aauucucagc     660
   uucccuugcu uucuaucuug uguguaguga aauaauagag cgagcaccca uuccaaagcu     720
   uuauuaccag ugacguuguu gcauguuuga aauucggucu guuuaaagug gcagucaugu     780
   augugguuug gaggcagaau ucuugaacau cuuuugauga agaacaaggu gguaugaucu     840
   uacuauauaa gaaaaacaaa acuucauucu ugugagucau uuaaaugugu acaauguaca     900
   cacugguacu uagaguuucu guuuugauuc uuuuuuuuua aauaaacucg cucuuugauu     960
   u                                                                     961
<210> 37
   <211> 2187
   <212> RNA
   <213> Homo sapiens
<400> 37
   cgcuggcucc gugcgccaug gucacccaca gcaaguuucc cgccgccggg augagccgcc      60
   cccuggacac cagccugcgc cucaagaccu ucagcuccaa aagcgaguac cagcuggugg     120
   ugaacgccgu gcgcaagcug caggagagcg gauucuacug gagcgccgug accggcggcg     180
   aggcgaaccu gcugcucagc gccgagcccg cgggcaccuu ucuuauccgc gacagcucgg     240
   accagcgcca cuucuucacg uugagcguca agacccaguc ggggaccaag aaccuacgca     300
   uccaguguga ggggggcagc uuuucgcugc agagugaccc ccgaagcacg cagccaguuc     360
   cccgcuucga cuguguacuc aagcuggugc accacuacau gccgccucca gggacccccu     420
   ccuuuucuuu gccacccacg gaacccucgu ccgaaguucc ggagcagcca ccugcccagg     480
   cacuccccgg gaguaccccc aagagagcuu acuacaucua uucugggggc gagaagauuc      540
   cgcugguacu gagccgaccu cucuccucca acguggccac ccuccagcau cuuugucgga     600
   agacugucaa cggccaccug gacuccuaug agaaagugac ccagcugccu ggacccauuc     660
   gggaguuccu ggaucaguau gaugcuccac uuuaaggagc aaaaggguca gaggggggcc       720
   ugggucgguc ggucgccucu ccuccgaggc acauggcaca agcacaaaaa uccagcccca     780
   acggucggua gcucccagug agccaggggc agauuggcuu cuuccucagg cccuccacuc     840
   ccgcagagua gagcuggcag gaccuggaau ucgucugagg ggagggggag cugccaccug      900
   cuuucccccc ucccccagcu ccagcuucuu ucaaguggag ccagccggcc uggccuggug     960
   ggacaauacc uuugacaagc ggacucuccc cuccccuucc uccacacccc cucugcuucc    1020
   caagggaggu ggggacaccu ccaaguguug aacuuagaac ugcaagggga aucuucaaac    1080
   uuucccgcug gaacuuguuu gcgcuuugau uugguuugau caagagcagg caccuggggg    1140
   aaggauggaa gagaaaaggg ugugugaagg guuuuuaugc uggccaaaga aauaaccacu    1200
   cccacugccc aaccuaggug aggaguggug gcuccuggcu cuggggagag uggcaagggg    1260
   ugaccugaag agagcuauac uggugccagg cuccucucca uggggcagcu aaugaaaccu    1320
   cgcagauccc uugcacccca gaacccuccc cguugugaag aggcaguagc auuuagaagg    1380
   gagacagaug aggcugguga gcuggccgcc uuuuccaaca ccgaagggag gcagaucaac    1440
   agaugagcca ucuuggagcc cagguuuccc cuggagcaga uggaggguuc ugcuuugucu    1500
   cuccuaugug gggcuaggag acucgccuua aaugcccucu gucccaggga uggggauugg    1560
   cacacaagga gccaaacaca gccaauaggc agagaguuga gggauucacc cagguggcua    1620
   caggccaggg gaaguggcug caggggagag acccagucac uccaggagac uccugaguua    1680
   acacugggaa gacauuggcc aguccuaguc aucucucggu caguaggucc gagagcuucc    1740
   aggcccugca cagcccuccu uucucaccug gggggaggca ggaggugaug gagaagccuu    1800
   cccaugccgc ucacaggggc cucacgggaa ugcagcagcc augcaauuac cuggaacugg    1860
   uccuguguug gggagaaaca aguuuucuga agucagguau ggggcugggu ggggcagcug    1920
   uguguugggg uggcuuuuuu cucucuguuu ugaauaaugu uuacaauuug ccucaaucac    1980
   uuuuauaaaa auccaccucc agcccgcccc ucuccccacu caggccuucg aggcugucug    2040
   aagaugcuug aaaaacucaa ccaaauccca guucaacuca gacuuugcac auauauuuau    2100
   auuuauacuc agaaaagaaa cauuucagua auuuauaaua aaagagcacu auuuuuuaau    2160
   gaaaaaaaaa aaaaaaaaaa aaaaaaa                                        2187
<210> 38
   <211> 2046
   <212> RNA
   <213> Homo sapiens
<400> 38
   aaggaaucuu caguacagaa acaagaccca aguguaaguu uaacuggcag gaggaaccaa      60
   ccaaggacag uuaaggagaa aacccaaccc uuagaagaac ucaccaguuu ccaagaggaa     120
   acugccaaaa gaauaucuuc caaaucucca caaccggaag agaaggaaac cuuagcaggu     180
   uuaaagaggc agcucagaau acaacuaauc aacgauggug uaaaagaaga gcccacagca     240
   cagagaaagc aaccauccag ggaaaccagg aacacacuca aagagccugu aggugacagu     300
   auaaauguug aagagguuaa gaagucuaca aagcagaaaa uugauccagu agcaagugug     360
   ccugucagca agaggccacg gaggguaccc aaggaaaagg cacaggcccu agaauuggcu 420
   ggucucaaag gaccaaucca aacccuaggc cacacugaug aaucagcaag ugauaaagga 480
   cccacacaga ugcccuguaa uucucuacaa ccagagcaag uugacagcuu ccaaagcuca 540
   ccaaggcgac ccaggacaag acgugggaaa guagaggcag augaagagcc uucagcagua 600
   agaaagacag uaucaacauc aaggcaaacu augcgauccc gcaagguccc ugaaauuggu 660
   aacaauggua cccaaguuuc aaaggccucc auaaagcaga cauuagauac aguagccaaa 720
   guaacuggca gcaggaggca gcuaaggaca cauaaaggau gggguucaac ccucuugaag 780
   uuguuaggug acuccaaaga aauaacccaa auaucagauc acucugagaa acuagcacau 840
   gacaccagua uccuuaagag cacucaacag caaaagccag acucaguaaa accucugaga 900
   acaugcagaa gagugcugag ggccucuaaa gaggucccca aggaaguguu gguggacacc 960
   agagaccaug caacauuaca aagcaaaagc aacccuuugc uguccccgaa gaggaagucu 1020
   gcaagagaug gaagcauugu gagaaccagg gcuuugcgcu cuuuagcacc aaagcaggaa 1080
   gcaacagaug agaagccugu accugagaaa aaaagggcug cuuccagcaa gagguaugua 1140
   ucaccugagc cugugaagau gaaacaccug aaaaucgugu caaacaaacu ugaaucugug 1200
   gaagagcagg uuagcacugu uaugaaaaca gaagaaaugg aagccaaaag agaaaauccu 1260
   gucacuccag aucagaacuc uagguaccga aagaaaacca auguaaaaca gccaaggccc 1320
   aaguuugaug caucugcaga gaaugucggg auaaagaaaa acgagaagac uaugaagacu 1380
   gccucccagg agacagagcu gcagaaucca gaugauggag ccaagaaauc uacaucucgg 1440
   ggccaaguca gugggaaaag aacaugcuug aggucuagag gaacgacuga gaugccccag 1500
   ccuugugaag cagaagagaa aacaagcaaa ccagcugcag aaaucuugau aaagccucag 1560
   gaagagaaag gagucucugg agagucugau guuagguguu ugagguccag aaaaacuaga 1620
   gucgcuuugg acagugaacc uaagccaagg guaacucgug gaaccaagaa agaugcaaaa 1680
   acucugaagg aggaugaaga cauuguaugc accaagaagu uaagaacaag aaguuaagaa 1740
   caagaaguua ccagaaaagu gaaacuaugu agcaaagaca uuuaagaagg aaaaguaaau 1800
   uugacuuagu gauaaguucc agugugguuu ucaccuccag uguaaagaug aacuguaaau 1860
   acuacugcua cugccugagu uuaaggaagg aagcuuugag cuuuccuggu cauacucucu 1920
   ucagacgcca auggagguca ugaggaagau caccagggau cucagcgcaa uuacaguuua 1980
   ggggugagca ggcagaaaug uggcccucug uccuauccaa uaaagcucug aaauucgcug 2040
   ccaaaa 2046
<210> 39
   <211> 423
   <212> RNA
   <213> Homo sapiens
<400> 39
   uuuuuuuuuu uuuuuuuaaa ucaaaaguua ugaugacuuu auuuuaaauc uuaauacacc       60
   aaaaauauuu uucaauguug ugagauaagc acuugaaaau aagaauucca acacugcugu      120
   gauuucgcug ugaggcuuga uagugaauuu ucccucugaa uauggguuua gggccuagga      180
   agcagaaugc cagucauuuu ccaaguagca gugagcuaag cccagcccgg ucaugcucag      240
   acccacacuu aacugaaaua uucacacuag gaggcggcac caccaggcaa caccuugauc      300
   aaccaggaga acaaaagucu gaagugccac caagcauugg ggaaaugaua uuguuuagau      360
   gcuagugagu cagguucuuu caaauguguc cuaacugggu ugcaaacaua guugcauccu       420
   uau                                                                    423
<210> 40
   <211> 434
   <212> RNA
   <213> Homo sapiens
<400> 40
   gucuguaaaa aucuguuuaa uaaauauaca ucuuagaagu accaaaauaa uuaccaacaa       60
   aauacaacau auacaacauu uacaagaagg cgacacagac cuuaguuggg ggcgacuuuu      120
   aagcacaugc cacugaacac cuggcucuua caugggagga cacacugggc ucacuuacua      180
   ggucuauggu gguucaauca aaagcacaau aaauaaaacg ugguccuuuc auuagguucu      240
   ggaaaaucac cucccccccc cccaaaaaaa aucccacaaa caugaaccuu aagagacauu      300
   uucuuugaau uucagugauc uguuuccccg gauuucacaa agacaacagc cgaaucaccc      360
   caguaaaaug ccugggucua ggcgcugugu gguguggugc uaaguauacc cuuucucauu      420
   uuuuuucuuu uucu                                                        434
<210> 41
   <211> 1000
   <212> RNA
   <213> Homo sapiens
<400> 41
   cggccgccag accuucaagg gcuggagugg acgcgcggac cgacucugaa cagacagacg 60
   aaccgcggcc gcaagguucc cagacaggau cucacccaga ggcaggcagc ggacagugcc 120
   uuaguggaac cucgcugucc uccaccgccu ggccccggug caggcgucca guggccgccg 180
   cauccaaagu gaucgcugcc uccccgucuc cgccagcucg ggaccaugaa gcugcugccg 240
   ucggugaugc ugaagcucuu ucuggccgca guguuguccg cguuggugac cggugagagu 300
   cuggagcggc uucggagagg ucuggcggca gcaaccagca acccugaccc ucccacugga 360
   uccacaaacc agcugcuacc cacgggaggu gaucgugcuc agggggucca ggacuuggaa 420
   gggacagauc ugaaccuuuu caaaguugcu uucuccucca agccacaagg ccuggccacc 480
   ccaagcaaag aaaggaaugg gaaaaagaag aagaaaggaa agggguuagg gaagaagaga 540
   gacccaugcc ucaggaaaua caaggacuac ugcauccacg gggagugcag auaccugcag 600
   gaguuccgua cucccucuug caaaugccuc ccugguuacc acggacacag gugucauggg 660
   cugacucuac caguggagaa uccccuauac acauaugacc acacuacagu cuuggcugug 720
   guggcuguag uacugucguc cgucugucuu cuugucaucg ugggacuucu cauguuuagg 780
   uaccacagga gaggagguua ugacuuggaa agugaagaga aagugaaguu gggcguggcu 840
   agcucccacu gaggaggacc ugagcuauag gaaccuucag aggcuacuuc ugagacagug 900
   guucguuaca cguucuacau agaggagaaa uauuucacca gcagccauga aaacgucuuc 960
   auucauuucc aguugcuacc cugacugggc cuccuguaau 1000
<210> 42
   <211> 1653
   <212> RNA
   <213> Homo sapiens
<400> 42
<210> 43
   <211> 485
   <212> RNA
   <213> Homo sapiens
<400> 43
   uuuuuuuuuu uuuuuucaug uuuaauaguu uauuucuuau uuuguugcuu auaucuucaa      60
   uaaaucauuu ugcagguuuu guuacagauu uuugauaagc caacucaagu acugauuuuu     120
   cauccucucu gaaaguuuua aaccaggaaa ggaaaacguu ccauggaauc caucuuccac     180
   auggugauga gucacaugaa cuccaacauu cugaagccgc uugacauaca ugaguccauc     240
   aucucuuagg acaucauacu ggcaagugau gauauagguc uuagguaaau gaugcaauau     300
   auugucauug gccaacagag ggcaugccuu cacaucuaug aacccuggau acuuuugagc     360
   cagcucagaa cuaccaggag ugggauuuuu guaaacggga cuuuucuugu aucucucagg     420
   gagcaaggaa cuccaauuca caaacuguaa caaguggcua gauuccaugg guacauguug     480
   guuga                                                                 485
<210> 44
   <211> 3449
   <212> RNA
   <213> Homo sapiens
<400> 44
   gagcacccgg gacccuggga ccacaacgca cuugcucccu cucgaccgcg cuccugaccc      60
   gcagcccucg ccaacccuac ggauccuaac caccgccagc cuaggugggc gucaggauga     120
   aggcagcccg cuucgugaug cgcagcgcca gcucgcugag cagcgccagc cuggucccca     180
   gggaggucga gcuguucucc cgcuacagcc cguccccgcu guccaugaag cagcugcugg     240
   acuuugguuc agaaaaugcc ugugaaagaa cguccuuugc uuuucugcgg caagagcugc     300
   ccguccgccu ggccaauauc cugaaggaga uugacauccu gccugaccgc uuagugaaca     360
   cuccuucggu gcagcuggug aagagcuggu auauccagag ccugauggau uugguggagu 420
   uccaugagaa gagcccagaa gaccagaaag cccugucaga guuuguagac acgcugguca 480
   aaguucgaaa cagacaucau aauguggucc cuacaauggc ucaaggcauc cuggaguaua 540
   aagacaccug cacaguggac cccguuacca aucaaaaucu ucaguauuuu uuagaccggu 600
   uuuacaugaa ccgcauuucu acucggaugc ucaugaauca gcacauccuc auauucagug 660
   acucaaagac gggaaaccca agccacauug gaaguaucga cccaaacugu gaugugguag 720
   caguagucca agaugccuuu gagugugcaa agaugcucug cgaccaguau uaucuaacau 780
   cgccagaauu aaaccucaca caagucaaug gaaaauuucc aggccaacca auccacauug 840
   uguacguucc uucacaccuu caccacaugc ucuucgaacu cuucaagaau gccaugaggg 900
   ccacggucga gcaucaagaa aaccguccuu ccuugacccc aguagaggcc acugucgucu 960
   ugggaaaaga agaccuuaca aucaagauuu cugaccgagg aggcgguguu ccucugagga 1020
   uuacugaccg ccucuuuagu uacacguacu ccacugcucc aacaccugug auggacaauu 1080
   cccggaaugc cccuuuggcu gguuuugguu auggcuugcc aauuucucgu cucuacgcca 1140
   aguauuuuca aggagaucug aaucucuacu cuaugucagg uuaugggaca gacgcuauca 1200
   ucuacuuaaa ggcuuuaucu ucugagucug uagaaaagcu cccagucuuu aacaagucag 1260
   ccuucaaaca uuaucagaug agcuccgaag cugaugacug guguauccca agcagggaac 1320
   cgaagaaccu ggcgaaggag aagcuggcag ugugaagcgg augacgccug acauuuuacg 1380
   ggaucaaagu gggucugugg cauugcugcu ucgugaaugu guguggacuc uaguuuccgc 1440
   aaaacaacgc aacacaaaac caagcaagca aaacacaaac acgaguacaa accuugaccu 1500
   gaugagggac agagcuuggu uggaugaccc gggagaaguc agggcagggc uccaggggau 1560
   aacagguguc cugcuucucc uuuggcaaug caaaaugacu ccugacuguu ccaaauacug 1620
   aaaagaaguc ugccucugag uuacagcucu uucucaacaa guacagaguu ugaggcuugc 1680
   aguugcaaca gcuggauguu uggugguucu ugcugccagc caaauaaauu gguguuuagu 1740
   gaacauuuuc aguguuuccc cgccaugcaa agcuuggcgc cuugggagaa auguguguaa 1800
   auguacauug uauagguauu agugugcucu agaaaggaca ggauggaagg aaucaaagca 1860
   cuuuaucgag cuuguggcug agcauugcag ccuaugugca aacccagagg aaaaguaucu 1920
   cugucaagac agcuccagua ucaugcagcu uuuuauguuu gcacucaaaa agccagugcc 1980
   uucuggcugg ugccgaggcu ugggugaaau guuaaauaug cacugaccuc agaaagucga 2040
   guucaaaagg gagauaaaau ugccaaagug auccaaggau ugugcauguu gggaaaccca 2100
   uaugagagaa aggauucuca uacuuagaac uuuccuauga agaaauggug guaaacuuuc 2160
   ucuaccuaga aguaguggaa auuucaaggu caucuuaaaa aagaugugcg uuguauauuu 2220
   uaacuacauu cucuacacuc uaacauuaac auaucuauuc aaauuugucu aguugccaau 2280
   ugucuucaga gugugaaaau uuaaauccuu cuugaaguau cuuucgugag aguaguaugg 2340
   aaguaaaacg uucucauauc aggaggaugu cauuugugaa gcauggggac aucaugaacu 2400
   agugaugugc gugaggcuug ggaggcugaa ggguaaggau cagcgggagg ccauccaugu 2460
   aggagagaga auuaaaacga ggagcgaggg aagcaaugga gagagggaag caagaaagga 2520
   accagaaggc uggcaucauc cuauuuccca caggcuaacc caagggaugc ucugugccuu 2580
   uccuggggag ggaagggggu gaacugguag auuugaaagc aguauggcuu cuucuguggg 2640
   ucucccucuu acuagacaag gugaaaugau aauucguguc aaauuaaugu gaaauuuuuu 2700
   uccugcauug uaauauuaug aggccugagu cgcaguugag uuugaaauuu guauuuaauu 2760
   ucacagugac cuagagcuaa ggugcucccg guuguggcaa uaggagccac aaguauuuuc 2820
   uuucuuucuu ucguucuuuc uuucuuucuu ucuuucuuuc uuucuuucuu ucuuucuuuc 2880
   cuuccuuccu uccuuccuuc cuuccuuccu uccuuccuuc cuucuuccuu uccuuuucuu 2940
   uucucuucuc uucuuuucuu uuuucuguuu cuuuuucuuu uuuugcauug uagauguugu 3000
   ccuuaaaaga ucagggcagu gacuuucaca gcaggacuuu gacucccaca uugguugauc 3060
   acacaaaacu gucagcauuu ggguaaucug auguauaguu guuuuguugc ugauguuucc 3120
   auugaaauuu cagcucugag uuugugcaca ugaauacuua cuuguguuua ccaaaggucu 3180
   aaggcauuug guuacuuaac ccaaauaucc ugaacugugc guaaaguaau agagaaaagc 3240
   uuuagggucu caauaguguc accuguguaa aucaaaucaa aauagccuuc ccuauuauuu 3300
   augaacccau gggagacuuu aaacucuugu agauagaugc uaaaugccca ggcccacuua 3360
   acuuauuaau gugugaauua cauuuauguu uuuaguuuau augcaaagaa uugugauaau 3420
   uuuauaauaa auauuuuuau uauaauagu 3449
<210> 45
   <211> 226
   <212> RNA
   <213> Homo sapiens
<400> 45
   uuuucuuuuu uuuuuuucuu cucuuuuuuc ugccaccaac agcacugugc aguuuauuaa 60
   ccauucaugu acaguagcca ucuggggaga uugggacaga auugggaucg caaaguggau 120
   agauauucag caucuaaugg guuggcagaa gccgccauau acucuucaca aauaucuucc 180
   acagucaaua cagaacuagc cauuauccca gcacaccgau uugugc 226
<210> 46
   <211> 952
   <212> RNA
   <213> Homo sapiens
<400> 46
   agccaggagc cucgccccgc agcugcacag agagcaaggg uauaggcacu aacuuguuug    60
   cagagacccc aucaccuucg ggagcucagg ugcgcaccuu gcaaacucca cuuucugcau   120
   cugccacuga gcccgcggga gccucggaaa gagccauggc caacgcgggg cugcagcugc   180
   uggguuucau ccuggcuucu cugggaugga ucggcuccau cgucagcacu gcccugcccc   240
   aguggaagau uuacuccuau gcuggggaca acaucgugac cgcucaggcc aucuacgagg   300
   gacuguggau guccugcguu ucgcaaagca ccgggcagau acagugcaaa gucuucgacu   360
   ccuugcugaa ucugaacagu acuuugcagg caacccgagc cuugauggua auuggcaucc   420
   ugcuggggcu gaucgcaauc uuugugucca ccauuggcau gaagugcaug aggugccugg   480
   aagaugauga ggugcagaag auguggaugg cugucauugg gggcauaaua uuuuuaauuu   540
   caggucuggc gacauuagug gccacagcau gguauggaaa cagaauuguu caagaauucu   600
   augaccccuu gacccccauc aaugccaggu augaauuugg ccaggcccuc uuuacuggcu   660
   gggccgcugc cucccucugc cuucugggag guguccuacu uuccugcucc uguccccgga   720
   aaacaaccuc uuacccaaca ccacggccuu aucccaagcc aacaccuucu agugggaaag   780
   acuaugugug acagaggcaa aggaagagau cuuccuggag caaauacaaa auggacauug   840
   aaccuaggau ugacauuaac gccuuagacu guugaugaug guuaucggaa cugugguaga   900
   acagaaggaa gcauauuuuu auacaucccc auggcuaugc aggccuuggc ug           952
<210> 47
   <211> 1777
   <212> RNA
   <213> Homo sapiens
<400> 47
   cggcacgagc ggccacugac cgagaagugc ccccgccugg agucagccug ggggcaggcc      60
   aggguccacc agaccccggg augaccgcag ccagucgggc caaccccuac agcaucguau     120
   caucagagga ggacgggcug caccugguua ccaugucagg cgccaacggu uuuggcaaug     180
   gcaaggugca uacacggcgc cggugccgca accgcuucgu caagaagaac ggucagugca     240
   acauugaauu cgccaacaug gacgagaagu cacaacgcua ccuggcugac auguuuacca     300
   cgugugugga cauccgcugg cgcuacaugc ugcucaucuu cucucuggcc uuucuugccu     360
   ccugguuguu guuuggcauc aucuucuggg ucauugcugu cgcccacggg gaccuggagc     420
   cagccgaggg ccguggccgu acacccugug ugcugcaggu ccacggcuuc auggcagccu     480
   uucucuucuc cauugagaca cagaccacca uuggcuacgg gcuacgcugu gugacugaag     540
   agugcccggu ggcugucuuc augguggugg cgcaguccau ugugggcugc aucauugacu     600
   ccuucaugaa uggugccauc auggccaaga uggcacggcc caagaagcgc gcacagacuc     660
   ugcuuuucag ccauaaugcc gugguggcuc ugcgugacgg caagcucugc cucauguggc     720
   gcgugggcaa ccugcguaag agucacaucg uggaggccca ugugcgggcc cagcucauca     780
   agcccagggu cacagaggag ggugaguaca ucccacugga ccagauugac aucgaugucg     840
   gcuuugacaa gggccuagac cguaucuucc ugguaucacc caucaccauc uugcacgaga     900
   uugaugaggc cagcccacug uuuggcauua gccgucagga ccuugagaca gacgacuuug     960
   agauuguggu cauccuggag ggcaugguag aggccacagc caugaccaca caggcucgca    1020
   guuccuaccu ggcuaacgag auccuguggg gccaccgcuu ugagccagug cucuucgaag    1080
   agaagaacca guacaagauu gacuauucac acuuccacaa gaccuacgag gugccaucua    1140
   caccccgcug cagcgccaag gaccuggugg agaacaaguu ccuccugccc agcgccaacu    1200
   cuuucugcua ugagaacgag cuggccuucc ugaucagaga ugaggaggac gaggugucua    1260
   ccgaccggga uguccgcacc ccucagcccg agcaugacuu ugacagacug caggccagca    1320
   gcgcugcccu ugugcggccc uacagacggg agucggagau uugaaugccc uuggcuuaga    1380
   ugcagcacca cccugaccac aauaggaccc auguccciug ggggccugcg uuugagcaga    1440
   gcaggccgaa agccucgggu cacagacuca guagcaucuu agucuuuuuc auguuuuuuc    1500
   gcaguagcuu gggaaaguug gcgggagcgu ggauggccca aaugacuggc ucacggccuc    1560
   ggaggcugau guauacccau gggcaaggag gugacuucuu gggguagggu ugcucaggag    1620
   uuagggacuc ugcuggaggc cuuaggugca ggucccaacc ccggugggag gaggcugugu    1680
   auguacacuu cauugguuuu uaacuugggc aagacuguuu acaaaccaaa acaaacaaac    1740
   aaacaaucca aaaaaaaaaa aaaaaaaaaa aaaaaaa                             1777
<210> 48
   <211> 175
   <212> RNA
   <213> Homo sapiens
<400> 48
   uuuuuuuuuu uuuuguuccu uuuuuggaau ucccaaagcu gguuuuaauu ucaaaaaauu      60
   augaggucuc uucccacacu ggggauaaug ggaugggaua gcccaaacua uuaucccagu     120
   ucaaccccag ccugguccaa acaccauuac ugucacuggg cccugucauu ucacc          175
<210> 49
   <211> 1629
   <212> RNA
   <213> Homo sapiens
<400> 49
   uccucguccu cgucuguucc aucuucucca aauagcucua acugugaugc caacggcaau      60
   cccaagaacg cugauaucuc uagcaucgau gguguucuga agagugaccg cacagauugu     120
   ccugugaaaa caggcaaaac cagugcuccu ggcaugacua agagucacag uggaaugaca     180
   aaauuuagug gcaugguucu acuguguaaa gucugugggg auguggcauc aggauuccac     240
   uauggaguuc augcuuguga aggcuguaag gguuucuuuc ggaggagcau ucagcaaaac 300
   auccaguaua agaagugccu gaagaaugag aacuguucca ucaugaggau gaacaggaac 360
   cggugccagc agugccgcuu uaagaagugu cugucugugg ggaugucacg agaugcuguu 420
   cgauuuggcc gaauuccuaa gcgugaaaaa cagagaaugc uaauugaaau gcaaagugca 480
   augaagacca ugaugaacac ccaguucagu ggccaccugc agaaugacac cuuagcagaa 540
   cagcaugauc agucagcacu accagcucag gaacagcugc ggcccaaguc ccagcuggag 600
   caagaaaaca ucaaaaacac uccuucugau uuugcaaagg aggaagugau ugguauggug 660
   accagagccc acaaggauac cuuucuguau aaucaggaac aucgagaaaa cucaucugag 720
   agcaugccac cucagagagg agaacggauu cccaggaaca uggagcaaua uaauuuaaau 780
   caagaccauc guggcagugg gauucacaac cacuuccccu guagugagag gcagcaacau 840
   cucaguggac aguacaaagg gaggaacaua augcauuacc caaacggcca ugccguuugu 900
   auugcaaaug gacacuguau gaacuucucc agugcuuaua cucaaagagu cugugauaga 960
   auuccaguag guggauguuc ucagacugag aacagaaaua guuaccugug caacacugga 1020
   gggaggaugc aucuggugug uccuaugagc aagucuccau auguggaccc ucagaagucu 1080
   ggacaugaaa ucugggaaga auuuucaaug aguuuuaccc cagcaguaaa agagguggug 1140
   gaauuugcaa agaggauucc uggcuuccga gaucugucuc agcaugauca ggucaaucug 1200
   uuaaaagcug ggacuuuuga gguuuuaaug guacgauuug cuucauuauu ugaugcaaag 1260
   gaacggaccg ucaccuuucu aagugguaag aaguacagug uggaugaccu gcacucaaug 1320
   ggagcagggg aucugcucag cucuauguuu gaguucagug agaagcugaa ugcccuccag 1380
   cucagugaug aggaaaugag cuuguucaca gcaguuguuc ugguaucugc agaucgaucu 1440
   ggaauugaaa augucaacuc aguggaggcu uugcaggaaa cacucauccg ugcacuaagg 1500
   accuuaauaa ugaaaaacca uccaaaugag gccuccauuu uuacaaaauu acuucuaaag 1560
   uugccagauc uucgaucuuu aaacaacaug cacucugagg aacucuuggc cuuuaaaguu 1620
   cauccuuaa 1629
<210> 50
   <211> 831
   <212> RNA
   <213> Homo sapiens
<400> 50
   auggcguuac uggaucugug cggugccgcu cgggggcagc ggcccgagug ggcugcccug 60
   gaugcgggaa gcgggggucg cucggacccg ggacacuaca guuucuccgc gcaagcuccg 120
   gagcucgcac uuccccgggg aaugcagccc accgcauucc ugagguccuu ugguggugac 180
   caggaaagga auguucaaau ugagauggcc cacggcacaa ccacacucgc cuucaaguuc 240
   cagcauggcg ucaucguggc uguggacucc agggccacug cagggaguua cauuagcucc 300
   uuaaggauga acaaagugau cgagauuaac ccuuaccugc uuggcaccau gucugguugu 360
   gcagccgacu gccaguacug ggagaggcug uuggccaagg agugcagguu guauuaucuu 420
   cggaaugggg aacgcaucuc cgugucugca gcauccaagc ugcuuuccaa caugaugcug 480
   caguaccggg ggaugggccu cuccaugggc agcaugaucu guggcuggga caagaaggga 540
   ccaggacuuu acuacguaga ugacaauggg acucggcucu cgggacagau guuuuccacu 600
   ggcagcggga acaccuaugc cuacggggug auggacagug guuaccggca ggaccucagu 660
   ccugaagagg ccuacgaccu uggccgcaga gcuauugcuu augcuaccca cagagacaac 720
   uauucuggag gagucgucaa cauguaccac augaaggaag acgguugggu gaaaguggag 780
   aguuccgaug ucagugaccu gcuguacaag uaccgagagg ccgcucugug a 831
<210> 51
   <211> 3601
   <212> RNA
   <213> Homo sapiens
<400> 51
   auuccaggag uuccagcugc uggagaggac uguguagaag guaaccuacc ccaucucucu      60
   uacuucgucu cuagauggga gcagacaagu acauauagcc ugcuuggagc gaggacuuug     120
   aaaggcugag acuugcgucc acucugaggg caacuagucc agacucugac aggucagcau     180
   uuccugacug ggugcacuga augccaagca ccagaggucu gucaccuucc gacauuggac     240
   caagagaguc ccagagaccc ucaaagacac aggaacagag guguccuuug ggugagagac     300
   cugugcccug ccaagccucu cagccccuag aaaggcggca gggcugagua gcugagugug     360
   ugcaacuugg gagcagccug auuucagugc uugucaccug ugagggcaaa ggcagcaugc     420
   uuaaugccau caguccuacu cuucucaccc guggaacaga cgcauaacug accuuuuuuc     480
   gugaccacua uuagggugca uuuaaaaauc aaucucucuc uuuucugcuc cucuuucucc     540
   accuucccuc augugugugu cugugugugu guguuucugu gugugugugc gugcaccaua     600
   cagguauaug ugccaugaca ugugugcagg ucaguagaca gccuugguug ucagucccug     660
   ccuucuacuu uguucaaggc agggucuauu cuuuauuauu gacugcuauc cagguacacc     720
   aagacaguug gccugugagg uuccagggag ucuccugucu cuggccccca ucuuauagca     780
   ggaguuguga gauuucagaa augugcaccc acaucuagcu uuauuuggca ucuuggcacc     840
   caaacuuggg uucucaugcu uuccaagcaa ggacuucacc cacugaacca ucucaccagc     900
   uccacuaugg cgguuuucug aaacugaagg gaguggggag aaggcgcuga gugugaacgg     960
   gucuggaagg cggguauaac cuuuaaggcu ggcugguucu gagagaggaa agccggcuug    1020
   ugucccauuc aggugccagg ugcagcauca aauguggcuc cacccaacgg ucaguaacuc    1080
   cgcagacagc accgugguua acugccucac agagggggcc cagggaccua guuccuuaaa 1140
   gcccacuuag uuuugagaga cgacauggag gggcaagccc agcccugucc agcugcauuc 1200
   acacgaggcc cuucuccucc gaggacccua ccuuuguguu uaguggcuga ggcugugugg 1260
   cucugcuuga agcucuggcu aucaggaagg accuggccca ccggcuggca ggacaaacug 1320
   gcccagugaa ggcacugucc ggucuccugg uggaucacaa ggaaaggggc guggcauguc 1380
   cagauuugaa cccuggaugc cucuucccag gguaggaagu cuaaggguca acaugaauag 1440
   gugagggugg ugguggaaag aaucugcaug caaaaucagg cacucauguc ugacaucguc 1500
   aaacacuaua uuuauguaga uuuauauggg ggugaggcuu ggggagcacu uguauuuuug 1560
   ugugcaauug caagagcuuc cuccacauug caguguuaaa uaucauaugg ugcuggacca 1620
   accaucucug gaacgagggu gaggguuugg uacccgugac uaugguucuc uguguaguag 1680
   aaacggcuuc uugggaagag agaggaagug cuuugaaaug cauccucucu uguuucauuc 1740
   ugcaucaugg ccagguuucu cugcacaaag augcaauaga cauucaggaa aauaagcgca 1800
   uugcaagaug ucaaaaguca ugaaaaauga aaagcaaggu cacugcagcu gggguguagu 1860
   guggcuguag aagcacguuu augauguaca gguuccggga gagggaggaa gagagaggga 1920
   gggaaggagg aauggaggaa gagagggaga gggagcaaga gaggggggag ggagggaggg 1980
   gagaagggag agagagagag aagaaagaga gggagggaag aaggaaugga ggaagagaga 2040
   gaaagagggg aagaaagaga gggagggaag gaggggugga ggaagagaga ggugugaggg 2100
   auggagggac agagagggag ggaaggagaa auggaggaag agagaaacag agagauggag 2160
   gaagagagaa aagucaccaa uuauuuuucu ccccagucgc ccccugcccu cacaccaagg 2220
   guacccccac uuuucuuuca cucaugcaca cacacuacac ccaauguuua gguaacacau 2280
   guagggcagg cagagccuuu gugaaugugc uugguaguuu ucugccuuac uucuaaauaa 2340
   ccaaucacac ggaugccaca ggguuccuuu agacugucca gggcuccggg gcucuuagac 2400
   uguucccaau guggguguca caugcaaaca guaagcaccu ucuuguuugu ggugcccagg 2460
   augcugcagg uacuagaaau uccugcacag accuuugcua cuucuacaca gacuuacccu 2520
   uuaaaacauu uaauuauaug uuuuuuucag cauaaagauc uauuuugauu auuuuuaguu 2580
   gcguguaugg gugugugccu gugugugauu guguaauugu cuguauggga guguaagugc 2640
   cuacagaggc aucggauccc cuggagcugc cguuacaagu aagggacugg gacucagacc 2700
   ugagugguuu ggaugagcag ugaaugagcg uggugagcca ucccaccaug cccuauaaac 2760
   acuuucaaga ggacuuugcu ggguaugguc aaguuguccc ccugcgacag ccaccccaac 2820
   uugccauccu cauuuacugu acuggaagca gcaaccaccu cugcaauuuu gcacaagaac 2880
   cugagagucu gaaagacuuu uguuggcacu ggccuguguu ccacuggggu ggacacauua 2940
   aucuaaaagc uaagaagcuc acuucagcuu ugccaggcaa ccagcacguu ugagcacaac 3000
   gguguagcca ggaagggugu gugggaauuu ugaugcuagu uuuucuuuaa uuagguugag 3060
   agugaaauca uggucuguau gcauccaaca acuuaaucca aaaaguuggu ugcacggcuc 3120
   cuggaagguu uaccggcaag gaaccugucc cauuuuguaa guagguggga cuggagaguu 3180
   gccaagaagc cccccucccu gcccccuucc uccuggcaga uucugcuuaa aaugagugug 3240
   guucacugca gaauaguguc accugucuuc agaugucuaa agacauaaga aaggccuagc 3300
   cagcuaaguc agaaaagggg acuuuccggu gggaaugcau uuuccagucu cuaggagaag 3360
   ucuuuaucca aauaaaauua gcauaguggu ggagucacug ccagggcgcu ggugcggaag 3420
   cacagcccag agccgaggac guggccaucc uccuuccucu gaugcaaagu guucucuauc 3480
   cccaguuacc augagcaaua ucaguagcau cgugaaccgg gcccgugaug ccuuuaacuc 3540
   aggcaagacu cgaccgcugc aguuccgggu ugagcagcug gaggcguugc agcgcaugau 3600
   c 3601
<210> 52
   <211> 329
   <212> RNA
   <213> Homo sapiens
<400> 52
   uuuuuuuuuc caaaaaauuu uauuggggga aacuacaaaa cauuuacagu acaauguuua 60
   cagucacaau uuguagugaa cugauucccc aaaauauauu acaacucaag uugacuuaau 120
   cuuguuacau ucaaaaaccu acuucuguca aaguagucca gagugcacac gcggugcucc 180
   aacuguaccu acauacaaac uaaacaacug cucauuuauc ugccauccag gaaagccgga 240
   gacauuccug ccucuuuaca uugaaaaaua auaguacaag uuuuuggacu gucauugaac 300
   aaggcauauu cauguaccac caacauuuc 329
<210> 53
   <211> 3671
   <212> RNA
   <213> Homo sapiens
<400> 53
   ggugauccuc caggaccuaa gguuucggua uuaucuucgg ggccucuuca cccggaggug      60
   agucugcuga gcccgaccuc agguguucac ucgucacgcg agggccuggc cacggcgggg     120
   aagcacuggg agagagcggg aggccuugac cgcagcgugu aggaucuggg gucccggcgc     180
   ucugcucccg ggugcagagu gguuggagca ugcgaggccc ucugcagcgu gcucuguucc     240
   uaccaucacc ccacccccac cccaguccuu ccugcacguu ucuggagagg caaaggcccc      300
   ugcugagccu ucacugggua ccccuaucuc gacuccacuu gucugcuugg gaucuccaaa     360
   gggggauaca cccucguaaa cuagagcagg aggcuggggg guggggguag ggguaggguc    420
   ugaccaguua gauugaggcu ggacccuccc cagagugagg agcgggaagc ucuuugucuu 480
   auccaggauu uugcauauca augcugaggu ucuggugauu aaguaggguc ccuggcuagc 540
   ggggguaacc ugaacaaccu uccuccgcug gccgcuagcc aauagacacc uggcuuccgc 600
   ggaugagccc uuugaguuug uggagguguu ccucaggucc ccagggcccu gccccguugc 660
   cugcagaugu guggacagac aaaacauugg acucguuuuc caaccacuuc accccuuccu 720
   ucgcuagucc uccgguggac gaugggaugc cucauugacu uguuuguggc ugugcaugcc 780
   agagcuuccu agcagacagc ucagggcucc aucguuccua aaucccacug uguaacgaaa 840
   aaaaaaaaaa caaaaacaac aacaacaaca acaacaaaaa caaaaccaga aaugaggaca 900
   ggcccagcug ggcuuggcac ugaacuuggc caccuggagc uuuggcuacc cacuacaaga 960
   ugucagcaag ucucaggaua gaagaggccu aaggccaguc cuggaagaua ccaaaacuuc 1020
   accuccucua uaccccguuc cucuaggaga acuguaagcc accuuugucu cugggacgcc 1080
   ccucucccac ucaacagaug gcaccaguca ucuuccuccu caagaggcca gugguauuca 1140
   aaauaggaua uugaaaccaa gcaagcuggu ccucuccucu acccuaugcc aaagacuucu 1200
   gcccaaaggg cccagaaaag ucccauagcc aucuggcugg cuagugugcu gacugcuaca 1260
   ggugucagug uccccagcga gcagguagug gaguggaggu gucuucuugu ggaaaugggu 1320
   ccugagccuc uaccuuguac agaaaggaau uagugaagcc cagaggccuc cagcccgugu 1380
   cucugccaca agagagaggg ggugggggug gggcuguauu ugguucccag ggcucaggga 1440
   agguuuucgg uucaugcaug cucauuuauc ugaccacugu cuuaacccca ggacaaccac 1500
   uuaacugucc acuuccauau cagaguucuu gguuccccuu uacuucgucu auggagcccc 1560
   ugaguuuggg aagggguauu aacugaaagg uaccauuaga ugaacuugga gaaagauuug 1620
   uaggugccac gggagauuuc agguaaaagc ucuuuuaaua auuggcuaca auagcagcag 1680
   gaggaggcca ggaauucuga gguagagauu ugauuagcag cacguggagc aaaggagacu 1740
   ucugacuccu acagguuuca gaaagugggg agaggcucuc agaugaaugg cuuggacugu 1800
   gagguaagug uuaguguagc acgagacuag cuuaaggcug uacaaauugu ccgucugauu 1860
   ggugcauugg gccagguaga cacuagauaa gcaauugggc cuuaacucga guugucuuaa 1920
   uggcuacccc cagggcagca gggagucaau acugcucucc ugucuggggc cugaaugcug 1980
   aaaccaucua caaaggggac auaaggcaua uuggggaggu guagaguggg ccaggcccag 2040
   ggucugcugg uaucuaugcc aggauccuga gugguguggg cguagccugu cuuuuaacca 2100
   acugccucuc ucacaguagg cuucuuuggc uugcgaaccu ucagcagaug ucugccacuu 2160
   ccaacaugag agucuuccug ccugugcugu uggcagcccu ucugggcaug gagcaaggua 2220
   uggagcucug agauaacccu gcagccuggu ccuccuccug aucucucauu cuuucuccug 2280
   aguagaugcc cagggcuccu ccugagccag cccuccuagg aacgucuggc cuucccaccu 2340
   ccuacucucu agccaacuga ccuaguuccc cugaugcugu gcuggcccag cuacaccuuu 2400
   gucaccucgu guugacuuua gccacuccag uaccaaaguc ugaagucagu ugucauuggc 2460
   uggcuuuuuu cccugaccca gggcugagca ugcuguugcu ucuugucagu ggguugggga 2520
   ucaagggccc cacuggaaag ucaccuuacc cuggaaggcu uccccaggca auaggcaggu 2580
   gucaccuaca gcauuguccc uuuugcaguu cauucccuga ugugcuucuc auguaccgau 2640
   cagaagaaca auauaaacug ccuguggcca guuucaugcc aggagaaaga ccauuacugu 2700
   aucacguuau cugccgcugc gggcuuuggu gaguagcugc cuguuucccu agccagggcc 2760
   agggaccugc ggggcuuucc ccauuuccug cuacccuguc ugucuguccc cucauccuca 2820
   cuuuccaugc agggaauguc aaccuuggcu acacccugaa caagggcugc uccccgaucu 2880
   gccccaguga aaaugucaau cucaaucucg guguggcguc cgugaacagc uacugcugcc 2940
   aaagcuccuu cugcaacuuc agcgcagcug gccucggacu ucgugccagu aucccacuac 3000
   ugggccuugg acuccugcuu agcuuguugg cucugcugca gcugagcccc ugaccauccc 3060
   ccauguguuc uccauauccc cccagcucag gaaaagccca gcuccuuuua ggucccaggg 3120
   gacccuagga accuucagcu ccuccugggu gugucuaguu cccccuccac acucucucaa 3180
   cgucagggcu aaguaccaaa cucacccaua ccugcucugu ugaaguggua cuggcuaccc 3240
   uugcuuccag agccaauccu auaaccuccc uuggggaacc agcgaagggg ugaagaucuc 3300
   cuuggagucu caagaguacc agagucagcg ccgaaucuug uggacacacu gacaaggaug 3360
   ucuaauccaa auagauguau aucugugugc ucaguguauc ccugugugaa ugaagccacu 3420
   uggauucugg ggugggcaaa gaagaccuga aaagauucua cagcagaagg ccugugucgc 3480
   caccaaaacc ccucccccug guaucauugu acccaccuug uacucuguuc aggaggcugc 3540
   ccauggagga cugccacccc uccagaugaa ggcucccacu acccgaugca guugaguccc 3600
   auccugcccu cucugcccac acuggcuucc ugcugcuauu cuagugccuc aaauaaaccg 3660
   uucacacccu u 3671
<210> 54
   <211> 951
   <212> RNA
   <213> Homo sapiens
<400> 54
   cuuccucccu cuacagaaga agagcaagaa gaugaggaag aaauugaugu ggugucugug       60
   gagaagaggc aaaccccugc caagaggucg gagucgggcu caucuccauu ccgaggccac      120
   agcaagccuc cgcacagccc acugguccuc aagaggugcc acgucuccac ucaccagcac      180
   aacuacgccg caccccccuc cacaaggaag gacuauccag cugccaagag ggccaaguug       240
   gacaguggca ggguccugaa gcagaucagc aacaaccgca agugcuccag ccccaggucc      300
   ucagacacgg aggaaaacga caagaggcgg acacacaacg ucuuggaacg ucagaggagg      360
   aacgagcuga agcgcagcuu uuuugcccug cgugaccaga ucccugaauu ggaaaacaac      420
   gaaaaggccc ccaagguagu gauccucaaa aaagccaccg ccuacauccu guccauucaa      480
   gcagacgagc acaagcucac cucugaaaag gacuuauuga ggaaacgacg agaacaguug      540
   aaacacaaac ucgaacagcu ucgaaacucu ggugcauaaa cugaccuaac ucgaggagga      600
   gcuggaaucu cucgugagag cuaaggagaa cgguuccuuc ugacagaacu gaugcgcugg      660
   aauuaaaaug caugccaaag ccuaaccuca caaccuuggc uggggcuuug ggacuguaag      720
   cuucagccau aauuuuaacu gcucaacuaa auaguauaaa agaacuuuuu uuuaugcuuc      780
   ccacucuuuu uucuuuuucc uuuuaacaga uuuguauuua auuguuuuuu uaaaaaaucg      840
   uuaaaaucua uccaauuuuc cauguaaaua gggccuugaa auguaaacaa cuuuaauaaa      900
   acguuuauaa caguuacaaa agauuuuaag acauguacca uaauuuuuuu u               951
<210> 55
   <211> 1822
   <212> RNA
   <213> Homo sapiens
<400> 55
   gugggacggg ccccccucga ggucgaccca cgcguccggg aguacccccg accuuggcug 60
   cgugcugacu cgcuuccuuc ugccugccca ggcuugcacu ccccggggau cugccucugc 120
   aucucuugcc uucgcuguug uuucccucuc uguccagcuc cccucccgcu cucgcccugg 180
   agaauggcuc agaaggagaa cgccuacccg uggcccuacg gcucaaagac gucucagucu 240
   ggccugaaca cguuguccca gagaguccua cggaaggagc cugccacgac aucugcgcuu 300
   gcucucguga acugguccaa cagccagucc acagcugccc cuggccagaa guuggcugag 360
   aacaagaguc agggcuccac ugccucgcaa ggaucccaga acaagcagcc uuucacuauu 420
   gacaacuuug agauugggcg uccuuugggc aaaggcaaau uuggaaacgu guacuuggcu 480
   cgggagaaga agagccguuu caucguggca cucaagaucc ucuucaaguc ucagauugag 540
   aaggaggggg uagagcacca gcuucgccga gagaucgaaa uccaggcgca ccugaaacau 600
   cccaacaucc uucaacucua caacuacuuc uacgaccagc agaggaucua cuuaauccug 660
   gaauacgccc cucgcgggga acucuacaag gaacugcaga agagucggac cuucgaugag 720
   cagcggacug ccacgaucau ggaggaacug ucagaugccc ugaccuacug ccacaagaag 780
   aagguaauuc acagagacau aaagccggag aaccugcugu uaggucugca gggagaacug 840
   aagauugcag acuuuggcug gucggugcau gccccauccc ugaggaggaa gaccaugugc 900
   ggcacgcugg acuaucugcc cccagagaug auugaggggc gcaugcauaa ugaaauggua 960
   gaucuauggu gcaucggggu gcucugcuau gaacugaugg uggggaaccc ccccuucgag 1020
   agcccuagcc acagugagac guaucgucgg auugucaagg uggaccugaa guuccccucu 1080
   ucugugccuu cgggcgccca ggaccucauc uccaagcugc ucaaacauaa ccccuggcaa 1140
   cggcugcccc uggcggaggu ugcagcucac ccuugggucc gggccaacuc aaggaggguu 1200
   cugccucccu cugcccuuua gccugcuccu ugguuuuuug ucccugucau uuuucagugu      1260
   ucuuuguaug ucuguguaug uguucugaga agggguggga acuggaaacu auuccuagcu      1320
   ccaguucuag gggaucugau cucucuucug accucuacag gcaaaauuag gcaccccugu 1380
   ggugcacaua uaugcacgcc aaacacauga aguuacaaac aaacaacaaa cacacagaua 1440
   gugcuggaga gauggcucgg caguuaaaag cacuggcugc ucuucccagg aaccuagaac 1500
   ucaauucuag cacuacaugg ugcucacggc cacugucugu aacacccagu ccuggggaau 1560
   cuggggccuu cgagccucug caggcacuag gcauggaugu gguauacaug uaugcaggca 1620
   aaacacccau gcacugacuu uuaagaaacc cucuagucug auuccuuuca auuugucaaa 1680
   uguugaaugu uauuuuuaaa auauuauaag ccauuuaaua caauuuuucu uugaaacaug 1740
   guauagccua gucugucuua aauucagaaa aauuaugaag aacaacauuu uauaauaaag 1800
   ucuuaaaugu uucauguuuu ug 1822
<210> 56
   <211> 2663
   <212> RNA
   <213> Homo sapiens
<400> 56
   uugccuagga agggcgcguc gucucucugc ucguccggcu gugacgggga agggucccgc        60
   ugcguuuugg ucacugugag uaccaaguuu gggggauccc cgagggacuc ucgagagcuc       120
   auuuuaggga ugcaggggcu acuccccggu gguagagagc uuucuaguug gcaggagguu       180
   ucguaugugg aggaggccag cuuaggcaga aagcacaugu ucucagagau gaggacaaga       240
   cuaagaccgu cuaaucccug aucuuuaccu uccggcccgc ugaccugggc cuggaugcua       300
   aagcccucug cuuucgucua aacagcgcua aauaguaaac aguauugccu aagauaaaug       360
   cggauuauua cccgauucag ugucgggaaa aggcagcuag gagagagcgg cuggcacgug       420
   guaagcacac gguaaguuuc gguuaaauua aaacaaccau ccguugagca ucucuuagca       480
   agcuccuucc acccuucaaa caaucaguga uagugcgucu guuucacuga uuagggagcu       540
   aaggcuccaa cagcagcaaa ggaacuaauc cgccucugau caacauggcg uuucuuacag       600
   ggcauuccuu auacgcuuuc ccacgugcgu aacaggaauc gggguguccc ggguuuuguu       660
   uuguuguugu uguuuuuguu uuguuuucuu agugaaagag gcaggguggg cuccaggccg     720
   cugaggauua auaaagagau ucuaugagga ggaaauaaca ggcagguggu augaucgagg       780
   caaggcccug aggaaggcuu ggguggggug aguagaacca gagccggaag uccacucagc 840
   agccuggggc acuuaaagcu ucugcugggg caaaugguaa ggcggcguaa ggucacauuc 900
   cuuucauuuc uuccagacuc aggaggagac cacaccuucc ggagaaccag gccugaaccg 960
   agguacuauu uuguagcucu cagaagccag gacucugcaa cacuguuugc ugccugugga 1020
   ucuucuauau ucacaguguc ccaguugcuu cugaucuacc acuguuagau acuucugcca 1080
   cccauccuaa gaguauaguu guucuuggaa aggagucuca gcugcuguca gcaggagucc 1140
   cucauucgac uccugugguu gcccuuucca ccaugccaaa gaauaaaggc aaaggaggca 1200
   aaaacaggcg cagagguaaa aaugaaaaug aaucugagaa aagagaguug guguuuaaag 1260
   aggaugggca ggaguaugcu caggugauca aaaugcuggg aaauggacgg uuggaagcaa 1320
   ugugcuuuga cggugugagg aggcugugcc auauaagagg gaagcugaga aagaagguuu 1380
   ggauaaauac cucggacauu auauugauug gucuacgaga cuaucaagau aacaaagcug 1440
   auguaaucuu aaaguauaau gcagaugaag caagaagucu gaaggccuau ggagaacuuc 1500
   cagaacaugc caaaaucaau gaaacggaca cauuuggucc uggggaugau gaugaaaucc 1560
   aauuugauga uauuggagau gaugaugaag acauugauga caucuagccu gaccuaagca 1620
   ugcuaccuuc caaguucucu gaagauagcu ccacacagug gcaucuugac cuucaucugu 1680
   uaaguaaaac uucauggcau guguaugacu uguuaaugca agcuaaugaa uuuuauuuuu 1740
   ugaaguacua uauuucuuug aaaaccaaag auguugaguu aucaucuuaa gugacauguu 1800
   aacacuuugu gcuuuugaau auaauugaac cuagcgcaca gcagugagca cuguuaagag 1860
   acugccuuuc cauuuguagc uucauuucug gcacgggagu guuuuguguc agcaguucug 1920
   ccagguggcc aucgaugagc ugaaguaagu ccuaguccag cacaucugcu ucaggccuuu 1980
   guacucuagu caucuggcug cguucgagac uucucagcag aacuuauaga uguguacggc 2040
   ugcacuugga gucagacaag auauggcuac uuuuguacuu auggagccau gccauuuuau 2100
   acuuucacgu uguauacauu cguuugaccc uuuaaguugu ugccacccau aaaaaggcau 2160
   cuuacagugc aguuuuuaaa uuacaugggu agcaauuuug aguuuuaaaa auuagucauu 2220
   gcagaaauua aauacuuaga ggagauaauc cauuaucuug auuuuaggaa uauaauaguu 2280
   gacaauguuu auauauaauu uuacuucucu aaggcauacc caaaaauaga aaaugaaaaa 2340
   gagcagugag ucuguucuga ugcuugcauu gcauagagaa guuuuccaac aaagcagcug 2400
   uuaauaacac auaaaauaug uuuuacuuug caaaguaggu uguguuaagu cauuuucaaa 2460
   aaguuaccua cuauaucgag gcucuggaua auuacuaugu guugauuaaa guuaguuaca 2520
   gaauuguaca agcuaaguuu uccuuaaacu aagcuuaggu uaaagggaga ggagccacag 2580
   cucaaugaaa acacgguucc uguuuucuaa auggaggcgc ccagaaacac aauaaaacau 2640
   guugguacaa aaaaaaaaaa aaa 2663
<210> 57
   <211> 884
   <212> RNA
   <213> Homo sapiens
<400> 57
   caugauccca gccacccgcu cgcuucucug ugcagcgcug cugcugcugg ccaccagccg      60
   ccuggccaca ggggcgccua ucgccaauga gcugcgcugu cagugccugc agaccauggc     120
   ugggauucac cucaagaaca uccagagcuu gaagguguug cccucagggc cccacugcac     180
   ccaaaccgaa gucauagcca cacucaagaa uggucgcgag gcuugccuug acccugaagc     240
   ucccuugguu cagaaaauug uccaaaagau gcuaaaaggu guccccaagu aacggagaaa     300
   gaagacagac ugcucugaug gcaccgucug gugaacgcug gcuucugaca acacuauaca     360
   auuucuuuug aggguccuau uuauuuaugu auuuauuuau uccacaaagu gugugguuuu     420
   uauuuuacau uaauauuuaa caguguggau acauuucauc gaugguaguu caguucugcu     480
   uguucaguuu aaagauggua ggcuuaaaau auuucauuaa aacuaauauu uauugggaga     540
   ccacuaagug ucaaccacug ugcuaguaga aggguguugu gcgaaaagaa gugcagagag     600
   auagaguuua guauuauguu uuguauguau uagggugagg acaugugugg gaggcugugu     660
   uuguaugucu ugaaaagaau gucaguuauu uauugaaagu cgucuuucau auuguauggu     720
   caacacgcac guguugacgc uucccuugga cauuuugugu cuaguuggua gcccauaaug     780
   ggcuuuuaca uucuuuaacc cuguuucucc uggucucguc ucgcucggga cagagacguu     840
   caaaggacug uuacaaauga aguaaaaaua aaaguuuuau uaag                      884
<210> 58
   <211> 1310
   <212> RNA
   <213> Homo sapiens
<400> 58
   cgucucccgc auucgucugg gccgugcacc ugcccgcuag cucgcugcac uaccguugcc 60
   cacaagccaa caugcugccg agauugggcg gccccgcgcu gccgcugcuc cugccgucgu 120
   ugcucuugcu gcugcucuug ggcgcgggcg gcugcggccc cggggugcgc gccgaggugc 180
   uguuccgcug cccacccugc acgcccgagg cucuggccgc uugcgggccc ccacccgacg 240
   cgcccugcgc cgagcuggug cgagagcccg gcugcggcug cugcuccgug ugcgcacggc 300
   aggagggcga agcaugcggc gucuacaucc cgcgcugcgc ccagacgcua cgcugcuauc 360
   ccaacccggg cuccgagcug ccccugaagg cgcuugucac aggcgcgggu accugugaaa 420
   agagacgcgu gggcaccacc ccacagcagg uugcagacag ugaugacgac cacucugagg 480
   gaggccuggu ggagaaccac guggauggga ccaugaacau guugggaggu gguagcagug 540
   cuggccggaa gccccucaag ucaggcauga aggagcuggc uguguuccgg gagaagguca 600
   augaacagca ccggcagaug ggcaagggug ccaaacaccu cagucuggag gagcccaaga 660
   aguugcgccc gccucccgcc aggaccccuu gccagcagga guuggaccag guccuggagc 720
   ggaucuccac caugcgccuu ccggaugauc ggggcccccu ggaacaucuc uacucccugc 780
   acauccccaa cugugacaag cauggccggu acaaccuuaa gcagugcaag augucucuga     840
   acggacagcg cggggagugc uggugugaga accccaauac cgggaagccc auccagggag     900
   cucccaccau ccggggagac cccgagugcc aucucuucua caacgagcag caggagacug 960
   guggggccca ugcccaaagu gugcaguaaa ccccagccag ucggugccug gcuuccccau 1020
   cccgaacacc agcagaaaug gagggcguca gggugacggg uguggaggag uucccaguuu 1080
   ugacacaugu auuuauauug gaaagagacc aacacugagc ucagaagccc cccucugacc 1140
   ccccccagcg gcuguuaacu gaaccucccu ugcuucuguu agagagggga aggguggauu 1200
   ggagggcacu ggguacaggc cugggaaugg ggaaagaaau uuuuauuuuu gaaucccugu 1260
   gucucuuuua cuuaagauua aaggaaggaa aaauaaaaaa aaaaaaaaaa 1310
<210> 59
   <211> 487
   <212> RNA
   <213> Homo sapiens
<400> 59
   gaugugaaaa acguuuuuau uauaaucucu uaaacuuuca guguauauuu ucauuacaau 60
   cauugguaca auaaauaugg aaaugcugag cagacaauua ucacaguggc cuaugggcug 120
   agggacaggg acccaggaau acuguuaccc uggauacuuc cucagggcca aucaggaggu 180
   cuucaaagag uauugagagg gagagggaua gaaauauuau uaagacuguc agugcagcaa 240
   cuuuuagaau gucuauuaaa gccauggaua caggauuuac gauaacagaa gauggauacu 300
   aaaaaagaac agagacucaa guucuccugu aagaggcaga agaaacauug cagaagccag 360
   ugccuuccuc ggguccccag ugugugcccc cauccaccca cgcauugugu uggucaucuc 420
   caccugcccu gugcccagcc cugugcccac ccagguucuc cuaggcaccc accuugcacc 480
   ucgcacg 487
<210> 60
   <211> 1430
   <212> RNA
   <213> Homo sapiens
<400> 60
<210> 61
   <211> 268
   <212> RNA
   <213> Homo sapiens
<400> 61
   uuuuuuuuuu uuuuuuuaaa uaccaaaaca uuuaauugaa auaccuguau aaaaaauaug 60
   aucuucagac auuucacacu uuugaacuua uacaacccca ccccugaugc uuagucacac 120
   cagggucaca gaaacacagc ugcuaaaaua aauuaagggc uugagacucu gucccccaac 180
   cccagcuuuc agagccagca agcagacugu acaaggucaa uaauuuaaac cccuccccag 240
   cgcagagugc ucagggugac agggucuc 268
<210> 62
   <211> 377
   <212> RNA
   <213> Homo sapiens
<400> 62
   uuuuuuuuuu uuuuuuuaau guaacgaccg gugugcuuca guuuguuuag cagaaccacu      60
   cucuugaauc acauuaacuu uugagauuua aaaaaaacaa aacaaaaaaa aaaaaaaaca     120
   acaaaaaaaa aaccaacccu ccauagcaca gcugucuuuu augcaagcaa gagcacaccu     180
   acuccagcau gauuugucau cuaaagacuu gaaaacaaaa caacaacaac aaaaaguuac     240
   uuauagucaa uggauaagca gaguccgaau uuacacuaau caagacagac cuucgagggg     300
   ucacgauaag uccggaacuu ucaaaccuug cuucguauga auuguacuau cugaacauaa     360
   acugcacuuu uauuuuc                                                    377
<210> 63
   <211> 756
   <212> RNA
   <213> Homo sapiens
<400> 63
   augagacucc acagccucau ccugcucucc uuccuucucc uggcuacuca ggcguucuca      60
   gaaaagguca gaaagagagc caagaacgca ccacacagca cagcggagga ggggguagag     120
   gguucagcuc ccucguuagg gaaggcccag aauaagcaga gaagcaggac aucuaaaucu     180
   cugacgcaug gcaaguuugu caccaaagac caagccacau gcagaugggc ugugacugag     240
   gaggagcagg gcaucagccu gaagguccag ugcacacaag ccgaucagga guuuucuugu     300
   guuuuugcug gugacccaac ugacugccuu aaacacgaca aagaccagau cuacuggaaa     360
   cagguugccc gcacgcugcg caaacagaaa aauaucugca ggaacgccaa gagugucuug     420
   aagaccagag ugugcagaaa gagauuucca gagucuaacc ucaagcuggu gaaccccaac     480
   gcacguggaa acacgaagcc caggaaggag aaagcagagg ucuccgcaag ggagcacaac     540
   aagguccaag aagcugucuc cacggagcca aacaggguca aagaagacau cacacucaau     600
   ccagcugcga cccagaccau ggccauuaga gauccagagu gucuagagga uccagaugug     660
   cucaaccaga ggaagaccgc ccuggaguuc uguggggaau cuuggagcuc cauuugcaca     720
   uucuuccuca acauguuaca ggcgacauca ugcuaa                               756
<210> 64
   <211> 4701
   <212> RNA
   <213> Homo sapiens
<400> 64
   cgggucgacc cacgcguccg cccacgcguc cggcggagcu ucuggguugc gggccgaaac        60
   ggcaagcgga uggagggcgc ucgaacggcc aggugucgug auuaaauuag ucagcccuca       120
   gagacaggcg uccuaccucc uuuauccaga ccucaaaagc cccguugugc acccguggug       180
   gcuucuucac cuucccuguu ucguccucca cuguauggcc cagacaugag ugguccccua 240
   gaaggggccg augggggagg agaccccagg cccggagaac cuuuuugucc uggaggaguc 300
   ccauccccug gggccccgca gcaccggccu uguccaggcc ccagccuggc ugaugacacu 360
   gaugcaaaca gcaauggcuc aaguggcaau gaguccaacg gacccgaguc caggggcgca 420
   ucucagcgga guucucauag uuccucuucu ggcaauggca aggacucagc ucugcuggag 480
   accacugaga gcagcaagag uacaaacuca cagagcccau ccccacccag cagcuccauu 540
   gccuacagcc uccugagugc gagcucagag caggacaacc caucuaccag uggcugcagc 600
   agugaacagu cagcucgagc caggacccag aaagaacuca ugacugcacu ucgggagcuc 660
   aaacuucgac ugccaccaga gcgucggggc aagggccgcu cugggaccuu ggccacacug 720
   caguacgcuc uggccugugu caagcagguu caggcuaacc aggaauauua ccagcagugg 780
   agucuggagg agggugagcc uugugccaug gacaugucua cuuacacccu ggaggaauug 840
   gagcauauca cauccgaaua cacacuucga aaccaggaca ccuucucugu ggcugugucc 900
   uuccugacag gccggauugu cuauauuucg gagcaggcag guguccugcu gcguugcaaa 960
   cgggaugugu uucggggugc ccgcuucuca gagcuccugg cuccccagga uguggguguc 1020
   uucuauggcu cuacuacacc aucucgacug cccaccuggg gcacuggcac cucugcaggu 1080
   ucaggucuca aggacuucac ccaggaaaag ucugucuucu gccgaaucag aggagguccu 1140
   gaccgggauc cagggccucg guaccagcca uuccgccuaa ccccauaugu gaccaagauu 1200
   cgggucucag auggagcccc ugcacagccg ugcugccuac ucauugccga gcgcauccac 1260
   ucugguuaug aagcuccccg gaucccuccu gacaagagga ucuucaccac ccgacacaca 1320
   ccaagcugcc ucuuccagga uguagaugaa agggcugccc cacugcuggg uuaccuuccc 1380
   caggaucucc ugggggcucc aguacuucuc uuucuacauc cugaggaccg accccucaug 1440
   cuggccauuc auaagaagau acugcagcug gcaggccagc ccuuugacca uuccccuauu 1500
   cgcuucugug cucggaacgg ggaauauguc accauggaca ccagcugggc cgguuuugug 1560
   caccccugga gccgcaaggu ggcuuucgug uugggucgcc auaaagugcg cacggcaccc 1620
   cugaaugagg acgucuucac ucccccagcc cccagcccag cuccgucccu ggacucugau 1680
   auccaggagc ucucagagca gauccaucga uugcugcugc agccugugca cagcuccagc 1740
   cccacggggc ucuguggagu uggcccucug auguccccug guccucuaca cagcccuggc 1800
   uccuccagug auagcaaugg gggggacgcu gaggggccug ggccuccugc uccagugacu 1860
   uuccagcaga ucuguaagga ugugcaucug guaaagcacc agggacaaca gcucuucauu 1920
   gaaucucggg ccaagccccc accccggccc cgccuccuug cuacagguac auucaaagcc 1980
   aaaguccuuc ccugccaguc cccaaacccc gaacuggagg uggccccagu uccugaccaa 2040
   gccucguuag ccuuggcccc ugaggagcca gagaggaaag aaaccucugg cuguuccuac 2100
   cagcagauca acugccugga cagcauccuc agguauuugg agagcugcaa cauucccagu 2160
   acaaccaagc guaaaugugc cuccuccucc uccuacacug ccucuucagc cucugaugau 2220
   gacaagcaga gggcaggucc aguuccugug ggggccaaga aagauccguc gucagcaaug 2280
   cugucugggg agggggcaac uccucggaag gagccagugg ugggaggcac ccugagcccg 2340
   cucgcccugg ccaauaaggc agagagcgug guguccguca ccagucagug uagcuucagc 2400
   uccaccaucg uccauguggg agacaagaag cccccggagu cggacaucau caugauggaa 2460
   gaccugccug gccuggcccc uggcccagcc cccaguccgg cccccagccc cacaguagcc 2520
   ccugacccaa ccccagaugc uuaucgccca gugggucuga ccaaggccgu gcugucccug 2580
   cacacacaga aggaagagca agccuuccuc aaccgcuuca gagaucuugg caggcuucgu 2640
   ggacuugaca ccucuucugu ggcccccuca gccccuggcu gccaccaugg ccccauuccc 2700
   ccuggucgcc gacaccacug ccgaucuaaa gcaaagcguu cccgccacca ccaccaccag 2760
   accccccggc ccgaaacucc cugcuauguc ucccauccuu caccugugcc cucuucugga 2820
   cccuggccac ccccaccagc cacgaccccc uucccagcaa ugguccagcc cuacccacuc 2880
   ccaguauucu ccccucgagg aggaccccag ccccuucccc cugccccuac aucugugucc 2940
   ccugcuaccu ucccuucucc cuuagugacc ccaauggugg ccuuggugcu cccuaacuau 3000
   cuauucccua ccccaccuag uuauccauau gggguguccc aggccccugu ugaggggcca 3060
   cccacgccug cuucccacuc gcccucucca ucccugcccc caccaccucu cagccccccc 3120
   caccgcccag acuccccacu guucaacucg agaugcagcu ccccacucca gcucaaucug 3180
   cugcagcuug aggagucccc ccgcacggag gggggcgcug cugcaggagg cccaggaagc 3240
   agugcugggc cccugccucc cagugaggag acugcugagc cagaggccag auugguggag 3300
   guuacugagu cguccaauca ggaugcacuu ucaggcucca gcgaccugcu ggagcuacug 3360
   cuccaagaag acucucgcuc gggcacaggc uccgcagccu caggcucccu gggcucuggc 3420
   cugggcucug ggucugguuc aggaucccac gaagggggaa gcaccucagc cagcaucacc 3480
   cgcagcaguc agagcagcca uacaagcaag uacuuuggca gcaucgacuc uuccgaggcu 3540
   gaagcugggg cugcucgggc caggacugag ccuggggacc aggucauuaa gugugugcuc 3600
   caggacccca ucuggcugcu cauggccaau gccgaccagc gugucaugau gacauaccag 3660
   gugccgucca gggaugcagc cucugugcug aagcaagacc gggagaggcu ccgggccaug 3720
   cagaaacagc agccacgguu cucagaggac cagaggcggg aacugggugc ugugcacucc 3780
   uggguccgga agggccagcu gccucgggcc cuugauguga uggcgugugu ggacuguggc 3840
   agcagcguuc aagauccugg ccacucugau gacccgcucu ucucagaacu ggauggauug 3900
   gggcuggagc ccauggaaga ggguggaggc gaggguggug gguguggugu uggcgguggu      3960
   gggggugaug guggugagga ggcccagacc caaauugggg cuaaggguuc aagcucucag 4020
   gacucugcca uggaggaaga agagcaaggu gggggcucau ccagcccagc uuuaccugca 4080
   gaagaaaaca gcaccagcua gauccauuuu ggggccgcuu acagcagucu aaugagaggc 4140
   uuccuuucga ccauguuggg guucuuauaa cucaagauac agcuggacca accaauagga 4200
   aacugcccca gcuucuccca acauaggggg cuggaccccc auuaccagcc caggcacagg 4260
   agcugccucu agcuucuuag cagaguggaa guucucagcc ccauuuggag gauuguccac 4320
   gcccguccca cugaggagac gggcgggucu ucgguuaagg uugcugacaa gcugcugaag 4380
   uggucugucc aaaucccagc ugagccugag ucccagucgc aggguugggg cugcacuuau 4440
   uuauuuggga gagacagcuc acucucccac cucaccccaa gaugggagga ggggaaccug 4500
   ggaucugugu aggauccagg uccgugaacc ccuagcugcu ccaggguggg ggagguuggu 4560
   ggaccaugga gucccuggug cugccccuca ggugggaccc agguguucuc agcucuaccc 4620
   ucuaccaaug acauuugugu uuuugauauu gugucuguua uuuuuuuuuu aauacaaaau 4680
   gacaaaauga aaaaccaaaa a 4701
<210> 65
   <211> 3036
   <212> RNA
   <213> Homo sapiens
<400> 65
   gacagcggag cgcgguggcg ucgacgucua gugucucagu gcucccgucu guggcuaacu 60
   aagcagccag cagccaggca gcucgcgacc ugcggccagg cagccaacca ugcucaacuu 120
   cggcgcuucu cuccagcaag cuucggaggg gaaaauggaa cuaauuucug aaaagcccag 180
   agaggggaug caucccuggg acaaagcuga gcagagugac uuugaagcgg uggaagcgcu 240
   cauguccaug agcugcgacu ggaagucuca uuucaagaaa uaccuugaaa acaggccugu 300
   cacaccagug ucugauaccu ccgaggauga cagcuugcuu ccagggacgc cugaccuuca 360
   gacaguccca gcauuuuguu uaacgccacc uuacagcccc ucugacuucg aacccuccca 420
   agggucaaau cugacugcau cagcgccauc uacuggccac uucaaaucuu ucuccgaugc 480
   ugccaagccu ccaggcgcca cuccuuucaa agaggaggaa aagaauccuu uagcugcccc 540
   uccucuuccu aaggcucaag ccaccagugu cauccgucac acagcugaug cccaacugug 600
   caaccaccag uccugccccg ugaaagcagc uagcauccuc aacuaucagg acaauucuuu 660
   ccggagaaga acccacggaa auguugaggc uacucgaaag aacauacccu gugcugcagu 720
   gucaccaaac agauccaagc cugagcccag cacagugucc gauggugaug agaaggcggg 780
   cgcugcacua uaugacuuug cugugccuuc cucagagaca guaauuugua ggucucagcc 840
   agcuccuucg uccccagugc agaagucagu acuggugucu ucaccuacag uauccacugg 900
   gggagugcca ccccugccug ucaucugcca gaugguuccc cuuccugcca acaacucucu 960
   uguuagcaca guugucccca gcacuccucc uagccagcca ccagcugucu gcucaccugu 1020
   guuguucaug ggcacucagg ugccugaggg caccgucgug uuugugguac cccagcccgu 1080
   ugugcagagc ccaaggccuc caguggugag ccccaguggc accagacugu cucccauugc 1140
   cccugcuccu ggauucucuc cuucagcagc aagggucacu ccucagauug acucguccag 1200
   aguaagaagu cacaucugua gccacccagg guguggcaag acuuacuuua aaaguuccca 1260
   ucugaaggcc cacgugagga cacacacagg ggaaaaaccu uucagcugca gcuggaaagg 1320
   cugugaaagg agguuugcuc gcuccgauga acuguccaga caccggcgga cacacacagg 1380
   ugagaagaag uuugccuguc ccauguguga ccgucgguuu augaggagcg accauuuaac 1440
   caagcaugcc cgacgccacc uaucagccaa gaagcugcca aacuggcaaa uggaaguuag 1500
   caaguuaaau gacauugcuc ugccuccgac cccugcuucc gcacagugac ggccagaaga 1560
   uggagacgca gaauaaacuu uggucagagu caggagccag ugauggugucaagugcuucu     1620
   gcaaggcugu ggcccuccaa aagggccuaa aguagaagcc cuggccuggg ggaggccccg 1680
   ccugggugaa augacaagaa gugcuucagc cacaggcagg ucacagagga cagggcucag 1740
   uucuuaccac agagagagag gagaacccuu uuauuccucc cuuauuuuag ucuggaaagu 1800
   uucggcugag gugagcgcag cacagguuuu gaaucacaua cacauugggg acuuuguuuu 1860
   ugccauuuau acuugagacc agcuuugcag ugugauucuu ucaaaggauu gguuucaaga 1920
   auauagaggc uggaaauuac gguacagaaa uggagcuaga aaaugaguuu guguuacaca 1980
   gagaugucau cuucuccuag aguuaucuug uuucuuauuc cuagucuuuc cagucaaauc 2040
   cguggaugua gcuaaguaua ucuaaaacuc auuuuuccac uauuguuggu auuugaaguu 2100
   gaacagcugu acauugcugu gggggagcca aaggauugga acccucauua auuuaauugc 2160
   uuggaaaugc agcuaaaauu cuucuuuggc auuuuguuuu gaaaguuuag gcauuuuacu 2220
   cuacuuuaga uuuuaguuug cuugcaguuu uuuguguaga uuugaaaauu guauaccaau 2280
   guguuuucug uaggcuuaaa auacacugca cuuuguuuag aaaaaaaucu ggagaugaaa 2340
   auauguauua uaaagaagag augucaagaa uuugagauaa cuccuugaga aaguuggcuu 2400
   uaugucauca gcaaaggaca cuuaacguca agcauacacu gugguuuuuu uguuuuuuug 2460
   uuuuuuuuuu ucaaauuaga aaguuuaaug accguuacag auggacagug ucuuuuuauu 2520
   uauaggaguu uuucaggaug ucagaguaga uagguaggaa aauuguuauu agaacauucg 2580
   cuucuaccuu gaaaaggaug uuaauguggu cauguucuua gcaccacagu gucugggcau 2640
   cugggaaacu ccgagacuuu uuuaaagugu caugauguga ucacaccugc aguuuggggc 2700
   aucgaaucca gggccuugca ugucuucugu aagagcucuc aucgcugacc uguauccccc 2760
   gcaagagcaa ugacuuuugc uaacaguauu ucuuuucugu uguaaagugg acagaugaua 2820
   cacuuggucg caaagguaaa uuauucaaaa uccacaguga aaaccucacc acacuuuccc 2880
   auuuaaacua uuuccauauc ucagagguuu cugacaugca aacuugaacc cuugaaagaa 2940
   gaguuuucuu aaaaauuaua aaaaaucacg aguuacaauu ugcacaauau uuuuuguuga 3000
   acuuuauacc uuguuuacaa uaaagacuuu ucuuug 3036
<210> 66
   <211> 526
   <212> RNA
   <213> Homo sapiens
<220>
   <221> misc_feature
   <222> (7)..(7)
   <223> n is a, c, g, or u
<400> 66
   uugucanuug cacgacagaa acugcaggaa gaugagaugc gccgggcugc ggaggagcgc 60
   aggaggguaa aggcugaaga guuagcugcc agacaaaggg uucuagaaaa aauugaaagg 120
   gacaaagcag agagagccaa gaaguauggu gguagugugg guucucgguc auccccacca 180
   gcaacagacc cagguccugu uccuucuucu cccagccagg agcccccuac uaagcgggag 240
   uaugaccagu gucguauaca gguuaggcug ccugauggga cuucacugac ccagacuuuc 300
   cgggcccggg aacagcuggc agcugugagg cucuacgugg agcuucaccg uggggaggag 360
   ccuggacagg accaggaccc ugugcaguug cucaguggcu uccccagacg ggcuuucuca 420
   gaggcugaua uggaacggcc ucugcaggaa cugggacucg ugccuucugc uguccucauu 480
   guggccaaga agugucccag cugaggguuc uccaucccac caucuc 526
<210> 67
   <211> 425
   <212> RNA
   <213> Homo sapiens
<400> 67
   uuuuuuuuuu uuuuuuucac aaauaaguaa uauaacuuua uuaaaaugaa aagacaauau    60
   ucaaaauaau gcaacaaaau gaauaaaucc uuuguccaau acuguacaca cagugcggag   120
   aucagugcau uuuucuaaag cauguuuuaa ccuucauuua guucauacua aaguaagcuu   180
   uaaauagcuc aaauaauguc auucagcagu uuaaacugaa cagcuuguug ggacauggca   240
   gcaguguccc ugcuagcaag caccuucucu uuguguuuau cugcacaaga uaaacaauca   300
   gaggauguaa aaacugaaca caaacugcgu gucucacuga aucucagggc agugaagcag   360
   ccagcgugag uuuucaaagc aggaagaugc ugaagugacc ucuggcauua agacguucug   420
   ugcua                                                               425
<210> 68
   <211> 2282
   <212> RNA
   <213> Homo sapiens
<400> 68
   uucauuggaa agcacauugc ucuucucagu aauuucuuua guaucuucau gaauuuuuuc          60
   cuuuugcguc guuauuucag caauucuauc ugcagaucau aaagguagua uugacagaug        120
   ugauucuuuu ucuuaaacau uucauuucca ggguaaggaa cucaauggcu auguuuuucu        180
   cucccucuag ugcauccuuc uccuuuucua ccauuuucac ucuauuuaac uucaucaugu        240
   ucagucuggc cuuuggguuu caucauagca aucuguucaa cuucacccug caagauuaaa        300
   aaucgauuau ggucuaaguc aaugccaugg cuucuaagga gauuuccaac aucuuuaaac        360
   gucuucuucu ucccacuuau gugguagaca gaagugcuau cucuguaggc gguucuggau        420
   acauagaagu ugcuguuagg aaggacuucg uaaucaucuc cuuccuuauc aauuaucuuu        480
   ugaaaaugaa cuucuacagu acaacucuga auauccuugu guucaucaga auuauguauc        540
   aguaccgaua gcuuuuuaga ccuuauuuuu ugugcucgau agccaaacac aaaaagcaug        600
   gaaucaauaa cauuggauuu uccacugcca uuugguccaa uaauacagga aaagcgcuua        660
   uggaaagguc ccacaaguuu cucuccagca uaggacuuga aguuuugguu uacaauauga        720
   guuaucauga gacgaggagc uccagcuuca cuggccaugg cuggaggugg ggguggaggg        780
   auacuauuca aaaucuccuc uaaacuucug uuauccagcu ccucaccugg aggcuuugcg        840
   ggugcagcgc uccccucacc uacuucgccu acugcaacuc ccaccaccuc cucucgauac        900
   aagaccgagc ucugucggac cuacucagaa agcgggcguu gucgcuacgg ggccaagugc        960
   caguuugcuc acggccuggg ugaacuucgc caagccaauc gccaccccaa guacaaaacg       1020
   gaacucugcc acaaguucua ccuccagggc cgcugccccu auggcucucg augccacuuc       1080
   auccacaacc ccaccgagga ccuagcucuc ccuggccagc cccaugugcu gcgacaaagc       1140
   aucagcuucu ccggcuugcc ucaggccgca gaagcucgcc gccaccucca ggcuuuucug       1200
   gcccuucccu guccucuugu uccuuuucgc cuuccagcuc cccaccgccc ccuggggauc       1260
   uuccacuuuc cccuucugcc uucucugcug ccccugggac cccugugacu cgaagagacc       1320
   cuaaccaggc cuguugcccc uccugccgaa ggucuacuac ccccagcacc aucugggggc       1380
   ccuugggugg ccuggcucgg agcccaucug cccacucucu gggauccgau ccugaugacu       1440
   acgccagcag cggcagcagc cugggggggu cagacucacc ugucuuugag gcaggggugu        1500
   uugggccucc ccagaccccu gcacccccaa ggcgucuccc caucuucaau cguaucucug       1560
   ucucugagug acaagugccu accuacccag uauggaucag cuagaucuca aagagagggc       1620
   agggacugcu cauugcugug gggaccuggg gcacuccucu aaguuaauaa gucccaucuu       1680
   cuggacauuc caagaugcaa uaacccauuu cccuggugcu gggcuggggc aggucccuag      1740
   uuugcaaauu caguguuugg guggauccgu uccuagggua ccuaagaugu uugagggaga      1800
   caguugacag uuggucuucc aggccccaag ucuucuguug uuuuugagau aggagcuuau      1860
   uaugguaccc caggcuggcu uugaacucaa uauaauccug ccuuagccuu uuccaaguuc      1920
   ugggguuaca gguaugcacc agccccucug caacucuggu cuccuggaau cuuaagugcu     1980
   gugaagagcc ggcucccaca auacuauccu aauuuuuacu agacccugaa guucaguguc      2040
   cgguggucga agccucuccu gagaauccug gugcucaaau uucccuccua aagcaaauag      2100
   ccaaagccau ugccaaaucc cuucuccccc aaccaguggg cccuuuauuu augacgacuu      2160
   uauuuauugu auuaagauuu uauaguauuu auauauauug ggucgucuac uccguuuuuc      2220
   uuuuuguaau guuaaaacug auacuguauu aaguauaugc uauaauauau uaauauauug      2280
   cu                                                                     2282
<210> 69
   <211> 364
   <212> RNA
   <213> Homo sapiens
<400> 69
   cggacgcugc agaccugacc gacgugcugu gcgaguucga cgcggugcug gccgacuucg        60
   cgucgcccuu ccacgagcgc acuuccacua ugaggagcac cuagagcgca ugaagcggcg       120
   cagcagcgcc agcgucagcg acagcagcgg cuucagcgac ucggagagug cagacucagu       180
   guacagggac agcuucaccu ucagugauga gaagcugaau ucuccaacca acuccucucc       240
   agcucuccug cccuccgcug ucacuccucg gaaagccaaa uuaggugaca cuaaagagcu       300
   cgaagacuuc auugccgauc uggacaggac cuuagcaugu augugaagca aggaguuugg       360
   gguc                                                                    364
<210> 70
   <211> 847
   <212> RNA
   <213> Homo sapiens
<400> 70
   ccgggccgag gagaagucug caaaacaaga ggcuggggau ugccuuagcg agaaaucagu 60
   ucucuuagga gguuagggaa ggaagucuuu cucuggaggu cugagggaag cgcucguguc 120
   agaugccggg uugucauggg uaagggugac cccaucaagc cgcugggcaa aauguccucu 180
   uacgccuuuu uugugcagac cugccgggag gagcacaaga agaagcaucc caauucgucg 240
   gucaacuucg ccgagaucuc caagaaaugc uccaagagau ggaagaccau gucugcaaag 300
   gaaaacucga aguuugaaga uuuggccaag agcgacaaag cuuguuauua cagggagaug 360
   aagaacuaug uuucucccaa aggugauaag aaaggaaaga aaaaagaucc aaaugcuccg 420
   aagagaccac cgucugccuu cugccuguuu ugcucugaaa aucgcccaaa gaucaaaauu 480
   gaauacccgg gccugucuau uggagauacu gcgaagaaac ugggugagau guggucugag 540
   cagucugcca aagagaaaca accguaugag cagaaagcag cuaaacuaaa ggagaaguau 600
   gaaaaggauu uugcugcaua ccgugucaag ggcaaaagug aagcaggaaa gaaggguccu 660
   gguaggccag caggcucaaa gaagaaugac ucagaagaug aggaagaaga agaggaggaa 720
   gaugaagagg gggaagaaga ggaugaagaa uaaguggcua uccuaaagug uggaguauau 780
   gugcucaggc aguuauuuug cuaagaaugu aaauucaagc gcagcucaac auuagcucca 840
   guaggaa 847
<210> 71
   <211> 779
   <212> RNA
   <213> Homo sapiens
<400> 71
   aucacgcucc uagaacucuu caaaccgauc ucucgucgau cuucaaccgc cgccuccacu    60
   cgccauggac cccaacugcu ccugugccuc cgguaagggg gacugcugac gggauuucug   120
   ggagagcuag acaggcuuuu uggccccucc uuuaguaauu acuuuaaggg uacgaccggc   180
   uaccccuucc gaaugaauuc ugaagcacuc cugcuccuuu aaacuagucc uugagauagu   240
   ggcucgccua cccgggugau uugccucacc uuccuaggag aacagcguuc agguacuccc   300
   gggucccacu caaccgcgcu cacugacugc cuucuacuuu uagauggauc cugcuccugc     360
   gcuggcgccu gcaaaugcaa acaaugcaaa uguacuuccu gcaagaaaag uaaguuggau   420
   cuucucugcc auuuccccgu cacucuccug gggucccuag cccgccgcgc cgcgccuucc   480
   cucccgggag cguucaggug gugugccucu gacaagguuu cucgcucacg uucaacucuu   540
   cucuccccac aggcugcugc uccugcugcc ccgugggcug ugcgaagugc ucccagggcu   600
   gcaucugcaa agaggcuucc gacaagugca gcugcugugc cugaaggggg gcggaggggu   660
   ccccacaucu guguaaauag accauguaga agccuagccu uuuuuguaca acccugacuc   720
   guucuccaua acuuuuucua uaaagcaugu aacugacaau aaaagccguu gacuugauu    779
<210> 72
   <211> 2275
   <212> RNA
   <213> Homo sapiens
<400> 72
   gcucugaguu uguggaagau uacaugcgau aucccgcgcg accccgcauc ccuuugccgg    60
   ccgggacagc cuuugcuaca gccugugaaa cauugcgucc ccgagcccca cgccugaggg   120
   cgacaugaac ccgcuggcuu cgcgagcagu ccggacccac gaucgcuuuu ggcaaccaga   180
   accggcgcuu cagcccccgg ggugacgugc agcccgccgc ccagacacau ggccccgagc 240
   ccaagacccc agcauguccu gcacuggagg gacgcccaca acuucuaucu ccugucccca 300
   cugaugggcu ugcucagucg ggccuggagc cgccugaggg gcccagaagu cccagaggca 360
   uggcuggcaa aaacaguaac aggagcagau cagauagaag cugcggcucu gcugacaccu 420
   accccugucu cugguaaccu ccucccucau ggggagacug aagaaagugg aucuccugaa 480
   cagagucaag cagcccagag gcucugccuu guggaagcug aaaguucccc uccugaaacu 540
   uggggacuuu caaauguuga ugaguacaau gcaaagccag gacaagauga ccuuagagag 600
   aaggaaaugg aacgcacagc uggcaaggcc acacuacagc ccgcuggccu gcaaggggcu 660
   gauaagaggc uuggggaggu gguggcuaga gaagagggag uggcugagcc cgcuuauccc 720
   acaucacagc uggagggugg uccagcugag aaugaagagg auggagaaac agugaagacu 780
   uaccaagcuu cugcugcuuc cauagcuccg ggauacaaac ccagcacccc ugugccuuuc 840
   uugggggagg cagaacauca agccacggaa gaaaaaggaa cagaaaacaa ggcugacccc 900
   uccaacucuc cuucuucagg cucccacucc agagccuggg aguacuacuc uagagagaag 960
   ccuaagcagg agggagaagc caagguagag gcacacaggg cagggcaggg ucacccuugu 1020
   cggaaugcug aggcugagga aggaggaccu gagacaacuu uugucuguac uggaaaugcc 1080
   uuccugaagg ccugggugua ucgcccagga gaggacacag aggaagaaga caacagcgau 1140
   ucggauucag cugaggaaga cacagcucag accggugcca ccccccauac aagugccuuc 1200
   cugaaggccu ggguguaucg cccaggagag gacacagagg aagaagacag cgauucggau 1260
   ucagcugagg aagacacagc ucagaccggu gccacccccc auacaagugc cuuccugaag 1320
   gccugggugu aucgcccagg agaggacaca gaggaagaaa acagcgauuu ggauucagcu 1380
   gaggaagaca cagcucagac cggugccacc ccccauacaa gugccuuccu gaaggccugg 1440
   guguaucgcc caggagagga cacagaggaa gaaaacagcg auuuggauuc agcugaggaa 1500
   gacacagcuc agaccggugc caccccacau acaaguccuu uccugaaggc cuggguguau 1560
   cgcccaggag aggacacaga agaugacaca gaagaggaag aggacaguga gaauguggcc 1620
   ccaggugacu cagaaacagc ugacucaagc cagaguccuu gccuucagcc ccagcguugu 1680
   cuaccaggag agaagaccaa gggacguggg gaagagcccc cucucuucca gguggccuuc 1740
   uauuuacccg gagagaagcc agaaucaccu ugggcugcac cuaagcugcc ccuucgacug 1800
   cagaggcggc ucagauuguu caaagccccc acccgggauc aggaccccga gauuccucua 1860
   aaagcucgga agguacacuu cgcugagaaa gucacagucc auuuccuugc ugucugggca 1920
   ggaccagccc aagcugcccg ucgaggaccc ugggagcagu uugcacgaga ucgaagccgc 1980
   uuugcucgac gcauugccca ggcagaggag aagcuggguc ccuaccuuac cccugauucc 2040
   agggccagag caugggcacg ccuuagaaac ccaucucuuc cacaguccga gccucgcucu 2100
   uccucugagg ccacucccuu gacccaagau gugaccacac ccucuccccu ucccagugaa 2160
   accccuucgc ccagccugua cuugggaggg aggcggggcu aagccugagu aguuuccuau 2220
   uauuuauuua uuuauuuauu ugaauaagaa auaaagccuu uuaauuugua gugau 2275
<210> 73
   <211> 355
   <212> RNA
   <213> Homo sapiens
<400> 73
   uuuuuuuuuu uuuuuuuaau caucgagaag uauuuauuga gcaccagcuu uggggucggg 60
   ugugaggacu cgggacaaug cagggugcug ucccuucucg ugagacgcuu acaaucugag 120
   uggagacagg gagggagcca caauacaugu ucuuggggug cgggcuaagg guagacaguc 180
   cagaccagga uguuacagaa acagggaugu uuggggcugg agucagaccc acuaagugcu 240
   uugacaccca cgguauucaa cacugagaaa ggaucagcca uugcucagug uccugugagc 300
   ucccuuagcc cccaagacac caucuuggcc uggcuccuug uacaacugcu acuaa 355
<210> 74
   <211> 289
   <212> RNA
   <213> Homo sapiens
<400> 74
   cccaaaaccc auucagcaaa guucccaacc ucgacgggcu agcaguauuu aaccagugau 60
   ggguucacug uuguauuugg ugaauacugu auuuuguuuc aguucuuucu cccagauaau 120
   uugaaaacgu uccaggagaa ggcagcuucc uauaugcagc gugugcuuuc uuauucuuuu 180
   uuuuaauaua ugacaguuau uugagaaccc auuucuacuu ugaauucauu uucguugaaa 240
   gugauguuuc uucaccuacc auuuuccuau uaaaguucug uauucaaau 289
<210> 75
   <211> 2018
   <212> RNA
   <213> Homo sapiens
<400> 75
   agaccgugag cgagagcgcc ccagagaagc gccugcaauc ucugcgccuc cuccgccagc      60
   accucgagag aaggacaccc gccgccucgg cccucgccuc accgcacucc gggcgcauuu     120
   gaucccgcug cucgccggcu uguugguucu gugucgccgc gcucgccccg guuccuccug     180
   cgcgccacaa ugagcuccag caccuucagg acgcucgcug ucgccgucac ccuucuccac     240
   uugaccagac uggcgcucuc caccugcccc gccgccugcc acugcccucu ggaggcaccc     300
   aagugcgccc cgggagucgg guugguccgg gacggcugcg gcugcuguaa ggucugcgcu     360
   aaacaacuca acgaggacug cagcaaaacu cagcccugcg accacaccaa gggguuggaa      420
   ugcaauuucg gcgccagcuc caccgcucug aaagggaucu gcagagcuca gucagaaggc      480
   agacccugug aauauaacuc cagaaucuac caaaacgggg aaagcuucca gcccaacugu      540
   aaacaccagu gcacauguau ugauggcgcc gugggcugca uuccucugug uccccaagaa      600
   cugucucucc ccaaucuggg cugucccaac ccccggcugg ugaaagucag cgggcagugc      660
   ugugaagagu ggguuuguga ugaagacagc auuaaggacu cccuggacga ccaggaugac      720
   cuccucggac ucgaugccuc ggagguggag uuaacgagaa acaaugaguu aaucgcaauu      780
   ggaaaaggca gcucacugaa gaggcuuccu gucuuuggca ccgaaccgcg aguucuuuuc      840
   aacccucugc acgcccaugg ccagaaaugc aucguucaga ccacgucuug gucccagugc      900
   uccaagagcu gcggaacugg caucuccaca cgaguuacca augacaaccc agagugccgc      960
   cuggugaaag agacccggau cugugaagug cguccuugug gacaaccagu guacagcagc     1020
   cuaaaaaagg gcaagaaaug cagcaagacc aagaaauccc cagaaccagu cagauuuacu     1080
   uaugcaggau gcuccagugu caagaaauac cggcccaaau acugcggcuc cugcguagau     1140
   ggccggugcu gcacaccucu gcagaccaga acugugaaga ugcgguuccg augcgaagau     1200
   ggagagaugu uuuccaagaa ugucaugaug auccaguccu gcaaauguaa cuacaacugc     1260
   ccgcauccca acgaggcauc guuccgacug uacagccuau ucaaugacau ccacaaguuc     1320
   agggacuaag ugccuccagg guuccuagug ugggcuggac agaggagaag cgcaagcauc     1380
   auggagacgu gggugggcgg aggaugaaug gugccuugcu cauucuugag uagcauuagg     1440
   guauuucaaa acugccaagg ggcugaugug gacggacagc agcgcagccg caguuggaga     1500
   augccaaggg gcugaugugg acggacagca gcgcagccgc aguuggagaa gacuucgcuu     1560
   cauaguacug gagcgggcau uauugcucca uauuggagca uguuuacgga ugacguucug     1620
   uuuucuguuu guaaauuauu ugcuaagugu auuuuuuugc uccagacccc cccccccuuu     1680
   cuugguucua caauuguaau agagacaaaa uaagauuagu ugggccaagu gaaagcccug     1740
   cuuguccuuu gacagaagua aaugaaagcg ccucucauuc cuucccgagc ggagggggac     1800
   acucugugag uguccuuggg gcagcuaccu gcacucuaaa acugcaaaca gaaaccaggu     1860
   guuuuaagau ugaauguuuu uuuauuuauc aaaguguagc uuuuggggag ggaggggaaa     1920
   uguaauacug gaauaauuug uaaaugauuu uaauuuuaua ucagugaaga gaauuuauuu     1980
   auaaaauuaa ucauuuaaua aagaaauauu uaccuaaa                            2018
<210> 76
   <211> 358
   <212> RNA
   <213> Homo sapiens
<400> 76
   ccaauaccac agccgugacc acagccaaga ccacagccaa aagccuggcc auccgcacuc      60
   ucggcagccc ccuggcagug cccuccauau ccugcuuguu uuucucauua guaaacuccu     120
   cuucuaaaga aaacugggga agcagaucuc caaccuccag gucauccucc cgagcucauu     180
   ucaggccagu gcuuaaacau acccgaauga agguuuuaug uccucagucc gcagcuccac     240
   caccuuggac cacagaccug caacacuagu gcacuugagg gauacaaaug cuugccugga     300
   ucuuucaggg cacaaauucc gcuucuugua aauacuuagu ccauccaucc ugcgugua       358
<210> 77
   <211> 870
   <212> RNA
   <213> Homo sapiens
<400> 77
   auggagaacg aguucacaua cgaagauuau gagaccacug ccaaguggcu ucugcaacac      60
   acugaauauc gaccucaagu ggcagugauc ugugguuccg gcuuaggagg gcugacugcu     120
   cacuuaaagg aggcucagau cuuugacuac aaugagauac ccaacuuucc ccaaagcaca     180
   gugcaagguc acgcaggccg acugguguuu ggauugcuga auggcagaug cugugugaug     240
   augcaaggcc gguuccauau guaugaagga uacucacugu caaaggugac auucccagug     300
   agaguuuucc aucuucuggg uguggaaacu uuggugguca ccaaugcugc uggaggacuc     360
   aaccccaauu uugaaguugg agauauuaug cugauccgug aucacaucaa ccuaccuggu     420
   uucuguggcc agaacccucu ccggggcccc aacgaugaaa gguuuggagu ucguuuuccu     480
   gccaugucug augcuuauga ccgggauaug aggcagaagg cuuucagugc cuggaaacaa     540
   augggggagc aacgaaagcu acaagaaggc accuacguga uguuggcagg ccccaacuuu     600
   gagacugugg cagagagucg ucugcuaaag augcuggggg cagaugcugu uggcaugagc     660
   acagucccag aaguuaucgu cgcaaggcac ugugggcucc gugucuuugg uuucucacuc     720
   auuacgaaca agguugucau ggauuaugag aacuuggaga aggccaauca cauggaaguu     780
   cuggaugccg ggaaagcagc ugcacagaca uuggaaaggu uugucuccau ucuuauggag     840
   agcauuccac ucccggaucg uggcagcuga                                     870
<210> 78
   <211> 4928
   <212> RNA
   <213> Homo sapiens
<400> 78
<210> 79
   <211> 374
   <212> RNA
   <213> Homo sapiens
<400> 79
   uuuuuuuuuu uuuuuuuaag cguccaggcu guacuuuauu uuacacaagu gguggcccca 60
   gaaccacagg gacaugaccu ggagaguagg cacagugccu gaggcugcaa gagccaaaua 120
   cagggauuca ugccuucucc uuggucccca ugaccaaauu aaaaaaaaaa aaaacaacaa 180
   aucacacagc acacaucgcc acacccaucc ccuccuuccu uucagcaaca gccaauucag 240
   cuuucuagcc aaagacagug gcuacaacug aauuuacaga gaaccaugca gccaagaaac 300
   cagagccacg gaggggagag gcuugcguug acuucccaca ugugcugucc cauagcagcu 360
   gagugacccc acca 374
<210> 80
   <211> 1285
   <212> RNA
   <213> Homo sapiens
<400> 80
   agaaagauaa gggccagcaa ggaaagaaug aggauguggg cgccgaggac ccguccaaga 60
   agaagcggca acgccggcag aggacucauu ucacuagcca gcagcugcag gagcuggaag 120
   ccacuuucca gagaaaccgc uacccagaca uguccacucg cgaagaaauc gccgugugga 180
   ccaaccuuac ggaagcccga guccggguuu gguucaagaa ucgccgggcc aaauggagaa 240
   agcgggaacg caaccagcag gccgagcugu gcaagaaugg cuuugggccg caguucaacg 300
   ggcucaugca gcccuacgau gacauguacc ccggcuauuc guacaacaau ugggcugcca 360
   agggccucac gucagcgucu cuguccacca agagcuuccc cuucuucaac uccaugaacg 420
   ucaauccccu guccucucag aguauguuuu ccccgcccaa cuccaucuca ucuaugagua 480
   ugucguccag cauggugccc uccgcgguga ccggcguccc gggcuccagc cucaauagcc 540
   ugaauaacuu gaacaaccug agcagcccgu cgcugaauuc cgcggugccc acgcccgccu 600
   guccuuacgc gccgccgacu ccuccguacg uuuauaggga cacauguaac ucgagccugg 660
   ccagccugag acugaaagca aagcagcacu ccagcuucgg cuacgccagc gugcagaacc 720
   cggccuccaa ccugagugcu ugccaguaug cagucgaccg gccgguguga accgcgccca 780
   gggcgcgggg auccgaggac ugucggagug ggcaacucug ccccagaaag acugagaauu 840
   gugcuagaag uucgugcgca cuaugggaag gaagaggggg gaaaaaagau cagaggaaaa 900
   gaaaccacug aauucaaaga gagagcgccu uugauuucaa aggaaugucc ccaagugucu 960
   acgucuuucg cuaagaguau ucccaacagu uggaggacgc guacgcccac aaauguuuga 1020
   cuggauauga cauuuuaaca uuacuauaag cuuguuauuu uuuaaguuua gcauuguuaa 1080
   cauuaaaaug acugaaagga uguauauaua ucgaaauguc aaauuaauuu uauaaaagca 1140
   guuguuagua cuaucacuac aguguuuuua aaggcuaggc uuuaaaauaa agcauguuau 1200
   acagaaucag uuaggauuuu ucgcuugcga gcaaaggaau guauauacua aaugccacac 1260
   uguauguuuc uaacauauua uuauu 1285
<210> 81
   <211> 801
   <212> RNA
   <213> Homo sapiens
<400> 81
   cugagaggcc aggugggcgg cgaaaucaac guggagaugg augccgcucc cgguguggac 60
   cugagccgca uccugucaga gaugcgugau caguacgaga agauggcgga gaagaaccgc 120
   aaggaugccg aagacugguu cuucagcaag accgaggagc ugaaccgcga gguggccacc 180
   aacagcgagc uggugcagag cggcaagagc gagaucuccg agcucaggcg caccaugcag 240
   gcccuggaga uugagcugca gucccagcuc agcaugaaag caucucugga gggcagccug 300
   gcagagacag agaaccgcua cugcgugcag cugucucaga uccaggggcu gaucggcagu 360
   guggaggagc agcuggcuca gcugcgcugc gagauggagc agcagaacca ggaguacaag 420
   auccugcugg augugaagac aaggcuggag caggagaucg ccaccuaccg ccgucugcug 480
   gagggagagg augcccaccu gacucaguac aagccaaaag aaccugugac cacccgccag 540
   gugcgcacca uuguggaaga aguucaggau ggcaagguca ucucaucccg ggaacaggug 600
   caccagacca cccguuaagg acucagcucc uuccgcccag uuccccgagg cugcagagag 660
   gcagcuuccc ucuccgcucc ggcaucaccc uccugcuaca gccucucccc agcauuccua 720
   ugcuugagac cauuaaagcu ugcugaccug aagugaacug uggccuuugu ucugaacacu 780
   gaaauaaaug accaugguga c 801
<210> 82
   <211> 216
   <212> RNA
   <213> Homo sapiens
<400> 82
   ccccaggggu gaaaugagga uucccccacc cugcggaaca gugaaaugug uauaauuaag 60
   aggagggcga cgacccuugc cgcgggaccc gggacucgag cccgggacuu cgcagcuaca 120
   gcaaaucuau uuuuaauauu gugcugagca agacagaucg cuugcauauu uuuaaaaauu 180
   uuuacuacag agacauucca auaaauucgu uaagcc 216
<210> 83
   <211> 2901
   <212> RNA
   <213> Homo sapiens
<400> 83
   cugacagucg ucucuguccc uucuucgccu cggagcugcu aacuggucuc gaaccucuca      60
   gcacuucagc uucuagcggc gaugcaugug aucaagcgag auggccgcca agagcgaguu     120
   auguuugaca aaauuacauc acgaauccag aaacucuguu auggacucaa cauggacuuu     180
   guugauccug cucagaucac caugaaagua auccaaggcc uauauagugg ggucaccaca     240
   guggaacugg acacccuggc ugcugagaca gccgcgaccu ugaccacgaa gcacccugac     300
   uaugccaucc uggcagcaag gauagccguc ucuaacuugc acaaagaaac aaagaaagug     360
   uucagugaug ugauggagga ucucuacaac uacauaaauc cgcacaacgg cagacacucu     420
   cccauggugg ccagcucaac acucgacauu guuauggcca auaaggaucg ccugaauucu      480
   gccauuaucu augaccgaga uuucucuuau aacuacuuug gcuuuaagac acuggaacgg     540
   ucauauuugu ugaagaucaa ugguaaagug gcugaaagac cacagcauau guugaugagg     600
   guuucugugg ggauucacaa agaagauauu gaugcugcaa uugaaaccua caaccuacuu     660
   ucugagaagu gguucacuca ugccccuccu acucucuuca augcugggac caaccgccca     720
   cagcugucua gcuguuuccu cuugaguaug aaagaugaca gcauugaagg aauuuaugau     780
   acucugaagc agugugccuu gauuucuaag uccgcugggg gaauuggugu ugcugugagu     840
   uguauucggg ccacugguag cuacaucgcu gggacuaaug gcaauucuaa uggccuugug 900
   ccaaugcuga gaguauauaa caacacagcu cgcuaugugg aucaaggugg aaacaagcgc 960
   ccaggcgcgu uugcuauuua ccuggagccu uggcacuuag acaucuuuga guuccuugac 1020
   uugaagaaga acacaggcaa ggaagaacag cgagcacgcg aucucuucuu ugcacuuugg 1080
   aucccagauc ucuucaugaa gcgaguggag acuaaccagg acuggucauu gauguguccc 1140
   aaugaguguc cuggucugga cgaggucugg ggagaggagu uugagaaguu auaugaaagu 1200
   uacgagaagc agggucgugu ccgaaaaguu guaaaagcuc agcagcuuug guaugccauc 1260
   auugaguccc agacggagac cgguacccca uacaugcucu acaaagauuc cuguaaccgg 1320
   aagagcaacc agcagaaccu gggaaccauc aaaugcagca accuguguac agaaauagua 1380
   gaguacacca guaaagauga gguugcaguu uguaacuugg cuucucuggc ucugaauaug 1440
   uaugucacac cggaacauac guaugacuuu gagaaacugg cagaagucac uaaagucauu 1500
   guccgaaauc ugaauaaaau aauugauaua aacuacuacc cuauuccaga ggcacacuua 1560
   ucaaacaaac gccaucggcc cauuggaauu gggguacaag guuuagcaga ugcuuucauc 1620
   cugaugagau accccuuuga gagcccagaa gcccaguuau uaaauaagca gaucuuugaa 1680
   accauuuacu auggagcccu ggaagccagc ugugaacuag ccaaggagua uggccccuau 1740
   gaaacguaug agggaucucc agucagcaag gguauucuuc aguaugacau guggaauguu 1800
   gcuccuacag accuguggga cuggaagccu cucaaggaga agauugcaaa guaugguaua 1860
   aggaacaguu uacuuauugc cccaaugccu acugcuucaa cugcccagau ucuggggaau 1920
   aaugagucca uugagccuua uaccaguaac aucuacacuc gaagagucuu gucaggggaa 1980
   uuucagauug ugaauccuca cuuacugaaa gaucuuacug agcggggcuu guggaaugag 2040
   agaugaaaaa ucagauuauu gcaugcaaug gcuccauuca gagcauacca gaaauuccug 2100
   augaccugaa gcaacucuau aagaccgugu gggaaaucuc ucagaagacu guucucaaga 2160
   uggcagccga gagaggugcu uucaucgauc agagccaguc uuuaaacauc cauauugcug 2220
   agcccaacua uggcaaacuc acuaguaugc acuucuacgg uuggaagcag gguuuaaaga 2280
   cuggaaugua uuacuuaagg acgaggccug ccgcuaaucc aauccaguuc acucugaaca 2340
   aggaaaaacu gaaagauaag gaaaaggcac ugaaggagga ggaggagaag gagaggaaca 2400
   cagcagccau ggugugcucu uuggagaaca gagaggagug ccugaugugu ggauccugag 2460
   aaaagcgggg ccugggagac gcagcgggcu cuccugcccg agaggcagac gauuugagca 2520
   uagauaggau aguggguuug cuugguuauc agcagcucug cuuggacgug ccugccagga 2580
   cagggagcca cgacuuacag uacuguuucu acacagugua aauaucauuu uuaacaaaca 2640
   gaaaaccaaa gccagcuuug auauuaggaa ucaagguaga ggcuuuggga auacuaaaga 2700
   gccuuccugc aaauagugag gagacuuagg aagucucguc ucuccagcuu ucccugccug 2760
   gccauucuca guuugggcaa agagauuuag uuugauuuga cugauugccu agaaguaaaa 2820
   ucaagcaauu acucaucagc uaaagaccuu ugucuagaca aacuucuaua agucauuuug 2880
   aaauaaacau uucuaaguga u 2901
<210> 84
   <211> 8752
   <212> RNA
   <213> Homo sapiens
<400> 84
   uugccucaga cgcuagcugu agcuggcagg cgguuguacg ugcuccagag ucgucgguac 60
   ccgcuacugc agucgcuuuc guguggcuuc cgcugagcuc uuccgagcug cucgcucucc 120
   acacgcgccg ccgccguaau ccgccaccau ggugaagcuc gcaaagguaa gaggccuugg 180
   cgcgccgacg cggacgacua ggccccugcu uucggaggga cgcgcgcgcg cccgcccguc 240
   cgucgcggag gggaggaggg cuugcgcgca aucccgggcg cguucgaggg cgccaugcug 300
   ggggggaaag ucucgcgcga cuagcgggag gucucgcggu gcuugcccuc ugacuuaggg 360
   ggaugagaag agcggaggca gguuuccggg agggcgauau cgaggguucg gauguagcgg 420
   gcgggagggg acggugugag gagagaucgg aggagcugag agcggauagg ggcacggcgu 480
   gggaagagag ggccaaccuu aggcggcgag cggucccggg gccccgccuc cccgcgcacg 540
   ugcucuggug cgcgcccgcc acgugcucug cggagccccg cacgugucgc gcgacccggg 600
   gcaguggggg agugucugua guaccccgga aagggggacg gcagcguggg gauggauggg 660
   uggcccggcg aucugcuguc ucugccggug accgggaugg acacguggug gaccccugag 720
   guggcggcgu ggugacucca cguggugggg cuggaagcga gagaaagugg gaagcaguug 780
   gguuacgugg ugcugcuuua agaggugauu ucgagauacc cccuucccca gcaaauaacu 840
   uaaagggauc ccuuuaacug gguuuuuuuu uuuuuuuuuu uuuuuuuuuu uguggaagau 900
   gccagaaaau agauggccag gauuaggaga cuuuauaacc uguggcuguu ucuuggugua 960
   gaguucuguc ugcucaguua ucugugagaa ggaaaaaaaa auuaugcgcg guucgcagaa 1020
   aaaacugcca ggagaaugcc augccuggcc aagaagaagu cuuuaugcuu guguccuuua 1080
   guaagaaaaa ggugguggcc aaaggcaaag ugacugaaaa ugcgugcaau uuuugugugc 1140
   guuuguaggc uggcaaaacc cacggugagg ccaagaaaau ggcuccuccu ccaaaggagg 1200
   uggaagagga uagugaagau gaagaaaugu cagaagauga agaugacagc aguggagaag 1260
   aggagguaag aagcuauuug cagcgaauua aaccggugga auugaauguc uggaagucuu 1320
   agaaauacag gauauguagu aaaugguuga auggcaagcc ccuuccuccc ucccucccuc 1380
   ccucccuccc ucccucccuc ccucccuucc uuccuuccuu ccuuccuucc uuccuuccuu 1440
   cugcaagaca gucggcaaaa caggggacag aaacaggcag aauuuugagu uccaggcaag 1500
   cagggaguag uacauaguga aaccuugucu caagaccguu guuaugguca ugcucaauca 1560
   gauuucuuag aaaagcucag gugcugaguc caguuuuuuu uuuuuuaaag uauugaaagc 1620
   caugucuccu uauuucaggg uuuaauguuu aucuuugugu gugcgcgcac ccauuaagca 1680
   ugcuugguac ccucauacua gaauguacuu ggauccccug gaacuggagu uaaagccaca 1740
   ugugaauguu acauguuaca agaguaacac augugcuuaa cuuuugaguc aucucuccag 1800
   uucuugguug uuuguuuuuu uuuuuuuaag ccuaucuaau guccauuuuc uugugcucaa 1860
   aguuagucuc uuaauguagc auuggguaua aaggaaugcu uaugauuugu uugcuuucaa 1920
   gguugucauc ccucagaaaa aaggcaaaaa ggcuaccaca accccagcaa agaagguggu 1980
   uguuucacaa acaaaaaagg cugcaguucc cacaccagcu aagaaagcag cugugacccc 2040
   aggcaaaaag gcaguagcca caccagcuaa gaaaaacauu acaccagcca aagucauucc 2100
   aacaccgggu aagaagggag cugcacaagc aaaagcguug guaccaacuc cugguaaaaa 2160
   gggagcugcc acuccagcua agggggcuaa gaacgguaag aaugccaaga aggaagacag 2220
   ugaugaggau gaagaugaag aggaugaaga ugauagcgau gaggaugaag augaugagga 2280
   agaggaugag uuugagccac caauaguaaa aggagugaag ccagcaaaag cagcuccugc 2340
   ugcuccugcc ucagaggaug aggaagauga ugaggaugaa gaugaugagg aagaugauga 2400
   ugaagaggag gaagauggug aguuagaucu uaggauauuu aggguacugc auguacauuc 2460
   ccucacuguu ucauuagauu aaaaacucau uuugugcucu uaguucuuuc cauaacuuaa 2520
   uagguuuuca uuugcuaagu aguuuuuguu uuuuuuaagu auuuguagca uuuaucuugu 2580
   cuggauuggu agguagcaaa uacauuugcc ugauuugcca ucuuucuucc agacucugag 2640
   gaagaaguua uggagaucac aacagccaaa ggaaagaaaa cuccugcaaa aguuguuccu 2700
   augaaagcca agaguguggc ugaggaggag gaugaugagg aagaggauga agaugacgag 2760
   gaugaggaug augaggaaga ggaugacgaa gaugaugaug aggaagaaga ggaggaaggu 2820
   aaccauauua acuuuuaaag uaugcugacc uaaguaaggc uuacuggcua ugcuaaagug 2880
   ucugcuuacu caugaauggc auuuuaaaac aucuagaacc uguuaaagca gcaccuggaa 2940
   aacggaagaa ggagaugacc aagcagaaag aagccccuga agccaagaaa cagaaaguag 3000
   aagguaagcc ugcaaaacug gggaaacaga ucagaguagc acuagcacaa gugaugagug 3060
   acaaagggac uuaauacuga accauggggu ugaaaugaaa uaugcugaug ugcuuuauag 3120
   uuuaugauga aauuuguugu gugcuuaagu gggcugaaag uucauuuuuu gugugugcag 3180
   gcucagaacc aacuacaccu uucaaucugu ucauuggaaa ccuuaaucca aacaagucug 3240
   uuaaugaauu aaaauuugcc aucagugaac uuuuugcuaa aaaugaucuu gcuguugugg 3300
   augucagaac ugguacaaau agguaaguuu uaauugaaug uuacaugugu aucagcuaga 3360
   auuuuuaguu uccaguugua uucuucccug ccuuuaaaca uggggcuaua ucuaacuaug 3420
   uuaguaaaag ucaguugucu ccucucgugg ccuuaaguac aguuaaggag cugcaguaag 3480
   aaagacuaua guauugaacu aaaugaucga gucauagggc cugcaauuug aaguuccugu 3540
   guuugacuug auaaagauaa aauaaaauuu aaagaagaaa agauauuaaa cacauaaaau 3600
   uuugugcagu aucuacaacu auggaucugc auagucauau gcuuuuagcu aaaaguauuc 3660
   ucuguacuuu uagcgggguc caugcuagcu acugcuguua guuacaauau acugaaugaa 3720
   gaaaucgagg ugaauuuguu guaaugucuu gguacaugga cuuguuuugu uuuuuguuuu 3780
   uuuuucuuua agauuuguuu auguauauga gcacacugua gcuguccaga ugguuuugag 3840
   ccuucaugug guuguuggga auugaauuuu uaggaucuca gcucgcucug cucucagucc 3900
   uugcuugcuc uggcccaaag auuuauuugu uguuauacau aaguacacug uagcugacuu 3960
   aagaugcauc agaagagggc auuaggucuc auuaugggug guggugagcc accauguggu 4020
   ugcugggauu ugaacucagg accuucagaa gagcagucaa ugugcuuacc cgcugagcca 4080
   ucucuccagc cuuuggacuu gguuuuaugg aagauaaggg ugaucuaguu uuauuuuugu 4140
   uagugcugua gaugcucugu gugugugcca caugguauaa gugcagauca ccuucucaua 4200
   ccuguaaucu uguuuuuucc aucuucaagg aaauuugguu auguggacuu ugagucugcu 4260
   gaagaccuag aaaaggccuu ggagcucacu gguuuaaaag uguuuggcaa ugaaauuaaa 4320
   cuagaaaaac caaaaggaag agauaguaag aaagguaugu aagggggucu gggugacugg 4380
   auacuaacag acuuaggcag ucuggugccu cuuccuuagu uucauccuca uugugaacca 4440
   augagauguc auaggucaug ugcuuguuga cagguuugau uccugggaua uauaauguca 4500
   gggcugacag gaggaauagc uuagugagua aagaugcuug cugcaaaaug uuugaucucu 4560
   agaagccaca ugaagagaga agaaccuuua aucccagcau uugggagaca gaggcaggca 4620
   gauuucagag uucgaggcca gccuggucua cagagugagu uccaggacau ccagggcuac 4680
   acagagaaac ccugucucgg aaaaaaaguu uuagcuuauc cucugaccac auguguaucg 4740
   ugacaugcuu gaagcuuacc uaucucuuaa augaauucuu gaucccuaua uuuugaguuu 4800
   cagaauuugg auuuuaagug uuuguuucuu aguugugcug aaaauugaac gugggcuuuu 4860
   cacaugcuag gcaaauuugu uggguuuuuu uguuuguuuu uuucucaaga caggguuuuu 4920
   cuguguagcc cuggcugucc uggagaccaa gcuagccuug aacucagaaa ucugccugcc 4980
   ucccaagugc ugggauuaaa ggcgugaguc accacugccc ugcuaggcaa ucacucuuaa 5040
   aacugcuaca uauccucugu ccccuuuugc ucauuuuaca agguugcugu gugcucaauc 5100
   ugcagucuau guuauaugcu uacuggaucu aggcuuuuga uguagaauga accauaugag 5160
   ugaugaggua ucuuagagau ggaaacuaag ucuaaauaga cuuguuccau auacaacuua 5220
   auacauaugg ucuaaggaac augauauaca uguaaacaag uaggaaggag auaagucugg 5280
   uguccaggga agccaggaga gccucaucug aaacuggaca gggguuugug agucaucagg 5340
   ugacaauuga acauggguac ucuucaugca aaugguuagu aaccauugag ccaccucucc 5400
   aucccuuuau accauuuuuu uuuuuagcau auauccuugu acuuuauagg aauuauuugc 5460
   uuuauucucu ugugacuugu aaauugaugu acuuaauuaa aucuuuuucc aacauaguuc 5520
   gagcugcaag aacacuucua gccaaaaacc ucucuuucaa caucacugag gaugaauuaa 5580
   aggaaguguu ugaagacgcc auggagauca gauuagucag ccaggauggg aaaaguaaag 5640
   gguaguuuug ugucuuugag uguuaaaguu uuauuaaguu uagugucuuc uuccccucuc 5700
   cuuguccuug acagucucua gucugcugcu ucaaacuuaa cuucauaacc agaaaauuga 5760
   auaucugguc cucuggccuc uaccucccaa guucugacau uaaaaaugcu caacugaugg 5820
   guuuugaagg ugugaucaau uuuacaggcu caauccaaau uggcaucuuu ugccacaagu 5880
   acuauccuuc ccuauuuuau gagagaaaug ugauucuagg caguucaguc uauuguguug 5940
   gcucuuuuuc cuccucacca guuuaaagga ugaagaugag cuaauacaua guaaaagaac 6000
   aguaaaagca caugugacua aguccuucau gucugaugcu ugaguaauau uuucucuaac 6060
   guaguaacug aauugucuug uacucuuuca ggauugcuua uauugaauuu aagucugaag 6120
   cugaugcaga gaaaaauuug gaagaaaagc agggggcaga aauugaugga cgaucuguuu 6180
   cacucuacua uacuggagag aaaggucaaa ggcaagagag aacuggaaag accagcacuu 6240
   ggagugguaa guuaaagggu uuauugugua gugggaacag gaaucauuug uaucuuugua 6300
   uuuuaaguaa uugguuaccu acaauuaguu caccuuuguu cauauagcug auguuuaguc 6360
   uucaugagug aaagcuauuu gaaaucauuu ccuuuggagu auaguaggca aauaaagcuu 6420
   uuuguugggu auguuuugua cuuuaaaugg cuuaaacuau uuuagaaaau aguguaagac 6480
   aacaaagaac aguuaucuaa uuagaaugaa aaugaaagga gcaaagaagg cauuacugua 6540
   uauaauggau auacacuggu gguucuagaa uuaugguaua ugguacaugg uugaagugcc 6600
   auuguuucag uuaacauucc aguaaccuug uggauuaggu uggagacaug cuuuauaggu 6660
   gacccacuua cugaguguuu aaauauacac agacauacuc uaacauaccu ugcuaaugug 6720
   uuaucuuugu auuugcaggu gaaucaaaga cuuugguuuu aaguaaccuu uccuacagug 6780
   caacaaaaga aacucuugag gaaguauuug agaaagcaac uuuuaucaaa gugccccaga 6840
   acccacaugg caaaccuaaa ggguaaaaua auuuuuacgu uagauguggg cuggacauac 6900
   auacucuuac guauaagagu aagacugucc uguuagcuua aaaaaaaacu aaaguuuuag 6960
   cuauacaaag ggcaguaaau auugauagua aauuacaugc ugaugccaag uguuucuaag 7020
   cuuuauucug agaacugacu uucaaccuuc agguaugcau uuauagaauu ugcuucauuu 7080
   gaagaugcua aagaagcuuu aaauuccugu aauaaaaugg aaauugaggg cagaacaauc 7140
   aggcuggagu ugcaaggauc caauucgaga agucguaagu ccuuugacau gauaugacuu 7200
   gguuggguga uuuuuuuuuu uauuuuuaug ugccuauaug cucauuuggg gcugucuuua 7260
   uguuguugcu gagaaaauga caacuggaua ugaugacuga uuaccugaga aauaauugau 7320
   gaaaucucaa gaaaauuccu cuagauaguc aaguucugau ccagcuaugu caacucaaag 7380
   cagcaaccuu gauugcccuc ugaguacgcu uuuuuuuuga uccaguguag ucuuuuuuuu 7440
   uuuaaccuua auuucuugug uuaauugcuu uuucugguaa aagggggaaa aaaagacaua 7500
   acaaaaucag uguaagggaa ggcucagugg uugagcacug agaggaccug gguucaaauc 7560
   ccagcacuca cauggcaccu aucgagacag gauuucucug uguaguccug ccugucgugg 7620
   aacucacucu guagaccagg cuggccucaa acucagaaau cugccugccu cugccuccua 7680
   ggugcuggga uuaaaggugu gcgccaccac ugcccaaccc ugucuguaac ucuuaagauc 7740
   ugacauagau ugcagacaaa acacuaaugc acauaaaaaa auuuuuuuuu uuaaaaaagg 7800
   aaucuacuuc agcugaaugu ggcaguaugg caguauucac caaggguuca uagugaaaca 7860
   ggaauuuuuc ucuuccagaa ccauccaaaa cucuguuugu caaaggucug ucugaggaua 7920
   ccacugaaga gaccuuaaaa gaaucauuug agggcucugu ucgugcaaga auagucacug 7980
   aucgggaaac ugguucuucc aaaggguaag aaggcguagu aguguugcug cuuuuuagug 8040
   aauucugcau ggagaacuug ggucugcagu aucuucucau ugagcuccuu ucuguccauc 8100
   agugauagau uauggauucg cacgagaaga agagagaauu cacagaacug gcacuuaucu 8160
   ucuguuuuug cagaaguaua uuuggcuguu gugugagaca uuaugagaua cuggcgauuu 8220
   ucucgaccug aagaguacuu uggucacucu acuuggguga cuugguacuu auuguguuac 8280
   uuuaaaaugu guuuacuuaa ugggugaggu uuuuuuguuu uucuuuucug uuuuagguuu 8340
   gguuuuguag acuuuaauag ugaggaagau gccaaagcug ccaaggaggc cauggaagau 8400
   ggagaaauug acggaaacaa aguuaccuug gacugggcca aaccuaaggg ugaagguggc 8460
   uuuggugguc gagguggagg cagaggaggu uucggaggca gagguggagg cagaggugga 8520
   agagguggau uuggaggaag aggccgggga ggcuuuggag guaaggaagg gaaaggaacu 8580
   ggaaacggau uccuaaaccu guguccuaac caaccaccuu aaaugggaag gucaguccua 8640
   auuguaucac ccuuugaugu uuuuccuucc uauagguaga ggaggcuucc gaggcggcag 8700
   aggaggaggg ggagacuuca agccacaagg aaagaagacg aaguuugaau ag 8752
<210> 85
   <211> 3889
   <212> RNA
   <213> Homo sapiens
<400> 85
   uuagacgguu gcgcgcgcag aggguuggua guugucgcug uaggccuucg cugccgcuuc 60
   ugcaucguga aucgggggac cuuggcagcc agaccucguu ccucuuagag uagcucucau 120
   cuagucgcca caacuccgcc accauguuug aggcacgccu gauccagggc uccauccuga 180
   agaaggugcu ggaggcucuc aaagaccuca ucaaugaggc cugcugggac gucagcucgg 240
   gcggcgugaa ccugcagagc auggacucgu cucacgucuc cuugguacag cuuacucugc 300
   gcuccgaagg cuucgacaca uaccgcugcg accgcaaccu agccaugggc gugaaccuca 360
   ccaggugagc ggguggcggg agcggggccc cacucuuccc gcuuccgcuc uuggcggggc 420
   ugugacucug cacgcucauu ggcuggcuug gccauccgcg cuuucugauu ggucuauggu 480
   gucgggggca gcccucacca aagcgcgcgg uuccgaaaag cccgcgcugg caguggcgcc 540
   cacucuguuu ccgcgccaaa gccacaaagc gggaguccgc gggaaaauga gugcuccgga 600
   gcugugcuca uuaaaugccu gcagcuuuga guggcugguc uuagcgccua auaaacgagu 660
   cuuagugcaa auguaauguc gacuuagagu gacaauagac cuuuucuuga cuuccagagu 720
   cucacugcgc aucauggauu ugaggggaaa ucugucaguu uuagcuuuua acuuugcuac        780
   agcuaccuag guuagugccu ccuguauacg uguucaagga caguguguga cuuauuuuag        840
   uacagauaca uggauuagug ccacuuguau acauuuugaa agauuuacga aaaggccaga    900
   cgugaugggg cacauucucc aguacacuag aaaccaagga caccccgcuc aaaaagaugc    960
   uuucucgaau guuggcuuuu agugcauuuu acuaagucgg uuuuaagaau cacauauacc 1020
   cgguaauuug cuucaccccu gagagaguuu gggguacccu uagccccuuu aacaguucuc 1080
   caaccgugag ugugaaaugg uacaacuugu aauugcuuuu uaaaauauag auguggauua 1140
   cauguugaua aagccugucu uuuuuuuuuu gggggguagc auguccaaaa uucuaaaaug 1200
   ugcugguaau gaagacauca uuacauuaag ggcugaagau aaugcagaca ccuuagcacu 1260
   aguauucgaa gcaccaagua aguuaaacac cuuuaaaacu cggaguuacg uguuguuucu 1320
   guuucucaaa accaaaaaaa auauuaacaa uauuguaaau uccaucauag auaggaccgu 1380
   guggugugcu ugguaacauu uuccuucuuu ugguagauca agagaaaguu ucagacuaug 1440
   aaaugaaguu aauggacuua gauguggagc aacuuggaau cccagugagu uaccuuguuu 1500
   cugauugugu guuacccugc ugugauacca gcugaugcgu guucugagug gagugguggu 1560
   auuggggaug aauggcacac ugccauuuca cuaaaccaca gcagucuaaa guugauugag 1620
   uuuuaaagaa accagaaguc uugcauucug aguucugguu aagaugcuaa aucuugagaa 1680
   caugaagcug agccuucccc cuuuucuaga cugaccuuua acuugugggu uuacaggaac 1740
   aggaguacag cuguguaaua aagaugccgu cgggugaauu ugcacguaua ugccgagacc 1800
   uuagccacau uggagaugcu guugugauau ccugugcaaa gaauggggug aaguuuucug 1860
   caaguggaga gcuuggcaau gggaacauua aguugucaca aacaaguaau guggauaaag 1920
   aagaggaggc ggugaguagu aagggggcgu ccaguuaggu gucugaagca gggauggagc 1980
   cucggcuuuu guuuuuauuu auucauucau uuugagaugg agucuugagu agaccaagcu 2040
   aucuuagagc ucagagacga cuccauaagc uuuuacaggu agcauuugga aagcuaagug 2100
   uacagccuuu ugcuuccugg aaauacucuu ggcaaauaag ugaggguugg caagugagca 2160
   aaagaaaaug guugggggug uauguagcuu uauguguugc agguucaaga guauuugcag 2220
   ucccaaggga aauaagaaag acuucacaaa auguggaaag aguuguauua aaugcucuug 2280
   acaguuacau ccauagagaa agcugggcau gaugucucaa acccacaacu gauguacuca 2340
   aagcuacagc aggaagauuc ucagcuuaaa gucaaccugg cagaaaaucu agcucaaaaa 2400
   gaaugaggaa gaaauuggga aggcaaagga agauguucuc cgaguccucc ucauucaagu 2460
   agaacauacu aggccucuuu aauuucuaag uaucccugaa ucgaggcuuu uucucaggaa 2520
   uccaauguau auuucauggc uacacuuuuu uuuuucuuuu uuaaguuuug cuagcuagcc 2580
   ugagcaucag aauuacacac agaagucuga acuaaauagg auuuuuaggg uuuaguauag 2640
   ugaaauucag agugcuucug caaguauuua agguaaauau aagguguuac uuggccucug 2700
   caugaauuua aaguaaauga aaguguaaga auucgaacau agauaaacac acaacccaag 2760
   aacuaguucu uaaccuuaau cugcugaauu auuucuacuu ccauaucaac uucagcuccu 2820
   caguucucaa auacugacau guaauucauc aguauuuguc ugaugugcaa gcauuuccac 2880
   aacaaaagaa auuaaggaau uuuucaguau ccacauguuc aaggauuggg aauugaauaa 2940
   aauugauaau cauacaauga agacugguuu acugucccuu agcuugcauu cagcuguugg 3000
   uucuuguuuu uggaaguggu uauguguuau cuuccucucu ucaugcauuc uuugcaagag 3060
   aaguauguac aaucugaaua ggaacaacuu ucuccuuugu uuugauugcu uggggugugg 3120
   cucuacagga ugggcaagcu agacuuuuuu cuucuuuagu caagguuuuc aucaacccuu 3180
   ccaaaaugau aacuauuugu uuugcuuugu gguauaauac cgugaucuaa uagugugagu 3240
   uucugauguc uacagugagc cuguuuucuc cucuagguaa ccauagagau gaaugagccu 3300
   guucaccuaa cguuugcucu gagguaccug aacuuuuuca caaaagccac uccacugucu 3360
   ccuacaguaa cacucaguau gucugcagau gugccccuug guaagaugau aaguuugaac 3420
   auuguuuugu aaugugguau uuauaguauu cggugguuua auuuuuccug ucuuucaguu 3480
   guagaguaua aaauugcuga caugggacac uuaaaguauu auuuggcucc caagauugaa 3540
   gaugaggaag caucuuaggc auugcuagaa auugagaaaa cuaaaccuuu gaagauugcu 3600
   ccugagaugc cagcgugucc ugaggucuuu ucugucacca aguuuguacc ugaguauucu 3660
   uaaauauuaa aauaaaauau guagauaucu ucuguaaaua accuacuuuc uuuucucucc 3720
   auucuccaua auuugcuuaa agaauaagcu ccaaaguaaa aacuaguuuu guuaacauga 3780
   auguuucugc uuuacaaaua cuggugauuu uccaucaaug aucuugacgc uaaaugcagu 3840
   uuuaagaaau auuguucaau uuaaauaaag uuaacaauuu gaaaaguca 3889
<210> 86
   <211> 1720
   <212> RNA
   <213> Homo sapiens
<400> 86
   ggugcaggau guaggccugc agaauuguca gacccagugg cggcuuuugc cacuggaaga     60
   auuuccaaca uaaaacagau gaucaguuug ggacaaucga uucugcgacc agagguagug    120
   ucauuugguu acuuuuaaaa uucagauugu cuggguguuu ccaaucacuc gcgacuguaa    180
   uuugaaguug gguucugaga uaauacaauc gcugucgcuc uaguuuauaa agcuguccaa    240
   gaucugccca gucccagaug uccugggucc ucagggccgc uguggucugc caacuucugc    300
   agcuggaugc cagaccaucc uggacucgga ucccuuuggg uauuucuaca ccgcuguguc    360
   ccggccuggc cuuggggagc ccugguucau aaucgucggc uauguggacg acaugcaggu    420
   ccugcgcuuc agcagcaagg aggagacucc gaggauggca cccuggcugg agcaggagga    480
   agcaggugac ugggagcagc agacucguau agucacaauu caaggacagc ugucugaaag    540
   gaaucugaug acccugguuc auuuuuacaa caagagcaug gacgacucuc acacacuaca    600
   guggcugcaa ggcugcgaug uggagccaga ucggcaccug ugucucuggu acaaccagcu    660
   cgccuaugau agcgaggauc uccccacccu gaacgaaaac ccaaguuccu guacaguggg    720
   aaacagcacu guaccucaca ucucucagga ccugaagagc cacugcucag aucugcugca    780
   gaaauaccug gaaaaaggga aggagaggcu gcugcguuca gacccuccaa aggcacaugu    840
   gacccgucac cccagaccug aaggugaugu cacccugagg uguugggccc ugggcuucua    900
   cccugcugac aucacccuga ccuggcaguu gaauggggag gagcugaccc aggacaugga    960
   gcuuguggag accaggccug caggggaugg aaccuuccag aagugggcag cugugguggu   1020
   gccucuuggg aaagagcaga guuacacaug ccauguguac caugaggggc ugccugagcc   1080
   ccucauccug agaugggagc cugcauggua ccaaaagccu uggauuugga uuguugccau   1140
   gguuuucauu uuguucauca uuugucucug ugugguuugc auaugcauga agaagaaugc   1200
   aggugggaga ggaaggcgug acacccaaga agcaggcaga gacagucccc aagacucuag   1260
   caagacuguu guggaugaug aggagauggg gguuugcuuu uggaagauua aguccuguaa   1320
   aacuugucua ggccacuccc caggaacuuc aguuggcgag ucuuuacugu caccuugacu   1380
   ggauuuagga ucaucuggga gaugccccuu ugaguggcug ggcugugagg acagcaggcc   1440
   aguucuugcc acccuggaca gaaacacauc ucaccuuucu ggcucaagga ucugaacacc   1500
   ugucucuugc cuacucggcu ucuagucagg cauuuuguca ccuugucaag ggucccaggg   1560
   acacaaagcu cccuccucuc acccacagca cucugggucc uacccucagu gcuucaggga   1620
   cauuuaauca ggucaaauug ggaucaaugg cuuugaugca gaaaagaacu guggacuaau   1680
   agagauaggg uuuaauuaaa aaauauaucu uuuuaauuuu                         1720
<210> 87
   <211> 367
   <212> RNA
   <213> Homo sapiens
<400> 87
   cuggcacgga cauggcuguc uucugucugc ugugugggaa acgcuuucag gcacaaagcg     60
   cacuccagca gcacauggag guccacgcag gcgugcgcag cuauauuugc agugagugca    120
   accgcaccuu ccccagccac acggcucuca agcgccaccu ucgcucacau acagguuuuu    180
   uucuccaugu gucaccaagu gaaguuugug ccuucuauag caaagagaau auuuuuuaca    240
   uccuacuaac aguagauuuu uuuguaguga acauuuuuug uauuuuuauu uauaagucuc    300
   auaagaaaaa uagcgauguu caguuguaua ccuugaaucu gcaguuagaa gagaauaaag    360
   uuaacuc                                                              367
<210> 88
   <211> 7879
   <212> RNA
   <213> Homo sapiens
<220>
   <221> misc_feature
   <222> (4457)..(4460)
   <223> n is a, c, g, or u
<220>
   <221> misc_feature
   <222> (4471)..(4471)
   <223> n is a, c, g, or u
<400> 88
   ggagccccag ugcaggaggc ugcgaaaguu gggggagugu gcuagaagga cugcggagcg     60
   gagcgcacgc gggaccaggc ugcgacuggg ucgcuggucc cggccacagg gagugggagc    120
   cggcugggua gguaccccgc agggagcgug uuucuguuuc uagggacagu gcaugaaaga    180
   gauggggugu acgcgcgggc gaaaaggaag ggguguuucg ggucggcuua cgaggagggg    240
   uguguagggu gcauuuuugg uauuaaagga uagcucuugg aguaggguga gccagggcuu    300
   gaaguuuagg aagcgggcga agggucugca aggugcuugg uuuugguagg uagugggcgc    360
   uuguuuagga gguuuccccg cuaggaucuc cagugugagg cacucuguac ucugguuuug    420
   gguguugauu gccggguagg ggguugucug agcauggacu gggaaaaggu acucagagcu    480
   ucggcguugc uggggaucca uccaaguaca aguaagauug gauuuccacg ggucuccuuc    540
   ccggcucuca gcccccuuuc ccaguguuua cuuaauacuu cauaggcugu acuuagauuu    600
   ucugauuccc uuaccgaacu uucuuucuga accgugcugg aagaccuggg gguugcugaa     660
   ggaaauggcc agcuagguug gcuggguaau gguuguguua gacuaaguug ucagaguauc     720
   uagaaaccua ggagcuguag ccuggugcag cuacugaaag ccgcacgugg agccugggug     780
   gaaguugcuc acgauagagu cucgauguag uacugacuag gggggagacc ccuucuguca     840
   ucagacagac uuguauaccc caguggucuu uugaaucugu uagguagggu gcagccugcg     900
   ggcuugguca ccuaacaggu guugccagga ccugccccaa gccggauucu cccacucccu     960
   cuuucaaccc cgccucuucc uccuccuuga gacaccccca cccccucagg aggcuagaug    1020
   cagaucugua ucuuguguug cugacuaucg gccugaagcu ggguagauug guggggguug   1080
   auccggaugu aggaugucca aguggagaaa caggauuuga auggagcugg aacaaacgcc    1140
   caguccugac ugcugccacc cuucuuccuc cacccccaac ccaucccuuc ccaggcuucu   1200
   ugcaaacaga ggcagaagug gccauauuuc uuucuuucuc ccaggugcug uaugcccuag    1260
   ggacuugaca cggggcaggc ggggugggaa cugguugggg ggggacgugc uguuuuaugg    1320
   gaagucguau gccuagccag cgguggagcu guccuggcua ggcacccagg gguggugugg    1380
   ggaaugggag aaauacuggg acuagagggu cucaagggcc agcagguggu gauagucuuu   1440
   uucuccaccc cacccucugc uccaccaccc cucugccuca gcaccccucu gcccucagca    1500
   ccccucugcc ucagcacccu cugccuccag ccacccucug ccucagcacc cucugccuca    1560
   gcacccucug ccucagcacc cucugccuca gcacccucug ccucaguguu ccugugugac    1620
   cugucuuuuc aacuagaaag ucuagaacug uacagacccc agaguuggag guggaaaggg    1680
   acacuagguc cuggagccuc cucugugggc uuuuuugacc agaugagaac guaagggccu    1740
   ccuccagcua ucccugguca uucaggugcu ucagguuugu gaccuuugcu gagacccugg    1800
   augcugcagc aaacagacau cugcuuuagc agagggacag cuugucucug ugcauccgcu    1860
   gguaggaucc uccacucugu ccaauuagcu ugcgcugcug cuggguucug aguguucucu    1920
   uacaaaauga caagguuagg gggcuggaga gauggcucag gggcuaagag cacccaugug    1980
   gcaguuugca acugucugua acuccuguuc caggggggau cugacacccu cacacggaca    2040
   aacaugcagg caaaacaccu auuggcaugg aaaaugaaaa uaaaugaccg gguuagcuga    2100
   gaaauuccuu uugagaguug ucuuuucucc aguccaggac ugcucucugg aucuuccugc    2160
   ucaguuccuc gccucccuuc cauauauggu auuuaagguu uuacuuuuuu uugcguuuuc    2220
   aauuguuuuu aauuguauuu auaugugucu cugugugugc acaugagcau ggguguccua    2280
   caaggcuaga gacaucagau guccuggagc uagaguuaca gaugguugug agccaccuga    2340
   uguaguugcu gggaacugga uuuaguuccu guggaagagc agugcaugcu cuuaaccacu    2400
   gagcaacuuc uccacgcggc ccccccccag uauacgguau uuaaaacucu auagcaaugc     2460
   uacccaaccc auuguggagc uggggauggu gagguggccu aguucccccc aacuccugga 2520
   aacaugucag aaaguacaga gugggugccu gugggaucag caugcggggg gugguggugg 2580
   ugaggugugg ggcugcuuuc ucccgaggcu accgugauag acaguuguac cuugcuggca 2640
   gccucuaccc cauuuccagg augucagucu cugcagacgu augggggacg guggggaagg 2700
   gguauacaga gccaugggug ugccugggaa uguacuccca ggaagugacu gguugaaaag 2760
   ucagcagauc uccuggggga uagaggggug ggcugaagcu cuguggguug ccucucucag 2820
   cccugaccug ugaacaggga ggcugggggu uggaggaaca cagcuucccc cuguuccugg 2880
   ggggacaugc uggacagccu uuccuagcuc ccccggccca cuggggugug gcuggucguc 2940
   ggacugagcu cuuuuggacc uggucucugc cugugagcuu ugugacucgu ugagauagca 3000
   uuugggcuga gggauuggga gucuucuguu cuuugguccu gacacccgcu uccuugucua 3060
   ggccugccac cuggcucccc ccaccccccu ccccggccua ccaaguuucu ugcuucccuu 3120
   acugaccccu ccuccucccu ccuuuguguc uccucccucu ccagagaugc ccuguauuca 3180
   agcucaauau ggaacaccag caacgagccc aggaccgcgu gaccaccuga ccggugaucc 3240
   ccuggcccuu gaguucggca agccuaccau ggaccuggcc agccccgaga cagcaccugc 3300
   cgcaccugcu acacugccca gcuucagcac cuucauggac ggguacaccg gagaguuuga 3360
   caccuuccuc uaccagcugc cggggacgac ccagccgugc uccucagcuu guuccucugc 3420
   cuccuccacg ucuucuuccu cauccucggc caccuccccc gcuucggcgu ccuucaaguu 3480
   ugaggacuuc cagguguacg gcugcuaccc gggcacccug agcggcccau uagaugagac 3540
   ccuauccucc agcggcucug aguacuaugg cagucccugc ucagcccccu cgccaucuac 3600
   acccaacuuc cagccguccc agcuuucucc cugggacggc ucauuuggcc acuucucccc 3660
   gagccagacu uaugaaggcc ucugggcaug gacagagcag uugccuaagg cuucuucagg 3720
   gccuccgcca ccuccaaccu ucuucuccuu caguccuccc acuggcccca gccccagccu 3780
   ggcccagagu ucucugaaau uguucccacc accagccacc caccagcuug gggaggggga 3840
   gagcuauucc augccagcag cuuuccccgg cuuggcaccc accucuccga accgugacac 3900
   uuccggcauu cuggacgcac ccgugaccuc caccaagucc cggagcgggg cuucaggugg 3960
   cagcgagggc cgcugugcag ucugugguga caaugcuucg ugucagcacu augggguccg 4020
   caccugugag ggcugcaagg gcuucuucaa gguauuuugu ugguucuggg ggacgaugau 4080
   aucauguugg aggugggggu ggaguggguc auccguuggg aucuguagug accucuccug 4140
   agguuucuuc ccagcugguu cuguccugca ggacgaggga caggugugug ccaucuuaga 4200
   ggcugggacu uuuuauucag cagggcacac aucucucuag ggcugcagaa agcuggggaa 4260
   gggggcagaa ggugugugug uguguggccu gcaguggugc ucagaaacag aaaaccuagu 4320
   gggcagccuc ugguucucca cagaacuugu guuucuggca cuggaugaaa ggacacaggc 4380
   agaggguuug guucuugcug gggguggacu ugggaacagg cuguguguuu gucccagugc 4440
   ugggugccug cuuggcnnnn cccaccucca nccccuuagu ccucuccucu gcgccaggaa 4500
   aagggcaggu ggacacaugc agacaccugu uagaacaggu gucuggacgg ccgugggagu 4560
   uccuagaccc uggucuuggg uucugggauc uucccuguga gguugaaacc uucuccagua 4620
   ucucccagac uccucucugc gcucuggccc uccguuucuc ccucccccua cauccaaaug 4680
   uuaggaaaau agcuaugaac agagggcgcu uuugucugcg ucggccacag gaucuggacg 4740
   guccccuccc cugggcucuc cacccccccc ccaaacccca ugcucugaca gccuguuccg 4800
   uguccccccu uccuccagcg cacaguacag aaaagcgcca aguacaucug ccuggcaaac 4860
   aaggauugcc cuguggacaa gaggcggcgg aaccgcugcc aguucugccg cuuccagaag 4920
   ugccuggcug ugggcauggu gaaggaaggu ggguggcaag auggugcccu cggcauaggc 4980
   gaccugaugg ggugggacag ccgggcucac caggaucugc accuaauucc cacucccacu 5040
   ucuagauucc agccccuaaa ugcaggugua gcuuccaccu gcuuucugga aagggugggg 5100
   ugaggagggc cuuguggggu ccccaucaug gucugaguuc ucugucucug acuucucaga 5160
   aguggggugu auagugugcc cugaagaccc uccuucucca ggucuccuuu cucaauacug 5220
   cccgucucuc ugcaguugua cggacagaca gccuaaaagg gcggcggggc cggcuaccuu 5280
   caaaacccaa gcagccucca gaugccuccc cuaccaaucu ucucacuucc cucauccggg 5340
   cacacuugga cuccgggccu agcacugcca aauuggacua uuccaaggug aggucuugcc 5400
   cgcccaucug cccugcccug agaacauaug caaugccuuu gugccuguua ggaaaggcuc 5460
   ucccuccagg gcaacaucag gaaacaagca uccucuaugu acuggcuagu uggagaaaug 5520
   cauugggaug gguguggggu cggggagcca guuacaaaca gcugccguag cccuguuuuc 5580
   cugugggaau ugacaagcac augggcccag aauagggcuc uuuugcacgc cuggaucugg 5640
   ugucccagug caugagagcu cuguaaugca gcuuguguag gcuuugcugu ggacccaaug 5700
   caggacacag cugugugaug caggccuugc uguggagcca gugcgugaca cagcugugug 5760
   gugcaggccc ucauguggaa cuuaccugcg uuccuuucag uuccaggaac uggugcugcc 5820
   ccgcuucggg aaggaagaug ccggugacgu gcaacaauuu uaugacuugc ucucugguuc 5880
   ccuggacguu auccgaaagu gggcagaaaa aaucccuggc uucauugagc uuugcccagg 5940
   agaccaagac cuguugcuag agucugccuu ccuggaacuc uucauccucc gccuggcaua 6000
   ccgguaagcu gcccaccauc cuccuagccc uggccccagc ccugcggccc cggccugccu 6060
   ggacccugag uccugacugu ucucugccuu cugccagauc uaaacccggu gaggggaagc 6120
   ucaucuucug cucaggccug guacuacacc agcugcagug ugcccguggc uuuggugauu 6180
   ggauugacaa cauccuggcc uucucacggu cccugcacag cuuggguguu gauguucccg 6240
   ccuuugccug ccuguccgcu cugguccuca ucacugguga guggcagaaa cuagacuggg 6300
   cccaaggguu gcaggaccau uggguaggua gcaucuaaca cuuuggggac cccuagagug 6360
   ccugcaacau ugggauguua ggaccugcaa agggacuuag cucuauucgc cccuaaagcu 6420
   uaaaucagcc uccgaaugac cccggacucc ucagguacga cuguaggcgc ugggcaucua 6480
   gcuuagggau ucuguuuguu uaaaaaccua gcugucaacc ucacaagaca caggaacgug 6540
   cacacaugaa uuuuucacau ucugcgcuug gauagcuauc gccauggucc agaaacagga 6600
   ccuacuucag cucuguuggg cucccuccuu auugucucug uaagagguaa ccacucugcc 6660
   agccucagug gcauuccugu cauggauuuu acugugcgua ucacagggua uggacucuag 6720
   uguuaaaugu agagguuucu ggguccuuuc ucguuccgcu cccauuucca cuuaaauuuc 6780
   uuaggggauc cuccugagua gaucaagacc aggagcugga ucuugaaagg guagggagug 6840
   auaucuacac ccagcucagu ccuuagucug uccugcgaga cuccaggauu uuagaacagu 6900
   guggagccug gaccuccaga aaaccuggau uaggcuuuug ccuuggccag ccauucagug 6960
   guuucaaaua uugaccaguc uggacagugg gcuccugugg gacgugcuag agggcugggg 7020
   aagcuuguca gggagaaggc gucaacugag cggggcugac ucuaccuucc cugcugcaga 7080
   ucgacacggg cuccaggacc cucgucgggu ggaagagcug cagaaucgca uugcuagcug 7140
   ucugaaggag cacauggcua ccguggcagg agacccacag ccggccagcu gccugucacg 7200
   ucugcugggc aaacugccug agcuucggac ccugugcacu caaggccugc agcgcaucuu 7260
   uugccucaag uuggaggacu ugguaccccc uccaccuauu guggacaaga ucuuuaugga 7320
   cacauugucu uucugacccc ugcccugaac augugugcgc acacgugcgu gcucuucugu 7380
   cacccaugug ccuuuaagcc uauagcccac ggacccccag accacccuac ccccagccug 7440
   guuuugagcu aagacugacg uaccuccuca cuccagaaga uggacagaga acucaagacc 7500
   ugggggaggg uguguauuca cgggggugac cccacuauuu gucuuauccc uccagcucag 7560
   uccuggccuu cgugugauuu uguaagauaa accauuuuua acacauacca cucuguugua 7620
   aauaagcuga cgcuacugua aauacagaaa ggaagagguu gagauggggg uugggaggaa 7680
   gggguggggc ucccaccagc ugggcgagcc uccaacucga gaucucuucc gcucuccuuc 7740
   cauguguaca uaacugucac ucaagaaggu gauugacaga uucugauuua uauuugugua 7800
   uuuuccugga uuuauaggau gugacuuuuc ugauuaauau auuuaauaua uugaauaaaa 7860
   aauagacaug uaguugaaa 7879
<210> 89
   <211> 586
   <212> RNA
   <213> Homo sapiens
<400> 89
   ggcacgaggc ugugcccgcc augucugcua cccaucacaa gaccucccug ccucagggcg      60
   uccgcguggg cacugucaug agaauucgag gcuugguccc ugaccaggcu ggcagguucc     120
   auguaaaccu gcuaugcggu gaggagcaag gagcagaugc cgccuugcac uuuaacccga     180
   ggcuggacac uuccgagguu gucuucaaca ccaaacaaca aggcaaaugg ggccgugagg     240
   agcgaggcac cggcaucccc uuccagcgug ggcagcccuu ugaagugcuc cucaucgcca     300
   cagaggaagg cuucaaggcu guggucgggg augacgaaua ucuccacuuc caccaccggc     360
   ugccgcccgc ccgcguucgc uugguggaag ugggcggaga cgugcagcug cauucauuga     420
   auaucuucua agcaaaggac ccaagggccu uugcccgguu acggguuggg gguuuuuuga     480
   ucccacaaga aagguuuugg aucggccaau aacauuuuuc uguuguucug aaaaauuaaa     540
   aaaaaaaaaa aaaaaaaaaa aaaaaaaaaa aaaaaaaaaa aaaaaa                    586
<210> 90
   <211> 540
   <212> RNA
   <213> Homo sapiens
<400> 90
   gccacauugu ugcaccaacu ccagugcugg agucucagga caccacaggc uacccggagu      60
   uguucugcuu uggagauccg agggcaggag caucacgcca gugacucuga uaggugcgau     120
   cgccggauug gaacagaacu gucauuuuuu uccgaaguug agccuuagug acccagugag     180
   ugaaguuagc gacgggacgc uaagcagcua gaccggucgg caggagugag acuuagggua     240
   ccuucuagua guugugauua aaaaaauuga aaaaaagaaa aaaaaaaacc cuguuucugg     300
   aaacuugagg cccucagcug gugagccauc gugguuaagc uucuuugugu ggcuccugga     360
   gucuucgauc ccagccggac acccgggccu gguuucaaag cggucggaca gcgcugccug     420
   cuccaucggu agcgcucgag ccucgguuuc ucuauuuggc cccgacucgc cgcaacaaga     480
   ugaucgccuc gcauaugauc gccugcuuau ucacggagcu caaccaaaac caagugcaga     540
<210> 91
   <211> 2000
   <212> RNA
   <213> Homo sapiens
<400> 91
   cccagcuguu ucguccguac cuagaccgcg guuauggcgu cguucacggu gaaggccuau        60
   cuucugggca aggaggaggc gacccgcgag auccgccgcu ucagcuucug cuucagcccg       120
   gagccggagg cggaagccca agccgcggcc ggcccggggc ccugcgagag gcugcugagc       180
   cgaguggcug ugcuguuccc cacgcugagg ccuggcggcu uccaggcgca cuaccgcgau       240
   gaggaugggg acuugguugc cuuuuccagu gaugaggagc ugacaauggc uauguccuau       300
   gugaaagaug acaucuuccg caucuacauu aaagagaaga aggagugccg gcgggaacau       360
   cgcccaccau gugcucagga ggcaccccga aacauggugc accccaaugu gaucugugau      420
   gguugcaacg ggccuguggu gggaacucgc uauaagugca gugugugccc agacuacgac      480
   cugugcagcg ugugcgaggg gaagggccug cacagggaac acagcaagcu caucuuuccc      540
   aaccccuuug gccaccucuc ugauagcuuc ucucauagcc gcuggcuucg gaagcugaaa      600
   cauggacacu uuggcuggcc uggcugggag augggcccac cggggaacug gagcccacgu      660
   ccuccucgug caggggaugg ccgcccuugc ccuacagcug agucagcuuc ugcuccacca      720
   gaagauccca augucaauuu ccugaagaau gugggggaga guguggcagc ugcccucagc    780
   ccucuaggca uugagguuga cauugaugug gaacauggag ggaagagaag ccgccugaca      840
   cccacuaccc cagaaaguuc cagcacaggc acagaagaca agaguaacac ucagccaagc      900
   agcugcucuu cggaagucag caaaccugac ggggcugggg agggcccugc ucagucucug      960
   acagagcaaa ugaaaaagau agccuuggag ucggugggac agccagagga acagauggag     1020
   ucgggaaacu gcucaggagg agacgaugac uggacacauu ugucuucaaa agaaguggac     1080
   ccaucuacag gugaacucca gucucuacag augccagaau cggaagggcc aagcucucua     1140
   gaccccucac aggaaggacc cacagggcug aaggaagcug cccuauaccc acaucuccca     1200
   ccagaggcug auccccggcu gauugagucc cucucccaga ugcuguccau ggguuucucg     1260
   gaugaaggcg gcuggcucac caggcuccua cagaccaaga auuacgacau cggggcugcu     1320
   cuggacacga uccaguauuc gaagcacccu ccaccauugu gauagugcug uggccaagcc     1380
   ccacccccuu ugucuuguag uugcaucacg uagagcagca gggcuucuau agauaggccc      1440
   agugucuugg cauucuugua gaaucuucag gugggaaugu gugaugccuu uucaggcaau     1500
   aggaaagugc augaggagag uuuugaaugu gcauaugcug acgccugaga acagacccag     1560
   guacccgugg cugagcugag cuuccucugc uuccccuagg ccuggccucu gcagggaacu     1620
   gcagcacaca cugcacuccc accugcucuu gccgccagca uugcaccagc aguccagaau     1680
   uccugccuga caacccgugu uuccuuuauu aaaagugauu aguacaacug cuaguuauuu     1740
   ucaacaaaua aagccauuau guuaagaggg gacuguccau agugagugaa agguggcagg     1800
   caggggccua cagcuccuag ggaauggaga auucauguga agccgaauga aggaucuuau     1860
   cuuauacugu cccccuuucu aauggccacu cuuuaguguu ugugucuaau guuaaugcuu     1920
   aaagcacagg acccccaugu agcuuccucu gacuugguuu guaaguaacc uguaauaaaa     1980
   ugccauaugc acuuuaacca                                                 2000
<210> 92
   <211> 2649
   <212> RNA
   <213> Homo sapiens
<400> 92
   gagggaauug cgggaccccg ucuggggaag cucccgccgc cccggggugc ucagcucucu 60
   gucucccuug acccagguac agucaugucg ggcuucgacg acccgggcau uuucuacagc 120
   gacagcuucg guggcgaccc cggugcggaa gagggccagg cccgcaaguc gcaacugcag 180
   aggcgauuca aggaguuccu gagacaguac cgagugggca ccgaucgcac gggcuucacc 240
   uucaaguaca gagaugaacu caagcggcau uacaaccugg gugaauacug gaucgaggug 300
   gagauggagg accuggccag uuuugacgag gaacuggcug accacuugca uaaacagccg 360
   gccgagcacu uacagcugcu ugaggaagcu gccaaggagg uggcagauga ggugacccgg 420
   ccccggccag cuggagauga gcugcuccaa gacauccagg ucaugcucaa gucagaugcc 480
   agcccgucga gcauucggau ucugaaguca gacaugaugu cacaccuggu gaagaucccu 540
   ggcaucauca uuucagccuc ugcaguccgu gccaaggcua cucguaucuc cauucagugc 600
   cgcagcugcc acaacacccu caccaauauc gccaugccca ggccuagagg gcuaugccuu 660
   cccaggaagu gcaauaugga ucaggcuggg cgcccaaagu gcccacugga uccauacuuc 720
   aucaugccug acaagugcaa guguguggac uuccagacuc ugaaacugca ggagcugccu 780
   gaugcagucc cucaugguga gaugcccagg cacaugcagc uuuauuguga cagguaccug     840
   ugugacaagg uuguuccugg gaacaggguc accaucaugg gcauuuauuc caucaagaag 900
   uuuggcuuga accccagcaa gggccgggac aggguaggug ugggcauccg gagcucguac 960
   auccgagugc ugggcaucca gguggacaca gauggcucug gccgaagcuu ugcugggucu 1020
   gucagcccac aggaagagga ggaauuucgu cgccuggcug cccuccccaa cauauaugag 1080
   cucaucucca agagcauuuc ccccuccauc uuugggggca uggauaugaa gaaggccauu 1140
   gccugccugc uuuuuggggg uucccggaag aggcucccag auggacucac ucgccgaggu 1200
   gauaucaacu ugcugauguu gggagacccu gguacagcca agucucagcu ucugaaguuu 1260
   guggagaagu gcucucccau ugggguguac acaucuggga aagguagcag ugcugcaggc 1320
   uugacugccu cagugauacg ggaccccuca ucucgaaacu ucaucaugga agguggagcc 1380
   augguucugg ccgauggugg gguugucugu auugaugagu uugacaagau gcgggaagau 1440
   gaccguguug caauccauga ggcuauggag cagcagacca ucuccauugc uaaagcuggg 1500
   aucacuacca ccuugaacuc ucgcugcucu guucuggcug cagccaacuc aguguuuggc 1560
   cgaugggaug agacaaaagg ggaggacaau auugacuuca ugccuaccau cuugucccga 1620
   uuugauauga ucuucaucgu caaagaugag cacaaugagg agagggacau gaugcuagcc 1680
   aaacauguga ugacucugca ugugagugca cugacacaga cacaggcugu ggagggugag 1740
   aucgaccugg ccaagaugaa gaaguucauu gccuacugcc gagcgaggug uggaccucgg 1800
   cuaucagcag aggcagcaga gaagcugaag aaccgcuaca ucaucaugcg gaguggggcu 1860
   cgucagcaug agagggacag ugaccggcgu uccagcaucc ccaucacugu gcggcagcug 1920
   gaggcuauug ugcgcauugc ugaggcccuc aguaagauga aacugcagcc cuuugccacu 1980
   gaggcugaug uagaggaggc auugagacug uuccaggugu ccacacugga ugcugcuuug 2040
   ucuggcaauc ugucgggggu ggagggcuuc acuacccagg aggaccagga gaugcugagc 2100
   cgcauugaga agcaacucaa gcgccguuuu gccauuggcu cucagguguc ugaacacagc 2160
   auuguccagg acuucaccaa acagaaauau ccagagcacg cuauccgaaa ggugcugcag 2220
   cucaugcuac gcagggguga gauccaacac cguaugcagc gcaaggugcu cuaucgccuc 2280
   aagugagccc auugcccauc aacccucaag ccugaaaugc ugccaccacc cuaucuccca 2340
   gucagugcuc caaaccuccu uuugcccugc cucuccaccu cagacugcug ucugcagcac 2400
   aucugcagcc ccuggaaaug uacuuugguc uguuggcuca uacuguguuu gagugucuga 2460
   ggacucucug cucugggugu cuauccccug ucaugccuuc ucaacaagau gagucuggag 2520
   caggaacagg cccuggaaug uagauggguc uguauauugg cucccgggcc acucacugcc      2580
   aagcuucuuu guauguacag agguaauaaa gcaauugagu cccuggcugc uaaggucagu 2640
   ggacccagu 2649
<210> 93
   <211> 450
   <212> RNA
   <213> Homo sapiens
<220>
   <221> misc_feature
   <222> (389)..(389)
   <223> n is a, c, g, or u
<400> 93
   ggaugcagcc ggaagugcag cgugcgugcg guuugguggg ucgcugugug cgcuccgcgu 60
   gugcagccgc gugggccaug gggcggcggg cgcggggccg gcgguuccag cagccgccgc 120
   agccugaggg cgaggaagac gccagcgacg gcggcagaaa gcgaggccag gcgggcuggg 180
   aagguggcua ucccgagauc guaaaggaga acaagcucuu cgagcacuac uaucaggaac 240
   ucaagaucgu gccagaggga gaaugggacc aauucaugga gucacuccga gaaccucucc 300
   cagccacacu gagaaucacu ggguacaaaa gccaugccaa agagauucuc cauugcuuga       360
   agaacaagua cuuuaaggag uuggagganc cugaaguaga uggacagaaa guugaguucc       420
   acaaccacua agcugguacc cugaagaacu 450
<210> 94
   <211> 929
   <212> RNA
   <213> Homo sapiens
<400> **94**
   gagcucaaau ucuggcuuuc uauuggguac gauauauuaa ccaaugggag aaacacaaac    60
   agaauaccuc caguuaguau aaaugcuugc uguucaguug cagaauuuac uauauauucu   120
   uuuccuuucu cugcuuugcc uuuacugaua cuuaaacgca uacaugucug gacgcggaaa   180
   gcaagggugc aaggcccgcg cuaaggccaa gacccgcucc ucccgggccg gccugcaguu   240
   ccccgugggc cgcgugcacc ggcugcuccg caagggcaac uacucggagc gcgugggcgc   300
   cggcgccccg guguaccugg cggcugugcu ggaguaccug acggccgaga uccuggagcu   360
   ggcgggcaau gcggcccgcg acaacaagaa gacgcgcauc aucccgcgcc accugcagcu   420
   ggccauccgc aacgacgagg agcucaacaa gcugcugggc cgcgugacca ucgcgcaggg   480
   cggcguccug cccaacaucc aggccgugcu gcugcccaag aagaccgaga gccaccacaa   540
   ggccaagggg aaguaaucug gcgauugucu guacugccca guugaaaguu aaccaaaaca   600
   aaggcucuuu ucagagccac ccacaucuuu ccauaaaaug agcugccacc ucgugaaacg   660
   uucuuccacu acaguuuuua uacuacauau gaaaaaguua cgaaguagcu uucaaucuua   720
   guaaauugau uuuaauacug uuagucccug cgauaaaucu uacgaccuuc cuuaguuuga   780
   gucaaaagug uguaagagau gaaaccuuua gaacauacua uaaauuuuua guagaaauuu   840
   ggcacccagg uuugucauuc acgucacgau ugucuagagc auaaugguag uaagggcuaa   900
   gggccauuaa aucccacuuc cauaguuuc                                     929
<210> 95
   <211> 325
   <212> RNA
   <213> Homo sapiens
<400> 95
   uuuuuuuuuu uuuuuuugga aaugaaggua auuuauugaa acugguuuug ggacaggcga    60
   guggacaacu guugaaagga gcuagcgcac agccgggugg gagcgggugc uuagccacag   120
   auccuaucug aggcccaacu uuuucuuuuc cuucugcuuc uuacggacca cauccagguu   180
   ccgguccuuc cacaugcuuu ugcgaagcuu gauggggcgu gagcccacau acuucccauu   240
   caucucucgc auggcgcgca cauagucacu ggggucuuug aagcugacaa agccauagcc   300
   cuugguuuug ccugugcgcu uguca                                         325
<210> 96
   <211> 367
   <212> RNA
   <213> Homo sapiens
<400> 96
   uucggauccu ugccaauaua uguaucccau uuggaauggu gaucuuaaaa ugugagugca        60
   ugcauacuau cuuauuuaag auacuugcac cccacccacu cccaucuccc gaagcuggaa       120
   cacugccaac uagguccuua agaaucacgc aauuaacaca agguugggug cugcuaauuc       180
   uucaugaaaa uccaaacacg uuaagggacc agggagaugc cacugccccc cugaauuuuc       240
   aucaaaaaua gacacguuua uguaaacaga acuauuuucc auauucauag ugacuuuuua       300
   aguauuugag ccuaaagauu uugaucucca uuuuuauaac uauuuaaauu guucacaauu       360
   auuacau                                                                 367
<210> 97
   <211> 418
   <212> RNA
   <213> Homo sapiens
<400> 97
   cggccgccgc cgcccccaca cugccccgcg uugacgagcg ccgcgacggc aaggacagcg        60
   ccucgcuuuu cgugguggcu cggauccuag cggaccucaa ccagcaggcg ccggcgcccg       120
   cccccgcgga acgcagagaa ggggccgcug cgcgcaaggc gaggaccccc ugccgccugc       180
   cgccugcgcc cccugcgccg ccacccggcc cagagcccgc cuccccggga caagcaggcg       240
   cgccggccgc gccccccagc cccgcgugga gcgagccgga ggcggcauug gagcaggagc      300
   ccggccccgc ggggagcggc gagccuggcc ucagacaaag gggucgggga ggccggagcc       360
   gcgcggaccu cgaguccccg cagaggaagc acaagugcca cuacgcgggc ugcgagaa         418
<210> 98
   <211> 1552
   <212> RNA
   <213> Homo sapiens
<400> 98
   cgucaguccc cgccaccucc ccggccccgc gcgcccggau cggcccuacg gccucgucuc 60
   gcccggccuu gcgcgccggg accgccgcga ucuccucucc ccgccgcccu ccggcuggcc 120
   cugccugcug cggcgcgaug aaugacuuug gaaucaagaa cauggaccaa guggccccug 180
   ucgccaacag uuuucguggg acacucaagc gccagccagc cuuugacacc uucgauggcu 240
   cucuguuugc ugugcucccu ucucucagug aagaucagac acuccaagaa gugcccacgg 300
   gccuggauuc ugucucccau gacucggcca gcugcgagcu gccuuugcuc acucccugca 360
   gcaaggcagu gaugagccaa gccuuaaaag ccaccuucag uggcuuccaa aaggagcaac 420
   gacgucuugg cauccccaaa aaccccuggc uguggagcga gcagcaggug ugccaguggc 480
   uucucugggc caccaacgag uucagccugg ugaaugugaa ccugcaccag uuuggcauga 540
   acggccagau gcuguguaac cucggcaagg agcgcuuccu ggagcuggcg ccugacuuug 600
   ugggugacau ccucugggaa caucuagagc agaugaucaa agagaaccaa gaaaagacag 660
   aagaccaaua ugaggaaaac ucucaccuca acgcgguucc ucauuggauc aacagcaaua 720
   cauuaggcuu cagcauggaa caggcuccau auggaaugca ggcaccaaac uaccccaaag 780
   acaaucuccu ggacagcaug ugcccgccau cggccacgcc ugcagcucug ggcucugagc 840
   uccagauguu gcccaagucu cggcucaaca ccgucaaugu caauuacugu uccaucagcc 900
   aggacuuccc cagcagcaac gugaauuugc ucaacaacaa uucuggaaaa cccaaggacc 960
   acgacucucc agagaacggu ggggacagcu ucgagagcuc cgacucgcug cugagguccu 1020
   ggaacagcca gucgucccua cuggauguac agcggguacc uuccuucgag agcuuugagg 1080
   aggacuguag ccagucucug ugccucagua agcugaccau guccuucaag gacuacaucc 1140
   aagagaggag cgacccaguc gagcaaggca aaccaguuau uccugcagca guacuggcug 1200
   gcuucacugg aagcggacca auccaguugu ggcaguuucu ucuggagcua cucucugaca 1260
   aguccuguca aucuuucauc agcuggacgg gggauggaug ggaguucaag cuugcugacc 1320
   ccgaugaggu ugcccgccgg ugggggaaga ggaaaaauaa accaaagaug aacuacgaga 1380
   agcugagccg gggcuuacgu uacuacuacg acaagaacau cauccacaag acuucgggca 1440
   agcgcuacgu guaccguuuc guaugugacc ugcagaacuu gcugggcuuc acuccggagg 1500
   aacugcaugc cauccugggc guccagccug auacagaaga cugagggccu ca 1552
<210> 99
   <211> 4043
   <212> RNA
   <213> Homo sapiens
<400> 99
   auggcucuaa caacgcugcu cuugguggug gcggccgccc ugacccugau cgagacccgc      60
   gcgggugagu gcggggucgg gagggaaaca gccccugugc cgcguccccg cuucgcccac     120
   cggaccuccg ccccuucucc acccgagccc cgagcccugc uccacucccg gcccgcguac     180
   ccgaccgggg ucccgggagg aggucggggu cucaccgcgc gccgccccca ggcccacacu    240
   cgcugcggua uuuccacacc gcuguguccc ggcccggacu cggggagccc cgguucauca    300
   ucgucggcua cguggacgac acgcaguucg ugcgcuucga cagagacgcg gaaaauccga     360
   ggauggagcc gcgggcgcgg uggauggagc aggaggggcc ggaguauugg gagcgggaga     420
   cacagaucgc caagggccau gagcagaguu uccaagggag ccugaggacu gcacagagcu     480
   acuacaacca gagcaagggc ggugagugac cccgggucgg aggucacuac cucuccacgu     540
   cccgaaacag aggccgguga ggucccgggu gcaaaguccg agguucagga gcagaacuga     600
   cccaggacug gauucccuuu caguuuggag gaguccgcgg ugggggggug gggggggggc      660
   ggaggaggga cugaccacug ggucccgcag gcucucacac acuccagugg auguauggcu     720
   gugacauggg guccgacggg cgccuccucc gcggguaccu gcaguucgcc uaugaaggcc     780
   gcgauuacau cgcccugaac gaagaccuga aaacguggac ggcggcggac auggaggcac     840
   agaucacccg acgcaagugg gagcaggcug guauugcaga gagagaccgg gccuaccugg     900
   aggucgcgug aggcuccgca gauaccugca gcucgggaag gagacgcugc ugcgcacagg 960
   ugcaggggcc gcgggcagcu ccucccucug cccucaggcu ggggcucagu ccuggggaag 1020
   aagaaacccu cagcuggggu ggugccccug gcucagaggg gagagaguga cccugggucu 1080
   ccugaucccu caucacagug acugcacuga cucucccagg gcucagccuu cucccuggac 1140
   agugcccagg cugucucagg agggaaggag agaauuuccc ugagguaaca acagcugcuc 1200
   ccuucaguuc cccuguagcc ucugucagcc auggccucuc ccaggccagg guucucagcc 1260
   uacacccacu gucuguagac acugacuccu guccugcuga gugugucagc ccuuacaccu 1320
   caugaccuga agucuccuuu acccgauggg agacauggac aucuacacua ggcugguucc 1380
   cccaguuucu agaacuuucc aaagaauaca gucuaccaga uccuucccug ucuguggggu 1440
   uugcauccuu ugacacccaa uucuaucuau uccugcaaug gugaauaguc acaugagcca 1500
   uuauggguua cccuaaacaa auacuuuucu uguguuuuuc cccucucguu uucuuuuuau 1560
   cuuuacuuuu uuuuuaaggg uauuauguug cuuauaaucg guuuuucuuc ggcacuggaa 1620
   ugauauugcu cucucucccc accauacccc cacccccgcc uauaucauuu guaucaguag 1680
   cccuggcugu cguggaacuc acucuguaga ccaggcuggc cuugaacuca gaaaucagcc 1740
   ugccucuguc ucugccucug ccucccaagu gcugggauua aaggcuuggg ccaccaccac 1800
   ugggcagaag aaagguuccu gugagcuuaa aauguuuucu ggcagaauua accauccaga 1860
   ucacuccuga uaucccugug ccccaccaag uuacagugcu cccccuggug aaucagaacu 1920
   uggacucuga gagacagggu cuucugcaau ccaggccuga gugagaggga agaccacaca 1980
   cccugugagc ccacuguguu ccagugagug cugcacuggg guccacagca cauuccaggg 2040
   auccugugug acacaucugu accuuguccc ccagagucag gggcugggag ucauuuucuc 2100
   uggcugagug ucagagguuc accacauuuc ugcuacacac ucccugaugg cuguuuacuu 2160
   ggacugacag uuaauguugg ucagcaagau gaccacagug guuuagucuc aaugguguca 2220
   cucuuccagu agcauauggu ccugauuucu aauuuagaua cgaacucaaa cacauaugaa 2280
   auuucuuauu uuccauucca ucuuccauua uauagcuacc uaucucgugc uauugaacau 2340
   cacauaagga ugaccauguu gacccacugg cucaugugga uucccucuua gcuucugagu 2400
   ccccucagga aaaugugcag uccugugcug agggggccag cucugccugc aggucacuag 2460
   ugccaugaca guuaaagugu ucauacagac acauaguuca uuguaauuac ugauuuagcg 2520
   uugucuuggc aguuuucagu uugcaugcau uuauuuauuu auuuauuuau uuauuuauuu 2580
   auuuaaugca uggaaguaca cuguugcugu acugaugguu guuugccuuu gugugguugu 2640
   ugggaauuga auuuuuuuuu uuaggaccuc ucuuugcucu ggucgacccu gcucacuccg 2700
   gucaacuccu augggucaac ucugcucauu cagucccugc uuguucuggc ccaaagauuu 2760
   auuuauuauu uauuauacau aaauacacug uagcugacuu cagaugcacc agaagagggc 2820
   gucagaucuc auuacagaug guugugagcc accauguggu ugcuggaguu ugaacucagg 2880
   accuucaaaa gagcagucag ugcucuuacc cucugagcca ucuccccagu ccucaguuug 2940
   ucuucuuaau ugugcgauuu cuugaaucuu ccaaacagau cccccaaaga cacaugugac 3000
   ccaucacccc auaucuuaug augcugucac ccugaggugc ugggcccugg gcuucuaccc 3060
   uguugacauc acccugacuu ggcaguugaa uggggaggag cugacccagg acacggagcu 3120
   uguggagacc aggccugcag gggauggaac cuuccagaag ugggcagcug ugauggugcc 3180
   uuuuggggag gagcagaauu acacaugcca ugugcaccau gaggggcugc cugagccccu 3240
   cacccugaga ugggguaagg aggguguggg ugcagagcug uggucaggga aagcuggagc 3300
   auucugcaga cucugagcug gucagggcug agagcuggga ucaugacccu caccuucauu 3360
   uccuguaccu guccuuccca gagccuccuc cauacacugu cuccaacaug gcgaccauug 3420
   cuauuguggu ugaccuugga gcuguggcca ucauuggagc ugugguggcu uuugugauga 3480
   auaggaggug aaacacaggu aggaaagggc agggucugag uucucucuca gucuccuuua 3540
   gaagugugcu cuaaucauua augggaaacc caucuacacc ccacauugcu accuucucca 3600
   acuggguccu cugucaguuc ugggaacuuc caagaucuuc cuugaacucu cacagcuuuc 3660
   cuucucacag guggacaagg aggggacugu gcuccagcuc cagguuagug uggggacagg 3720
   auuguccugu ggacauugca gugaagcugg agauguuggg gagcucuggg aacccauagu 3780
   aacucuucca gagaaaucuu ccagggccgc aguuguccaa uaugaauaca uauauguaca 3840
   uaugcauaua cauuuuuuac ccuuggcagg gacagcuccu agagcucuga uagaucucuc 3900
   ccagguggua aaggugacac ucugggaccu gauuggggag gggcaaugug gauaugauug 3960
   gguuucaggg acuccacgaa uccccucuga gugaguggug gguuguugga auguugucuu 4020
   cacagugaug ggucgugucc cuc 4043
<210> 100
   <211> 211
   <212> RNA
   <213> Homo sapiens
<400> 100
   uuaaaucugg agggauuuuu cacacagccu aucuuuuuag gugugccuuu cccauauuuu 60
   auuaaacucg aguuguuguu uuaaaaaaac agcagcauua ucaaagacac aucuguacaa 120
   acauuuuaca aaagagaacu cucuaggauc agcuacauca aggacaagca gaaaaauaga 180
   ugcaguccaa caaagacauu gaaaaugacu u 211
<210> 101
   <211> 8252
   <212> RNA
   <213> Homo sapiens
<400> 101
   ggucgcuuuc ugucuguaga ggcggcuugc cacccgagca gagggucgug aaguuccgac      60
   cggaccgguc cacagagguu caucuggaga gguggguccc cuccgaggug aaaggcgccg     120
   cugagaaacg cccccacccc ccgugguuca agugguucag cccaagaacu uuucauucau    180
   aaaaaagacc agacuccgag aggcgcgagu gagucagaac cgcagccgcc aacgcggacc     240
   cuaccgaaca uccagcccag ggcauguacc gagacuacgg ggaaccggga ccgagcuccg     300
   gggcuggcag cccguacggu cgccccgcgc agcccccgca agcucaggca cagaccgccc     360
   agcagcaggu gagacuggcc gaaucgucgg gggggggggg accugaguuu ggacagcauc     420
   agggaugcug ggauuagucu aguuugcucc gggauuugga cuggggcccc gagcagcauc     480
   ugacucuggu ggucgcgacc gaggauccug cacguucugu guggucgggg aaccuaugua     540
   cccgguggcc aaggggacga gcgcagcggg aagcgcgaau aucugcgaau uccccuucuc     600
   gcucgcccgu aucucccuag cugacugucu uucugccccu ccgucuccgu uacggucuua     660
   cauuuccucc uaucugcccc uaauacgcug ucccucuaaa uaccugcccc aucccugccu     720
   ggacaggauc agagguguuc uccaucucca guuaauaacu gggaccuggg gucugggcac     780
   auagagacgg gguccaucag aacucagccg ggacagagaa uucuuagcag ccuguccgag     840
   gcuguccgug uguugcucug guuguccgug ucccuuuauc cggucaaguc cucaucucuu     900
   ugugcgcagu auagagccca ugggccccag gcagugguuc cgagggguuc cuggagacca     960
   cgaaguguug ggaugugcgc ggggucaccu gcccggccac acucgcgcuc cacauucucg    1020
   gcacccgacg ucucucacug cuggauaggg gcacuugaga gguugcaggu guccauuucc    1080
   ugucgagggg ccgcgagcac gugucgccag gggagggaag gagcugcguc cguuucgccg    1140
   agucacagcg ggccgaguca cugaggcuga gucacccugg gugccccucc cuuccuggcc    1200
   ccaaacggcc ccuaaggacc gacgaccugg gagcgagaga ugccccuggc agugcuucua    1260
   gcccagaacg ggggucacug agaugcuggg ucccccagua uugggcuggg gacauagcug    1320
   uccagacuug ccuaagcaug ugaggugcuu ccuggacugg agggccccca cauccuuagc    1380
   ucacagagcu ugaacccagu uuucucuccc agaacgcuga gcccccaccc ccaccgacac    1440
   ucaaucccaa cacaugccuc agauuucugc aagaaaagga aggaaaggau gccagacccc    1500
   uuauaggagg cuuuuacucu cuucacuuua uuucauggac uuaaaaaacc ccaucaauuu    1560
   ugaacuuuca aguuuuuaag ucgacagcua ggcaugcauu uaaucccagc auucaggagg    1620
   cagaggcagg cagaucucug ggaguuugaa gccaauuugg ucuacaaagu gacuuccagg    1680
   ucagcuagag cuacauaaua gagauccugu cucaaaacuu aaaaaauaaa aaacaaaaac    1740
   aaaagccgau uaaaacucug auuucugagc uaugagagcu ggcuucucaa cagcaaugga    1800
   acguugaaug auguuaauaa cagggaaacu gagacuaaau aacaugcccc agucucaaag 1860
   cccaucaaug gccaagcucc aagcugaugc uggacuccca agcucuggug cuacaugucu 1920
   auagucuugg ugccugggag guagaggcag aggaaucgug aguuuagguc uagccugagc      1980
   uaugugagau ccugcccacc ccacccccgg cccccaaaca acaaauucuc auccugauuc      2040
   ucagaauugc cuugggagcu agaagcugaa aguaugccca ucugugagga cugggucuca 2100
   aaucuuaguu ucuacuuacu agcugugggu cuaugggcaa cugcucuccu gucugaaaac 2160
   aggauuaugg cagcugugug agucacuauu uguuaaauag uuggaacagu guuacgcagu 2220
   ccauacuuga uuuaaaacaa aaaaccaaac uaucucuggu ggggaaacag acagacgaag 2280
   agagacauuu ugugaccugc ccaaaaucac acagcuccug aacaaguaag uuucugguug 2340
   ccaaauguug cuccuugugg ucucccaaaa cugguaucua cacugaggug aagggagaca 2400
   gaaguccagc cucugucccc gggaagcccc ucaguccaca cagaccuuau cauuucccuu 2460
   cuuaucucuc agaaguucca ccuugugcca agcaucgaca gcagcagcca ggaacugcac 2520
   uggauggugc agccucauuu ccugggaccc acuggcuauc cccgaccucu ggccuauccc 2580
   caguacaguc ccccucagcc ccggccagga gucauacgag cccuagggcc accuccgggg 2640
   gugcgucgca ggcccugcga gcagguaaga aacagcgaug uuucacuuuc cauagcccgu 2700
   agggguccua cuagacaggg acaggaucuu gcuacgaggg aauuucuuau ucagcauuga 2760
   aguuccugag aggccaagaa ggagguaaaa ggucaccuuu gagucaagga aggcuuccug 2820
   gagaaggcua cacguuaacc uaaaccacgg auaggauuug cguauggaag cugaaaagaa 2880
   ucuucuggga ggaggguugg aggcacagaa uugaggugaa ggggacagag uguuaaguga 2940
   gcaugucucc acugucugac gugcacaggg uggaaggaag ccugaugcug gcuuuguacc 3000
   ucggggugac ucuucuuuuc aacagucacg gaaucucagc cuauuucuuu uaauuaucac 3060
   aaaaagugag ugggcagugg uggcucacac cuuaaauuuc agcacugggg aggcagaggc 3120
   agguggaucu cuggguucaa ggccagccug guuuacagag ugaguuccag gacagccaag 3180
   gcuacagaga aacccugucu caagaaaggg aaaaaaaaag ugaaugggaa auguauuuca 3240
   auuuuuccau cuuccaucag aggaagugaa gcauagaggg guacucacuu guccagauuc 3300
   auacaaaugu gaguaaugag acaggacugg uccuggccug aggucuacuc aacucccaaa 3360
   gucagagcuu aaugagccac ugucucaaug ggagcucaca gaagccugca gagggaguga 3420
   gcugagaacu uuugccuccc ugaaugccuu ucuaaaauaa agaagggugu guuuugguug 3480
   guuuguagag guggggucuu gagagccucc ugccuuccca agugcuagga guauaggugu 3540
   augcuagaua cccgacaggg augacggauc uuuuaggcca uagcacuuuc uuuccucucc 3600
   cugaaguacu gagacugagu cuagugcagg gaggccucca uacuaaaaag cuggguguac 3660
   ccguaauccc agaacucgga aaaucaagac aggaggagcc auucaaguca cgcucagaaa 3720
   cauggagagu uuaaggucag ccugggauau auaaacccua ucuccgauaa ccaaacaaca 3780
   aucaaagcaa accugacuua aguguuaagu aggaaagaau augcuguaag ucaucagccu 3840
   ggcuggggga cugaccgccu cugagggacu gaagaaccug cuucaagccc ggagcccugc 3900
   acucaacccc uaguggaugc acacacagau ccuugccaug auggcucgaa gagggauggc 3960
   aagacccugg gagauaugug agauuggcca gagaagagcc agcaacaggc uucccagcaa 4020
   gggaacagcu uaccucugug aucagcuggg gcgugagcaa gaggcaagcc cagggugaau 4080
   ccuuccuuua gcccuguccu gaggagacac cuuuugacca cggguacuag uggguuggag 4140
   cugugagcug uggguaggug gcuuccccuc ggugugcucu ggaacuugaa caaaucacuc 4200
   auccuuccug agcuuccuca caugugagug ugcagagauc uggaugggug acucagcagc 4260
   ggcucuggcu gcuccuuaga ggauccgggu uccaaucuca gcacccacgu ggcaacucac 4320
   uguccacaac gucccccaaa gguucugaug cccucuucug gccuccacca gcacugaaug 4380
   cacaaggugc ucauacaaac acacaggcaa aauacucaga gguaaauuug uucuuuuuuu 4440
   ucuuuugaga caggguuucu cuguauagcc cuggcugucc uagaacucac ucuacagacc 4500
   aggcuggccu caaacucaca gaggcaucug ccugccuccc aaguguuggg accaaagaug 4560
   uaugccauca cuauaagccu uuuuuuuuuu uguaaauuuu auuuaugaau gagugcuucc 4620
   auguauaccu ucaugccagg agagggcauc agauccuauu auaggugguu gugagccacc 4680
   guguguggcu gggauuugaa cucaggaccu cugaaagagg agcucuuaac ugcuaaaaca 4740
   ucucucuagc cccagucuac auauuuuaaa ucuuuuuuua agauugauuu auuuauuaua 4800
   cauaaguaca uuguagcugu cuucagacac uccagaagag ggcgucagau cuuguuaugg 4860
   augauuguga gccaccaugu gguugcuggg auuugaacuc aggaccuuug gaagaguagu 4920
   caaugcucuu acccgcugag ccaucucacc agccccuuuu uuaaucuuua aaaaaaaaag 4980
   ggggggggcc uggagagaug gcucagccgu uaagagcacu gaaugcuuuu ccagaggucc 5040
   ugaguucaau ucccaacaac cacaugguag cucacaacca ucuguaaugg gauccaaugc 5100
   ccucuucugg uguaucugaa gacagcaagg gucuacucac auauguuaaa uaaauaaaua 5160
   aaaauuuaaa agccaggugg uguugguuca gacagcagau cucuguuuaa ggccagccug 5220
   gucugaucua caaaccaagu uccaggacag ccagggcuac acagagaaac ccucuaaaaa 5280
   accaaucuau guagggggug cuggugaaau ggcuccgugg guaaagguac uugcugcgaa 5340
   auuaaugacc ugaguucaau ccuugaaauc cacacaguag aaggagagaa ccaaccucca 5400
   agggugcuau gacacacaca cacacacaca cacacacaca cacacacaca cacacacaca 5460
   cacgacuaua uauaugaaua uaugacucua gccaggcaua guguggcaca ugccuuuaau 5520
   cacaguacuu gggaggcaga ggcaggugaa guuugaggcc agccuacaga gugaauucca 5580
   ugacagcuag aacuaugaag augaacccug ucuuuaaaaa caacagcaac auaaagaauc 5640
   uauguaggga agcuggaagg gaugcugaua cagucugcaa uccuauuacu cuagaggugg 5700
   aagccaaaga aucagggguu ucaggccggu cuugccuaua cacugagcua aaggccagcc 5760
   ugggacacau gagacuuugu cucuuuuaaa aagacaaaac aaggggguug guuagauggc 5820
   ucagugggua agagcaccca acugcucuuc ugcagguccg aaguucaaau cccagcaacc 5880
   acaugguggc ucacaaccau cuguaacgaa aucugagucc cucuucugga gugucugaaa 5940
   acaacuacag uguacuuaca uauaauaaaa auaaauaaau cuuaaaaaaa aaaaaaaaag 6000
   acaaaacaag ccagaugagg ucugugaguu ccaggccagc cuggucuaua aaucaaguuc 6060
   aaggccaggc agggcuacuc agagauucug ucuaaaauac aaagaaacaa aacuacaaaa 6120
   agcagaaaaa ggaaucuacg aaagggcugg ugaaggggug uaacucagug guagagcauu 6180
   ugccgagcua gcauguacca agccaugggu uugaucccua gcacuaagca aaagaaaagu 6240
   ccuacaaagg gcuuuguugg cauaguagau ugauucccag uagcuuuugg cacucaagag 6300
   caccuucuac aauucacagc uccuggggga agaaauccca ucuuccacag augaggaggc 6360
   ugagaguccu gugaaaagag auaaucaugu cucauacacu caggagagaa ggcuacuucu 6420
   gccugagaaa ugaaaaggcu uccugggguc ccaacaucuu aguccagucc uaagaugcgg 6480
   aagggaggaa gaucaaaguu uacagagagg gaaagcauuu caggaaagaa accagcagua 6540
   aagcuaggug ugugugagcu ggaaauguca ccaaugagau gacaagcguc cgguacagag 6600
   aagcaacugu ggaguguugg gugguggcac cuagacuaca gacugaagga aagcuggaug 6660
   accagcucag ggcagcuggu ggcucagagu cagccucauu gucucccuuc uucuauccca 6720
   accuagauca gcccagagga ggaagagcgc cgcaggguga gacgcgagcg gaacaagcua 6780
   gcagcugcua agugcagaaa ccgaagaaag gagcugacag acuuccugca ggcggugagc 6840
   aucaucccca ggcccggacc cacagagccc caagaggggu cucggcuccc aagaacacaa 6900
   aagacccaaa auuacuccuc aggacucugu cauccucccu gccugugggg aaguccugga 6960
   aaaaggauaa gggaaagugg cuuaaauauu guuugucggg cuucgaggca gagucgaaga 7020
   ugguaggcag caauucuccu aagaugcccc cgucugaugg gagucauggc cauuuucucc 7080
   cagaggcuca cgggagggag uugcagucca gacuuguugg ggaugacagg cacagucccu 7140
   acuccagccu gaggcuuggg gaucuuuagc cuucauuuuc cuaucuuucu gcuaauccug 7200
   uaaaggagac cgacaaauug gaggaugaga aaucggggcu gcagcgagag auugaagagc 7260
   ugcagaagca gaaggaacgc cuugagcugg ugcuggaagc ccaucgcccc aucugcaaaa 7320
   ucccagaagg agacaagaag gacccaggug guucuggcag caccagcggu gcuagcagcc 7380
   caccagcccc cggccgccca gugccuugca ucucccuuuc uccaggaccc guacuugaac 7440
   cggaagcacu gcauaccccc acgcucauga ccacacccuc ucugacuccu uuuacuccga 7500
   gucugguuuu caccuauccu agcacaccag aaccuugcuc cuccgcucac cgaaagagua 7560
   gcagcagcag uggcgacccc uccuccgacc cccugggcuc uccuacacuc cuggcuuugu 7620
   gaggcaccca gccacauccc uugcuggugc uacuccaagc cauccccuuu cucccauuga 7680
   uccagcaggc cuggaccaua cccuugcccc aaaccagcag aucuuuuauc ucuuccgacu 7740
   agaacaaaca cauuaugcuu ugauguagag ccagcuugga ggggaucccc aaagcugcuc 7800
   acuguuuuuc uagagcuggc cuaucauaau uugcacaaaa uuagaggaaa auauguuccc 7860
   ucugccagag aacgccuggc agcccagacu uuguagaucc ccaggggucc uuugacaccc 7920
   uuaccccuug cagaccacuu ucccacacca cgucacuuuc uucauguuau ccagccuacu 7980
   cuacaccuag acagaaggug cccuuugacu agccuagaac acuaacucac acagcaucaa 8040
   cagccagcag caccggacau ccugcaggcu ccuccugaau ggcacaacgc aggaggcgcc 8100
   aggggcuucu gugaggagcg gagcugcacu cccuagcucu gagaagcgcu uagcuucagg 8160
   guauccgagc cuccaccgca agggcagcug cuauuuauuu uccuaaagag acuauuuuua 8220
   uacaaaccuu ccaaaaugga auaaaaggcu ug 8252
<210> 102
   <211> 1938
   <212> RNA
   <213> Homo sapiens
<400> 102
   ccguggguuc cuaauggugc uauugacgac gggcacaaag ucucauaauu uuagaaacuu      60
   cacuuauuuu gugacccacc uaguaugggg caaaggcagg agcuucggac uuccugucuu     120
   cccucuaucc cuuugaauag cgucucggga gugucccuaa guaaccauug caguuuugug     180
   uccgugugcu cuguugaaau cucugugaug uguguauggu uuucacguga acggaggcag     240
   gcagagcccg gucugugggu ucuagugugu ucuuuaucuu cuuggaaguu ugugcaauga     300
   cuucccuccu guuuccugcg cauaccucca aguaaguacg ugaauguguu uucugugguc     360
   ccuugaucuu gcuggacuuc acuaaggagg guggcuggac caucugcuac gugucugaga     420
   gccccucucu agcuaccuca uguccggacg ugagcuuacu cccaauguuu uuacccacaa     480
   gcauucacua cucccaggaa agaacgugca cuggggcacc uagagaauaa ccaaucaacg     540
   cuccgccuac auuuugcuuc cuccuuggaa uuuccagccc cuugcagcca acugcucccc     600
   agcugcgaag ggcggaguuc cccccggccc ggccccuucu uuggcucuau aaguagcucu     660
   gcuuugcggg ggauuugcac uccucuacac ucucugcaca acgucuaaau uaugugccac    720
   ucgcgcaacc aucuccacac caugacuggc cugagggccc cuucuccagc ucccuccacc     780
   ggcccggaac uccggcgggg cucugguccc gaaauuuuca ccuucgaccc ucucccggag    840
   cgggccgugg uguccaccgc gcguuugaac acuucucgcg ggcaccgaaa acgcagccga     900
   agggugcucu acccucgagu ggugaguauc gcccgagugg gcaucaggag gucgcgucgc 960
   ccuggaacuu guagguaaca acuaggagca gggaccuucg aucugacguu ucccucuuuu 1020
   aucugcucag guccggcgcc agcuaccaac cgaggaaccc aacauugcca agaggguccu 1080
   cuuucuccug uucgccauca ucuucugcca gauuuugaug gcugaagagg gugugucgca 1140
   gccccuggcu ccggaggaug cuaccagcgc cgugacaccu gagcccauuu cugcgcccau 1200
   uacugcgccc ccgguccucg agccuuugaa ccugaccucg gaguccucgg acuaugcgcu 1260
   ggaucuuaaa gcuuuucucu agcaacaucc ggcggccuuc uaaacgcgau gggucacagu 1320
   ccgaagaaac aaaggcacca uggaugggua ccuggugcga gagaacguau cccaaacugg 1380
   gauuucuaag gcaacgcuaa cucagaacac uaccgccaag agacaccgcg gguccuggcu 1440
   aggcccacug gggacggaca gagacuuucu ccgugucuaa uuaauauuua uguauuuaug 1500
   uauauccucc uaggugaagg agggguguau guaauauuua uucuaacuua ugcaggggug 1560
   cgagauaugc cucccugcug uaacacagau auuuauuacg auuuauaggg ucgguaagac 1620
   agaguugugg gagggaggac ccggguggua ggacucccag cuuggggauu agucuggggg 1680
   ggguguaaua agauuagggg uaacacuccg ucuuccagca cuucaacucu guagucuguu 1740
   guaaggcuuu ggaagacccu ugggaauccg gccuuugaug ucuucugguu gcuucucagg 1800
   ggcagcugca ggagucuugg guccauggau ugucagaggg cggcugucug gggucgccua 1860
   guauguaugu ucugugaaca cgaauaaacu ugauuugccu gucauuauua ucugcaguuc 1920
   ucgaagugua ucauucag 1938
<210> 103
   <211> 3036
   <212> RNA
   <213> Homo sapiens
<400> 103
   gacagcggag cgcgguggcg ucgacgucua gugucucagu gcucccgucu guggcuaacu 60
   aagcagccag cagccaggca gcucgcgacc ugcggccagg cagccaacca ugcucaacuu 120
   cggcgcuucu cuccagcaag cuucggaggg gaaaauggaa cuaauuucug aaaagcccag 180
   agaggggaug caucccuggg acaaagcuga gcagagugac uuugaagcgg uggaagcgcu 240
   cauguccaug agcugcgacu ggaagucuca uuucaagaaa uaccuugaaa acaggccugu 300
   cacaccagug ucugauaccu ccgaggauga cagcuugcuu ccagggacgc cugaccuuca 360
   gacaguccca gcauuuuguu uaacgccacc uuacagcccc ucugacuucg aacccuccca 420
   agggucaaau cugacugcau cagcgccauc uacuggccac uucaaaucuu ucuccgaugc 480
   ugccaagccu ccaggcgcca cuccuuucaa agaggaggaa aagaauccuu uagcugcccc 540
   uccucuuccu aaggcucaag ccaccagugu cauccgucac acagcugaug cccaacugug 600
   caaccaccag uccugccccg ugaaagcagc uagcauccuc aacuaucagg acaauucuuu 660
   ccggagaaga acccacggaa auguugaggc uacucgaaag aacauacccu gugcugcagu 720
   gucaccaaac agauccaagc cugagcccag cacagugucc gauggugaug agaaggcggg 780
   cgcugcacua uaugacuuug cugugccuuc cucagagaca guaauuugua ggucucagcc 840
   agcuccuucg uccccagugc agaagucagu acuggugucu ucaccuacag uauccacugg 900
   gggagugcca ccccugccug ucaucugcca gaugguuccc cuuccugcca acaacucucu 960
   uguuagcaca guugucccca gcacuccucc uagccagcca ccagcugucu gcucaccugu 1020
   guuguucaug ggcacucagg ugccugaggg caccgucgug uuugugguac cccagcccgu 1080
   ugugcagagc ccaaggccuc caguggugag ccccaguggc accagacugu cucccauugc 1140
   cccugcuccu ggauucucuc cuucagcagc aagggucacu ccucagauug acucguccag 1200
   aguaagaagu cacaucugua gccacccagg guguggcaag acuuacuuua aaaguuccca 1260
   ucugaaggcc cacgugagga cacacacagg ggaaaaaccu uucagcugca gcuggaaagg 1320
   cugugaaagg agguuugcuc gcuccgauga acuguccaga caccggcgga cacacacagg 1380
   ugagaagaag uuugccuguc ccauguguga ccgucgguuu augaggagcg accauuuaac 1440
   caagcaugcc cgacgccacc uaucagccaa gaagcugcca aacuggcaaa uggaaguuag 1500
   caaguuaaau gacauugcuc ugccuccgac cccugcuucc gcacagugac ggccagaaga 1560
   uggagacgca gaauaaacuu uggucagagu caggagccag ugauggugcc aagugcuucu 1620
   gcaaggcugu ggcccuccaa aagggccuaa aguagaagcc cuggccuggg ggaggccccg 1680
   ccugggugaa augacaagaa gugcuucagc cacaggcagg ucacagagga cagggcucag 1740
   uucuuaccac agagagagag gagaacccuu uuauuccucc cuuauuuuag ucuggaaagu 1800
   uucggcugag gugagcgcag cacagguuuu gaaucacaua cacauugggg acuuuguuuu 1860
   ugccauuuau acuugagacc agcuuugcag ugugauucuu ucaaaggauu gguuucaaga 1920
   auauagaggc uggaaauuac gguacagaaa uggagcuaga aaaugaguuu guguuacaca 1980
   gagaugucau cuucuccuag aguuaucuug uuucuuauuc cuagucuuuc cagucaaauc 2040
   cguggaugua gcuaaguaua ucuaaaacuc auuuuuccac uauuguuggu auuugaaguu 2100
   gaacagcugu acauugcugu gggggagcca aaggauugga acccucauua auuuaauugc 2160
   uuggaaaugc agcuaaaauu cuucuuuggc auuuuguuuu gaaaguuuag gcauuuuacu 2220
   cuacuuuaga uuuuaguuug cuugcaguuu uuuguguaga uuugaaaauu guauaccaau 2280
   guguuuucug uaggcuuaaa auacacugca cuuuguuuag aaaaaaaucu ggagaugaaa 2340
   auauguauua uaaagaagag augucaagaa uuugagauaa cuccuugaga aaguuggcuu 2400
   uaugucauca gcaaaggaca cuuaacguca agcauacacu gugguuuuuu uguuuuuuug 2460
   uuuuuuuuuu ucaaauuaga aaguuuaaug accguuacag auggacagug ucuuuuuauu 2520
   uauaggaguu uuucaggaug ucagaguaga uagguaggaa aauuguuauu agaacauucg 2580
   cuucuaccuu gaaaaggaug uuaauguggu cauguucuua gcaccacagu gucugggcau 2640
   cugggaaacu ccgagacuuu uuuaaagugu caugauguga ucacaccugc aguuuggggc 2700
   aucgaaucca gggccuugca ugucuucugu aagagcucuc aucgcugacc uguauccccc 2760
   gcaagagcaa ugacuuuugc uaacaguauu ucuuuucugu uguaaagugg acagaugaua 2820
   cacuuggucg caaagguaaa uuauucaaaa uccacaguga aaaccucacc acacuuuccc 2880
   auuuaaacua uuuccauauc ucagagguuu cugacaugca aacuugaacc cuugaaagaa 2940
   gaguuuucuu aaaaauuaua aaaaaucacg aguuacaauu ugcacaauau uuuuuguuga 3000
   acuuuauacc uuguuuacaa uaaagacuuu ucuuug 3036
<210> 104
   <211> 279
   <212> RNA
   <213> Homo sapiens
<400> 104
   uuuuuuuuuu uuuuuuuaaa uugccgaauu aaguucuuuu aauagauugc auauauagau 60
   guuuagccau acucuagauc aacucuuuaa gaguagaauu uuauauccaa uuuacaugcu 120
   ucagauauca ccucuguuug uuacauaagg ucuuguaucc aaauguccac uuguacacug 180
   agagcuuuag gaacaaaaaa ggacacagag agaguugcca uuuuuagcag caaugaaaca 240
   ucacuaaccc cuuuuuacau accgaauuca agucacuac 279
<210> 105
   <211> 143
   <212> RNA
   <213> Homo sapiens
<400> 105
   cggccgccgg uauuuuuuug caaguauuga gaguucugua uguuuugaaa agaguaauuu 60
   uaacguuugg gugccaagaa guggguuuuc ucagagucca uugccggcaa ugggcaagcc 120
   uggcgguacu ccucgugccg aau 143
<210> 106
   <211> 989
   <212> RNA
   <213> Homo sapiens
<400> 106
   auggcgcugg gaacgcugcu ccugcugcug gcggccgccc uggcccggac ccagaaccga 60
   gccggugagu gcagggucgg gagggaaaca gaacuuucca aacagucugc ggggaggggc 120
   gggugcggca ccggggaagc cgcgugcccg cgucgcccac cagacccucc gucucuuuac 180
   ccgcgucccu agccccgcgc ccugcucccc uccugucccg cgcauccgcc cggggucccg 240
   ggagaagguc ggggucucac cgcgcgccgc ccccaggcuc acacucgaug cgguauuucg 300
   agaccgucgu gucccggccg ggccucgggg agccccggua cgucucuguc ggcuacgugg 360
   acgacacgga guucgugcgc uucgacagcg acgcggagaa accgagguau gagccgcggg 420
   cgcgguggau ggagcaggag gggccggagu auugggagcg gaucacgcag aucgccaagg 480
   gccaugagca gugguuccga gugagccuga ggaaacugcu aggcuacuac aaccagagcg 540
   cgggcgguga gugaccccgg gucggagguc aggccccucc acuucccgac acagggacgc 600
   ugacguccug guucccaagu cugagguucg ggaacagaac ggacccggga ccgguuuccc 660
   uuucaguuug gaggaguccg cgggugggcg gggcugaccg cggggucccg cagguucuca 720
   cacacuccag gagauguaug gcugugaugu gggaucggac gggcgccucc uccgcgggua 780
   ccggcagucc gccuaugaug gcugcgauua cauugcccug aacgaagacc ugaaaaccug 840
   gacugcgaag gauguggcag cgcugaucac cagacgcaag ugggagcagg auggugcugc 900
   agaguauuac aaggcuuaca uggagggcga gugcgugcag ucgcuccgca gauaccugga 960
   gcucgggaag gagacgcugc ugcgcacag 989
<210> 107
   <211> 4484
   <212> RNA
   <213> Homo sapiens
<400> 107
   auggcgucaa caaugcugcu ucugcuggug gcagucgccc agacccugau cgagauccgc        60
   gcgggugagu accggguccg gagggaaaug gccucugagg aaaggggagg gggcggcacg       120
   ggggaagccg cgucccggcg ucgcccaccu gacccuccgc cccuucucca cccuagcccc       180
   gcgcccugcu ccccucccgg cccgcucacc cgcggggguc ccggaaggag uucggggucu     240
   caccgcgccc ugccuccagg cccacacuug cugaguuauu ucuacaccuc cgugucccgg       300
   ccgggccuug gggagccccg guucaucucu gucgguuacg uggacaacac ggaguucgug       360
   cgcuucgaca gcgacgcgga gaauccgaga uaugagccgc gggcaccgug gauggagcag       420
   gaggggccgg aguauuggga gcgggaaaca cagaaagcca agggcaauga gcagauuuuc       480
   cgagugaacc ugaggacccu gcucagcuac uacaaccaga gcgcgggcgg ugagugaccc       540
   cggaucggag gucacgaccc cuccacgucc caaaacaggg gcccgagacg ucccgggccc       600
   caaguucgag guucugagca gaacggacgc gggacugguu ucccuuucag uuuggaggag       660
   ccgcgggugg gcggggccgg ggcguguggg cggggcugac cgcgggaucc cgcaggcucu       720
   cacacuauuc aggugaucuc uggcugugaa gugggguccg acgggcgccu ccuccgcggg      780
   uaccagcagu ucgccuacga cggccgcgau uacaucgccc ugaacgaaga ccugaaaacg       840
   uggacggcgg cggacauggc ggcacagauc acccgacgca agugggagca ggcuggugcu 900
   acagagaaaa gcaaggccua ccuggagggc gcgugcgugc agucccuccg cagauaccug 960
   gagcucggga aggagacgcu gcugcgcaca ggugcagggg ccgcgggcag cuccucccuc 1020
   ugcccucggg cuggggcuca guccugggga agaagaaacc cucagcuggg gugaugcccc 1080
   ugucucagag ggagagagug acccuggucu ccugaucccu caucacagug acugcacuga 1140
   cucucccagg gcucagccuu cucccuggac agugcccagg cugucucagg agggaaggag 1200
   agaauuuccc ugagguaaca acagcugcuc ccuucaguuc cccuguagcc ucugucagcc 1260
   auggccucuc ccaggccggg uucucagccc acugucugua gacacugacu ccuguccugc 1320
   ugaguguguc agcccuuaca ccucaggacc agaagucgcc uuuaacugau cggagacaug    1380
   gacuacccua cacuaggcug auugccucag uuuccugaau uuucaaaaga auacauucuc 1440
   ccagaucccu cccugucugu gggguuucca ccccuucgac aaccuaauuc ucucuauucc 1500
   uauaguggug gucacaucag cccuuauggg guacccugga ggaauaucaa uaguggaauu 1560
   ucuucuucuu cuucuucuuc uucuucuucu ucuucuucuu cuucuucuuc uucuucuucu 1620
   ucuucuucuu cuucuucuuc uucuucuucu uccuucucuc ucucucucuc ucucucucuc 1680
   ucucucucuc ucucucucuc ucucucucuc ucucucucuu cuucaguuuu ugagauaggg 1740
   uuucucugua ugcccuggcu guccuggaac ucacuuguag accaggaugg ccucgaccuc 1800
   agaaauccgc cugccucugc cucccagugc ugggauuaaa ggcguguggc caccuugccc 1860
   agccuuucuu auuuucuuua cuuuuuuuuu uuuuuuugga ggggguaauu uuguuucuag 1920
   ucaucuuuug ucuuuugucu gcacuggagu gauccuguuu cucccugccc uuauauuauc 1980
   auguguauca gucuccacag gugccaggga aguuaagaca aguuaaauca ggguucucuu 2040
   uaaaggagag auuccuguga acuuagacug uuuccuguca gaacuaaaca uccagaagcc 2100
   uccugcucuu ccucuguccc acaaguuaca gugcuccccc ccccccccca gugaaucugg 2160
   acuuggacuc ugagagacag ggucuucugc aauccagggc uggagugaga gggaagacca 2220
   cacacccugu gagcccacug uguugcagug agugcugcac ugggguccac agcauacucc    2280
   agggauccug ugugacacac cuguaccuug ucccccagag ucaggggcug gaagucauuu 2340
   ucucuggcug agugucagag guugacacca uuucugcuac acacucugug auggcugcuc 2400
   acuuggacug gcgguuaugc uaguuagcaa gaugaacaca gugguuuacg ucucaguugu 2460
   cacacccuuc caguggcaua uggcucuaau uucuacuuuu gauacgaacu caaacacuua 2520
   uuaaauuagu uaguuuccau uccaucuucc auucuagucc auucaugcua aagaacauca 2580
   cauaaggacu gccaggauga cccacuggcu cauguggauu cccucuuagc uucugagucc 2640
   cccagaaaaa ugugcagcug aggaaaccag cucugccugc aggucaccag ugccaugaca 2700
   guugaagugu caaacagaca cauuguucag ugucaucagu gauuuaacug ugccuugugu 2760
   agauuucaga gggucuuguu aauuguggua cuuuuuuuug uuuuuuguuu uuuuuuuuuu 2820
   uuguuuuguu uucaagacag gauuuuucug uauagcccug gcuguccugg aacucacauu 2880
   guggaccagg cugaccacga acucagaaau cugccugccu cugccucccg agugcuggga 2940
   uuaaaggcgu gugccaccac caaccagcua aucgugagau uucuuucuuu uuucuuuucu 3000
   uuucuucuuu uuuuuuuuuu uuuuguuguu guuguuuugu uuuuugagac aggguucucu 3060
   guguaucccu ggcuguccuu gaacucacuu uguagaccag gcuggccuug aacuuagaaa 3120
   uuugccugcc ucugcuccgg agugcuggga uuaaaggcau gugccacuac caaccagcua 3180
   auugugggau uucuuaaauc uuccacacag auccuccaaa ggcacaugug acaugucacc 3240
   acagaucuga cggugauguc acccugaggu gcugggcccu gggcuucuac ccugcuaaca 3300
   ucauccugac cuggcaguug aauggggagg agcugaccca ggacauggag cuuguggaga 3360
   ccaggccuuc aggggaugga accuuccaga agugggcauc ugugguggug ccucuuggga 3420
   aggagcagaa uuacacaugc caugugcacc augaggggcu gccugagccc cucacccuga 3480
   gaugggguaa ggagggugug ggugcagagc uggggucagg gaaagcugga gccuuuugca 3540
   gacccugagc ugcucagggc ugagagcugg ggucaugacc ucaccuucau uuccuguacc 3600
   uguccuuccc agagccuccu ccauccacug ucuccaacau ggcgaacgua gcuguucugg 3660
   uuguccuugg agcuuggcca ucauugcagc ugugguggcu uuugugauga agagaaggag 3720
   acacacaggu aggaaagggc agagucugag uuuucucuca gccuccuuua gagugugcuc 3780
   ugcucaucaa uggggaacac aggcacaccc cacauugcua cugucuguaa cugugucugc 3840
   ucucaguucu gggaacuucc uagugucaag cucuuccuug aacucucaca gcuuuccuuc 3900
   ucacaggugg acaaggaggg gacuaugcuc uggcuccagg uuaguguggg ggacagaguu 3960
   guccugaggu cauuggagug aagcuggagu uguugggugc ucugggaacc cauaauagcu 4020
   ucucuguugu aauccucugg uggccugugu cagaucuugc uauagauaua ucuuuguaua 4080
   uauuuuuccc uaggcaggga cagcucccag agcucugaua uguuucucuc aagauuguaa 4140
   aggugacauu cuauggccug auugcagagg ggcacugugg acaugguugu guuucaggga 4200
   cucccacaau ccccugugag ugguggguug uugggauauu gucuucauug ugguguuccc 4260
   ugacccucau ucucuaucau gaagacagcu gccuggagug gacuuaguga cagccagugu 4320
   gaccuugggu cuucauuuuu cuuuagagaa cagcgccuga uguucccugu gagccuaugg 4380
   gcucaaugug aagaauugug gagcccagcc uucgccuaca caccaggacc ugucucuugc 4440
   auugcccugu guucccuucc accgccaacc uuccgggucu gcag 4484
<210> 108
   <211> 385
   <212> RNA
   <213> Homo sapiens
<400> 108
   uuuuuuuuuu uuuuuuucag aauaauugca gacaaauucc auuuauuuuu cuaaaaaccu        60
   cauuaucuaa aauuuauaca gccucacauu ccuaaaccac cucuggcacu uuucuugaau       120
   uaagucaagg cguacacagc uccgaaagaa aaauagagau ccgguuccag gaagauggcc       180
   augaggacuc gcagauaugu cuccucggac cuggaagcgu gucagccaug ggauccacua       240
   auccaccauc cgguacacag ggucacccac ucucaggcuc ccaagcuucu ccaccgagaa       300
   auacauccca aaaaguggag augacuggua uauacucuuc acagaaggau cacacaggcg      360
   auagcucuuc agggucucca guggc                                             385
<210> 109
   <211> 2247
   <212> RNA
   <213> Homo sapiens
<400> 109
   cucuagauga auacacucuc augagggcca ccuucucugg uaguucaggu cgccucugcc      60
   cauccuuccc ugcguccucu cccaaagugg ccuacaaccc uuacccagag gacuauggag     120
   acauugagau ugguucucac aagaguucca gcaguaaccu gggggcagau gauggcuaca     180
   ugcccaugac cccuggggca gcccuuagga gugguggucc caauagcugc aagagcgaug     240
   acuacaugcc caugagcccc acaagcgugu cugcucccaa gcagauccug cagccacgcu     300
   uggcagcggc cuugcccccu uccggagcag ccgugccagc acccccuuca ggggugggca     360
   ggaccuuccc aguaaacgga gguggcuaca aagccagcuc cccagcggag agcuccccag     420
   aagacagugg guacaugcga auguggugug gcuccaagcu gucuauggag aacccagacc     480
   cuaagcuacu ccccaacggg gacuaccuca acaugucccc cagcgaggca ggcacugcag     540
   ggaccccacc ugacuucuca gcagcuuugc guggaggcag ugaaggccuc aaaggcaucc     600
   cgggccacug cuacagcucu uugccccgcu cuuauaaggc ucccuguucc ugcagcggag     660
   acaaugacca guaugugcuc augagcuccc cugugggccg gaucuuggaa gaggagagac     720
   uggagcccca ggccacccca ggggcuggca ccuuuggggc agcugguggu agucauaccc     780
   agccucauca cucagcagug ccuuccucca ugaggccgag ugccaucggu ggccgcccug     840
   agggcuuccu gggccagcga ugucgggcag ugcggccuac acgccuaucg cuagagggac     900
   ugcagacccu ucccagcaug caagaguacc cucuacccac agagcccaag agcccuggcg     960
   aguacaucaa cauugacuuu ggugaggcag guacccgucu gucuccgccu gcccccccac    1020
   uacuggcauc cgcggccuca ucuucuucac ugcucucagc uaguaguccu gcuucauccc    1080
   uggguucagg aaccccaggc accagcagcg acagccggca gcgcucucca cucucugacu    1140
   auaugaaccu ggacuucagu ucucccaagu cccccaagcc uagcacccgc aguggggaca    1200
   caguaggcuc cauggauggc cuucucucuc cagaggcuuc auccccauac ccaccacugc 1260
   ccccacgucc uuccacuucc ccuuccuccu uacagcagcc ucugccaccu gccccgggag 1320
   accuauaccg ccugccucca gcaucagcug ccacuuccca gggucccacu gcuggcuccu 1380
   caauguccuc cgagccuggg gauaauggug acuauaccga gauggccuuu gguguggcug 1440
   caaccccgcc acaaccuauc guggcaccuc caaagccaga aggugcccga guggccaguc 1500
   ccacaucggg cuugaagcgg cuaagucuca uggaucaggu aucuggggug gaggcuuucc 1560
   uucaagucag ccagcccccu gacccccacc ggggugcuaa ggucauccgu gcagacccac 1620
   aggggggacg ucgucgccac aguucagaga ccuuuuccuc uaccaccacc gucaccccag 1680
   uguccccauc cuuugcccac aauuccaagc gccacaauuc ggccucugug gaaaaugucu 1740
   cacucaggaa aagcagugaa ggcagcagua cccugggagg aggugaugag ccgcccacau 1800
   ccccaggaca ggcacagccc uugguggcug ugcccccagu gccacaggcu aggccgugga 1860
   accccgguca gcccggagcu uugauuggcu guccuggagg cagcaguucu cccaugcgca 1920
   gagagaccuc cguggguuuc cagaacggcc ucaacuauau cgccaucgau gugagaggcg 1980
   agcagggguc cuuggcgcag ucucagccgc agccaggaga caagaacucc uggagccgga 2040
   cccguagccu uggggggcuc cucggcaccg ucggaggcuc uggcgccagc ggagugugug 2100
   gggguccagg cacuggagcu uugcccucug ccagcaccua ugcaagcauc gacuuccugu 2160
   cccaucacuu gaaggaagcc acagucguga aaggugaggc ccuuugaccu ugaggauggg 2220
   ggagggagag uggaguauug gguggcu 2247
<210> 110
   <211> 315
   <212> RNA
   <213> Homo sapiens
<400> 110
   uuuuuuuuuu uuuuuuucag cugugaacua uuggauuuga gacaggaaca gaacaaaucg 60
   acgggccaga ggagggugga gagagcacga gugguuuaaa uaggggagga ugggagcaug 120
   gcgguggggg ugggggaaga guuauuuaca agaaggcuca gggggccaga ggcucaucuu 180
   ggaauauuuu auaacaauau auauaagauu cugguuugcu uuuccuuuuc gucucguaaa 240
   ggagagagaa uugcauaguu cgauucuguc caagggggca gcugcauaug gucggccggg 300
   cgggucacug gucgu 315
<210> 111
   <211> 481
   <212> RNA
   <213> Homo sapiens
<400> 111
   uuuuuuuuuu uuuuuuuaag ugucaccacu ugugacaguc agcauguuac uaucagcucc        60
   agccgcagcg uuuuuaaggc guuauagauu aggcaggcaa uacaaggaac acgauuaaga       120
   aacugacacg uaccacacga gcaauuucca gaggcuccuc uucugcggug cacacguaac       180
   agugcucuug uugacauuca gacaguucug agggccacuc ugagaggcgc cuuccuguuc       240
   ucaccugaca aggauauugu uugguuuggu uugguuuggu uuugccuuac uauggcuuuu       300
   cuuucaacua cauuuugugu caugcuuguu agcuaacuca aauuuugucu uuguauauuu       360
   acuacuguaa aauuagaaua auuuacuguu caucucaucc ucugucacug auggaaccua       420
   gagacgccac aagagccacu gccgugacau accucacaag cuacaucccu guccucaaaa       480
   u                                                                       481
<210> 112
   <211> 379
   <212> RNA
   <213> Homo sapiens
<400> 112
   cggccgcggc caccggccug cgccaagcug cugcugcggc agccaguacc ucggugaagc        60
   ccauuuucag ucgcgaccug aacgaggcca agcggagggu gcgcgagcuc uaccgcgcuu       120
   gguaucggga gcgugccgaa caccgugcac uuaaugcagc uggauaucac ggugaaacaa       180
   ggacgggaua aaguccgaga aauguucaug aagaaugccc augucacaga ccccagagug       240
   guugaucugc uggucauuaa gggaaagaug gagcuccagg aaaccaucaa aguauggaag       300
   cagcggacac acguuaugcg guuuuuccau gaaacagaaa caccaaggcc aaaggauuuc       360
   uuauccaagu ucuauaugg                                                    379
<210> 113
   <211> 3733
   <212> RNA
   <213> Homo sapiens
<400> 113
   gcaccugagc gcgggugccu ggcgcgcccg augggaucgu ugagaggccc ucgacggaaa       60
   gucccaaacu cggaucgcau ucagccaaag ugaggcggcg ccauggagcu ccgagcgcug      120
   cugugcuggg cuucccucgc cacugcuuua gaagagaccc uguugaacac aaaacuggaa      180
   acggcggauc ugaaaugggu gacuuacccu caggcagagg gccaguggga ggagcuaagc      240
   ggccuggaug aggaacagca cagcguccgc accuaugagg ugugcgacau gaagcgucca      300
   gggggccagg cucacuggcu gcgcacuggc ugggucccaa ggcgaggugc uguccacgug      360
   uaugccacga uacgcuucac caugauggaa ugccuguccc ugccgagggc cagucgcucc      420
   ugcaaggaga cauucacugu cuucuauuac gagagcgaac gugauacggc cacggcccau      480
   acgcccgccu ggauggagaa ccccuacauc aagguggaca caguggccgc agaacaucug      540
   acucggaagc gcccuggagc ugaagccaca gggaaaguua auaucaagac gcugcgccug 600
   gguccucuca gcaaagcugg cuucuaccug gcuuuccagg accaaggagc cugcauggcu 660
   cugcucuccc ugcaucucuu uuacaagaag ugcuccuggc ugaucacgaa cuugaccuac 720
   uuccccgaga cggugccucg ggagcucgug gugccggugg cagguagcug cguggccaac 780
   gcggucccua ccgccaaccc cagccccagc cucuacugcc gggaagaugg ucaaugggcu 840
   gagcagcagg ucacgggcug cagcugcgcg ccaggguacg aggcugcgga aagcaacaaa 900
   guaugcagag ccuguggcca gggaaccuuc aagccccaaa uaggagacga guccugccug 960
   ccgugcccag ccaacagcca cucgaauaac auugggucuc cugucugccu gugucgaauu 1020
   ggguauuacc gggcccgcuc agacccccgg aguucaccuu gcacuacccc acccucugcu 1080
   ccaagaagcg ugguucacca uuugaauggu uccacccugc gccuggagug gagugcuccc 1140
   cuugaguccg gaaggccgag agaccucacu uaugcuguuc gcugccgaga gugccguccu 1200
   ggggguuccu gcuugcccug ugggggcgac augaccuucg accccggucc ucgagaccug 1260
   guugagcgcu ggguggcaau ccgagggcug cguccugaug ucaccuauac cuuugagguu 1320
   gcugcuuuga augguguguc uaccuuagcc acuggaccac cuccuuuuga gccugucaau 1380
   gucaccacug accgugaggu gccuccugca gugucugaca uccgagugac ucggucguca 1440
   cccagcagcu ugauccuguc augggcuauc cccagagcac ccaguggggc cgugcuggac 1500
   uacgagguca aguaucauga gaagggcgca gagggcccca gcaguguucg uuuccugaag 1560
   acaucagaaa accgagcuga gcuccggggg cugaagcggg gagccagcua ucugguccag 1620
   guacgcgcac gguccgaggc uggcuacggu cccuucggcc aggagcauca cagucagacu 1680
   caacuggaug agagcgagag cuggcgggag cagcuggccc ugauugcagg cacugcgguu 1740
   gugggugugg uccugguccu gguggucguc aucauugcag uucucugccu caggaagcag 1800
   agcaauggga gggaaguuga guacucggau aagcaugggc aguaucucau cgggcacggu 1860
   accaaggucu acauugaucc uuuuacuuac gaagacccua augaggcagu gagggaauuu 1920
   gccaaagaga ucgaugucuc cuaugucaag auugaagagg uaauuggugc aggugaguuc 1980
   ggcgaggugu gccggggucg gcugaaggca ccagggaaaa aggagagcug uguggccauc 2040
   aagacucuga aggguggcua caccgagcgc cagagggcug aguuccugag cgaggccucc 2100
   aucaugggcc aguucgagca ucccaacauc auccgccucg agggcguggu caccaacagu 2160
   gugccgguua ugauccucac ggaauucaug gagaacggag cccuggacuc cuuccugcgg 2220
   cugaacgacg ggcaguucac agucauccag cuggugggca ugcugagggg caucgccucg 2280
   ggcaugcggu accuggcuga aaugagcuau guccaccgag accuggcugc ucggaacauc 2340
   uuggucaaca guaaccuggu cugcaaggug uccgacuuug gccucuccag auucuuggag 2400
   gagaacuccu cugaucccac cuacacaagu ucccugggag gcaagauucc cauccgaugg 2460
   accgccccug aagccauugc cuucaggaag uucaccucug ccagugaugc cuggcgcuau 2520
   gggaucguca ugugggaggu caugucuuuu ggggaacggc cauacuggga caugagcaac 2580
   caggauguga ucaaugccau ugaacaggac uaccggcugc cuccuccucc agacugcccc 2640
   accucccucc accagcucau gcuggacugu uggcagaagg accggaaugc ccggccccgc 2700
   uuuccccagg uggucagcgc ucuggacaag augauccgga aucccgcuag ccucaaaauc 2760
   guggccaggg agaauggcgg ggccucacau ccacucuugg accaacggca gccucacuac 2820
   ucugcuuucg guucuguggu cgaguggcuu cgagccauca agaugggaag auacgaggaa      2880
   aguuuugcag cggcuggauu cggcuccuuu gagaugguca gucagaucuc ugccgaggac      2940
   cuucuccgaa uuggagucac ucuggcagga caccagaaga aaaucuuggc cagugugcag 3000
   cauaugaagu gggaagcuaa gccaggagcc ccugguggga cagggggacc agcccagcag 3060
   uucugaccuc caaggacuca ccaccguggc agauucuucu uuccgggagg cagaguuggg 3120
   uggggacuca caagaugagc cccucccccu cgucacagcc uucccauugg auugcacuuu 3180
   gaacagaggg ggucggagac acagauuugg ggaaccgugc cauaugggau cauacaugug 3240
   cccuccaggc ggggaacccc aaacucagag ugagucuuuc ccucaagacu gggcaaagaa 3300
   acaucccuac gucucuaacc ucccaucuuc ccagaggcuc ucuccccaag cgccuuccac 3360
   cucaacgggc augucccugc agaccaaaga gaaaggguga ccagccugcc aacuugggag 3420
   uggaaaaugc cgucccagga ggcaggaagg ggcugucagg acccggugau guaaucauug 3480
   gguuuugaug uccugacuug cugucaccac caaaggcaau cauuuuuccc uuguaaaugc 3540
   cccuccccuc aucugccuuc auauugaagg uucugaaguu uuacuguuuu uuauuuuguu 3600
   aauuuuuucc uccuuccccc cucccucccc uucuugucca gauuuugugu guuaaagggc 3660
   accugguucc acuaucuccu guugggaaca aggacccauc gauauguucu agaacagugc 3720
   cuuggaaaug cca 3733
<210> 114
   <211> 560
   <212> RNA
   <213> Homo sapiens
<220>
   <221> misc_feature
   <222> (107)..(107)
   <223> n is a, c, g, or u
<400> 114
   ggcagcgaga cgagcucacg gucgaggaua cgggugaagc gggacaggag caggagccgg 60
   agccgggcca aagcagaagg ugcaggucgg cgccggcggu ugggcangac ccagucaagc 120
   cggacaguga ggagcggaug cagacggcac gaccauggcc acgauggugc uuccucgaga 180
   ggagaagcug agucaggacg agauagugcu gggcaccaag gcggugaucc aggguuuaga 240
   gacccugaga ggggagcauc gugcccugcu agcuccccua gcuucucaug aagcaggcga 300
   ggcugagcgg gcucacagga gcgcugccuc cuccugcgcc gcucccugga ggccaucgag 360
   cuggggcuug gggaggcuca ggugauccug gcauuaucaa gccaucuggg ggcuguggag 420
   ucagagaagc agaagcugcg ggcucaggug cggcccuggu acaagagaac agugguugcg 480
   ugaggagcug gcagggacac agcagaagcu cagcgcagug aacaggcggu ggcucagcug 540
   gagaagagag cagcacaucu 560
<210> 115
   <211> 560
   <212> RNA
   <213> Homo sapiens
<220>
   <221> misc_feature
   <222> (107)..(107)
   <223> n is a, c, g, or u
<400> 115
   ggcagcgaga cgagcucacg gucgaggaua cgggugaagc gggacaggag caggagccgg 60
   agccgggcca aagcagaagg ugcaggucgg cgccggcggu ugggcangac ccagucaagc 120
   cggacaguga ggagcggaug cagacggcac gaccauggcc acgauggugc uuccucgaga 180
   ggagaagcug agucaggacg agauagugcu gggcaccaag gcggugaucc aggguuuaga 240
   gacccugaga ggggagcauc gugcccugcu agcuccccua gcuucucaug aagcaggcga 300
   ggcugagcgg gcucacagga gcgcugccuc cuccugcgcc gcucccugga ggccaucgag 360
   cuggggcuug gggaggcuca ggugauccug gcauuaucaa gccaucuggg ggcuguggag 420
   ucagagaagc agaagcugcg ggcucaggug cggcccuggu acaagagaac agugguugcg 480
   ugaggagcug gcagggacac agcagaagcu cagcgcagug aacaggcggu ggcucagcug 540
   gagaagagag cagcacaucu 560
<210> 116
   <211> 1649
   <212> RNA
   <213> Homo sapiens
<400> 116
   augaugugug gcgcgccauc ugccacaaug ccagccacgg ccgagacgca ggaggucgcc      60
   gaccagguga ggcugggccc aggucaggcc agucugagcc aggccugcgg agacccggcg     120
   gccucaggga ccggccugcc cagacugguc agguccugca gcggguuucc ggggcggcca     180
   caagugugac ugggagcugg gggcucugga ucugguugga agauucaggg ccgggaacug     240
   gggcgagucu ucugccgcuu gcauacaaga gggccacuca ccuauuaggg aacuagcccc     300
   gggaucggug gagggauccg guggucccag agaauucagg aaggcagugu uagaaccuag     360
   acggcaccuu uuugacuuac acccaggccu aaacaagaga aagccagacu gggcuacugu     420
   gcuugucucc ucaaaagaaa gagcuaggac uguuuagcuc aguggcagga auucaacuga     480
   uaccaccacc accaucacca acaccgcccc ucagggaaaa aaaaaaggau caaaaccaga     540
   aguuguagaa cuugcuugug ccuagucuga aacgaggggg uggaccuugg gccugggcug     600
   cccuuccugu gacuguuagc agagcagaga uucaguacaa gguaggggga gguucagggu     660
   auuagcaaga gaagaaaagu uaaacaaauc cucucuucag cucuccugcc acgccccaag     720
   cccaggaccc uguccacuaa gccuagcuga ucuugggagg uguuugcucu gaacugaagu     780
   ggccaagaag gaagugaguc agcuccauga gacccuagaa augaggaaau guuacagaca     840
   cacuggccag gcaagggaac cuuggccacg ugccacauca gcacucagga ggcagagaca     900
   ggcgagucuc ucugaauuug aagccagccc aguuugcuua guuccaugcc agccauagcu     960
   acauagugag acccuguccc cccccccccc aaaaaaaagg agcuguguug uucuuuauca    1020
   guggggccaa caguuuacca ugucccccgg aaugaggagu auugaaggcu ggcagugugu    1080
   gugugugggg gcaccugugc augaaucuaa guccuuccuu cucacccacc auccagguga   1140
   agucccagcu ugaaucgaaa gaaaaucaga aguuugaugu cuuuaaagcc auauccuuca    1200
   agagacagau aguggcuggc accaaccucu ucaucaaggu ggguacugau aguagcuugc    1260
   caugaacugg ggacauaguc ucagaguaga gcagaguguc cugcaacuuc cugcagagaa    1320
   ccccuuaagg ggacauguac auguucugag aggaugaauu ugggguguua ggguucccgg    1380
   ccuuaaagga ggagacaagg guuaucacug gcuaaguuag uggcuggugg ccuguucugg    1440
   cucaguuucu aaggcugggu uaagccugga acuggaaccu uaccuuucac ucacaugucu    1500
   gucugucugu cuuccuccag guugauguug guggagauaa augcgugcac uugagggugu    1560
   uucaaccccu cccccaugaa aacaagccuu ugacccuguc uuccuaucag accaacaaag    1620
   aaaggcacga ugagcucucc uacuucuga                                      1649
<210> 117
   <211> 334
   <212> RNA
   <213> Homo sapiens
<400> 117
   gaaaauguua agagccauca aauuucugga uauuuugcua ggaaaaugaa auucuacacu      60
   uauuuuuugu agacuuuuuu uaaaugcugu uuacaugaau uguauuuugg aaaaaauauu     120
   auacugugca cccugugaug caugaaguga uuuuauguau ggucugcuau gugggcagag     180
   gucaccuuau uccuaugauc uggaauuguu uacuuucuac aaaguaagcu uuguggggau     240
   uuugcuuuca uuuucuuugu agcugauguu auuuuaccag gugugcagca ggaauuacac     300
   cacugugugg aauuauaaau acaucccaug ugca                                 334
<210> 118
   <211> 481
   <212> RNA
   <213> Homo sapiens
<400> 118
   uuuuuuuuuu uuuuuuuccu ggugaauauu uuuauuagag gugacaguuu cccaggugac      60
   aguuuuuccc aaggaagcaa aucucugcgu cuauaaggga agaccacaga accuucacuu     120
   uguaauuuac cuguguaauu uauccaagaa cacagcacag caauugcuuu augugguacu     180
   cugaccuuaa guaacaaguu guuaacagaa aacacaucaa acaaaaggau aauucucuaa     240
   uuaucaaguc agccaucagc uuuucuuagg agagagagag agagugugug ugugugugug     300
   ugugugugug ugugugugucugucugucug ucugucuggg uaucccacuu agggccaugu       360
   gcauguuagg uaaaugcucc accacugagc uguuucuuag ccgcacuuuu cucagauuuc     420
   agguuuguuu guuuguuugu uuguuuuuua acuaggcaug aaaauaaacu ucacuucaaa     480
   u                                                                     481
<210> 119
   <211> 481
   <212> RNA
   <213> Homo sapiens
<400> 119
   uuuuuuuuuu uuuuuuuccu ggugaauauu uuuauuagag gugacaguuu cccaggugac 60
   aguuuuuccc aaggaagcaa aucucugcgu cuauaaggga agaccacaga accuucacuu 120
   uguaauuuac cuguguaauu uauccaagaa cacagcacag caauugcuuu augugguacu 180
   cugaccuuaa guaacaaguu guuaacagaa aacacaucaa acaaaaggau aauucucuaa 240
   uuaucaaguc agccaucagc uuuucuuagg agagagagag agagugugug ugugugugug 300
   ugugugugug uguguguguc ugucugucug ucugucuggg uaucccacuu agggccaugu 360
   gcauguuagg uaaaugcucc accacugagc uguuucuuag ccgcacuuuu cucagauuuc 420
   agguuuguuu guuuguuugu uuguuuuuua acuaggcaug aaaauaaacu ucacuucaaa 480
   u 481
<210> 120
   <211> 302
   <212> RNA
   <213> Homo sapiens
<400> 120
   uuuuuuuuuu uuuuuuugug gaaauuacuc uuuauugaaa aauaccagua auacugacag 60
   acuucaaaau caauuuacgg uuccagaaua caaaguacuu aauacauuuu uuuccaaacc 120
   uguuuguauc ucaaaguuag cauuuuugua aaucaagaua caaauaugau aacuucacua       180
   aaauauuuuc cagcuuuauu cuuuaaggag cuguauaacc uucaaaguca gggucccgag       240
   gucagcaggg cauggggcag aaugcaccug gcacucccug ugcagcagac ugcaaccaca 300
   uu 302
<210> 121
   <211> 1266
   <212> RNA
   <213> Homo sapiens
<400> 121
   auggccagcu uucccccgag gguuaacgag aaagagaucg ugagaucacg uacuauaggg      60
   gaacucuugg cuccagcagc uccuuuugac aagaaaugug guggugagaa cuggacgguu     120
   gcuuuugcuc cugaugguuc cuacuuugcg uggucacaag gauaucgcau agugaagcuu     180
   gucccguggu cccagugccg uaagaacuuu cuuuugcaug guuccaaaaa uguuaccaau     240
   ucaagcuguc uaaaauuggc aagacaaaac aguaauggug gucagaaaaa caagccuccu     300
   gagcacguua uagacugugg agacauaguc uggagucuug cuuuuggguc uucaguucca     360
   gaaaaacaga gucguugcgu uaauauagaa uggcaucggu uccgauuugg acaggaucag     420
   cuacuccuug ccacaggauu aaacaauggu cgcaucaaaa ucugggaugu auauacagga     480
   aaacuccucc uuaauuuggu agaccacauu gaaaugguua gagauuuaac uuuugcucca     540
   gaugggagcu uacuccuugu aucagcuuca agagacaaaa cucuaagagu gugggaccug     600
   aaagaugaug gaaacauggu gaaaguauug cgggcacauc agaauugggu guacaguugu     660
   gcauucucuc ccgacuguuc uaugcugugu ucaguuggcg ccaguaaagc aguuuuccuu     720
   uggaauaugg auaaauacac caugauuagg aagcuggaag gucaucacca ugauguugua     780
   gcuugugacu uuucuccuga uggagcauug cuagcuacug cauccuauga cacucgugug     840
   uaugucuggg auccacacaa uggagaccuu cugauggagu uugggcaccu guuucccucg     900
   cccacuccaa uauuugcugg aggagcaaau gaccgauggg ugagagcugu gucuuucagu     960
   caugauggac ugcauguugc cagccuugcu gaugauaaaa uggugagguu cuggagaauc    1020
   gaugaggauu guccgguaca aguugcaccu uugagcaaug gucuuugcug ugccuuuucu    1080
   acugauggca guguuuuagc ugcugggaca caugauggaa guguguauuu uugggccacu    1140
   ccaaggcaag ucccuagccu ucaacauaua ugucgcaugu caauccgaag agugaugucc    1200
   acccaagaag uccaaaaacu gccuguuccu uccaaaauau uggcguuucu cuccuaccgc    1260
   gguuag                                                               1266
<210> 122
   <211> 1266
   <212> RNA
   <213> Homo sapiens
<400> 122
   auggccagcu uucccccgag gguuaacgag aaagagaucg ugagaucacg uacuauaggg 60
   gaacucuugg cuccagcagc uccuuuugac aagaaaugug guggugagaa cuggacgguu 120
   gcuuuugcuc cugaugguuc cuacuuugcg uggucacaag gauaucgcau agugaagcuu 180
   gucccguggu cccagugccg uaagaacuuu cuuuugcaug guuccaaaaa uguuaccaau 240
   ucaagcuguc uaaaauuggc aagacaaaac aguaauggug gucagaaaaa caagccuccu 300
   gagcacguua uagacugugg agacauaguc uggagucuug cuuuuggguc uucaguucca 360
   gaaaaacaga gucguugcgu uaauauagaa uggcaucggu uccgauuugg acaggaucag 420
   cuacuccuug ccacaggauu aaacaauggu cgcaucaaaa ucugggaugu auauacagga 480
   aaacuccucc uuaauuuggu agaccacauu gaaaugguua gagauuuaac uuuugcucca 540
   gaugggagcu uacuccuugu aucagcuuca agagacaaaa cucuaagagu gugggaccug 600
   aaagaugaug gaaacauggu gaaaguauug cgggcacauc agaauugggu guacaguugu 660
   gcauucucuc ccgacuguuc uaugcugugu ucaguuggcg ccaguaaagc aguuuuccuu 720
   uggaauaugg auaaauacac caugauuagg aagcuggaag gucaucacca ugauguugua 780
   gcuugugacu uuucuccuga uggagcauug cuagcuacug cauccuauga cacucgugug 840
   uaugucuggg auccacacaa uggagaccuu cugauggagu uugggcaccu guuucccucg 900
   cccacuccaa uauuugcugg aggagcaaau gaccgauggg ugagagcugu gucuuucagu 960
   caugauggac ugcauguugc cagccuugcu gaugauaaaa uggugagguu cuggagaauc 1020
   gaugaggauu guccgguaca aguugcaccu uugagcaaug gucuuugcug ugccuuuucu 1080
   acugauggca guguuuuagc ugcugggaca caugauggaa guguguauuu uugggccacu 1140
   ccaaggcaag ucccuagccu ucaacauaua ugucgcaugu caauccgaag agugaugucc 1200
   acccaagaag uccaaaaacu gccuguuccu uccaaaauau uggcguuucu cuccuaccgc 1260
   gguuag 1266
<210> 123
   <211> 1287
   <212> RNA
   <213> Homo sapiens
<400> 123
   gaauuccgug ugcaaggcga ggucuguaag cuggagcggg gcagaggcug gcgggcaccc 60
   cuuccugacc gcuggugccg ccgccgccgc cuucgggagg aucagacaug gcccagaacu 120
   ugaaggacuu agcuggacgc cugcccgccg ggccucgggg caugggcacg gcgcugaagc 180
   ugcugcuggg ggccggggcg guggccuacg gcguccgcga auccguguuc accguggaag 240
   gcggucauag agccaucuuu uuuaaucgua uugguggcgu gcagcaggac acgauccugg 300
   ccgaauuuca cuucaggauc cccugguucc aguaccccau caucuaugac auucgggcca 360
   gaccucggaa aaucuccucc cccacaggcu ccaaagaccu gcagauggug aacaucuccc 420
   ugcgugugcu gucccgaccc aaugcccagg agcuccccag cauguaccag cgucuagggc 480
   uggacuauga ggagcgagug cugccgucca uuguuaauga ggugcucaag aguguggugg 540
   ccaaguucaa ugccucgcag cugaucaccc agcgggcuca ggugucccug uugauccgaa 600
   gagagcugac agagcgcgcc aaggacuuca gccucauccu ggaugaugua gcuaucacag 660
   agcugagcuu cagccgagag uacacagcug cuguagaagc caagcaagug gcccagcagg 720
   aagcccagcg ggcccaguuu uugguggaga aagcgaagca ggaacagcga cagaagauug 780
   ugcaggcuga gggggaggcg gaggcugcca agaugcuugg agaagcacug agcaagaauc 840
   cuggcuauau caagcuccga aagauccggg ccgcccagaa caucucuaaa acgaucgcca 900
   caucacagaa ccgaaucuau cucacagcug acaaccuugu gcugaaucua caggaugaaa 960
   guuuuacucg gggaagugac agccucauua aggguaagaa augagugugg acaucaagaa 1020
   ccccaccacc agagaaguug gcacacuugu ccagcuugga ggagccagcu cggggucaag 1080
   cacagcccac ccugccccag gcaucaugug auggacuuuu cuguaucugc ccucuuggau 1140
   uaaggaagac ugagaccagc ccuuucagag gcuuuccucc uuccuguguu ggcugggaag 1200
   cgggguggac aaugugauuu cuccgugauu uccuacagcc uugagccucu cccagagugg 1260
   gggagauaac caccaugcca ggaauuc 1287
<210> 124
   <211> 612
   <212> RNA
   <213> Homo sapiens
<400> 124
   acccaccggc uuucggacca uggccaaccu cgagcguacc uucauugcca ucaagccaga 60
   uggcgugcag cgcggccugg ugggcgagau caucaaacgg uucgagcaga agggguuccg 120
   ccugguggcc augaaguucc uucgggccuc ugaagaacac cugaagcagc auuacaucga 180
   ccugaaagac cguccuuucu ucccggggcu ggugaaguac augaacucgg ggcccguggu 240
   ggccaugguc ugggaggggc ucaauguggu gaaaacgggc cgagugaugc ugggggagac 300
   caauccagcu gauucaaaac caggcaccau ccguggggau uucugcauuc aaguuggcag 360
   gaacaucauu cauggcagug auucagugga gagugcugag aaagagaucc aucugugguu 420
   uaagcccgaa gaacugaucg acuacaaguc uugugcccau gacugggugu acgaguagac 480
   augaagaaac cagaauccuu uucagcacua cugauggguu ucuggacaga gcucuucauc 540
   ccacugacag gauggaucau cuuuucuaaa acaauaaaga cuuuggaacu gaaaaaaaaa 600
   aaaaaaaaaa aa 612
<210> 125
   <211> 529
   <212> RNA
   <213> Homo sapiens
<400> 125
   uuuugcuuuc aacaugacag augccgcugu guccuucgcc aaggacuucu uggccggugg 60
   aguggccgca gcaucuccaa gacagcgguc acaccaucga gagggucaag cugcugcugc 120
   aggugcacau gccagcaagc aaaucacggc agauaagcaa uacaagggca ucauagacug 180
   cgugguucgu auccccaagg aacagggagu gcucuggucc uuugggcgug ggaaccgggc 240
   caaugucauc agauacuucc ccaccaaggc ucucaacuug gccuucaaag uuaauuccaa 300
   gcagaucuuu cuggguggug uugacaagag gacccaguuc uggcgcuacu uuugcaggga 360
   accuggcauc aggugguugc cgcuggggcu acauccuugg gcuuugugua cccucuugau 420
   uuuugccggu accgucuagc agcugaugug ggcaagcugg agcuaaaggg aauucaaggg 480
   ccuugggacu gccugguaag acuucaaucu gaugggauaa gggcuguac 529
<210> 126
   <211> 291
   <212> RNA
   <213> Homo sapiens
<400> 126
   auccgggacc cccacggccc cuuugcagcu ugccacaagg uucugagccc cuuggaauac 60
   uuccgccaau guguguauga caugugugcc cauaagggug acaaagccua ucucugccgu 120
   agccuggcug cuuauacugc agccugucag gcagcugggg cagcagugaa gcccuggagg 180
   acagacagcg ucugcccucu ccaguguccu gcccacagcc acuacuccau cugcacccgc 240
   uccugccagg gcuuccugug cugcucucuc uggccucacu ggcugcacca c 291
<210> 127
   <211> 3454
   <212> RNA
   <213> Homo sapiens
<400> 127
   ggcacgagcc gaguuggagg aagcagcggc agcggcagcg gcagcgguag cggugaggac 60
   ggcugugcag ccaaggaacc gggacagcga agcgacggca ggucgcagcu ggaucgcagg 120
   agccugggag cugggagcuu cagaggccgc ugaagcccag gcugggcaga ggaaggaagc 180
   gagccgaccc ggaggugaag cugagagugg agcguggcag uaaaaucaga cgacagaugg 240
   acagugugac aggaacguca gagaggauug ggccucgcug cgagagucag ccuggaguca 300
   agguguugac aaguugcuga gaaggacacg ugggaggacg guggcgcgcg gagggagagc 360
   ccugucuuca gucaccccgu ugauggagga cagauggaca gcagccggac ggccagucac 420
   cucucuuaaa ccuuuggaua gugguccuuu gugcucugcu ggacaccugu uggggauuuu 480
   agcccauucu cugaacucac uuucucuuaa aacguaaacu cggacggcag ugugcgagcc 540
   agcuccucug uggcagggca cuagagcugc agacaugagu gcagagggcu accaguacag 600
   agcacuguac gacuacaaga aggagcgaga ggaagacauu gaccuacacc ugggggacau 660
   acugacugug aauaaaggcu ccuuaguggc acuuggauuc agugauggcc aggaagcccg 720
   gccugaagau auuggcuggu uaaauggcua caaugaaacc acuggggaga ggggagacuu 780
   uccaggaacu uacguugaau acauuggaag gaaaagaauu ucacccccua cucccaagcc 840
   ucggcccccu cgaccgcuuc cuguugcucc ggguucuuca aaaacugaag cugacacgga 900
   gcagcaagcg uugccccuuc cugaccuggc cgagcaguuu gccccuccug auguugcccc 960
   gccucuccuu auaaagcucc uggaagccau ugagaagaaa ggacuggaau guucgacucu 1020
   auacagaaca caaagcucca gcaacccugc agaauuacga cagcuucuug auugugaugc 1080
   cgcgucagug gacuuggaga ugaucgacgu acacgucuua gcagaugcuu ucaaacgcua 1140
   ucucgccgac uuaccaaauc cugucauucc uguagcuguu uacaaugaga ugaugucuuu 1200
   agcccaagaa cuacagagcc cugaagacug cauccagcug uugaagaagc ucauuagauu 1260
   gccuaauaua ccucaucagu guuggcuuac gcuucaguau uugcucaagc auuuuuucaa 1320
   gcucucucaa gccuccagca aaaaccuuuu gaaugcaaga guccucucug agauuuucag 1380
   ccccgugcuu uucagauuuc cagccgccag cucugauaau acugaacacc ucauaaaagc 1440
   gauagagauu uuaaucucaa cggaauggaa ugagagacag ccagcaccag cacugccccc 1500
   caaaccaccc aagcccacua cuguagccaa caacagcaug aacaacaaua uguccuugca 1560
   ggaugcugaa ugguacuggg gagacaucuc aagggaagaa gugaaugaaa aacuccgaga 1620
   cacugcugau gggaccuuuu ugguacgaga cgcaucuacu aaaaugcacg gcgauuacac 1680
   ucuuacaccu aggaaaggag gaaauaacaa auuaaucaaa aucuuucacc gugauggaaa 1740
   auauggcuuc ucugauccau uaaccuucaa cucugugguu gaguuaauaa accacuaccg 1800
   gaaugagucu uuagcucagu acaaccccaa gcuggaugug aaguugcucu acccaguguc 1860
   caaauaccag caggaucaag uugucaaaga agauaauauu gaagcuguag ggaaaaaauu 1920
   acaugaauau aauacucaau uucaagaaaa aagucgggaa uaugauagau uauaugagga 1980
   guacacccgu acuucccagg aaauccaaau gaaaagaacg gcuaucgaag cauuuaauga 2040
   aaccauaaaa auauuugaag aacaaugcca aacccaggag cgguacagca aagaauacau 2100
   agagaaguuu aaacgcgaag gcaacgagaa agaaauucaa aggauuaugc auaaccauga 2160
   uaagcugaag ucgcguauca gugagaucau ugacaguagg aggagguugg aagaagacuu 2220
   gaagaagcag gcagcugagu accgagagau cgacaaacgc augaacagua uuaagccgga 2280
   ccucauccag uugagaaaga caagagacca auacuugaug uggcugacgc agaaaggugu 2340
   gcggcagaag aagcugaacg aguggcuggg gaaugaaaau accgaagauc aauacucccu 2400
   gguagaagau gaugaggauu ugccccacca ugacgagaag acguggaaug ucgggagcag 2460
   caaccgaaac aaagcggaga accuauugcg agggaagcga gacggcacuu uccuuguccg 2520
   ggagagcagu aagcagggcu gcuaugccug cuccguagug guagacggcg aagucaagca 2580
   uugcgucauu aacaagacug ccaccggcua uggcuuugcc gagcccuaca accuguacag 2640
   cucccugaag gagcuggugc uacauuauca acacaccucc cucgugcagc acaaugacuc 2700
   ccucaauguc acacuagcau acccaguaua ugcacaacag aggcgaugaa gcgcugcccu 2760
   cggauccagu uccucaccuu caagccaccc aaggccucug agaagcaaag ggcuccucuc 2820
   cagcccgacc ugugaacuga gcugcagaaa ugaagccggc ugucugcaca ugggacuaga 2880
   gcuuucuugg acaaaaagaa gucggggaag acacgcagcc ucggacuguu ggaugaccag 2940
   acguuucuaa ccuuauccuc uuucuuucuu ucuuucuuuc uuucuuucuu ucuuucuuuc 3000
   uuucuuucuu ucuuucuuuc uuucuaauuu aaagccacaa cacacaacca acacacagag 3060
   agaaagaaau gcaaaaaucu cuccgugcag ggacaaagag gccuuuaacc auggugcuug 3120
   uuaacgcuuu cugaagcuuu accagcuaca aguugggacu uuggagacca gaagguagac 3180
   agggccgaag agccugcgcc uggggccgcu ugguccagcc ugguguagcc ugggugucgc 3240
   uggguguggu gaacccagac acaucacacu guggauuauu uccuuuuuaa aagagcgaau 3300
   gauauguauc agagagccgc gucugcucac gcaggacacu uugagagaac auugaugcag 3360
   ucuguucgga ggaaaaauga aacaccagaa aacguuuuug uuuaaacuua ucaagucagc 3420
   aaccaacaac ccaccaacag aaaaaaaaaa aaaa 3454
<210> 128
   <211> 199
   <212> RNA
   <213> Homo sapiens
<220>
   <221> misc_feature
   <222> (136)..(136)
   <223> n is a, c, g, or u
<400> 128
   uuuuuuuuuu uuguuuuaau uuggcuccaa ugaucgcauu cucaaacucc uuugggaggg 60
   cauuagauga cccaguaucc gacgacuuag auucacacgu guuuuucucc ucugagcucu 120
   uuucuccagg cucacngucu uuguuuuuca agcuuucuug ggccuuugaa caauuuucuu 180
   ccuuugaaga uucuccugg 199
<210> 129
   <211> 1706
   <212> RNA
   <213> Homo sapiens
<400> 129
   agccgaguag gaccgagcug cugcagacgc gccgggucac ucgagccagc accaccguuc 60
   ucacgcccug agcugcagac agcuaggcgg uuuuaucuag uuugaaccag gcugcuggag 120
   cuugcucccu cccgcccucu cucuuuuuuu uccacggggc uguuuuuuua auuuggcugc 180
   aauugcauga aaucccaaug guguagacca guggcgaugg aucuaggagu uuaccaacug 240
   agacauuuuu ccauuucuuu cuugucgucu uugcugggaa ccgaaaacgc uuccgugaga 300
   cuugacaaua gcucuggugc aaguguggua gcuaucgaca acaaaauaga gcaagcuaug 360
   gaucugguga aaagccauuu gauguaugcg gugagggagg aaguggaagu ucugaaggag 420
   cagaucaaag aacuaauaga gaaaaacucc cagcuggagc aggagaacaa ucugcugaag 480
   acgcuggcca guccggagca gcucgcccag uuucaggccc agcugcagac uggcuccccu 540
   ccggccacca cgcagccaca ggggaccaca cagcccccug cacagccagc aucccagggc 600
   ucaggaucaa ccgcauagcc uccuaggccc caacagaacu ggcugcugcu gcugcugucu 660
   gaacugaaca gaccgaagag augugcuaga gagaagccgc cuccacaguc acccauuuca 720
   uugcugucua cgaaagagac gugagacuca cacgcuguuc ucgcuuucuc cccaguauua 780
   agcacucaua agcuuuuggc uugaagaaau guacuaguug agugaauuaa agguuaauca 840
   gagagugagc agggaugugc ccugugcaac guggcagaug ucugaggaau gguuuaauug 900
   accccgagga gcucugugcc uuuucaaccc uccccagccg cccacccugc uucugagagc 960
   ucgggcggcu cgccuucgug gggcucgccu gcgugggguu cgaaaguggg cugcuccugg 1020
   auucugcgcu cucuucuccu ucccuucaaa gaacucggag aggccagaaa caagacugca 1080
   auggggggcg gggggaggga ugaugcaguc cuuauacaaa accgacaacu gucaccaaag 1140
   cuuauaaaac acgauaguac ugucccucuu uucugaacca ucagaagaca caaaacuguu 1200
   agugacacaa cggugacagg uagcugggac cuaggcuauc uuauuaugaa gguuguuuug 1260
   cuuguuguau auuuguguau guaguguaac gaauuuguac cauagaggac uguccguaac 1320
   uacuguuuag cuucuacaca uugaaaugua gauguuucau uggcugucug aaaaggugug 1380
   gcuuguccuu ccuagagaga ucuacuuaaa aacugcuuug uggcaaaaac cacaccugaa 1440
   gaaauuuuaa gaauuuggcc caguuaguca cucuguguaa ucccggaauc uagcugcuga 1500
   agucuugcga aguaaacucc ccgugaccga ugucaguuaa gcuggugaua ccuggagaag 1560
   uggucaguug cuaaggaagu ggauuuccca guagggguuu cugcaccuca ccuguauagu 1620
   cguucugcgc auguccccca cacagucccc accuguauuu accuguucua cuugucaccu 1680
   uucaauaaag cauaucaaau guugau 1706
<210> 130
   <211> 2175
   <212> RNA
   <213> Homo sapiens
<400> 130
   auggcugcgc acgucuggcu ggcggccgcc cugcuccuuc ugguggacug gcugcugcug 60
   cggcccaugc ucccgggaau cuucucccug uugguucccg aggugccgcu gcuccggguc 120
   uggguggugg gccugagucg cugggccauc cuaggacuag ggguccgcgg gguccucggg 180
   gucaccgcag gagcccaugg cuggcuggcu gcuuugcagc cgcugguggc cgcacugagu 240
   uuggcccugc cuggacuugc cuuguuccga gagcuggccg ccuggggaac acuccgggag 300
   ggugacagcg cuggauuacu guacuggaac agucguccag augccuucgc uaucaguuau 360
   guggcagcau ugcccgcagc cgcccugugg cacaaguugg ggagccucug ggcgcccagc 420
   ggcaacaggg acgcuggaga caugcugugu cggaugcugg gcuuccuggg cccuaagaag 480
   agacgucucu accugguucu gguucucuug auucucucuu ggcuugggga aauggccauu 540
   cccuucuuca cgggccgcau cacugacugg auucuucagg auaagacagu uccuagcuuc 600
   acccgcaaca uauggcucau guccauucuc accauagcca gcacagcgcu ggaguuugca 660
   agugauggaa ucuacaacau caccauggga cacaugcacg gccgugugca cagagaggug 720
   uuucgggccg uccuucgcca ggagacaggg uuuuuccuga agaacccagc agguuccauc 780
   acaucucggg ugacugagga cacagccaac gugugcgagu ccauuaguga cacgcugagc 840
   cugcugcugu gguaccuggg gcgagcccug ugucucuugg uguucauguu uuggggguca 900
   ccguaccuca cucuggucac ccugaucaau cugccccugc uuuuucuuuu gccuaagaag 960
   cugggaaaag ugcaccaguc acuggcagug aaggugcagg agucucuagc aaaguccacg 1020
   cagguggccc uugaggccuu aucggcgaug ccuaccgugc ggagcuuugc caacgaggag 1080
   ggugaggccc agaaguucag gcagaaguug gaagaaauga agacgcuaaa caagaaggag 1140
   gccuuggcuu acgucgcuga agucuggacc acgagugucu cgggaaugcu gcugaaggug 1200
   ggaauucugu accugggcgg gcagcuggug aucagaggga cugucagcag cggcaaccuu 1260
   gucucauucg uucucuacca gcuucaguuc acccaggcug uucagguccu gcucucccuc 1320
   uaccccucca ugcagaaggc ugugggcucc ucagagaaaa uauucgaaua cuuggaccgg 1380
   acuccuugcu cuccacucag uggcucguug gcacccucaa acaugaaagg ccuuguggag 1440
   uuccaagaug ucucuuuugc cuacccaaac cagcccaaag uccaggugcu ucaggggcug 1500
   acguucaccc ugcauccugg aacggugaca gcguuggugg gacccaaugg aucagggaag 1560
   agcaccgugg cugcccugcu gcagaaccug uaccagccca ccgggggcca gcugcugcug 1620
   gauggccagc gccuggucca guaugaucac cauuaccugc acacucaggu ggccgcagug 1680
   ggacaagagc cgcugcuauu uggaagaagu uuucgagaaa auauugcgua uggccugaac 1740
   cggacuccaa ccauggagga aaucacagcu guggccgugg agucuggagc ccacgauuuc 1800
   aucucugggu ucccucaggg cuaugacaca gagguaggug agacugggaa ccagcuguca 1860
   ggaggucagc gacaggcagu ggccuuggcc cgagccuuga uccggaagcc acuccugcuu 1920
   aucuuggaug augccaccag ugcccuggau gcuggcaacc agcuacgggu ccagcggcuc 1980
   cuguaugaga gccccaagcg ggcuucucgg acgguucuuc uuaucaccca gcagcucagc 2040
   cuggcagagc aggcccacca cauccucuuu cucagagaag gcucugucgg cgagcagggc 2100
   acccaccugc agcucaugaa gagaggaggg ugcuaccggg ccaugguaga ggcucuugcg 2160
   gcuccugcag acuga 2175
<210> 131
   <211> 970
   <212> RNA
   <213> Homo sapiens
<400> 131
   gaauuccggg gccgcucucu ccggcaggau cgccgcgaug gccgcccagg gagagccgca 60
   gguccaguuc aaggucgucc uggugggcga cggcggcacc ggaaagacga cauucaugaa 120
   gcgccacuug accggagagu uugagaagga guauguagcc acccugggcg uggaggugca 180
   cacauuaguc uuccauacca acagaggacc uaucaaguuc aauguguggg acacagccgg 240
   ucaggagaag uucggggggc ugcgcgaugg cuacuacauc caagcccagu gugccauuau 300
   aauguuugac guaacaucaa gaguuacuua caagaaugug ccuagcuggc auaaagaucu 360
   agugcgugug ugugaaaaca uccccauugu auuguguggc aacaaagugg auguuaaaga 420
   caugaaagug aaggcaaaac cuauucucuu ccaccgaaag aagaaucuuc aguacuauga 480
   cauuucugcc agaaguaacu acaacuuuga aaagccuuuc uucuggcuug ccagaaagcu 540
   cauuggagau ccuaacuugg aguucguugc caugccugcu cuugccccac cugagguagu 600
   cauggaccca gcuuuggcag cacaguacga gcaugauuua gagguugcuc agacgacugc 660
   ucucccagau gaggaagaug accugugaga aagugaagcu ggagcccugc gucagaaguc 720
   uauuuuaggc aacuguccug ugaugccagc cagcggugca gugugugugc caccuuauuu 780
   agcuaaagga gaucgugcaa uucauuggga ugcugaagga gaugaauggg cuucggagug 840
   aauguggcag uuaaaauaca ccuucauuuu uuuggacuug cguauuuagc ccccuggaac 900
   agaguuguuc uggauuucaa agauaagacu gcuaccguag caucacaaua gucaguggug 960
   accggaauuc 970
<210> 132
   <211> 356
   <212> RNA
   <213> Homo sapiens
<400> 132
   uuuuuuuuuu uuuuuuugau uuaugaaaag uuuuauuuau caguacugug aagaauucuc 60
   aucauaauug cuacguuaau caaggaaaag gcacagagaa gcaugugucg uuugaguccu 120
   cgauacugga ccucccagcc caugcucccu augaggaguc uagcugcugu gguggucuuc 180
   acagcuuugg uuuucuggag acgaagcuca ugauugcguu caugcacucc ucugacagcc 240
   accuugcuug cagaguagug cacucuucag cguugacugc guagagcuuu ucuuucucau 300
   uuuuucugau uaacucuuug gaaauucuca uuggguuugg cgggagcuuc gcauau 356
<210> 133
   <211> 626
   <212> RNA
   <213> Homo sapiens
<400> 133
   ucgggaaucg auugagagac cgcgaaccug uaaacggaug auaccgaguc gggcaggcgu 60
   uaugccagcc caacucggga ccucgcauca uguccgcggc uaccuuagag uguuucggga 120
   augauuugcg guggaugaac gaaaccccgg uacguccugu gccaaggagc auaugucagg 180
   acggacgcuc guuaaugccu caguggaggg gcaacguucg cucucuaucu auacgacucu 240
   cgacaaugga uaucucggcu cucgcaucga ugaagaacgu agcgaaaugc gauacuuggu 300
   gugaauugca gaauccugug aaccaucgag ucuuugaacg caaguugcgc ccgaggccuu 360
   ucgguugagg gcacgccugc cugggcguca cgccuuguuu ugcucugugc ccgugcucuu 420
   ucgggggcgg ucauggaugc ggagauuggc ccuccgugcc ucgugugcgg cgggcuuaag 480
   cgcggggugu cggcgucggg aagggcacga cgaguggugg acggagcacc agcaggaugu 540
   uguggucccc cgucaccuua aggggcucaa gagacccgga cuaggcgagc cgcgcuucgu 600
   aagaggaggg cgagcugucu cgcaau 626
<210> 134
   <211> 495
   <212> RNA
   <213> Homo sapiens
<220>
   <221> misc_feature
   <222> (485)..(485)
   <223> n is a, c, g, or u
<400> 134
   uuuuuuuuuu uuuuuuugaa guugcugccc cuuuauuggu gacccgggca aguuuaagga 60
   gaacaacauu aaagcacaca aaguguaucc augucaccag cucaaccagg aggagugagg 120
   gucacggcag gguucuccca ugguguaaga aaucaacgca auuucaucac accccguuac 180
   uuuccaaguu aacgaaccga uaagaaaaga uucucccuua aacugacaag uacaauguac 240
   auguacauga uuuuggaaua auuuaauacu uuaaccucaa gauacaacua uauucuaaga 300
   ccauuauuuu aaaggaacgg auccuuacaa aaccaaaaua acccauauag cacgagguug 360
   guuuagccuu ucuucuucuu ucaacaaacg ugcaccacau guuucaguag caaggccgau 420
   gccauggaua ugagagcugu gauuugcagg gaccaaccac aucuagaacc ggggaggcca 480
   aucanacggu ggguu 495
<210> 135
   <211> 257
   <212> RNA
   <213> Homo sapiens
<220>
   <221> misc_feature
   <222> (254)..(254)
   <223> n is a, c, g, or u
<400> 135
   cccuuggagg auaguuuuau ugacaagucg aguuagguuu uagaguaaac uuuuaucacc 60
   ccagucaggc cccuucccag gggaggcucg cugguagcuc agauggccuu gguggugggc 120
   agauuguugu aguugucuuc cuggcccucc agcaggcuuc gggagguggc uaucuccugc 180
   uccagccugg acuugauguc caguagcugc uuauacuccg gguucuggcg cucuaugugc 240
   ggcacgcagg ugcngcg 257
<210> 136
   <211> 1186
   <212> RNA
   <213> Homo sapiens
<400> 136
   ggacugacuc cuugggcaga uugccucucc uccuucucau gccagaggcu gcugaugagg 60
   aaagguccag gggacugucc augcugucuu cauccucaga gucacugccu gaugcugcaa 120
   caagacccuu cuuguuuagc aauagugguu ggaacacucu cuuguaaguu accggagcac 180
   uaguauagga ggaggaucau cgacuacccu cccgccacuc cacggcugcu ggcuccuaga 240
   aaccccagcu ucaccucuca cugggacucg aguuccagaa ugaaaagcaa gaagggucuu 300
   guugcagcau caggcaguga cucugaggau gaagacagca uggacagucc ccuggaccuu 360
   uccucaucag cagccucugg caugagaagg aggagaggca aucugcccaa ggagucaguc 420
   cagauucugc gagacuggcu guaugaacac agauacaacg ccuaucccuc agagcaagag 480
   aaagcacugc ugucccagca gacacaccug uccacacuac aggucuguaa cugguucauc 540
   aacgcccgcc gcaggcuccu uccugacaug cugagaaagg auggcaaaga uccaaaucag 600
   uucacgauuu cccgccgugg ggccaagauu ucagaagcua gcucuauuga agcugcaaug 660
   gguaucaaaa acuucaugcc aacucuagaa gagagcccau uucauuccug cguaguugga 720
   cccaacccaa cccuagggag accagugucu cccaaaccuc ccuccccagg auccauuuug 780
   gcucgcccgu cagugaucug ccauaccacu gugacugcau ugaaggaugg gccuuucucu 840
   cucugucagc cgauuggugu gggacagagu acagauguac cgcaaauagc acccagcaac 900
   uuuacagaca ccucucucgu guacccagag gacacuugca aaucuggacc caguccaaac 960
   ccucagagug gucuuuucaa cacuccuccc ccuacuccac cagaccucaa ccaggauuuu 1020
   aguggauucc agcuucuagu ggauguugca cucaaacgag cggcagagau ggagcuucag 1080
   gccaaacuca cagcuuaacc guuuuuucaa acaaaacagu ucuccaaaau acgguccuga 1140
   uugccggggg ugauggcaag agaugcauua uuuuauauau uuuuuc 1186
<210> 137
   <211> 455
   <212> RNA
   <213> Homo sapiens
<400> 137
   uuuuuuuuuu uuuuuuuggu gguuaucaag ugcacuuuau ugaauccacu guggauagau 60
   aaaugagugu uacaccugcg uguagggagg ggcaaggagg gacgcagcug cggaggguga 120
   agcacuucag gaccggaagu cggaauccuc uauuaagugu gaagguuuug agcguuaaga 180
   acaaugauga ugacacuaac aauggugaua acaaccauca ggaugcugag gaccaaggug 240
   cugauguuca ggcacuuagc aguggaggcg uaggccuggg cuccagucac aucacccacc 300
   aucuuccgau cccuagacuu cacggaguag gcauaggcua ugaagcccag gcagcagaag 360
   uucaugaaga guguauugaa cagggaccag accacauggu caggcaccga caccucucug 420
   ggcauguuga ucacaguagu ucugacagaa gccga 455
<210> 138
   <211> 1797
   <212> RNA
   <213> Homo sapiens
<400> 138
   aagcuucagg auacagugca cacucguaaa uaaaaacuac aggcugcugc gaauuauauu 60
   ucaacugacc ggagaggcaa agccugacug uccauuaacc cuuaacuucc aaacgcaaac 120
   ugcuuacugc aucuuuuggc auuuuaccuu augccuuguu agguccaagg caagagaagc 180
   gucaucaaua accacgcaug ugcaacagcu uuuccagagg aaaggugugg guggcucuua 240
   aaagagccuu ugaguuagga gugugaguua aacgagcuca cuuggagcug guguacuugg 300
   ugacugccuu ggugcccucc gacaccgcgu gcuuggccag cuccccgggc agcagcaggc 360
   gcacggccgu cuggaucucc cgggacguga uggucgagcg cuuguuguaa ugcgccaggc      420
   gggaagccuc gcucgcgaug cgcucgaaga ugucguucac gaacgaguuc augaugccca      480
   uggccuugga ggagaugccg gugucggggu gcacuugcuu cagcaccuug uacacguaca      540
   ccgaguagcu cuccuugcgg cugcgcuugc gcuucuugcc guccuucuuc ugggccuugg      600
   ugacggccuu cuuggagccc uucuucgggg cgggagcgga cuuggcgggc ucaggcauac      660
   ugagaggaug aagugaacua aguugaaaaa ggauaacuaa aaguuaauga cuguucuggc      720
   ugcaauuuua aacaaacuua cggcuauggc aaccugaauc accauacguc auguacuaac      780
   aguccaauca aaacaaggga uuuucaaacc agggcgccau ugguaaccaa uguguaacca      840
   augaaaucuc uccguuuucg cguccagccu ugacuauaua uacuaugcgu auacguuuuu      900
   gcuucuuacu gcggugguua ucuacagcug aguuaugucu ggacguggca agcaaggacg      960
   uggcaagcaa ggaggcaagg cccgcgccaa ggccaagacg cgcuccuccc gggccggccu     1020
   gcaguucccc gugggccgcg ugcaccggcu gcuccgcaag ggcaacuacu cggagcgcgu     1080
   gggcgccggc gccccggugu accuggcggc cgugcuggag uaccugacgg ccgagauccu     1140
   ggagcuggcg ggcaacgcgg cccgcgacaa caagaagacg cgcaucaucc cgcgccaccu     1200
   gcagcuggcc auccgcaacg acgaggagcu caacaagcug cugggccgcg ugaccaucgc     1260
   gcagggcggc guccugccca acauccaggc cgugcugcug cccaagaaga ccgagagcca     1320
   ccacaaggcc aaggggaagu gaaaccaaac auuacgaauc accaaggcuc uuuucagagc     1380
   cacucacuuu cucaaagaga ccuaacacua cugggauagu gcauuguggg aaauacgugu     1440
   auuaaccuuc cuccuauuuu cccugcuugu gguuaguuca accccuaagc cuuaggcuaa     1500
   gaguauauug guuuuuggaa ggcaggcacc caaccucgga ccuaguacau aaaacagaca     1560
   caucuugaac uccaggccag ccuacucugc aggacgaguu ccaggacaga ccggacugca     1620
   caaagaauug ucuugaaaug uuccuuuauc agcacauaug cugauaaaca acuaaucacu     1680
   guacaaucaa uccucacuug aauccuguuu auguggcaug auugacaagu ccugccauuu      1740
   ggcaaaguca aaaucagcaa aggauguuaa agcauuuggu gguaucacag cuaaaac        1797
<210> 139
   <211> 376
   <212> RNA
   <213> Homo sapiens
<400> 139
   uuuuuuuuuu uuuuuaugca aauaguuuuc aagauuuuau ugcaaaccaa aauugguuua 60
   ucgcacacaa aaaaaguugu gugguaagga ggaggaauug uacaggauua uaacccaugu 120
   uaauuacagu acauuaaaau gaugguuuac aaauaagccu guaaguuuaa auaucuagug 180
   uuauaaccca auguacagac uuccuuuaca cgauacauac aauaaucagg aaugcaaaag     240
   aauaugaaca aagggaaaaa aaacauaaau aaugcccguu uuauagguga cauuuuaaac     300
   aauugaaaac accaaccggc uuugacugac aacuggggca uugguccaua aaaacccuuu 360
   cuaaaaauag aaauau 376
<210> 140
   <211> 1068
   <212> RNA
   <213> Homo sapiens
<400> 140
   gaggcugcuc aagagcugcg guugggucac cgcuucaugu uucucugccg auucugggga 60
   aagauggcaa cgaaugaugc uguucugaag aggcuggagc agaagggugc agaggcggau 120
   cagaucaucg aauaucucaa gcagcagguu gcucuucuua aggagaaagc aauuuugcag 180
   gcaacaauga gagaagaaaa gaaacuucga guugaaaaug cuaaacugaa aaaagaaaua 240
   gaagagcuaa agcaagagcu gauucuggca gaaauucaua acggagugga gcaagugcgu 300
   guucgauuga guacuccacu gcagacgaac uguacugcuu cugaaagugu ggugcagucu 360
   ccaucaguag caaccaccgc cucuccugcu acaaaagagc agaucaaagc gggagaagaa 420
   aagaagguga aagagaagac ugaaaagaaa ggagagaaaa aggagaagca gcagucggca 480
   gcagcaagua cugacuccaa gccuaucgac gcaucgcguc uggaucuucg aauugguugu 540
   auuguuacug ccaagaagca cccugaugca gauucacugu auguggagga aguagaugug 600
   ggagaagcag ccccgcgcac ggucgucagc gggcugguga aucauguucc ucuagaacag 660
   augcaaaauc guauggugcu uuuacucugu aaucugaagc cugcaaagau gcggggaguu 720
   cugucucaag ccauggugau gugugccagu ucaccagaga aaguggagau ucuggccccu 780
   cccaacgggu ccguuccugg ggacagaauu acuuuugaug cuuuuccugg agagccugac 840
   aaggagcuaa acccuaagaa gaagaucugg gagcagaucc agccugaccu gcacaccaau 900
   gcugagugug uggccacaua caaaggagcu cccuuugagg ugaaggggaa gggaguuugc 960
   agagcccaaa ccauggccaa uaguggaauu aaauaagugc ucuguaacug aaagacauug 1020
   gcgaaaacuu aauaacaaua aagagaagug uguuuaucac uuacauau 1068
<210> 141
   <211> 165
   <212> RNA
   <213> Homo sapiens
<400> 141
   ggccuuguuu uugguuugca auaaagagua uuucuuuaaa aggcacauuu uguuaaauag        60
   gcaguucccc uccugccucu uccuuuguag caguguacug cauccuagaa acauuuagca       120
   aagcagcccu uagccucccc gacccccuuu cccucccucc cagca                       165
<210> 142
   <211> 324
   <212> RNA
   <213> Homo sapiens
<400> 142
   uuuuuuuuuu uuuuuuuaaa caaugacgcc guuuauuuaa aauguuuacu cccagaaaua        60
   uagauauaaa aaaaaaaaua agacaauuaa cagcacuaaa ccaggcaccu ucaaccgaau       120
   cccaccaucc ucguuaacuc ccuuccuguu acccuuugua gaugaccaga agauuucagg       180
   agccccugga cagccagagu gguuccugcc cagggcuucc cgccuuccuc cuguccuaga       240
   gcuucccgug ggaaagcuug ggugagaauu uuagccuaaa gggagggggc uguggccggg       300
   cacuuugcgc ucauccacug cagg                                              324
<210> 143
   <211> 2473
   <212> RNA
   <213> Homo sapiens
<400> 143
   acaggguaca guugugcguc agggcgugga ggucuggcgg gagacgcaua guuacagcgc 60
   guccguucuc cgucucgcag ccggcacagc uagagcuucg agcgcagcgc ggccauggau 120
   cccagcagca agaaggugac gggccgccuc auguuggcug ugggaggagc agugcucgga 180
   ucacugcagu ucggcuauaa cacugguguc aucaacgccc cccagaaggu uauugaggag     240
   uucuacaauc aaacauggaa ccaccgcuac ggagagccca ucccauccac cacacucacc 300
   acgcuuuggu cucucuccgu ggccaucuuc ucugucgggg gcaugauugg uuccuucucu 360
   gucggccucu uuguuaaucg cuuuggcagg cggaacucca ugcugaugau gaaccuguug 420
   gccuuugugg cugcugugcu uaugggcuuc uccaaacugg gcaaguccuu ugagaugcug 480
   auccugggcc gcuucaucau cgguguguac ugcggccuga cuacuggcuu ugugcccaug     540
   uaugugggag aggugucacc uacagcucua cguggagccc uaggcacacu gcaccagcug 600
   ggaaucgucg uuggcauccu uauugcccag guguuuggcu uagacuccau caugggcaau 660
   gcagacuugu ggccucugcu gcucaguguc aucuucaucc cagcccugcu acaguguauc    720
   cuguugcccu ucugccccga gagcccccgc uuccugcuca ucaaucguaa cgaggagaac 780
   cgggccaaga gugugcugaa gaagcuucga gggacagccg augugacccg agaccugcag 840
   gagaugaaag aagagggucg gcagaugaug cgggagaaga aggucaccau cuuggagcug 900
   uuccgcucac ccgccuaccg ccagcccauc cucaucgcug uggugcugca gcugucccag 960
   cagcugucgg guaucaaugc uguguucuac uacucaacga gcaucuucga gaaggcaggu 1020
   gugcagcagc cuguguacgc caccaucggc uccgguaucg ucaacacggc cuucacugug 1080
   gugucgcugu uuguuguaga gcgagcugga cgacggaccc ugcaccucau uggccuggcu 1140
   ggcauggcag gcugugcugu gcucaugacc aucgcccugg ccuugcugga acggcugccu 1200
   uggauguccu aucugagcau cguggccauc uuuggcuuug uggccuucuu ugaaguaggc 1260
   ccugguccua uuccaugguu cauuguggcc gagcuguuca gccaggggcc ccguccugcu 1320
   gcuauugcug uggcuggcuu cuccaacugg accucaaacu ucauuguggg caugugcuuc 1380
   caguaugugg agcaacugug cggccccuac gucuucauca ucuucacggu gcuccucgug 1440
   cucuucuuca ucuucaccua cuucaaaguc ccugagacca aaggccgaac cuucgaugag 1500
   aucgcuuccg gcuuccggca ggggggugcc agccaaagug acaagacacc cgaggagcuc 1560
   uuccacccuc ugggggcgga cucccaagug ugaggagccc cacacccagc ccggccugcu 1620
   cccugcagcc caaggaucuc ucuggagcac aggcagcuag augagaccuc uuccgaaccg 1680
   acagaucucg ggcaagccgg gccugggcgc cuuuccucag ccagcaguga aguccaggag 1740
   gauauucagg acuuugaugg cuccagaauu uuuaaugaaa gcaagacugc ugcucagauc 1800
   uauucagaua agcagcaggu uuuauaauuu uuuuauuacu gauuuuguua uuuuuuuuuu 1860
   uuaucagcca cucuccuauc uccacacugu agucuucacc uugauuggcc cagugccuga 1920
   ggguggggac cacgcccugu ccagacacuu gccuucuuug ccaagcuaau cuguagggcu 1980
   ggaccuaugg ccaaggacac acuaauaccg aacucugagc uaggaggcuu uacgcuggag 2040
   gcgguagcug ccacccacuu ccgcaggccu ggaccucggc accauagggg uccggacucc 2100
   auuuuaggau ucgcccauuc cugucucuuc cuacccaacc acucaauuaa ucuuuccuug 2160
   ccugagacca guuggaagca cuggagugca gggaggagag ggaagggcca ggcugggcug 2220
   ccagguucua gucuccugug cacugagggc cacacaaaca ccaugagaag gaccucggag 2280
   gcugagaacu uaacugcuga agacacggac acuccugccc ugcuguguau agauggaaga 2340
   uauuuauaua uuuuuugguu gucaauauua aauacagaca cuaaguuaua guauaucugg 2400
   acaaacccac uuguaaauac accaacaaac uccuguaacu uuaccuaagc agauauaaau 2460
   ggcugguuuu uag 2473
<210> 144
   <211> 2125
   <212> RNA
   <213> Homo sapiens
<400> 144
   gcuuucuaaa cuagcaagca ucgugugggu ccuucaggag ugggagugca gacagaccug        60
   acagcguccg cuaagcgaca cugacuguac uuccacuccu gaaggaccca cacgaugcuu       120
   ucuaaacuag caagucugca gaccauugcu gcucugcgcc gaggagucca caccucaguc       180
   gccucugcca cgucuguugc cacaaagaag acagagcaag gcccaccauc cuccgaguac       240
   auuuuugaac gggaaucuaa auauggugca cacaauuacc auccuuugcc uguagcccug       300
   gagagaggaa aaggcauuua uaugugggau guggaaggca ggcaguacuu cgauuuccug 360
   agugcuuaug gugcugucag ccaaggacac ugccacccaa agaucauaga ugccaugaag 420
   agucaggugg acaagcugac auuaacaucu cgggcuuucu auaacaaugu ccuuggugaa 480
   uacgaggagu acaucaccaa gcuuuucaac uacaacaaag uucucccuau gaauacagga 540
   guggaggcug gagagacugc auguaagcuc gcucgucguu ggggcuacac cgugaaaggc 600
   auccagaaau acaaagcaaa gauuguuuuu gcugauggga acuuuugggg ucgaacacua 660
   ucugcaaucu ccaguuccac agauccgacc aguuaugaug gcuuuggacc cuucaugcca 720
   ggcuuugaaa ccaucccaua uaacgaucug cccgcacugg agcgugcucu ucaggaucca 780
   aauguugcug ccuucauggu ggagcccauc cagggugaag caggcguuau cguuccggau 840
   ccaggauacc ugacaggagu ucgggaacuc ugcaccaggc accagguccu guuuauugcu 900
   gaugaaauac agacaggauu ggccagaacu gguagauggc uggcugugga ucaugagaau 960
   gucagaccug auaugguucu ucuugggaag gcccuuucug gcgguuuaua cccugugucu 1020
   gcagugcugu gugacgauga gauaaugcug accauuaaac caggcgagca cggcuccaca 1080
   uacggcggaa acccacuagg cugccgaauu gccauugcgg cucuugaggu uuuagaagag 1140
   gagaaucuug cugagaaugc agacaagaug ggcgcuaucc ugaggaagga gcucaugaag 1200
   cugcccucug acguugugac cucagugaga gggaaagggu ugcuaaaugc cauugucauc 1260
   agagaaacca aagacuguga ugcuuggaag gugugccugc gacuucgaga uaacgggcuu 1320
   cuggccaagc caacccacgg ugauaucauc aggcuugccc cuccccuugu gaucaaggag 1380
   gaugagaucc gggaguccgu ggagaucauc aacaagacua ucuuguccuu cugagaguag 1440
   gaacucuggg gagccaucuu cagacagggc ucuugugaaa cucugcuugc aguggccaga 1500
   gccugucucc ugaaaggcau auauuucagu ugaugcauaa uagagugaca ccuaggaacc 1560
   ugcagguggc ugcgugacag aaaagugaga gcgagaggcg aggcgucucu uuguugaggu 1620
   uugacugugu gggaacuuuc uaaggagaaa cggacccauc ugcguacagc cugcagaugg 1680
   aggccugcag ucauuuacgu gcgucuuuac aguuuccuug cugaugugaa ugguuuguau 1740
   uuagaaguua uuucugagau acuacagaac aguuaaauca uuauaaucaa ugaauguuaa 1800
   guugauugaa gguuaagcau auguaaaaua cuaguuuaaa guaaacuuuu cauuggccaa 1860
   caccagaaug uauuauauag auucugagaa uucauuacua aauuacacuu ugcuugauug 1920
   caauuuguaa aacauuuauu uucaguauuu cuuugaauaa agcuuaaugu uucuuuuuac 1980
   gccaacagag uauuuuguau uuccauuuug guaauaauca guguauuauu ucauccugau 2040
   gacuggcauu ucaucaccua uugagaucac uggguguguu ucaggccuuu uauucuaaau     2100
   aaagcuauga ccaguuucug ucugu 2125
<210> 145
   <211> 199
   <212> RNA
   <213> Homo sapiens
<400> 145
   uuuuuuuuuu uucuuuuccg acgcccacaa gaggaauaag agauuuaaug auggaaagua         60
   uggggaaauc acaguuuuca gacaugagua aucaaaaacu ugacauuuuu cuugauaucc        120
   aaaucuagau gucuguauca aaccagaggu gauggccuug gggauggcag ugaagacugu        180
   uaggaccauu agaucagau                                                     199
<210> 146
   <211> 412
   <212> RNA
   <213> Homo sapiens
<400> 146
   uuuuuuuuuu uuuuuuuaca guuuuaaaac aauacacagc uuucucgggc ugaagcaauu         60
   gcaagaacgu auugguauug guauauuaca gcuacauaca agguuuauga auagcaaugg        120
   agaaaaauaa guuauuuaaa uauugacuuc auaaagagaa agugcaaugu uguuaguugu        180
   cauaucacuu gcuugacagu uugugggguu ucuucccuau caauuuuaac aaucaagaua        240
   acauggacuc aagacagaau uuuucgggaa ccucacucag uccucacaca gcagugacuu        300
   gggaaucuac guguguucca ccgcaguugu gaaacacacu acuccguguc caggacucau        360
   uucucagaga agaaucaauu cgaguuccau ccacaccugg ggucgggaca ca                412
<210> 147
   <211> 2373
   <212> RNA
   <213> Homo sapiens
<400> 147
   ccgggcucga auucucuaga cucgaguuua gagaauucga gcucucccag uuuuaaagca      60
   aaauuuugga cugugaagca aggcacuggg caaacacaac auggccuccc cggcugacag     120
   cuguauccag uuuacccggc acgcuaguga uguucuucuc aaccuuaauc gccuccggag     180
   ucgggacauc uugacggacg uugucaucgu ggugagccgu gagcaguuua gagcccauaa     240
   gacagugcuc auggccugca gcggccuguu cuacaguauc uucacugacc aguugaaaug     300
   caaccuuagu guaaucaauc uagauccuga aaucagcccu gagggguuuu gcauccuccu     360
   ggacuucaug uacacaucua ggcucaaccu gagggaaggc aauaucaugg cggugaugac     420
   cacagccaug uaccugcaga uggagcaugu ugucgacaca ugcaggaagu ucaucaaggc     480
   cagugaagca gaaauggccc cugcacuuaa accuccccgu gaagaguucc ugaacagccg     540
   gaugcugaug ccccaugaca ucauggccua ccgaggucgu gaggucgugg agaacaauau     600
   gccacugaga aauacucccg ggugugagag cagagcuuuu gcuccuccuc uguacagugg 660
   ccugucaaca ccaccagccu cuuaucccau guacagccau cucccgcuca gcaccuuccu 720
   cuucucugau gaggagcucc gagaugcccc ccgaaugccu guggccaacc cuuuucccaa 780
   ggagcgugcc cuccccugcg acagugccag gcaagucccu aaugaguaua gcaggccagc 840
   cauggaggug ucccccaguu ugugucacag caacaucuac ucgcccaagg aggcaguccc 900
   agaggaggcu cggagugaca uacacuacag ugugccugag ggccccaagc cugcuguccc 960
   uucugcucgg aaugcuccau acuuccccug ugacaaagcc agcaaagaag aagagagacc 1020
   uucuucggag gaugagauug cccugcauuu cgagcccccc aaugcacccu ugaaccggaa 1080
   gggucugguu aguccccaga guccccagaa auccgacugc cagcccaacu cacccacaga 1140
   guccugcagc agcaagaacg ccugcauccu ucaggccucu ggcucuccgc cagccaagag 1200
   ccccacugac ccgaaagccu gcaacuggaa gaaguauaag uucaucguuc ucaacagccu 1260
   caaucagaau gccaaacccg agggcucuga gcaggcagag cugggucgcc ucuccccucg 1320
   agccuacccu gcaccgcccg cuugccagcc gccuauggag cccgcgaacc uugaucucca 1380
   guccccgacc aagcucagug ccagugggga ggacucuacc aucccccaag ccagccggcu 1440
   caauaaucuc gugaacaggu cccugggagg cuccccccga agcagcagug agagucacuc 1500
   accacucuac augcaccccc caaagugcac auccugcggc ucucaguccc cacagcauac 1560
   agagaugugc cuccauacug cugggcccac guucccggag gagauggggg aaacccaguc 1620
   agaguauucg gauucuagcu gugagaaugg gaccuucuuc ugcaacgaau gugacugccg 1680
   uuucucugag gaggccucgc ucaagaggca cacgcugcag acgcacagug acaaaccaua 1740
   caaaugugau cgcugccagg ccuccuuccg cuacaagggc aaccucgcca gccacaagac 1800
   uguccacacg ggugagaaac ccuaucgcug uaacauuugu ggagcgcagu ucaaucggcc 1860
   agccaaccug aagacccaca cucgaauuca cucuggagaa aagcccuaca aaugugaaac 1920
   cuguggggcc agguuuguuc agguggccca ccuccgugcc cacgugcuca uccacacugg 1980
   agagaagccg uaccccugug aaaucugugg cacucgcuuc cggcaccuuc agacucugaa 2040
   gagccaucug cgcauccaca caggagagaa accuuaccau ugugagaagu guaaccugca 2100
   cuuucgucac aaaagccaac ugcgacuuca uuugcgccag aagcacggcg ccaucaccaa 2160
   caccaaggug caauaccgcg ugucggccgc ugaccugccu ccggagcucc ccaaagccug 2220
   cugaaugaag cauggagugu uccucgcccu uuccucucca gccccuucuc agaaucuacc 2280
   caaaggaugc uguaacacuu uauacaaagg ucaucccaug auguagugcc ucucucaucc 2340
   acuagugcaa aucauaguug gggugggggu ggg 2373
<210> 148
   <211> 989
   <212> RNA
   <213> Homo sapiens
<400> 148
   ucgcgguccg acggaggagu gggcgcuggg aucucgcuga gcguccgccu ggccucgucu      60
   cuuccucgcu cgucggagcu ucagcacggu ccgagauggc uggcgguaag gcuggaaagg     120
   acuccggaaa ggccaagaca aaggcgguuu cccgcucgca gcgagccggc uugcaguucc     180
   cugugggccg uauucaucga caccugaaau cuaggacaac cagccacgga cgugugggcg     240
   cgaccgccgc uguguacagc gcagccaucc uggaguaccu caccgcagag guacuugagu     300
   uggcaggaaa ugcgucaaaa gacuuaaagg uaaagcguau caccccucgu cacuugcagc     360
   uugcuauacg uggagaugaa gaauuggauu cucugaucaa agcuaccauu gcugguggug     420
   gugucauccc acacauccac aaaucgcuga ucgggaagaa aggacaacag aagacuguuu     480
   aaggaugccu ggauuccuua uuaucucagg acucuaaaua uuccuaacag cuguccagug     540
   uuggugauuc caguggacug uaucucugug aaaaacacaa uuuugccuuu uuguaauucu     600
   auuugagcaa guuggaggcu uaauuagccu uccaaccaac caaauuucug cauucgaguc     660
   uuaaccauau uuaaguguua cuguggcuuc aaagaagcua uugauucuga aguagugggu     720
   uuugauugag uugacuguuu uuaaaaaacu guuuggauuu uaauugugau gcagaaguua     780
   uaguaacaag cauuugguuu uguacagaca uuguuuccac ucugguggau aagcucaaua     840
   aaggucauau cccaaacuag cuuuaaacuu gcuuaauaau cgggucuuac cuuagaucuc     900
   acucagcaac aaguacauuc ucugcuuacu aauuaaacag ugcaucugua gucauaaaaa     960
   aaaaaaaaaa aaaaaaaaaa aaaaaaaaa                                       989
<210> 149
   <211> 6342
   <212> RNA
   <213> Homo sapiens
<220>
   <221> misc_feature
   <222> (439)..(538)
   <223> n is a, c, g, or u
<220>
   <221> misc_feature
   <222> (1133)..(1232)
   <223> n is a, c, g, or u
<220>
   <221> misc_feature
   <222> (1698)..(1797)
   <223> n is a, c, g, or u
<220>
   <221> misc_feature
   <222> (2105)..(2204)
   <223> n is a, c, g, or u
<220>
   <221> misc_feature
   <222> (2861)..(2960)
   <223> n is a, c, g, or u
<220>
   <221> misc_feature
   <222> (3546)..(3645)
   <223> n is a, c, g, or u
<220>
   <221> misc_feature
   <222> (4057)..(4156)
   <223> n is a, c, g, or u
<220>
   <221> misc_feature
   <222> (4736)..(4835)
   <223> n is a, c, g, or u
<220>
   <221> misc_feature
   <222> (5487)..(5586)
   <223> n is a, c, g, or u
<400> 149
   caccaccgug cacgcagcuc cgggcccgug ggguguuggu ucuugcccuc guaacccccu 60
   cuguccagcc accaugauaa gcgccagcag agccgcggcc gcgcgucucg ugggcaccgc 120
   ugcgucccgg agccccgcag ccgcccgucc ccaggugaga agcugccaug ccuuccggug 180
   ggggcuccag gcccggacuc gagugaggca ggccuugccu ucgggucaga cucuaggaaa 240
   aauccggagc gaagggaugu aacggaccuu cugugggcau uguuggccuu cuugcagggc 300
   uuuagcuucg aacugugcug agucacaauc cuuggcguuc cuaagucuuu accccgcuaa 360
   uugagacguc uguccccccu cuaaccugug cgcuuugaau gugccuggac uuaggcagug 420
   gacguaguuu acuggaaann nnnnnnnnnn nnnnnnnnnn nnnnnnnnnn nnnnnnnnnn 480
   nnnnnnnnnn nnnnnnnnnn nnnnnnnnnn nnnnnnnnnn nnnnnnnnnn nnnnnnnnuu 540
   uuacauacua aggacuaaag cuugcaugug uuccucagaa uaugaaaagc ccaaguucuu 600
   guguaguuga uggcugaugc aacuuuuuuc cccccaggau ggcuggaaug gccuuagcca 660
   ugaggcuuuu agauuuguuu caagaagaga uuaugcguaa guacaaccuc aguuucucug 720
   agaaaaaaaa aaaacacuua uugaaccuca aagcuuggau ggguugggug cguuauacau 780
   uuguacuugu aguuuauuca auaugccacu gguaacacca acauaaaaca caguucuucg 840
   uauuggagac cacuguucag augaccaugg aauuucauuu cuuacagauc agaagcaauc 900
   aagggugcag ugguugguau ugauuugggu acuacuaacu ccuguguggc uguuauggag 960
   ggcaaacaag caaaggugag caugauugga aaccugaggu cacuuagaua cccagucugg 1020
   cauuaaguac auaggaaugc ugagucggag cccagguuag uggugggcac uuuaauccua 1080
   guaaaggcag agggaucucu gaguucagga ccagccuaga guacaaagug agnnnnnnnn 1140
   nnnnnnnnnn nnnnnnnnnn nnnnnnnnnn nnnnnnnnnn nnnnnnnnnn nnnnnnnnnn 1200
   nnnnnnnnnn nnnnnnnnnn nnnnnnnnnn nnacuuuaau cagugucuuu gggaauuuua 1260
   gguccuggag aaugcugaag gugccagaac uaccccuucu gugguugccu uuacagcaga 1320
   uggagaacga cuuguuggua ugccagcaaa acggcaagcu gucaccaauc caaacaauac 1380
   cuucuaugcu acuaagcguc uuauuggacg acgauaugau gacccugaag uacagaaaga 1440
   cacgugagua auaggaaaau caguccagaa gacuggugcu uugaucaaag uucuguggau 1500
   accuugaguu cuguggauca ccuuggauca cuuuuucauu auuucugcuu gggaagaaau 1560
   cacaccacca ucagaggcau auagguuuuu uuuuuguuau uucuuuguug uuguuguuuu 1620
   ccuauuuauu uguuuguuug uuuggggggg gguuucuuug uguauuuucc uggaacucau 1680
   ucuguagacc aggcuggnnn nnnnnnnnnn nnnnnnnnnn nnnnnnnnnn nnnnnnnnnn 1740
   nnnnnnnnnn nnnnnnnnnn nnnnnnnnnn nnnnnnnnnn nnnnnnnnnn nnnnnnnuca 1800
   ugugauauau gaugaacuua gagugguuaa guuucacagg gauaguuuac uuacauaauc 1860
   uuucugucuu uaaguaagaa uguuccuuuu aaaauugucc gugccuccaa uggugaugcu 1920
   uggguugagg cucauggaaa acucuauucu ccaagucaga uuggagcauu uguguugaug 1980
   aagaugaaag agacugcagg uaaguggauu uauuucacau uuaggaaaau uggaaugugc 2040
   uguuuauuuc ucugcauuaa uacugauuaa cuucauauuc uguagauaau ggagucugaa 2100
   gcuunnnnnn nnnnnnnnnn nnnnnnnnnn nnnnnnnnnn nnnnnnnnnn nnnnnnnnnn 2160
   nnnnnnnnnn nnnnnnnnnn nnnnnnnnnn nnnnnnnnnn nnnngaauuc augcuccucc 2220
   ugcugccucc ugggugcuaa guugcaaggu gauagcucag guacucacuc uagugucuuu 2280
   cuuggggugc ucuuuuccaa ggccucucua cauauuaagc cacaaaggag ucuguugccc 2340
   cucaagagga ugagaugugg aauauuaggc uacaguuuug uugccuuuuu uuuauuuucc 2400
   uaacauggua ccacaauuga auuuuauucu uuguuuccca gaaaauuacu ugggccacac 2460
   agcaaaaaau gcugugauca cagucccugc uuauuucaau gauucacagc gacagguaaa 2520
   auuagaucuc uuguuugcug ggaguggagg ugggguaccu gaguuaaagg auggaaagau 2580
   agauuuauuu cuacuuucuc uaggccacua aggaugcugg ccagauaucu gggcuaaaug 2640
   ugcuucgagu gaucaaugag ccuacagcug cugcucuagc uuacggucug gacaaaucug 2700
   aagauaaagu guaaguuggu cagaugacgu agcauuaccu gcauuuacag ggguugugug 2760
   ugugugugug uguguguaau uuuacuacaa uuuguguggg uccguguguu gugguacauu    2820
   uguacauuug uacauggcau ggauauggau gucaaagauu nnnnnnnnnn nnnnnnnnnn 2880
   nnnnnnnnnn nnnnnnnnnn nnnnnnnnnn nnnnnnnnnn nnnnnnnnnn nnnnnnnnnn 2940
   nnnnnnnnnn nnnnnnnnnn aaaccaguag uugguguguu acuaauuuua cuuguuucug 3000
   uaugacaugc uuauaugaua cguuaacagg caugccacuu agguugauaa cuacauucag 3060
   uuauuaagua uuucaggaaa uaucauaguu aauaauuaaa cuuuuggucu uuuaauucgu 3120
   uccuuuguuu uguuccuugg aacuuaacuu acuuauuuau uuuuuagcau ugcuguguau 3180
   gauuuaggug guggaaccuu ugacauuucu auccuggaaa uucagaaagg aguguuugag 3240
   gugaaaucua ccaaugggga cacuuucuua ggaggggaag acuuugacca agcuuuguug 3300
   cggcacauug ucaaggaguu caagagagag guuaguuacc acugcuuagu caccacuggu 3360
   uaagguguag gcguuggguu guugagaauu uuuguuuguu ugcauugcuu uuagcuuugu 3420
   uaauagcuuu uuuauacuaa gguaacuaac uauacuucag auucaugggu aaacuaaacc 3480
   aguuuaguua uauaaucuua gauugggaac aaaagaccaa gugacagugu uauaguaggg 3540
   agaagnnnnn nnnnnnnnnn nnnnnnnnnn nnnnnnnnnn nnnnnnnnnn nnnnnnnnnn 3600
   nnnnnnnnnn nnnnnnnnnn nnnnnnnnnn nnnnnnnnnn nnnnnacuac ucucucgcag 3660
   ucuauauaug ucuccuaauc cuauuuauag acagggguug auuugaccaa agacaacaug 3720
   gcgcuucaga ggguucggga agcugcugag aaggcuaaau gugaacuuuc cucaucugug 3780
   caggugaggg auggaaaaau cccaguacug agcauauuug aauaguguau ucuaauuuac 3840
   cuaaugucag uguagcucuu uacaguuuuc uguuggcuga aaacuugggg caugagcaaa 3900
   ggaacaacuu gaugaucagu ucuuuucauu ugaaugaaug aaguagauuu auggaugugu 3960
   guaucuuuug ccugcaugug ugucuguacu acauuugugc uugguuucug uggcggccag 4020
   aagaggguau cagaacugac augucagugu uggggannnn nnnnnnnnnn nnnnnnnnnn 4080
   nnnnnnnnnn nnnnnnnnnn nnnnnnnnnn nnnnnnnnnn nnnnnnnnnn nnnnnnnnnn 4140
   nnnnnnnnnn nnnnnncaca cugagucuaa auucugauca ugucuuucag ucguguuaug 4200
   uuacuuugag ugaguaucaa agaucacguc ucccaucuga cguguggucc ugugcagacu 4260
   gacaucaacu ugccauaccu uaccauggau gcuucuggac caaagcauuu gaauaugaag 4320
   cugacucgag cucaguuuga aggcauuguc acagaucuaa ucaagagaac uauugcuccg 4380
   ugucagaaag cuaugcagga ugcagaaguc agcaagagug acauaggaga agugauucug 4440
   guugguggca ugacaaggau gcccaaggua uggacucaug guauuucucc uagaggaaaa 4500
   aaauaacaau gcauucuuga ggcaaauggc uuguguugug uguggaaaca aaugugaucc 4560
   auucuucuag uguucuuuaa agagugguga gaccagacuc accaaaaagu gcuuuuaguc 4620
   gccugugaug gcucauguag gaggauggcc uugaguucug gguaagaugc agcuacagaa 4680
   uucuaccuug cacacacacu uaaacccagu cugguaaaga gaaguuguua agcuunnnnn 4740
   nnnnnnnnnn nnnnnnnnnn nnnnnnnnnn nnnnnnnnnn nnnnnnnnnn nnnnnnnnnn 4800
   nnnnnnnnnn nnnnnnnnnn nnnnnnnnnn nnnnncagau guaaaauuga uaauacuucu 4860
   uccuaagauu gggcgauugu guagaauaug agaacuguau uuuauaaccc cuugucaugu 4920
   gccuucuauu ucaaaugaua uccucuggga agcuagauaa uuaaauucuu cucucauuua 4980
   aguggacagu ggaugagaug cucugaaguu gucaaauaca aacaagucug cagucuugga 5040
   uaugaaucuc ucugacuugc ugucuggcag gcguauucug uuuggcucca ucagucgccc 5100
   uggguguugg cuaacagguu cuuucucccu gaugcuuagg uucagcagac uguacaagau 5160
   cuuuuuggca gagccccgag uaaagcuguu aauccugaug aggcuguagc caucggagcu 5220
   gccauccagg gagguguguu ggcuggugac guuacagacg ugcugcuccu ggaugucacu 5280
   ccccucucuc uggguauuga gacucuggga ggcgucuuua ccaaacuuau uaauaggaac 5340
   accacuauuc caaccaaaaa gagccaggua agagccauuc uuuuuuccug ccuauuaaca 5400
   gucccaaguu guacaagugc uguuuacaau cacuuuauga acucuuuaaa acuuuguuuc 5460
   uaagacuaua cuaacuggac ugggugnnnn nnnnnnnnnn nnnnnnnnnn nnnnnnnnnn 5520
   nnnnnnnnnn nnnnnnnnnn nnnnnnnnnn nnnnnnnnnn nnnnnnnnnn nnnnnnnnnn 5580
   nnnnnnccua gguguuuucu acugcugcug auggacaaac ucaaguagag auuaaagugu 5640
   gucaggggga acgagagaug gcuggagaca acaaacuucu aggacaguuc acuuugguaa 5700
   guguuuuuga gucugaguau caugcuuuug gggucuauag cuugcaaagc uccaaacugc 5760
   ugacauuaca ggcauauugu guuauuuuuu aaaaagaacg uuauguacau gaguuaugaa 5820
   acccaugauu uuaguuuuuu accuaaagug cuuuguguuu ucagaauuug aaauuuucaa 5880
   agccugggaa auguucagac ucagaagcaa ugcuaacuca gaaguuaguu uuauccugau 5940
   uacucauuuu aaaaaacuua auagcuacug guucugguca gcauugcuac aguagagaag 6000
   uuuauuugcu guaaauucug ggcacauaua accaucaugg uuauuaacuu cuugaagccc 6060
   agugauuuca gaaagcacua aaacuaccac caucacuuaa aaucucaagg uuuugaacau 6120
   ucagugaaga uacuuguuuu aggaaacaag ugguuaguug gccugauuug gaaaugagaa 6180
   uacaugggcc uuucaaagga gcucacucug gauauuuauu uuagauugga auucccccag 6240
   ccccucgugg agugccccag auugaaguua cauuugacau ugaugccaau gggauugugc 6300
   acguuucugc caaagauaaa ggcacugguc gugagcaaca ga 6342
<210> 150
   <211> 4145
   <212> RNA
   <213> Homo sapiens
<220>
   <221> misc_feature
   <222> (4045)..(4045)
   <223> n is a, c, g, or u
<400> 150
   auaaauucuu auuuugacac ucaccaaaau agucaccugg aaaacccgcu uuuugugaca 60
   aaguacagaa ggcuugguca cauuuaaauc acugagaacu agagagaaau acuaucgcaa 120
   acuguaauag acauuacauc cauaaaaguu uccccagucc uuauuguaau auugcacagu 180
   gcaauugcua cauggcaaac uaguguagca uagaagucaa agcaaaaaca aaccaaagaa 240
   aggagccaca agaguaaaac uguucaacag uuaauaguuc aaacuaagcc auugaaucua 300
   ucauugggau cguuaaaaug aaucuuccua caccuugcag uguaugauuu aacuuuuaca 360
   gaacacaagc caaguuuaaa aucagcagua gagauauuaa aaugaaaagg uuugcuaaua 420
   gaguaacauu aaauacccug aaggaaaaaa aaccuaaaua ucaaaauaac ugauuaaaau 480
   ucacuugcaa auuagcacac gaauaugcaa cuuggaaauc augcaguguu uuauuuaaga 540
   aaacauaaaa caaaacuauu aaaauaguuu uagagggggu aaaauccagg uccucugcca 600
   ggaugcuaaa auuagacuuc aggggaauuu ugaagucuuc aauuuugaaa ccuauuaaaa 660
   agcccaugau uacaguuaau uaagagcagu gcacgcaaca gugacacgcc uuuagagagc 720
   auuacugugu augaacaugu uggcugcuac cagccacagu caauuuaaca aggcugcuca 780
   gucaugaacu uaauacagag agagcacgcc uaggcagcaa gcacagcuug cugggccacu 840
   uuccucccug ucgugacaca aucaauccgu guacuuggug uaucugaagc gcacgcugca 900
   ccgcggcacu gcccggcggg uuucugggcg gggagcgauc cccgcgucgc cccccgugaa 960
   accgacagag ccuggacuuu caggagguac agcggcgguc ugaaggggau cugggaucuu 1020
   gcagagggaa cuugcaucga aacuugggca guucuccgaa ccggagacua agcuuccccg 1080
   agcagcgcac uuuggagacg uguccggucu acuccggacu cgcaucucau uccacucggc 1140
   cauagccuug gcuucccggc gaccucagcg uggucacagg ggccccccug ugcccaggga 1200
   aauguuucaa gcuuuucccg gagacuacga cuccggcucc cgguguagcu caucacccuc 1260
   cgccgagucu caguaccugu cuucggugga cuccuucggc aguccaccca ccgccgccgc 1320
   cucccaggag ugcgccgguc ucggggaaau gcccggcucc uucgugccaa cggucaccgc 1380
   aaucacaacc agccaggauc uucaguggcu cgugcaaccc acccucaucu cuuccauggc 1440
   ccagucccag gggcagccac uggccuccca gccuccagcu guugacccuu augacaugcc 1500
   aggaaccagc uacucaaccc caggccugag ugccuacagc acuggcgggg caagcggaag 1560
   uggugggccu ucaaccagca caaccaccag uggaccugug ucugcccguc cagccagagc 1620
   caggccuaga agaccccgag aagagacacu uaccccagaa gaagaagaaa agcgaagggu 1680
   ucgcagagag cggaacaagc uggcugcagc uaagugcagg aaccgucgga gggagcugac 1740
   agaucgacuu caggcggaaa cugaucagcu ugaagaggaa aaggcagagc uggagucgga 1800
   gaucgccgag cugcaaaaag agaaggaacg ccuggaguuu guccuggugg cccacaaacc 1860
   gggcugcaag auccccuacg aagaggggcc ggggccaggc ccgcuggccg aggugagaga 1920
   uuugccaggg ucaacauccg cuaaggaaga cggcuucggc uggcugcugc cgcccccucc 1980
   accacccccc cugcccuucc agagcagccg agacgcaccc cccaaccuga cggcuucucu 2040
   cuuuacacac agugaaguuc aaguccucgg cgaccccuuc cccguuguua gcccuucgua 2100
   cacuuccucg uuuguccuca ccugcccgga ggucuccgcg uucgccggcg cccaacgcac 2160
   cagcggcagc gagcagccgu ccgacccgcu gaacucgccc ucccuucuug cucuguaaac 2220
   ucuuuagaca aacaaaacaa acaaacccgc aaggaacaag gaggaggaag augaggagga 2280
   gaggggagga agcaguccgg gggugugugu guggacccuu ugacucuucu gucugaccac 2340
   cugccgccuc ugccaucgga caugacggaa ggaccuccuu uguguuuugu gcuccgucuc 2400
   ugguuuucug ugccccggcg agaccggaga gcuggugacu uuggggacag gggugggggc 2460
   ggggauggac accccuccug cauaucuuug uccuguuacu ucaacccaac uucuggggau 2520
   agauggcugg cugggugggu aggguggggu gcaacgccca ccuuuggcgu cuugcgugag 2580
   gcuggagggg aaagggugcu gagugugggg ugcagggugg guugaggucg agcuggcaug 2640
   caccuccaga gagacccaac gaggaaauga cagcaccguc cuguccuucu uuucccccac 2700
   ccacccaucc acccucaagg gugcagggug accaagauag cucuguuuug cucccucggg 2760
   ccuuagcuga uuaacuuaac auuuccaaga gguuacaacc uccuccugga cgaauugagc 2820
   ccccgacuga gggaagucga ugcccccuuu gggagucugc uaaccccacu ucccgcugau 2880
   uccaaaaugu gaaccccuau cugacugcuc agucuuuccc uccugggaaa acuggcucag 2940
   guuggauuuu uuuccucguc ugcuacagag cccccuccca acucaggccc gcucccaccc 3000
   cugugcagua uuaugcuaug ucccucucac ccucaccccc accccaggcg cccuuggccg 3060
   uccucguugg gccuuacugg uuuugggcag cagggggcgc ugcgacgccc aucuugcugg 3120
   agcgcuuuau acugugaaug aguggucgga uugcugggug cgccggaugg gauugacccc 3180
   cagcccucca aaacuuuccc ugggccuccc cuucuuccac uugcuuccuc ccuccccuug 3240
   acagggaguu agacucgaaa ggaugaccac gacgcauccc gguggccuuc uugcucaggc 3300
   cccagacuuu uucucuuuaa guccuucgcc uuccccagcc uaggacgcca acuucucccc 3360
   acccugggag ccccgcaucc ucucacagag gucgaggcaa uuuucagaga aguuuucagg 3420
   gcugaggcuu uggcuccccu auccucgaua uuugaauccc caaauauuuu uggacuagca 3480
   uacuuaagag ggggcugagu ucccacuauc ccacuccauc caauuccuuc agucccaaag 3540
   acgaguucug ucccuucccu ccagcuuuca ccucgugaga aucccacgag ucagauuucu 3600
   auuuuuuaau auuggggaga ugggcccuac cgcccguccc ccgugcugca uggaacauuc 3660
   cauacccugu ccugggcccu agguuccaaa ccuaauccca aaccccaccc ccagcuauuu 3720
   aucccuuucc ugguucccaa aaagcacuua uaucuauuau guauaaauaa auauauuaua 3780
   uaugagugug cgugugugug cgugugcgug cgugcgugcg ugcgugcgag cuuccuuguu 3840
   uucaagugug cuguggaguu caaaaucgcu ucuggggauu ugagucagac uuucuggcug 3900
   ucccuuuuug ucaccuuuuu guuguugucu cggcuccucu ggcuguugga gacagucccg 3960
   gccucucccu uuauccuuuc ucaagucugu cucgcucaga ccacuuccaa caugucucca 4020
   cucucaauga cucugaucuc cggunugucu guuaauucug gauuugucgg ggacaugcaa 4080
   uuuuacuucu guaaguaagu gugacugggu gguagauuuu uuacaaucua uaucguugag 4140
   aauuc 4145
<210> 151
   <211> 1756
   <212> RNA
   <213> Homo sapiens
<400> 151
   caccugauuc ccggaggccc gagcccuuag ucugggcggg guggcgcggg ccggaaggac 60
   gccaucccgg ccugggccau ggaggcuccc gcaccguccc ucacggagga ggauuugacu 120
   gaagugaaga aggacgcuuu agagaauuua cguguuuacc ugugugagaa aaucauagcu 180
   gagagacauu uugaucaucu acgugcaaaa aaaauacuaa guagagaaga cacugaagaa 240
   auuucuugcc gaacuucaag uagaaaacgg gcugggaagu uguuagacua cuuacaggag 300
   aaccccaggg gccuggacac ccugguggaa uccauccgca gggagaaaac acagagcuuc 360
   cugauucaga agauaacgga ugaggugcua aagcuucgga auauaaaacu ggagcaccuc 420
   aaaggccuga agugcagcag cugugagccc uuugcagccg gagccaccaa caaccucucu 480
   aggugcaauu ccgaugagag caaucucucu gagaaacaga gagcauccac ugucauguac 540
   cacccggagg gagaguccag cacggcuccc uucuucucua uggcgucguc ccugaacuug 600
   ccaguccugg aaguuggcag gacugaaaac agcagcuucu cuucagccac ucuuccucga 660
   ccuggggacc cuggggcucc cccuuugccc ccagaccuuc gguuggaaga ggggggaagu 720
   uguggaaacu caagugagau guuucucccc uuacggucac gggcucuuuc acgccaauga 780
   uacaucaccg ccuaguuguu uuacuaguga ugcaaaaugc ugugaaggag gccaucuuuc 840
   uauacaaacc acggugacag gucacucaca uucgaugcgu gccuuuaaaa ucaguguaca 900
   cauucucugu aaauaggauu uguuagggua aagaagcgcu cuggggcggc gugguguaau 960
   cauggugguc gugacuuuuc cauaaugucc uuucuuuuuu auuauuuuua gguguuugcg 1020
   uauuuugaac uuuucauaag auuaauuuua ucggaauauu ucucaauuug agaaaacaac 1080
   uuguggauug ggaauaaugu uuuuagcaca uuuaugcuac aaauuuucag ucugauuguu 1140
   uuucccacug aucuggcagu auauuuuagc aguaagcugu ugugugucag gaaagcugga 1200
   cacgggaaag cugccgacac acucagcagu gucccacucc uuaguucuga gaagccgucg 1260
   gguucugagg agacaccugg uggcacugag ccuggugacc ucagugggcc aaaauuuguu 1320
   uuauacucac ccugccagcg ugagugucuu acuuucacag gccuuguguc cucagucuua 1380
   ucuuaaagga uguuaucuug gcagggcauc acuuguaauu aauggaugau acuuguaauu 1440
   gacuaaaguc cucgcucuga gccguuuguu cuggcuccga gagcgcugac augugaagca 1500
   uggugagcag cgagggaacu gacaggaugu ggccguggcc aguguggcuu uaguguuugc 1560
   aucaggcagc caccagcucc auccguguuc uuacugcuuu acaaaguuug acuaacuuua 1620
   cacauuuuaa aaaugcugau ugucuucguu uaaauuauaa uuuuaccuau uucuugacau 1680
   cuaacuccua uuuauuucua uuauuuaaaa auuaagaaau gaaaauuugc uauuaacaau 1740
   aaaguuuuuu uaaugu 1756
<210> 152
   <211> 1113
   <212> RNA
   <213> Homo sapiens
<400> 152
   gggggggggg ggucagcgcc gacguccccu gccgccacca ugcccaaaag aaaggcugaa      60
   ggggaugcua aaggagacaa aaccaaggug aaggacgagc cacagagaag aucugcaagg     120
   uugucugcua aaccugcucc uccaaagcca gagcccaaac cuaaaaaggc cccugcgaag     180
   aagggagaga agguacccaa ggggaagaag gggaaagcug acgccgggaa ggaugcgaau     240
   aauccugcag aaaauggaga ugccaaaaca gaccaggcac agaaagcuga aggugcugga     300
   gaugccaagu gaugugugug cauuuuugau aacuguguac uucuggugac uguacaguuu     360
   gaaauacuau uuuuuaucaa guuuuauaaa aaugcagaau uucguuuuac uuuuuuuuuu     420
   uuuuuaagcu auauuguuag cacacagaac acuucauuac uggguggggg aaggaucaug      480
   ugucaguaac aaaaucucuc ccaagcugga uugaggacag aaaaccucuu ucccugauaa     540
   uuuuggaagg cuccuguugg cucccaggag agagauccug gucuugaccu agguggccac     600
   caaggcacaa caaugccuug uggucuggga aaacuauaaa uucacuuuua uauccucuuc     660
   ccccuguacu aucaacauag acuuaauucc cuuaaaaacc agagaccugu uggaaccugg     720
   cccccaaaau ugguuuccca guccauugag ugauggggac uuugcaguga cuucauugag     780
   uguucucaaa agagcacugg uuccuuuuau aaaagauugu ggaucuucag auugauaauu     840
   cugccuaaaa gucagggucg gcuugugaaa aguuguuaaa acaacauccu uaaugugaaa     900
   ugucaacccu cacucuaagc uacuuccccc uuuucaaagc auugaaugaa gacuucauug     960
   gguuuuauag uggcuuucug auuuugguag ucauaucaga agggaauuug gaaguucuug    1020
   uauauuguug cauugucugc ccauguccug ccugaauacc augauuguuu augaaagaau    1080
   cuuaauaaag cugguuacag uuaggcugga aaa                                 1113
<210> 153
   <211> 321
   <212> RNA
   <213> Homo sapiens
<400> 153
   uuuuuuuuuu uuuuuuugga aguccauaag uaguuuauug ucuucaagac uacagugugg         60
   auuccucucc cagagaaggg ucuuucagag gcaggggacu gucacccagg ugcaggccgu        120
   cuacuuguca uuuucauaca uggcuggauu cuuccuuuuc gacugcucaa acuccugguu        180
   gccccaugug uagaucaggu agaccacuac aaacggcggc gccacgcgca ggaugcgcuc        240
   gcgagugcgg cgcaacacgu uggggaugcc uuugcugaaa uagcuuggga aggcgcgcug        300
   cucaaagggc gacaagcugu a                                                  321
<210> 154
   <211> 1720
   <212> RNA
   <213> Homo sapiens
<220>
   <221> misc_feature
   <222> (952)..(952)
   <223> n is a, c, g, or u
<400> 154
   caugaagcca gagaugugga agaugugucu agacugcauc aaugaacuga uggauacguu         60
   ggaugcacau uccaacaucu cugucggaga gaacauuuug gcagagagug agaacuuaca        120
   caacuuugau caguucacuc cguguacgac gcugcauccu aacuuuggug gagcgaaugg        180
   augaagaauu uaccaaaaua augcaaaaua cugauccuca cucccaagag uauguggagc        240
   accugaagga ugaggcacaa gugugugcau cauugagcga gugcagcgcu accuggagga        300
   gaaagguacc acugaggaga ucugccagau cuacuuaagg cgcauccugc acacguacua        360
   caaguuugac uacaaggccc aucagcggca cguuacuccu ccugaaggau ccucaaaguc        420
   ugagcaagac caggcagaaa augaggguga ggacucagcu gugcuaaugg aaagacugug        480
   caaguacauc uaugccaagg accguacaga ccggauccgu accugugcca uccucugcca        540
   uaucuaccau caugcgcucc acucccgcug guaucaggcc cgugaccuca ugcucaugag        600
   ccaccuacag gacaacauuc agcacgcaga cccgccggug cagauccugu auaaccguac        660
   uauggugcaa cugggcaucu gugcuuuccg ccaaggccug acaaaggaug cacacaaugg        720
   cacuucugga uauucaguca agugguggug ccaaggagcu ucuaggucag gguuugcugc        780
   ugcgcgcuug caggagcgaa aucaggaaca ggaaaaggua gagcgacgcc ggcaggugcc        840
   cuuucaccug cacaucaacc uggagcugcu ggaguguguc uaucuggugu cagcuaugcu        900
   ccuggagauc cccuacaugg cugcccauga gagcgaugcc cgccgacgca unaucagcaa      960
   gcaguuccac caccaacugc gggugggcga gcggcacgcc cugcuagguc cucccgaguc    1020
   aaugagggag cauguggucg cugccuccaa ggccaugaag augggcgacu ggaagaccug     1080
   ccacaguuuc aucauuaaug aaaagaugaa ugggaaagug ugggaccuuu ucccugaggc     1140
   ugacaaaguu cgcaccaugc uaguucggaa gauccaggaa gagucucuga ggaccuaccu     1200
   uuuuaccuac agcagugucu augacucaau caguauggag acacuaucag auauguuuga     1260
   gcuggaucua cccacuguuc acuccaucau cagcaagaug aucauuaacg aagaauugau     1320
   ggcuucccug gaccagccga cacagacugu ggugaugcac cguacugagc ccucugccca     1380
   gcaagaaacu uggcucugca agcuggcuga gaaaacuugg cacccuagug gagaauaaug     1440
   gacggguguu ugaccaaaaa cagggaaccu augguggcua uuuccgagac cccaagggug     1500
   gcuaccggaa aaauggaggc uaaaugcccc gugguggcua cccccagcaa cagucucaga     1560
   caaccuccug aguuccccac uucagucacc cuguggacag accaucuaac cuuuuucucc     1620
   uaacucaccc caaucauuaa agaucuuuug aggaauuaaa aaaaaagaaa gaaaaaaaaa     1680
   aaaaaaaaaa aaaaaaaaaa aaaaaaaaaa aaaaaaaaaa                           1720
<210> 155
   <211> 453
   <212> RNA
   <213> Homo sapiens
<400> 155
   uuauuaauua aaaaacaauu uauugaaaaa gaguagugcu uuguacaaau ucccauugca       60
   gcccccagau aucaacugau cucuuuccag cuuugguagu aggguauaaa aauaucaaaa      120
   cuagguaagu ucugauguaa gauuucuaug ggauuauuuu agaauauaua gauuugugua      180
   guuuggcaag uaucauuucu augcauuuac auuacauauu aagcacagau ucuguggcaa      240
   aacaucuuug caugauuacu uuacacacac aaauauagua aaacuuacau aguacaaauu      300
   cacauaagac uccauucugu cuauaacuuc auccaugugg uuuuaccaug aauuauaauu      360
   cuuaaccucu ccuauaacug ucagcuucca uuuaauuuug aaaguaucac uucacaaaga      420
   gcacuucauu ugcuuuuaga guauacauag acu                                   453
<210> 156
   <211> 361
   <212> RNA
   <213> Homo sapiens
<220>
   <221> misc_feature
   <222> (230)..(230)
   <223> n is a, c, g, or u
<400> 156
   cggccgccaa gaaguauaac augcgagugg aagacuacga gccauacccc gaugauggca 60
   ugggguaugg cgacuacccg augcucccca accgaucaca gcaugagagg gauccguggu 120
   aucaguggga ccacucagaa cucaggauga acugggguga accgauacac ugggaccuag 180
   acauguacau caggaaucgu guggacacgu caccuacccc uguguccugn gaugucaugu 240
   guaaacaucu cuucggcuuu guggcuuuca ugguuuucau guucugggua gggcacgugu 300
   ucccuuccua ccagccugug gguccgaagc aguacccuua caaaaaucug uaccuggagc 360
   g 361
<210> 157
   <211> 452
   <212> RNA
   <213> Homo sapiens
<400> 157
   uuuuuuuuuu uuuuuuugau gcaaaugucu uuauuuucca cuuaaacaag uuucccuuuu 60
   gcacuggccu guggcacaaa acagauggcu ggguggugac auuaacuguc aaguuaguga 120
   gaugcagaga uggugagaca cugcauuuga gugcauaaua cuuuuauucc agaggaacgc 180
   caugcagccc aguuacaccu uuaggucaga aaggcugaug cgugaccagc cucuauugcc 240
   ucccuuggua agaaggaccc acaagugcag aguccaacag augcuggcuc ugagcugaac 300
   ucagggcauu ccaauuacca cuuucuucuu caccuacaca gggccugcuc agauguccuu 360
   uuuacaacuc cauaagcccu uuggccaaag ucccugcagu guuuggggag gaccuuccca 420
   cccuucacca gucagugucu gaucuggugg ag 452
<210> 158
   <211> 392
   <212> RNA
   <213> Homo sapiens
<400> 158
   uuuuuuuuuu uuuuuuuaau aucuguaaua guuuauuuca aagauuugac auuuacaagu 60
   agaggcacca caugcuaucu gacaguaaaa uacugcaggg acugaaggcc aaggagagag 120
   auccacagaa gacaggccug uagugcaggc auugcaucga ccuugcccac agugcuuugu 180
   ucccaacuca ggacaguacu uuagugcuug cuucauuuac uggaaaaguu cacuggacau 240
   aguuuccacu ucuuccccca cagcuuccag cucagcaaac uuaagcuacu acuccucgau 300
   gcucucauag aggcucuuga uuugugucug gaagaacuug gcaagcucua cucguuuggc 360
   uuucaguguc ggugucagaa guccguuuuc aa 392
<210> 159
   <211> 217
   <212> RNA
   <213> Homo sapiens
<400> 159
   uuuuuuuuuu uuuuuuucug agguauuaaa auaucuagac ugaauuuugc caaauguaag 60
   agggagaaag uuccugaaga cucugacuac uugcuuauuu uugauugacc uucuaugcuu 120
   augucauuac ugccucacaa cguguuugau guccuuuaau gauacaaagu gagccugugc 180
   cuucauuuuc uugcccauuu ugguaccccu cgugccg 217
<210> 160
   <211> 1515
   <212> RNA
   <213> Homo sapiens
<400> 160
   gcgcccgaac gucuagcaga guaccugcug cuguaagcuu gucgucuggg cugcaccgcc 60
   cgucuuaacc cauucucgac uuaacuacuc ucgucgaaca agcauggaag uacagaaaga 120
   agcgcagcgc aucaugacuc ugucgguaug gaagauguac cacucucgca ugcagcgagg 180
   uggcuugcga cuccaccgga gucugcagcu aucccucguu augcgcagcg cucgagagcu 240
   cuaccucuca gccaagguag aagcccacca gcccgaguuc ccgccauccc gcagggcucu 300
   ugacccucgc cugcacccgc cgcgggaagc cgaaguugca guggaaguag cgucccccga 360
   agccgugcag ccuccggagc ccauggauac gcaagaggaa gugcugcgag uccaggagac 420
   cccugcgcuc ugugacccgc cccccgcuag agucagccgc aagcgccgga gcagcagcga 480
   uuugagcgac aguagugaug ccggacuggu accaagcaag aaggcccguc uagaagaggu 540
   ggagggggag gcgacgucgg agguucccga ucgccugcag cuuccuccgg cacaaagcga 600
   aggugccuuc ccuaaccucg cccgcguccu ccaaaggcgc uucuccaguc uccugaacug 660
   uggacccgcc gugcccccgc cgacgccccc cacgugcgag gccaagccag ccugccgccc 720
   ggccgacaau augcucaacg ugcuggugcg agcuguggug gccuucugag agcucuggug 780
   gcuucuuucg agcggcgcca ccggagcgga gaacgcacac ccgaggcgaa ggccggcggg 840
   ggccgugaag aagagccgcg gcccgagcug ccgagaggcc agggcaagga cugaggagcg 900
   aggggcgcgg gcgccuucuc ccagacgugc guccauaggu gcuauuaaag gacugucccu 960
   uccuuggcuu ggagaaggga caccuagauc uugaaucuca gggucgaacu cucuaggggc 1020
   caggcugccc uuucaaggcc guuucacuac cauucgcguu ucggccccua caagugggca 1080
   cgcuugugca agcggucaga guugcgucau gggacagacg cgggugcuuc cuguugccuu 1140
   gcgugggugu ggggccuggg aggaggccag gguguggacc cgcccuaggg acugggaagu 1200
   gacuugaguc accucgcccc cacaggcugc ugugggugag ccugaacuga accaaucaaa 1260
   ucugcgcaga guugaagugg cuggagaccc cgggacuggu caaccuagau gaucgccugg 1320
   cguggaccac cgcgggacgg gugggccgcu ggucguaguu gcugccguag acacagcuuc 1380
   uucgggcagg aaagaaaauu uuuuuuuuac cagcguguuu aagaaagucu guuuacuuuu 1440
   cccacggugg guuguuuaau uagcaacuac cuggaguuuu acaaugucag cuaggaaaau 1500
   aaagaccauc ggugu 1515
<210> 161
   <211> 801
   <212> RNA
   <213> Homo sapiens
<400> 161
   cugagaggcc aggugggcgg cgaaaucaac guggagaugg augccgcucc cgguguggac      60
   cugagccgca uccugucaga gaugcgugau caguacgaga agauggcgga gaagaaccgc     120
   aaggaugccg aagacugguu cuucagcaag accgaggagc ugaaccgcga gguggccacc     180
   aacagcgagc uggugcagag cggcaagagc gagaucuccg agcucaggcg caccaugcag     240
   gcccuggaga uugagcugca gucccagcuc agcaugaaag caucucugga gggcagccug     300
   gcagagacag agaaccgcua cugcgugcag cugucucaga uccaggggcu gaucggcagu     360
   guggaggagc agcuggcuca gcugcgcugc gagauggagc agcagaacca ggaguacaag     420
   auccugcugg augugaagac aaggcuggag caggagaucg ccaccuaccg ccgucugcug     480
   gagggagagg augcccaccu gacucaguac aagccaaaag aaccugugac cacccgccag     540
   gugcgcacca uuguggaaga aguucaggau ggcaagguca ucucaucccg ggaacaggug     600
   caccagacca cccguuaagg acucagcucc uuccgcccag uuccccgagg cugcagagag     660
   gcagcuuccc ucuccgcucc ggcaucaccc uccugcuaca gccucucccc agcauuccua     720
   ugcuugagac cauuaaagcu ugcugaccug aagugaacug uggccuuugu ucugaacacu     780
   gaaauaaaug accaugguga c                                               801
<210> 162
   <211> **447**
   <212> RNA
   <213> Homo sapiens
<400> 162
   uuuuuuuuuu uuuuuuuggu uccacuuaag uugcauguuu uauuucuccc aaucccagca      60
   auagcacaga agcccaucau guccauccca gacugguuuc uaguaggcug agaugacagg     120
   gagccucagu aacgcuaaug gcacagaggg cucccaaaug ccaggcacaa cugugccucc     180
   acacugguga cugcccagag ugcccuggcc ccaguguguu ggcacucagu cugacuuuac     240
   aacgcaaccu gcaccuuuga aagggacagu cugggagugg ggagugugag ggagugaagc     300
   ucaaacugcc uccugucagc ucacccuuuc aacauuaaac agagaccaag agagaaacag     360
   uuccaauauu ucacauauau uucuucuugu gcagucuaag ccgagaaugc cauguaaaug     420
   ggucacugcg aaaugcagca auuuagu                                         447
<210> 163
   <211> 475
   <212> RNA
   <213> Homo sapiens
<400> 163
   gucucgggcg agauggcuuc aagguuacuu cgcggagugg gcgcuuuggc ggcgcaggcc 60
   cugaggcgca cggcccgugg cgcggccgug acccgcucca uggcuucugg aggugguguc     120
   cccacugaug aggagcaggc uacugggcug gagagggaga ucaugauagc agcacagaag 180
   ggacuggacc cauacaauau gcuaccucca aaggcagcuu caggcaccaa ggaagacccu 240
   aaucuagucc cguccaucag caacaagaga auagugggcu gcaucuguga agaggacaac 300
   uguacuguca ucugguuuug gcugcacaaa ggcgagaguc agcgaugccc caacugugga 360
   acccauuaca agcuggugcc ccaccaaaug gcccacugag ccccuguguu aucuuuucag 420
   aauguaaaga aaaacuucuc ucuaauaaag acuagccauu gcaccugcuc cuccc 475
<210> 164
   <211> 1897
   <212> RNA
   <213> Homo sapiens
<400> 164
   gaauucggca cgagcagcga gacgccgcgc acggugcuuc cccaguggag ccaaucggcu 60
   aacccgcgcu ccggcagagu ccuuggcgcu cgcccgccgg cgggacagac cacccgccuc 120
   uggccgcucu cuggacccug gccgccccga gcgaagacug gagcaaaaug augcuucaac 180
   auccaggcca ggucucugcc ucagaaguca gugcgaccgc cauugucccc ugccucucac     240
   cuccuggguc acugguauuu gaggauuuug cuaaccugac acccuuuguc aaggaagagc 300
   ugagauucgc cauccagaau aaacaccucu gccaucggau guccucugcg cuggagucag 360
   uuaccgucaa caacagaccc cuggagaugu cagucaccaa gucugaggcg gccccugaag 420
   aagaugagag gaaaaggagg cggcgagaaa gaaauaaaau ugcugcugcc aagugucgaa 480
   acaagaaaaa ggagaagaca gagugccugc agaaagaguc agagaaacug gagaguguga 540
   augcugagcu gaaggcccag auugaggagc ugaagaauga gaaacagcau uugauauaca 600
   ugcucaaccu gcaccggccc accuguaucg uccgggcuca gaauggacgg acaccggaag 660
   acgagaggaa ccucuuuauc caacagauaa aagaaggaac auugcagagc uaagcagagg 720
   uggcacggag gcaauugggg aguucuuacu gaauccuccu uuuccacccc acacccugaa 780
   gccauuggaa aacuggcuuc cugugcacuu cuagaauccc agcagccaag agccguuggg 840
   gcaggagggc cuguggugac cuacugcauu gacccacucu gcccccgagu gaaccgugga     900
   gcaggcagga gcauccuuug ucucaccaau uccaggauuu aggccuuauc aucccggcca 960
   gucucagaug accuagcugg ccccaggcug ggguccuaug caaagcagga ucccacuaau      1020
   gggauucagg cagaaguguc uaccuugaua gguggggugg gaccacaucc uccacugugg      1080
   cugacaacgc ccuuccaagg gaauauggaa ugagaacauu cauuauugag guuguccaau 1140
   ggccagggua ugcuuucuag aaaauaugcu guucuguccc agaaugacug ugcauagggu 1200
   auccguuuca gagccuggug uugugcuauu uagauguuug ucuugcacaa cauuggcaug 1260
   auuuuuccgg gaguuucauc agaucugauu ucugagaguc uggggaucug ccauggugga 1320
   aagugccccu caaaagcauu uguguggcca caugaacugg cuggcaccag gggagugaaa 1380
   cuggcugaug accagcugag ccacuuugug ccaacagagg auggacgaca ccuuucccug 1440
   uacccacugc agaggaagaa cccugggcac agcagcuuug uccuuggcua caaacuguua 1500
   caacgucaca caaugaaggc acaaagucca acuuucaaag gguguaggac uccauacuca 1560
   gugacagggc aggaagagcc aaagauaacc acagccacag ccuguggaga ccaggguugg 1620
   aagccaggug cagggccagg caucugcauu gugggauguu aauggcacuu uugucuugua 1680
   gcuauuuuga gauguggucc agagcauuuc agcugggaga ucucccucug gccaccagga 1740
   cucuggcuac uguuaaaauc cugauguuuc uguggaaucc ucaguguuua aucccacuca 1800
   auaguaucau uacaguuuuc uguaagagaa aauauuacuu auuuauccca guauuccuag 1860
   ccugucaaca uaauaaauau cggaacaaaa ccuggua 1897
<210> 165
   <211> 2167
   <212> RNA
   <213> Homo sapiens
<400> 165
   gcgcaccgcc ygyguygcgc gscagcgucg ucuaggugca ucgcgggccc ccgcagrwag      60
   aaaaauaugg cucaggagac uaaccagacc ccagggccca ugcuguguag uacaggaugu     120
   ggcuuuuaug ggaauccuag gacaaaugga auguguucug uuugcuacaa agaacaucuu     180
   cagagacagc agaauagugg cagaaugagc ccaaugggga cagcuagugg uuccaacagu     240
   ccuaccucag auucugcauc uguacagaga gcagaugcug guuuaaacaa cugugaaggu     300
   gcugcuggca gcacaucuga aaaaucaaga aaugugccug uggcugccuu gccuguaacu     360
   caacaaauga cagaaaugag cauuucaaga gaggacaaaa uaacuacccc gaaaacagag     420
   gugucagagc caguugucac ucagcccagu ccaucaguuu cucagcccag uucuucucaa     480
   agugaagaaa aagcuccuga guugcccaaa ccaaagaaga acagauguuu uauguguaga     540
   aagaaaguug gccuuacagg guuugacugc cgauguggaa auuuguuuug uggacuucac     600
   cguuacucug acaagcacaa cuguccauau gauuacaaag cagaagcugc agcaaaaauc     660
   agaaaagaaa auccaguugu uguggcugaa aaaauccaga gaauauaaaa uuacuacaug     720
   ugaagagacu gaaacuuugu uuuuauuuua auauaucgua ggaaaacauu aaagagcaga 780
   ugcauggcca uuuuccuuug auguucucca gaguuuugcu uuauacuugu cugucauaua 840
   auugaucuuu uaggauguuu ggguguuugu uacaggcaga auuggauaga uacagcccua 900
   caaauguaua ugcccucccc ucaguaaaau uggacaaaaa uaugcacaac aaauugaaau 960
   acacauauac uaggaacaaa auuuaguucc acgugccaaa cuaaaggaau gaaaucucug 1020
   cauguuugca gcauaucugc cuuuugggaa uguaaucaag guauaaucuu uggcuagugu 1080
   uaugugccug uacuuaaaaa aaaaaaaaug guacaccaga aaaggacugg cagucuacua 1140
   ccauagucaa acuucaccuu aauuucgaca ugacuuuugg aagcaggaag aaagcuacaa 1200
   aacugguauu ugguaccaug ugugagccug guuaaauugg ucuucuaaaa gcugucaauu 1260
   aggacauucu gcgaaaggua acaucacaac ugguucuaag ucaaaccauc aagucaacag 1320
   cagggugccu gagauaaucu uggaagcuua uugugcuggc cugcaccaga agauaucugc 1380
   auucucauua cuaaaauugu agcacagaac ugcacuagga uuuguuuaca aggagaaauu 1440
   aaacucuguu ugguuuucac auauagcagc ucuguuaaau aacaugcauc ugaauuuuaa 1500
   guugcaaagg uaucugaaca guuaauuuuu caugugcauc uuuuguugaa uguuuugguu 1560
   caagaaagaa uguuuaaagc uuuuuaaaga cuucaguucu uaauguaacu guacccuucu 1620
   gcauggaaaa ucauaaccaa cauggcugca guagacuucu uuagugguau ccagcaccac 1680
   uugcagaggg cugcuuuauc auauuguacu ugggguaggg acucuagugu ucuugggugu 1740
   auugcauggg cugcauuauc uacagcauug uacaauaaca acuagaaaag gcgguauacu 1800
   ucacugaugc uugucuggua auaucacuuc uguguuaaaa uggaagguuu uuugugaugu 1860
   augaaacuug uguuuuuuau auauaaauga guauaguuag auuaguguug ugguaaugcc 1920
   uguuuucauc uguaaauagu uaaguaugua cacaacaagg cacuacuucu gauuuuugca 1980
   guguucaguc cuaguuuuuc uuuauuaaaa cauugaguuu ugcuucaauu uuauguaccu 2040
   uaguucuaag uuagauuugc agauguguac agauaguuca uauuuaugua uugcacauaa 2100
   ucaugcuauu cagcauugau gcuauauugu auuauguaaa uaauaaaagc aguguacaga 2160
   gggaaaa 2167
<210> 166
   <211> 897
   <212> RNA
   <213> Homo sapiens
<400> 166
   augacagaug ccgcuguguc cuucgccaag gacuucuugg ccgguggagu ggccgcagcc      60
   aucuccaaga cagcgguagc acccaucgag agggucaagc ugcugcugca ggugcagcau     120
   gccagcaagc aaaucacggc agauaagcaa uacaagggca ucauagacug cgugguucgu     180
   auccccaagg aacagggagu ccuguccuuc uggcguggga accuggccaa ugucaucaga     240
   uacuucccca cccaggcucu caacuuugcc uucaaagaua aauacaagca gaucuuucug     300
   gguggugugg acaagaggac ccaguucugg cgcuacuuug cagggaaccu ggcaucaggu     360
   ggugccgcug gggcuacauc cuugugcuuu guguacccuc uugauuuugc ccguacccgu     420
   cuagcagcug augugggcaa agcuggagcu gaaagggaau ucaaaggccu uggugacugc     480
   cugguuaaga ucuacaaauc ugaugggauu aagggccugu accaaggcuu uaauguguca     540
   guacagggca uuaucaucua ccgagcugcc uacuuuggua ucuaugacac ugcaaaggga     600
   augcucccag aucccaagaa uacucacauc uucaucagcu ggaugauugc acagucuguc     660
   acugcugucg cuggccugac uuccuauccu uuugacacgg uucgccgucg uaugaugaug     720
   cagucuggac gcaaaggaac ugauaucaug uacacaggca cgcuugacug cuggcggaag     780
   aucgcgcgcg augaagggag caaggcuuuu uucaagggcg caugguccaa cguucucaga     840
   ggcaugggug gcgccuuugu gcuugucuug uaugaugaga ucaagaaaua cacauaa        897
<210> 167
   <211> 3873
   <212> RNA
   <213> Homo sapiens
<400> 167
   cguagaaguu gucucugucg gcgggcgggc gggcaggauu ggggcaccga gaccggcgug      60
   cggacagcag ggaucgcggg gagcgagggg ugcggcaugg agcuccgggc agucgguuuc     120
   ugccuggcgc ugcugugggg uugcgcgcug gcggccgcgg cggcacaggg aaaggaaguu    180
   guuuuguugg acuucgcagc aaugaaggga gagcucggcu ggcucacgca ccccuauggc     240
   aaaggguggg accugaugca gaacaucaug gacgacaugc cuaucuacau guacucggug    300
   ugcaacgugg uauccggcga ccaggacaac uggcuccgca ccaacugggu guaccgggag     360
   gaggccgagc gcaucuuuau ugagcucaag uucacggugc gagacuguaa cagcuucccg     420
   gguggcgccc augccugcaa agagaccuuc aaccucuacu augcagaguc agauguggac     480
   uauggcacca acuuccagaa gcgccaguuc accaagauug acaccaucgc cccugacgag     540
   aucacggucu gcagugacuu cgaggcucgc aacgucaagc ugaacguaga ggagcgcaug    600
   guggggcccc uuacccggaa gggcuucuac cuggccuucc aggacaucgg cgccugcgug    660
   cggcugcucu ccguucgcgu cuacuacaag aagugucccg agaugcugca gagcuuggcc     720
   ugcuuccccg agaccauugc ugucgcuguu uccgauacac aaccccuggc cacgguggcc      780
   gguaccugcg uggaccaugc cguggugccu uaugggggcg agggggcucu caugcacugc    840
   acgguggaug gcgaguggcu ggugccaucc gagugccugu gccaggaagg cuacgagaag     900
   gucgaggaug ccugccgagc cuguucucca ggauucuuca agucugaggc aucugagagc     960
   ccuucccugg aguguccaga gcauacccug ccauccacag agggugccac cuccugccag 1020
   ugugaagaag gcuauuucag ggcaccugag gacccacugu ccaugucuug cacacgucca 1080
   cccucugccc cuaacuaccu cacggcaugc augggugcca aaguagaacu gcguuggaca 1140
   gcucccaagg acacuggugg ccgccaggac auugucuaca gugucacuug ugaacagugc 1200
   ugcgcagagu cuggcgagug ugggcccugu gaggccacgg ugcgcuauuc agaaccuccu 1260
   cacgcccuga cccgcacgag ugugacaguc agugaccugg agccccacau gaacuauacc 1320
   uucgcugucg aagcacgcaa uggcgucuca ggccugguga cuagccgaag cuuccggacu 1380
   gccagcguca guauuaacca aacagagccc cccaaaguga ggcuggagga ccgaagcacc 1440
   accucccuga gugucaccag gagcaucccg gugucacagc agagccgugu guggaaguac 1500
   gaagucaccu accgcaagaa gggggaugcc aacagcuaua auggccgccg cacggaaggc 1560
   uucuccguga cccuggauga ccuugcuccg gauaccacgu accuggugca ggugcaggca 1620
   uggacgcagg agggccaagg agccggcagc aaagugcacg aguuccagac gcuguccacg 1680
   gaaggaucuc gcaacauggc ggugaucggc gguguggcug uagguguugu uuugcuucug 1740
   guacuggcag gaguuggccu cuucauccau cgaaggagga ggaaccugcg ggcucgccag 1800
   uccucugagg auguccguuu uuccaaguca gaacaacuaa agccccugaa gaccuaugug 1860
   gauccucaca cuuacgaaga ccccaaccag gcuguacuca aguuuaccac cgagauccac 1920
   ccauccugug uggcaaggca gaaggucauu ggagcaggag aguuuggaga ggucuauaaa 1980
   gggacgcuga aggcauccuc ggggaagaag gagauaccgg uggccaucaa gacacugaaa 2040
   gcgggcuaca cugagaagca gcggguggac uuccugagcg aggccagcau caugggccag 2100
   uuuagccacc acaauaucau ccgccuggag gcgguggucu cuaaauacaa acccaugaug 2160
   auuaucacag aguacaugga gaauggagcg cuagacaagu uccuuaggga gaaggauggu 2220
   gaguucagcg uacuucaguu ggugggcaug cugaggggua ucgcauccgg caugaaguac 2280
   cuggccaaca ugaacuacgu gcacagagac cuggcugccc gcaacauccu cgucaacagc 2340
   aaccuggugu gcaagguguc cgauuuuggc cugucgcgug ugcuggagga ugaccccgag 2400
   gccaccuaca ccacaagugg cggcaagauc ccuauucgau ggacagcccc agaggccauu 2460
   uccuaccgca aguucaccuc agccagcgau guguggagcu acggcauugu caugugggaa 2520
   gugaugacuu auggcgaacg gcccuuacug gaacugucaa accacgaggu caugaaagcc 2580
   aucaacgacg gcuuccggcu ccccacgccc auggacugcc cuucagccau uuaccagcuc 2640
   augaugcagu gcuggcagca agagcgcucc cgccggccca aguuugccga caucguuagc 2700
   auccuggaca agcucauccg acgccccgac ucccucaaga cgcuggcuga cuucgauccc 2760
   cgagugucca uccggcugcc cagcaccagc ggcucggagg gaguccccuu ccguacggug 2820
   uccgaguggc uggagagcau caagaucgaa caguacacgg aacacuucau gguggcuggc 2880
   uacacggcca ucgagaaggu gguacagaug uccaacgaag acaucaaaag gaucggagug 2940
   cgucuuccug gccaccagaa gcgcauugcc uacagccugc ugggacucaa ggaccagguc 3000
   aacacagugg ggauuccuau cugaguccau uggggcuguc acacaauacu ugaagagcca 3060
   caguggucuc ccugccgauc uggugcuggc ccacuggaac uuuauuuauu ucuguuuccu 3120
   cgucuaugcc ucccuaggac ucugcagggg cuuuuugaau gacaccugcc ugagccuggg 3180
   aaacuuggau ugcuggucag ggcucucuuu ccccugaaaa ggacccagcu aagaacuuag 3240
   caguuugcca uggccuuccc agcauccccu gaggcuaaag uuccaccaag agucgauauc 3300
   gacgagggac auuuccaaac ggaccucccc aucuucauuu ggccuccuga gaagccacuc 3360
   uggagcugag gcuaagcacu aagcccagga ccauaugacu agcacuguac cgcccgcccc 3420
   uaguuagagg guagguuuug gacuuggcug gguugugguc acaagcaauc ucccagugcc 3480
   uuuuacagac cccagucugc ccucccgucg agggccagcu ucuugcuuuc cuagggcccu 3540
   cucaggaugc uuggcugugc ugagguuuuu auuaaauaua uauuuuauac uugcggaaag 3600
   aaugagugug uggcagggac uugccagggc uggagacaga ggauccccug caacaagaca 3660
   uucccgggcu gggggcuggc ggaccugcag gagacuuucc gccaccaccc cgucuccagc 3720
   cccuuuggac aaaugucgcu gucaguguua cagauuucuu uuauuggguu guuuuuuugu 3780
   uguauuuuuu ugaaccuuaa cuuauuauuu uuuuuauauu uauuguuaga aaaugacuua 3840
   uuucugcucu ggaauaaagu ugcagauggu uca 3873
<210> 168
   <211> 1591
   <212> RNA
   <213> Homo sapiens
<400> 168
   ccaaaaaccu uaauuuucuu ucuuguucgg uaccuacauu ggaaccacca aaaacaauua        60
   uuucaguaaa ccguagccau gagggaaauc gugcacaucc aggccggaca guguggcaac       120
   cagaucggug cuaaguucug ggaggucaua agcgaugaac auggcaucga ccccaccggu       180
   accuaccacg gugacagcga ccugcagcug gaccgaaucu cuguguacua uaaugaagcc       240
   acagguggca aguauguccc ucgagcuauc uugguggauc uagaaccugg gacuauggac       300
   uccguucgcu cagguccuuu uggccagauc uucagaccag acaacuucgu uuucggucag       360
   ucuggggcag gcaacaacug ggcuaaaggc cacuacacag agggagcuga guugguugac       420
   ucugucuugg auguggugcg gaaggaggcg gagagcugug auugccugca aggcuuucag       480
   cugacccacu cacugggugg aggcacuggc ucuggcaugg gcacccugcu caucagcaag       540
   auccgggaag aauauccuga ccguaucaug aauaccuuca guguggugcc cucgcccaaa       600
   gucucugaua ccguggucga gcccuacaau gccacccugu cuguccauca guugguugag       660
   aacacggaug agaccuacug caucgacaac gaggcccucu acgacaucug cuuccguacc       720
   cucaagcuca ccacgccaac cuacggagac cugaaccauc ucgucucggc caccaugagc       780
   ggcgucacca ccugccuccg uuucccgggc cagcuuaaug cugaccuucg aaagcuggcu       840
   gucaacaugg ugccauuccc acgucuccac uucuucaugc cuggcuuugc cccucucacc       900
   agccguggaa gccagcagua ccgggcccuc acugugccug aacuuaccca gcaggucuuc       960
   gaugccaaga acaugauggc cgccugcgac ccgcgccacg gccgguaccu cacaguugcc      1020
   gccgucuucc guggacggau guccaugaag gagguggaug agcagaugcu caacgugcag      1080
   aacaagaaua gcagcuacuu cguggaaugg auccccaaca augucaagac agcugucugu      1140
   gacaucccac cgcguggccu caagauggca gucaccuuca uuggaaacag cacagccauc      1200
   caggagcugu ucaagcgcau cucugagcag uuuacggcua uguuccgccg gaaggcuuuc      1260
   cuccacuggu acacggguga gggcauggac gagauggagu ucaccgaggc ugagagcaac      1320
   augaacgacc uggugucuga guaccagcag uaccaggaug ccaccgcgga agaggaagag      1380
   gauuucggag aggaggcaga agaggaggcc uaacggcaga gagcccugca ucagcucagg      1440
   cugcuuagau cccucagccu uucuccaacu gcccuuuguc cuccaguuuc uuucugcugc      1500
   cucugucuug uauuuguuuu gcuucuguuu ucucauuggg gguaaauggu gccuggcaca      1560
   uggcaggcac ucaauaaaua uuuguuugug g                                     1591
<210> 169
   <211> 1992
   <212> RNA
   <213> Homo sapiens
<400> 169
   ggcagacaaa agaggccggc agugcagcuc gcgggacgca uggccgggcg cggaggacgg     60
   gugcugcugg cgcugugugc cgcgcuggug gccggcgggu ggcugcugac gcgugaagcc    120
   caggagcccg gggcgccagc ggcuggcaug aggcgccgcc ggcggcucca gcaagaggac    180
   ggcaucuccu ucgaguacca ccgcuaucca gagcugcgcg aggcgcuggu gucgguaugg    240
   cugcagugca ccgccaucag cagaaucuac acagugggcg ccagcuucga gggccgggag    300
   cuccugguca ucgagcuguc ugacaacccc gggguccaug agccggguga accugaauuu    360
   aaauacaucg ggaacaugca uggcaaugag gcgguuggac gggaauugcu cauuuucuug    420
   gcccaguacc uguguaacga guaccagaaa ggcaaugaga caauugucaa ccugauccac    480
   agcacccgaa uucauaucau gcccuccuug aaccccgacg gcuuugagaa agccgcaugg    540
   cagcccgggc agcugaagga cugguuugug ggccgcagca acgcccaggg aauagaucug    600
   aaccguaacu ucccagaccu ggacaggauc guauauguua augagaaaga aggcgguccc    660
   aacaaccacc ugcugaagaa ucugaagaaa auuguggacc aaaauucaaa gcuugccccc     720
   gagaccaagg cugucauuca cuggaucaug gacauuccau uugugcuuuc ugccaaucug     780
   cacggaggag accuuguggc uaauuaccca uaugaugaga cacggagcgg uacugcucac     840
   gaauacaguu ccugcccuga ugacgcaauu uuccaaagcu uggcucgcgc guacucuucu     900
   uucaacccag ucaugucuga ccccaaucga ccucccugcu gcaagaauga cgaugacagc     960
   agcuuugaug auggaacgac caaugguggu gcaugguaca gcguccccgg uggaaugcaa    1020
   gacuucaauu accugagcag cagcaacugc uuugagauca cuguggagcu uacgugugag    1080
   aaguucccac cugaagagac ucucaaaagc uacugggaag auaacaaaaa cucccucauc   1140
   aacuaccugg agcagauaca ccgagguguu aaaggguuug uccgugaccu ucaggguaac     1200
   ccgaaugcca acgcaaccau cucuguggau gggauagacc augaugucac cucggcuaag   1260
   gauggggauu acuggcgauu gcuugcuccu ggaaacuaua aacuuacagc cuccgauccu   1320
   ggcuaccugg caaucacaaa gaaaguggca guuccuuuua gcccugcugu ugggguggac   1380
   uuugagcuug agucuuucuc ugaaaggaag gaggaggaga aggaagaauu gauggagugg    1440
   uggaaaauga ugucagaaac uuugaauuuu uaagaaaggc uucuaacuaa uugcuuucau    1500
   cuaucuauag acuguaguaa gaugcaaugu ggcucuuuuc uuuuagguug ugugcaguug    1560
   auauuuaaca uugauuuauu uuugaucauu aaguaauagu uacuaaucac guaaauacac    1620
   ccggacagaa auauaaugcu ggacaucuuc auucuacauc aacauucgcu uaaaucauuc    1680
   gaagccucuu uuaacguaau gggugacaau gucacuugac agaugcauga gagucacgau    1740
   auagcugacu gugacccugc acugcaauca cauaguucca uauaaguugu ccuuagucuc    1800
   uugugcugau ucacuguaua agcaugaucc ugguaaugca cuuuggaugg gaagaaaaug    1860
   uacgugcuuu ucagaggggc ucugaacaga augaaaaccu aguucuugcg uguacuuuga    1920
   agaauggaau uguauuaguc agcuguuaau gccacuucag aaguuugggg uuuugucuug    1980
   auuguagauu gg                                                        1992
<210> 170
   <211> 1828
   <212> RNA
   <213> Homo sapiens
<400> 170
   gccacggacg ccucucugaa cggggaucca ggcaggauua gagcugccuc acugacuaca     60
   ggccgugucg ugucaccguu ucugcaggca ccaugagcca ggacaccgaa guggacauga    120
   aagaugugga gcugaacgag cuagaaccgg agaagcagcc caugaaugca gcggacgggg    180
   cggcggccgg ggagaagaac ggucugguga agaucaaggu ggcggaggac gagacggagg    240
   ccggggucaa guucaccggc uuauccaagg aggagcuacu gaagguagcg ggcagcccug    300
   gcugggugcg cacccgcugg gcgcugcugc ugcucuucug gcucgguugg cugggcaugc    360
   uggcgggcgc cgugguuauc aucguucggg cgccgcgcug ccgugagcug ccuguacaga    420
   ggugguggca caagggcgcc cucuaccgca ucggcgaccu ucaggccuuu guaggccggg     480
   augcgggagg cauagcuggu cugaagagcc aucuggagua cuugagcacc cugaagguga    540
   agggccuggu guuaggccca auucacaaga accagaagga ugaaaucaau gaaaccgacc    600
   ugaaacagau uaaucccacu uugggcuccc aggaagauuu uaaagaccuu cuacaaagug    660
   ccaagaaaaa gagcauucac aucauuuugg accucacucc caacuaccag ggccagaaug    720
   cgugguuccu cccugcucag gcugacauug uagccaccaa aaugaaggaa gcucugaguu    780
   cuuggcugca ggacggugug gaugguuucc aauuccggga ugugggaaag cugaugaaug    840
   cacccuugua cuuggcugag uggcagaaua ucaccaagaa cuuaagugag gacaggcuuu    900
   ugauugcagg gacugagucc ucugaccugc agcaaauugu caacauacuu gaauccacca    960
   gcgaccugcu guugaccagc uccuaccugu caaauuccac uuucacuggg gagcguacug   1020
   aaucccuagu cacuagguuu uugaaugcca cuggcagcca auggugcagc uggagugugu   1080
   cgcaagcagg acuccucgca gacuuuauac cggaccaucu ucuccgacuc uaccagcugc   1140
   ugcucuucac ucugccaggg acuccuguuu uuagcuacgg ggaugagcuu ggccuucagg   1200
   gugcccuucc uggacagccu gcgaaggccc cacucaugcc guggaaugag uccagcaucu   1260
   uucacauccc aagaccugua agccucaaca ugacagugaa gggccagaau gaagacccug   1320
   gcucccuccu uacccaguuc cggcggcuga gugaccuucg ggguaaggag cgcucucugu   1380
   ugcacgguga cuuccaugca cugucuuccu caccugaccu cuucuccuac auacgacacu   1440
   gggaccagaa ugagcguuac cugguggugc ucaacuuccg agauucgggc cggucagcca   1500
   ggcuaggggc cuccaaccuc ccugcuggca uaagccugcc agccagcgcu aaacuuuugc   1560
   uuaguaccga cagugcccgg caaagccgug aggaggacac cucccugaag cuggaaaacc   1620
   ugagccugaa uccuuaugag ggcuugcugu uacaguuccc cuuuguggcc ugauccuucc   1680
   uaugcagaac cuaccacccu ccuuuguucu ccccaggccu uuuggauucu agucuuccuc   1740
   uccuuguuuu uaaacuuuug cagauuacau acgaauucuu auacugggug uuuuugucuu   1800
   caaauaaaaa caucaccccu gccucaug                                      1828
<210> 171
   <211> 2610
   <212> RNA
   <213> Homo sapiens
<220>
   <221> misc_feature
   <222> (2)..(2)
   <223> n is a, c, g, or u
<220>
   <221> misc_feature
   <222> (11)..(11)
   <223> n is a, c, g, or u
<220>
   <221> misc_feature
   <222> (26).. (26)
   <223> n is a, c, g, or u
<220>
   <221> misc_feature
   <222> (41)..(41)
   <223> n is a, c, g, or u
<220>
   <221> misc_feature
   <222> (45)..(45)
   <223> n is a, c, g, or u
<220>
   <221> misc_feature
   <222> (56)..(56)
   <223> n is a, c, g, or u
<220>
   <221> misc_feature
   <222> (70)..(70)
   <223> n is a, c, g, or u
<220>
   <221> misc_feature
   <222> (2262)..(2262)
   <223> n is a, c, g, or u
<220>
   <221> misc_feature
   <222> (2276)..(2276)
   <223> n is a, c, g, or u
<220>
   <221> misc_feature
   <222> (2288)..(2288)
   <223> n is a, c, g, or u
<220>
   <221> misc_feature
   <222> (2290)..(2290)
   <223> n is a, c, g, or u
<220>
   <221> misc_feature
   <222> (2298)..(2298)
   <223> n is a, c, g, or u
<220>
   <221> misc_feature
   <222> (2309)..(2309)
   <223> n is a, c, g, or u
<220>
   <221> misc_feature
   <222> (2311)..(2311)
   <223> n is a, c, g, or u
<220>
   <221> misc_feature
   <222> (2324)..(2324)
   <223> n is a, c, g, or u
<220>
   <221> misc_feature
   <222> (2333)..(2333)
   <223> n is a, c, g, or u
<220>
   <221> misc_feature
   <222> (2335)..(2335)
   <223> n is a, c, g, or u
<220>
   <221> misc_feature
   <222> (2412)..(2412)
   <223> n is a, c, g, or u
<220>
   <221> misc_feature
   <222> (2418)..(2418)
   <223> n is a, c, g, or u
<220>
   <221> misc_feature
   <222> (2435)..(2435)
   <223> n is a, c, g, or u
<220>
   <221> misc_feature
   <222> (2447)..(2447)
   <223> n is a, c, g, or u
<220>
   <221> misc_feature
   <222> (2462)..(2462)
   <223> n is a, c, g, or u
<220>
   <221> misc_feature
   <222> (2475)..(2475)
   <223> n is a, c, g, or u
<220>
   <221> misc_feature
   <222> (2483)..(2483)
   <223> n is a, c, g, or u
<220>
   <221> misc_feature
   <222> (2493)..(2493)
   <223> n is a, c, g, or u
<220>
   <221> misc_feature
   <222> (2513)..(2513)
   <223> n is a, c, g, or u
<220>
   <221> misc_feature
   <222> (2523)..(2523)
   <223> n is a, c, g, or u
<220>
   <221> misc_feature
   <222> (2535)..(2535)
   <223> n is a, c, g, or u
<220>
   <221> misc_feature
   <222> (2552)..(2552)
   <223> n is a, c, g, or u
<220>
   <221> misc_feature
   <222> (2555)..(2555)
   <223> n is a, c, g, or u
<220>
   <221> misc_feature
   <222> (2566)..(2566)
   <223> n is a, c, g, or u
<220>
   <221> misc_feature
   <222> (2590)..(2590)
   <223> n is a, c, g, or u
<400> 171
   gncuuaagcc ncguuuauuu ugaugnccug uuggcucagu naugnccaag augccnauug      60
   uuuuugcccn aaauaaauuu acugaacuug ggcuaaaacc aaaccuuggc acacaggugu     120
   gauacaacuu aacaggaauc aucgauucau ccauaaauaa uauaaggaaa aacucaagug     180
   guagccuguc uuaaggcuuu ugauacuugc agauugggga aaaacaaaca acaaacgucu     240
   ugaagcauau uaauggaauu aguuucuaau guggcaaacu guauuaaguu aaaaguucug     300
   auuugcucac ucuauccugg auagguauuu agaaccugau aauagucuuu aaacaagcca     360
   uuccagucau gaugagguga uguauggaua caugcauaca uucaaagcac uguucucaaa     420
   guuaaugcaa guaaauacag caauuccucu uucaauguuu aggcagaucg uuaacuauga     480
   gcuagccaaa ugugggcaug uuauuacagg gaaaguuuaa aggucugaua acuugaaaua     540
   gguuuaggag aauucaucua cuuagacuuu uuaaaugccu gccauaaaaa auugaaaugg     600
   uagaauggcu gaccacagca augaccagcc cucaccuagg gcucuggaug auuuuugguc     660
   uaauaacgca ugcuaguguu gauguuuuuu ggucaagaug ggaaugaaca ggaagaauua     720
   ugcagcaggc uuuauuuuaa augccgauuc acauuacucu guucaagcug cguugagaug     780
   uuaaacuggc uuacuauaga cuuuguaaaa aaaaaaaaaa ccaaacaaau ggcuccagaa     840
   gaguaacaaa cugaaaucug agaucacaca gguuggaaau auguacauaa cugaacaagg     900
   ugucaauucu gcucuacagu gcaguuuagu caguuuuagu ugcauagguu uccauuguuu     960
   uuauagucug uuuaugcuaa aucuggccaa agaugagcau uguccaccac uaaaaugccu 1020
   augccacugg gaauucuggg uuaauuuugu gaccagaaug cagugaucaa aauguucaau 1080
   cuuuuuacag uggcauagga agauggcaaa aauuuccuaa agugcaauag auuuucaagu 1140
   guauugugcc uuguucuaaa acuuuuauua aguaggugca cuugacagua uugaggucau 1200
   uuguuauggu gcuauuucaa uuagucuagg uuuaggcccu uguacauuug gcccauaacu 1260
   uuuuacaaag uacuucuuuu auugcacauu cagagaauuu uauauauaug ucuugugugc 1320
   guguccuuaa acuuccaauc uuauuuuguc ucuuggagau uguugaacgc agcuugucua 1380
   ggaaagggau gggacuagau ucuaaaauuu auuugggacc augggaauga uaguugggaa 1440
   gaaacuuugc acacgacaga uuucuagaua cuuuuugcug cuaguuuuau guaauauuua 1500
   uugaacauuu ugacaaauau uuauuuuugu aagccuaaaa gugauucuuu gaaaguuuaa 1560
   agaaacuuga ccaaaagaca guacaaaaac acuggcacuu gaauguugaa ugucaccgua 1620
   ugcgugaaau uauauauuuc gggguagugu gagcuuuuaa uguuaaguca uaauaaacuc 1680
   uuaagucaaa uuaagcagac ccggcauugg cguguagcca uaacuuucug auguuaguaa 1740
   aaacaaaauu ggcgacuuga aacuaaauca ugccaagguu ugauacacuu gucuugagau 1800
   auuaacgaaa cacuuccaaa cacugauaca aaguguccag auucucagau guuuguugug 1860
   ugaguuuugu uuaguuguau uuuuuuuuca gugaaugucu ggcacauugc aauccucaaa 1920
   caugugguua ucuuuguugu auuggcauau ucagugacuu guacauucag caauagcauu 1980
   ugagcaaguu uuaucagcaa gcaauauuuu caguuauguu uccaaauuaa gaauggguuu 2040
   aaacuugcug aauguaaaga uugacccuca agucacugua gcuuuaguag uugcuuauug 2100
   uauuaguuua gaugcuagca cugcaugugc ugugcauauu cugguuuuau uaaaauaaaa 2160
   aguugaacug cacagucucc uuuguuguug ucaauugugg uuuacuuuua gaggugaaaa 2220
   uaaaguugug cucuugccuc gugccaauau guacauaacu gnacaaggug ucaauncugc 2280
   ucuacagngn aguuuagnca guuuuagung nauagguucc caunguuuuu aungncuguu 2340
   uaugcuaaau cuggccaaag augagcauug cccaccacua aaaugccuau gccacuggga 2400
   auccuggguu anuuuggnga ccagaaugaa gugancaaaa uguccanucu uuuuacagug 2460
   gnauaggaag auggnaaaaa uunccuaaag ugnaauagau uuccaagugu uungugccug 2520
   guncuaaaac uuuunuuaag uagggugcac unganaguau ugaggncauu gguuauggug 2580
   cuauuuccan uuagucuagg uuuaggcccu 2610
<210> 172
   <211> 3657
   <212> RNA
   <213> Homo sapiens
<400> 172
   cucggucucc aagauggugg cuaacaaaac gugaggccua gagguugauc cuaggucacu 60
   ggaagcaugu ccuugaagag gacuaugggc aucuggguuc uuccucuucu acuuggccac 120
   gagguaaccg gcuucuugua ccacgugugc ucaccucuac aaucugugcg gauucagacu 180
   ccaagcaacu aggcuaccca gugacaggcu aaaaacuaca gcucuaacgu cuuggaaggc 240
   gauuccaugg accaggaugu ggaaagccca guggccauuc accagccaaa guugccuaag 300
   caggccaggg acgaccugcc gagacacauc agccgagaca ggaccaaaag gaaaauccag 360
   agguacguga ggaaggaugg gaagugcaac guucaccacg gcaaugugcg ggagacguac 420
   cgauaccuga cggacaucuu caccacccug guggaccuga aguggagauu caaccuguug 480
   aucuuuguca uggucuacac agugacgugg cuuuucuuug ggaugaucug guggcugauu 540
   gcguacaucc ggggagauau ggaccacaua gaggaccccu cguggacucc uugugucacc 600
   aaccucaacg gguuugucuc ugcuuuuuua uucuccauag agacagaaac caccaucggu 660
   uauggcuacc gggucaucac ggacaagugc ccugagggga uuauucuccu cuuaauccag 720
   uccguguugg gguccauugu caacgccuuc augguaggau guauguuugu gaaaauaucc 780
   caacccaaga agagggcaga gacccugguc uuuuccaccc acgcggugau cuccaugcgg 840
   gaugggaaac ugugcuugau guuccgggug ggggacuuga ggaauucuca cauuguggag 900
   gcauccauca gagccaaguu gaucaagucc aaacagacuu cagaggggga guuuauuccc 960
   cucaaccaga cugauaucaa cgugggguac uacacagggg acgaccggcu cuuucuggug 1020
   ucaccauuga uuauuagcca ugaaauuaac caacagaguc ccuucuggga gaucuccaaa 1080
   gcgcagcugc cuaaagagga acuggagauu guggucaucc uggagggaau gguggaagcc 1140
   acagggcagu ucugaaaucg aaaucaagca gaggucuaua agaacacccu ggagaccccc 1200
   augcugcugg aucuuggguc caugaacugc uuuuauuugc ugcaaucaaa aaugcuaguc 1260
   gcugaucuga uaggagagga aacgagacuc agagccugaa ggauaacacg cugagggcug 1320
   auuucauaca cucuucccgg cuggaucauc ccagccccca cagcguccag gcuuaguuuc 1380
   uuccuuuguu uuaacaaucu auacuuccuc cagccugggc gagcuaguau acccagaguu 1440
   ugguuuuguu ucuuuucaga gcuguaagcc cagugcccag ugaccucauc ugggaggaga 1500
   guuaagcaau aagaccugaa augcuaaacu cugggguaga aaaccucugc agagacagcg 1560
   ucucugggaa gucucuacag agacagagua gggaagucuc acgaagguca gagucuuacu 1620
   uccauaccug gagaaauccg cccguccguu cagugccggu uucaacuccu cugccaaaga 1680
   cuucuuucca agacacuggu aucagcuauc ccagcaguaa cuuuggcagu auauaaauca 1740
   auggcacugc cccauaaaac cccguugagu aaaaccuaug gucuucaaca gcuggggagc 1800
   cuguagccca gcuccugugu gaggcuaagg cuguggagac caugccugcu caccuccucg 1860
   uugaaaagca aaacacugua agaaaccuaa caugacuuuu uccaacauuu cucaggcugg 1920
   ggagaagacu ugguccacaa agagcuugag gucuugaucu ggaucccuag cauccaugga 1980
   agaagguggg caugaucacc cacacuccuc aucccauagc ucugaaugag guaggcagau 2040
   uccuggggcu caccagcuca uccuaacagc caggucccaa ugagggacug ucuacaaaag 2100
   aacaagaggu ggauccugag aagcgacacc caagcuugac cucuagcccc uguacacacc 2160
   uguuaagcac cugggcaucg ggacacacac ucgcaauuug caaacaaggc aaauucucac 2220
   acuucagaag gcacugagag aaucccauaa gcucaaaaag uuaggagcca agauugacca 2280
   uuaaugacuu ggguugaaaa gacuaaacca cuggauagaa uguuucauua cuaaaacacc 2340
   uuaccugaaa aguauuacug cucuucuuuc ugcaacugga ccaugcagag auccacaaag 2400
   agaugcccag agauuauuag ugauggucau augauauaca caauguggac ucuaagccaa 2460
   ggccccauca acucagaucc agaggcugac agugugcuua ucuuagagau accacagugg 2520
   cugccuaauc accacguccu uaagucaggg gaguuugauu auuuccuaug aacaccgagu 2580
   ggggacagcg gauuaccaau gaagcaaucc aaccugacaa uccuaaccac ccagaagagg 2640
   aucgcugggg gaaagcauga auuauuuacg ugucaacaug ugcacacucc ggccacgcag 2700
   cucaacagcc aggagugcuu cuccacuuag cccugcccug gcauccauuu augaucucgu 2760
   cuguuguguu aauuacccag cuagcuuuuu ucucaaaaau aauaucuccc agccauagac 2820
   cuacucaucu guguccucuu uaauuucaac ccacaguuac aucauucacu ggcuugcuca 2880
   guuucuucaa cucugaaaug ggaaugauga ugaugaugcc ucuuccccca agccaccauc 2940
   cucccccgug accuuccuag guacagacuc aaaccaggga agauuauuuc cuucucauag 3000
   gccacagggu gaaaugcaau aaagaacaaa gccuuguagg gaggcagagg gaaagaccag 3060
   uccucacaag aggcuccauu guuuccaggg acuuugaagc uuggacagaa ugucagauga 3120
   uccccuuuga aggugcucca aagagucaau gugaaaaaua uugacugaug ugugcccguc 3180
   cacaagccaa ggugccucug cccuggucac cuaccaugaa uuauaaacug augauauuug 3240
   aaauaauaag gaacacugga gccggccaga aaggauucug cagucccaua aauagcaaca 3300
   uucaucacua caaugccugc caacgguggc cgugaaugua gauuaccccc ggcucuucug 3360
   aggccacuga ggacagggca aacuaccucu gagaauggag gcucacuguu cugcauccca 3420
   cuucuaaaug uuccaugaau uuuuugagac aucucccaua ucccuguuag aaagauucaa 3480
   ccuugugcua uuaaccaaau cauuuugaau uccauaaacc ucuacucuaa aguauacacu 3540
   uaauucuaca auacagacaa caaauaugac uuuuuccuau gaaagaguga uaaagauacu 3600
   guaucagucu gcuuugacuc aaaaaaaaaa aaaaaaaaaa aaaaaaaaaa aaaaaaa 3657
<210> 173
   <211> 2267
   <212> RNA
   <213> Homo sapiens
<400> 173
   uggaaugggu gcuaggugaa ggcuuuguug aaucaggaug cugagcuggu guuuuuacag 60
   guccagauau gaugagcugg aguuuuggua ggcccaggua ccuccuccaa aauugaccac 120
   auaauugguc acaaaucagg ccuccacaga uacaaaaaua uugaaauuau cccaugcauc 180
   cugucugauc accacagacu aaggcugauc uucaauaaca acauaaaaau agaaagccag 240
   guucacugcu gcggcugcgc cuccuuguuc ucagcgucac cacugccgcc augcccggag 300
   gguugcuucu cggggacgaa gcccccaacu uugaggccaa uaccaccauc ggccgcaucc 360
   gcuuccacga uuuccuggga gauucauggg gcauucucuu uucccaccca cgggacuuua 420
   ccccagugug caccacagaa cuuggcagag cugcaaagcu ggcgccagag uucgccaaga 480
   ggaauguuaa guugauugcu cuuucaauag acaguguuga ggaucaucuu gccuggagca 540
   aggacaucaa ugcuuacaau ggugaaacac ccacggaaaa guugccauuu cccaucauug 600
   augauaaggg cagggaccuu gccauccuuu ugggcauguu ggauccaguc gagaaggacg 660
   auaacaacau gccugugacg gcccgugugg uguucauuuu uggcccugac aagaaacuga 720
   agcugucuau ccucuacccu gccaccacga gcaggaacuu ugaugagauu cucagagugg 780
   uugacucucu ccagcugaca ggcacaaagc cgguugccac cccaguugac uggaagaagg 840
   gagagagcgu gaugguaguc cccacccucu ccgaagagga agccaaacaa uguuucccua 900
   aaggagucuu caccaaagag cucccgucug gcaaaaaaua ccuccguuau acaccccagc 960
   cuuaagucuu ugcggaaauu ggggcugcau cugcacgucc agcacugggg ccugaggacg 1020
   ucagccggca gccgugggac cuugcagcag guccguagaa agaucguggc augaucacag 1080
   ccgguccugu agaucgcucg cuauacuacu gggucauuaa auggaaaugg caccaaaacc 1140
   uucucgggau ucuuuacucu gugccuucgc cagcauucug ccccucugcc ugucacagug 1200
   cccuacugac uggcucucuu ugaaacgaau uauguauuga agauuccuua ggucucugca 1260
   gggucuuuga ucagcaagca agguaguguc agugugggcu cugugcuaga augaugaaac 1320
   accuuuugua gcuuuccgaa cggaaucuuc uguuacccau uuuggagagc acugacaugg 1380
   ggagaagcuu ucaauucugu auuuuuagua aauaaagugg ggacagccgg gagaauucuu 1440
   acagggaauc uauuguaagu uucuaucgaa gugggcucag aaagccuuuc gccucccaag 1500
   agugcgcaug uaccuccuag aguuuccaca ucugcucucu ggugaugucu gccugugaac 1560
   gcaccuuaua aaagacgggc ggugacagug uuuuaccacu caguguccua guagugggug 1620
   gccauuucug aauucugcuu uuugagguuc aacaaauaaa auccugauca gaaaaaaaaa 1680
   aaaauagaaa gccaacauuc auguggaaac ugaacaacac uacucaauga uuccuugguc 1740
   agagaugaaa uaaagaaaga aauuaaagac uuuuuagagu uuaaugaaaa ugaagccaca 1800
   acauacccaa acuuauggga cacaaugaag gcauuucuaa gaggaaaacu cauagcccug 1860
   agugcaucca aaggaaaaaa aaaaaaaccu agagagagug uacacuagca gccugacugc 1920
   acacuuagaa gcucugcaaa aaaaggaauc aaauucaccc aagaggaaua gacagcagga 1980
   aauaaucaaa cuuagggcug aaaucaacca aauggaaaca aaaagaacua uucaaagagu 2040
   gggccaaacc aggagcuagu ucuuugagaa aaucaacaag auagauaaac ccuuagccag 2100
   acucacuaga gggcacaggg acagcauccu aauuaacaaa aucagaacug aaaagggaga 2160
   cauaacaaca gauccugaag aaauccaaaa caccaccaga uccucuacaa aaggcuauac 2220
   ucaacaaaac uggaaaaccu ggaugaaagg aaaagcuucu agacaga 2267
<210> 174
   <211> 2834
   <212> RNA
   <213> Homo sapiens
<400> 174
   augagcaccg cggaucugau gcgucgcugg gucaucgccc ugcuccuugc ugcugccgga      60
   guugcaguag aagacucagg cagcaggaac gaguuccagu guagagacgg aaaaugcauc     120
   gcuagcaagu gggugugcga uggcagcccc gagugcccgg auggcuccga ugagucccca     180
   aagacaugca ugucugucac cugucagucc aaucaauuca gcuguggagg ccgugucagc     240
   cgaugcauuc cugacuccug gagaugugau ggacagguag acugugaaaa ugacucagac     300
   gaacaaggcu guccccccaa gacgugcucc caggaugacu uccgaugcca ggauggcaag     360
   ugcaucuccc cgcaguuugu gugugaugga gaccgagauu gccuagaugg cucugaugag     420
   gcccacugcc cagccaccac uuguggcccc gcccacuucc gcugcaaauc auccauaugc     480
   auccccaguc uuugggccug cgacggggau gucgacugug uugacggcuc ccaugagugg     540
   ccacagaacu gccaggccga agacacggcc uccaaaggcg uuagcagccc cugcuccucc     600
   cuggaguucc acugugguag cagugagugu auccaucgca gcugggucug ugacggcgag     660
   gcagacugca aggacaaguc agaugaggag cacugcgcgg uggccaccug ccgaccugau     720
   gaauuccagu gugcagaugg cuccugcauu cacgguagcc gccaguguga ccgugaacau     780
   gacugcaagg acaugagcga cgagcucggc ugcgucaaug ugacacagug ugauggcccc     840
   aacaaguuca agugucacag uggggagugc aucagcuugg acaaggugug cgacuccgcc     900
   cgcgacugcc aggacugguc ggaugagccc aucaaggagu gcaagaccaa cgaguguuug     960
   gacaacaaug guggcuguuc ccacaucugc aaggaccuca agauuggcuc ugagugccug    1020
   ugucccagcg gcuuccgguu gguggaccuc cacaggugug aagauauuga cgagugucag    1080
   gagccagaca ccugcagcca gcucugugug aaccuggaag gcagcuacaa gugugagugc    1140
   caggccggcu uccacaugga cccacacacc agggucugca aggcuguggg cuccauaggc    1200
   uaucugcucu ucaccaaccg ccacgagguc cggaagauga cccuggaccg cagcgaguac 1260
   accagucugc uccccaaccu gaagaaugug guggcucucg acacggaggu gaccaacaau 1320
   agaaucuacu gguccgaccu gucccaaaaa aagaucuaca gcgcccugau ggaccaggcc 1380
   ccuaacuugu ccuacgacac caucaucagu gaggaccugc augccccuga cgggcuggcg 1440
   guagacugga uccaccgcaa caucuacugg acagauucag ucccaggcag cguaucugug 1500
   gcugacacca agggcguaaa gaggaggaca cuguuccaag aggcaggguc cagacccaga 1560
   gccaucguag uggacccugu gcauggcuuc auguacugga cagauugggg aacacccgcc 1620
   aagaucaaga aaggggguuu gaauggugug gacauccacu cacuggugac cgaaaacauc 1680
   caguggccaa auggcaucac acuagaucuu uccaguggcc gucucuauug gguugauucc 1740
   aaacuccacu cuaucuccag caucgauguc aaugggggca aucggaaaac cauuuuggag 1800
   gaugagaacc ggcuggccca ccccuucucc uuggccaucu augaggacaa aguguauugg 1860
   acagauguca uaaacgaagc cauuuucagu gccaaucgac ucacggguuc agaugugaau 1920
   uugguggcug aaaaccucuu guccccggag gacauugucc uguuccacaa ggucacacag 1980
   ccuagagggg ugaacuggug ugagacaaca gcccuccucc ccaauggugg uugccaguac 2040
   cugugccugc ccgccccaca gaucgguccc cacucgccca aauucaccug cgccugcccu 2100
   gauggcaugc ugcuggccaa ggacaugcgg agcugccuca cagaagucga cacuguacug 2160
   accacccagg ggacauccgc cguccggccu guggucaccg caucagcuac caggccaccg 2220
   aagcacagug aggaucucuc agcucccagu acuccuaggc agccugugga caccccaggg 2280
   cucagcacag uggcgucagu gacagugucc caccaagucc agggugacau ggcuggcaga 2340
   gggaaugagg agcagccaca ugguaugagg uuccugucca ucuucuuccc uauugcacug 2400
   guugcccucc uuguccuugg ggccguccug cuguggagga acuggcggcu gaagaacauc 2460
   acaaucaaca gcauaaacuu ugacaaccca gucuaccaga agaccacaga ggacgagcuc 2520
   cacauuugcc gaagccagga uggcuauacc uaccccucaa gacagauggu cagccuggag 2580
   gacgaugugg caugagcagc cgggagagcc gucucuuucc gggauccauu gccaagcuua 2640
   ggcagaaaag acacucucuc cagaccuccc cauccagcac ugguccugcc accucccugg 2700
   gucuguguug cucaaagcaa gauagagcaa agcugggcug gggggccaag cucagcuucc 2760
   ugucugcccc agguucuguu uuauauauuu auugucuggg acagaaaagg cuacuggcug 2820
   ugcuugaaau ucga 2834
<210> 175
   <211> 2940
   <212> RNA
   <213> Homo sapiens
<400> 175
   accggucauc gggcucuucc uugggcucag ggcacggauc aaguccucgg cucgcucggc      60
   cagcaccgcg gcaccauggc gcaggagcug cagcaccccg aguucgcgcg cgcaggccag     120
   caggcugggc ugcaggugug gagggucgag aagcuggaac ugguaccggu gccccagggu     180
   gccuauggug acuuuuacgu cggagagccc uaccuggugc ugcacaccac caaguccagc     240
   aggggcuucu ccuaccgccu gcauuucugg cugggaaagg aguguuccca ggaugagagc     300
   acagcggcug ccaucuuuac gguccagaug gacgacuauu uggguggcaa gccaguccag     360
   agcagagagc uucaaggcua ugagucgacu gauuuugugg gcuauuucaa aggcggucug     420
   aaguacaagg cuggaggugu ggcuucugga cuaaaccaug uccucaccaa ugaucugacu     480
   gcgaaaagac uucugcacgu gaagggucgg agagugguca gagccacuga aguuccccuc     540
   agcugggaga gcuucaacaa gggcgacugc uucaucauug accuuggcac cgaaauuuac     600
   caguggugug guuccuccug caacaaauau gagcgucuga aagcaagcca gguggccauu     660
   ggcauccggg acaaugagag gaaaggaaga ucucaacuca uuguggugga agaaggaagu     720
   gaaccaucag agcucaugaa gguuuuaggg agaaagccug agcuuccaga uggggacaau     780
   gaugacgaug ucguagcaga cauaaguaac aggaagaugg cgaagcucua caugguuuca     840
   gaugcaagug gguccaugaa aguaacacug guggcugaag aaaacccguu cuccauggga     900
   auguugcuuu cugaagaaug cuucauuuug gaccauggug cugcaaaaca aauuuuugua     960
   uggaaaggua aaaaugcuaa cccacaggag aggaagacug ccaugaagac agcugaggag    1020
   uuuuuacaga aaaugaagua uucuacuaau acucaaauuc agguucuucc ggaaggcggu    1080
   gaaacaccaa uuuucaaaca guucuuuaag gacuggaagg auaaagacca gagugauggc    1140
   uuugggaagg uguacaucac ggagaaagug gcucagauaa agcagauucc guuugaugcc    1200
   ucaaaacugc acaguucucc gcagauggca gcccagcaca acauggugga cgauggcucu    1260
   ggcggggugg agaucuggcg uguagagaac agugguagag uccagauuga cccaagcucc    1320
   uauggcgagu ucuauggcgg ugacugcuac auuauccucu acacuuaucc cagaggacag    1380
   aucaucuaca cauggcaagg agcaaaugcu accagagaug aacugaccau guccgcguuu    1440
   cugacugucc aguuggaccg gucccuugga gggcaggcug ugcagguccg ugucucucaa    1500
   ggcaaagagc cugcucaccu gcugaguuug uucaaagaca aaccacucau uauuuauaag    1560
   aaugggacau ccaagaaaga agggcaggca ccggcucccc cuacacgccu uuuucaaguc    1620
   cggaggaacc uggcaucuau caccagaauu guggagguug acguugaugc aaauucauua    1680
   aauucuaaug acacuuuugu ccuaaaacug ccacgaaaca auggcuucau cuggauagga    1740
   aaaggugcua gccaggagga ggagaaagga gcagaguaug uggcugaugu ccucaagugc    1800
   aaagcuucaa gaauucaaga aggcaaggaa ccagaggaau ucuggaacuc ucuuggaggg    1860
   aggggagacu accagacuuc accauugcua gaaacucggg cugaagacca uccaccucgg    1920
   cuuuaugguu gcuccaacaa aacuggaaga uucauuauug aagaaguucc gggagaguuc 1980
   acccaggaug accuggcaga agaugauguc augcuacuug augcguggga acagaucuuu 2040
   auuuggauug gcaaagaugc caaugaaguu gagaaaaagg aaucagugaa gucugccaaa 2100
   auguaccugg agacagaccc uucuggaaga gacaagagga caccgauugu caucaucaag 2160
   caagggcacg agccccccac auucacaggc ugguuucugg gcugggacuc cagcaggugg 2220
   uaaaaccagc aacuauccug gcugcauugg ggcagcugcc acuuuguugu uggggaauug 2280
   uuuacuuuuu guuauuggcu uuugaagaua aacuccugcc aaauggauau auaucuauau 2340
   cuauaucuau aucuauaucu auaucuauau cuauaucuau aucuauaucu aucuauaucu 2400
   aaucuauauc uaucuaucua ucuaucuauc uaucuaucua ucuaucuauc uaucuauaug 2460
   cuccucuuuu ccuucucuuu caaagggaau ugcuguaugu uacuauacug aaauaaccua 2520
   aagcaaccau uuguuuucga gcaauuuugc aaucugggga ccucugagga aguaauuuug 2580
   ucauucagcc acugcuagcc aaacuugucu uuucccauag agaggaagga gagccacagu 2640
   gcuucuaagc auuuccccgu cugcuacucu guuugcagug agcuuuacuu uauguauggc 2700
   uuuaacaaug ccuugcuguu ucccaucuca agucaaugcc acuuggaugc cauucacucc 2760
   caaguguccu uacauaggau gaacuucuuu agcuuuuuua gaaaacuaaa aucaugucuu 2820
   uuaugauaaa acacauuuua uuucuauaag uuuaacuuua uauauuugau agcacaugcu 2880
   caauagcaua aagaauaugc auugaaugau guuuuucaua auuaaaauau auccuuuugg 2940
<210> 176
   <211> 1714
   <212> RNA
   <213> Homo sapiens
<400> 176
   caggguugag agcaagaagg ugcaggaugu aggccagcag aauuaucaga cccaguggcg 60
   gcuucugcca cuggaagaau uuccaacaua aaacagauga ucaguuuggg acaaucgauu 120
   cugcgaccag agguaguguc aucugguuac uuuuaaaauu cagauugucu gguguuuucc 180
   aaucacucgc gacuguaauu ugaaguugga uucugagaua auacaaucgc ugucgcucua 240
   guuuauaaag cuguccaaga ucugcccagu cccagauguc cuggguccuc agggcggcug 300
   uggucugcgc ccuucuccug cagcuggaug ccagaccauc cuggacucgg aucccuuugg 360
   guucacacuc gcuuagguau uucuacaccg cugugucccg gccuggccuu ggggagcccu 420
   gguucauaau cgucggcuau guggacgaca ugcagguccu gcgcuucagc agcaaggagg 480
   agacuccgag gauggcaccc uggcuggagc aggaggaagc agaugacugg gaacagcaga 540
   cucauauagu cacaauucaa ggacagcugu cugaaaggaa ucugaugacc cugguucauu 600
   uuuacaacaa gagcauggac gacucucaca cacuacagug gcugcaggac ugcgaugugg 660
   agccagaucg gcaccugugu cucugguaca accagcucgc cuaugauagc gaggaucucc 720
   ccacccugag cgaaaaccca aguuccugua cagugggaaa cagcacugua ccucagaucu 780
   cucagcaccu ggagggccac ugcucagaug ugcugcagaa auaccuggaa aaagggaagg 840
   agaggcugcu gcguucagac cccccaaagg cacaugugac ccgucacccc agaccugaag 900
   gugaugucac ccugaggugc ugggcccugg guuucuaccc ugcugacauc acccugaccu 960
   ggcagaagga uggggaggag cugacccaag auguggaguu uguggagacc aggccugcag 1020
   gggauggaac cuuccagaag ugggcagcug ugguggugcc ucuuggaaag gugcagaguu 1080
   acacgugcca uguggaccau gaggggcugc cugagccccu cacccugaga ugggagccug 1140
   caugguacca aaagccuugg auuuggauug uugccacggu uuuuuccauu uugcucauuu 1200
   gucucugugu ggcucgcaga cccaugaaga agaaugcagg ugggagagga aggcgugaca 1260
   cccaagaagc aggcagagac aguccccaag acucuagcaa gacuguugug gaugaugagg 1320
   agaugggggu uugcuuuugg aagauuaagu ccuguaaaac uugucuaggc cacuccccag 1380
   gaacuucagg aucaucuggg agaugccccu uugaguggcu gggcugugag gacagcaggc 1440
   caguucuugc cacccuggac agaaacacau cucaccuuuc uggcucgagg aucugaacac 1500
   cugucucuug ccuacucggc uucuagucag gcauuuuguc accuugucaa gggucccagg 1560
   gacacaaagc ucccuccucu cacccacagc acucuggguc cuacccccaa ugcuucaggg 1620
   acauuuaauc aggucaaauu gggaucaaug gcuuugaugc agaaaagaac uguggacuaa 1680
   uagagauagg guuuaauaaa aaaaauaucu uuuu 1714
<210> 177
   <211> 467
   <212> RNA
   <213> Homo sapiens
<400> 177
   uuuuuuuuuu uuuuuuucaa cuuuaaagac uggauuugag guucagucug ggucucuggg     60
   ggggaccucu gucaucacgc cuauaaucau cccgagagua gucaucccug gagcuccacg    120
   accgaucauc ccgucuguca uagcggucuu cauagcgguc cccaccuccu cuguagucau    180
   caucucuccg guacccacuu ccaaaugccc uucugccacu gccuauccug gagucauagc    240
   cucggucaua gucucugcug ccucggucau cauagcgauc ccggccccca uagcggucca     300
   ugucucugcg ugggccgucc cgauauccgu cccuauaccc aucccgauac cggucugaau    360
   cguaacgauc ucgauacuug ucuccaaagc uaucaucgcc ucuucuaggu ggguagucau    420
   cacaacuguc ugugguggga cgggcccucc agucuguguc uguuuug                    467
<210> 178
   <211> 346
   <212> RNA
   <213> Homo sapiens
<400> 178
   uuuuuuuuuu uuuuuuuggc ccccaggcuc ugucucaagg augggaauag aucaagccaa     60
   acagugaaaa auaaggcaaa ucguggcuuc gggguuugag acuggcacca auggcaaauc    120
   agcagaggag augcaaaugg gguaacaauc acaguuagug ggguaacaug agcaggcagg    180
   aaacccuuga gacaacaccc aagguccacg ucuucgcaug ugcagggcac aacuccagca    240
   gcaguuucug ggcuuggagg cuuguuacuc uuccuaccuu ucccaccccc uaaaagacac    300
   caagauggag cccacgaaga gauuacauca agcucuucug gcuggg                    346
<210> 179
   <211> 4902
   <212> RNA
   <213> Homo sapiens
<400> 179
   ucuaggacag ccagggcuac acagagaaac ccugucucaa cuaaaacaaa gcaaaccccc      60
   cgaauauugu uuuuuauuug cggaugucug uuuauugaga cgggguucau ggcagacaug     120
   agugucucag ugcauuugcu auccucucuu cuaaaggggc gggggggggg gugcgcugga     180
   gagguggcuc agcaguuaag agcacugacu ucucuuccag agguccugag uucaauuccc     240
   agcaaccaca ugguggcuua uaaccaucua uagugagauc ugaugccuuc uucuugccug     300
   uaggcagacc acuguauaca uaauaaauaa aucuuaaaag gggggggaag guguggagcu     360
   aaaaguaugg caauaugcau aaguuuagcu auuucuguug uuuugcaguc aucagugagc     420
   caaacuaauu cgaaugggua gcuagaucuu guugcuuuua gcgaauauau auugagugaa     480
   uggaauugac agucuucugu auucucauuc aguuuguuug uguucuuccc caucagugau     540
   aacauguagu gaacuaaccc guguggacca ucuuaaccau ggcuucuccu uccuuuucug     600
   uuuuaaacau aggacaugga uuugauugac auccuuugga ggcaagacau agaucuugga     660
   guaagucgag aaguguuuga cuuuagucag cgacagaagg acuaugagcu ggaaaaacag     720
   aaaaaacucg aaaaggaaag acaagagcaa cuccagaagg aacaggagaa ggccuuuuuc     780
   gcucaguuuc aacuggauga agaaacagga gaauuccucc caauucagcc ggcccagcau     840
   auccagacag acaccagugg auccgccagc uacucccagg uacacucguc guggugggag     900
   cuaaggaaaa cucuagugag aaagcagacu cucuggaguu gaguucuugg ucugccauuu     960
   acugugucuu uugugaggag gagaaguucu caaacuucgc uucuugauaa ucaggacaca    1020
   gaucacaggg aggacuuugu gaguucagaa acauccucug uggugaugaa acagcggcag    1080
   aaauacuguu gggaguaaaa gaaguaggca uugcucauug ugguaggcag ggcccugauu    1140
   guauggguaa cugacuuaac uguguuaagu augauuccca uuuuauauuc ccaugucuaa    1200
   acaacuaaag cauuugccca gaacacucag aaagaaauga agggagguua uugccuagca 1260
   cagagcccug guucaguccc ccacgcuguu cguuaaaggg gagggacuaa uaauuugaac 1320
   acaaucuggu uugaaucuau gcaaaucgau uucugaaaug agaccauagg uuauuuuaug 1380
   aacaagucuu auugcucguu gugacccugu gcuuagaaca uuucauaaau gaugcucucu 1440
   gugccuuucc ccuuccucca gguugcccac auucccaaac aagaugccuu guacuuugaa 1500
   gacuguaugc agcuuuuggc agagacauuc ccauuuguag augaccauga gguauaaaaa 1560
   uguuuguuua acagcaaaac ucccuuaucu gauauuaguu ccuuucaugu gucuccaauu 1620
   aagagaagaa aagaaaauuu uagaaggaaa aaauugauca aagaaauugu caaguaaacu 1680
   guaugagagc uauauaaugc uuaaaaauaa gaccuguaug ggcuggugag auggcucagu 1740
   ugguaagagu acccgacugc ucuuccgaag guccagaguu caaaucccag caaccacaag 1800
   guggcucaca acauccauaa caagaucuga cucccucuuc uggagugucu gaagacagcu 1860
   acaguguacu uacauauaau aaauaaauaa aucuuuaaaa aaaaaaaaua aggccuguaa 1920
   acuacaaguc cauuuuacug uauagcugga aacaggaauc agaauaauuu ucccuggaaa 1980
   cuggauauag auauauaaaa uauuuugacu aguaaagaac aacuauuaau cagcauuugg 2040
   auuaaaaaau cuuaaucugu uguuugaagc auucugcuag auauuauggg uacagauuaa 2100
   guccuaauga auguuuuuau ccauuuugaa gucugccuuu aaauacaugg agugaaauaa 2160
   ccuaggagug uauuaauaug gagucacugg gaggaggaaa uguuucauuu uauaaaagca 2220
   gccugagagc uguaggcccu gcugcugucu guucuucaug ccuuggcucu cacucacaug 2280
   aaucaauguc acgucaaucu uggcuuucuu cacuugcauu ucagucgcuu gcccuggaua 2340
   uccccagcca cgcugaaagu ucagucuuca cugccccuca ucaggcccag ucccucaaua 2400
   gcucucugga ggcagccaug acugauuuaa gcagcauaga gcaggacaug gagcaaguuu 2460
   ggcaggagcu auuuuccauu cccgaauuac agguaagaga gcucuaggag ugugcuguuu 2520
   ucugcgggcc cuuuuaaauu agucauccua guuauuuauu auuuacaugc uaccuccuca 2580
   aaggaagaaa uugauggugu auuuaaauua cucaugagag cuucccagac ucacuuaaca 2640
   cacauaguuu uuagguaauc agacugaaua uuucuggaua aauucauuca aagacugaaa 2700
   gcuaauuuag aguucugaca aagauaaaau acuuaucuau ugaaaaaugg gaguugaagg 2760
   aauuauugaa aagaacaccu uggauuuggg gguagggaau ugaucuaaaa ugcacuuagc 2820
   cucugcucau acaaugugac cuucuuuccu agugucuuaa uaccgaaaac aagcagcugg 2880
   cugauacuac cgcuguuccc agcccagaag ccacacugac agaaauggac agcaauuacc 2940
   auuuuuacuc aucgaucucc ucgcuggaaa aagaaguggg caacuguggu ccacauuucc 3000
   uucaugguuu ugaggauucu uucagcagca uccucuccac ugaugaugcc agccagcuga 3060
   ccuccuuaga cucaaauccc accuuaaaca cagauuuugg cgaugaauuu uauucugcuu 3120
   ucauagcaga gcccagugac gguggcagca ugccuuccuc cgcugccauc agucagucac 3180
   ucucugaacu ccuggacggg acuauugaag gcugugaccu gucacugugu aaagcuuuca 3240
   acccgaagca cgcugaaggc acaauggaau ucaaugacuc ugacucuggc auuucacuga 3300
   acacaagucc cagccgagcg uccccagagc acuccgugga gucuuccauu uacggagacc 3360
   caccgccugg guucagugac ucggaaaugg aggagcuaga uagugccccu ggaaguguca 3420
   aacagaacgg cccuaaagca cagccagcac auucuccugg agacacagua cagccucugu 3480
   caccagcuca agggcacagu gcuccuaugc gugaauccca augugaaaau acaacaaaaa 3540
   aagaaguucc cgugaguccu ggucaucaaa aagccccauu cacaaaagac aaacauucaa 3600
   gccgcuuaga ggcucaucuc acacgagaug agcuuagggc aaaagcucuc cauauuccau 3660
   ucccugucga aaaaaucauu aaccucccug uugaugacuu caaugaaaug auguccaagg 3720
   agcaauucaa ugaagcucag cucgcauuga uccgagauau acgcaggaga gguaagaaua 3780
   aagucgccgc ccagaacugu aggaaaagga agcuggagaa cauugucgag cuggagcaag 3840
   acuugggcca cuuaaaagac gagagagaaa aacuacucag agaaaaggga gaaaacgaca 3900
   gaaaccucca ucuacugaaa aggcggcuca gcaccuugua ucuugaaguc uucagcaugu 3960
   uacgugauga ggauggaaag ccuuacucuc ccagugaaua cucucugcag caaaccagag 4020
   auggcaaugu guuccuuguu cccaaaagca agaagccaga uacaaagaaa aacuagguuc 4080
   gggaggaugg agccuuuucu gagcuagugu uuguuuugua cugcuaaaac uuccuacugu 4140
   gaugugaaau gcagaaacac uuuauaagua acuaugcaga auuauagcca aagcuaguau 4200
   agcaauaaua ugaaacuuua caaagcauua aagucucaau guugaaucag uuucauuuua 4260
   acucucaagu uaauuucuua ggcaccauuu gggagaguuu cuguuuaagu guaaauacua 4320
   cagaacuuau uuauacuauu cucacuuguu acagucauag acuuauauga caucuggcua 4380
   aaagcaaacu auugaaaacu aaccagacca cuauacuuuu uuauauacug uaugaacagg 4440
   aaaugacauu uuuauauuaa auuguuuagc ucauaaaaau uaaaaggagc uagcacuaau 4500
   aaaagaauau caugacuuaa acuacuuugg acuuuuugaa uuuauucaca cuauuuucca 4560
   uaggacaauc acucauuuac cacauuuggu uauuuuacau uuucaaaaug gguuugaaaa 4620
   uacagaggca uuuuauagcc auguguggca guccaugauu uuuuauuccc gacauucagg 4680
   aggcagaagc aggcagaucc cugggcucca ggacggccaa ggcuacauga gagccugucu 4740
   caagaaagac aaacccuuuc uauacuaaac guuagcuagg auugucaagg agaugguaua 4800
   uauccacaau gguaugccug cuguacagua cuguggcaca gagaacaaaa ccuguaaccu 4860
   ccuguguucu uagaaguggc auucuaagaa gggcuaggaa ga 4902
<210> 180
   <211> 462
   <212> RNA
   <213> Homo sapiens
<400> 180
   ggccagcagg acucuccuug cagcagcggc ccgaguucag aguccggagc ugcgguggug     60
   gcggcgaagg cgagagucau ggcuggacaa gcuuuuagga aguuucuucc gcucuuugac    120
   agaguauugg uugaaaggag ugccgccgaa acuguaacca aagguggcau uaugcuucca    180
   gaaaagucuc aaggaaaagu guugcaagca acggucgugg cugugggguc aggagggaaa    240
   ggaaagagug gagagauuga accugucagu gugaaaguug gagauaaagu ucuucuccca    300
   gaauauggag gcaccaaagu aguucuagau gacaaggauu auuucuuauu uagagauagu    360
   gacauucuug gaaaguaugu cgacugaaau cacuguugaa auggugucac gugaagcugc    420
   cauuccacug augucugaac uauuucauca uguaaauaau uu                       462
<210> 181
   <211> 417
   <212> RNA
   <213> Homo sapiens
<400> 181
   uuuuuuuuuu uuuuuuucag gucucauuuu cguuuauuug aaauucggug cucguguaag     60
   uuuuuucucu uccccucaaa uuuuauuuca guaaaaggag acuuggcgga gguggauacc    120
   ccacagccgg auucuucccc cccugccccc caggguggcu aaugcuaucu ggggaagucg    180
   ucauagggaa gagaacuaug ggugggcucc ugccugaggc cuccaaucuc agcccagugg    240
   acauaucaca ggcagcuuaa aaaaaaaacc cuaaaaaaaa caccccaaaa cacacauuua    300
   aauagguauu caagacagcu uuaaaaaaug cacccacuca caccccccuc ccuuuucuuu    360
   uuggaaaaaa aaauaggaaa aaaaaaaaaa ccaaaccgaa uucucgcuug gccucua        417
<210> 182
   <211> 1160
   <212> RNA
   <213> Homo sapiens
<400> 182
   auuuggcucc gaggccaaga auucggaucc aaggcgggcg cggggaaaau ggcggcggca      60
   gcugcggcgg gggcgaaugg gagcggaggc agcagcggca uggaagugga ugcagcaguc     120
   cccagcguga uggccuccgg agugacuggg aguguuuccg ucgcucuuca uccccuuguc     180
   auccuuaaca ucucagacca uuggauccgc augcgcuccc aggaggggcg gccuaugcag     240
   gugauugggg cucugaucgg gaagcaggag gggcgaaaua ucgaagugau gaacuccuuu     300
   gagcugcugu cccacaccgu ggaagagaag auuaucauug acaaagaaua uuauuacacc     360
   aaggaggagc aguuuaaaca gguuuucaag gagcuggagu uucuggguug guauaccaca     420
   ggggggccac cugaccccuc agacauccac guccauaagc aggugaguga gauaauugag     480
   aguccgcucu uucugaaguu gaacccuaug accaagcaca cagaucuucc ugucagcguu     540
   uuugagucug ucaucgauau aaucaaugga gaggccacaa ugcuguuugc ugagcucacu     600
   uacacucugg ccacugagga agcugaacgg aucgguguag accacguggc ccggaugaca     660
   gcaacaggca guggggagaa cuccacugug gcugaacacc ugauagcuca gcauagugcc     720
   aucaagaugc ugcacagccg ugugaagcuc auuuuagaau augucaaggc cucugaagca     780
   ggagagguuc ccuucaacca ugagauccug cgggaggccu augcccuaug ucacugucuc     840
   ccaguucuca gcacugacaa guucaagaca gacuuuuaug aucaaugcaa ugacgugggg     900
   cucauggccu accucggcac caucaccaaa acgugcaaca caaugaacca guuugugaac     960
   aaguucaacg uccucuacga ccgacaaggc auuggccggc gaaugcgggg acuguuuuuc    1020
   ugaugauggu ucuggaaggg auggugugug gggcucagac agcuguucca uggaccugag    1080
   uaccacauuc ccuuuagaga aacucauuaa uaaaagagca gccccuuaaa aaaaaaaaaa    1140
   aaaaaaaaaa aaaaaaaaaa                                                1160
<210> 183
   <211> 8322
   <212> RNA
   <213> Homo sapiens
<220>
   <221> misc_feature
   <222> (1298)..(1397)
   <223> n is a, c, g, or u
<220>
   <221> misc_feature
   <222> (2040)..(2139)
   <223> n is a, c, g, or u
<220>
   <221> misc_feature
   <222> (2495)..(2594)
   <223> n is a, c, g, or u
<220>
   <221> misc_feature
   <222> (3800)..(3899)
   <223> n is a, c, g, or u
<220>
   <221> misc_feature
   <222> (4897)..(4897)
   <223> n is a, c, g, or u
<220>
   <221> misc_feature
   <222> (4909)..(4909)
   <223> n is a, c, g, or u
<400> 183
   aaaacccaau guuugguuuu aaagccaaaa auauaaggga agugcauagg uuuggguuuu 60
   guuuuuuguu uuuucccgag guccuugauc uuugccccca aauuugaggg cauaaaguaa 120
   ucccucaguu accuaaaaac acagccauuc cuuugccauu ccacucuccu ggauuggccg 180
   cuucugugcg cgggggagag gugacucacu auuacuacug aggaaagggg agccaguggu 240
   ggaagugggg ugagucacug augggcagca gcuucagccc uccccccaac uuccuuggcu 300
   cucuggggau gccugauccu cucccucacu uugcaccuac uuccccuggu cccucaccac 360
   uacccuuugc ccacccacuc aaauucuuug gcucugcucu uuacuccaga aggccaaagg 420
   caagcuuaga guugaguagg caggaaccaa cauugugaag ccccaggcca gaaaggggug 480
   uucugcagag ugaggguugg caugcuggcu ccuccucacc auaccugccc ccgccccuuu 540
   ggguacagga uggcuccuuu aagagcagug gauccacccc cuguagagga gggccuauua 600
   gagccucugc cuggcuguca gugacucagu guucgcggga acgcugccuc agccucaaca 660
   ccagccaacc cagaucccga ggugcgccag cgcccagccc agaucuccac gccugccagg 720
   agcgagcuuc gccggcucgc ugucccccug agcagccucu guccuucugu ccaagucccg 780
   cgcccuucuc gggaccccug cccagcgggc agcacuguca cccugccggc cauggagacc 840
   ccgucacagc ggcgcgccac ccgcaguggg gcgcaggcca gcucuacccc acugucgccc 900
   acucggauca cccggcugca ggagaaggag gaccugcagg agcucaauga ccgccuggcc 960
   guguacaucg aucgcgugcg uucccuggag accgagaacg cggggcugcg ccuucgcauc 1020
   acugagucug aagagguggu cagccgagag guguccggca ucaaggcggc cuacgaggcc 1080
   gagcuggggg augcccgcaa gacccuugau ucuguggcca aggagcgcgc ccgccuccag 1140
   cuagagcuga gcaaagugcg ugaggaguuc aaggagcuga aggcucggug agugaggccc 1200
   ggccggccgg caccagggag gcagcagucg ccuguaacug gccaucuagu ccccucccuc 1260
   cccggaacug ccucccgcgg gugacuggca gugccaannn nnnnnnnnnn nnnnnnnnnn 1320
   nnnnnnnnnn nnnnnnnnnn nnnnnnnnnn nnnnnnnnnn nnnnnnnnnn nnnnnnnnnn 1380
   nnnnnnnnnn nnnnnnngcu aggccagaua uagaaagcuu ucuguauuua auacacagua 1440
   caugcaucau ucaugucuac auaauuaaga uaaaggaagc ugcauuguua augggaaaaa 1500
   aaauaggguc ugggaaugua gugugccuag cauuuacgaa cuucgggguu uggguuugau 1560
   cccagcaucu cacaaaccac guguaauccc agcacuugga agguagaggc cggaggaucc 1620
   gaaguuuaag gucauucuug acuacuuagc aaauucgggg cacccuagga uaccugagac 1680
   ccugucauga auaaaaauaa auaauaaaua aaccaauaug ggucuagguu gagcagcagc 1740
   uugggcaggg uagggccgga aguuagccag guagaggguu gcagucccag gaggacccug 1800
   gcugggagca gcaccucagu ccccugccca accacagggg gccaccgggu cuuuccggaa 1860
   cuccugaggg cgcaaggccu ugcucucucu ggcccagcca uggggaacgc ggagggccgg 1920
   ugaggcaggc ggcaggcggg cgggcggggc gcgggccguc auccccuccu gcuccuuauu 1980
   uuuagcccag uguagagucu gggccgccug ucccuccccc aggacagggg gaggaaauun 2040
   nnnnnnnnnn nnnnnnnnnn nnnnnnnnnn nnnnnnnnnn nnnnnnnnnn nnnnnnnnnn 2100
   nnnnnnnnnn nnnnnnnnnn nnnnnnnnnn nnnnnnnnng augccaacaa uccuaaugua 2160
   augaugcccu cuucugacag cugcggggac cgcagagagg ucccauccca gaugcacucc 2220
   ugaaaccugc uuuucuuuuu cuagcaacac caagaaggag ggggacuugu uggcugcgca 2280
   ggcccggcuc aaggaccucg aggcucuucu caacuccaag gaagcugccc ugagcacugc 2340
   ucucagugag aagcgcacau uggagggcga gcuccaugac cugcgggggc agguagccaa 2400
   gguaggccgc uguccuguga ccccagugac cccaccuggu ccgacauauc auucgguccc 2460
   auuugccugc ucaccuucac uucuccaguc uagannnnnn nnnnnnnnnn nnnnnnnnnn 2520
   nnnnnnnnnn nnnnnnnnnn nnnnnnnnnn nnnnnnnnnn nnnnnnnnnn nnnnnnnnnn 2580
   nnnnnnnnnn nnnnucuaga uggggacagc caucaccucu ccuuuggcau uuccugaacc 2640
   cccucccacc ucuccaugaa cuuggcuuuc ucccucucag cuugaggcgg cccugggaga 2700
   ggcuaagaag cagcuucagg augagaugcu gaggcgagug gaugcugaga acaggcuaca 2760
   gacgcugaag gaggagcuug acuuccagaa gaacauuuac agcgagguaa gcgucccgcu 2820
   guacaggucu uucuuacugu ggacagcugg gaggggucac cuaauaaugc caggcuaagc 2880
   gagggcugcc cgugccuggc cgguggaguu acgacuucug gucucagcuu cuaaggaacc 2940
   auugcgaugu ucuaaucuaa gugucucccc uuaaccuuuc aggaacugcg ugagaccaag 3000
   cgccggcaug agacgcggcu uguggagauc gauaacggga agcagcgaga guuugagagc 3060
   cggcuggcag augcccugca ggagcugcgg gcucagcaug aggaccaggu ggaacaguau 3120
   aagaaggagc uagaaaagac auacuccgcc aaggugcugg ccucauccug uccucucccu 3180
   ggugcugccc uggggacggg uggguggugg caggggccca gggaugccuu ccucaggccc 3240
   ccagcuccag guuccugcuc ucauaacugu gugcucccug cagcuggaua augccaggca 3300
   gucugcugag aggaacagca accucguggg ggcugcccau gaggaacugc agcagucucg 3360
   aauccgcauu gacagccucu cggcccagcu cagccagcuc caaaagcagg ugacccucag 3420
   uuuaccccuc ccaccuuggc ucuggucuaa gcagauacug cagaagccca cugagaaggg 3480
   ggugggggag ggacuccagg accacaugcu augguucuga aucugaugcc ugucuggcuu 3540
   uccagggcuc uccuuuagcu agcccugauc cucagagccu cuauuuuacu gugcaugaag 3600
   gguuuuucau guuucuucug ugccugccgg agacugaacc agaggccucu ugcuugguag 3660
   auaggugcuu ucucacugag uuacaaaccc agccgcauuc ccuacuugga gauagagcuu 3720
   cccuuggacu ggacauguag cucaguuggu acagugcuug cuuauuauac acgauggccu 3780
   ggcuucuauc cccaggaccn nnnnnnnnnn nnnnnnnnnn nnnnnnnnnn nnnnnnnnnn 3840
   nnnnnnnnnn nnnnnnnnnn nnnnnnnnnn nnnnnnnnnn nnnnnnnnnn nnnnnnnnnu 3900
   cuagagagag ccuacagauc cugagugucc cucugccugc cccugugccc cucccucuuc 3960
   aucgacauca gcccuuggga gcucaucaga cccuuugucu uccccgcccc aguuggcagc 4020
   caaggaggca aagcugcgug accuggagga cucgcuggcc cgugagcgcg auaccagccg 4080
   gcgccugcug gcugagaaag agcgagagau ggcggagaug cgggcgagga ugcagcagca 4140
   gcuggacgag uaccaggagc ugcuggacau caagcuggcc cuggacaugg agauccaugc 4200
   cuaucgaaag cugcuggagg gcgaggagga gaggugaacu aggagggguc uacagaacuu 4260
   gucaggggcc ucuggccgca acuaccauga cuaaccccgc accgugucuc caccuucccc 4320
   aggcugcgcc ugucccccag cccuaccucg cagcgcagcc guggccgcgc cuccucccac 4380
   ucaucccagu cucagggugg aggcagcguc accaaaaagc gcaagcugga gucuuccgag 4440
   agccggagca gcuucucgca gcaugcucgc acuagcgggc guguggcggu agaggaaguc 4500
   gaugaagagg gaaaguucgu gcggcugcgc aacaagucca acgagguggg ccugugaacc 4560
   aggguggucu uuucaguggc ugggaguucu ugguucucgu ggagcaucug ucucccuauc 4620
   ugagaauuug gagugugugg uaguccuguc aucuuuaaau uguugggaag augcaugaua 4680
   uagggauggg ugacccuuga gccaauaguc uagguuugau gccagaggua gugguggaag 4740
   ccugucuuuc uuuucuuuuu cuuuuucuuu uucuuuuucu uuuucucuuu cuucucucuu 4800
   uuuuuuuuuu ccgauggggu cuugcccagg uuggcuuuga ucucccuagu uaagcuguuc 4860
   cccugccuca gccuccauag uagcuggaac ugcaggngca cacuggcgnc cagccuaccu 4920
   aagcuccuga gugagauaau ccaagagucg guugaacucc cuguccuccu uccuccuucc 4980
   uccuuccucc acccuacuuc aggaccaguc caugggcaac uggcagauca ggcgucagaa 5040
   uggugacgau ccuuugauga ccuaucgcuu cccaccgaag uucacccuaa aggcugggca 5100
   gguggugacg gugaguggaa gggcacuugg gacucuggcu ggaguggaga aguuggccuc 5160
   aggacaggag cauuaaaaau aagcacaucu cuuaaaccau cuuuucccag aucugggcuu 5220
   caggagcugg ggccacccau agccccccua cugacuuggu guggaaggcg cagaacaccu 5280
   ggggcugugg gagcagccuu cgcaccgcuc ucaucaacuc cacuggagaa gugaguaugu 5340
   ugcagccggu agcuugcugg acaaggcucc cccgggugac cauaauggga acuagcuacc 5400
   uccaacccaa gggaaccugc cuuggguuua ggaucgcuuu ccugagccca agucccaccc 5460
   aguaagcaag ccagaagucu ccccaguaga auaaugggug gaagucagcc agugaguguu 5520
   aauagcagac uccagcuuac agagcaccga gcucucaguu guguccuuuu ugcgcgugcg 5580
   ugcgcgcgug ugcacaugug cauguguuua uccuuagucc ccagcaucag agguuggaca 5640
   agguuguaua aaggcccggg acaguucuaa gugguuacua uggguagaca ggcugcacag 5700
   cccucacccc cugacucuug ggccugggcu uaugucccca caggaagugg ccaugcgcaa 5760
   gcuggugcgc ucacugacca ugguugagga caaugaggau gacgacgagg auggagaaga 5820
   gcuccuccau caccaccgug ugaguggcag ccgccgcuga ggcccagccc acaaggguag 5880
   cccugccagc cuagggcagc ucucccaccu ccaugccaaa gucuuuucau uaaagaaugu 5940
   uuuggaaugc cacuugcugc ccuggccuuu cuucucucuc cucccucuac cuugaacagg 6000
   gaacccaggu gucugguaag gaagggagug gggacuugcu gaugccaugg auacuccacg 6060
   guggcagugg acaggguucu ggauuugugu ccugggaagg ggcugggagg acagaggugg 6120
   ccccagcccu gccucucuuc cucacuccca uugcaugcac acuucucucc ucucuccuuc 6180
   cacccuauug caugcuucuc cucagauuuc ccugcaacaa uguucucuuu ccuuccuguc 6240
   cccucacaaa uuaagucucu ccaucuuugc ucuuccucuu gauugcccca uaagugucua 6300
   agauucagga gagaguuaaa gccacagcuc uuuauuucga aggcuuccug gcuauuuccc 6360
   ccaucaugcc cuuccuccca gccacagguc ucccaagucc ccaucacuug guugucuggg 6420
   uacagacaga ggucaccuuc cugcccaaug gccaggaagc uccaagagcc cacagccuag 6480
   gugccggucc uaagaaguca gucccaaacu cgcugucccu ccugagccuu gucucccuuc 6540
   ccaggguucc cacugcagcg gcucggggga ccccgcugag uacaaccugc gcucacgcac 6600
   cgugcugugc gggacgugug ggcagccugc ugacaaggcu gccgguggag cgggagccca 6660
   ggugggcgga uccaucuccu cuggcucuuc ugccuccagu gucacaguca cucgaagcuu 6720
   ccgcagugug gggggcagug gggguggcag cuucggggac aaccuaguca cccgcuccua 6780
   ccuccugggc aacuccaguc cccggagcca ggugagucau cucugcccua cagcaggaca 6840
   cugcucacug agcagcaggg cagggcagcc caagggagug gggucccccu ccuugcaguc 6900
   ccucuugcau ccugccccuc cugucugaac cccagacucg aggucagggc aaggcccaga 6960
   gugugagggu uggggagaca accccuuugg ggucagggag ggagaggaag ggccagccac 7020
   ugcugcucac accucugccu ucucuucucu cuuagagcuc ccagaacugc agcaucaugu 7080
   aaucugggac cugccaggca gggcuggggg cagaggccac cugcuccccc cucaccacau 7140
   gccaccuccu gucugcuccu uaggagagca ggccugaagc caaagaaaaa uuuauccccu 7200
   gccuuugguu uuuuuuuuuc uucuauuuuu uuuucuuuuu cuaagagaag uuauuuucua 7260
   cagugguuuu auacugaagg aaaaacucaa gcaaaaaaaa aaaucuuuau cucaauccua 7320
   aguccuuccc cuuucuuucc uuguaucugc cuuaaaacca aagggcuucu cuaggagccc 7380
   agggaaagga cugcuuuuua uagagucuag auuuuugucc ugcugccuug gcuuuacccu 7440
   caucccagga cccugugaca auggugccug agaggcaggc auggaguucu cuucaccagc 7500
   cuccuccaac agcuggccca cugccacgcc agcugcagag aaauggggcg cagagaggau 7560
   gacugagaag gucaagcccc uccccggcac uacacgaggc cgaggcuccu cugccugccu 7620
   uaccuucuuc cugcccuucc cuagccuggg gcgaguggau ucccagaggc aaaucugccg 7680
   ugcuugcuuu uucuauauuu uauuuagaca agagauggga augacgggga aggagaaggg 7740
   aagaucaguu ugagccuacc uuuucccagc uucugagccu ggugggcucu gucucaauga 7800
   uggagggcaa ugucaagugg gauacaggga agaguggggg acgaaggcuc ccagagaugg 7860
   ggagaaccug cuggggcugg ugagaagucu agaggugcgg cgauuggugg cuacagcaaa 7920
   cacuaaggaa cccuucaccc cauuucccau cugcaccucu gcucuccccu ccaaaucaau 7980
   acacuaguug uuuccauccc agaugcugug gugucucuuu guugggugug augugugguu 8040
   ucaggggcag acacaugcac acagaggugc cacacauuca cuauauauuc acuacccagc 8100
   uauaaaggug uguaugaggg agacuucuag aaaggucagc auaugugggg ugagcgaggg 8160
   guguccuucc uaucccucau ccauccagca ccuuuuaaaa ggggccagca auccacaugu 8220
   gcaucagaca caggagcaca gagagacgga ggguagagua gggcagagua gcagagcuuc 8280
   cuugcugccc uguagucgca ggcucuugau cgugugaucg cu 8322
<210> 184
   <211> 1054
   <212> RNA
   <213> Homo sapiens
<400> 184
   gcggugcgaa gcgggccucc gccaacaugu acuacaaguu uagcaguuuc acgcagaagu 60
   uggcuggagc uugggcuucg gaagccuaca ccccgcaggg guuaaagcca guuuccacag 120
   aagcaccacc uaucauauuu gccacaccaa ccaaacugac cuccagugug acagcauaug 180
   auuauucugg gaagaacaaa guuccagagc ugcagaaguu uuuccagaag gcugaugguu 240
   uccaccugaa acgaggccuu ccagaccaaa ugcuuuaccg gaccaccaug gcucugacac 300
   ugggagggac caucuacugc cugaucgccc ucuacauggc cucgcagccc agaaacaaau 360
   gagcuugccu guggaggacu gguucacuuu guggcacaaa cccuuugaau ccucacguuu 420
   cauguuuucc acuuggauag ucuacuuaac auuuugcaaa caaaggaaaa gauaagaaua 480
   cauuguuuug auuuguuuau ggugugcaga uggccuguca gaugucagag cugguuugac 540
   aguuaaaacu auuguuuaag gaaaugucac ugagccauca cugagcugug cuucugcucc 600
   ugauuucccu ggaguucugc aggaaaguug cucuccagcu cauucguggc cagccgugcu 660
   cagggccucg ggacucaggc ggugucugag cgggaagcga acgcggaagc cuuuuggagu     720
   guagguguga uugaggaagg aaaacaaaag ccagcggaca gguggugaag ugagggcuga     780
   guuagccacc cuagggauuc guccgccuug cagaaaacau ugagaggaau gauagcaacc 840
   ugccucuauu ugugggcagu ugguuucaaa ggguugugug ucucgcccag aaccuaggga       900
   aauggguguu uguuccaucg ugggaggagc accugucagu gcugcacauu agacagcugu       960
   gucaggacuu cuccuuuaau aaugcugugg cuuuacguua ugauugaccg gacugcggaa 1020
   uaaacacugg aaucaaaaaa aaaaaaaaaa aaaa 1054
<210> 185
   <211> 4208
   <212> RNA
   <213> Homo sapiens
<400> 185
<210> 186
   <211> 249
   <212> RNA
   <213> Homo sapiens
<400> 186
   gcccccucaa gggcauccug ggcuacacug agcaccaggu ggucuccucu gacuucaaca 60
   gcgacaccca cuccuccacc ugacgcuggg gcuggcauug cccucaacga ccacuuuguc 120
   aagcucauuu ccugguauga caacgaauuu gcuacagcaa cagggugugg accucauggc 180
   ccacauggcc uccaagguaa gccccuggac caccagcccc agcaaggcac aagaggaagg 240
   agagacccu 249
<210> 187
   <211> 410
   <212> RNA
   <213> Homo sapiens
<400> 187
   uuuuuuuuuu uuuuuuucau auuaaacuug uaguuuuauu cagguuugau uuuuaacaaa        60
   ugugucaggg agagagccca caggaaaggg uaaagcccgu gggggcaagg ccuucccaga       120
   ugccugagga gggaucgugu ccccuccccc ccuccucuuc ucaccacccc uacagggguu       180
   ugggaagaga cacaggcagg gaaggggcug guccccaguc uguacagugg ugcuuggggg       240
   ugaaggacua uggagaacag gggaccagau cggggaugag uaggauaaag ggcacaagac       300
   cauuuaccag aauccagcuu ucugauucca aauugaauua aaaagaaaaa aaggagaggg       360
   gaaccuaaac cacaagcagu acccaacucc cuuuccccca ucagggcugc                  410
<210> 188
   <211> 382
   <212> RNA
   <213> Homo sapiens
<400> 188
   uuuuuuuuuu uuuuuuuagg gaagaggcug augccagaua aguuuuuauu auauuaaaaa        60
   aaaaaaaaaa accagugcaa cuggaaauca gggugagucg cuggagguga gugagagucg       120
   gaaggcccuc cacaccucag ugguggcagg aucuggaccc guagccuaag ccuggucuga       180
   uccagccgag augcuggaaa gcagagcaca cgguggugcc caucagggca aagagggcaa       240
   gagagcccac ggcuccucca uaccgcuugc uagggucucc uguguaguag ccauaugcgu       300
   aaaggacucg cccaauaauc caggccaggc ccaggccaga agcuaugcgc gggugguaaa       360
   caccucccac cguuaggaaa aa                                                382
<210> 189
   <211> 253
   <212> RNA
   <213> Homo sapiens
<220>
   <221> misc_feature
   <222> (32)..(32)
   <223> n is a, c, g, or u
<220>
   <221> misc_feature
   <222> (41)..(41)
   <223> n is a, c, g, or u
<400> 189
   cggccgcgug aguuuugacu gagcuucugc angaaguuca naugcaacuc cauacaucag 60
   uucauuuucu agcauuacca cuggguuauu aucacgaaug gccgauuuaa uaaguccuuu 120
   ugcauccucg gaauuccagg ggcugaccac uuuuaaaccu gggcagugcc cauaccaugc 180
   agcaaagcau ugcgagugcu gagcagcuac accugcugag gcgccauugg gcccccugaa 240
   uacuaugggc aca 253
<210> 190
   <211> 371
   <212> RNA
   <213> Homo sapiens
<400> 190
   uuuuuuuuuu uuuuuuugaa gggccauugg aguuuauuua cagacaaccu uaggugaggc 60
   cuuuuccucu aggaucuaca ugcuuuugaa guuacuuggu uucaggcuuc uugucuccag 120
   cuucgagcuu gagacucuca ggaggcuggc gauaggcagg gaaagccucc caggggcugu 180
   ucaggucaaa cuugcggaau ucuugugcca gcuccacugg uucagccacu acccgcuuua 240
   ccucaucguc guaacgaagc ucaacauagc cagugagggg aaagucuuuc cggaaaggau 300
   gucccucgaa gccauaaucu gucaggaucc uucuuaaauc agggugguua aaaaaaaaaa 360
   aaaaaaaaaa a 371
<210> 191
   <211> 324
   <212> RNA
   <213> Homo sapiens
<400> 191
   uuuuuuuuuu uuuuuuuggg gggcagcgaa cuuuauugau gguauucaaa aaaauaggga 60
   gggcucccua ggcccccccu guuauuaugg gggucuggga uggaaauuuu gagggaaaug 120
   cucaauuuug ggggcccauu ugggaaaagg ccccccuugc ccaauguccu ugcuggggug 180
   ggugguccaa gguuucuuac uccuuggagg ccauuuuggc caagaggucc accacccugu 240
   ugcuguagcc guauucauug ucaaaccaag aaaugagcuu gacaaauuug ucauugaaaa 300
   aaaugccaac cccggaauca aagg 324
<210> 192
   <211> 2110
   <212> RNA
   <213> Homo sapiens
<400> 192
   gcuccucauc ucacucgggc cuaugccaaa gauguaaaau uuggugcgga ugcucgagcc      60
   uuaaugcuuc aagguguaga ccuuuuagcc gaugcuguag cuguuacaau ggggccaaag     120
   ggaagaacag ugauuauuga acagaguugg ggaaguccca aaguaacaaa agaugggguc     180
   acuguugcaa agucaauuga uuuaaaggau aaauacaaaa auaucggagc uaagcuuguu     240
   caggauguug ccaauaacac aaaugaagag gcuggggaug gcaccaccac ugccacuguu     300
   cuggcacggu cuauugccaa ggagggcuuu gagaagauca gcaaaggggc uaauccagug     360
   gaaauccgga gaggugugau guuggcugug gaugcuguaa uugcugaacu uaagaaacag     420
   ucuaaaccug ugacaacccc ugaagaaauu gcucagguug cuacaauuuc ugcaaacgga     480
   gacaaagaca uugggaacau cauuucugau gcaaugaaga agguuggaag aaaggguguc    540
   aucacaguga aggauggaaa aacccugaau gaugagcuag aaauuauuga aggcaugaag     600
   uuugauagag gauauauuuc cccauauuuu auuaacacau caaaagguca aaaaugugaa     660
   uuccaagaug ccuauguuuu guugagugaa aagaaauuuu ccaguguuca guccauuguc     720
   ccugcucuug aaauugcuaa ugcucaucgg aagccauugg ucauaaucgc cgaagauguu     780
   gacggagaag cucuaagcac gcugguuuug aacaggcuaa aaguuggucu ucagguugua 840
   gcagucaaag cuccaggguu uggggacaac aggaagaacc agcuuaaaga uauggcuauc 900
   gcuacuggug gugcgguguu uggagaagag gguuugaauc uaaaucuuga agauguucaa 960
   gcucaugauu uagggaaagu uggagagguc aucgucacca aagaugaugc caugcuuuug 1020
   aaaggaaaag gugacaaagc ucacauugaa aaacguauuc aagaaaucac ugagcagcua 1080
   gacaucacaa cuagugaaua ugaaaaagaa aagcugaacg agcgacuugc uaaacuuuca 1140
   gauggaguag cuguguugaa gguuggagga acaagugaug uugaagugaa ugagaagaaa 1200
   gacagaguua cugaugcucu caaugcuaca agagcagcug uugaagaagg cauuguucua 1260
   ggagggggcu gcgcucugcu ucggugcauc ccagccuugg auucauuaaa gccugcuaau 1320
   gaagaccaga aaauagguau agaaauuauu aaaagagcac uuaaaauucc ugcaaugacg 1380
   auugcuaaga augcaggugu ugaaggaucu uugauaguug agaaaauucu gcagaguucc 1440
   ucagaaguug guuaugacgc caugcuugga gauuuuguga acauggugga aaaagggauc 1500
   auugauccaa caaagguugu gagaacugcc uuacuggaug cugcuggggu ggccuccuug 1560
   cuaacuacag ccgaagcugu agugacagaa auuccuaaag aagagaagga cccuggaaug 1620
   ggugcaaugg guggcauggg aggggguaug ggaggcggca uguucuaacu ccuagaguag 1680
   ugcuuugccc uuaucaauga acugugacag gaagcucaag gcagguuccu caccaauaac 1740
   uucagagaag ucaccuggag aaaaugacug aagagaaggc uggcugacca cuguaaucau 1800
   caguuacugg uuuccuuuga cgauauauaa ugguuuacug cugucauugu ccaugccuac 1860
   agauaauuua uuuuguauuu uugaauaaag aacauuugua cauuccugau gcugguugca 1920
   agagccauau accagugucc ugcuuucaac uuaaaucacu gaggcaucuc uacucuucug 1980
   ugagucauca ggacuguagc gcugugucaa caaaacauag agaguucaga agacagccuu 2040
   ucuguggaag ggugggaaug auuguguaca aaguagagaa guauccaauu augugacaac 2100
   cuuuguguaa 2110
<210> 193
   <211> 1228
   <212> RNA
   <213> Homo sapiens
<400> 193
   acagccgcau cuucuugugc agugccagcc ucgucccgua gacaaaaugg ugaaggucgg      60
   ugugaacgga uuuggccgua uugggcgccu ggucaccagg gcugccauuu gcaguggcaa     120
   aguggagauu guugccauca acgaccccuu cauugaccuc aacuacaugg ucuacauguu     180
   ccaguaugac uccacucacg gcaaauucaa cggcacaguc aaggccgaga augggaagcu     240
   ugucaucaac gggaagccca ucaccaucuu ccaggagcga gaccccacua acaucaaaug     300
   gggugaggcc ggugcugagu augucgugga gucuacuggu gucuucacca ccauggagaa     360
   ggccggggcc cacuugaagg guggagccaa acgggucauc aucuccgccc cuucugccga     420
   ugcccccaug uuugugaugg gugugaacca cgagaaauau gacaacucac ucaagauugu     480
   cagcaaugca uccugcacca ccaacugcuu agccccccug gccaagguca uccaugacaa     540
   cuuuggcauu guggaagggc ucaugaccac aguccaugcc aucacugcca cccagaagac     600
   uguggauggc cccucuggaa agcuguggcg ugauggccgu ggggcugccc agaacaucau     660
   cccugcaucc acuggugcug ccaaggcugu gggcaagguc aucccagagc ugaacgggaa     720
   gcucacuggc auggccuucc guguuccuac ccccaaugug uccgucgugg aucugacgug     780
   ccgccuggag aaaccugcca aguaugauga caucaagaag guggugaagc aggcaucuga     840
   gggcccacug aagggcaucu ugggcuacac ugaggaccag guugucuccu gcgacuucaa     900
   cagcaacucc cacucuucca ccuucgaugc cggggcuggc auugcucuca augacaacuu     960
   ugucaagcuc auuuccuggu augacaauga auacggcuac agcaacaggg ugguggaccu    1020
   cauggccuac auggccucca aggaguaaga aacccuggac cacccacccc agcaaggaca    1080
   cugagcaaga gaggcccuau cccaacucgg cccccaacac ugagcaucuc ccucacaauu    1140
   uccaucccag acccccauaa uaacaggagg ggccuaggga gcccucccua cucucuugaa    1200
   uaccaucaau aaaguucgcu gcacccac                                      1228
<210> 194
   <211> 723
   <212> RNA
   <213> Homo sapiens
<400> 194
   agaagcuucc uaaggaacaa gcaaguugaa uagagaaaau agugaucaau aauaggcauu 60
   uuaguggucu uuuuaauguu uucugcugcg gaacauuuca agauuuauug auuuccuccu 120
   ccccccauuu uuuucccacc acacucacac acgcacgcuc acacuuuuua uuugccauaa 180
   ugaaccgucc agccccugug gagaucucuu augagaacau gcguuuucug auaacucaca 240
   accccaccaa ugcgacucuc aacaaguuca cagaggaacu uaagaaguac ggagugacaa 300
   cuuugguucg aguuugugau gcuacauaug auaaagcucc aguugaaaaa gaaggaaucc 360
   acguucuaga uuggccguuu gaugauggag cuccaccccc uaaucagaua guagaugauu 420
   ggcuaaaccu guuaaaaacc aaauuucgug aagagccagg cuguuguguu gcagugcauu     480
   guguugcagg auugggaagg gcuccugugc uaguugcgcu ugcauugauu gaaugcggaa 540
   ugaaguauga agaugcuguu caauuuauaa gacaaaaaag aagaggagca uucaauucca 600
   aacagcugcu uuacuuggag aaguaccgac cuaagaugcg guuacgcuuc agagauacca 660
   augggcacug cuguguucag uagaaguaga agcaggcugg cuggaucgug gcauuagagg 720
   gaa 723
<210> 195
   <211> 2690
   <212> RNA
   <213> Homo sapiens
<400> 195
<210> 196
   <211> 269
   <212> RNA
   <213> Homo sapiens
<400> 196
   uuuuuuuuuu uguucccuug aaagccagau guuccaaaaa guagccugcu ccauuguucu 60
   ucucagucuc auagcgacug ccagcgucaa uccacacucc caccgugcag guagcaugcg 120
   aggacugcuc cgaggccaca cgcagcccgu uguccaagau gcugaccugg gucuccggca 180
   cgcucuggag ggccugggcg aagguugcgg uaccccgcaa ggcagguaac cucagcaggg 240
   ccggcgagcg gcgggugcgc cucgugccg 269
<210> 197
   <211> 1335
   <212> RNA
   <213> Homo sapiens
<400> 197
   ggugcuuaau guuuugaccu guagaggucc ucacuuuucg ucauggcgcu gaagguggcg 60
   auagcugcug gcggugcugc aaaggcaaug cucaagccaa cucuccucug ccguccuugg 120
   gagguucugg cugcccaugu ggccccccga aggagcauuu ccucacaaca aacaauuccu 180
   ccaucugcua aguauggugg gcggcauaca gugacuauga ucccagggga uggcaucggc 240
   ccagagcuca uguugcaugu uaagucugua uucaggcaug caugugugcc gguggacuuu 300
   gaagaggugc auguaagcuc caacgcugau gaggaggaca uccgcaaugc caucauggcc 360
   auccgccgga accguguggc ccugaagggc aacauugaaa caaaucauaa ccugccacca 420
   ucccacaaau cucgaaacaa cauccuucgc accagccuag accucuaugc caacgucauc 480
   cacuguaaga gccugccagg aguggugacc cggcacaagg acauagacau ccucauugua 540
   cgggaaaaca cagaaggcga guacagcagc cuggagcaug agagcguagc aggaguggug 600
   gagagcuuga agauuaucac caaagccaag ucccugcgca uugcugaaua ugcuuucaag 660
   cuggcccagg agagugggcg uaagaaagug acggcugugc acaaggccaa caucaugaaa 720
   cugggugaug gacucuuccu ccagugcugc agggaaguag cagcccacua cccucagauc 780
   accuuugaca gcaugauugu agacaacaca acaaugcagc ugguaucccg gccucagcag 840
   uuugauguca uggugaugcc uaaucucuau gguaacauug ucaacaacgu cugugcaggg 900
   cuaguuggag gcccaggccu uguggcuggg gccaacuaug gccaugugua ugcaguauuc 960
   gagacagcua caaggaacac aggcaaaagu auugccaaua agaacauugc uaacccgacu 1020
   gccacacugc uagcaagcug caugaugcua gaccaccuca agcuccacuc cuaugccacu 1080
   uccauccgca aagcugucuu agcauccaug gacaaugaaa auaugcauac cccagauauu 1140
   ggaggccagg gcaccacauc ccaagccauc caggacauca uucgucauau ccgcaucauu 1200
   aauggacggg cuguggaggc uuagcuaucc cuacaguuuu gcucagcuug ucuguaggac 1260
   ucucuucuca cuuuagcacu ccagcuagcu ugggggacag gacccagaau aaagccacuu 1320
   cuguuccaga aaaaa 1335
<210> 198
   <211> 382
   <212> RNA
   <213> Homo sapiens
<400> 198
   uuuuuuuuuu uuuuuuuguu cuuaauagaa aacuuuauuu ucacugauaa ugucacugua 60
   acauaauuuc auagcagacc ugugcaaaag aucccacauc accaaugucu ccaagagauu 120
   ucacacacuu cugggcagga cgcacagcuc ugcccccacc cccguguuga cagucaacau 180
   uuuacccccg cuaugaguac agaaaggcga ggcaucauaa cgaagccgcc ugaaggcagc 240
   gugagcugaa gucggacgcu ugccaccucu gaaugaaugg ucaccacagc aacagcacau 300
   gguugccuca gugugcucag ggugggucuu ugaaaaaacg ucccacuaug uaaauaugcu 360
   gcacuuaucc cuucaacauu gu 382
<210> 199
   <211> 1992
   <212> RNA
   <213> Homo sapiens
<400> 199
   ggcagacaaa agaggccggc agugcagcuc gcgggacgca uggccgggcg cggaggacgg      60
   gugcugcugg cgcugugugc cgcgcuggug gccggcgggu ggcugcugac gcgugaagcc     120
   caggagcccg gggcgccagc ggcuggcaug aggcgccgcc ggcggcucca gcaagaggac     180
   ggcaucuccu ucgaguacca ccgcuaucca gagcugcgcg aggcgcuggu gucgguaugg     240
   cugcagugca ccgccaucag cagaaucuac acagugggcg ccagcuucga gggccgggag     300
   cuccugguca ucgagcuguc ugacaacccc gggguccaug agccggguga accugaauuu    360
   aaauacaucg ggaacaugca uggcaaugag gcgguuggac gggaauugcu cauuuucuug     420
   gcccaguacc uguguaacga guaccagaaa ggcaaugaga caauugucaa ccugauccac     480
   agcacccgaa uucauaucau gcccuccuug aaccccgacg gcuuugagaa agccgcaugg     540
   cagcccgggc agcugaagga cugguuugug ggccgcagca acgcccaggg aauagaucug     600
   aaccguaacu ucccagaccu ggacaggauc guauauguua augagaaaga aggcgguccc     660
   aacaaccacc ugcugaagaa ucugaagaaa auuguggacc aaaauucaaa gcuugccccc     720
   gagaccaagg cugucauuca cuggaucaug gacauuccau uugugcuuuc ugccaaucug     780
   cacggaggag accuuguggc uaauuaccca uaugaugaga cacggagcgg uacugcucac      840
   gaauacaguu ccugcccuga ugacgcaauu uuccaaagcu uggcucgcgc guacucuucu     900
   uucaacccag ucaugucuga ccccaaucga ccucccugcu gcaagaauga cgaugacagc     960
   agcuuugaug auggaacgac caaugguggu gcaugguaca gcguccccgg uggaaugcaa    1020
   gacuucaauu accugagcag cagcaacugc uuugagauca cuguggagcu uacgugugag    1080
   aaguucccac cugaagagac ucucaaaagc uacugggaag auaacaaaaa cucccucauc    1140
   aacuaccugg agcagauaca ccgagguguu aaaggguuug uccgugaccu ucaggguaac    1200
   ccgaaugcca acgcaaccau cucuguggau gggauagacc augaugucac cucggcuaag     1260
   gauggggauu acuggcgauu gcuugcuccu ggaaacuaua aacuuacagc cuccgauccu    1320
   ggcuaccugg caaucacaaa gaaaguggca guuccuuuua gcccugcugu ugggguggac    1380
   uuugagcuug agucuuucuc ugaaaggaag gaggaggaga aggaagaauu gauggagugg    1440
   uggaaaauga ugucagaaac uuugaauuuu uaagaaaggc uucuaacuaa uugcuuucau    1500
   cuaucuauag acuguaguaa gaugcaaugu ggcucuuuuc uuuuagguug ugugcaguug    1560
   auauuuaaca uugauuuauu uuugaucauu aaguaauagu uacuaaucac guaaauacac    1620
   ccggacagaa auauaaugcu ggacaucuuc auucuacauc aacauucgcu uaaaucauuc 1680
   gaagccucuu uuaacguaau gggugacaau gucacuugac agaugcauga gagucacgau 1740
   auagcugacu gugacccugc acugcaauca cauaguucca uauaaguugu ccuuagucuc 1800
   uugugcugau ucacuguaua agcaugaucc ugguaaugca cuuuggaugg gaagaaaaug 1860
   uacgugcuuu ucagaggggc ucugaacaga augaaaaccu aguucuugcg uguacuuuga 1920
   agaauggaau uguauuaguc agcuguuaau gccacuucag aaguuugggg uuuugucuug 1980
   auuguagauu gg 1992
<210> 200
   <211> 210
   <212> RNA
   <213> Homo sapiens
<220>
   <221> misc_feature
   <222> (118)..(121)
   <223> n is a, c, g, or u
<400> 200
   gauccuguau augugugguu ugggggagcu augauaaguu uuauggcaaa cgguugguau 60
   uguuaacuuu uuauugucau caaaaguuca uaaaaguccu auuaaucccc auauucunnn 120
   ncugcccuua acucugguau acaccaaaaa gaaaucuuua cuuuccuugu uuuaucauua 180
   uaaaaauaaa guauuuugcu aguauggaaa 210
<210> 201
   <211> 677
   <212> RNA
   <213> Homo sapiens
<400> 201
   aguccgcguc ccggcgucgg cccgucccgc accaugguga cgcucgccga gcugcuggcg 60
   cugcuggccg cgcugcuggc cacggccucg ggcuacuuug ucagcaucga cgcgcacgcc 120
   gaggagugcu ucuucgagcg ggucaccucc ggcaccaaga ugggccucau cuucgaggug 180
   gcggagggcg gcuuccugga caucgacgug gagaucacag gaccagauaa uaaaggaauc 240
   uauaaaggag accgggaguc cagcgggaag uacacauuug cagcccacau ggaugggaca 300
   uacaaguucu gcuuuagcaa uaggaugucc acuaugacuc caaagauagu aauguucacc 360
   auugacauug gggaggcucc caaaggacaa gacauggaga cagaagcuca ucagaacaag 420
   cuagaagaaa ugauuaauga gcuggcagug gcaaugacag ccguaaagca cgaacaggag 480
   uacauggaag uccgggagag aauacacaga gccaucaaug acaacacaaa cagcagagug 540
   guccuuuggu ccuucuucga agcucuuguu cuaguugcca ugacauuggg acagaucuac 600
   uaccugaaga gauuuuuuga aguccggagg guuguuuaaa aggccuuuuc cuguugaucc 660
   caaauucaug auuuacu 677
<210> 202
   <211> 2849
   <212> RNA
   <213> Homo sapiens
<220>
   <221> misc_feature
   <222> (2158)..(2158)
   <223> n is a, c, g, or u
<400> 202
   gcauuggcuc ugggcugcgg ccggcucggc gacgcuccuc gggcagcuca cugcaugguc 60
   gucuggugcc cccgccgccu gcauccccgc cgccgccccg cgacgcccac cgccgccugc 120
   ccugccgccg ccgccugcgc cgccucggga ccggcuguau gauuaggcca caaucuucaa 180
   ugaguagaca uauuccucag uucuguggug uucucgguca cacauuuaug gaguuucuga 240
   agggcagugg agauuacugc caggcacagc acgaccucua ugcagacaag ugaacuguag 300
   aaauucauua cuacuccacc aagaaacccc cauaagagug gauaaccugg acacaggcgu 360
   guugaauuga aaucugcaca gcauuugaga agagcucaga ccuggauggg guaaaccuca 420
   gugccacuuc cuuuguauug ccucuaguau uacugggauu gaagagucac ugcuucuugu 480
   uuaggagguu cauuucauug gcccguuucu cccaauuuca uacucaagca cugagaauuu 540
   caaguggagu auaucgaaua ucgaaguaga cuucagguug uuuuuugguu uuguuuuguu 600
   uuuuuuuugu uuugguuuuu uuuuuuuuuu ucuguuuugg uuuaaaucau uucuguauuc 660
   aauuuuuuaa uucuuucaua acccuauugg guguuuuuuu aaacuaaauu aacauggcuc 720
   gaaugaaccg cccugcuccu guggaaguca cauacaagaa caugcgauuu cuuauuacac 780
   acaauccaac caaugcgacc uuaaacaaau uuauagagga acuuaagaag uauggaguua 840
   ccacaauagu aagaguaugu gaagcaacuu acgacacuac ucuuguggag aaagaaggca 900
   uucauguucu ugacuggccu uuugaugaug gugcaccacc auccaaccag auugucgaug 960
   acuggcuaag ucuugugaag auuaaguuuc gugaagaacc ugguugcugu auugcugucc 1020
   auugugucgc aggccuuggc agagcuccgg ugcuuguugc ccuagcauua auugaaggug 1080
   gaaugaaaua ugaagaugca guacaauuca uaagacaaaa gcggcgugga gcuuuuaaca 1140
   gcaagcaacu uuuguaucug gagaaguacc guccgaaaau gcggcuccgc uucaaggauu 1200
   ccaaugguca uagaaacaac uguuguauuc aauaaaacug gggugccuga ugccauugcc 1260
   uuggaagugg aacuucagau gggaccugau uugucaugca uauuacccaa ugugucggcu 1320
   uacugaauaa gucuacugca gcuccacagg aauacugaaa aaccagucuu accaggccac 1380
   aaguuugaca gaauugcaac cucuauauuu gggcuaugau caacauguuu ggacacuuag 1440
   caaaagauuu uugcuguuca gcauuuaaaa ugugcuuauu auuuguacca auugaccuuu 1500
   ccuaaaauaa gguauugagu uaugucauua aauguacucc ugugccagaa uauuauuagu 1560
   cuauaaggaa uuuagaagga uuaggugcca aaauacccag cacaauacuu guauauuuuu 1620
   agcaucauac agaaccaaaa uugcaggaac ugagaacucu cagaccaucc augguguauu 1680
   ccuucaguca uuucaaacac ugcagggcuc cucucguuau cugccugcuc acucuguuua 1740
   caucucccac acuuaugcca gaauacguca gguuugcuua gccauccuuu auuuuuuuua 1800
   uuuuuuuuuu uaacuaaguc uugcgcugau uauuuaauau gucugucuca uuuuguuuug 1860
   uuuugggaaa ccucugucug aaaaaucaac uuuguuacag aagcacauau cuucaacaau 1920
   gucuccagac aaaaagccuu auaguuaauu uaauguuugc acucagaagu gcaaccuaac 1980
   agggagggcc ugaaaaagaa acgagaggag gcuauuaaau auuuuuagua auauguugcc 2040
   uuugucaugu gcagaacaug uagaguaugc ucuuaauuua guaaauauuu uuaagacaua 2100
   gagauacaug uguagcuaac ccauucuuau ucaaaauucu ggaauuuugu guuuuccnau 2160
   accuaucagg aaguuuccag cuuguuugaa uuauggcuuu ccucuuccca aucucuugca 2220
   aaaaagacaa agugggauga aucugcuagu gaacugagca gaaauguuuu auaacgccuu 2280
   uugagcuaug uaacuuaaua auuggauacu ugaucauuug uuuuauuaug uaaucggaua 2340
   aaauggugau guguauuaaa guuaguucaa ccauauauuu auacugucug ggaaugugug 2400
   guuauaguuc ugugggagaa auaguuuguc aguguucacc agcuuguaaa aacuuaguau 2460
   gagagcuuca acauuuaaau aaaugaugaa ccgcauucgu cacugaggac acuuuugccu 2520
   aaaauuaacu uaauuuguag aaaacaaugg auucaguuaa uaucauuuca auuuauggaa 2580
   aaaauuguua ggguugccaa gugcuuuuua uuaaaaguuu cucuuuaaag gucuagauaa 2640
   uugugaauca guugaauguu gggcaccgag gggaaacagu uuguaauaga ugaucuagau 2700
   uuuucaguuc aguuccauca gucacuugua gcucugcaau uuccagacca guuuucucau 2760
   uuuuaaguuc auuacaugcc uguauauauu uugaaauuaa cuugaaccug aguauuuggc 2820
   acaugauggc uuaauaaauu uuaacuuuc 2849
<210> 203
   <211> 3023
   <212> RNA
   <213> Homo sapiens
<400> 203
   acgcucccca accuccaccu ccccucgcuc ggccucuaua ugcucccggg cucccuagug 60
   uuggcuggaa gugggugacu uagaggcuua aaggaggggc gccuaaccac ggaccacgug 120
   ugugcggggg cgacagcgcc gccggggugg ggcugagcgc ugcaagccgg guucgccuug 180
   cagcgcagga gucagugggc guugcgccac gaucucucuc cacuagcacu augcucccgc 240
   cccacucacc gccuuggaaa gucacaggag aaggcgggcu cuaagaccca gcaggcacca 300
   uccuacuggc gccuucgauc cgagacccgu uuggacacca gggggcgaug ccgacccucu 360
   auaaaagcgg uccccgcgcg ggccuggcca uucgcgaccc gaagcugcgc gggcgcgagc 420
   caguuggggc acuggguggg cggcggcgac agcggcgcca cgcgcaggcu ggaggccgcc 480
   gaggcucgcc augccgggag aacucuaacu cccccaugga gucggccgac uucuacgagg 540
   uggagccgcg gcccccgaug agcagucacc uccagagccc cccgcacgcg cccagcaacg 600
   cccgccuuug gcuuuccccg gggcgcgggc cccgcgccgc ccccagcccc accugccgcc 660
   ccggagccgc ugggcggauc ugcgagcacg agacgucuau agacaucagc gccuacaucg 720
   acccggccgc cuucaacgac gaguuccugg ccgaccucuu ccagcacagc cgacagcagg 780
   agaaggccaa ggcggcggcg ggccccgcgg guggcggcgg ugacuuugac uacccgggag 840
   ccccggcggg ccccggcggc gcggucaugu ccgcgggggc gcacgggccc ccucccggcu 900
   acggcugugc ggcggccggc uaccuggacg gcaggcugga gccccuguac gagcgcgucg 960
   gggcgcccgc gcuacggccg cuggugauca aacaagagcc ccgcgaggag gacgaggcga 1020
   agcagcuggc gcuggccggc cucuuccccu accagccacc gccgccaccg ccaccgccgc 1080
   acccgcacgc gucucccgcg caccuggccg ccccccacuu gcaguuccag aucgcgcacu 1140
   gcggccagac caccaugcac cuacagccug gccaccccac accgccgccc acgcccgugc 1200
   ccagcccgca cgcugcgccc gccuugggug cugcgggccu gccuggcccc gggagcgcgc 1260
   ucaagggcuu ggccggugcg caccccgacc uccgcacggg aggcggcggc gguggcagcg 1320
   gugccggugc gggcaaagcc aagaagucgg uggacaagaa cagcaacgag uaccggguac 1380
   ggcgggaacg caacaacauc gcggugcgca agagccgaga uaaagccaaa caacgcaacg 1440
   uggagacgca acagaaggug cuggaguuga ccagugacaa ugaccgccug cgcaagcggg 1500
   uggaacagcu gagccgugaa cuggacacgc ugcggggcau cuuccgccag cugccugaga 1560
   gcuccuuggu caaggccaug gcaacugcgc gugaggcgcg cggcugcggg accgccuugg 1620
   gccggccccc uggcuggaga cccagaggau gguuucgggu cgcuggaucu cuaggcugcc 1680
   cgggccgcgc aagccaggac uaggagauuc cgguguggcc ugaaagccug gccugcuccg 1740
   cguguccccu cccuuccucu gagccggacu cggugcgucu aagaugaggg agucaggccg 1800
   uggugguuuc uccuugagac cgagagacuu uuccgcggag cugagcuggg ggcccggcag 1860
   uacuaguauu aaggaaguaa ccuugugccu uggauacuca aaacucgcuc cuuuuccuac 1920
   cgaguagggg gagcaaaaau gugccuugau auuuuauuug gaggauuccu gcuuccucuc 1980
   gggccucagc uggcccccgu gagaaaaaug aagggugcag gcccagggca ggaggaagau 2040
   acaggaagcu gagaucccgg cagugcccug agcugccccu cagucccugu cuuuagaggg 2100
   gagggacuua gguguugggg auuugagucu guguccucac ccccagcuac agggaggugg 2160
   agggcuccua aucccuugcu uuuugcaccu ccaccuacau cccccccccc ccacucagcu 2220
   uacaacaggc cagguuuccu gggugaguuc auggagaaug ggggcaccac ccccagucag 2280
   acagaaagcu gaguugugag uuagccaugu gguaggagac agagaccuag guuucugggc 2340
   uuuguggggu gggggauagg aggacacggg gaccauuagc cuugugugua cuguaugucg 2400
   ccagccgcug uugcugaagg aacuugaagc acaaucgauc caucccagag ggacuggagu 2460
   uaugacaagc uucccaaaua uuuugcuuua ucauccgaua ucaacacuug uaucuggucu 2520
   cuguguccca gcggugccuu gugcaauggc agugugcacg ucuaugcuaa accaccauuu 2580
   uauuuggucu uuuguuuugu uuugguuuug cucugauucu ugccaaacug agacucuuca 2640
   cuaacggcug ggggaaggag cugagugagg cucucauucu uuuugguuua gggauguuug 2700
   gguuuuuucg ucugccuccc agaggaccaa ugaaaugaag ugggcuuccc ccucuccccu 2760
   aguuguccaa ggguguaugu aguagugggu cuuagcuucc uccggcuaag acuuaggcuu 2820
   ccccacccac ccaaccccau ccccaacggc ccuggcucug ggucuggaaa gaaggccacc 2880
   uccagccagu ucauacacac accccugugg cugggagcag ggcuggaccg cuuccuucuc 2940
   uucuuuuuuu gggggggggg acacaaaguu ucaugcuaga ugucguaugu auuauaucua 3000
   uaauauaaac auaucaaacu caa 3023
<210> 204
   <211> 380
   <212> RNA
   <213> Homo sapiens
<220>
   <221> misc_feature
   <222> (76).. (76)
   <223> n is a, c, g, or u
<400> 204
   gaagcucagu gucgugaaau ggaaaccaaa ccuuggagcc aucgcugagu auaaaaaaaa 60
   ggaagauuua uauucngcaa agaguagccg aacuggacaa aauuacuucu gaaagagaua 120
   auuuuagaca agcauaugaa gaucuucgaa aacaaaggcu gaaugaauuu auggcugguu 180
   uuuacguaau aacaaauaaa cuaaaagaaa acuaccagau gcucacauug ggaggagaug 240
   cugaacugga gcuuguggac aguuuagauc cuuuuucuga aggaaucaug uucaguguuc 300
   ggccaccuaa gaaaaguugg aagaagaucu uuaaccucuc aggaggcgag aaaacccuua 360
   guuccuggcc uuaguguuug 380
<210> 205
   <211> 1391
   <212> RNA
   <213> Homo sapiens
<400> 205
   gccgguuuga guugugcgcu cggguguccc uuuccucuuc cccucccgca gggcuugcgg      60
   ccaccauggc guauuagagg cagcagugcc ugcggcagcg uuggccuuug cagcggcggc     120
   agcagcacca ggcucugcag cggcaacccc caccggccua agccauggcg cucuucacga     180
   aauccagcag caguguugcu guaacggaca aagauaccuu cgaguuaagc acauuccugg     240
   aauccagcaa agccccacaa caugaccgag augagcuucc ugaacagcga aguguuggcg     300
   ggggacuuga ugucccccuu cgaccagucg gguuuggggg cugaagaaag ccuaggucuc     360
   uuagaugacu aucuggaggu ggccaagcac uugaaaccuc auggguucuc cagcgacaag      420
   gcgggcuccu cggaauggcc ggcuauggau gauggcuugg ccagugccuc agacaccggc     480
   aaggaggaug ccuuuuccgg gacagauugg auguuggaga aaauggaucu gaaagaguuu     540
   gacuucgaug cucuguuucg aauggaugac cuggaaacca ugccagauga gcucuugacc     600
   acguuggaug acacauguga ucuuuuugcc ccucuagucc aagagacuaa uaaggagccc     660
   ccucagacag ugaacccaau uggccaucuc ccagaaaguu uaauaaaagu cgaccagguu     720
   gcccccuuua cauucuugca gccuuucccc uguuccccag ggguucuguc uuccacucca     780
   gagcauuccu uuaguuuaga gcuaggcagu gaaguugaua ucucugaagg agacaggaag     840
   ccugacucug cugcuuacau uacucuaauc ccuccaugug uaaaggagga agacacuccc     900
   ucugacaaug acaguggcau cuguaugagc ccggaguccu accugggcuc uccccagcau     960
   agccccucca ccuccagggc cccaccagac aaucugccuu cuccaggugg uucccguggg   1020
   ucuccucggc ccaaaccuua ugacccaccu ggaguuaguu ugacagcuaa agugaagacu    1080
   gagaaauugg auaagaagcu gaaaaagaug gagcaaaaca agacagcagc cacuagguac    1140
   cgccagaaga agcgggcuga gcaggaggcc cucacuggcg aguguaagga gcuagaaaaa    1200
   aagaaugagg cucugaaaga gaaggcagau ucucuggcca aggagaucca guaucugaaa    1260
   gaccugauag aagagguccg uaaggcaagg gggaagaaga gaguuccgua auaggguagu    1320
   caggugcuuu gugcuuguac auagucuugu guugcugugu uugcuguaau aaauuauuuu    1380
   guagugaaag u                                                         1391
<210> 206
   <211> 356
   <212> RNA
   <213> Homo sapiens
<400> 206
   uuuuuuuuuu uuuuuaugua gggaaguuca uauuuuauuu guccagugac auuuuuuaca 60
   guugaauaca aguuaaaggc cugcuugcac accaaagcca gguccuuugg gugguucagu 120
   caaagaagua aggccuccag cuggcucaca acagaagcgg ccacuccuug gcccugguuu 180
   gggaacuuuu ccagcuuuga guucaucaau aaucucuuca auauccuugg gugucagauc 240
   cucauaguag uugucauuua uuugaaccau cggugcauuu acacaggccc cuaaacauuc 300
   cacuucuaua agagugaaaa guuugucagg uguagucucu ccaaccuuua uuccaa 356
<210> 207
   <211> 413
   <212> RNA
   <213> Homo sapiens
<400> 207
   uuuuuuuuuu uuuuuuuggg gugaauauag ccaaguauuc cauuuauuaa caaaauaguc 60
   uuagcaaggg agagcucugu ucaccccaca agaggccccg cagccgaggc cggcccgaag 120
   ccccagcgcu gcugcguaag accgggaggg agggaaggug uuggggagaa gacuuguauu 180
   aagucuuuaa uccuagccac cgcaggaacc cacggaaacc uaaugccagc uuuggcgauu 240
   gcuggcucag gucugggaca uggcgaaggg agugcucuga uccuagggcu cccugagucc 300
   ccagccugcc cccaacagag cuccuaaagu ugucuggucu gcugacuuga ggacugguaa 360
   gcuuuggagg gauccaucaa ggauuccccg accccacccc uaucgccagg gga 413
<210> 208
   <211> 1707
   <212> RNA
   <213> Homo sapiens
<400> 208
   ccucugcggc gcgguccucg gagcggcgcg guucucggag ccacgcgucu gucuuccucc 60
   guggucaugg cggacuaccu gauuagcgga ggcaccucuu acgugccgga cgacgggcuc     120
   acagcgcagc agcucuucaa cugcggggac ggccucaccu acaaugauuu ucucauucuu 180
   ccuggguaua ucgacuucac ugcagaucag guggacuuga cgucugcucu aacuaagaag 240
   auuacacuaa agaccccauu gguuuccuca cccauggaca cugucacaga ggcuggaaug 300
   gccaucgcga uggcgcuuac aggagguauu gguuucaucc accacaacug cacaccugaa 360
   uuccaggcca augaaguucg gaaagugaag aaauacgaac agggauucau cacugacccc 420
   gugguccuua gccccaagga ucguguacgc gauguuuuug aggccaaagc caggcauggc 480
   uucuguggua uccccaucac agauacaggc cggaugggga gucgauuggu gggcaucauc     540
   uccucaaggg acauugauuu ccucaaggag gaagagcaug accgguucuu ggaagagauc 600
   augacuaaga gggaagauuu gguggucgcc ccugccggcg ucacucugaa agaggcaaau 660
   gagauucugc agcgaaguaa aaagggaaag uugcccauug ugaaugaaaa ugaugagcug 720
   guagccauca uugcccggac agaccuaaag aagaaucgug auuacccccu ggccuccaaa 780
   gaugccaaga agcaacugcu guguggggca gccauuggca cucaugagga ugacaaguau 840
   aggcuggacu uacuggcccu ugcuggugug gauguagugg uuuuggacuc uucccaggga 900
   aacuccaucu uccaaaucaa uaugaucaaa uacaucaagg agaaguaucc cagucuacag 960
   gucauuggag gcaauguagu cacugcugcg caagccaaga accucauaga ugcaggugua 1020
   gaugcuuugc gagucggcau gggaaguggu uccaucugca ucacccagga aguguuggcc 1080
   ugugggcggc cccaagccac agcaguguac aaggucucug aguaugcccg ucgcuuuggu 1140
   guuccuguua uugcugaugg aggaauccaa aauguggguc auauugccaa agcuuuggcu 1200
   cuuggggcuu ccacagucau gaugggcucc cuccuggcug ccaccacuga ggccccuggc 1260
   gaguacuucu ucucagaugg gauccggcug aagaaauacc gagguauggg uucucuugau 1320
   gccauggaca aacaucucag cagccagaac cgauacuuca gugaagcuga caaaaucaaa 1380
   guggcccaag gaguuucagg ggcagugcag gacaaggggu cuauccacaa guucguuccu 1440
   uaccugauug cuggcaucca gcauuccugu caagacauug gugccaagag uuuaacccaa 1500
   gucagagcca ugacguacuc gggggagcuu aaauuugaga agaggacauc cucugcucag 1560
   guggaaggug gcguccacag ccuccauucg uacgagaaac ggcuuuucug aaaacagauc 1620
   caguauaugc cuugaauuuu ucaauaaaag uuugggaaaa aaaaagugaa aaaaaaaaaa 1680
   aaaaaaaaaa aaaaaaaaaa aaaaaaa 1707
<210> 209
   <211> 261
   <212> RNA
   <213> Homo sapiens
<400> 209
   gugugggugc agagcugggg ucagggaaag cuggagccuu uugcagaccc ugagcugcuc        60
   acggcugaga gcugggguca uuacccucac cuucauuucc uguaccuguc cuucccagag      120
   ccuccuccau ccacugucuc caacauggcg aacguagcug uucugguugu ccuuggagcu       180
   uggccaucau ugcagcugug guggcuuuug ugaugaagag aaggagacac acagguagga       240
   aagggcagag ucugaguuuu u                                                 261
<210> 210
   <211> 173
   <212> RNA
   <213> Homo sapiens
<400> 210
   acaggagggg aaggccaaag caagaaugaa agucccccgc cccguaucac cacgggcacu        60
   cggggucacc cucaggaaca ugcagucucu gcacgugagg aaagaaacac gcagagaugg       120
   acgagcacuu uuacacccua auaaaauuag augcacuaac cacagacccc ccu              173
<210> 211
   <211> **494**
   <212> RNA
   <213> Homo sapiens
<400> 211
   uuuuuuuuuu uuuuuuuaaa aaaaaaaaug gcaucuuuua uucaucaauc ccuguuacac        60
   acugaaauac augcaucuuu cuuauguuac uuagcaacgu ccuuucuauc cuuucaccca       120
   uaaaucaguc ugaugaauuc aucuuuaaau ucaagaaguu uuucuuuuug aaacgcuacu       180
   uacauuuuau ugucucaaua ucaaagccaa ucuagagauu uauucuuccu acaggugaaa       240
   uagauguuaa uacgggaaca gucaguacau guucaagaug acaucaaagu uaugcccuaa       300
   gcagauauuu caaggcaaua ucaugccacu agcucugaga cucuaaguau cacugauagu       360
   acuaaaaggu agaagucagc uucaaaaaca cacucuugca auggacacug cucaugaugu       420
   cuaagauugu ucacucccac agucaugauu ucagaugcac aguuuuuuuu gcuuuccuga       480
   gcauucgguu ugac                                                         494
<210> 212
   <211> 384
   <212> RNA
   <213> Homo sapiens
<400> 212
   uuuuuuuuuu uuuuuuuaag agugucacca aagcuuuauu uacaugcguc aucaucucuu 60
   uuacaaacua gauuaugguu uuaaauggaa uacacaggca auaucuacaa acgccacggg 120
   aaguacgcac cuccauucca ccgggaaggg gcagauuccc aaaucaaacu gguuuugauc 180
   cuugagaaga aaggcggcag agcuaacuca cggcagcgua ugguuagaca agguccucag 240
   uacccagaau gcagcaggau ugcgucugcc ucaaaccaga cgaccaacug cugcaggugu 300
   uuaaacaugg ccacgcgcca cacgaaauuc uaguuugugu gggguagaag caagaaaaaa 360
   aaaaaaccau ggcgccucgu gccg 384
<210> 213
   <211> 982
   <212> RNA
   <213> Homo sapiens
<400> 213
   gguccggaua guaaacugcu cccuuacauc uccuuaaaua uaaaguaagg aaaaagaaaa 60
   aacucaguuc ggguaaccaa guucaacaag uaauccuugg ggccgcugug guaucgccaa 120
   aaucuacaua guaucucuua uuaaaauguu uugacgaaaa uguaacaauu acgguacuua 180
   augguaacaa uguggcugag gaagcaauag uuaacaaaga ggagcuaagc uaugcaacaa 240
   accagauuuc uauuggucac aaauuugaag uugagaccug uuauccaauu accaaguacu 300
   uccgcauaca ucaucauagg cauuugaaga uuucaaccaa ucaggagcau guuccuucua 360
   uaaaggaacc cagaaccuaa ccucugcauu uccuauuucu uuguagaaau ggcucguacg 420
   aagcagaccg cucgcaaguc cacuggcggc aaggccccgc gcaagcagcu ggccaccaag 480
   gccgcccgca agagcgcccc ggccaccggc ggcgugaaga aaccucaccg cuaccguccc 540
   ggcaccgugg cgcugcgcga gauccggcgc uaccagaagu cgaccgagcu gcugauccgc 600
   aagcugccgu uccagcgccu ggugcgcgag aucgcgcagg acuucaagac cgaccugcgc 660
   uuccagagcu cggccgucau ggcucugcag gaggcgagcg aggccuaccu ugugggucug 720
   uuugaggaca ccaaccugug cgccauccac gccaagcgug ucaccaucau gcccaaggac 780
   auccagcugg cccgccguau ccgcggcgag cgggcuuaau aggcacgcuu ucuacacugg 840
   cacguaaacc aaaacggcuc uuuuaagagc caccuccauu auccaccaaa gaugcuugaa 900
   guacaaguug ugagaguuuu cuaggguuuc cuauuauagc cuuucuugac aaugugagca 960
   ccacccgacg aagcagucug ag 982
<210> 214
   <211> 424
   <212> RNA
   <213> Homo sapiens
<400> 214
   uuuuuuuuuu uuuuuuugga cuucugcggc uuuuauuuuu gcauguaaac cacugggggg      60
   agggggaucu ugaugguggg cacccuagag auuacacugg aguuccgagg gcuccagaca     120
   cuagcuggga agucagguga cagaaacaau gauucagacc aaucacauga uugcaaaacu     180
   gggucuccag cagggauucu gguggucagg uguggaugcc uaaggaagca gugacauggg     240
   aggggcacgc acugggcugg ccuugagcug cugggaugac aucagggaua gaggaccuag     300
   cagcuggugg cuccagggau cuccggucca ugcuuuauuu ggccaggggg uuccugaagg     360
   accugccagc aaacuuggcu uugcugccca gcucuuccuc aauucugagg aucugauugu     420
   acuu                                                                  424
<210> 215
   <211> 1407
   <212> RNA
   <213> Homo sapiens
<400> 215
   auucuaagga ucaugucugc gagucaggau ucucgaucca gagacaaugg ccccgacggg      60
   auggagccgg aaggcgucau cgagaguaac uggaacgaga uuguggauag cuuugaugac     120
   augaaucucu cagagucccu ccuccguggu auuuaugccu augguuuuga gaagcccucu     180
   gccauccagc agcgagcuau ucuuccuugu aucaaggguu augaugugau ugcucaagcc     240
   cagucuggga cugggaaaac agcuacauuu gccauaucaa uucugcagca gauugaauua     300
   gaucuaaagg ccacucaggc uuugguucug gcacccacac gugaauuggc ucagcagaua     360
   caaaaggugg uuauggcauu aggagacuac augggugccu cuugucaugc cugcauuggg 420
   ggcaccaaug ugcgugcuga ggugcagaag cugcagaugg aagcucccca uaucaucgug 480
   gguaccccug gccggguguu ugacaugcuu aaccggagau accugucccc caaauacauc 540
   aagauguucg uacuggauga agcagaugaa auguuaagcc gaggguucaa ggaucagauc 600
   uaugacauau uccagaagcu caacagcaac acacagguag uuuuguuguc ugcuacaaug 660
   ccuucugaug uccuugaggu gaccaagaaa uuuaugagag acccuauucg gauucuuguc 720
   aagaaggaag aauugacccu ggaggguauc cgccaauucu acaucaaugu ggaacgagag 780
   gaguggaagc uugacacauu gugugacuug uaugagacgc ugaccaucac ccaggcaguc 840
   aucuuuauca acaccagaag gaagguggac uggcucaccg agaagaugca ugcccgagau 900
   uucacuguuu cugccaugca cggagauaug gaccaaaagg aacgagaugu gaucaugagg 960
   gaguuccggu cuggcucuag cagaguauua auuaccacug accuguuggc cagaggcauu 1020
   gaugugcagc aggucuccuu agucaucaac uaugaccuuc ccaccaacag ggaaaacuac 1080
   auccacagaa ucggucgagg uggucgguuu ggucguaagg guguggcuau uaacauggug 1140
   accgaagaag acaagaggac ucuucgagac auugagacuu ucuacaacac cuccauugaa 1200
   gagaugcccc ucaacguugc ugaccucauu ugaggggcug uccugcgacc uggcccuagc 1260
   ccaggguuca guccuggggu ggggcuaagg aagagcugga ggggggaggg gagggagcca 1320
   agggauggac aucuuguuuu uguuuuggcu uuuuuuuuuu uuuguuucag uuuuuuuucu 1380
   cuaugaauaa augucacuuu uugaggc 1407
<210> 216
   <211> 562
   <212> RNA
   <213> Homo sapiens
<400> 216
   uuuuuuuuuu uuuuuuugcc aggauauauu uauuacugaa aguacaagca acugagguuu        60
   acccugggaa cacccacaau gaaacgugua ucuucccugu uucucagaug cugccuccuu       120
   ccacagagug aguuuucguu uaaaacucau aaugaggaga aggcaggggg cuccaccccu       180
   uuccuguuca aucugaagac ugaauugggc uacacuggga uggagauuuc aggugcugca       240
   ggucaguguc aaccaagggu ucguguguca ggaacucugg cugguaagau gacacaaagg       300
   cuucaugcug aagcucaggg agguccaggg agccuggcag aauggaauca ucauggucau       360
   caucaaacgu cugugucugg aaggcuucaa ggcucauaau ugcaucauuc ucuggcaguu       420
   ccugagagag cugccccuuc cuggguuugc acgcaucaaa uuucacccac ugguaauuga       480
   cacaguuuug agcuucggga caacucuuaa ucagguugug gaugguugga ugagaaaacc       540
   caaagaaguc ggcaccgcau ga                                                562
<210> 217
   <211> 1238
   <212> RNA
   <213> Homo sapiens
<400> 217
   auuguaacag aagaaggaaa ggaaaagaaa guuaacauug acuuugaacc uuucaaacca 60
   auuaauacau cauuguauuu gugcgacaau aaguuucaua cagaggcucu uacagcguua 120
   cuuucagaug acagcaaguu ugguuuuauu guaauagacg gaaguggugc ucuuuuuggc 180
   acgcuccaag gaaacaccag agaaguccug cacaaauuca cuguggaucu cccaaagaaa 240
   cacggcagag gaggucaauc agccuugcgu uuugcccguu uaagaaugga aaagagacau 300
   aacuauguuc ggaaaguagc cgagacugcu gugcagcugu uuauuucugg agaccaagug 360
   aauguggcug guuugguuuu agcuggauca gcugacuuua aaacugaacu aagucaaucu 420
   gacauguuug accagagguu gcaaucuaaa guuuuaaaau uaguugauau auccuauggu 480
   ggugaaaaug gauucaacca agcuauugaa uuaucuacug aaguucucuc caacgugaag 540
   uucauucaag agaagaaauu aauaggacga uauuuugaug aaauuagcca ggacacaggc 600
   aaguacuguu uuggaguuga agauacacua aaggcuuugg aaaugggagc uguacaaauu 660
   cuaauagucu augaaaaucu ggauauaaug agauacguuc uucauugcca aggcacagaa 720
   gaggagaaaa uucucuauuu aacuccagaa caagagaagg auaaaucuca cuucacagac 780
   aaagagacug gacaggaaca cgaacugaua gaaagcaugc cucucuugga augguuugcu 840
   aacaacuaua aaaaauuugg agcuacacua gaaauuguca cagauaaguc acaagaagga 900
   ucacaguuug ugaaaggauu ugguggaauu ggagguaucu ugcgguaccg aguagauuuc 960
   cagggaaugg aguaccaagg aggagaugau gaauuuuuug accuugauga cuacuaggua 1020
   gucgacaugg guccggcaac cgugccucac ccuccagcau ucaacccaag gagcauaccc 1080
   gugguggagu ccaacagauc ccugccuuac aauuggagca uuuccagaac uuaauccgug 1140
   agcauuggau acugaaaaga aaagugaaac aaaaccagac ccaacccuac acuuugguuu 1200
   gucguggugu cagcgcagca gccgacaacu aagucucu 1238
<210> 218
   <211> **464**
   <212> RNA
   <213> Homo sapiens
<400> 218
   cagaggaguc auuguugcug ugguggcuuu ugugaugaag aggaggagaa acacagcgua 60
   agccagcugc cuggagugga cuaagugaca gacaaugucu ucacacaucu ccugugacau 120
   ccagagcccu caguuuucuu uagucaagua ucugauguuc ccugugagcc uaugggucaa 180
   agugaagaac ugugcagccc agccugcccu gcacacagaa cccugucccu gcacugcccu 240
   ggguucccuu ccacagccaa ccuugcugcu ccagccaaac acugggcgac aucugcaucc 300
   ugccagcucc augcugcccu gagcugcagc uccucacuuc cacacugaga guaagaaucu 360
   gaaugggacc uugauucuua acauccugac cgaggguuga uuucuuguua auuucaugga 420
   uugagaauac uuagaguuuu gguuugucuu gauuuuuuuu caag 464
<210> 219
   <211> 841
   <212> RNA
   <213> Homo sapiens
<400> 219
   ugaauaaaaa cgaggagccg aagauucuag ggggcuacag cugcgcuuuu gcagcacuga 60
   acaugguucc ggguacucaa gauauugcgu uuguguuugg agagguguag auuguagauu 120
   ccagccgaga agaccaggaa aagauaagga uaaagaaugu cauauaucuc aggagcuaga 180
   ucacuucccg augaacaagu aagaauugcc ucaacaaaaa uggauggaau uggaccgaaa 240
   aaagccauuc agcuucguua ucgauuaggu aucaguggga acaucaagau gaaugaguua 300
   acuaaguauc agaucgacca aauugaacaa augauagcuc aagaucaugu uguucauugg 360
   gaauugaaga ggggagaacg agcagacauc gaacgauuaa uuucuauuuc ucguuaucgu 420
   ggaauucguc aucaagaugg aucgcccuua cgcggucaac gaacucauac uaaugcaagg 480
   acugcucgca agcaaauucg gaaaugaaag aaggcuaccg aaagaacaag caacggauuu 540
   cgcgcucauc ccuugcuaaa gcgcauacgu uuucuugcuc cuugguacuu gucugaucaa 600
   ucacacuguu cauuaguuca cuugauuuuu cguucgaugu cuugaaccgc uuacuaauca 660
   cguauacgua uaguaggccc ccuucgccac uccacgucug gcccgucuug gucucgcuca 720
   cuucgcccgc cuaucguauc ggcucggcuu cguccgucaa gcgacagcuu cugccucuga 780
   cggcuucacu aucgcucaug acugguagua cucuauguag uagucggccu uuguuaagcu 840
   u 841
<210> 220
   <211> 136
   <212> RNA
   <213> Homo sapiens
<400> 220
   uauaaccaug uuagaagcgg aaguuggccu gaaaaccuga ggcuuaggcu ucauagcugg 60
   gcuguagauu ggauuuaaac ccaguuggag ugcaaaguca uggugugcuc aaggugauga 120
   cagugaacag aguaug 136
<210> 221
   <211> 752
   <212> RNA
   <213> Homo sapiens
<400> 221
   caugggccuc cugagagauc cuuagaucca gguuagugca uaggaaagug uccccccacu 60
   accuacagcu aagggauugg ggugguggga ucauggugga gggccguggu gaauacuagc 120
   gauguccccc gcuacccgug cgucugccuc cagggugccc cuccaaccag gaugaggcuc 180
   uucaucgcuc uuccuguccu gauugugguc guagccauga ccuuggaagg uaagaaagag 240
   ccuuggaagg uaagaaagau gcuuggaagu gugaaguugg ccuugugccu gcggcccagg 300
   cuuagaagac ccucgaggag ggcucugagg ucccuuucug ugucaucauu ccacuaccgc 360
   ccucccaucg ucccccaucc caccugccag gugccuuauu uuugugucaa agugggugcu 420
   gaaggaggca acucugucca gaaaagacgc aguaaccaau gaccuaggau accacccuuu 480
   ggaauuggcu aaucuuccua gaaggggcgg acgguaaaaa caaggaggug agaggugcag 540
   uaaaaucaag uguccaauac ccucccccau gcuaaugagu uugcucgcaa cccucucgcg       600
   gcaggcccag cccccgccca ggcggccccg gauuuguccg gaacauugga gagcauaccg 660
   gauaaacuga aggaguuugg gaacacuuug gaagacaagg cccgggcagc cauugaacau 720
   aucaaacaga aggaaauuuu gaccaagacc cg 752
<210> 222
   <211> 2858
   <212> RNA
   <213> Homo sapiens
<400> 222
   cagcgccugg ggcgcggcgg gcgucggccc aggagacgcg uggcggcgcu cggccucgcg      60
   gcaucggcgg cugccuggcc guuggcggcg agcgcacuug cgccugcgca gcgggguccg     120
   ugccccuccu ccccugggcg gcccccccac ccccccggcg gcgugugaau ggcggccucg     180
   gcggcagcga cugcagcggc cucggccgcg acggccgccu cggcggccuc ugguagccca     240
   gggucgggcg agggcucggc gggcggugag aagcguccgg cugcuuccuc agccgcggcg     300
   gccucugcag ccgcgucguc cccugcgggg ggcgguggcg aggcgcagga gcuucuggag     360
   cacugcggcg ugugucgcga gcgccugcgg cccgagcggg auccucggcu gcugcccugu     420
   cuacauucgg ccugcagugc cugccugggc cccgcuacac ccgccgcagc gaauaauucg     480
   ggggauggcg gcucggcggg cgacggcgcu augguggauu guccagugug caaacagcag     540
   ugcuacucca aagacaucgu ggagaauuau uuuaugcgug auaguggcag uaaggccucu     600
   ucugauuccc aggaugcuaa ccagugcugc acuagcugug aagauaaugc cccagccacu     660
   agcuauugug uggagugcuc ugaaccacuu ugugagaccu guguggaggc ucaccagcgg     720
   gugaaauaca ccaaggacca cacugugcgc uccacaggac cugcuaagac ucgagaugga     780
   gagcgaacag ucuacuguaa ugugcacaag caugagcccc ucgugcuguu cugugagagc     840
   ugugacacac ucaccugccg cgacugccag cucaacgcuc acaaggacca ucaguaccag 900
   uuuuuggaag augcagugag gaaccaacgu aaacucuugg cuucacuggu gaaacgucuu 960
   ggggacaaac augccacacu ucagaaaaac accaaggagg uucgaagcuc gauccgccag 1020
   gugucugaug ugcagaagcg agugcagguu gaugucaaga uggccauucu gcagaucaug 1080
   aaggagcuga auaagcgggg ucgaguucug gucaaugaug cccagaaggu gaccgagggu 1140
   cagcaggaac gucuggagcg ccagcacugg accaugacca aaauucagaa gcaccaggaa 1200
   cacauuuugc guuuugccuc uugggcucug gagagugaua acaauacagc ucucuugcuc 1260
   ucuaagaagc ugaucuauuu ccagcugcau cgggcccuca aaaugauugu ggauccugug 1320
   gagccucaug gugagaugaa guuucagugg gaucucaaug ccuggaccaa gagugcugaa 1380
   gccuuuggca agauuguggc ugagcguccu gguacgaacu ccacaggucc ugggcccaug 1440
   gcuccuccaa gagccccagg cccucuaagc aagcaagguu cuggcaguag ccagcccaug 1500
   gaaguacaag agggauaugg cuuuggguca gaugaucccu auucaagugc agagccgcau 1560
   guaucaggca ugaagcgguc ccgcucuggu gagggagagg uaaguggccu cuuaaggaag 1620
   gugccacgug ugagccuuga acgccuggau cuggaccuca ccucugacag ccagccacca 1680
   gucuucaagg ucuuuccugg aagcacuacu gaggacuaca aucugauugu uauugagcgu 1740
   ggugcugcug cagcagcugc uggucaggcu gggacugugc caccaggagc cccuggugcc 1800
   ccaccccuuc cuggcauggc cauugucaag gaagaagaga cagaagcugc uauuggagcu 1860
   cccccggcug cccccgaggg uccugaaacc aagccugugu ugaugccucu gacugaaggu 1920
   ccuggugccg agggaccucg ucuagcuuca ccuaguggca guaccagcuc aggcuuggag 1980
   gugguggcuc cugagguuac uucagcccca guaagugggc cagguauccu ggaugacagu 2040
   gccacuaucu gccgagucug ccagaaacca ggugaccugg ucauguguaa ccagugcgaa 2100
   uuuugcuucc accuggauug ccaccucccu gcccugcagg auguuccagg ggaggaaugg 2160
   aguugcucac ucugccacgu gcucccugac cuaaaggagg aagauggaag ccucagccug 2220
   gauggagcag auagcacugg ugugguagcu aaacucucac cagccaacca gcggaaaugu 2280
   gagcguguuc uccuggcccu guucugccau gaaccaugcc gucccuugca ucagcuggcu 2340
   accgacucua cauucuccau ggagcagccu ggugguaccc uagaccugac cuugauucgu 2400
   gcucgccucc aagagaagcu gucaccuccu uauagcuccc cccaggaguu ugcucaagau 2460
   gugggccgca uguucaaaca guucaacaag cugacugagg acaaggcaga uguucagucc 2520
   aucaucggcu ugcagcgcuu cuuugagaca cgcaugaaug augccuuugg ugacaccaag 2580
   uuuucugcug ugcugguaga accaccacca uugaaccuuc ccagugcugg ccuaaguucu 2640
   caggagcucu cuggcccugg ugauggcccc ugaagcuggg gcucuugugg ucagcccagu 2700
   ccagcucugg ucucuguauu uucaccccau acccuguccu uugguggccu gacuccuguu 2760
   cuugcuggcc ccaucguccc cucagucccu cuucacaaaa ugguuuuuac uucuguggau 2820
   uuaauaaaaa cuucacugag ucaguuaaaa aaaaaaaa 2858
<210> 223
   <211> 420
   <212> RNA
   <213> Homo sapiens
<400> 223
   uuuuuuuuuu uuuuuuuaaa aauaauuuca uuuuuuaaua uucuguacaa aauuucucaa 60
   ccuaugaaaa uaaaguuugc aaaagucaaa guaguacaug ggccugcccu ggagccaggc 120
   ccagccggau cuacacuaug uacaggucuc ccagggugag ccucagcuag ggaaaagcca 180
   cuaaggugcc uacagagcaa gagggugcca ucgggccagu gcagccucug caauuuccag 240
   aucugccacu agaggucggc augguccuga uugcuuguca ggcucacccu cuggaagacc 300
   acagccaaug acaaaggucc ucaagggaaa gcuuuggggu cuagcuggaa gugacggugg 360
   guccgccgcc caggcgcagg agcaucugcu ggcaguccag caguaaccgg ugaucgccaa 420
<210> 224
   <211> 963
   <212> RNA
   <213> Homo sapiens
<400> 224
   uccgucguaa ccuguugagu aacuauggaa gacuauauca aaauagagaa aauuggagaa 60
   gguacuuacg guguggugua uaaggguaga cacagaguca cuggccagau aguggccaug 120
   aagaagauca gacuugaaag cgaggaagaa ggagugccca guacugcaau ucgggaaauc 180
   ucucuauuaa aagaacuucg acauccaaau auagucagcc ugcaggaugu gcucaugcag 240
   gacuccaggc uguaucucau cuuugaguuc cuguccaugg accucaagaa guaccuggac 300
   uccaucccuc cugggcaguu cauggauucu ucacucguua agaguuacuu acaccaaauc 360
   cuccagggaa uuguguuuug ccacucccgg cgaguucuuc acagagacuu gaaaccucaa 420
   aaucuauuga uugaugacaa aggaacaauc aaacuggcug auuucggccu ugccagagcg 480
   uuuggaauac cgauacgagu guacacacac gagguaguga cgcuguggua ccgaucucca 540
   gaaguguugc ugggcucggc ucguuacucc acuccgguug acaucuggag uauagggacc 600
   auauuugcag aacuggccac caagaagccg cuuuuccacg gcgacucaga gauugaccag 660
   cucuucagga ucuucagagc ucugggcacu ccuaacaacg aaguguggcc agaagucgag 720
   ucccugcagg acuacaagaa caccuuuccc aaguggaagc cggggagccu cgcaucccac 780
   gucaagaacc uggacgagaa cggcuuggau uugcucucaa aaaugcuagu cuaugauccu 840
   gccaaacgaa ucucuggcaa aauggcccug aagcacccgu acuuugauga cuuggacaau 900
   cagauuaaga agauguagcc cucuggaugg augucccugu cugcuggucg uaggggaaga 960
   ucg 963
<210> 225
   <211> 479
   <212> RNA
   <213> Homo sapiens
<400> 225
   uuuuuuuuuu uuuuuuuacu aggcaaagaa uuuuauuaac ccuuuccaaa cuuuauuccc 60
   aggcuucuuc agcuuuauuu gccgcaaaga augaauuagg uauagcgaaa acugaaaaga 120
   gcugcagugu ccgggggcuu gggcuuaaaa auauuagaga ucuagauuuu aucagaucca 180
   uaauaaacaa aaaaauuuua aaaagcaguc augauauaaa auagcagcuc cuguaauuuc 240
   ugcaaguauc accuucuuca gaaguugcuu caauucaguu ugccucauuc uuagaagccu 300
   caucaaaauu cuccaccaga ucuggaacuu caucaucauc auccucucca guagcaaggg 360
   gugcuuuucc auccacagau uguuugggca gagcuucagc cagucuccuu aaacuaguca 420
   ggcugucugc accaagcugg uugaggaugc ugggaagcau uucugucagc ugcuuuguc 479
<210> 226
   <211> 520
   <212> RNA
   <213> Homo sapiens
<400> 226
   uuuuuuuuuu uuuuuuuauu caugcuugcc uagggauggg gaauagauca uucaauaaaa        60
   acauacagua aaaacagggg guggggaggg ggaaggcuua ucauguacaa uguuuaaacu       120
   acaauaguga uguaccuuaa uuacuuccau gcacacaagu cuaacauuac aaguuuuuaa       180
   aaaauaaaca ccauuaagac uucuaggagc auuuuauaau aaauuccuaa uuuuuucuuu       240
   guagauagau caagcaccuc caaaauacag auuccuauac acagugagca cuuuacuuaa       300
   cguacaugga cagccucagg acgagcugac gucucggauc agcucggcag gcaacaaacc       360
   auagugccaa auggaaagaa gggcaguugc aaauaaacuu aaaacuaagu uaacuuuuau       420
   aauuaaauac agaaaauaua cugauuugcu aaaaauaaau aagaugugau guauuuaaca       480
   cuucacuaua aagaaugaac accaugacau ccucgugccg                             520
<210> 227
   <211> 468
   <212> RNA
   <213> Homo sapiens
<400> 227
   agaagaagga ggagugggac cgcaagcaug aggaugcuag uagggaguau gagaaaggca        60
   ugaaagagua ugaaggagga agaggggacu caucuaaaag ggacaagucu aagaagaaaa       120
   agaaaguaaa agcaaagaug gaaaaaaagu ccacuccuuc ccggggcucg ucauccaagu       180
   cuucauccag gcaguugagu gacagcuuca agagcaaaga guuugugucc agugaugaga       240
   gcucuucagg cgagaacaag agcaaaaaga agaggaggcg gacgaggacu cugaagagga       300
   gcuagccagu accccuccaa gcucagagga cucugccucg ggaucugaug aauaaaggag       360
   ggaauuccca ccccgucaca gcuccagucu cucacauagu ccuuggauuc ugugccaucu       420
   gaguaacugc ucuugguggc uuccacugcc cugaggcuuu gagggaag                    468
<210> 228
   <211> 1740
   <212> RNA
   <213> Homo sapiens
<400> 228
   aucgucguaa ucguuugcag acuucucgcc gucgccuugu aagcuuuguc uucgccuugc 60
   aagcuuuguc uucaggguug gaaagaugag acauucaaag agaacuuacu guccugacug 120
   ggaugaaaga gacugggauu auggaacaug gagaagcagc agcagucaca aaagaaagaa 180
   gagaucacau agcagcgccc gugagcaaaa gcgcugcagg uacgaucacu ccaaaacgac 240
   agacagcuau uaucuggaaa gcagauccau aaaugagaaa gcuuaucaua gucgacgcua 300
   uguugaugaa uacaggaaug acuacauggg cuacgagcca gggcaucccu auggagaacc 360
   uggaagcaga uaccagaugc auaguagcaa guccucuggu aggaguggaa gaagcaguua 420
   caaaaguaaa cacaggaguc gccaccacac aucgcagcac cauucacacg ggaagaguca 480
   ccgaaggaaa agaucgagga guguagagga ugaugaggag ggucaccuga ucugucagag 540
   uggagacgua cuaagugcaa gauaugaaau uguugauacu uuaggugaag gugcuuucgg 600
   aaaaguggug gaaugcaucg aucauaaagu gggagguaga cguguagcag uaaaaauagu 660
   uaaaaaugug gauagauacu gugaagcugc ucaaucggaa auacaaguuu uggaacacuu 720
   gaauacaaca gacccccaua guacuuuccg uuguguccag auguuggagu gguuugagca 780
   ucgaggucac auuugcauug uguuugaacu ucuggggcuu aguacuuaug auuucauuaa 840
   ggaaaacagu uuucugccgu uucgaaugga ucauaucagg aagauggcau aucaaauaug 900
   caaaucugua aacuuuuugc auaguaauaa auugacucau acagacuuga agccugaaaa 960
   caucuuauuu gugaagucug acuacacaga ggcuuauaau cccaaaauga aacgugauga 1020
   acguacuaua guaaauccag auauuaaagu gguggacuuu ggaagugcaa cauaugauga 1080
   ugaacaccac agcacauugg uaucuacaag acauuauaga gcaccggaag uuauuuuagc 1140
   ccucgggugg ucacagccau gugaugucug gagcauagga uguauucuua ucgaguauua 1200
   ucuuggauuu acaguuuuuu cgacucauga uagcagggaa cauuuagcaa ugauggaaag 1260
   gauucuugga ccacuaccaa agcacaugau acagaaaacc aggaaacgca gauauuucca 1320
   ucaugaucga uuagauuggg augaacacag uucugcuggc agauauguuu cucggcgcug 1380
   uaaaccucug aaggaguuua ugcuaucuca ggaugccgaa caugagcuuc ucuuugaccu 1440
   cauugggaaa auguuggagu augaucccgc caaaagaauu acucucaaag aagcccuaaa 1500
   gcauccuuuc uuuuacccac uuaaaaagca uacgugauuu auaaacacag ugcucugaaa 1560
   ggaaucuuac agacuguauc agucuagcuu uuaauuaagu uauuuuguau agcuuaauuu 1620
   guaaaacauu uuauguuuuu uagaugcuuu auuaaauaca uggccaaacc aaauaacauc 1680
   uuucaguaau uauagaauga uuuauuugga auaaaauuug ugcuuaugaa uguaaaaaaa 1740
<210> 229
   <211> 815
   <212> RNA
   <213> Homo sapiens
<400> 229
   ggacucgcga caagcguccu cagcgcgaag aggcggacuc ggaguccucg ccuugagccu 60
   ugcaucugag aaguuccagg uacuuuguac aacugcaucc cagaaccugu guguuuucag 120
   caccuuuaua agugauggcu gccaucagga agaaacuggu gauuguuggu gauggagcuu 180
   gugguaagac augcuugcuc auagucuuca gcaaggacca guucccagag gucuaugugc 240
   ccacgguguu ugaaaacuau guggcggaua ucgaggugga ugggaagcag guagaguugg 300
   cuuuauggga cacagcugga caggaagauu augacugccu gaggccucuc ucuuauccag 360
   acaccgaugu uauauugaug uguuuuucca uugacagccc ugauaguuua gaaaacaucc 420
   cagaaaaaug gacuccagaa gucaagcauu ucuguccaaa ugugcccauc auccugguug 480
   ggaacaagaa ggaccuucgg aaugacgagc acacgagacg ggaguuggcc aaaaugaagc 540
   aggagccggu aaaaccugaa gaaggcagag auauggcaaa caggauuggc gcuuuugggu 600
   acauggagug uucagcaaag accaaagaug gagugagaga gguuuuugag auggccacga 660
   gagcugcucu gcaagcuaga cgugggaaga aaaagucugg gugccucauc uugugaagcc 720
   uugugaacgc agccucaugc gguuaauuug aagugcuguu uauuaaucuu aguguaugau 780
   uacuggccuu ucauuuaucu auaauuuacc uaaga 815
<210> 230
   <211> 287
   <212> RNA
   <213> Homo sapiens
<400> 230
   uucaagaccg accugcgcuu ccagagcucg gccgucaugg cucugcagga ggcgagcgag 60
   gccuaccucg ugggucuguu ugaggacacc aaccugugcg ccauccacgc caagcguguc 120
   accaucaugc ccaaggacau ccagcuggcc cgucgcauuc guggggagag ggcguaaauu 180
   aggguaguga gugaauuugg accccaaagg cucuuuucag agccacccac auuuucuaua 240
   aaaggcugua uaucgauaag cuuuuauaaa ccccacucag caacucc 287
<210> 231
   <211> 945
   <212> RNA
   <213> Homo sapiens
<400> 231
   uuaugugaua aaaaaauuca acuugguauu aacuuaacua agggccuugg ugcuggugcu        60
   uugccugaug uugguaaagg ugcagcagaa gaaucaauug augaaauuau ggagcauaua       120
   aaagauagcc auaugcucuu uaucacagca gggaugggug gugguacugg aacaggugcu       180
   gcaccgguaa uugcaaaagc agccagagaa gcaagagcgg uaguuaaaga uaaaggagca       240
   aaagaaaaaa agauacugac uguuggaguu guaacuaagc cguucgguuu ugaaggugug       300
   cgacguaugc gcauugcaga gcuuggacuu gaagaguugc aaaaauacgu agauacacuu       360
   auugucauuc ccaaucaaaa uuuauuuaga auugcuaacg agaaaacuac auuugcugac       420
   gcauuucaac ucgccgauaa uguucugcau auuggcauaa gaggaguaac ugauuugaug       480
   aucaugccag gacugauuaa ucuugauuuu gcugauauag aaacaguaau gagugagaug       540
   gguaaggcaa ugauugguac uggagaagca gaaggagaag auagggcaau uagugcugca       600
   gaggcugcga uaucuaaucc auugcuugac aauguaucaa ugaaaggugc gcaaggaaua       660
   uugauuaaua uuacuggugg uggagacaug acucuauuug aaguugauuc ugcagccaau       720
   agagugcgug aagaagugga ugaaaaugca aauauaauau uuggugccac uuuugaucag       780
   gcgauggagg gaagaguuag aguuucuguu cuugcaacug gcauugauag cuguaacgac       840
   aauucaucug uuaaucaaaa caagauccca gcagaggaaa aaaauuuuaa auggccuuau       900
   aaucaaauuc caacauuaga aacaaaagaa uaugcuucaa cugag                       945
<210> 232
   <211> 238
   <212> RNA
   <213> Homo sapiens
<400> 232
   ugaucagguc acauuccggu gccuuccacc cccuauggca cgggcgcccc gccuugccau        60
   accacagcuc ccuccaggcu uagaccuggc uucaccgcau uucaggugcu auaccccccc       120
   cugcuuuucc ccccauugcc cuuaaaugcc ccucggcccc uccauccccc cggaacaggg       180
   uggcacuugc cacucucagg accaccuugc caaggagaau aaaccgaauc cuguugcu         238
<210> 233
   <211> 341
   <212> RNA
   <213> Homo sapiens
<400> 233
   uuuuuuuuuu uuuuuuucac ucaucacguu uauucagaag agaauacacc caauccucuc 60
   aucucuggaa aguuuuguuc cuagccauca uuaagaucag gaccccuguu cuccccuuaa       120
   ccccaggaga ggaagcuuau agucuuugua gauauucuga accucugauc acaaaggguu       180
   auaaaauagu gaagggguuu ggacuaggaa aggcacagac ugaaaggaau cugccagggg 240
   acugaggcca cagcuccccc aggagucaga ggaaggggga agucacguau uuuauagaag 300
   gccaagggcc ucagagcaag aguauuccuu ugaagagcuc a 341
<210> 234
   <211> 495
   <212> RNA
   <213> Homo sapiens
<400> 234
   ugguggagca ggucucagga aucucuucgc uucagcuuca aucauggccu guggucuggu 60
   cgccagcaac cugaaucuca aaccugggga augucucaaa guucggggag agguggccuc 120
   ggacgccaag agcuuugugc ugaaccuggg aaaagacagc aacaaccugu gccuacacuu 180
   caauccucgc uucaaugccc auggagacgc caacaccauu guguguaaca ccaaggaaga 240
   ugggaccugg ggaaccgaac accgggaacc ugccuucccc uuccagcccg ggagcaucac 300
   agaggugugc aucaccuuug accaggcuga ccugaccauc aagcugccag acggacauga 360
   auucaaguuc cccaaccgcc ucaacaugga ggccaucaac uacauggcgg cggauggaga 420
   cuucaagauu aagugcgugg ccuuugagug aagccagcca gccuguagcc cucaauaaag 480
   gcagcugccu cugcu 495
<210> 235
   <211> 861
   <212> RNA
   <213> Homo sapiens
<400> 235
   gugguucuuc ggcgccaggu ucgcccgcuu cugcccuuag guaacauucu cuaaacugcg 60
   uuucucuccc aaucuuuugc aggcauuugg ggacuuuuuc uuuucuuuuu acuuucucuu 120
   uuucuuuugc acaagaagaa gucuacaaga ucuuuuaaga cuuuuguuau cagccauuuc 180
   accaggagaa cacguugaau ggaccuuuuu aaaaagaaag cggaaggaaa acuaaggaug 240
   aucgucuugc ccaggugucu uguucuccgg ccuggacugu gauaccguua uuuaugagag 300
   acuuucagug cccuuucuac aguuggaagg uuuucuuuau auacuauucc caccaugggg 360
   agcgaaaagg uuaaaaaaaa gaaaaaaauc acaaggaauu gcccaaugua agcagacuuu 420
   gccuuuucac aaagguggag cgugaauucc aggaggaccc aguauucggu uacuuaaaug 480
   aagucuucgg ucagaaaugg ccuuuuugac acgagccuac ugaaugcugu guauauauuu 540
   auauauaaau auauauauau ugagugaacc uuguggacuc uuuaauuaga guuuucuugu 600
   auaguggcag aaauaaccua uuucugcauu aaaauguaau gacguacuua ugcuaaacuu 660
   uuuauaaaag uuuaguugua aacuuaaccc uuuuauacaa aauaaaucaa guguguuuau 720
   ugaauguuga uugcuugcuu uauuucagac aaccagugcu uugauuuuuu uuuaugcuau 780
   guuauaacug aacccaaaua aauaccaguu caaauuuaug uagacuguau uaagauuaua 840
   auaaaaugug ucugacauca a 861
<210> 236
   <211> 830
   <212> RNA
   <213> Homo sapiens
<400> 236
   cgaugaaggc ggugagcccg gugcgccccu cgggccgcaa ggcgccgucg ggcugcggcg 60
   gcggggagcu ggcgcuacgc ugccuggcgg agcacggcca cagccugggu ggcucggcag 120
   ccgccgccgc cgcugcggcg gccgcgcgcu gcaaggcggc cgaggcggcg gccgaugagc 180
   cggcgcugug ccugcagugc gauaugaacg acugcuacag ucgccugcgg aggcucgugc 240
   cuaccauccc gcccaacaag aaagucagca aaguggagau ccugcagcac guuaucgacu 300
   acauccugga ccugcagcug gcgcuggaga cucacccugc uuugcugaga cagccgccac 360
   cgcccgcgcc accucuccac ccggccgggg cuuguccggu cgcgccgccg cggaccccac 420
   ucaccgcgcu caacacugac ccggugagaa gccuuggcgg gcacccuggg caucgcggga 480
   aagguggcgg ggcggcgaga uacggguggu cuugcuccuc ucagggaaug acagccgcuu 540
   cucccgucuc caccgagagc cgccugcugg gcuuggugau ccacuggucc cugagccgag 600
   ggcgguuggg cuuggagccc ugcgucuccg gagugucccu ugcaucacag gaggcuuccc 660
   cagcuucggg cucgggcggg gacucugcuc accugccuag uuuuccagga cgucuccugg 720
   gugguggcga cacugugaua ugcgcacucu aaccgcuuuu ccccuuggug uuggguugcu 780
   guuccaggcc ggcgccguga acaagcaggg ugacagcauu cucugccgcu 830
<210> 237
   <211> 5560
   <212> RNA
   <213> Homo sapiens
<400> 237
<210> 238
   <211> 416
   <212> RNA
   <213> Homo sapiens
<400> 238
   uuuuuuuuuu uuuuuuuacc guucuuuauu uaaaaaaaua aaauaggagu ccguagggug 60
   cacccucccc ccuccugagu gaaggagggc acggugcagu ccggaccugg gagaggggaa 120
   agcccggcag gcaggcgaga ccgcuucaca caguggucuc gccaugccgg cuguugguga     180
   gacgagugua gaacuuccuc cacgagugca gugucuugcc ggaccagauc cagaagcccg 240
   acgugaugcc cacgaugagc gucaugaggu auuugaucau guagacugug aagucgggcg 300
   acaugcgggg cguguagugg gccgggcagg ggauggcuag gcucuugcag ugcuggcuua 360
   cccaggagcg cucccagugc ucgcggaagg ccugcucaua gaaguagcag gcgaug 416
<210> 239
   <211> 2380
   <212> RNA
   <213> Homo sapiens
<400> 239
   ggccgcgucg acgccucucc agcugcucug uggcagccca gcuaccgguc gugaccagau 60
   ccagcuugca gcucagcuuu guucauucga auugggcggc ggccagcgcg gaacaaacau 120
   gcagcggcuc ggggguauuu ugcuguguac acugcuggcg gcggcggucc ccacugcucc 180
   ugcuccuucc ccgacgguca cuuggacucc ggcggagccg ggcccagcuc ucaacuaccc 240
   ucaggaggaa gcuacgcuca augagauguu ucgagaggug gaggagcuga uggaagacac 300
   ucagcacaaa cugcgcagug ccguggagga gauggaggcg gaagaagcag cugcuaaaac 360
   guccucugag gugaaccugg caagcuuacc ucccaacuau cacaaugaga ccagcacgga 420
   gaccagggug ggaaauaaca caguccaugu gcaccaggaa guucacaaga uaaccaacaa 480
   ccagagugga cagguggucu uuucugagac agucauuaca ucuguagggg augaagaagg 540
   caagaggagc caugaaugua ucauugauga agacuguggg cccaccaggu acugccaguu 600
   cuccagcuuc aaguacaccu gccagccaug ccgggaccag cagaugcuau gcacccgaga 660
   cagugagugc uguggagacc agcugugugc cuggggucac ugcacccaaa aggccaccaa 720
   agguggcaau gggaccaucu gugacaacca gagggauugc cagccuggcc uguguugugc 780
   cuuccaaaga ggccugcugu uccccgugug cacaccccug cccguggagg gagagcucug 840
   ccaugacccc accagccagc ugcuggaucu caucaccugg gaacuggagc cugaaggagc 900
   uuuggaccga ugccccugcg ccaguggccu ccuaugccag ccacacagcc acagucuggu 960
   guacaugugc aagccagccu ucgugggcag ccaugaccac agugaggaga gccagcugcc 1020
   cagggaggcc ccggaugagu acgaagaugu uggcuucaua ggggaagugc gccaggagcu 1080
   ggaagaccug gagcggagcc uagcccagga gauggcauuu gaggggccug ccccugugga 1140
   gucacuaggc ggagaggagg agauuuaggc ccagacccag cugagucacu gguagaugug 1200
   caauagaaau ggcuaauuua uuuucccagg agugucccca aguguggaau ggccgcagcu 1260
   ccuucccagu agcuuuuccu cuggcuugac aagguacagu gcaguacauu ucuuccagcc 1320
   gcccugcuuc ucuggcuugg gaaagacagg cauggcgggu aagggcagcg gugagucguc 1380
   ccucgcuguu gcuagaaacg cugucuuguu cuucauggau ggaagauuug uuugaaggga     1440
   gaggauggga aggggugaag ucugcucaug auggauuugg gggauacagg gaggaggaug     1500
   ccugccuugc agacguggac uuggcaaaau guaaccuuug cuuuugucuu gcgccgcucc     1560
   caugggcuga ggcaguggcu acacaagagc uaugcugcuc uguggccucc cacauauuca     1620
   ucccuguguu ucagcuccua ccucacuguc agcacagccc uucauagcca cgcccccucu     1680
   ugcucaccac agccuaggag gggaccagag gggacuucuc ucagagcccc augcucucuc     1740
   ucucaacccc auaccagccu cugugccagc gacagcccuu ccaaauggag ggagugaaau     1800
   ccuuugguuu uauuauuuuc uccuucaagg cacgccugcc acuaagguca ggcugacuug     1860
   caugucccuc uaacguucgu agcagugugg uggacacugu cuuccaccga cugcuucaau     1920
   accucugaaa gccagugcuc ggagugcagu ucguguaaau uaauuugcag gaaguauacu     1980
   uggcuaauug uagggcuagg auugugaaug aaauuugcaa agucgcuuag caacaaugga     2040
   aagccuuucu cagucacacc gagaagucac aaccaagcca gguuguguag aguacagcug     2100
   ugacauacag acagaagaag gcugggcugg augucaggcc ucagaugacg guuucaggug     2160
   ccaggaacua uuaccauucu guaucuaucc agaguuauua aaauugaaag uugcacacau     2220
   uuguauaagc augccuuucu ccugaguuuu aaauuauaug uauacacaaa cauguggccc     2280
   ucaaagauca ugcacaaacc acuacucuuu gcuaauucuu ggacuuuucu cuuugauuuu     2340
   caauaaauac aaauccccuu caugcaaaaa aaaaaaaaaa                           2380
<210> 240
   <211> 2659
   <212> RNA
   <213> Homo sapiens
<220>
   <221> misc_feature
   <222> (1274)..(1274)
   <223> n is a, c, g, or u
<220>
   <221> misc_feature
   <222> (1358)..(1358)
   <223> n is a, c, g, or u
<220>
   <221> misc_feature
   <222> (1507)..(1508)
   <223> n is a, c, g, or u
<400> 240
   gcacgagcag cccgcagccc gccgcgccug ugcgagccgg gacagcacuc ggccccgcgc 60
   gcuccccgcc ccgcgccagc cccgccgcgg cgaccugcug cagcggagga ccccaucgau 120
   cggagacccg gggagcagcg cgcagcccgc gagccgacgg gccccgacug cgucuuuguc 180
   cccggaggcu ccgggaaguu ugcagcggga cgcgcgcgug aaggcagcgu gggcagcccc 240
   gacgucgccg agcaacaugg gcgucgggcg cagcgcgcgg ggucgcggcg gggccgccuc 300
   gggagugcug cuggcguugg ccgccgcucu gcuggccgcg gguucggcca gcgaguacga 360
   cuacgugagc uuccaguccg acaucggcuc guaucagagc gggcgcuucu acaccaagcc 420
   cccgcagugc guggacaucc cgguggaccu gaggcugugc cacaacgugg gcuacaagaa 480
   gauggugcug cccaaccugc uggagcacga gaccauggca gaggugaagc agcaggccag 540
   cagcugggug ccgcugcuca acaagaacug ccacaugggc acccaggucu uccucuguuc 600
   gcucuucgcg cccgucuguc uggaccggcc caucuacccg ugucgcuggc ucugcgaggc 660
   cgugcgcgac ucgugcgagc cggucaugca guucuucggc uucuacuggc ccgagaugcu 720
   caaaugugac aaguuccccg agggcgacgu cugcaucgcc augaccccgc ccaauaccac 780
   ggaagccucu aagccccaag guacaaccgu guguccucca ugcgacaacg aguugaaguc 840
   agaggccauc auugaacauc ucugugcaag cgaguuugca cugaggauga aaaucaaaga 900
   agugaagaag gaaaacggug acaagaagau uguccccaag aagaagaaac ccuugaagcu 960
   ggggcccauc aagaagaagg agcugaaggc gcuugugcug uuccugaaga acggugccga 1020
   cugucccugc caccagcugg acaaccucag ccacaacuuu cucaucaugg gccgcaaggu 1080
   gaagagccag uaccugcuga cagccauuca caagugggac aagaaaaaca aggaguucaa 1140
   aaacuucaug aagagaauga aaaaccacga gugucccacc uuccagucug uuuuuaagug 1200
   auacuggggc ggacugggga aggggagugu ggcuuggggu gagggugggg gcgcguggau 1260
   gacccuggcu cuunggggcu cacauauugc ucucacccau acaguugugg cuuuugcauu 1320
   gcaccuggcu cuguuccuac agcgaacccu cucccuuncc uccauagcca cauccagcua 1380
   aggccacggc cuuuagauua ggaaggcuuu uuuuuuuuua agggcugcag cagggccagc 1440
   agcgacgugc aaaaggagag gcagaauccu uucacugagc cuggggcaca aaaaacagaa 1500
   aauguunncc gguuuggaaa aacaaaacaa aacggauugu aaagaacugc agacggacag 1560
   cugcucagcu caacguuguu cgggacauca uuaccaauug cuuguggagu cuaagccucu 1620
   acagguagaa gagucugauc auugccaagc caggcugcuu ucaguuuauu auuaaucccc 1680
   cucuuucugc cuuagauaga ccaucgccac cuucaaaaca cacacacaca cacacacaca 1740
   cacacacaca cacacacaca cacacacaca cacuucugaa aguagccagg guaucccagu 1800
   auagaacggg auagcuaagg guuugggugg gaggccacug cuacucuacc uucagcuuuu 1860
   gaacuggcca ccuuugauag gaaacugagg ucucagaugg acacuucuac caguccaucg 1920
   ggauacaagg augccaggca agggucugcu uuugucugaa ggagguacgu gggcaugaag 1980
   agacaugagg cauuucaggc ugagaagcaa cagcuacuag uuuucaacaa uagaguggaa 2040
   gaaaugagca aagguagaaa ugucaagcag gucacaaguc agggugauug ggggaauccu 2100
   gugccaacag ccucacuuug uaauuccauc ugucacuuuc aaaagaacag cagcauaaga    2160
   cagggauaaa agcccacaua cccuccaagg cuugaguaaa aguccacacu cagcauuuca    2220
   aagacuaacg ucguugacug cccaaggcug cccucuuaau acaccgccua ugcaugugcu    2280
   guggaaggca acucugugca ugugcugugg aggagauggg ccucauggcu gugcuggcug    2340
   cccggaauca guauagcgug gaaggagaca guauccauag acucugcuuu ucugcaagga    2400
   aagcccuuuu ccuuauacau gauugccuau aauucagaca aauuuaaaau cgcugccugc    2460
   cugagcccuc caccuuuacu uuugcauucu ccggucauau ucuuuugagg cuaaagugcc    2520
   cuauccgagg agaugguuuc aaaggcuaac uaaucugcag cuuucccaag ugcccagagg    2580
   uauuucucaa aguugguaug cuuaauaagu gauguaaaua uuccaguucu cuuaggcagc    2640
   cuuacuccug uugucccug                                                 2659
<210> 241
   <211> 3347
   <212> RNA
   <213> Homo sapiens
<400> 241
   aauuccgagu ggccgcgcgg cuauuuaagu ggcguuacuc cgcguccucg gcgcugcagc 60
   cuugaggcuu cgggcgcggg ggaagucaug cuggcuccac agaagcacua gcuaguccgu 120
   guacuuugug gguucugucc uuuugagacc ugcccgccgg ggauuggcug guaucaguga 180
   cugucuacug cuggauuuuc ugcuugccuu cccgucaugu cuaacgaagu agaaacaagc 240
   acaaccaaug gccagccuga ccaacaggcu gccccgaaag cgccaucaaa gaaggagaag 300
   aagaaagguu cugaaaagac agacgaguac uuguuggcca gguucaaagg ugaugguguu 360
   aaauacaagg ccaagcuaau cgguauugau gaugugccug augcucgagg agacaaaaug 420
   agucaggauu cuaugaugaa acucaaggga auggcagcag cuggucgcuc ucagggacaa 480
   cacaagcaaa gaaucugggu caacauuucc uugucuggca uaaaaaucau ugaugagaaa 540
   acugggguaa uugaacauga acauccagua aauaagauuu ccuucauugc ucgugaugug 600
   acagacaaca gagcauuugg uuaugugugu ggaggugaag gccagcauca auuuuuugcu 660
   auaaaaacag ggcaacaggc ugagccauua gucgucgauc uuaaagaccu uuuucaaguu 720
   aucuauaaug uaaagaaaaa ggaagaagau aagaaaaagg uugaagaagc caacaaagca 780
   gaagagaaug gaagugaggc ccuaaugacc cuugaugauc aagcuaacaa auugaagcug 840
   gguguugacc agauggauuu guuuggggac augucuacac cuccugaccu aaauagucca 900
   acagaaagca aagauauccu guuaguggau cuaaacucug aaaucgacac caaucagaac 960
   ucuuuaagag aaaauccauu cuuaacaaau ggagucaccu ccuguucucu cccucgacca 1020
   aagccucagg cauccuucuu gccugagaac gccuuuucug ccaaucucaa cuucuuuccc 1080
   accccuaacc cugauccuuu ccgugaugau cccuuugcac agccagacca aucggcaccc 1140
   ucuucguucg auucucucac aucuccagau cagaagaaag cgagucugag uagcucgucu 1200
   acuccacaga guaaagggcc ccugaacguc gauacugauu acuuugguca gcaauuugac 1260
   cagcucucua accggacugg caaaccggaa gcucagggag gcccguggcc cuacccaagu 1320
   ucgcagaccc agcaagcagu gagaacucaa aaugggguau cugaaagaga acagaacggc 1380
   uuccauauca aaucuucccc gaacccuuuu gugggaagcc cucccaaagg acuaucggua 1440
   ccgaauggcg uaaagcagga cuuggaaagu ucuguccagu ccucagcaca ugacuccaua 1500
   gccauuaucc caccuccaca aaguaccaaa ccaggaagag gcagaaggac ugcuaagucu 1560
   ucagcaaacg acuugccugc uucagacauc uuugccucag aaccuccagg ccagaugucc 1620
   cccacaggac aaccugcagu cccgcagucg aacuuccugg aucucuucaa aggcaaugcu 1680
   ccucccccag uggggccccu uguaggucua gguacggucc caguaacacc cccccaagca 1740
   ggacccugga cgccuguugu cuacaguccu ucgacaacug uggucccagg agccauaaua 1800
   aguggccagc cucccaguuu ucgccagcca cucguuuuug guacaacccc agcaguacaa 1860
   gucuggaauc agucuccauc auuugcaacc ccagcuuccc cuccaccccc cacaguuugg 1920
   uguccuacca caucuguggc gcccaacgcu uggucaucca caagcccucu ggggaauccu 1980
   uuucagagua auaauaucuu uccaccuccc accaugucca cucaguccuc uccucagccu 2040
   augauguccu cuguucuggc cacaccgccu caaccaccuc cccgaaaugg cccacuaaag 2100
   gacauuccca gugacgcuuu cacuggcuua gacccccuug gggauaaaga ggucaaggaa 2160
   gugaaagaaa uguuuaagga cuuccagcug cggcagccac cucuuguucc cucaaggaag 2220
   ggggagacgc cucccucugg gacuucaagc gccuucucca guuacuucaa caauaaaguu 2280
   ggcauuccuc aggagcaugu agaccaugau gauuuugaug ccaaucaacu guugaacaag 2340
   auuaaugaac caccaaagcc agccccgaga caaggugucc ucuuggguac caagucugcu 2400
   gacaauucac ucgagaaccc uuucuccaaa ggguucagcu caucaaaccc cucugugguu 2460
   ucucagccug caucuucuga uccccacagg agcccuuucg gaaauccuuu ugccuagcuu 2520
   cugaaguugu aaugaugacu auccagauga gcaaaagacu ggcuuugguc aagaaugaag 2580
   cagacagcca gaaacauguu gaccucuguc cucgcuccag cuuugacgua uuaucuguua 2640
   cccuauuugu uuuggccucu uguacuugua aaaugccuuu cauuuuccug ucuaggcuaa 2700
   agcuaaacuu aaacuauggc uuuacguaaa uuaagcuccu aaacucucua gcuccaauau 2760
   aaaugaagua gcuucccuau caaaucccug ucuguugugc ccccuugaaa cuuccagaau 2820
   auucuccauu cuacccucca uuugggagga gcggcuaccu uuacccuuaa uaucacacug 2880
   ccuugaguca auguccaaau acucauagcu cucaaaguca uuugggguuc cuggugugcg 2940
   gccuaaaccu aaagcauccu auuaauaggg aaguaagaca ccuugcuucc uauguccacu 3000
   cagggagaau uuuauuuaau aaaaugaaag caagacuaac uucucaaauc cacccaagga 3060
   ccauuuugag auggucguuu cucagcuaac ugcaccauuu accaauccug ccccaagugg 3120
   ugcuuacauu ugacuugaag aagagaaaga gcuaacucaa aacacaaggc auuauucaaa 3180
   gcuaauaaaa caauuucucc cuggggcccc acauuguuuu cauuccagau acguugcagc 3240
   uguuugaccc ugaugacauu augcccuaca uuuuccuuga agauccugau uuuauuucau 3300
   gugauuuuug uuucucaaua aagaugauua uugugugcac ggaauuc 3347
<210> 242
   <211> 555
   <212> RNA
   <213> Homo sapiens
<400> 242
   augaaucgca ccgcauacac ugugggagcg uugcuucucc uccuggggac ccuacugcca 60
   acagcugagg ggaaaaagaa agguucccaa ggagccauuc cgccuccuga caaggcucag 120
   cacaaugacu cugagcagac ccagucccca ccacaaccug gcuccaggac ccgggggcgg 180
   ggccaggggc ggggcaccgc caugccugga gaggaggugc uugaguccag ccaagaggcc 240
   cugcacguga cagagcgcaa guaucugaag cgagauuggu gcaaaacuca gccccugaag 300
   cagaccaucc acgaggaggg cugcaacagc cgcacuauca ucaaccgcuu cuguuauggc 360
   cagugcaacu ccuucuacau ccccaggcac auccgaaagg aggaaggguc cuuucagucu 420
   ugcuccuucu gcaagcccaa gaaguucacc accaugaugg ucacacucaa cuguccugag 480
   cuacagccac ccaccaagaa gaaaaggguc acacgcguga agcagugccg uugcauaucc 540
   aucgacuugg auuaa 555
<210> 243
   <211> 2526
   <212> RNA
   <213> Homo sapiens
<400> 243
   guggaauauc cauggaggua cggagccuug uuaccaaccu uuaaccgcag aacugggaug 60
   uggggcugga agugccuccu cuucugggcu gugcugguca cagccacucu cugcacugcc 120
   aggccagccc caaccuugcc cgaacaagcu cagcccuggg gagucccugu ggaaguggag 180
   ucucuccugg uccacccugg cgaccugcua cagcuucgcu gucggcuucg cgaugaugug 240
   cagagcauca acuggcugcg ggauggggug cagcuggugg agagcaaccg uacccgcauc 300
   acaggggagg agguggaggu gcgggacucc auccccgcug acucuggccu cuacgcuugc 360
   gugaccagca gccccucugg cagcgauacc accuacuucu ccgucaaugu cucagaugca 420
   cucccauccu cggaagauga ugacgacgac gaugacuccu ccucggagga gaaagagacg 480
   gacaacacca aaccaaaccg uaggccugua gcucccuacu ggacaucccc agagaaaaug 540
   gagaagaaac ugcaugcggu gcccgcugcc aagacgguga aguucaagug cccgucgagu 600
   gggacaccca accccacucu gcgcugguug aaaaauggca aagaguuuaa gccugaccac 660
   cgaauuggag gcuacaaggu ucgcuaugcc accuggagca ucauaaugga uucuguggug 720
   ccuucugaca agggcaacua caccugcauc guggagaaug aguaugggag caucaaccac 780
   accuaccagc uugacgucgu ggaacgaucu ccgcaccgac ccauccuuca ggcagggcug 840
   ccugccaacg agacaguggc ccugggcagc aauguggagu ucauguguaa gguguacagc 900
   gauccgcagc cucacauuca guggcugaag cacaucgagg ugaacgggag uaagaucggg 960
   ccagacaacu ugccguaugu ccagauccug aagacugcug gaguuaauac caccgacaag 1020
   gaaauggagg ugcuucaucu acggaauguc uccuuugagg augcggggga guauacgugc 1080
   uuggcgggua acucuaucgg acucucccau cacucugcau gguugaccgu ucuggaagcc 1140
   cuggaagaga gaccagcugu gaugaccuca ccgcucuacc uggagaucau uaucuacugc 1200
   accggggccu uccugaucuc cugcauguug ggcucuguca ucaucuauaa gaugaagagc 1260
   ggcaccaaga agagcgacuu ccauagccag auggcugugc acaagcuggc caagagcauc 1320
   ccucugcgca gacagguaac agugucagcu gacuccagug cauccaugaa cucugggguu 1380
   cuccugguuc ggcccucacg gcucuccucc agcgggaccc ccaugccggc uggagucucc 1440
   gaauaugagc ucccugagga uccccgcugg gagcugccac gagacagacu ggucuuaggc 1500
   aaaccacuug gcgagggcug cuucgggcag gugguguugg cugaggccau cgggcuggau 1560
   aaggacaaac ccaaccgugu gaccaaagug gccgugaaga uguugaaguc cgacgcaacg 1620
   gagaaggacc ugucggaucu gaucucggag auggagauga ugaaaaugau ugggaagcac 1680
   aagaauauca ucaaccuucu gggagcgugc acacaggaug guccucuuua ugucauugug 1740
   gaguacgccu ccaaaggcaa ccuccgggag uaucuacagg cccggaggcc uccugggcug 1800
   gaguacugcu auaaccccag ccacaacccc gaggaacagc ugucuuccaa agaucuggua 1860
   uccugugccu aucagguggc ucggggcaug gaguaucuug ccucuaagaa guguauacac 1920
   cgagaccugg cugcuaggaa cguccuggug accgaggaua acguaaugaa gaucgcagac 1980
   uuuggcuuag cucgagacau ucaucauauc gacuacuaca agaaaaccac caacggccgg 2040
   cugccuguga aguggauggc cccugaggcg uuguuugacc ggaucuacac acaccagagc 2100
   gauguguggu cuuuuggagu gcucuugugg gagaucuuca cucugggugg cuccccauac 2160
   cccggugugc cuguggagga acuuuucaag cugcugaagg agggucaucg aauggacaag 2220
   cccaguaacu guaccaauga gcuguacaug augaugcggg acugcuggca ugcagugccc 2280
   ucucagagac cuacguucaa gcaguuggug gaagaccugg accgcauugu ggccuugacc 2340
   uccagccagg aguaucugga ccuguccaua ccgcuggacc aguacucacc cagcuuuccc 2400
   gacacacgga gcuccaccug cuccucaggg gaggacucug ucuucucuca ugagccguua 2460
   ccugaggagc ccugucugcc ucgacacccc acccagcuug ccaacagugg acucaaacgg 2520
   cgcuga 2526
<210> 244
   <211> 468
   <212> RNA
   <213> Homo sapiens
<400> 244
   auggcugaag gggagaucac aaccuucgca gcccugaccg agagguucaa ccugccucua 60
   ggaaacuaca aaaagcccaa acugcucuac ugcagcaacg ggggccacuu cuugaggauc 120
   cuuccugaug gcaccgugga ugggacaagg gacaggagcg accagcacau ucagcugcag 180
   cucagugcgg aaagugcggg cgaaguguau auaaagggua cggagaccgg ccaguacuug 240
   gccauggaca ccgaagggcu uuuauacggc ucgcagacac caaaugagga augucuguuc 300
   cuggaaaggc uggaagaaaa ccauuauaac acuuacaccu ccaagaagca ugcggagaag 360
   aacugguuug ugggccucaa gaagaacggg agcuguaagc gcgguccucg gacucacuau 420
   ggccagaaag ccaucuuguu ucugccccuc ccggugucuu cugacuag 468
<210> 245
   <211> 3115
   <212> RNA
   <213> Homo sapiens
<400> 245
   auggacaccu ccugcgucca caugcuccug uccuugcugg cgcugcugca guugguggcc      60
   gccggcagcu caccgggacc agaugcgaua ccgcggggcu gcccaucaca cugucacugu     120
   gagcuggaug gcaggaugcu gcucagggug gacugcucgg accuggggcu cucggagcug     180
   cccuccaacc ucagcgucuu caccuccuac cuggaccuca guaugaacaa caucagucag     240
   cuacccgcca gucuccuaca ucgccucugc uuccuagaag aguuacgucu ugcuggaaau     300
   gcuuugacac acauucccaa gggagcguuc acgggccuuc acagccucaa agugcuuaug     360
   cugcagaaca accagcugag aaagguuccg gaggaagcgc uacagaauuu gagaagccuu     420
   caaucccugc gccuagaugc caaccacauc agcuacgugc cacccagcug uuucagcggc     480
   cugcacuccc ugaggcaccu guggcuagau gacaaugcuc ucacagacgu cccuguccag     540
   gcuuucagaa guuuaucagc ccugcaagcc augaccuugg cccugaacaa aauacaccac     600
   auagcagacu acgccuuugg aaaccucucc agccucgugg uucugcaucu ccauaauaau     660
   agaauccacu cccugggaaa gaaaugcuuu gauggacucc acagccugga gacuuuagau     720
   uuaaauuaua auaaccuuga ugaauucccc acugcaauca agacacucuc caaccuuaag     780
   gaacuaggau uccacagcaa caacaucagg ucaauaccgg agcgagcguu cguaggcaac    840
   ccuucucuua ucacaauaca cuucuaugac aaccccaucc aauuuguugg aguaucugcu    900
   uuucagcauu ugccugaacu aagaacacug acuuugaaug gugccucgca cauuacugaa    960
   uuuccucacu ugacaggaac ugccacccug gagagucuga cuuuaacugg agcaaagauc   1020
   ucaucucuuc cccaggccgu cugugaucag uuaccuaauc uccaagugcu agauuugucu   1080
   uacaaccuac ucgaagacuu acccaguuug ucaggcugcc aaaaacuuca gaaaauugac   1140
   cugaggcaua acgagaucua ugaaauuaag ggcagcacuu uucagcaguu guuuaaccuc   1200
   cgaucucuga acuuagcaug gaauaaaauu gcuaucauuc accccaaugc guuuucuacg   1260
   uugccgucuc uaauaaaguu ggaccuauca uccaaucucc ugucguccuu cccugugacu   1320
   ggguuacaug guuuaacuca cuuaaaauua acagggaacc gagccuuaca gagccugaua   1380
   ccaucugcaa acuucccaga gcucaagauu auagaaaugc caucugcuua ccaguguugu   1440
   gcauuugggg ggugugagaa ugucuauaaa auuucuaacc aauggaauaa agacgacggc   1500
   aacagugugg acgaccuuca uaagaaagac gcuggguuau uucaaguuca agaugagcgg   1560
   gaccuugaag auuuccuacu ugacuuugag gaagaccuga augcccuuca cucggugcag   1620
   ugcucgccuu ccccaggucc cuucaagccc ugugagcacc uauuugguag cuggcugauc   1680
   cgaaucgggg uguggaccac ggcaguacug acgcuuuccu gcaaugccuu gguggcuuug   1740
   accguguuca gaacuccccu guacaucucu uccauaaagc ugcuaauugg gguaaucgcg   1800
   guaguggaca uucucauggg ggucuccagu gcugugcugg cugccgugga ugcauucacu   1860
   uuuggccguu uugcucagca cggugcgugg ugggaagacg gaaucggcug ccaaaucguu   1920
   ggcuuccugu ccauuuuugc uuccgaaucg ucgaucuucc ugcucacucu ggcagcgcug   1980
   gaacgagguu uuucugucaa gugcucuucg aaguuugaag ugaaagcucc ccuuuuuagc   2040
   cugagagcga ucguuuugcu auguguccug  uuggcccuga ccauugccac aauccccuug   2100
   cuaggaggca guaaguacaa ugccucuccc cucugccugc ccuugcccuu uggggagccc   2160
   agcaccacgg gcuacauggu ggcucucgug uugcucaacu cucucuguuu ccucauaaug   2220
   accauugccu acacaaagcu cuacugcagu uuggagaaag gagagcugga gaaucuuugg   2280
   gauuguucga uggugaagca cauugcucug uugcucuucg ccaacugcau ccuuuacugc   2340
   cccguggcuu ucuuauccuu cuccucuuug cuaaaccuca ccuuuaucag uccugacguc   2400
   auuaaauuua uacuucucgu gaucguccca cuuccuuccu gucucaaccc acuucucuac   2460
   auugucuuca auccccauuu uaaggaggau augggcagcc ugggaaagca uacccguuuc   2520
   uggaugagau caaaacacgc gagucugcug uccauuaacu cggacgaugu ugagaaacgg   2580
   uccugugagu caacccaagc cuuaguaucc uuuacccacg ccagcauagc cuaugacuug   2640
   ccuuccacuu ccggggcauc accagcuuac cccaugacug aaagcuguca ucucucuuca 2700
   guugcauuug ucccaugucu cuagugacua ugagagagga acguuuuuaa gaguuggaaa 2760
   ccugaaaagu gauuucuauc agagcaguag cuaagaaaag cugagcuaaa aaccuaccuu 2820
   aaaacccaag caaacaucuc uaaauuggug uggaaacagu ggugccuuag agcaggagag 2880
   caucauuaaa caccgcuugu aucauuuguu cagcuaagaa ggaaagccau caagucacuu 2940
   aggugaaccc agaugagaaa agcagccuga aaugcucuuc gcauuguagu cucuucugac 3000
   ucaccagcau agucucccau agugagaaga cucguuggau gacucaaugg guguauuuaa 3060
   auccacaaau uccuuguuua aaagguuaga guuuuaagaa aaaaaaaaaa aaaaa 3115
<210> 246
   <211> 5561
   <212> RNA
   <213> Homo sapiens
<220>
   <221> misc_feature
   <222> (4879)..(4879)
   <223> n is a, c, g, or u
<220>
   <221> misc_feature
   <222> (5116)..(5116)
   <223> n is a, c, g, or u
<400> 246
   uuuuuuuucu ucaccuccuc ccuuuucaag gccuccaagc uaauuauuuc uguugcuuug      60
   gagugagcaa uucugugguu cucuccacca ccacccccaa uucugacccg aucccgccug      120
   gggguuucua cggucuccgc ucacucugcg ugcaccuggc gcgccucuuu uuuucacccc      180
   caaccuguug caagucuuua auccucgcaa uugggacuug cgugcaggca ccugaauccu      240
   ccuugccuca uauuuugcaa guguuugggg gagagcaccu gcucuaccug caagagauuu      300
   aaaaggaaaa aaaucuccag gcucccucuu ucuccacaca cucucgcucu ccugccccgc      360
   cccgagguaa agccagacuc cgagaaaaug gugaucagcg ugguccuccu gcugcuggcc      420
   gccuaugccg uaccggcuca aggccugggu ucuuucgugc auugugaacc cugcgacgag      480
   aaagcucugu ccaugugucc ccccagcccu cugggcugug agcuggucaa agagcccggc      540
   uguggcugcu gcaugacuug cgcccuggcg gagggacagu cguguggugu cuacacagag    600
   cgcugcgccc aggguuugcg cugccucccc cggcaggaug aggagaagcc gcugcacgcc      660
   cugcugcacg gccgcggggu uugccucaac gaaaagagcu acggcgagca aaccaagaua      720
   gagagagacu cucgggaaca cgaggaaccc accaccuccg agauggcuga agagaccuac      780
   ucccccaagg ucuuccggcc caagcacacu cgcauuuccg agcugaaggc ugaggcugug      840
   aagaaggacc gcagaaagaa gcugacccag uccaaguuug uggggggugc agagaacacu      900
   gcccacccca gagucauccc ugcaccugag augagacagg aauccgaaca aggccccugc 960
   cgcagacaca uggaagcuuc ccuccaggag uucaaagcca gcccacgcau ggugccccgu 1020
   gcuguguacc ugcccaacug ugaccgcaaa ggauucuaca agagaaagca guguaagccc 1080
   ucccguggcc gcaaacgugg caucugcugg uguguggaca aguacggaau gaagcugccg 1140
   ggcauggagu acguggaugg ggacuuucag ugccacgccu ucgacagcag uaacguugag 1200
   ugacgcgucc ccucccuucc ucccccuauc cuaccccccc agccccaacu ccagccagcg 1260
   ccucccucca ccccaggacg ucacucauuu caucucauuu aggggaaaua uauauacaua 1320
   uauauuugag gaaacugagg accucggaau cucuagcaag ggcuaaggag acacucccca 1380
   ccaugacccc ggaaauguau uccuuuuuga agcaaguuga acggacagag aagggaagga 1440
   gagaagaagc aagagggagc gagagaugga aagaaagcaa agcguuggaa uagaggaaaa 1500
   gagggaagga cagauaggau uagagagaga agagagaaac agcaaggcag aaaggacucc 1560
   acaaccaagg cugaaucugc ccuuuugcuu ucagcucuag ccugggguca gaaaaagugu 1620
   ggcauucagu gacacccagu uuagauuggu caaggggaga aaagaaacaa ggugugucag 1680
   ugccucucgg gucugucccc uccugcagcc agcagugugg auggcuagac cccucacccu 1740
   ccucuccucu uacccaagug cagggugauu ucauccccaa auuuacaaag acuaaaaugc 1800
   auuccauccc ucugaaaaua aacaaaagug agugauugaa gauagguuuu cccccagcag 1860
   acaagugaac ucagaaugug ugcaaauuuu acucuuguua aagauuuuuu uaagaaguca 1920
   guacgcaccc ccaacacugg aaagacuuga uucuccaggg ugacaagcaa uucagaagcg 1980
   cguggcuucg gcccuugauu ucacuagacu caaagcuggc ccggcagccu cuguggagga 2040
   ggaugagagg uggagaaaac caaggggcuu guacucaccc acaagacucc auguagacuu 2100
   uauaggcaua uaaaucuauu uucuuuaccu uuuuuucccu uucccuuucu uucgaaguuu 2160
   ugcauuaccu cuuuaaagua guuuuuuuua ggacacugaa gaucuuccuc auucugggaa 2220
   aaauccauau uucacaaaua caacccagaa cgccagcuug gccugcgucc aggcagccuu 2280
   ucucgugagc uacaagugug gcucuuuugu ggggcaccga uuuggaucuu cucaugauuc 2340
   caaacgugug uugaagugaa uccaccaagc cagguaacug ccagcaccca agggugcauc 2400
   aagugcauag cccaggucac cccauuucag ccuuccaacc cgcagaaagu aacugucuca 2460
   caccacacca cauaaaccug ccagauccau cuguaaccca cuggccugcc cagaccuuuu 2520
   uuucccaucu gcauuuuuuu uuuugaacug cauuuugaaa gccucccuca gaugccaggc 2580
   ugacagauca gagagaaacu aacaugagag augacagagg aggaggaagu ggaggguggg 2640
   ggcagagacu uccacagaga gacauagaag auggagcaga ggucuggggg uggggaggac 2700
   aagaaagaga cagagagagg aaaauaccaa uagaauuuuc cuuggugucu cccaucuaau 2760
   caacucucug agauuugaga ggaaaaagaa ggcaggggaa gaacuugagg uagaaaugag 2820
   gucaguucaa gucacagggc ccagauggug gguaacugag gcaggaucca gacuugagac 2880
   acacgguugg aaacaaggcu gguuagccug acuggguauu gaagggugaa gaggaugccu 2940
   uggaaagaca gcacaacuuc aguucaacuu caggccccca aggaggaacu gaggccaaag 3000
   aauccuucaa gugcuucaug agucuccucu gcccgacuca aacauccuuc ccugugaugg 3060
   aggaugguag aaagcccagg acaacucagg cccggaucuu cacgacuguu gucauuugcc 3120
   agccugauuu ugauccaaga gaagcaucuc auugcccacu ggcuucuuca acaaagaggu 3180
   gcuuaacaaa aggcucagga cuaucuuuga agacugaaga uaaccuucca ggagaggaga 3240
   cucggacauu gguacaggag gccccuuuug gcgggggaca cagcucuuug cgcucucuug 3300
   auggcauggc auaguagagg cccccuccaa cccggaacau ggagcaacac aaagggagag 3360
   caaagaaacu gacgugcguc gacucauagg acaugggugg cugcgggcac agaaagggau 3420
   gccuccuguu gccuggacag gacaguuggc ugggaaggaa agagaaaauu gaucuucaua 3480
   agacaaaggg ccugauggga uggcaauaga aggacuuacc agaccugcag ggucaguaua 3540
   cccaucaccc cgcacaacau ccccagcccc caacucaaac uucaauauau cuuaggccag 3600
   uauccuagac cuaagucucu ccuuucugcc auuauuucuc cgcaucuuga gcagucaucu 3660
   gacugagauu ugccaagugg auacuggggu accacugccc cccaagaaaa gacugagcca 3720
   ggaacugccu acucgcuccc ccucccgagc cuggagcuaa cuccugugag gggugcucuc 3780
   uucaccccac aacuuacuag accuugagug agccucuguc ccuuaugugg gcucuucgcu 3840
   gugagccaca gauggagguc auuguauaga caguuagccu uccccagguc agccuaccuc 3900
   ccccaaacuu gugagucucc ccgcugcuca uauggagagg caugucuaag acagcaaguc 3960
   uucuagagga agcuugccuu uaacagacag auggaacuaa accuuccaaa ugggagaucu 4020
   ggcugaaccc aggauacaga gaccauggac auggaugggg ucaucaagag aagagggaug 4080
   uccuucucca gguuagggag agagaaggca aguuugcaac gaucccauca ugcccugagc 4140
   aagaagcuuu uggcccaggc uagccuuuaa cuccauuaga ggccucucug uuggguuuau 4200
   ccacagcagu aggcccaagu auggccuguc cccaccucua cuaucccgug gaagguuucu 4260
   ucccaccccu uuuugacaaa ugccucacuc gagcagugga aagauagcuc ucuucccccu 4320
   cuucugccag guaacaaaga gaccuaacca ggaccuauuc uccaccccag ccagucuuga 4380
   ccagccagaa caaagcaggg aaccuggaga auaaaagacu cuacguuccu cugacaaaga 4440
   cucuacguuu cucugacggc caggccuaaa cagacaaggc uugggaacau cugccccaca 4500
   ggauacggag gaggucagcu gugcucacuc cucuucuccu uccaagaccc ucuuccgacc 4560
   augacuuauc uccaugguaa cauccucacc aucauucucc cacuaccaag gguugccaug 4620
   gcaaccuccc aaccaccugc ccaucccagg caggcagcug uccuucugcu cagaacccua 4680
   gaggacucua ggugaaauuu uacagcuuaa gagaggagug agccaaggag aagagacccu      4740
   guaguccucu ggcuuucaag agaaagaagg cuaugauuua aaacacagua gaagggaaag      4800
   aggucucguc gaggucgacc ucucccgggg agcuuagggg uuguacuguc uuuauuuuuu      4860
   aaaccacuaa agugcaaunu uuccugcacu cuuguuaccc cgccucucuu cccuguuagg      4920
   uuuucauuuc cuugagcaga cuuucuuggu uuuuuaaugg aguauagacu uucaccacuu      4980
   cacagacucu ggccuccucu ccaagucucu cuggaugggg aaaaggaagg uagaggguca      5040
   gaggggaagg ggucccucgu caccccgcau ccauucaccc ccacuucucu ggucccuagu      5100
   caccggcuuc accccnaucu ccgacaccau cacugucaca caguagccug ucacacggau      5160
   aguacaguuc agacaagacu ccuucagauu ccgagacgcc uaccgguugu uuuugguuuu      5220
   uguuuuguuu ucuuuuguuu guuuguuugu uuguuuuuua caacagcaau aaccacauca      5280
   cauauuacug uagcucucua uaguguuacg uucagacacc guagcucugu ccucucuuau      5340
   uuuguugguu uugacuuaaa aaaaaauacu uaugcuuuuu acuggugaaa cagauugaaa      5400
   aaaaaauuga acaacaaacc aguuugugaa aaaaaaaaaa aaugugaaaa aaaaucaccc      5460
   cgauguggaa gagcucggcu ccucuuuagc auuuuguacu uaaggaaaua aaaaagaaaa      5520
   accuggaaga ucucacauuu uauuacaaag ugaaaaaaaa a                          5561
<210> 247
   <211> 1113
   <212> RNA
   <213> Homo sapiens
<400> 247
   guccagucug cagucuguau ucggaagaca uagauacuaa auacauggca acucuuuuuu 60
   uuguuuguuu uaauucauca ggauguggag cgcuagucug gguaggagag ccagucaccc 120
   ugaggacagc ugaaacaauc gcuggcaagu auggagugug gaugagagac cccaagccca 180
   cccaccccua cacccaggaa agcacaugga ggauugacac gguuggcaca gagauccgcc 240
   agguguuuga guacagucag auaagccagu ucgagcaggg cuauccuucc aagguccaug 300
   ugcucccucg ggcacuggag agcacgggug cuguggugua ugcggggagc cucuauuucc 360
   agggggcuga guccagaacu guggucaggu augagcuaga cacggagacc gugaaggcag 420
   agaaggaaau uccuggagcu ggcuaccacg gacacuuccc guacgcgugg gguggcuaca 480
   cagacauuga cuuagcugug gaugagagcg gccucugggu caucuacagc acggaggaag 540
   ccaagggggc cauaguccuc uccaaauuga acccagcgaa ccuggaacuu gagcguaccu 600
   gggagacuaa cauccguaag cagucugugg ccaaugccuu uguuaucugu ggcaucuugu 660
   acacggugag cagcuacucu ucagcccaug caaccgucaa cuucgccuac gacacuaaaa 720
   cggggaccag uaagacccug accaucccau ucacgaaucg cuacaaguac agcaguauga 780
   uugacuacaa cccccuggag aggaagcugu uugccuggga caacuucaac auggucaccu 840
   augauaucaa gcucuuggag augugaggag ccucuaugcc uaccagcaaa ggccagaaaa 900
   ggugaaguuc cgggcucccg ggugaagcag cugucagcag aggcagccag augcauggag 960
   uuucuccucc ugcuaaagau uuuguuuauc cgggucaaug uacagcuagc uccccucuga 1020
   cugacacguc cuccaggcuu guauagucgc auagacucug uucucuucug ucagcuuuca 1080
   aagggcuguu ccucuuuuaa aaaucacaua gug 1113
<210> 248
   <211> 1635
   <212> RNA
   <213> Homo sapiens
<400> 248
   gggagaccug agcucgcugc ugccugugga agacugggag aggagacacu aagugcugcu 60
   caagcaagcg cgauccucuc cucuuucaac cugcagccca agauacugau ucgagccgcg 120
   ccuuaccgcg cagcccgaag auucaccaug gugaagaucg ccuucaacac cccuacggcg 180
   gugcaaaagg aggaggcgcg gcaagauaua gaggcgcucg ucagucgcac uguccgagcu 240
   caaauccuga cuggcaagga gcucagaguu gucccgcagg agaaagaugg cucaucuggg 300
   agaugcaugc uuacucuccu aggccucuca uucaucuugg caggacugau uguuggugga 360
   gccugcauuu acaaguacuu caugcccaag agcaccauuu accaugguga gaugugcuuc     420
   uuugauucug aggauccugu caauuccauu ccuggaggag agccauacuu ucugccugug 480
   acugaggagg cugauauccg ugaggaugac aacauugcca ucauugaugu gccugugccc 540
   aguuucucug auagcgaucc ggcggcaauu auucacgacu uugagaaggg aaugacugcu 600
   uaccuggacu ugcuuuuggg aaacuguuau cugaugcccc ucaauacuuc cauuguuaug 660
   acuccaaaga aucuggugga acuuuuugga aaacuggcaa guggcaagua uuugccucau 720
   acuuaugugg uucgugaaga ccugguugcu guggaagaaa uucgugaugu uaguaaccuu 780
   gguauuuuua uuuaccaacu uugcaacaac cgaaaauccu uccgccuuag acgcagagac 840
   cuucugcugg guuucaacaa gcgugccauu gacaaaugcu ggaagauuag acacuucccc 900
   aaugaauuua ucguugaaac caagaucugu caggagugaa augugacaga uaaagaguau 960
   ccuugauaau aagaagucag gaacuuaccg ucugacuugg aaaauugaaa uugaugggau 1020
   acucaugcua uuuacucaua cauuuacucu auugcuuaua cuggaaaagg aaagggaaag 1080
   gggggagaaa acuacuaacc acugcaagcg auuguccaau ucuacuuuaa uugacauugc 1140
   uugcuguuuu caacaaguca aaugauuauc uuuucucuug aauuuauagg guuuagauuu 1200
   cugaaagcag caugaaugug ucaucuuacc auccugacaa uaaagcccau ccucugguuu 1260
   uauuuaaagc aagcucuuuc caacaucacu uggcuagagc augcuuuaaa uuuaaaauau 1320
   uugaaauuug uuuuugacau uuuuuugugu gaaacauguc aaaucucuua ccauucuuug 1380
   guuuucuucu uuauuauguu caacucuccu gauuucagaa guuacauuuu ugcauuucua 1440
   ucaggugcug uguaacgaau cugacugaua ugugaacaau cuucaugagg aagcaauuuu 1500
   uuacucaugu aaugauucuu ucucacugau aucuguauug ugaaauccac agaacuguac 1560
   aggugcugaa ugcuguaagg aguucugguu guaugaauuc uacaacccua uaauaaaguu 1620
   uaccguauuc aauca 1635
<210> 249
   <211> 3550
   <212> RNA
   <213> Homo sapiens
<400> 249
   ggccauuacc aaucgcgacc cgcgcacaca cggcccgggc ggcgggcgaa gcgggcuccc 60
   ggggcgcugg gcgcagggcg cggggcaagc cccagcagcg ugucugcaac ggggcgcggc 120
   gggcgcucca gcuccgggau cuuucucccu cggucaccuc ccucgcgucu agggaggucg 180
   uggcacuccc ugaggagcgc ggcugcucgg agggcggauc cuagaacaga ggcgugagag 240
   ccggcaugaa uggucauaug ucuaaccgcu ccagugggua uggagucuac ccuucucaac 300
   ugaaugguua cggaucuuca ccacccuauu cccagaugga cagagaacac agcucaagaa 360
   caagugcaaa ggcccuuuau gaacaaagga agaacuaugc ccgagacagu gucagcagug 420
   ugucggacgu gucccaguac cgcguggaac acuugaccac cuucgugcug gaucggaaag 480
   augcaaugau cacugucgag gacggaauaa gaaagcugaa guugcuggau gccaagggca 540
   aaguguggac ucaagauaug auucuccaag uggaugaccg agcugugagc cugauugacu 600
   uagagucaaa gaaugaauug gagaauuuuc cucuaaacac aaucucgcau ugucaagcag 660
   uggugcaugc augcagcuau gacuccauuc ucgccuuggu augcaaagag ccaacgcaga 720
   gcaagccaga ccuucaccuu uuccagugug augagguuaa ggcaaaccua auuagugaag 780
   auaucgaaag ugcaaucagu gacaguaaag gugggaaaca gaagaggcgg ccggaggccc 840
   ugaggaugau ugccaaagca gauccuggca ucccuccucc ucccagagcu ccugccccug 900
   ugccaccggg gacugucaca cagguggacg uuaggagucg cguagcagcc uggucugccu 960
   gggcagcuga ccagggugac uucgagaagc cccggcagua ccacgagcaa gaagagacgc 1020
   ccgagaugau ggcagcccgg aucgacaggg augugcaaau cuuaaaccau auuuuggaug 1080
   acauugaauu uuuuaucacc aaacuccaaa aagccgccga agcguuuucu gagcuuucua 1140
   aaaggaagaa aaguaagaaa aguaaaagga aaggaccugg agagggcguu uuaacacuga 1200
   gggcaaaacc gccaccuccu gacgaguuug uugacuguuu ccagaaguuu aaacauggau 1260
   ucaaccuucu ggccaaguug aagucccaua uccagaaccc gagugcuuca gaucugguuc 1320
   auuuuuuguu uacuccacua aauauggugg uccaggcaac agguggcccu gaacuggcca 1380
   guucgguacu cagcccacug uugacaaaag acacaguuga uuucuuaaac uacacagcca 1440
   cugcggagga acggaagcug uggaugucac ugggagauag uugggugaaa gugagagcag 1500
   aguggccgaa agaacaguuc aucccaccuu acgucccgag guuccgcaac ggcugggagc 1560
   ccccgaugcu gaacuucaug ggcgcgccca cagagcaaga cauguaucaa cuggccgagu 1620
   ccguggccaa cgcagaacac cagcgcaaac aggacagcaa gaggcugucc acagagcauu 1680
   ccaauguguc cgacuauccu ccagccgacg gauaugcgua caguagcagc auguaccaca 1740
   gaggaccaca ugcagaccac ggggaggcug ccaugccuuu caagucaacu ccuaaucacc 1800
   aaguagauag gaauuaugac gcagucaaaa cacaacccaa gaaauacgcc aaauccaagu 1860
   acgacuuugu ggcgaggaac agcagcgagc ucucgguuau gaaagaugau gucuuagaga 1920
   uacucgacga ucgaaggcag ugguggaaag uccggaaugc caguggagac ucuggguuug 1980
   ugccaaauaa cauucuggau aucaugagaa cuccagaauc uggagugggg cgcgcugacc 2040
   ccccauacac acauaccaua cagaaacaaa ggacggaaua cggccugaga ucagcugaca 2100
   cuccuucugc cccaucaccc ccuccaacgc cagcacccgu uccggucccc cuuccaccuu 2160
   cuguaccagc acccguuucu gugcccaagg uuccagcaga ugucacccgc cagaacagca 2220
   gcuccaguga cagugggggc agcauugugc gggacagcca gagauacaaa caacucccag 2280
   uggaccgaag gaagucccag auggaagagg uucaggauga gcucuuccag aggcugacca 2340
   ucgggcgcag ugcugcgcag aggaaguucc acgugccacg gcagaacguu ccagugauca 2400
   auaucacuua ugacuccuca ccggaagaag uaaagacuug gcugcaguca aagggauuca 2460
   aucccgugac ugucaauagc cucggggugu ugaacggagc acaacucuuu ucucucaaca 2520
   aagacgaacu gaggucuguc ugcccggaag gugccagagu cuuuaaccaa aucacuguuc 2580
   agaaagcugc uuuggaggac aguaauggaa gcuccgaguu acaagagauc augcggagac 2640
   ggcaggagaa gaucagcgcc gcugcgagcg acucgggagu ggagucuuuu gaugaaggga 2700
   gcagccacug aguccaugaa cuuccuuauu cuuggugugg ucguugaaca gugauggaca 2760
   ugcuuuguuu uaagaagccu ugaagggaau gucaaagcug ucgucuuggu auauguaauu 2820
   uaucgccaua uaaggaaaca guauaugccu gaguaagcag aggacccgcu gcuucugugc 2880
   acauuaguuu gauuaaaacu gagaagcggg uaggugagau ggcucagcaa guaaaggugc 2940
   uugcugccaa gcccaaugac ccaaguucga gucccugggu cuacauggua ggagagagcu 3000
   ggcuucugca aguuguccuc ugaccaccac acauaaauaa auaacaaaug uaauuuacaa 3060
   acuuuuaaaa gaaaauguaa uuuaaaaaac cagacguucu agacuguucu gggcuuggga 3120
   aauauuuuuu ucacuuuccu aagguguacu uuccuuugcu acauuaauua uugcagccuu 3180
   guucgaugau cuaagugggg auauuugaca auggcagauu uauucauugc aacaaggaaa 3240
   gacacagcca uugaugaaaa aaaaaagaaa gucucagcuu ucagugacug ggauaccugc 3300
   uguccaggga ggaggcucag uuagacuacc cucugcuuac uugaggucug acaugcccaa 3360
   ugagagugua uuuagcuuua uuuaaaguuc uuaaugccaa caguuuuaaa aaucacauuu 3420
   aaaugaacug uacaagguag ccagaccuug aauguaugaa uagacuauau aauaugaccc 3480
   gagaaacuuu guuacucuca gcucuguuga uugcgaaauc uugcauagau uaugcuuuga 3540
   uuuaguuucu 3550
<210> 250
   <211> 2299
   <212> RNA
   <213> Homo sapiens
<400> 250
   ccugggcccc gccgcggacg cgcggagccg ccugggccgc gccggaggag ggcggggaga 60
   ggaccaugug aaugugcucc ggagcugagc gccaagccaa gcaguguuug aaaggaacag 120
   gaugcugauc uaaucguggc aaaaagucag uccgaccgcu gguuucgaag acauguggug 180
   uauauaaagu uugugauagu ugguggaaau uugggagcuu ggauaauggg cugugugcaa 240
   uguaaggaua aagaagcagc gaaacugaca gaggagaggg acggcagccu gaaccagagc 300
   ucuggguacc gcuauggcac agaccccacc ccucagcacu accccagcuu cggcgugacc 360
   uccaucccga acuacaacaa cuuccacgca gcugggggcc agggacucac cgucuuuggg 420
   ggugugaacu ccuccucuca cacugggacc cuacgcacga gaggagggac aggagugaca 480
   cuguuugugg cgcuuuauga cuaugaagca cggacggaag augaccugag uuuucacaaa 540
   ggagaaaaau uucaaauauu gaacagcucg gaaggagauu ggugggaagc ccgcuccuug 600
   acaaccgggg aaacugguua cauucccagc aauuacgugg cuccaguuga cuccauccag 660
   gcagaagagu gguacuuugg aaaacuuggc cgcaaagaug cugagagaca gcuccugucc 720
   uuuggaaacc caagagguac cuuucuuauc cgcgagagcc aaaccaccaa aggugccuac 780
   ucacuuucca uccgugauug ggaugauaug aaaggggacc acgucaaaca uuauaaaauc 840
   cgcaagcuug acaauggugg auacuauauc acaacgcggg cccaguuuga aacacuucag 900
   caacugguac agcauuacuc agagaaagcu gaugguuugu guuuuaacuu aacugugguu 960
   ucaucaaguu guaccccaca aacuucugga uuggcuaaag augcuuggga aguugcacgu 1020
   gacucguugu uucuggagaa gaagcugggg cagggguguu ucgcugaagu guggcuuggu 1080
   accuggaaug gaaauacaaa aguagccaua aagacccuua agccaggcac caugucuccg 1140
   gaguccuucc uggaggaggc gcagaucaug aagaagcuga agcaugacaa gcuggugcag 1200
   cucuacgcgg ucgugucuga ggagcccauu uacaucguca cggaguacau gagcaaagga 1260
   aguuugcuug acuucuuaaa agauggugaa ggaagagcuc ugaaguugcc aaaccuugug 1320
   gacauggcgg cacagguugc ugcaggaaug gcuuacaucg agcgcaugaa uuauauccac 1380
   agagaucugc gaucagcaaa cauucuagug gggaauggac uaauuugcaa gauugcugac 1440
   uuuggauugg cucgguugau ugaagacaau gaauacacag caagacaagg ugcgaaguuu 1500
   cccauuaagu ggacagcccc cgaagcggcc cuguauggaa gguucacaau caagucugac 1560
   guauggucuu uuggaaucuu acucacagag cuggucacca aaggaagagu gccauaccca 1620
   ggcaugaaca accgggaggu gcuggagcag guggagagag gcuauaggau gcccugccca 1680
   caggacugcc cgaucucccu gcacgagcuc augauccacu gcuggaaaaa ggauccggaa 1740
   gagcgcccga ccuucgagua cuugcagggc uuccuggagg acuacuuuac ggccacagag 1800
   ccccaguauc agcccgguga aaaccuguga gagccugcgc uucagacgcc ucuucccgag 1860
   gccucccuac cccuccccau uagcuuccaa uucuguagcc agcugcccca gagcaggaga 1920
   accguccagg aucagauugc augugacucu ugaagcugaa cuuccacggc ccucauuaau 1980
   gacacuuguc ccccaguccg aaccuccucu gugaaccauc ugagacagaa gcguguuauu 2040
   ucucagacuu ggaaaugcau uguaucgaug uuaugucaaa ggccaaaccu cuguucagug 2100
   uaaauagcug cuccugugcc aacaauccca gugcuuuccu uuuuuaaaaa agaaaaagca 2160
   aauccuaugu gauuuuaacu cugauuucac cugauucaac uaaaaaaaaa aaaguauuau 2220
   uuuccaaaag uggccucuuu gucuaaaaca auaaaauuuu uuuucauguu uuaacaaaaa 2280
   aaaaaaaaaa aaaaaaaaa 2299
<210> 251
   <211> 3991
   <212> RNA
   <213> Homo sapiens
<400> 251
   cccucccugg cucucuccuc agcucugggc ucugacugca gcaagcagag acaaccucuc 60
   acucugccuu ucccagcgcc cacccugacc cuggcccaca uuugacggug acucgcaggc 120
   cagccagaaa caugaggcug gcccacgcuc ugcugccccu gcugcuacaa gccugcuggg 180
   uggccacaca ggacauccag ggcuccaaag cgauugccuu ccaagacugc ccuguggauc 240
   uauucuucgu gcucgacacc ucggagagug uggccuugag gcugaaaccu uauggggccu 300
   ugguggacaa ggugaagucc uucacuaagc gcuucauuga caaccugaga gacagguacu 360
   accgguguga ccgcaaccug guuuggaaug cgggugcgcu gcacuacagu gacgaggugg 420
   agaucauccg agggcucacg cgcaugccca guggccgcga ugagcucaag gccagcgugg 480
   augcggucaa guacuucggg aaaggcaccu acaccgacug cgccauuaag aaggggcugg 540
   aggagcugcu cauagggggc ucccaccuga aggagaacaa guacuugauc guggugaccg 600
   acgggcaucc ucuagagggc uacaaggaac caugcggggg ucuggaagau gcaguaaaug      660
   aggccaaaca ccugggcauc aaggucuuuu cuguggccau cacaccugac caccuggagc      720
   cacgucuaag uaucauugcc acagaccaca cauaccggcg caauuucacg gcagcugacu      780
   gggggcauag ccgcgaugca gaagagguca ucagccagac cauugacacc auuguggaca      840
   ugauuaaaaa uaacguggaa caaguguguu guucuuuuga gugccaggcu gccagaggac      900
   cuccagggcc ccgaggcgac ccuggguaug agggggagcg aggaaagcca ggucuuccgg      960
   gagagaaggg agaagcugga gacccuggac gaccugggga ucuuggacca gucggguacc     1020
   aggguaugaa gggagaaaag gggagccgug gagagaaggg uuccagagga ccgaaagguu     1080
   acaagggcga gaaaggcaag cgcggaaucg acggggucga cggcaugaag ggagagacgg     1140
   gguacccagg acuaccgggc ugcaagggcu ccccaggauu ugauggcauu caaggacccc     1200
   cgggucccaa gggugaugcu ggugccuuug ggaugaaggg agaaaagggu gaagcuggag     1260
   cagacgguga ggcugggaga ccagggaacu cagggucacc uggagaugag ggugauccug     1320
   gagagccugg uccccccgga gaaaaaggag aggccgguga ugaaggaaau gcuggcccag     1380
   acggugcccc uggagagagg gguggcccug gugaaagagg accucggggg accccuggug     1440
   ugagaggacc aaggggagac ccgggugaag cuggaccaca gggugaccaa ggaagagagg     1500
   ggcccgucgg caucccugga gacucgggug aggcuggccc cauuggaccu aaaggauacc     1560
   gaggugauga ggguccucca gguccugagg gccucagagg agccccagga ccuguugguc     1620
   cuccuggaga ccccggacug augggugaga gaggugagga uggaccacca ggaaacggca     1680
   cggaagguuu ccccggcuuc ccuggguauc caggcaacag aggcccuccu gggcuaaaug     1740
   gcacaaaagg cuacccuggc cucaaggggg augaggguga agugggagac ccaggagagg     1800
   auaacaacga cauuucaccc cgugggguca aaggggcaaa gggauaccga ggcccagaag     1860
   gaccccaggg accuccagga caugugggac caccugggcc agaugagugu gagauccugg     1920
   auaucaucau gaaaaugugc uccugcugug agugcacaug uggacccauu gacauccucu     1980
   ucgugcugga cagcucggag agcauuggcc uacagaacuu ugagauugcc aaggacuuca     2040
   ucaucaaggu cauugaccgg uugagcaagg augagcuggu caaauuugag ccagggcagu     2100
   cucacgcggg cgugguacag uacagccaca accagaugca agagcacgug gacaugcgga     2160
   gccccaacgu ccgcaacgcc caggacuuca aagaagcugu caagaagcua caauggaugg     2220
   cugguggcac auucaccgga gaagcgcugc aguacacccg ggaccggcua cucccaccca     2280
   cacagaacaa ccgaauugcc cuggucauua cggauggacg uucugacacu caacgggaca     2340
   cgacaccucu cagugugcuc uguggugcag acauucaggu aguuucugug ggaaucaagg     2400
   auguguuugg cuuuguggcg ggcuccgacc agcucaaugu cauuuccugc caaggcuuau     2460
   cgcaaggucg uccagguauc ucccugguga aggagaacua ugcagagcuu cucgaugacg     2520
   gcuuucugaa gaacauaaca gcccagaucu guauagauaa gaaguguccg gauuauaccu 2580
   guccaaucac auucuccucc ccggcugaca ucaccauccu gcuagacagc ucagccagug 2640
   ucggcagcca caacuucgaa accaccaagg ucuucgccaa gcgccuagcu gagcgauucc 2700
   ugucagcagg cagggcggau ccuucccagg augugcgggu ggccguggua caguauagug 2760
   gccaggggca gcaacagcca ggucgggcgg cucuucaguu cuuacagaau uacacagugc 2820
   uggccagcuc uguggacagc auggauuuca ucaacgacgc cacagacguc aacgaugcuc 2880
   ugagcuacgu gacucguuuc uaccgggaag ccucgucagg ugccaccaag aagagagugc 2940
   uguuguuuuc agacggcaac ucucaggggg ccacagcaga ggccauugag aaggcugugc 3000
   aggaggccca gcgugcaggc auugagaucu uugugguggu ggugggaccc caggugaacg 3060
   agccccacau ccgugugcuu gucacuggca agacugcaga guacgacgug gccuuuggcg 3120
   agcgccaccu auuccgugua ccaaacuacc aggcccugcu acguggcgua cucuaccaga 3180
   cagucuccag gaagguggca cugggcuaga gggccacaca cguggcugga cacacauggc 3240
   auggagacac auuucaacag gccuucccgc ccuucccacu gacaaaacag gaauaggaaa 3300
   ugugacccaa cuggucaacu caacugucuu aaagggaacg cugagaugca cacucuuugc 3360
   uuuguguaau guccccugug gcucaccuga gcuccuaucu agaucccgcc cuugguuugu 3420
   acaucauggu ggccaucuug cugaccccuc ccccaucugg gacuggaucc agccaucucg 3480
   ucuuccuccu cacugccccu aaccuauccg uggugucuuc acaccaucac ugcaguuucc 3540
   gucuguguuc ugucuuccau gcucaacaug aagcagaccu ucucaugagu ucagcuugcu 3600
   ggauuauggc uuuuaggaaa uugaacacag gaggaguucc aaacacaaac uuggaggaga 3660
   ccccuccucu ucaucaggug cuugucagug accuacaugc aucuuggucu gguccuuagu 3720
   ggcuaguccu uccacucuga aagcaaaggu gcuaucuauc uguaagggcu cucucuacac 3780
   acccagaggc uuagcuugga caguucacac ucaagugucc ugucagaauc aauccagagc 3840
   uuucucccuc aaaauaguga cuugucuccc ccuggucccc aaaggcuccc cuuuaguuag 3900
   uuucuucaug gcucccccac auuccccgua aucugaucca agccagcuau cucugcuaau 3960
   aaagguuucc auuuuucaaa aaaaaaaaaa a 3991
<210> 252
   <211> 235
   <212> RNA
   <213> Homo sapiens
<400> 252
   accccaugcc ccacccccac cuucgauguu uugaacauuu cuaacaacug aagccaguaa 60
   agucauauuc uuuaaauuuc caggacauuc auauuauuca cauaaucaug gucaugguga 120
   ugauggaaac ugaggacuuu aaaagagauu uucccuuccc aaacguuucu ggacaguacc 180
   ugauuguauu uuuuuuguuu uguuuuguuu uuuaauaaaa gcacaguacu uuucc 235
<210> 253
   <211> 2005
   <212> RNA
   <213> Homo sapiens
<400> 253
   gucugcccug ccguuucucu acuucccagc cuucucaucu ccaggaacca ugucuaccaa 60
   aaccaccauc aaaagucaaa ccagccaccg uggcuacagu gccagcucag ccagagugcu 120
   uggacucaac cgcucgggcu ucagcagugu guccgugugc cgcucccggg gcagcggugg 180
   cuccagugca auguguggag gagcuggcuu uggcagcagg agccucuaug guguggggag 240
   cuccaagagg aucuccaucg gagggggcag cuguggcauu ggaggaggcu auggcagccg 300
   auucggagga agcuucggca uugguggugg agcugguagu ggcuuuggcu ucgguggugg 360
   agcuggcuuu ggugguggcu augggggagc uggcuucccg gugugcccac uuggaggcau 420
   ccaagagguc accaucaacc agagccuccu cacaccccug aaccugcaaa uugaccccac 480
   cauccagcgg gucaggacug aggagaggga gcagaucaag acccucaaua acaaguuugc 540
   cuccuucauc gacaaggugc gguucaugga gcagcagaac aaggucaugg acaccaagug 600
   ggcccugcug caagagcagg acaccaagac cgugaggcag aacauggagc ccauguuuga 660
   gcaguacauc agcaaccucc gcagacagcu ggacagcauc auuggagaga ggggucgcau 720
   gaacucagag cugaggaaca ugcaggaacu cguagaagaa cuacggaaca aauaugaaga 780
   ugaaaucaac aagcgcacag acgcagagaa ugaauucgug acccugaaga aggauguaga 840
   ugcugccuac augaacaaag uugaacugca agccaaggca gacagucuaa cagaugauau 900
   caacuucuug agagcucucu augaagcaga acugucucag augcaaacuc acaucucaga 960
   cacaucugug guccucucca uggucaacaa ccguagccuc guccuagaca gcaucaucgc 1020
   ugaggucaag gcccaguuug aggucauagc ucagagaagu cgggcugaag cugagucauu 1080
   guaccagacu aaauaugagg agcugcaggu cacagcuggc agacaugggg acgaccugcg 1140
   caacaccaag caggagauug cugagaucaa ccgcaugauc cagaggcuga gaucugagau 1200
   cgaccacguu aagaagcagu gugccaaccu gcaagcugcu auugcugaug cugagcaacg 1260
   uggggagaug gcccugaagg augcaagggg caagcuggaa gggcuggagg augcccugca 1320
   gaaggccaaa caggacaugg ccaugcugcu gaaggaguac caugaacuca ugaaugucaa 1380
   gcuggcccuu gauguggaaa uugccaccua caggaagcug cuggaaggag aggagugcag 1440
   guugaauggu gaagguguug gaccagucaa caucucugug gugcagucca ccguguccag 1500
   cggcuauggc agugccgggg gugccagcag cagcuuaggc auggguggag gcagcagcua 1560
   cuccuauagc agcagccaug gccuuggagg uggcuucagu gcuggcagug gcagagccau 1620
   cggagguggc cucagcucuu cugguggccu cagcucuucu accaucaaau acaccaccac 1680
   cuccuccagc aagaagagcu acaggcagug aauucuguca ccaagagcuu gucucugguc 1740
   ccagauguca uggcugcagc ugaaccacau gcuuugguuc ccggaaggga acgaauccca 1800
   accucuggcc uccccauggc ucaguucuac auuuguguug cacgucagca ccuauacaug 1860
   uucuuuggug acccagaccc caaaauguug cagaauguag accuccaaga cgaaacccca 1920
   aacccuaccc agaauaccca ccuaaauucu gucaugguuc ugacuuccuc cagagucugu 1980
   aaaauaaaau gcccccacaa caaac 2005
<210> 254
   <211> 357
   <212> RNA
   <213> Homo sapiens
<400> 254
   uuuuuuuuuu uuuuuuugau uuuaauuaca aacuuuauuu guccuccagu ucacaguuua 60
   uacgugguac aucccaccau gucagcuucc agaacggcua uucaggagau ggguggagcu 120
   uucuuuguaa aggaacccga cauuuuaaaa uuuugguuag aaucuucaua gguuuauaaa 180
   aguacucucu gcaagcgaac uggauauauu uacauuuaua gcuuaaauuc aaauuuugga 240
   aaauaggaau cuuuuugugu uuuuaaacau ccugguuuua ugucuuaaga cuuuacucug 300
   aaugccacau gaucacguaa gcccaagccu cccccagaag ggaaaaauca guuuugc 357
<210> 255
   <211> 271
   <212> RNA
   <213> Homo sapiens
<400> 255
   uuuuuuuuuu uuuuuuuggg ugccaugcca cagcaagggg ccuuuauuag auguucaggg 60
   cacagacagg uggaugcuag augguguagc acaccuucuc aggguggagu ggugccugga 120
   uguccaguuu gcgggucuug cuguccuuau caaugaucuc cagucgcaau guagguggac 180
   gcuucucagc cuggggugag ggcagcucac ggcucuugag gaggugcuug uccgaauccu 240
   ccacagugau acccagggug caacccccug g 271
<210> 256
   <211> 255
   <212> RNA
   <213> Homo sapiens
<400> 256
   uuuuuuuuuu uuuuucugac acaguaguaa uccuuccaga cuccuuacac aauauuacaa 60
   cucccccagu acauaaaugu uccuauacca ugcacacagc aacauggggu ccacugaugu 120
   cgcaggcgac uuuucuaaug guggaacaua gcaccucaag uucugccauc uacacaguga 180
   agggacguga uggucggggc uccagaguga cagcaaacug ccucuugggu gacacgcuug 240
   uggcgaagug ccucc 255
<210> 257
   <211> 4031
   <212> RNA
   <213> Homo sapiens
<400> 257
   gagaauguug cugaggaggu ggggcugcug cagcuccuug gagacccccu accugagaag 60
   aucucacaaa ucgaugaccc ucacgucggg ccggccuaca ucuuuggacc agacuccaac 120
   aguggccagg uggcccagua ucauuuccca aaacucuucu uccgggacuu uucgcugcug 180
   uuucaugucc ggccagccac agaggcagca ggggugcuau uugccaucac agaugcugcc 240
   caggugguag ucucacuggg cgugaagcuc ucagaggucc gagauggaca gcaaaacauc 300
   ucauugcucu acacggagcc uggggccagc cagacccaga cgggagccag cuuccgccua 360
   ccugcauuug uugggcagug gacacacuuc gcgcucagcg ucgacggagg cucuguggcu 420
   cucuacguag acugugaaga auuccagagg gugccauuug cucgggccuc gcagggacug 480
   gagcuagagc guggcgcugg ccucuuugug ggucaggcug gaacagcaga cccugacaag 540
   uuccagggga ugaucucaga gcugaaggua cgcaaaaccc cccgggugag cccugugcac 600
   ugucuggaug aagaagauga ugaugaagac cgggcaucug gagauuuugg aaguggcuuu 660
   gaagaaagca gcaagucaca caaggaggau acaucucuac uaccugggcu cccucagcca 720
   ccuccuguca cuuccccacc ccuggcugga ggcagcacca cagaagaucc uagaacagaa 780
   gaaacggagg aagacgccgc gguagauucu auaggagcug agacccuucc uggcacaggu 840
   ucaagcggug caugggauga ggcuauccag aaccccggaa ggggcuugau aaagggaggu 900
   augaaaggac aaaagggaga accaggugcc cagggcccac cuggcccagc uggcccccag 960
   gguccugccg guccaguggu ccagagcccc aacucacaac cugucccugg agcacaagga 1020
   cccccgggac cucaggggcc accagggaag gauggcacuc caggaaggga uggugaaccg 1080
   ggugacccug gugaagaugg gagaccgggu gacacuggac cucaaggcuu uccagggacc 1140
   ccaggagaug ugggcccuaa gggcgagaag ggagauccug guauugggcc ccgaggaccu 1200
   ccagggccuc cagggccacc aggacccucc uucagacaag acaagcugac cuucauugac 1260
   auggagggau ccgguuucag cggagacaua gagagccuua gaggcccacg aggcuucccu 1320
   ggccccccgg ggcccccugg ugucccagga cuuccuggug agccaggacg cuuugggauc 1380
   aaugguuccu augcaccagg accugcaggc cuuccuggug uaccugggaa ggaaggaccc 1440
   cccgguuuuc cagguccccc gggaccucca gguccuccag gcaaagaggg cccaccagga 1500
   guggccggcc agaaaggcag uguuggugau gugggcaucc caggacccaa ggggagcaaa 1560
   ggagaccuug ggcccaucgg uaugccuggc aagucuggcu uggcuggauc cccugggcca 1620
   guuggacccc caggaccucc agggccucca gggccaccag gaccaggauu ugcugcugga 1680
   uucgaugaua uggaaggcuc uggaauaccc cucuggacaa cagcccgaag cucugauggg 1740
   cugcagggac cucccggguc gccgggacuc aagggggauc cuggaguggc aggccuaccu 1800
   ggagccaagg gagaaguugg agcagaugga gcccagggca ucccuggucc cccaggaaga 1860
   gaaggugcag cuggaucucc ggggccaaaa ggagagaagg ggaugccggg agaaaaggga 1920
   aacccaggaa aagauggagu gggccggccg ggccucccug ggccuccagg accuccaggg 1980
   cuggugaucu augugucaag ugaggauaaa gcaauaguga gcacgccagg accugagggc 2040
   aagccagggu acgcaggcuu uccuggaccu gcuggaccga agggugaccu ggguuccaaa 2100
   ggcgagcagg gucuuccggg guuuaagggu gagaagggag agccaggcac uaucuuuagu 2160
   ccugauggca gacgucuggg ccauccccag aagggagcca agggagagcc aggcuuucga 2220
   ggaccccggg guccuuaugg acgaccuggg cacaagggug aaauuggcuu cccuggacgg 2280
   ccgggucgac cuggaacgaa uggcuuaaag ggagagaagg gagagccugg agaugccagc 2340
   cuuggguuca gcaugagggg auugccuggc cccccugggc cuccaggacc cccagguccu 2400
   ccugggaugc ccaucuauga cagcaaugca uuuguggagu cuggccgacc uggacuacca 2460
   ggacagcagg gugugcaggg gccuucagga ccaaagggug acaaaggaga gguggggcca 2520
   ccugggccac cagggcaauu ccccauugac cucuuccacc uggaagcgga aaugaagggg 2580
   gacaagggag accgagggga ugcuggacag aaaggagaga ggggagaacc uggggcuccu 2640
   gguggaggau ucuucagcuc aaguguaccu ggcccacccg gcccaccugg auacccugga 2700
   auuccggguc caaagggaga gagcauccgg gggccaccug gcccuccugg cccgcaggga 2760
   ccuccuggca uuggcuauga gggucgccag ggucccccag gaccuccagg accuccagga 2820
   ccucccuccu ucccuggccc ucacagacag acugucagug uuccuggucc uccgggccca 2880
   ccugguccuc cagguccccc aggagccaug ggugccucug cugggcaggu gaggaucugg 2940
   gccacauacc agaccaugcu ggacaagauc cgggaggugc cggagggcug gcucaucuuu 3000
   guggccgaga gggaagagcu cuauguacgc guuagaaaug gcuuccggaa ggugcugcug 3060
   gaggcccgga cagcccuccu gagaggcacg ggcaaugagg uggcugcuuu ccagccccca 3120
   uugguccagc uucaugaggg caguccauac acccggaggg aguacuccua uuccacggca 3180
   cgacccuggc gagcagauga cauccuggcc aacccaccgc gccugccaga ccgccagccu 3240
   uacccuggag uuccacauca ccacaguucc uaugugcacc ugccgccagc ccgccccacc 3300
   cucucacuug cucauacuca ucaggacuuu cagccagugc uccaccuggu ggcacugaac 3360
   accccccugu cuggaggcau gcgugguauc cguggagcag auuuccagug cuuccagcaa 3420
   gcccgagccg uggggcuguc gggcaccuuc cgggcuuucc uguccucuag gcugcaggau 3480
   cucuauagca ucgugcgccg ugcugaccgg gggucugugc ccaucgucaa ccugaaggac 3540
   gaggugcuau cucccagcug ggacucccug uuuucuggcu cccaggguca agugcaaccc 3600
   ggggcccgca ucuuuucuuu ugacggcaga gauguccuga gacacccagc cuggccgcag 3660
   aagagcguau ggcacggcuc ggaccccagu gggcggaggc ugauggagag uuacugugag 3720
   acauggcgaa cugaaacuac uggggcuaca ggucaggccu ccucccugcu gucaggcagg 3780
   cuccuggaac agaaagcugc gagcugccac aacagcuaca ucguccugug cauugagaau 3840
   agcuucauga ccucuuucuc caaauaggcc ucugccagcu aggguggcag acagaggcca 3900
   ugcagaacuu ugacacagcg cagggagcau ucagucagca cccagggcuc uggcugggau 3960
   acaacuccug uauaguuccc auuuuuaugu aauccucaag aaauaaaagg aagccaaaga 4020
   guaaaaaaaa a 4031
<210> 258
   <211> 1503
   <212> RNA
   <213> Homo sapiens
<400> 258
   aacugccuuc gagaagcguu agccuagaga uccgagccuc uucuccauac cauaguuggu        60
   ucaggugguu uccucuucaa accuugcguc ugcggauaau ccgcgcggcc gggcguuaag       120
   cuccaggucc cugucgcucc gucgaggugg caagccaugg ccggcugcug cuguuugucu       180
   gcggaggaga aagagucuca gcgcaucagc gcggagaucg agcggcacgu ucgccgcgac       240
   aagaaggacg cgcgccggga gcucaagcug cuguugcugg gaaccgguga gagugggaaa       300
   agcaccuuua ucaagcagau gaggauaauc caugggucug gcuacaguga ugaagauaga       360
   aagggcuuca cgaagcuggu uuaccaaaac auauucacgg ccaugcaagc caugaucaga       420
   gcaauggaua cccugaggau acaauacaug ugugagcaga auaaggaaaa ugcccagauc       480
   aucagggaag uggaaguaga caaggucacu gcacucucua gagaccaggu ggcagccauc       540
   aagcagcugu ggcuggaucc cggaauccag gaguguuacg acaggaggag ggaguaccag       600
   cugucagacu cugccaaaua uuaccugacg gacauugagc guaucgccau gcccucuuuc       660
   gugccaacac aacaggaugu gcuucguguu agagugccca ccacuggcau cauagaauau       720
   ccauucgacc uggaaaacau caucuuccga augguggaug uugguggcca gcgaucugaa       780
   cgacggaaau ggauucacug cuuugagagu gucaccucca ucauuuucuu gguugcucug       840
   agugaauaug accagguucu ggcugagugu gacaaugaga accgcaugga ggagagcaaa       900
   gcccuguuua gaaccaucau caccuacccc ugguuucuga acuccuccgu gauucuguuc       960
   uuaaacaaga aggaucuucu agaggagaaa aucauguacu cucaucuaau uagcuacuuc      1020
   ccagaguaca caggaccaaa gcaagauguc aaagcggcca gggacuuuau ccugaagcug      1080
   uaucaagacc agaauccuga caaagagaag guuaucuauu cucacuucac uugugcuaca      1140
   gacaccgaga auauccgcuu uguguuugcu gcugucaaag acacaauccu acagcuaaac      1200
   cuacgggagu ucaacuuggu guaaauggag ggccuacucc uccgagacag agggugaucu      1260
   gagcccuucc ugccugaucu acaagugcuu cuggaccagg accuaaggac auuauguagc      1320
   ccacaggaca gagaugggua gugcaaugug aaaaauacuu caccaacccu uuuaaguguc      1380
   uuuaauucuu cacugucuaa cucuuuucuc gccuuuuggu ugaacgauua gguaucauuu      1440
   uugagugguu cccccucucc uauuuuuuua aacuaguguu caacaguuau uaaaaaauca      1500
   ugc                                                                    1503
<210> 259
   <211> 876
   <212> RNA
   <213> Homo sapiens
<400> 259
   gaauucccug caaccuuguc ugagaggaag caaggacugg ugugaggagg gagcugugag        60
   guuuuaucug ugcagcccuu cucugaggau ggacacuucu cacacuacaa aguccuguuu       120
   gcugauucuu cuuguggccc uacugugugc agaaagagcu cagggacugg aguguuacca       180
   gugcuaugga gucccauuug agacuucuug cccaucaauu accugccccu acccugaugg       240
   agucuguguu acucaggagg cagcaguuau uguggguucu caaacaagga aaguaaagaa       300
   caaucuuugc uuacccaucu gcccuccuaa uauugaaagu auggagaucc uggguacuaa       360
   ggucaaugug aagacuuccu guugccagga agaccucugc aaugcagcag uucccaaugg       420
   aggcagcacc uggaccaugg caggggugcu ucuguucagc cugagcucag uccuccugca       480
   gaccuugcuc ugaugguccu cccaaugacc uccacccuug uccuuuuauc cucaugugca       540
   acaauucuuc cuggagcccu cuagugauga auuaugaguu auagaagcuc caagguggga       600
   guagugugug aaauaccaug uuuugccuuu auagcccugc uggguaggua ggugcucuaa       660
   uccucucuag ggcuuucaag ucuguacuuc cuagaauguc auuuuguugu ggauugcugc       720
   ucaugacccu ggaggcacac agccagcaca gugaagaggc agaauuccaa gguauuaugc       780
   uaucaccauc cacacauaag uaucuggggu ccugcaaugu ucccacaugu auccugaaug       840
   ucccccuguu gaguccaaua aacccuuugu ucuccc                                 876
<210> 260
   <211> 6929
   <212> RNA
   <213> Homo sapiens
<220>
   <221> misc_feature
   <222> (2080)..(2080)
   <223> n is a, c, g, or u
<220>
   <221> misc_feature
   <222> (2082)..(2082)
   <223> n is a, c, g, or u
<220>
   <221> misc_feature
   <222> (4019)..(4019)
   <223> n is a, c, g, or u
<220>
   <221> misc_feature
   <222> (4119)..(4119)
   <223> n is a, c, g, or u
<220>
   <221> misc_feature
   <222> (5039)..(5039)
   <223> n is a, c, g, or u
<220>
   <221> misc_feature
   <222> (5048)..(5048)
   <223> n is a, c, g, or u
<220>
   <221> misc_feature
   <222> (5054)..(5054)
   <223> n is a, c, g, or u
<220>
   <221> misc_feature
   <222> (5079)..(5079)
   <223> n is a, c, g, or u
<220>
   <221> misc_feature
   <222> (5131)..(5131)
   <223> n is a, c, g, or u
<400> 260
   ggaagaaaga gagggaggga aaagagaagg aaggaguaga ugugagaggg uggugcugag       60
   ggugggaagg caagagcgcg aggccuggcc cggaagcuag gugaguucgg cauccgagcu      120
   gagagacccc agccuaagac gccugcgcug caacccagcc ugaguaucug gucuccgucc      180
   cugaugggau ucucgucuaa accgucuugg agccugcagc gauccagucu cuggcccucg      240
   accagguuca uugcagcuuu cuagaggucc ccagaagcag cugcuggcga gcccgcuucu      300
   gcaggaacca auggugagca gggcaaccug gagaggggcg cuauucugag gauucgaggu      360
   gcacccguag uagaagcugg ggauggggcu caggcuguaa ccgaggcaaa aguuggccua      420
   uuccuccuuc cuucuccaac aguguuggag gugggaugau ggaggcuaaa aggcaccucc      480
   auauauguua cugcgucuau caaccuacuu uagggaggug cgggccagga gaggcgggaa      540
   ggagagaagg ccuuggaaga gaggucauug ggaagaacug ugggguuugg uggguuugcu 600
   uccacuuaga cuauaagagu gggagaggag ggagucaacu cuaaguuuca acaccagugg 660
   gggacugagg acugcuucau uaggagagag aaccuagcca gagcuagcuu ugcaaaagag 720
   gcuguagucc ugcuuugcuc uaaagcgcga cccgggauag agaggcuucc uugagcgggg 780
   ugucaccuaa ucuugucccc aacgcacccc cucccagccc cugagagcua gcgaacugua 840
   gguacacaac ucgcucccau cuccaggagc uauuuucuua gacaugggca cccaugauuc 900
   ugccuucugg uacucucccc ucccugggaa agggguguaa gguuccgacg gaaccguggc 960
   caggaugccg aaaggcuacc ugugcggguc uucugccaug cugugucugu gcggacaugc 1020
   cagcagggcu aaugaggagc uugcgauacu ccaaaggguu cgggaauugc gggguccuua 1080
   cacgcagugg aguugggccc cuuuuacuca gaagguuucc gccacggcuu ugguugauag 1140
   uuuuuuuagu auccugguuu augaacugaa gguuuuguga gauguugaau cacuagcagg 1200
   gucauauuug gcaaaccgag gcuacuauua aauuuugguu uuagaagaag auucugggga 1260
   gaaagugaag gguaacugcc uccaggagcu guaucaaccc cauuaagaaa aaaaaaaaua 1320
   ccaggagaug aaaauuuacu uugaucugua uuuuuuaauu aaaaaaaauc agggaagaaa 1380
   ggagugauua gaaagggauc cugagcgucg gcgguuccac ggugcccucg cuccgcgugc 1440
   gccagucgcu agcauaucgc caucucuuuc ccccuuaaaa gcaaauaaac aaaucaacaa 1500
   uaagcccuuu gcccuuucca gcgcuuuccc aguuauuccc agcggcgacg cgugucgggg 1560
   aauagagaaa ucgucucaga aagcugcgcu gaugguggug agagcggacu gucgcucagg 1620
   ggcgcccgcg gucucugcac ccagggcagc agugugggau ggcgcugggc agccaccgcc 1680
   gccaggaagg acgugacucu ccauccuuua cacuucuuuc ucaaagguuu cccgaaagug 1740
   ccccccgccu cgaaaacugg ggccggugcg ggggggggga gagguuaggu ugaaaaccag 1800
   cuggacacgu cgaguuccua agugaggcaa agaggcgggg uggagcgggc ucuggagcgg 1860
   gggaguccug ggacucgguc cucggaugga ccccgugcaa agaccuguug gaacaagagu 1920
   ugcgcuuccg agguuagaac aggccaggca ucuuaggaua gucaggucac cccccccccc 1980
   aaccccaccc gaguuguguu ggugaauuuc uuggaggaau cuuagccgcg auucuguagc 2040
   uggugcaaaa ggaggaaagg ggugggggaa ggaaguggcn gngcgggggu ggcggugggg 2100
   guggaggugg uuuaaaaagu aagccaagcc agagggagag gucgagugca ggccgaaagc 2160
   uguucucggg uuuguagacg cuugggaucg cgcuuggggu cuccuuucgu gccggguagg 2220
   aguuguaaag ccuuugcaac ucugagaucg uaaaaaaaau gugaugcgcu cuuucuuugg 2280
   cgacgccugu uuuggaaucu guccggaguu agaagcucag acguccaccc cccacccccc 2340
   gcccaccccc ucugccuuga auggcaccgc cgaccgguuu cugaaggauc ugcuuggcug 2400
   gagcggacgc ugagguuggc agacacggug uggggacucu ggcggggcua cuagacagua 2460
   cuucagaagc cgcuccuucu aacuuuccca caccgcucaa accccgacac ccccgcggcg 2520
   gacugaguug gcgacggggu cagagucuuc uggcugaaag uuagauccgc uaggggucgg 2580
   cugccugucg cuagaagcau uauuuggccu cucggagacc cguguggagg aagugcugga 2640
   gugugcgagu guguuugcgu gugugugugu gugugugugu gugugugugu gugugugugu 2700
   gugcgcgcgc ccuuggaggg ucccuaugcg cuuuccuuuu cauggaacgc ugucgugagg 2760
   cuuugguaaa cugucuuuuc gguuccucuc ucggcugcac uuaagcuuug ucggcgcugu 2820
   aaagagacgc gucuucaagu gcacccugau ccucaggcuu cagauaaccc guccccgaac 2880
   cuggccagau gcauugcacu gcgcgccgca gguagagacg ugccccacgu ccccugcgug 2940
   cagcgacuac gaccgagagc cgcgccagug uggugucccg ccgagaguuc cucagagcag 3000
   gcggggacaa cucccagacg gcuggggcuc cagcugcggg cgcggagguu ggccucgcuc 3060
   gcaggggcug gacccagccg gggugggagg auggaggagg ggcgggcggg cucuucggug 3120
   aguggggcgg ggccucuggg uccacgugac uccuaggggc uggaagaaaa acagagccug 3180
   ucugcuccag agucucauua uaucaaauau cauuuuagga gccauuccgu agugccauuc 3240
   ggagcgacgc acugccgcag cuucucugag ccuuuccagc aaguuuguuc aagauuggcu 3300
   cccaagaauc auggacuguu auuaugccuu guuuucuguc agugaguaga caccucuucu 3360
   uucccuucuu gggauuucac ucuguccucc caucccugac cacugucugu cccucccguc 3420
   ggacuuccau uucagugccc cgcgcccuac ucucaggcag cgcuaugguu cucuuucugg 3480
   ucccugcaag gccagacacu cgaaauguac gggcuccuuu uaaagcgcuc ccacuguuuu 3540
   cucugauccg cugcguugca agaaagaggg agcgcgaggg accaaauaga ugaaaggucc 3600
   ucagguuggg gcugucccuu gaagggcuaa ccacucccuu accagucccg auauauccac 3660
   uagccuggga aggccaguuc cuugccucau aaaaaaaaaa aaaaaaacaa aaaacaaaca 3720
   gucguuuggg aacaagacuc uuuagugagc auuuucaacg cagcgaccac aaugaaauaa 3780
   aucacaaagu cacuggggca gccccuugac uccuuuuccc agucacugga ccuugcugcc 3840
   cgguccaagc ccugccggca cagcucuguu cuccccuccu ccuguucuua accagcugga 3900
   aguuguggaa auugggcugg agggcggagg aagggcgggg gugggggggu ggagaaggug 3960
   gggggggggg aggcugaagg uccgaaguga agagcgaugg cauuuuaauu cucccuccnc 4020
   cuccccccuu uaccuccuca auguuaacug uuuauccuug aagaagccac gcugagauca 4080
   uggcucagau agccguuggg acaggaugga ggcuaucuna uuugggguua uuugagugua 4140
   aacaaguuag accaaguaau uacagggcga uucuuacuuu cgggccgugc auggcugcag 4200
   cuggugugug uguguguagg gugugaggga gaaaacacaa acuugaucuu ucggaccugu 4260
   uuuacaucuu gaccgucggu ugcuaccccu auaugcauau gcagagacau cucuauuucu 4320
   cgcuauugau cgguguuuau uuauucuuua accuuccacc ccaacccccu ccccagagac 4380
   accaugauuc cugguaaccg aaugcugaug gucguuuuau uaugccaagu ccugcuagga 4440
   ggcgcgagcc augcuaguuu gauaccugag accgggaaga aaaaagucgc cgagauucag 4500
   ggccacgcgg gaggacgccg cucagggcag agccaugagc uccugcggga cuucgaggcg 4560
   acacuucuac agauguuugg gcugcgccgc cguccgcagc cuagcaagag cgccgucauu 4620
   ccggauuaca ugagggaucu uuaccggcuc cagucugggg aggaggagga ggaagagcag 4680
   agccagggaa ccgggcuuga guacccggag cgucccgcca gccgagccaa cacugugagg 4740
   aguuuccauc acgaagguca guuucugcuc uuaguccugg cgguguaggg ugggguagag 4800
   crccggggca gagggugggg ggugggcagc uggcagggca agcugaaggg guuguggaag 4860
   cccccgggga agaagaguuc auguuacauc aaagcuccga guccuggaga cuguggaaca 4920
   gggccucuua ccuucaacuu uccagagcug ccucugaggg uacuuucugg agaccaagua 4980
   guggugguga ugggggaggg gguuacuuug ggagaagcgg acugacacca cucagacunc 5040
   ugcuaccncc cagngggugu ucuuuagcua uaccaaagnc agggauucug cccguuuugu 5100
   uccaaagcac cuacugaauu uaauauuaca ncuguguguu ugucagguuu aucaauaggg 5160
   gccuuguaau acgaucugaa uguuuccuag cggauguuuc uuuuccaaag uaaaucugag 5220
   uuauuaaucc uccagcauca uuacuguguu ggaauuuauu uucccuucug uaacaugauc 5280
   aacaaggcgu gcucuguguu ucuaggaucg cuggggaaau guuugguaac auacucaaaa 5340
   guggagaggg agagagggug gccccucuuu uucuuuacaa ccacuuguaa agaaaacugu 5400
   acacaaagcc aagagggggc uuuaaaaggg gaguccaagg gugguggagu aaaagaguug 5460
   acacauggaa auuauuaggc auauaaagga gguugggaga uacuuucugu cuuugguguu 5520
   ugacaaaugu gagcuaaguu uugcugguuu gcuagcugcu ccacaacucu gcuccuucaa 5580
   auuaaaaggc acaguaauuu ccuccccuua gguuucuacu auauaagcag aauucaacca 5640
   auucugcuau uuuuuguuuu uguuucuugu uuuuguuuug uuugguuuuu uuuuuuuuuu 5700
   uuuuuuuuuu gucucagaaa agcucauggg ccuuuucuuu uccccuuuca acugugccua 5760
   gaacaucugg agaacauccc agggaccagu gagagcucug cuuuucguuu ccucuucaac 5820
   cucagcagca ucccagaaaa ugaggugauc uccucggcag agcuccggcu cuuucgggag 5880
   cagguggacc agggcccuga cugggaacag ggcuuccacc guauaaacau uuaugagguu 5940
   augaagcccc cagcagaaau gguuccugga caccucauca cacgacuacu ggacaccaga 6000
   cuaguccauc acaaugugac acggugggaa acuuucgaug ugagcccugc aguccuucgc 6060
   uggacccggg aaaagcaacc caauuauggg cuggccauug aggugacuca ccuccaccag 6120
   acacggaccc accagggcca gcaugucaga aucagccgau cguuaccuca agggagugga 6180
   gauugggccc aacuccgccc ccuccugguc acuuuuggcc augauggccg gggccauacc 6240
   uugacccgca ggagggccaa acguaguccc aagcaucacc cacagcgguc caggaagaag 6300
   aauaagaacu gccgucgcca uucacuauac guggacuuca gugacguggg cuggaaugau 6360
   uggauugugg ccccacccgg cuaccaggcc uucuacugcc auggggacug ucccuuucca 6420
   cuggcugauc accucaacuc aaccaaccau gccauugugc agacccuagu caacucuguu 6480
   aauucuagua ucccuaaggc cuguuguguc cccacugaac ugagugccau uuccauguug 6540
   uaccuggaug aguaugacaa gguggguuug aaaaauuauc aggagauggu gguagagggg 6600
   uguggaugcc gcugagauca gacaguccgg agggcggaca cacacacaca cacacacaca 6660
   cacacacaca cacacacaca cacguuccca uucaaccacc uacacauacc acacaaacug 6720
   cuucccuaua gcuggacuuu uaucuuaaaa aaaaaaaaaa gaaagaaaga aagaaagaaa 6780
   gaaaaaaaau gaaagacaga aaagaaaaaa aaaacccuaa acaacucacc uugaccuuau 6840
   uuaugacuuu acgugcaaau guuuugacca uauugaucau auuuugacaa auauauuuau 6900
   aacuacauau uaaaagaaaa uaaaaugag 6929
<210> 261
   <211> 277
   <212> RNA
   <213> Homo sapiens
<400> 261
   gaaucccacu uacaugcgag caucucuaua uacauccuca aucuaucccc ucgaauccac 60
   ggacuuauca gucaaucaau acuacaguca aagaauuuuu uucuacguua uccuggagca 120
   uugcgcuggc accucuuuuu cuaaucaucu ugguuguggg gccaauaugg augcgcagac 180
   ggguaaaacg cagggcugga aagacauaug gacugaccaa gcuacggacu gacaaccagg 240
   acuucccuuc cagcccaaac uaaauaaagg aaaugaa 277
<210> 262
   <211> 2810
   <212> RNA
   <213> Homo sapiens
<400> 262
   gaauucaugu cuuacgguca aggcagaggg cccagcgcca cugcagccgc gccaccuccc      60
   agggccgggc cagcccaggc guccgcgcuc ucggggugga cuccccccgc ugcgcgcuca      120
   agccggcgau ggcuccucuc ggauaccucu uagugcucug cagccugaag caggcucugg      180
   gcagcuaccc gaucuggugg uccuuggcug ugggacccca guacuccucu cugagcacuc      240
   agcccauucu cugugccagc aucccaggcc ugguaccgaa gcagcugcgc uucugcagga      300
   acuacgugga gaucaugccc agcguggcug agggugucaa agcgggcauc caggagugcc      360
   agcaccaguu ccgaggccgg cguuggaacu gcaccaccgu cagcaacagc cuggccaucu      420
   uuggcccugu ucuggacaaa gccacccggg agucagccuu uguccaugcc aucgccuccg 480
   cuggaguagc uuucgcagug acacgcuccu gugcagaggg aucagcugcu aucugugggu 540
   gcagcagccg ccuccagggc uccccaggcg agggcuggaa guggggcggc uguagugagg 600
   acauugaauu uggaggaaug gucucucggg aguuugccga ugccagggag aaccggccgg 660
   augcccgcuc ugccaugaac cgucacaaca augaggcugg gcgccaggcc aucgccaguc 720
   acaugcaccu caagugcaaa ugccacgggc uaucuggcag cugugaagug aagaccugcu 780
   gguggucgca gccggacuuc cgcaccaucg gggauuuccu caaggacaag uaugacagug 840
   ccucggagau ggugguagag aaacaccgag agucucgugg cuggguggag acccugaggc 900
   cacguuacac guacuucaag gugccgacag aacgcgaccu ggucuacuac gaggccucac 960
   ccaacuucug cgaaccuaac cccgaaaccg gcuccuucgg gacgcgugac cgcaccugca 1020
   augugagcuc gcauggcaua gaugggugcg accuguugug cugcgggcgc gggcauaacg 1080
   cgcgcacuga gcgacggagg gagaaaugcc acuguguuuu ccauuggugc ugcuacguca 1140
   gcugccagga gugcacacgu gucuaugacg ugcacaccug caaguaggag agcuccuaac 1200
   acgggagcag gguucauucc gaggggcaag guuccuaccu gggggcgggg uuccuacuug 1260
   gaggggucuc uuacuugggg acucgguucu uacuugaggg cggagauccu accugugagg 1320
   gucucauacc uaaggacccg guuucugccu ucagccuggg cuccuauuug ggaucugggu 1380
   uccuuuuuag gggagaagcu ccugucuggg auacggguuu cugcccgagg guggggcucc 1440
   acuuggggau ggaauuccaa uuugggccgg aaguccuacc ucaauggcuu ggacuccucu 1500
   cuugacccga cagggcucaa auggagacag guaagcuacu cccucaacua ggugggguuc 1560
   gugcggaugg gugggagggg agagauuagg gucccuccuc ccagaggcac ugcucuaucu 1620
   agauacauga gagggugcuu cagggugggc ccuauuuggg cuugaggauc ccgugggggc 1680
   ggggcuucac cccgacuggg uggaacuuuu ggagaccccc uuccacuggg gcaaggcuuc 1740
   acugaagacu caugggaugg agcuccacgg aaggaggagu uccugagcga gccugggcuc 1800
   ugagcaggcc auccagcucc caucuggccc cuuuccaguc cugguguaag guucaaccug 1860
   caagccucau cugcgcagag caggaucucc uggcagaaug aggcauggag aagaacucag 1920
   gggugauacc aagaccuaac aaaccccgug ccuggguacc ucuuuuaaag cucugcaccc 1980
   cuucuucaag ggcuuuccua gucuccuugg cagagcuuuc cugaggaaga uuugcagucc 2040
   cccagaguuc aagugaacac ccauagaaca gaacagacuc uauccugagu agagaggguu 2100
   cucuaggaau cucuaugggg acugcuagga aggauccugg gcaugacagc cucguaugau 2160
   agccugcauc cgcucugaca cuuaauacuc agaucucccg ggaaacccag cucauccggu 2220
   ccgugauguc caugccccaa augccucaga gauguugccu cacuuugagu uguaugaacu 2280
   ucggagacau ggggacacag ucaagccgca gagccagggu uguuucagga cccaucugau 2340
   uccccagagc cugcuguuga ggcaaugguc accagauccg uuggccacca cccugucccg 2400
   agcuucucua gugucugucu ggccuggaag ugaggugcua cauacagccc aucugccaca 2460
   agagcuuccu gauugguacc acugugaacc gucccucccc cuccagacag gggaggggau 2520
   guggccauac aggagugugc ccggagagcg cggaaagagg aagagaggcu gcacacgcgu 2580
   ggugacugac ugucuucugc cuggaacuuu gcguucgcgc uuguaacuuu auuuucaaug 2640
   cugcuauauc cacccaccac uggauuuaga caaaagugau uuucuuuuuu uuuuuuucuu 2700
   uucuuucuau gaaagaaauu auuuuaguuu auaguauguu uguuucaaau aauggggaaa 2760
   guaaaaagag agaaaaaaaa aaaaaaaaaa aaaaaaaaaa aaaaaaaaaa 2810
<210> 263
   <211> 2948
   <212> RNA
   <213> Homo sapiens
<400> 263
   cgcaccggag guccacacug ccgcguggac uccagcgaug gcgcugcgcu aggcuagcgg 60
   gugccaggau cccucucugc guccugugcg cgggaagagc ucuggagaac cagaguugca 120
   uucuggaguu gaagacucau ggcacagacu guuagagaau gcucauuggc acuucuuuuu 180
   uuguucaugu ggcugcugau uaaagcaaau auagaugugu gcaagcuugg cacugugacc 240
   guccagccug ccccugugau uccucuuggg ucagcugcca auauuuccug cuccuugaau 300
   cccaagcaag gcuguucaca uuaucccagu ucuaacgaau uaauccucuu aaaguuuguc 360
   aaugaugucc uuguugaaaa ucuccauggc aagaaagucc augaccacac uggucacucc 420
   uccacuuuuc aagucaccaa ccugucccuu gguaugaccu uguuugucug caagcuaaac 480
   uguagcaacu cucaaaagaa gccaccaguc ccaguaugug ggguggagau cucaguuggu 540
   guugcuccag agccaccuca aaacauauca uguguccagg aaggagaaaa uggaacugug 600
   gccuguuccu ggaacucugg aaaaguuacu uaucugaaaa ccaauuacac uuuacaguua 660
   aguggaccaa acaaucugac cugucagaaa caauguuuuu cugacaaucg ucagaauugc 720
   aaucgccugg aucuugggau caaucuaagc ccugauuuag cugaauccag guucauaguc 780
   cguguuacug ccaucaacga ucuuggaaau ucuucuucac uuccgcauac guucacguuc 840
   uuggacauag ugaucccucu uccuccgugg gacaucagaa ucaacuuucu aaaugcuucu 900
   gggagcagag guacacugca gugggaagau gaggggcaag ugguacucaa ucaacucaga 960
   uaucagccuc uuaacagcac guccuggaac auggucaaug cuacaaaugc caaaggaaaa 1020
   uaugaccugc gagaucugag accguuuaca gaauaugaau uucaaaucuc cucuaagcua 1080
   caucucucug gaggaaguug gaguaauugg agugagucac ugagaacacg aacaccagag 1140
   gaagagccug uugggauauu agacaucugg uacaugaaac aagacaucga cuaugacaga 1200
   cagcagaucu cucuuuucug gaagagucug aauccaucag aggcaagggg gaagauccuc 1260
   cacuaucagg ugacguuaca agaggugaca aagaaaacaa cacugcagaa uacuacaaga 1320
   cacaccuccu ggaccagggu caucccccga acuggggcuu ggacggcauc agugucugca 1380
   gccaacucaa aaggcgcuuc ugcacccacu cacauuaaca uaguggaccu auguggcacu 1440
   ggguugcugg cuccucacca ggucucugca aagucggaga acauggacaa cauucuagug 1500
   accuggcagc cuccuaagaa agcugauucu gcuguucggg aguacauagu ggaauggaga 1560
   gcucuccaac cagggagcau cacgaaguuu cccccacacu ggcugcggau ccccccggac 1620
   aacaugucug cucugauuuc agagaacaua aagcccuaua ucuguuauga aaucagggug 1680
   caugcacugu cagagagcca aggagggugc agcuccaucc ggggugacuc caagcacaaa 1740
   gcaccaguga guggcccuca cauuacugcc aucacagaga aaaaggaacg ccuuuucauu 1800
   uccuggaccc acauuccauu cccggagcaa aggggcugca uccuccauua cagaauauac 1860
   uggaaagaac gagacucgac agcacaaccu gagcucugcg aaauucagua ccgacgcucu 1920
   caaaacucac auccaauaag cagccuacag cccaggguga cauauguccu auggaugaca 1980
   gcugugacag cugcugguga aaguccccaa ggaaaugaaa gggaauuuug uccacagggc 2040
   aaagccaacu ggaaagcauu cgugauauca agcauuugca ucgcuaucau cacggugggc 2100
   acguucucaa uucguuacuu ccggcaaaag gcauuuacuc uccugucuac ucucaaaccu 2160
   caaugguaua gcagaaccau uccagaucca gcaaacagca cuuggguaaa gaaguauccc 2220
   auucuggagg agaagaucca gcuaccuacg gauaaucucc ugauggcaug gcccacuccu 2280
   gaagagccug agccccugau cauccaugaa guccucuacc acaugauccc aguugucaga 2340
   caaccauauu acuucaaaag aggccaagga uuccaaggcu acucuaccuc caagcaagau 2400
   gcaauguaua uugccaaucc acaagcuaca ggaacucuca cagcugagac cagacaacua 2460
   gugaaccuau acaaggugcu agaaagcaga gacccugacu caaaacuggc caaccugacc 2520
   agccccuuga cagucacccc agugaacuac cuuccuagcc augaaggcua uuuacccucc 2580
   aacauagaag aucugucacc acaugaggcu gacccaacug auucuuuuga ccuggagcau 2640
   caacauauuu cucuuuccau uuuugcauca aguucucucc gcccacucau cuucgguggu 2700
   gagcggcuga cucuagaucg guuaaagaug ggcuaugacu cccucaugag uaaugaggcu 2760
   ugauacuaga aagccaacgu accucauuuu aucugcccag uuccuacucc aaaggucugu 2820
   gacagugaag acaagccagc ugucucugga uaaaguuagc uucaccauag guacuuaagu 2880
   cuuauggaua agguggcaau acaccaacac ugauaucaua uagaaaggac cccaagauag 2940
   ucaugcuc 2948
<210> 264
   <211> 2487
   <212> RNA
   <213> Homo sapiens
<400> 264
   ggcacgaguu cacccucugc augcguuccc cuccccccuc uccagcaaac acggcgcgcc 60
   aguccaaagc ggacucagug gccucgggga cgggagcaug ccaccuccug uggugacguc 120
   acuuggggua gaacccuuag acacuacaug gggggggggg guacagaacu cccgagccag 180
   gacagucacu cacucuucag gcgguggggc ugggccagac aguaccgccc ccaccgcgcc 240
   cgccucgcac acccucggaa gcgcaggcuc gcagcgcggc gcuggggugg gggguugcgc 300
   cccagaacuu cggccuccag uccccagccc gcugcaccuc cuuacccucu agaggccccc 360
   ucccccuuac ccucuagagg caccaggagu ugucgcaagg ggcccuuggg aaauucccug 420
   gaccccugug ccaggaggug cccgguucgc ccgcucccca uccacccccc cgagggcggu 480
   gcccgggggc gcugccccau ggagcgggga ggcgggcgcc gucugcugcg ggagcuguga 540
   ccugaguagg agcugugugu cgcagccgcc ccaccccugc cgaucaugcg ccggcgaccc 600
   ugguucgcca gucccacugg gcugugagcc ccccacuccu ggccugucac ggcccgcgcg 660
   ccaugggcag cgcccacccu cgccccuggc ugcggcuccc acaagggccc cagccgcggc 720
   cugaguucug ggcgcuccug uucuuccuac ugcugcuggc ugccgcugug cccaggucag 780
   cacccaacga cauccugggc cuccgccuac ccccagagcc cgugcucaac gccaacacag 840
   ugugccugac auugcccggc cugagccggc ggcagaugga ggugugugug cgucacccug 900
   acguggccgc cucugcuauc cagggcaucc agaucgccau ccaugagugc cagcaucagu 960
   uccgggacca gcgcuggaac ugcuccagcc uggagacucg gaacaaaguc cccuacgaga 1020
   gccccaucuu cagccgaggu uuucgagaga gugcuuucgc cuacgccaua gcagcugccg 1080
   ggguggugca cgcagugucc aacgcgugcg cucuggguaa acugaaggcu ugcgguugcg 1140
   acgccuccag acguggggac gaagaagcuu uccgucggaa gcugcaccgc uugcagcugg 1200
   acgcgcugca gcgcggaaag ggcuugagcc acgggguccc ugaacacccg gccauacuuc 1260
   cugccagccc aggucugcag gacuccuggg aguggggugg cugcaguccg gaugugggcu 1320
   ucggagaacg cuucucuaag gacuuucugg acucccgaga gccucacaga gacauccaug 1380
   cucgaaugag acuccacaac aaccgugugg gccggcaggc ggugauggag aacaugcggc    1440
   guaagugcaa augccacggc accucaggca gcugccagcu caagaccugc uggcagguga    1500
   cgccugaguu ccgcacagua ggggcgcugc ugcgcaaccg cuuccaccgc gccacgcuca 1560
   uccggccgca caaccgcaac gguggccagc uggagcccgg ccccgcggga gcacccucgc 1620
   cagcaccggg cacuccaggg cugcgccgca gggccagcca cuccgaccug gucuacuuug 1680
   agaaaucucc cgacuucugu gagcgcgagc cgcgccugga cucggcaggc acugugggcc 1740
   gccugugcaa uaagagcagc acgggucccg auggcugcgg cagcaugugc uguggccgcg 1800
   gccacaacau ucugcgccag acgcgcagcg agcgcugcca cugccgguuc cacuggugcu 1860
   gcuucguggu cugcgaagaa ugccgcauca ccgagugggu cagcgucugc aagugagcag 1920
   acccaagcuc cucugggucu caagaauggu uguccucuug gugccuggcu ucugccgcua 1980
   gcggaucuga gccaggcagc aagcagcagc cuuggcuccu gagagaggug guuggcucuu 2040
   acagccccga gggucuacaa ucaccagaca guccagaucu gauugacauu ccuccgcuca      2100
   ccucuguagg uuccccucuu ucuguuccua gcucagacag cuggggguga uaguggagac      2160
   uguuccacac ccuaggacag gucaccaaag cagcccagcc uggcaugccu accuccuguc 2220
   aucucuucuu cccuucccca ggagugauag gcaaugcacu gaagcugaug ggcaccgggg 2280
   aagaaaacua aaaggcagaa auggccguca ucgggcugaa gugacucuaa gggcuccaga 2340
   ccucugcucc ugucuuucac uuaacagaua uuuauuuuug cgcucucuuu gagacacucu 2400
   cuggggaaaa agaagcuccg gagucuacag gcugauuaag ggacauggac aauaaaccag 2460
   uaaacacaca aaaaaaaaaa aaaaaaa 2487
<210> 265
   <211> 2093
   <212> RNA
   <213> Homo sapiens
<400> 265
   gaauuccggg ccgcuugcac uuggcgacua gucugcggcg gacgugacgc caaggccacg        60
   ggcagcgcgg guccccuguc agaggugucc cucgcgcagg aaugggcccg caggcggcag       120
   cuggcaggau gauucugcug gugguccuga ugcugucugc gaaggucggg aguggagcuu       180
   ugacgagcac cgaggauccu gagccucccu cggugccugu accgacgaau guucuaauua       240
   agucuuauaa cuugaacccu gucguaugcu gggaauacca gaacauguca cagacuccua       300
   uuuuuacugu acagguaaag guguauucgg guuccuggac ugauuccugc accaacauuu       360
   cugaucauug uuguaauauc uaugaacaaa uuauguaucc ugauguaucu gccugggcca       420
   gaguuaaagc uaagguugga caaaaagaau cugacuaugc acggucaaaa gaguuccuua       480
   ugugccuaaa gggaaagguc gggcccccug gccuggagau caggaggaag aaggaagaac       540
   agcucuccgu ccucguauuu cacccugaag ucguugugaa uggagagagc cagggaacca       600
   uguuugguga cgggagcacc uguuacacau ucgacuauac uguguaugug gagcauaacc       660
   ggagugggga gauccuacau acgaaacaua cggucgaaaa agaagagugu aaugagacuc       720
   ugugugaguu aaacaucuca guauccacac uggauuccag auauuguauu ucaguagacg       780
   gaaucucauc uuucuggcaa guuagaacag aaaaaucgaa agacgucugu aucccuccuu       840
   uccaugauga cagaaaggau ucaauuugga uucugguggu ugcuccucuu accgucuuua        900
   caguaguuau ccugguauuu gcguauuggu auacuaagaa gaauucauuc aagagaaaaa    960
   gcauaauguu accuaagucc uugcucucug ugguaaaaag ugccacguua gagacaaaac   1020
   cugaaucgaa guauucacuu gucacaccgc accagccagc uguccuagag agugagacgg   1080
   ugaucuguga agagccccug uccacaguga cagcuccaga cagccccgaa gcagcagaac   1140
   aggaagaacu uucaaaagaa acaaaggcuc uggaggcugg aggaagcacg ucugccauga   1200
   ccccagacag cccuccaacu ccgacacaaa gacgcagcuu uucccuguua aguaguaacc   1260
   agucaggccc uuguagccuc accgccuauc acucccgaaa cggcucugac aguggccucg   1320
   ugggaucggg cagcuccaua ucggacuugg aaucucuccc aaacaacaac ucagaaacaa   1380
   agauggcaga gcacgacccu ccacccguga gaaaggcccc cauggccucc gguuaugaca   1440
   aaccgcacau guugguggac gugcuugugg auguuggggg gaaggagucu cucauggggu   1500
   auagacucac aggagaggcc caggagcugu ccuaaggucu cccgaggccu gcugguggua   1560
   aagaaacuga ccuuuuaggc aguuuuucug cauugauuuc augaaagaag cuauacauua   1620
   gcuaauacua accacauaga auaucagacu uagauacgug aauaaggauc cugugggcac   1680
   ugcugggucc acucugcaaa ugccaagacu aucaaaggaa cguauugucg cuucuggcuc   1740
   cuucccaggu gggcuagcau cugugaguuu gccucggcua gccuugcuuc cuacagccgc   1800
   cacugcuccu ccacccugau caucucacag gacagggugg accggguuuu uuuuuuuuuu   1860
   ucacacaccu uuguauaugu aaguucaugu auauaauaug uuuacauguu ucacuuugaa   1920
   cugaaagcua cucaaagcca gccguaaguc uaugguagaa ugugauggaa cauguuggug   1980
   gaagcuugua caauagaaca cauugguggg agcuuguaca uacuuuuuua uggagcauua   2040
   cuuacgauuu uuuaaguaaa auguuuugaa accaaaaaaa aaaaaaggaa uuc            2093
<210> 266
   <211> 756
   <212> RNA
   <213> Homo sapiens
<400> 266
   augagacucc acagccucau ccugcucucc uuccuucucc uggcuacuca ggcguucuca 60
   gaaaagguca gaaagagagc caagaacgca ccacacagca cagcggagga ggggguagag 120
   gguucagcuc ccucguuagg gaaggcccag aauaagcaga gaagcaggac aucuaaaucu 180
   cugacgcaug gcaaguuugu caccaaagac caagccacau gcagaugggc ugugacugag 240
   gaggagcagg gcaucagccu gaagguccag ugcacacaag ccgaucagga guuuucuugu 300
   guuuuugcug gugacccaac ugacugccuu aaacacgaca aagaccagau cuacuggaaa 360
   cagguugccc gcacgcugcg caaacagaaa aauaucugca ggaacgccaa gagugucuug 420
   aagaccagag ugugcagaaa gagauuucca gagucuaacc ucaagcuggu gaaccccaac 480
   gcacguggaa acacgaagcc caggaaggag aaagcagagg ucuccgcaag ggagcacaac 540
   aagguccaag aagcugucuc cacggagcca aacaggguca aagaagacau cacacucaau 600
   ccagcugcga cccagaccau ggccauuaga gauccagagu gucuagagga uccagaugug 660
   cucaaccaga ggaagaccgc ccuggaguuc uguggggaau cuuggagcuc cauuugcaca 720
   uucuuccuca acauguuaca ggcgacauca ugcuaa 756
<210> 267
   <211> 181
   <212> RNA
   <213> Homo sapiens
<400> 267
   aauucgucga caugcgccgu uccagugcau ggugugccaa cgcagcuucu cccgcuccga 60
   ccaccucgcg cugcacauga agcgccacca gaacugagcg agcgagcgcu gccccacccg 120
   ccugacgccu ugcaguccgc ucugccaucc uuuaaaccgc agaccuaacu ucauaaaaag 180
   a 181
<210> 268
   <211> 2056
   <212> RNA
   <213> Homo sapiens
<220>
   <221> misc_feature
   <222> (1942)..(1943)
   <223> n is a, c, g, or u
<400> 268
   gcuaaacuau cccgcaaaga uuuuucuuuc cucccuaaac ccuccuuuuu gcucuccuuu 60
   ucuauacccu uaacugcaaa caaaccauua aacgaccccu cuccugggcc uccgacggca 120
   ggaguccgcg gaccucccag gccgacagcc cucccucuac ccgcgagguu ccgggccggg 180
   cgagagggcg cgagcacagc cgaggacaug gaggugacug cggaccagcc gcgcugggug 240
   agccaccauc accccgcggu ccucaacggu cagcacccag acacgcacca cccgggccuc 300
   ggccauucgu acauggaagc ucaguauccg cugacggaag agguggacgu acuuuuuaac 360
   aucgaugguc aaggcaacca cgucccgucc uacuacggaa acuccgucag ggcuacggug 420
   cagagguauc cuccgaccca ccacgggagc cagguaugcc gcccgccucu gcugcacgga 480
   ucucugcccu ggcuggaugg cggcaaagcc cugagcagcc accacaccgc cucgcccugg 540
   aaccucagcc ccuucuccaa gacguccauc caccacggcu cuccggggcc ucuguccguu 600
   uacccuccgg cuucauccuc uucucuggcg gccggccacu ccaguccuca ucucuucacc 660
   uucccgccca ccccgccgaa agacgucucc ccagacccgu cgcuguccac cccgggaucc 720
   gccgggucgg ccaggcaaga ugagaaagag ugccucaagu aucaggugca gcugccagau 780
   agcaugaagc uggagacguc ucacucucga ggcagcauga ccacccuggg uggggccuca 840
   uccucagccc accaccccau uaccaccuau ccgcccuaug ugcccgagua cagcucugga 900
   cucuucccac ccagcagccu gcugggagga uccccuaccg gguucggaug uaagucgagg      960
   cccaaggcac gauccagcac agaaggcagg gaguguguga acugcggggc aaccucuacc     1020
   ccacuguggc ggcgagaugg uaccgggcac uaccuuugca augccugcgg acucuaccau 1080
   aaaaugaaug ggcagaaccg gccccuuauc aagcccaagc gaaggcuguc ggcagcaagg 1140
   agagcaggga cauccugcgc gaacugucag accaccacca ccacccucug gaggaggaac 1200
   gcuaaugggg acccggucug caaugccugu gggcuguacu acaagcuuca uaauauuaac 1260
   agaccccuga cuaugaagaa agaaggcauc cagacccgaa accggaagau gucuagcaaa 1320
   ucgaaaaagu gcaaaaaggu gcaugacgcg cuggaggacu uccccaagag cagcuccuuc 1380
   aacccggccg cucucuccag acacauguca ucccugagcc acaucucucc cuucagccac 1440
   uccagccaca ugcugaccac accgacgccc augcauccgc ccuccggccu cuccuucgga 1500
   ccucaccacc cuuccagcau ggucaccgcc auggguuaga gaggcagagc ccugcuccac 1560
   augcgugagg agucuccaag ugugcgaaga guuccuccga ccccuucuac uugcguuuuu 1620
   cgcaggagca guaucaugaa gcccgaaagc gacagaucug uguuuuugaa ggcagaaagc 1680
   aaaauguuug cuucuuuuuu caaaggagcu cgagguggug ucugcauucc aaccacugaa 1740
   uccggauccc auuugugaau aagccauuca gacucauauu cccuauuuaa cagggucucu 1800
   agugcuguga aaaaaauauu gcugaacauu gcauauaacu uauauuguaa gaaauacugu 1860
   acauuugagg aagacuuuau uguaccugga uagcuguaag aaaggcauga aggacgccaa 1920
   gaguuuuaag gaauauaggg gnnuuaaagu auggagauac agaagaaacc acuaagucug 1980
   auguccaaau gggcacacug ucaguuuugu uucccuucag uuguuugaug cauuuaaaaa 2040
   aaaaaaaaag aaagaa 2056
<210> 269
   <211> 1508
   <212> RNA
   <213> Homo sapiens
<400> 269
   caaccaccuc cuaccugccu gcccaaagcu ccagggcugg agcacggaga ccugucaggg 60
   auggauuuug cccacaugua ccaaguguac aaguccaggc ggggaauaaa acggagcgaa 120
   gacagcaagg aaacuuacaa acugccgcac cggcugauug agaaaaagag acgugaccgg 180
   auuaacgagu gcauugccca gcugaaggau cuccuacccg aacaucucaa acuuacuacu 240
   uugggucacu uggaaaaagc agugguucug gagcuuacgu ugaagcacgu gaaagcauug 300
   acaaaucuaa uugaucagca gcagcagaaa aucauugccc ugcagagcgg uuuacaagcu 360
   ggugauuugu cgggaagaaa ucucgaggca gggcaagaaa uguucugcuc agguuuccag 420
   acuugugccc gugagguacu ucaguaccug gcgaagcaug agaacacucg ggaccugaaa 480
   ucuucccagc ucgucacuca ucuccaucgu guggucucgg agcugcugca ggguggugcu 540
   uccaggaaac cauuggacuc ggcucccaaa gccgucgacu ugaaagagaa gcccagcuuc 600
   cuagccaagg gaucagaagg cccagggaaa aacugugugc cagucaucca gcggacuuuu 660
   gcucccucgg guggggagca gagcggcagu gacacggaca cagacagugg cuauggaggu 720
   gaauuggaga aaggggacuu gcgcagugaa cagccguacu ucaaaagcga ccauggacgc 780
   agguucgccg ugggagaacg ugucagcaca auuaagcaag aauccgaaga gccccccacc 840
   acaaagagcc gaaugcagcu cucagaagag gaaggccacu ucgcgggcag ugaucugaug 900
   gguuccccau uucuugggcc acacccacau cagccuccuu uuugccuucc cuucuaucuc 960
   aucccaccau cggccacugc cuaccugccu augcuggaga aaugcuggua ccccaccucu 1020
   gugccagugu uauacccagg ccucaacacc ucagcugcag cccucuccag cuucaugaac 1080
   ccagacaaga uaccgacucc cuugcuucug ccccagagac ucccuucucc uuuggcacau 1140
   ucgucccuug acucuucggc cuugcuccag gcuuugaagc agaucccucc uuuaaacuua 1200
   gaaaccaaag acuaaacucu ggagggaucu ccugcugccu ugcuuucuuu ccucccuaau 1260
   uccaaaaacc acgaagguuu cccugagugc agagagauca gcccacccug cagacccaca 1320
   gagaagauuc agagugugug ugagagugag ugagugugcg ugcgugcgug cuuguaugua 1380
   uguuuguaua uguaggacaa uaaguuccuu cugacacaag ggagacacga gaaggauagc 1440
   cugacaucag augacagacu ggaggacugu agcacaucuc ugggcguuuc ccuacccaga 1500
   gaagagcc 1508
<210> 270
   <211> 9471
   <212> RNA
   <213> Homo sapiens
<220>
   <221> misc_feature
   <222> (121)..(121)
   <223> n is a, c, g, or u
<400> 270
   agucaccgac guuguauaac gacggccagu gaauuguaau acgacucacu auagggcgaa        60
   uuggguaccg gccccccccu cgaggucgac gguaucgaua agcuugauau cgaauucucg      120
   nccgcgugcg ggucuggucg gggcggagcg aaggccgcgg guggccgugg ucgguccucc       180
   gcggcuaagg agccgagggc uccgacgcgg gcugcgcccg gugagcggcg gccagagcua       240
   acuugcgcug acuggaccag cugaggagcg gcccggcggg gcgacugcga gcuucaccga       300
   gaggcuucuc cgcccugguc cgcagucccg acggccgggc ggaccauggc guccucggcu    360
   caccugguca ccaucaagcg gagcggcgau gacggcgcac acuucccgcu gagccucagc    420
   uccugccugu uuggaaggag uauugaaugu gacauucgua uccagcugcc uguagugucu    480
   caaagacauu gcccaauugu aguccaagag caagaggcga uauuauauaa uuucaguucu    540
   accaauccaa cucaaguaaa cgggguuacu auagaugagc cugugaggcu gagacaugga    600
   gacauaauaa ccaucauuga ccgcuccuuu agguaugaag auggaaauca ugaggaugga    660
   agcaaaccaa cagaauuucc aggaaagucc cuuggaaagg aaccaucaag gcgagccuca    720
   agagauagcu ucugugcuga cccugauggg gaaggucaag auaccaaagc uucaaaaaug    780
   acugcuucaa gaagaucuuu uguguaugcc aagggccuuu cugcagauag cccugccuca    840
   gauggcucaa agaacagugu uagccaagac ucaucagggc auguagaaca gcacacuggc    900
   agaaacauag uagagcccac uucuggggga ucucuuuuaa gaaguccagg ucuacaggga    960
   gcaguuacag ggaaccgaag ucuucuuccu acacagagcc uuagcaauag caacgaaaag   1020
   gaaucucccu uugagaaacu uuaucaauca augaaggaag aguuggaugu aaaaucccag   1080
   aaaucuugua ggaaaucaga accccaaccu gaccgugcag cagaggaauc gcgggagaca   1140
   cagcuauugg ugucaggcag ggcaagagca aagucuagug gaagcacccc uguuacugca   1200
   gccucuucac ccaaaguagg aaagaucugg acugagagau ggcgcggugg aauggugccu   1260
   guccagacuu ccacagagac agcuaaaaug aagaccccug ugcggcauuc acagcaacuu   1320
   aaggaugaag acucucgugu uacuggcaga cgacauucug ugaaucugga ugaaggugga   1380
   agugcccagg caguccauaa aacagucacu ccugggaaac uggcgacuag aaaccaaacu   1440
   ccgguggagg cuggggaugu uggcagcccc gcugauacac cagaacauuc cucuuccccc   1500
   cagagaagua uuccugcaaa gguagaggcu ccaucugcag agacacaaaa ucggcucucu   1560
   uuaacucagc gccuuguucc aggugaaaag aaaacuccca aggguuccuu cagcaagccu   1620
   gagaaacugg ccacagccgc cgaacagacu ugcucuggcc uaccuggucu uaguuccguu     1680
   gauaucagca acuuugguga uuccauuaac aagagugagg gaaugccuau gaagagaaga     1740
   cguguauccu uugguggaca ucuaagaccu gaauuauuug augaaaacuu gccuccuaau   1800
   acaccacuga aaagaggaga aacgccaacc aagaggaagu cucuuggcac ucacagccca   1860
   gcuguccuca agacaaucau caaggaacgg ccccagucuc cagggaaaca agagucuccu   1920
   gggauaacgc caccgaggac aaaugaucaa agacgcagau caggcaggac uuccagugga   1980
   agcaauuucu uaugugagac agacauuccc aagaaagcag gcaggaagag cgguaaccug   2040
   ccugcgaaga gagcauccau cagccggagu cagcauggca uucuacagau gauuugcucc   2100
   aaaaggcgaa guggagcuuc ugaagccaac uugauuguug caaaaucaug ggcugauguu   2160
   guaaaacuug gcgugaaaca aacacaaacg aaaguugcga aacauguccc uccaaagcag 2220
   acgagcaaga gacaaagaag acccagcacu ccaaagaaac ccacaagcaa ucuucacaau 2280
   caauuuacua caggccaugc aaacucuccc uguaccauug uaguagguag agcgcagauu 2340
   gaaaaaguaa gugugccugc ccgacccuac aaaaugcuga auaacuugau gcuaaaccga 2400
   aaaguggacu ucagugaaga ucugucagga cuaacugaaa uguucaagac uccagugaag 2460
   gagaagcagc agcagaugag ugauacaggc uccguacuuu ccaauucagc gaauuugucu 2520
   gaaagacaau ugcaaguaac uaauucagga gacauaccug agcccaucac cacagagauu 2580
   uugggagaaa aagugcuauc caguacucgg aaugcagcaa agcagcaguc ugauagauau 2640
   ucugcaaguc cuaccuuaag acggcggagc aucaaacaug aaaacacagu gcaaacuccu 2700
   aagaaugucc auaacauuac ugaccuugag aagaagacuc cggucucuga gacagagccc 2760
   cugaagacug caucgagugu gagcaaguua agaagaucua gagagcucag acauacccuu 2820
   guggaaacua ugaaugaaaa aacagaagca guccuugcug agaacaccac agcaagacau 2880
   uuaaggggga cauuucgaga acaaaaagua gaucaacagg ugcaggacaa ugaaaacgcu 2940
   ccucaaagau gcaaggaaag uggugaauua agugaagguu cagaaaagac aucagcuagg 3000
   agaucaagug ccaggaagca gaagccgaca aaagacuuac uaggaaguca gauggucacc 3060
   caaacagcag acuaugcuga ggaacuacuu agucaaggac aaggaaccau acaaaaccua 3120
   gaggaaucca ugcacaugca aaacacauca auaagugagg aucaaggaau uacagaaaag 3180
   aaagugaaca uaauaguaua ugcaaccaaa gagaagcacu cgccaaagac cccuggcaaa 3240
   aaggcacaac cucuagaagg gccagcuggu cucaaggaac acuuugaaac accaaacccc 3300
   aaagauaaac cuauaacgga agacagaacu agaguccuuu gcaaaucacc acaagucaca 3360
   acagagaaua ucacaacaaa cacaaagcca cagacuagca caucugggaa gaaaguagac 3420
   augaaggaag aaagcucugc cuugacaaaa cguauacaua ugccagggga auccaggcau 3480
   aaucccaaaa uuuuaaaacu ugagugugag gauaucaaag cuuugaagca aucugaaaau 3540
   gaaaugcuga ccucaacagu aaauggaagc aagaggacuu uaggaaaauc uaaaaaaaag 3600
   gcucagcccc uggaagaccu gacuuguuuc caggaacucu uuauaucacc aguuccuacu 3660
   aacauaauca aaaaaauucc cagcaaaucu ccacacacac aaccagucag aaccccagcg 3720
   agcacaaaga gacucuccaa gacaggucuc aguaaagugg augugagaca agaaccuuca 3780
   acacuuggga aaagaacgaa gucaccaggc agagccccag gcacaccagc accagugcag 3840
   gaagaaaaug acugcacagc cuacauggaa acuccaaagc agaaacugga gucuauagaa 3900
   aauuuaacag ggcuuaggaa acaguccaga acaccuaaag acaucacugg uuuccaggau 3960
   aguuuccaaa uaccagauca ugcuaauggc ccauuagugg uugucaaaac caaaaaaaug 4020
   uucuuuaauu cuccacaacc agaaagugcc auaacccgaa agagcagaga gagacagucu 4080
   agggcaagua uaaguaaaau agauguuaaa gaagaacuuu uagaaucaga ggaacaccua 4140
   caauuaggag aagguguaga cacauuucag guauccacca acaaagucau uagaucaucu 4200
   aggaaaccug caaagcguaa acuggauuca acagcuggua ugccuaacag caagaggaug 4260
   cgcuguucuu caaaggauaa cacaccaugc cuagaagacc ugaauggcuu ccaagagcuc 4320
   uuccaaaugc caggcuaugc uaaugacucu uugaccacug gaaucucaac aaugcuugcu 4380
   agaucaccac aauuaggacc aguuagaacc caaaucaaca aaaagagucu gcccaagauc 4440
   aucuugagaa aaauggaugu gacagaagaa auuucagguc ucuggaagca gucacugggc 4500
   agaguccaca ccacacaaga gcaggaggau aaugcaauca aagcaauuau ggagauucca 4560
   aaggaaacac ugcagacugc agcagaugga acuaggcuua ccagacagcc acaaacaccu 4620
   aaggaaaaag uucaaccgcu ggaagaucac agugucuucc aagaacucuu ccaaacauca 4680
   cgcuacuguu cugauccauu aauugguaac aaacaaacaa gaauguccuu gagaucucca 4740
   caaccaggau uuguuagaac uccacgaacc ucaaagagac uggcuaagac aaguguuggg 4800
   aauauugcug ugagagaaaa gaucucucca gugagucugc cacagugugc uacaggggag 4860
   guuguacaca uacccauagg gccagaagau gacacagaga acaaaggugu gaaggaaucc 4920
   acaccucaga cacuggacuc aucagcaagu cgaacuguca gcaagaggca gcaaggggca 4980
   caugaggaaa ggccucaguu cucaggagac uuauuucauc cccaagagcu cuuucaaaca 5040
   ccagccagug gcaaagaccc aguaacuguu gaugaaacua caaaaauagc ucugcagucu 5100
   ccacaaccag gacauaucau aaacccagca agcaugaaga gacaguccaa caugagucuc 5160
   aggaaagaca ugagagaauu uuccauacuu gaaaaacaaa cacagucacg aggcagagac 5220
   gcaggcacac cagcaccaau gcaggaagaa aauggcacca cagccauuau ggaaacacca 5280
   aagcagaaac uggauuucau aggaaauuca acaggacaua agaggaggcc ucggacaccc 5340
   aaaaacaggg cucagccccu agaagaccug gauggcuucc aagaacucuu ucaaacacca 5400
   gcuggugcca gugacccugu gaguguugaa gaaagugcaa agauaucuuu ggcaucuuca 5460
   caagcagaac cagucagaac cccagcaagu acaaagagac gcuccaagac aggucucagu 5520
   aaaguggaug ugagacaaga accuucaaca cuugggaaaa gaaugaaguc acuaggcaga 5580
   gccccaggca caccagcacc agugcaggaa gaaaaugaca gcacagccuu cauggaaacu 5640
   ccaaagcaga aacuggauuu cacaggaaau ucaucaggac auaagaggag gccacagaca 5700
   ccuaagauca gggcucagcc ccuagaagac cuggauggcu uccaagaacu cuuccaaaca 5760
   ccagcuggug ccaaugacuc agugacuguu gaggaaagug uaaagauguc uuuggaaucu 5820
   ucacaagcag aaccagucaa aaccccggca agcacaaaga gacucuccaa gacaggucuc 5880
   aguaaggugg augugagaga agacccuuca auacuugaga aaaaaacaaa gucaccaggc 5940
   acaccagcac cagugcagga agaaaaugac ugcacagccu ucauggaaac uccaaagcag 6000
   aaacuggauu ucacaggaaa uucaucagga cauaagagga ggccacggac accuaagauc 6060
   agagcucagc cccuagaaga ccuggauggc uuccaagaac ucuuccaaac accagcuggu 6120
   gcuagugacu cagugacugu ugaggaaagu gcaaagaugu cuuuggaauc uucacaagca 6180
   aaaccaguca aaaccccggc aagcacaaag agacucucca agacaggucu caguaaggug 6240
   gaugugagag aagacccuuc aacacuuggg aaaaaaacaa agucaccagg cagagcccca 6300
   ggcacaccag caccagugca ggaagaaaau gacagcacag ccuucaugga aacuccaaag 6360
   cagaaacugg auuuugcaga gaauucauca gggaguaaga gaaggucacg aacaucuaag 6420
   aacaggucuc agccccuaga agaccuggau ggcuuccaag aacucuucca aacaccagcu 6480
   ggugccagua acccugugag uguugaagaa agugcaaaga uaucuuugga aucuucacaa 6540
   gcagaaccag ucagaacccg ggcaagcaca aagagacuuu ccaagacagg ucucaauaag 6600
   auggauguga gagaagggca cucuccgcuc aguaagucaa gcugugcauc acagaaaguc 6660
   augcaaaccc ucacacuugg agaagaucau ggcagagaga ccaaagaugg gaagguauug 6720
   uuagcucaga aauuggaacc agcaauauau guuacucgug gcaagaggca gcaaagguca 6780
   uguaagaaaa ggucccaguc cccagaagac cucucuggug uucaggaggu cuuccaaaca 6840
   ucaggccaua acaaggauuc agugacagug gacaaucuug caaaacugcc cagcucgucu 6900
   ccaccacuag agccaacaga cacuucagua accucacgga gacaggccag aacuggucug 6960
   aggaaaguuc acgugaaaaa ugaacuuuca ggaggcauaa ugcauccaca aauaucaggg 7020
   gaaauugugg acuuaccuag agaaccagaa ggugaaggca aagucauuaa aacaaggaag 7080
   caaucuguaa aacggaaauu ggacacagaa gucaaugugc cucgcaguaa gaggcaaaga 7140
   auuacaagag cagaaaagac ccuagaggau cugccuggcu uccaagagcu cugccaagcu 7200
   ccaagcuugg uaauggacuc aguuauuguu gagaaaaccc caaagaugcc cgacaaaucu 7260
   ccagaaccug uggauacaac uucagagaca caggcaagaa gaagacucag gagacugguu 7320
   guuacugaag agcccauacc acaaagaaag acuacaagag uuguaaggca aaccagaaac 7380
   acacagaaag agcccauaag ugacaaucaa gguauggaag aguuuaagga aucuucagua 7440
   cagaaacaag acccaagugu aaguuuaacu ggcaggagga accaaccaag gacaguuaag 7500
   gagaaaaccc aacccuuaga agaacucacc aguuuccaag aggaaacugc caaaagaaua 7560
   ucuuccaaau cuccacaacc ggaagagaag gaaaccuuag cagguuuaaa gaggcagcuc 7620
   agaauacaac uaaucaacga ugguguaaaa gaagagccca cagcacagag aaagcaacca 7680
   uccagggaaa ccaggaacac acucaaagag ccuguaggug acaguauaaa uguugaagag 7740
   guuaagaagu cuacaaagca gaaaauugau ccaguagcaa gugugccugu cagcaagagg 7800
   ccacggaggg uacccaagga aaaggcacag gcccuagaau uggcuggucu caaaggacca 7860
   auccaaaccc uaggccacac ugaugaauca gcaagugaua aaggacccac acagaugccc 7920
   uguaauucuc uacaaccaga gcaaguugac agcuuccaaa gcucaccaag gcgacccagg 7980
   acaagacgug ggaaaguaga ggcagaugaa gagccuucag caguaagaaa gacaguauca 8040
   acaucaaggc aaacuaugcg aucccgcaag gucccugaaa uugguaacaa ugguacccaa 8100
   guuucaaagg ccuccauaaa gcagacauua gauacaguag ccaaaguaac uggcagcagg 8160
   aggcagcuaa ggacacauaa aggauggggu ucaacccucu ugaaguuguu aggugacucc 8220
   aaagaaauaa cccaaauauc agaucacucu gagaaacuag cacaugacac caguauccuu 8280
   aagagcacuc aacagcaaaa gccagacuca guaaaaccuc ugagaacaug cagaagagug 8340
   cugagggccu cuaaagaggu ccccaaggaa guguuggugg acaccagaga ccaugcaaca 8400
   uuacaaagca aaagcaaccc uuugcugucc ccgaagagga agucugcaag agauggaagc 8460
   auugugagaa ccagggcuuu gcgcucuuua gcaccaaagc aggaagcaac agaugagaag 8520
   ccuguaccug agaaaaaaag ggcugcuucc agcaagaggu auguaucacc ugagccugug 8580
   aagaugaaac accugaaaau cgugucaaac aaacuugaau cuguggaaga gcagguuagc 8640
   acuguuauga aaacagaaga aauggaagcc aaaagagaaa auccugucac uccagaucag 8700
   aacucuaggu accgaaagaa aaccaaugua aaacagccaa ggcccaaguu ugaugcaucu 8760
   gcagagaaug ucgggauaaa gaaaaacgag aagacuauga agacugccuc ccaggagaca 8820
   gagcugcaga auccagauga uggagccaag aaaucuacau cucggggcca agucaguggg 8880
   aaaagaacau gcuugagguc uagaggaacg acugagaugc cccagccuug ugaagcagaa 8940
   gagaaaacaa gcaaaccagc ugcagaaauc uugauaaagc cucaggaaga gaaaggaguc 9000
   ucuggagagu cugauguuag guguuugagg uccagaaaaa cuagagucgc uuuggacagu 9060
   gaaccuaagc caaggguaac ucguggaacc aagaaagaug caaaaacucu gaaggaggau 9120
   gaagacauug uaugcaccaa gaaguuaaga acaagaaguu aagaacaaga aguuaccaga 9180
   aaagugaaac uauguagcaa agacauuuaa gaaggaaaag uaaauuugac uuagugauaa 9240
   guuccagugu gguuuucacc uccaguguaa agaugaacug uaaauacuac ugcuacugcc 9300
   ugaguuuaag gaaggaagcu uugagcuuuc cuggucauac ucucuucaga cgccaaugga 9360
   ggucaugagg aagaucacca gggaucucag cgcaauuaca guuuaggggu gagcaggcag 9420
   aaauguggcc cucuguccua uccaauaaag cucugaaauu cgcugccaaa a 9471
<210> 271
   <211> 463
   <212> RNA
   <213> Homo sapiens
<400> 271
   gacguagagc cccuugcgcc cgguuuccug aucccgcuua cuccucugcg cgccggcagg 60
   auggcccaca agcagaucua cuacucagac aaguacuucg augagcacua cgaguaccgg 120
   caugucaugu uacccagaga acucucuaaa caaguaccca aaacucaucu gauguccgaa 180
   gaggagugga ggagacuugg uguccaacag agucuaggau ggguucauua caugauucau 240
   gagccagaac cgcauauucu ucucuuuaga cgaccucuuc caaaagaaca acaaaaauga 300
   agugcagcug ggaucaucua aucuuuuuca aauuuaaugu auauguguau auaagguagu 360
   auucagugaa uacuugaaaa guguacaaac cuuucaucca uaccugugca ugcgcuguau 420
   ucuucacagc aacagagcuc agucaaaugc aacugcaagu agg 463
<210> 272
   <211> 1280
   <212> RNA
   <213> Homo sapiens
<400> 272
   cuacccuaga caaucacggc uuagccggcg cgcggagucg aucgucucgg ucgcuagagc 60
   uguccugagc ucgaacgguc cgacgccccc gccgcgccgg uccgugacgc cggggccgac 120
   acgaugaagg auuugggggc caagcacuug gcagguggcg aagggguuca gcuuuucgga 180
   uuguugaacu ucuaccugga acaagaacag agauaccaac cucgggaaaa agggcugauc 240
   uugauggagg cuaccccgga gaaugauaac acuuuguguu caagacugag aaaugccaaa 300
   guggaagauu uaagaaguuu aacuaacuuc uuuggaucug gcacugaaac uuucguucug 360
   gcugucaaua uuuuggauag auucuuggcc cuuaugaagg ugaaaccgaa acaccugucc 420
   ugcauuggcg ucugcugcuu uuugcuggcc gccaggcugg cggaagaaga aggugacguu 480
   ccccccacgc acgacgugau ccgcaucagu caguguaaau gcacagcguc ugacauuaaa 540
   cgcauggaga aaaucaucuc agagaaacug cacuaugagc uggaagcuac cacugccuua 600
   aacuuuuugc acuuguacca cgcgauugua uuuugucaca cuucagaaag gaaggagauu 660
   cucagccucg auaaacucga agcgcagcug aaagcuugca acugccgagu ugucuucucc 720
   aaagcaagac caucuguauu agcucugugc cuucucaauu uggaaauaga aacgauaaaa 780
   uccguggaac ugcuggaaau ucucuugcuu guuaaaaaac auuugaagcu cagcgacacu 840
   gaauucuuuu acuggaggga acugguuucu aaaugucuag cagaguauuc uucgccucgc 900
   ugcugcaagc cugaucugaa gaagcuggua uggauuguuu cgcgacgcac ugcgcagaac 960
   cuccacagca gcuacuacag uguuccugag cugcccacua ucccagaggg ggguugcuuu 1020
   gacggaagug aaagugagga cucuggugaa gacaugaguu guggagagga gagucucagc 1080
   agcuccccac ccagcgauca ggagugcacc uucuucuuug acuuccaagu ggcucagaca 1140
   cugugcuuuc caccauagag gaaucugaca uuguucugug ucagggaauu uauaagugug 1200
   uguaccuagg uuucaaagca auaaacuugg ggguugaaua ggguaguuuu ccuagguuuc 1260
   cagccccccg ucuagucagg 1280
<210> 273
   <211> 291
   <212> RNA
   <213> Homo sapiens
<400> 273
   aacuaccuug ggcagguucu auuaacugca ccuaacucag acgugaguag gacagaagga        60
   agcugucccg ggcgaacuga ggucacaaag acuugcuuuu gauucaagag agaccuuaaa       120
   ggcuaguuau gauaguuaag uacaaguuuu aacaucuggu agcuaacuuu uuuucucuac       180
   cccguaauuc uacuaugacu gcucuucuag agguccugag uucaaauccc agcaaccaca       240
   ugguggcuca caaccaucua uaaugggauc ugaugcccuc uucuggugug c                291
<210> 274
   <211> 391
   <212> PRT
   <213> Homo sapiens
<400> 274
<210> 275
   <211> **448**
   <212> PRT
   <213> Homo sapiens
<400> 275
<210> 276
   <211> 232
   <212> PRT
   <213> Homo sapiens
<400> 276
<210> 277
   <211> 1210
   <212> PRT
   <213> Homo sapiens
<400> 277
<210> 278
   <211> 1207
   <212> PRT
   <213> Homo sapiens
<400> 278
   Met Leu Leu Thr Leu Ile Ile Leu Leu Pro Val Val Ser Lys Phe Ser
   1               5                   10                  15
   Phe Val Ser Leu Ser Ala Pro Gln His Trp Ser Cys Pro Glu Gly Thr
               20                  25                  30
   Leu Ala Gly Asn Gly Asn Ser Thr Cys Val Gly Pro Ala Pro Phe Leu
           35                  40                  45
   Ile Phe Ser His Gly Asn Ser Ile Phe Arg Ile Asp Thr Glu Gly Thr
       50                  55                  60
   Asn Tyr Glu Gln Leu Val Val Asp Ala Gly Val Ser Val Ile Met Asp
   65                  70                  75                  80
   Phe His Tyr Asn Glu Lys Arg Ile Tyr Trp Val Asp Leu Glu Arg Gln
                   85                  90                  95
   Leu Leu Gln Arg Val Phe Leu Asn Gly Ser Arg Gln Glu Arg Val Cys
               100                 105                 110
   Asn Ile Glu Lys Asn Val Ser Gly Met Ala Ile Asn Trp Ile Asn Glu
           115                 120                 125
   Glu Val Ile Trp Ser Asn Gln Gln Glu Gly Ile Ile Thr Val Thr Asp
       130                 135                 140
   Met Lys Gly Asn Asn Ser His Ile Leu Leu Ser Ala Leu Lys Tyr Pro
   145                 150                 155                 160
   Ala Asn Val Ala Val Asp Pro Val Glu Arg Phe Ile Phe Trp Ser Ser
                   165                 170                 175
   Glu Val Ala Gly Ser Leu Tyr Arg Ala Asp Leu Asp Gly Val Gly Val
               180                 185                 190
   Lys Ala Leu Leu Glu Thr Ser Glu Lys Ile Thr Ala Val Ser Leu Asp
           195                 200                 205
   Val Leu Asp Lys Arg Leu Phe Trp Ile Gln Tyr Asn Arg Glu Gly Ser
       210                 215                 220
   Asn Ser Leu Ile Cys Ser Cys Asp Tyr Asp Gly Gly Ser Val His Ile
   225 230 235 240
   Ser Lys His Pro Thr Gln His Asn Leu Phe Ala Met Ser Leu Phe Gly
   245 250 255
   Asp Arg Ile Phe Tyr Ser Thr Trp Lys Met Lys Thr Ile Trp Ile Ala
   260 265 270
   Asn Lys His Thr Gly Lys Asp Met Val Arg Ile Asn Leu His Ser Ser
   275 280 285
   Phe Val Pro Leu Gly Glu Leu Lys Val Val His Pro Leu Ala Gln Pro
   290 295 300
   Lys Ala Glu Asp Asp Thr Trp Glu Pro Glu Gln Lys Leu Cys Lys Leu
   305 310 315 320
   Arg Lys Gly Asn Cys Ser Ser Thr Val Cys Gly Gln Asp Leu Gln Ser
   325 330 335
   His Leu Cys Met Cys Ala Glu Gly Tyr Ala Leu Ser Arg Asp Arg Lys
   340 345 350
   Tyr Cys Glu Asp Val Asn Glu Cys Ala Phe Trp Asn His Gly Cys Thr
   355 360 365
   Leu Gly Cys Lys Asn Thr Pro Gly Ser Tyr Tyr Cys Thr Cys Pro Val
   370 375 380
   Gly Phe Val Leu Leu Pro Asp Gly Lys Arg Cys His Gln Leu Val Ser
   385 390 395 400
   Cys Pro Arg Asn Val Ser Glu Cys Ser His Asp Cys Val Leu Thr Ser
   405 410 415
   Glu Gly Pro Leu Cys Phe Cys Pro Glu Gly Ser Val Leu Glu Arg Asp
   420 425 430
   Gly Lys Thr Cys Ser Gly Cys Ser Ser Pro Asp Asn Gly Gly Cys Ser
   435 440 445
   Gln Leu Cys Val Pro Leu Ser Pro Val Ser Trp Glu Cys Asp Cys Phe
   450 455 460
   Pro Gly Tyr Asp Leu Gln Leu Asp Glu Lys Ser Cys Ala Ala Ser Gly
   465 470 475 480
   Pro Gln Pro Phe Leu Leu Phe Ala Asn Ser Gln Asp Ile Arg His Met
   485 490 495
   His Phe Asp Gly Thr Asp Tyr Gly Thr Leu Leu Ser Gln Gln Met Gly
   500 505 510
   Met Val Tyr Ala Leu Asp His Asp Pro Val Glu Asn Lys Ile Tyr Phe
   515 520 525
   Ala His Thr Ala Leu Lys Trp Ile Glu Arg Ala Asn Met Asp Gly Ser
   530 535 540
   Gln Arg Glu Arg Leu Ile Glu Glu Gly Val Asp Val Pro Glu Gly Leu
   545 550 555 560
   Ala Val Asp Trp Ile Gly Arg Arg Phe Tyr Trp Thr Asp Arg Gly Lys
   565 570 575
   Ser Leu Ile Gly Arg Ser Asp Leu Asn Gly Lys Arg Ser Lys Ile Ile
   580 585 590
   Thr Lys Glu Asn Ile Ser Gln Pro Arg Gly Ile Ala Val His Pro Met
   595 600 605
   Ala Lys Arg Leu Phe Trp Thr Asp Thr Gly Ile Asn Pro Arg Ile Glu
   610 615 620
   Ser Ser Ser Leu Gln Gly Leu Gly Arg Leu Val Ile Ala Ser Ser Asp
   625 630 635 640
   Leu Ile Trp Pro Ser Gly Ile Thr Ile Asp Phe Leu Thr Asp Lys Leu
   645 650 655
   Tyr Trp Cys Asp Ala Lys Gln Ser Val Ile Glu Met Ala Asn Leu Asp
   660 665 670
   Gly Ser Lys Arg Arg Arg Leu Thr Gln Asn Asp Val Gly His Pro Phe
   675 680 685
   Ala Val Ala Val Phe Glu Asp Tyr Val Trp Phe Ser Asp Trp Ala Met
   690 695 700
   Pro Ser Val Ile Arg Val Asn Lys Arg Thr Gly Lys Asp Arg Val Arg
   705 710 715 720
   Leu Gln Gly Ser Met Leu Lys Pro Ser Ser Leu Val Val Val His Pro
   725 730 735
   Leu Ala Lys Pro Gly Ala Asp Pro Cys Leu Tyr Gln Asn Gly Gly Cys
               740                 745                 750
   Glu His Ile Cys Lys Lys Arg Leu Gly Thr Ala Trp Cys Ser Cys Arg
           755                 760                 765
   Glu Gly Phe Met Lys Ala Ser Asp Gly Lys Thr Cys Leu Ala Leu Asp
       770                 775                 780
   Gly His Gln Leu Leu Ala Gly Gly Glu Val Asp Leu Lys Asn Gln Val
   785                 790                 795                 800
   Thr Pro Leu Asp Ile Leu Ser Lys Thr Arg Val Ser Glu Asp Asn Ile
                   805                 810                 815
   Thr Glu Ser Gln His Met Leu Val Ala Glu Ile Met Val Ser Asp Gln
               820                 825                 830
   Asp Asp Cys Ala Pro Val Gly Cys Ser Met Tyr Ala Arg Cys Ile Ser
           835                 840                 845
   Glu Gly Glu Asp Ala Thr Cys Gln Cys Leu Lys Gly Phe Ala Gly Asp
       850                 855                 860
   Gly Lys Leu Cys Ser Asp Ile Asp Glu Cys Glu Met Gly Val Pro Val
   865                 870                 875                 880
   Cys Pro Pro Ala Ser Ser Lys Cys Ile Asn Thr Glu Gly Gly Tyr Val
                   885                 890                 895
   Cys Arg Cys Ser Glu Gly Tyr Gln Gly Asp Gly Ile His Cys Leu Asp
               900                 905                 910
   Ile Asp Glu Cys Gln Leu Gly Val His Ser Cys Gly Glu Asn Ala Ser
           915                 920                 925
   Cys Thr Asn Thr Glu Gly Gly Tyr Thr Cys Met Cys Ala Gly Arg Leu
       930                 935                 940
   Ser Glu Pro Gly Leu Ile Cys Pro Asp Ser Thr Pro Pro Pro His Leu
   945                 950                 955                 960
   Arg Glu Asp Asp His His Tyr Ser Val Arg Asn Ser Asp Ser Glu Cys
                   965                 970                 975
   Pro Leu Ser His Asp Gly Tyr Cys Leu His Asp Gly Val Cys Met Tyr
               980                 985                 990
   Ile Glu Ala Leu Asp Lys Tyr Ala Cys Asn Cys Val Val Gly Tyr Ile
           995                 1000                1005
   Gly Glu Arg Cys Gln Tyr Arg Asp Leu Lys Trp Trp Glu Leu Arg
       1010                1015                1020
   His Ala Gly His Gly Gln Gln Gln Lys Val Ile Val Val Ala Val
       1025                1030                1035
   Cys Val Val Val Leu Val Met Leu Leu Leu Leu Ser Leu Trp Gly
       1040                1045                1050
   Ala His Tyr Tyr Arg Thr Gln Lys Leu Leu Ser Lys Asn Pro Lys
       1055                1060                1065
   Asn Pro Tyr Glu Glu Ser Ser Arg Asp Val Arg Ser Arg Arg Pro
       1070                1075                1080
   Ala Asp Thr Glu Asp Gly Met Ser Ser Cys Pro Gln Pro Trp Phe
       1085                1090                1095
   Val Val Ile Lys Glu His Gln Asp Leu Lys Asn Gly Gly Gln Pro
       1100                1105                1110
   Val Ala Gly Glu Asp Gly Gln Ala Ala Asp Gly Ser Met Gln Pro
       1115                1120                1125
   Thr Ser Trp Arg Gln Glu Pro Gln Leu Cys Gly Met Gly Thr Glu
       1130                1135                1140
   Gln Gly Cys Trp Ile Pro Val Ser Ser Asp Lys Gly Ser Cys Pro
       1145                1150                1155
   Gln Val Met Glu Arg Ser Phe His Met Pro Ser Tyr Gly Thr Gln
       1160                1165                1170
   Thr Leu Glu Gly Gly Val Glu Lys Pro His Ser Leu Leu Ser Ala
       1175                1180                1185
   Asn Pro Leu Trp Gln Gln Arg Ala Leu Asp Pro Pro His Gln Met
       1190                1195                1200
   Glu Leu Thr Gln
       1205
<210> 279
   <211> 781
   <212> PRT
   <213> Homo sapiens
<400> 279
<210> 280
   <211> 349
   <212> PRT
   <213> Homo sapiens
<400> 280

## Description

### FIELD OF INVENTION

The present invention provides compositions for use in treating baldness in a subject and non-therapeutic methods for generating new hair follicles.

### BACKGROUND OF THE INVENTION

Follicular neogenesis is defined as the generation of new hair follicles (HF) after birth. Humans are born with a full complement of HF, which can change in size and growth characteristics as in early baldness or can ultimately degenerate and disappear as in late stages of baldness or in permanent scarring (cicatricial) alopecias. Therefore, the generation of new HF is desirable in the treatment of common baldness as well as less common hair loss conditions, such as discoid lupus erythematosis, congenital hypotrichosis, lichen planopilaris and other scarring alopecias.

Messenger et al. (British Journal of Dematology, 2004, 150(2): 186-194) discusses the mechanism of action of minoxidil on hair growth.

Argyris (The Anatomical Record, 1962, 143(3): 183-188) discusses the growth-promoting effects of wounds on hair follicles already stimulated by plucking.

Argyris et al., (The Anatomical Record, 1962, 142(2): 139-145) discusses factors affecting the stimulation of hair growth during wound healing.

Kligman (Annals of the New York Academy of Sciences, 1959, 83(3): 507-511) discusses the neogenesis of human hair follicles.

Kligman et al., (The Journal of Investigative Dermatology, 1956, 27(1): 19-23) discusses the formation of vellus hair follicles from human adult epidermis.

Breedis (Cancer Research, 1954, 14(8): page 17PP) discusses the regeneration of hair follicles and sebaceous glands from the epithelium of scars in the rabbit.

### SUMMARY OF THE INVENTION

The present invention provides the use of minoxidil for the preparation of a pharmaceutical composition for treating baldness in a scalp, eyebrow or scarred region of a subject, wherein the treating comprises (a) disrupting an epidermis of the scalp, eyebrow or scarred region, wherein said disrupting is perforating the epidermis or epidermal layer; and (b) contacting the scalp, eyebrow or scarred region with the pharmaceutical composition.

The present invention also provides minoxidil for use in treating baldness in a scalp, eyebrow or scarred region of a subject, wherein the treating comprises (a) disrupting an epidermis of the scalp, eyebrow or scarred region, wherein said disrupting is perforating the epidermis or epidermal layer; and (b) contacting the scalp, eyebrow or scarred region with the minoxidil.

In an embodiment, the subject has androgenetic alopecia (AGA) or another disease or disorder comprising balding.

The present invention also provides a non-therapeutic method for generating a hair follicle in a scalp, eyebrow or scarred region of a subject, the method comprising (a) disrupting an epidermis of the scalp, eyebrow or scarred region, wherein said disrupting is perforating the epidermis or epidermal layer; and (b) contacting the scalp, eyebrow or scarred region with minoxidil, thereby generating a hair follicle in the scalp, eyebrow or scarred region of the subject.

In an embodiment, the disruption is performed with a tool that comprises sandpaper, a laser, such as a Fraxel laser, a CO2 laser or an excimer laser, a felt wheel, a microdermabrasion device, a light-based method, irradiation with visible light, irradiation with infrared light, irradiation with ultraviolet radiation, orthovoltage radiation, x-ray radiation, a surgical tool, a dermal biopsy punch, or wherein the disruption is performed with a burn treatment or chemical treatment. In an embodiment, the disruption comprises perforating the epidermis and dermis of the area of the balding scalp. In an embodiment, the disruption comprises removal of basal and supra-basal layers of epidermis with a felt wheel.

In an embodiment, the disrupting step is performed between 3-12 days, 4-12 days, 5-12 days, 4-11 days, 6-11 days, 6-10 days, 6-9 days, 6-8 days, 7-8 days, 5-11 days, 5- 10 days, 7- 10 days, or about 1 week prior to the contacting step.

In an embodiment, the treating further comprises contacting the area of the balding scalp with a precursor cell, an inductive cell, or a hair follicle or a portion thereof, or with one or more of the following compounds: finasteride, dutasteride, Fluridil^{®}, spironolactone, cyproterone acetate, bicalutamide, flutamide, nilutamide, an inhibitor of an androgen receptor, an androgen antagonist or an antiandrogen. In an embodiment, the compound is formulated to be administered as a cream, gel, lotion, emulsion, suspension, oil, non-aqueous solution, aqueous solution, or drop, or by direct injection, or is encapsulated in a liposome. In an embodiment, the disrupting step is performed between 3-12 days, 4-12 days, 5-12 days, 4-11 days, 6-11 days, 6-10 days, 6-9 days, 6-8 days, 7-8 days, 5-11 days, 5- 10 days, 7- 10 days, or about 1 week prior to the further contacting step.

In an embodiment, the area of the balding scalp is subjected to depilation or administration of a retinoid prior to disrupting. In an embodiment, the depilation is performed by plucking, waxing, abrasion, laser, electrolysis, or administration of thioglycolic acid on the area of the balding scalp.

In an embodiment, the wounded area of the balding scalp is not surgically closed, or not contacted with a bandage, dressing, or ointment that facilitates wound healing for a period of time following the disrupting step. In an embodiment, the period of time is within about 2 days, about 3 days, about 4 days, about 5 days, about 7 days, about 10 days, about 11 days, about 14 days, about 17 days or about 3 weeks.

In an embodiment, the treatment or the method results in differentiation of an uncommitted epidermal cell into a hair follicle cell.

### BRIEF DESCRIPTION OF THE FIGURES

Figure 1: Epidermal abrasion results in *de novo* hair follicle (HF) formation. HF at progressive stages of development are depicted in the left, center, and right panels. The arrow in the left panel indicates a hair germ. The dark stained cells are progeny of HF stem cells in the bulge.
Figure 2: BrdU labeling of HF following epidermal abrasion. HF at progressive stages of development are depicted in the left, center, and right panels.
Figure 3. The wound site did not contain HF immediately after re-epithelialization. Top view (left panel) and tissue section (right panel) of the site 10 days after wound induction.
Figure 4. Appearance of hair germs 12 days after wound induction. Arrow indicates hair germ.
Figure 5. Epidermal Disruption-Induced HF neogenesis (EDIHN)-induced hair germs express K17. Two different hair germs are depicted in the left and right panels.
Figure 6. EDIHN-induced hair germs contain dermal papilla (DP) cells, as evidenced by alkaline phosphatase (AP) staining. Arrows indicate DP cells. Left panel: hair germs. Right panel: HF at a further developmental stage.
Figure 7. Histological comparison between EDIHN-induced and embryonic HF. Top left, second from left, third from left, and right panels: Progressive stages of EDIHN-induced HF development. Bottom left, center, and right panels: Progressive stages of and embryonic HF development.
Figure 8. Induction of several markers of embryonic HF development, Lef1 (left panel), wingless/ int (Wnt) 10b (center panel), and sonic hedgehog (Shh; right panel), by EDIHN. HF structures are indicated by arrowheads.
Figure 9. Proliferative activity during EDIHN, as evidenced by BrdU pulse-labeling. Progressive stages of HF development are depicted in the left, center, and right panels.
Figure 10. Induction of HF markers S100A3 (left panel; tissue section parallel to HF axis) and S100A6 (right panel; cross-sectional view of follicle) by EDIHN.
Figure 11. New hair growth 25 days (left panel) and 45 days (right panel) after wound induction.
Figure 12. Schematic of whole-mount EDIHN assay.
Figure 13. Repopulation of stem cells in the bulge of EDIHN-induced HF, as evidenced by retention of BrdU label following a chase period. Left panel: lower magnification: 50 X. Right panel: higher magnification: 400 X.
Figure 14. A. Stem cells in EDIHN-induced HF express K15. Left top panel: Top view of wound site. Bottom, far left panel: epidermis whole mount; bottom, second from left panel: same as [bottom, far left] panel but viewed under white light; right panel: tissue sections. B. Neogenesis HF proceed to next hair cycle.
Figure 15. Schematic of creation of new HF by EDIHN.
Figure 16. No new HF are evident 11 days after wound induction in 21-day-old mice. Top panel: macroscopic examination; bottom left panel: AP staining of the dermis; bottom right panel: K17 staining of the epidermis.
Figure 17. 14 days after wound induction, new HF have begun to form as evidenced by AP staining of the dermis (left panel) and K17 staining of the epidermis (right panel). Main panels: 10 X magnification. Inserts: 80 X magnification.
Figure 18. 17 days after wound induction, new HF are more developed. Left panel: AP staining of the dermis; right panel: K17 staining of the epidermis. Main panels: 10 X magnification. Inserts: 80 X magnification.
Figure 19. Wounds closed similar in mice subjected to depilation, then wounding (left 3 mice in each panel) vs. wounding alone (right 4 mice in each panel). Left panel: immediately following wounding. Right panel: 10 days following wounding.
Figure 20. Anagen induction by depilation prior to wounding enhances the efficiency of EDIHN. A. Top panel: lower left panel AP staining of the dermis; lower right panel: K17 staining of the epidermis. B. Graphical representation of enhancement of EDIHN by depilation.
Figure 21. A. EDIHN in human skin grafted to immunodeficient (scid) mice, seven days after induction of an excisional wound. Arrows indicate new HF. B. Dermal abrasion of human skin grafts results in EDIHN. Human adult skin (W) was grafted onto mice, abraded, and examined seven days later, by staining for S100A6 (first, second, and fourth rows) or S100A4 (third row). Hair germs (HG) and dermal papilla (DP) are indicated. Human fetal skin (F) with normal developing hair follicles is shown for comparison. Mouse skin 17d post wounding was included as a control (top left panel).
Figure 22. Transcripts up-regulated at least 2-fold in activated HF cells, as assessed by dChip analysis. (A). Mean values and standard errors of the up-regulated transcripts in non-activated ("bs-line") and activated ("expt") samples and fold-changes and differences between non-activated and activated values are depicted. (B). Raw data for up-regulated transcripts in non-activated and activated cells. "Ctrl" denotes non-activated and "High-dep" denotes activated cells. (C). Additional information about up-regulated transcripts.
Figure 23. Pigmented hair follicle neogenesis observed in the skin of Dkk1-expressing mice following EDIHN A. 3.2x magnification. B. 8x magnification.
Figure 24. Control mice lacked pigmented HF.
Figure 25. EGF inhibits HF formation by EDIHN. A. K17 staining of wounded skin of representative mouse treated with EGF. Magnification is 4x. B. High magnification view (10×) of skin depicted in (A). C. K17 staining of wounded skin of representative control mouse that received no EGF after wounding. Magnification is 4x. D. Higher magnification view (10×) of skin depicted in (D).

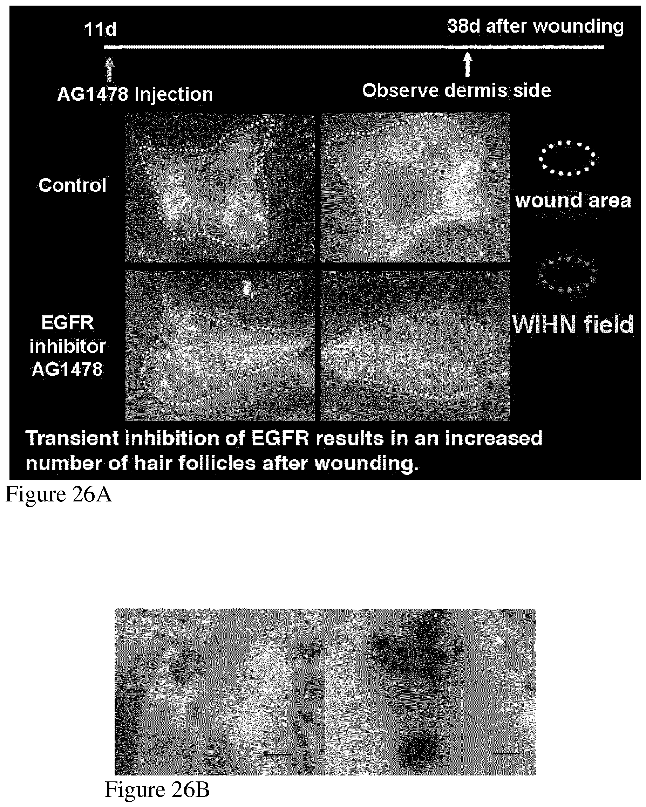

Figure 26. Administration of an EGF receptor inhibitor (AG1478) leads to generation of more and larger HF compared with controls. A. Top: skin of 2 control mice. Outer dashed line indicates the extent of the wounded area after contraction and healing; inner dashed line indicates the area of neogenesis. Bottom: skin of 2 treated mice, in which the wounded area and area of neogenesis largely coincide, with the exception of a small area on the left side of the encircled area in each panel. B. Large hair follicles developed in the wounded area in the AG1478-injected mice. Left panel: epidermis stained for K17, with three large hair follicles next to each other. Right panel: dermis stained for AP with large coalescing DP areas. Scale Bars: 200 µm.
Figure 27. A. Increased hair follicle formation in K14-Wnt7a mice. Left panel: Wnt7a transgenic mice. Right panel: control (wild-type) mice. B. Quantiation of experiment with 4 week old mice. C. Quantiation of experiment with 3 week old mice.

### DETAILED DESCRIPTION OF THE INVENTION

The present invention provides the use of minoxidil for the preparation of a pharmaceutical composition for the treatment of baldness in a scalp, eyebrow or scarred region of a subject, wherein the treatment comprises (a) disrupting an epidermis of the scalp, eyebrow or scarred region, wherein said disrupting is perforating the epidermis or epidermal layer; and (b) contacting the scalp, eyebrow or scarred region with the pharmaceutical composition.

The present invention also provides minoxidil for use in the treatment of baldness in a scalp, eyebrow or scarred region of a subject, wherein the treatment comprises (a) disrupting an epidermis of the scalp, eyebrow or scarred region, wherein said disrupting is perforating the epidermis or epidermal layer; and (b) contacting the scalp, eyebrow or scarred region with the minoxidil.

The present invention also provides a non-therapeutic method for generating a hair follicle in a scalp, eyebrow or scarred region of a subject, the method comprising (a) disrupting an epidermis of the scalp, eyebrow or scarred region, wherein said disrupting is perforating the epidermis or epidermal layer; and (b) contacting the scalp, eyebrow or scarred region with minoxidil, thereby generating a hair follicle in the scalp, eyebrow or scarred region of the subject.

"Contacting" as used herein refers, in another embodiment, to bringing the scalp, eyebrow, into to contact with the compound, factor, cell. In another embodiment, the term refers to embedding the compound, factor, cell into the scalp, eyebrow. In another embodiment, the term refers to injecting the compound, factor, cell into the scalp, eyebrow. In another embodiment, term refers to any other type of contacting known in the art.

As disclosed herein, minoxidil is a compound or factor that promotes a differentiation of an uncommitted epidermal cell into a hair follicle cell.

"Promotes a differentiation" refers, in another embodiment, to the act of increasing the percentage of cells that will differentiate as indicated. In another embodiment, the term refers to increasing the number of cells per unit area of skin that will differentiate. In another embodiment, the promoter of differentiation is active in the milieu of the skin.

"Uncommitted epidermal cell" refers, in another embodiment, to an epidermal stem cell. In another embodiment, the epidermal cell is a bulge cell. In another embodiment, the epidermal cell is a bulge-derived cell. In another embodiment, the epidermal cell is any other type of cell known in the art that can be induced to differentiate into an HF cell.

The "HF cell" that results from the differentiation is, in another embodiment, an HF stem cell. In another embodiment, the HF cell is a dermal papilla cell. In another embodiment, the HF cell is a bulb cell. In another embodiment, the HF cell is a matrix cell. In another embodiment, the HF cell is a hair shaft cell. In another embodiment, the HF cell is an inner root sheath cell. In another embodiment, the HF cell is an outer root sheath cell. In another embodiment, the HF cell is a melanocyte stem cell. In another embodiment, the HF cell is a melanocyte.

In another embodiment, the minoxidil is administered in a solution. In another embodiment, the minoxidil is administered in a cream. In another embodiment, the minoxidil is administered in an ointment. In another embodiment, the minoxidil is administered in a slow release formulation. In another embodiment, the minoxidil is administered in a liposome encapsulated formulation. In another embodiment, the minoxidil is directly injected. In another embodiment, the minoxidil is administered by any other method known in the art.

It is disclosed herein that a compound or factor that promotes a differentiation of an uncommitted epidermal cell into a HF cell may be an inhibitor of an Epidermal Growth Factor (EGF). In another embodiment, the compound or factor may be an inhibitor of an EGF receptor.

As provided herein (Example 11), activating one or more pathways in which EGF is involved (by injection of EGF) blocks the formation of HF. Thus, antagonizing the pathway increases HF formation, as demonstrated in Example 12.

As provided herein, FGF and its receptor are upregulated, under the conditions utilized herein, upon HF stem cell differentiation (Example 9). Moreover, FGF-bp1 is downregulated, under the conditions utilized herein, upon HF stem cell differentiation. Thus, it is disclosed that FGF and its receptor promote differentiation of uncommitted epidermal cells into HF cells.

In another embodiment, the compound or factor that promotes a differentiation of an uncommitted epidermal cell into a HF cell acts directly on the uncommitted epidermal cell. In another embodiment, the compound or factor acts on the uncommitted epidermal cell via a mesenchymal cell.

In another embodiment, the mesenchymal cell is a dermal condensate cell. In another embodiment, the mesenchymal cell is a DP cell. In another embodiment, the mesenchymal cell is another cell type or differentiation stage in the dermal condensate-DP lineage. In another embodiment, the mesenchymal cell is any other type of mesenchymal cell known in the art.

It is disclosed that the EGFR has, in another embodiment, the sequence of SEQ ID No: 277 (GenBank Accession No: NM_005228). In another embodiment, the EGFR has a sequence selected from the sequences set forth in GenBank entries NM_201282, NM_201283, NM_201284, BC094761, AF288738, AY588246, AY573061, X17054, AF125253, U48722, K03193, and AY698024. In another embodiment, the EGFR is encoded by a nucleic acid molecule having a sequence set forth in the one of the above GenBank entries.

It is also disclosed that EGF has, in another embodiment, the sequence of SEQ ID No: 278 (GenBank Accession No: NM_001963). In another embodiment, the EGF has a sequence selected from the sequences set forth in GenBank entries BC093731, AY548762, and X04571. In another embodiment, the EGF is encoded by a nucleic acid molecule having a sequence set forth in the one of the above GenBank entries.

As provided herein, the transcripts depicted in Table 3 (Example 8; SEQ ID No: 1-232), the proteins they encode, and the pathways in which the proteins participate contribute significantly to HF stem cell activation. Accordingly, under the conditions utilized herein, anagen can be induced by activation of these transcripts, proteins, and pathways. It is thus disclosed that activation of the transcripts, proteins, and pathways depicted in Table 3 may be a method for enhancing EDIHN or for enhancing hair growth in a subject.

As provided herein, the transcripts depicted in Table 4 (Example 9; SEQ ID No: 233-257), the proteins they encode, and the pathways in which the proteins participate, contribute significantly, under the conditions utilized herein, to induction of epidermal cells to differentiate into HF stem cells. It is thus disclosed that activation of the transcripts, proteins, and pathways depicted in Table 4 may be a method for enhancing EDIHN or for enhancing hair growth in a subject.

As demonstrated by findings of the present invention, the transcripts depicted in Table 5 (Example 9; SEQ ID No: 258-273), the proteins they encode, and the pathways in which the proteins participate, contribute significantly, under the conditions utilized herein, to preventing induction of epidermal cells to differentiate into HF stem cells. It is thus disclosed that inhibition of the transcripts, proteins, and pathways depicted in Table 5 may be a method for enhancing EDIHN or for induction of hair growth in a subject.

In another embodiment of methods and compositions of the present invention, a composition comprising minoxidil is administered.

The step of disrupting the epidermis is performed, in another embodiment, by abrading the scalp, eyebrow, or scarred region. In another embodiment, the term "abrading" refers to an act of creating an abrasion. In another embodiment, "abrading" refers to rubbing. In another embodiment, "abrading" refers to wearing away by friction. As provided herein (Example 1), epidermal abrasion causes, under the conditions utilized herein, *de novo* HF neo-genesis. In another embodiment, the epidermal layer is disrupted.

In one embodiment, "abrasion" has the same meaning as "abrading." In another embodiment, "abrasion" refers to an area of the scalp or skin from which the epidermis is removed. In another embodiment, "abrasion" refers to an area of the scalp or skin from which the epidermis and dermis are removed.

As provided herein, under the conditions utilized, epidermal disruption converts the skin back, in another embodiment, to an embryonic-like state, in which the follicle regenerates. In another embodiment, a subsequent window of opportunity is created, during which the number and size of new HF in the skin can be manipulated. In another embodiment, the administration of a compound or factor that promotes a differentiation of an uncommitted epidermal cell into a HF cell during this window causes regeneration of larger and more numerous HF. The morphology of HF in abraded skin is similar to that of embryonic HF (Example 1-2 and subsequent Examples), and the markers expressed are similar as well.

The present application discloses stimulating hair growth in a scalp, eyebrow, or scarred region of a subject. As demonstrated in Example 3, EDIHN-induced HF are capable of generating hairs. Thus, the compositions, uses and methods of the present invention can be used to stimulate hair growth.

"EDIHN," in another embodiment, refers to HF neogenesis induced by disruption of the epithelial layer. In another embodiment, the term refers to HF neogenesis induced by abrasion. In another embodiment, the term refers to HF neogenesis induced by wounding. In another embodiment, the term refers to HF neogenesis induced by disruption of the epithelial layer, followed by administration of a compound or factor that promotes a differentiation of an uncommitted epidermal cell into a HF cell.

In another embodiment, excisional wounds may be created using a surgical tool. In one embodiment, the surgical tool is a dermal biopsy punch (Example 2). In another embodiment, the excisional wounds are induced by freezing or cryoinjury. The use of freezing or cryoinjury is well known in the art, and is used, for example by dermatologists to injure skin. In one embodiment, the freezing or cryoinjury results in a blister. In another embodiment, the blister is used as a "chamber" to introduce drugs and or cells into the reepithelialized area.

In another embodiment, the excisional wounds are not surgically closed. In another embodiment, the excisional wounds are not contacted with a bandage or dressing before they heal or during a period of time after wound induction. In another embodiment, the excisional wounds are not contacted with an ointment before they heal or during a period of time after wound induction. In another embodiment, the excisional wounds are allowed to heal by secondary intention.

The subject of the present invention, is, in another embodiment, a human. As provided herein (Example 7) human skin responds to EDIHN in the same manner as mouse skin. In another embodiment, the subject is a male. In another embodiment, the subject is a female. In another embodiment, the subject is any other type of subject known in the art.

In another embodiment, the subject is an adult. In one embodiment, "adult" refers to an age greater than about 18 years. In another embodiment, "adult" refers to an age greater than about 20 years. In another embodiment, "adult" refers to an age greater than about 25 years. In another embodiment, "adult" refers to an age greater than about 30 years. In another embodiment, "adult" refers to an age greater than about 35 years. In another embodiment, "adult" refers to an age greater than about 40 years. In another embodiment, "adult" refers to an age greater than about 45 years.

In another embodiment, the subject is elderly. In one embodiment, "elderly" refers to an age greater than about 45 years. In another embodiment, "elderly" refers to an age greater than about 50 years. In another embodiment, "elderly" refers to an age greater than about 55 years. In another embodiment, "elderly" refers to an age greater than about 60 years. In another embodiment, "elderly" refers to an age greater than about 65 years. In another embodiment, "elderly" refers to an age greater than about 70 years.

In another embodiment, the subject is a laboratory animal. In another embodiment, the subject(s) is/are mice. In another embodiment, the subject(s) is/are rats. In another embodiment, the subject(s) is/are gerbils. In another embodiment, the subject(s) is/are hamsters. In another embodiment, the subject(s) is/are guinea pigs. In another embodiment, the subject(s) is/are rabbits. In another embodiment, the subject(s) is/are pigs. In another embodiment, the subject(s) is/are dogs. In another embodiment, the subject(s) is/are cats. In another embodiment, the subject(s) is/are primates. In another embodiment, the subject(s) is/are any other laboratory animal known in the art.

In another embodiment, the subject(s) to be treated has a disease or disorder comprising balding. In another embodiment, the disease or disorder is androgenetic alopecia (AGA).In another embodiment, the disease or disorder is a discoid lupus erythematosis. In another embodiment, the disease or disorder is a congenital hypotrichosis. In another embodiment, the disease or disorder is a lichen planopilaris. In another embodiment, the disease or disorder is a scarring alopecia. In another embodiment, the disease or disorder is any other disease or disorder comprising balding known in the art.

In another embodiment, the scalp, eyebrow, or scarred region(s) has a majority of HF in the telogen stage of the hair cycle. The findings of Examples 5-6 show that (a) EDIHN can restore hair growth to the scalp, eyebrow, or scarred region at the telogen stage; and (b) the efficiency of EDIHN at the telogen stage can be enhanced by depilation prior to abrasion or wound induction. In another embodiment, the scalp, eyebrow, or scarred region(s) has more than about 60% of HF in the telogen stage of the hair cycle. In another embodiment, the scalp, eyebrow, or scarred region(s) has more than about 70% of HF in the telogen stage of the hair cycle. In another embodiment, the scalp, eyebrow, or scarred region(s) has more than about 80% of HF in the telogen stage of the hair cycle. In another embodiment, the scalp, eyebrow, or scarred region(s) has more than about 90% of HF in the telogen stage of the hair cycle. In another embodiment, the scalp, eyebrow, or scarred region(s) does not have a majority of HF in the telogen stage of the hair cycle.

In another embodiment of methods, uses and compositions of the present invention, the first step (e.g. epithelial disruption) is performed 3-12 days prior to the second step (e.g. addition of minoxidil). In another embodiment, the interval is 4-12 days. In another embodiment, the interval is 5-12 days. In another embodiment, the interval is 4-11 days. In another embodiment, the interval is 6-11 days. In another embodiment, the interval is 6-10 days. In another embodiment, the interval is 6-9 days. In another embodiment, the interval is 6-8 days. In another embodiment, the interval is 7-8 days. In another embodiment, the interval is 5-11 days. In another embodiment, the interval is 5-10 days. In another embodiment, the interval is 7-10 days. In another embodiment, the interval is about 1 week.

The present application further discloses the step of suppressing an activity or expression of a Wnt protein in the scalp, eyebrow, or scarred region. As provided herein, suppressing Wnt activity induces pigmentation in HF generated by the compositions, uses and methods of the present invention. In another embodiment, the step of suppressing Wnt activity or expression is performed within about 10 days of epidermal disruption. In another embodiment, the step of suppressing Wnt activity or expression is performed prior to the second step (e.g prior to addition of minoxidil).

In another embodiment, the Wnt protein is Wnt1. In another embodiment, the Wnt protein is a Wnt7. In another embodiment, the Wnt protein is a Wnt7a. In another embodiment, the Wnt protein is a Wnt3. In another embodiment, the Wnt protein is a Wnt3a. In another embodiment, the Wnt protein is a Wnt10. In another embodiment, the Wnt protein is a Wnt10a. In another embodiment, the Wnt protein is any other Wnt protein known in the art.

In another embodiment, the present application discloses a method for inducing growth of a pigmented scalp hair or eyebrow of a subject, comprising generating a hair follicle in the scalp, eyebrow, or scarred region according to the method of the invention and suppressing expression of a Wnt protein in the hair follicle, thereby inducing a growth of a pigmented scalp hair or eyebrow of a subject.

In another embodiment, the step of suppressing expression of a Wnt protein comprises inducing an expression of a Dkk1 protein. In another embodiment, the step of suppressing expression of a Wnt protein comprises inducing an expression of any other Wnt inhibitor known in the art. In another embodiment, the step of suppressing expression of a Wnt protein is performed immediately or shortly after epidermal disruption. In another embodiment, the step of inducing expression of a Dkk1 protein is performed immediately or shortly after epidermal disruption. In another embodiment, the step of suppressing expression of a Wnt protein is performed at the time of epidermal disruption. In another embodiment, the step of inducing expression of a Dkk1 protein is performed at the time of epidermal disruption. In another embodiment, the step of suppressing expression of a Wnt protein is performed several days before generation of the follicle. In another embodiment, the step of inducing expression of a Dkk1 protein several days before generation of the follicle.

In another embodiment, the step of suppressing expression of a Wnt protein is performed for about 8 days. In another embodiment, the step of inducing expression of a Dkk1 protein is performed for about 8 days. In another embodiment, the step of suppressing expression of a Wnt protein is performed for about 9 days. In another embodiment, the step of inducing expression of a Dkk1 protein is performed for about 9 days. In another embodiment, the step of suppressing expression of a Wnt protein is performed for about 10 days. In another embodiment, the step of inducing expression of a Dkk1 protein is performed for about 10 days. In another embodiment, the step of suppressing expression of a Wnt protein is performed for about 12 days. In another embodiment, the step of inducing expression of a Dkk1 protein is performed for about 12 days. In another embodiment, the step of suppressing expression of a Wnt protein is performed during the period of re-epithelialization. In another embodiment, the step of inducing expression of a Dkk1 protein is performed during the period of re-epithelialization. In another embodiment, expression of a Dkk1 protein is halted after several days. In another embodiment, halting expression of Dkk1 protein after several days induces, or enables induction of Wnt protein expression. In another embodiment, the expression of a Wnt protein is induced about 9 days after the abrading or wounding. In another embodiment, the expression of a Wnt protein is induced following the period of re-epithelialization. In another embodiment, induction of Wnt protein expression is necessary for formation of new HF.

In another embodiment, "several" refers to about 1 day. In another embodiment, "several" refers to about 2 days. In another embodiment, "several" refers to about 3 days. In another embodiment, "several" refers to about 5 days. In another embodiment, "several" refers to about 7 days. In another embodiment, "several" refers to about 10 days. In another embodiment, "several" refers to about 12 days.

In another embodiment, the step of contacting in the present invention comprises directly contacting the scalp, eyebrow, or scarred region with the relevant compound.

In another embodiment, the epidermal disruption in the present invention further removes dermal tissue from the scalp, eyebrow, or scarred region. In another embodiment, the epidermal disruption does not remove dermal tissue from the scalp, eyebrow, or scarred region.

"Disrupting" an epidermis or epidermal layer refers, in another embodiment, to removing part of the epidermis or epidermal layer. In another embodiment, only part of the epidermal layer need be removed. In another embodiment, the entire epidermal layer is removed. In another embodiment, the term refers to abrading the epidermis or epidermal layer (Examples). In another embodiment, the term refers to wounding the epidermis or epidermal layer (Examples).

In another embodiment, the epidermal disruption is performed with a tool that comprises sandpaper. In another embodiment, the epidermal disruption is performed with a laser. In another embodiment, the laser is a Fraxel laser. In another embodiment, the laser is a CO₂ laser. In another embodiment, the laser is an excimer laser. In another embodiment, the laser is any other type of laser capable of inducing trans-epithelial injury. In another embodiment, the epidermal disruption is performed with a felt wheel. In another embodiment, the epidermal disruption is performed with a surgical tool. In another embodiment, the epidermal disruption is performed with any other tool known in the art that is capable of epidermal disruption. In another embodiment, the epidermal disruption comprises use of a micro-dermabrasion device. In another embodiment, the epidermal disruption comprises a burn treatment.

In another embodiment, the epidermal disruption comprises a disruption of a follicle of said epidermis and a disruption of an interfollicular region of said epidermis. In another embodiment, the epidermal disruption comprises a disruption of a follicle of said epidermis and does not comprise a disruption of an interfollicular region of said epidermis.

In another embodiment, the epidermal disruption comprises a light-based method. In another embodiment, the epidermal disruption comprises irradiation with visible light. In another embodiment, the epidermal disruption comprises irradiation with infrared light. In another embodiment, the epidermal disruption comprises irradiation with ultraviolet radiation. In another embodiment, the epidermal disruption comprises orthovoltage irradiation. In another embodiment, the epidermal disruption comprises X-ray irradiation. In another embodiment, the epidermal disruption comprises any other type of irradiation known in the art.

In another embodiment, the epidermal disruption is performed by mechanical means. In another embodiment, "mechanical means" refers to abrading. In another embodiment, the term refers to wounding. In another embodiment, the term refers to ultrasound. In another embodiment, the term refers to radio-frequency. In another embodiment, the term refers to ab electrical process or the use of an electrical current. In another embodiment, the term refers to electroporation. In another embodiment, the term refers to excision. In another embodiment, the term refers to tape-stripping. In another embodiment, the term refers to microdermabrasion. In another embodiment, the term refers to the use of peels. In another embodiment, the term refers to any other type of mechanical means known in the art.

In another embodiment, the epidermal disruption comprises chemical treatment. In another embodiment, the chemical is phenol. In another embodiment, the chemical is trichloroacetic acid. In another embodiment, the chemical is ascorbic acid. In another embodiment, the chemical is any other chemical capable of epidermal disruption that is known in the art.

In another embodiment, epidermal trauma is utilized in the present invention.

In another embodiment, the epidermal disruption of the present invention creates an abrasion at least about 1-1.5 centimeters (cm) in width. In another embodiment, the abrasion is at least about 1 cm in width. In another embodiment, the abrasion is at least about 1.5 cm in width. In another embodiment, the abrasion is at least about 2 cm in width.

In another embodiment, the scalp, eyebrow, or scarred region is not contacted with a bandage or dressing following the epidermal disruption. In another embodiment, the scalp, eyebrow, or scarred region is not contacted with an ointment following the epidermal disruption. In another embodiment, the scalp, eyebrow, or scarred region is allowed to heal for a period of time without being contacted by any substance, device, ointment, etc., that is ordinarily administered to an abrasion or wound to facilitate healing. In another embodiment, the scalp, eyebrow, or scarred region is allowed to heal for a period of time without being contacted by any substance, device, ointment, etc., that is ordinarily administered to an abrasion or wound to prevent infection. In another embodiment, the period of time is the time it takes the epidermal disruption to heal. In another embodiment, the period of time is any time or range of times between 2 days and 3 weeks.

In one embodiment, "following" refers to a period of time of about 2 days. In another embodiment, "following" refers to a period of time of about 3 days. In another embodiment, "following" refers to a period of time of about 4 days. In another embodiment, "following" refers to a period of time of about 5 days. In another embodiment, "following" refers to a period of time of about 7 days. In another embodiment, "following" refers to a period of time of about 10 days. In another embodiment, "following" refers to a period of time of about 2 weeks. In another embodiment, "following" refers to a period of time of about 3 weeks.

In another embodiment, the present invention further comprises the step of depilating the scalp, eyebrow, or scarred region. As provided herein, the findings of Example 6 show that the efficiency of EDIHN can be enhanced by depilation prior to abrasion or wound induction.

In another embodiment, the depilation is epilation. In another embodiment, the depilation comprises the step of waxing. In another embodiment, the depilation comprises the step of plucking. In another embodiment, the depilation comprises the use of an abrasive material. In another embodiment, the depilation comprises the use of a laser. In another embodiment, the depilation comprises the use of electrolysis. In another embodiment, the depilation comprises the use of a mechanical device. In another embodiment, the depilation comprises the use of thioglycolic acid. In another embodiment, the depilation comprises the use of any other method of depilation or epilation known in the art.

In another embodiment, the present invention further comprises the step of administering a topical retinoid to the scalp, eyebrow, or scarred region. In one embodiment, the topical retinoid induces resting (telogen) HF in the scalp, eyebrow, or scarred region to enter anagen.

In another embodiment, the additional step (depilation or administration of a retinoid) is performed prior to the step of disrupting the epidermis. In another embodiment, the additional step is performed following the step of disrupting the epidermis, but prior to the addition of the minoxidil. In another embodiment, the additional step is performed concurrently with the addition of the minoxidil. In another embodiment, the additional step is performed following the addition of the minoxidil.

In another embodiment, the additional step is performed between about two days and about three weeks before the step of abrading. In another embodiment, the additional step is performed about two days before the step of abrading. In another embodiment, the additional step is performed about three days before the step of abrading. In another embodiment, the additional step is performed about four days before the step of abrading. In another embodiment, the additional step is performed about one week before the step of abrading. In another embodiment, the additional step is performed about ten days before the step of abrading. In another embodiment, the additional step is performed about two weeks before the step of abrading. In another embodiment, the additional step is performed about three weeks before the step of abrading.

In another embodiment, the present invention further comprises the step of contacting the scalp, eyebrow, or scarred region with an inductive cell capable of inducing an epidermal cell to differentiate into an HF cell. In another embodiment, the HF cell is an HF stem cell.

In another embodiment, the inductive cell is a dermal papilla cell. In another embodiment, the inductive cell is a follicular papilla cell. In another embodiment, the inductive cell is a dermal sheath cell. In another embodiment, the inductive cell is a cell that has been genetically modified; for example, with a gene encoding a factor that activates one of the proteins or pathways shown to be up-regulated in HF stem cells (Table 4). In one embodiment, the factor is hedgehog. In another embodiment, the factor is a DP cell protein. In another embodiment, the factor is wingless/ int (wnt). In another embodiment, the factor is a Noggin protein. In another embodiment, the factor is a bone morphogenic protein (BMP). In another embodiment, the factor is a fibroblast growth factor (FGF). In another embodiment, the factor is a transforming growth factor beta (TGF-beta) protein. In another embodiment, the factor is sonic hedgehog protein. In another embodiment, the factor is a neurotropin. In another embodiment, the factor is any other factor known in the art that can contribute to induction of an epidermal cell to differentiate into an HF cell.

In an embodiment, the Hedgehog protein has the sequence set forth in GenBank Accession No. NM_000193. In another embodiment, the Hedgehog protein has a sequence selected from those set forth in GenBank entries L38518 and NP_000184. In another embodiment, the Hedgehog protein has any other Hedgehog sequence known in the art. In another embodiment, the Hedgehog protein has any other sonic Hedgehog sequence known in the art.

In an embodiment, the Wnt protein has the sequence of SEQ ID No: 280 (GenBank Accession No: BC008811). In another embodiment, the Wnt protein has a sequence selected from the sequences set forth in GenBank entries NM_004625, D83175, U53476, and NP _004616. In another embodiment, the Wnt protein is a Wnt7 protein. In another embodiment, the Wnt protein is a Wnt7a protein. In another embodiment, the Wnt protein is Wnt1 protein. In another embodiment, the Wnt protein is a Wnt3 protein. In another embodiment, the Wnt protein is a Wnt3a protein. In another embodiment, the Wnt protein is a Wnt10 protein. In another embodiment, the Wnt protein is a WntlOa protein. In another embodiment, the Wnt protein is a WntlOb protein. In another embodiment, the Wnt protein is encoded by a nucleic acid molecule having a sequence set forth in the one of the above GenBank entries. In another embodiment, a biologically active fragment of a Wnt protein is utilized.

In an embodiment, the Noggin protein has the sequence of SEQ ID No: 276 (GenBank Accession No: NM_005450). In another embodiment, the Noggin protein has a sequence selected from the sequences set forth in GenBank entries BC034027 and U31202. In another embodiment, the Noggin protein is encoded by a nucleic acid molecule having a sequence set forth in the one of the above GenBank entries. In another embodiment, a biologically active fragment of a Noggin protein is utilized.

In an embodiment, TGF-beta1 protein has the sequence set forth in GenBank Accession No. BC000125. In another embodiment, TGF-beta1 protein has a sequence selected from those set forth in GenBank entries BC001180, BC022242, and NM_000660. In another embodiment, the TGF-beta1 protein has any other TGF-beta1 sequence known in the art.

In an embodiment, TGF-beta3 protein has the sequence set forth in GenBank Accession No. J03241. In another embodiment, TGF-beta3 protein has a sequence selected from those set forth in GenBank entries NM_003239, BC018503, BT007287 and X14149. In another embodiment, the TGF-beta3 protein has any other TGF-beta3 sequence known in the art.

In another embodiment, the inductive cell has been genetically modified with a gene encoding a factor that represses one of the proteins or pathways shown to be down-regulated in HF stem cells (Table 5). In another embodiment, the inductive cell has been genetically modified with a gene encoding a factor that activates one of the proteins or pathways shown to be up-regulated in HF stem cells upon their activation.

In another embodiment, the inductive cell is an autologous cell. In another embodiment, the inductive cell is an allogenic cell.

In another embodiment, the inductive cell is derived from a mesenchymal stem cell. In another embodiment, the inductive cell is derived from a mesodermal progenitor cell. In another embodiment, the inductive cell is derived from a hematopoietic stem cell. In another embodiment, the inductive cell is derived from an embryonic stem cell. In another embodiment, the inductive cell is derived from an embryonic carcinoma cell. In another embodiment, the inductive cell is any other type of cell known in the art with inductive properties for an epidermal cell.

In another embodiment, the epidermal cell (e.g. the epidermal cell that is induced to differentiate into an HF cell) is an epidermal stem cell. In another embodiment, the epidermal cell is a bulge cell. In another embodiment, the epidermal cell is any other type of cell known in the art that can be induced to differentiate into an HF stem cell.

In another embodiment, the present invention further comprises the step of contacting the scalp, eyebrow, or scarred region with an anti-androgen compound. In one embodiment, the anti-androgen compound is finasteride. In another embodiment, the anti-androgen compound is Fluridil^{®}. In another embodiment, the anti-androgen compound is dutasteride. In another embodiment, the anti-androgen compound is spironolactone. In another embodiment, the anti-androgen compound is cyproterone acetate. In another embodiment, the anti-androgen compound is bicalutamide. In another embodiment, the anti-androgen compound is flutamide. In another embodiment, the anti-androgen compound is nilutamide. In another embodiment, the anti-androgen compound is an inhibitor of an androgen receptor. In another embodiment, the anti-androgen compound is any other anti-androgen compound known in the art.

In another embodiment, the present invention further comprises the step of contacting the scalp, eyebrow, or scarred region with an estrogen compound. In another embodiment, the present invention further comprises the step of contacting the scalp, eyebrow, or scarred region with an estrogen receptor agonist. In another embodiment, the present invention further comprises the step of contacting the scalp, eyebrow, or scarred region with an estrogen analogue. In one embodiment, the estrogen analogue is estradiol. In another embodiment, the estrogen analogue is 17 beta-estradiol. In another embodiment, the estrogen analogue is 17 alpha-estradiol. In another embodiment, the estrogen analogue is ZYC3. In another embodiment, the estrogen compound, estrogen receptor agonist, or estrogen analogue is any other estrogen compound, estrogen receptor agonist, or estrogen analogue known in the art.

In another embodiment, the present invention further comprises the step of contacting the scalp, eyebrow, or scarred region with an inhibitor of an EGF protein. In another embodiment, the present invention further comprises the step of contacting the scalp, eyebrow, or scarred region with an inhibitor of an EGFR. In another embodiment, the present invention further comprises the step of contacting the scalp, eyebrow, or scarred region with a compound that reduces an expression of an EGF protein or an EGFR.

In an embodiment, the inhibitor of an EGF or an EGF receptor is panitumumab. In another embodiment, the inhibitor is AG1478. In another embodiment, the inhibitor is nimotuzumab. In another embodiment, the inhibitor is an antibody that binds EGF or EGFR. In another embodiment, the inhibitor is HuMax-EGFR^{®} (Genmab, Copenhagen, Denmark). In another embodiment, the inhibitor is cetuximab. In another embodiment, the inhibitor is IMC 11F8. In another embodiment, the inhibitor is matuzumab. In another embodiment, the inhibitor is SC 100. In another embodiment, the inhibitor is ALT 110. In another embodiment, the inhibitor is PX 1032. In another embodiment, the inhibitor is BMS 599626. In another embodiment, the inhibitor is MDX 214. In another embodiment, the inhibitor is PX 1041. In another embodiment, the inhibitor is any other inhibitor of an EGF or an EGF receptor known in the art.

In another embodiment, the EGF or EGFR antagonist is a carboxypeptidase inhibitor from potato (PCI) protein or a homologue, fragment or mimetic thereof. In another embodiment, the EGF or EGFR antagonist is a sprouty protein or a homologue, fragment or mimetic thereof. In another embodiment, the EGF or EGFR antagonist is an Argos protein or a homologue, fragment or mimetic thereof. In another embodiment, the EGF or EGFR antagonist is a lefty protein or a homologue, fragment or mimetic thereof. In another embodiment, the EGF or EGFR antagonist is an antibody that recognizes EGF or EGFR, or a fragment or mimetic thereof. In another embodiment, the EGF or EGFR antagonist is small molecule inhibitor that binds and reduces the activity of EGF or EGFR. In another embodiment, the EGF or EGFR antagonist is CRM197. In another embodiment, the EGF or EGFR antagonist is IMC-C225 (ImClone Systems, New York, NY). In another embodiment, the EGF or EGFR antagonist is any other antagonist of EGF or EGFR known in the art.

In another embodiment, the present invention further comprises the step of contacting the scalp, eyebrow, or scarred region with a Hedgehog protein. In another embodiment, the present invention further comprises the step of contacting the scalp, eyebrow, or scarred region with a nucleotide encoding a Hedgehog protein. In another embodiment, the present invention further comprises the step of contacting the scalp, eyebrow, or scarred region with an activator of a Hedgehog protein.

In another embodiment, the present invention further comprises the step of contacting the scalp, eyebrow, or scarred region with a lithium compound. In one embodiment, the lithium compound contains a lithium ion. In another embodiment, the lithium compound contains a lithium atom.

In another embodiment, the present invention further comprises the step of contacting the scalp, eyebrow, or scarred region with (2'Z,3'E)-6- bromoindirubin-3'-oxime (BIO). In another embodiment, the present invention further comprises the step of contacting the scalp, eyebrow, or scarred region with any other compound known in the art that is capable of inducing an epidermal cell to differentiate into an HF stem cell.

In one embodiment, the compound is administered systemically. In another embodiment, the compound is administered topically. In another embodiment, the compound is administered to the site of the abrasion. In another embodiment, the compound is administered to the site of the wound induction. In another embodiment, the compound is administered to the site of the depilation. In another embodiment, the compound is administered during wound healing. In another embodiment, the compound is administered prior to HF neo-genesis. In another embodiment, the compound is administered during HF neo-genesis.

The present application also discloses a kit, comprising a tools and/or a compound suitable for performing the present invention.

### Pharmaceutical Compositions

In another embodiment, the present invention comprises administering a pharmaceutical composition comprising minoxidil and a pharmaceutically acceptable carrier.

The pharmaceutical compositions can, in another embodiment, be administered to the scalp, eyebrow or scarred region of a subject by any method known to a person skilled in the art, such as topically, parenterally, transdermally, intradermally, or subcutaneously.

In another embodiment, the pharmaceutical compositions are administered topically to body surfaces and are thus formulated in a form suitable for topical administration. Suitable topical formulations include gels, ointments, creams, lotions, drops. For topical administration, the compound is prepared and applied as solutions, suspensions, or emulsions in a physiologically acceptable diluent with or without a pharmaceutical carrier.

In another embodiment, the pharmaceutical compositions are administered by subcutaneous implantation of a pellet. In another embodiment, the pellet provides for controlled release of the compound.

For liquid formulations, pharmaceutically acceptable carriers are, in another embodiment, aqueous or non-aqueous solutions, suspensions, emulsions or oils. Examples of non-aqueous solvents are propylene glycol, polyethylene glycol, and injectable organic esters such as ethyl oleate. Aqueous carriers include, in another embodiment, water, alcoholic/aqueous solutions, emulsions or suspensions, including saline and buffered media. Examples of oils are those of petroleum, animal, vegetable, or synthetic origin, for example, peanut oil, soybean oil, mineral oil, olive oil, sunflower oil, and fish-liver oil.

In one embodiment, the pharmaceutical compositions are controlled-release compositions, i.e. compositions in which the compound is released over a period of time after administration. Controlled- or sustained-release compositions include, in another embodiment, formulation in lipophilic depots (e.g. fatty acids, waxes, oils). In another embodiment, the composition is an immediate-release composition, i.e. a composition in which all the compound is released immediately after administration.

### EXPERIMENTAL DETAILS SECTION

### EXAMPLE 1

### DEPILATION AND EPIDERMAL ABRASION CAUSES DE NOVO HAIR FOLLICLE

### FORMATION

### MATERIALS AND EXPERIMENTAL METHODS

### Depilation and epidermal abrasion

Mice were anesthetized with an injection of sodium pentobarbital before the hair on the back was clipped and depilated with Nair (Carter-Wallace, New York, NY), then epidermis was removed using a rotating felt wheel as described by Argyris T, J Invest Dermatol, 75: 360-362, 1980). After scrubbing with 70% ethanol and drying under an incandescent lamp, the basal and supra-basal layers in an area of (1.5 cm)² cm of the interfollicular epidermis were removed by careful abrasion with a felt wheel mounted on a Dremel Moto-tool (Racine, WI). After abrasion, the skin was shiny and smooth, and there was no blood. One day later, the abraded area was covered by a fibrin crust, which fell off after 3-7 days, exposing the newly regenerated epidermis. A group of control mice was sacrificed immediately after abrasion to confirm microscopically the complete removal of the interfollicular epidermis.

### Immunohistochemistry

Skin samples were fixed in PBS-buffered 10% formalin. Six-micron thick paraffin sections were cut and stained, where applicable, with antibodies.

### BrdU labeling

The protocol described by Bickenbach and colleagues (Bickenbach et al, Cell Tiss Kinet 19: 325-333, 1986; Bickenbach et al, Exp Cell Res 244, 184-195, 1998) was used. Mice were injected with 50 milligrams per kilogram (mg/kg) bodyweight 5-bromo-2'-deoxyuridine (BrdU) every 12 hours for a total of four injections.

### RESULTS

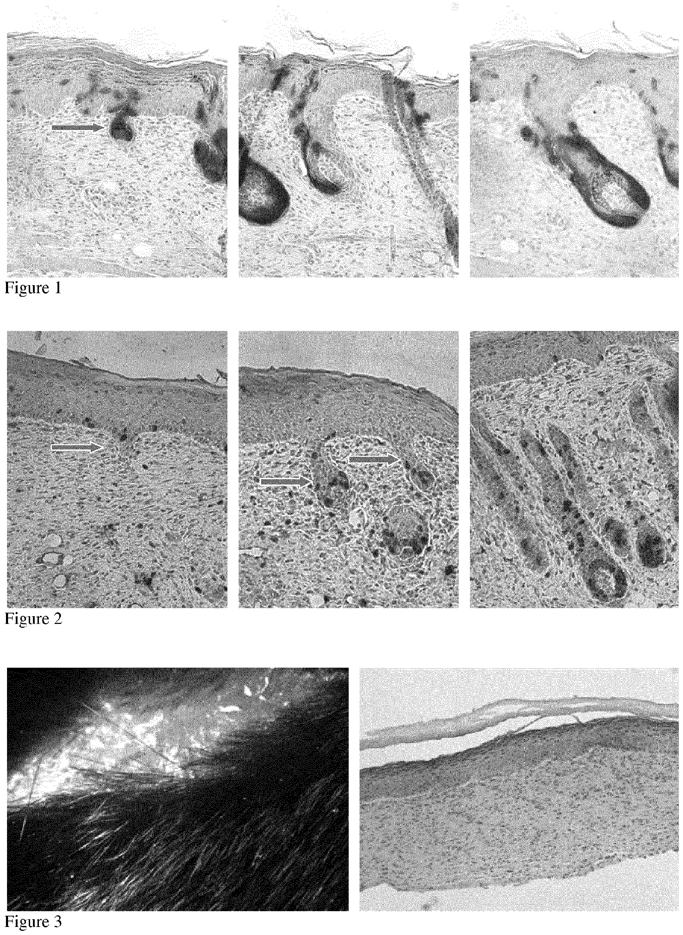

An area of the backs of 50-day old mice was subjected to depilation and removal of the epidermis using a rotating felt wheel. Fifteen days later, HF placodes, hair germs and other signs of follicle neogenesis were present (Figure 1; arrow indicates a hair germ). Morphology of the follicles was similar to embryonic follicle development. To further characterize proliferation in the new follicles, the skin was labeled with BrdU 60 minutes before sacrifice. As depicted in Figure 2, the proliferation pattern was similar to developing follicles in the embryo.

These findings demonstrate that (a) disruption of the epidermis causes generation of new HF, and that this generation of new HF can occur (b) in adult subjects and (c) during telogen (50-day-old mice are in the second telogen stage of the hair cycle).

### EXAMPLE 2

### INDUCTION OF A LARGE EXCISIONAL WOUND. BUT NOT A SMALL PUNCH WOUND. CAUSES DE NOVO HAIR FOLLICLE FORMATION

### MATERIALS AND EXPERIMENTAL METHODS

### Punch wound and excisional wound induction

The backs of 21-day-old mice were depilated as described for Example 1 and sterilized with alcohol, followed by 1% iodine solution. Punch wounds, 4 mm in diameter, were induced using a dermal biopsy punch, down to, but not through, the muscle fascia. Excisional wounds were full thickness and 1 cm in diameter; skin and panniculus carnosus was excised using fine surgical scissors.

### RESULTS

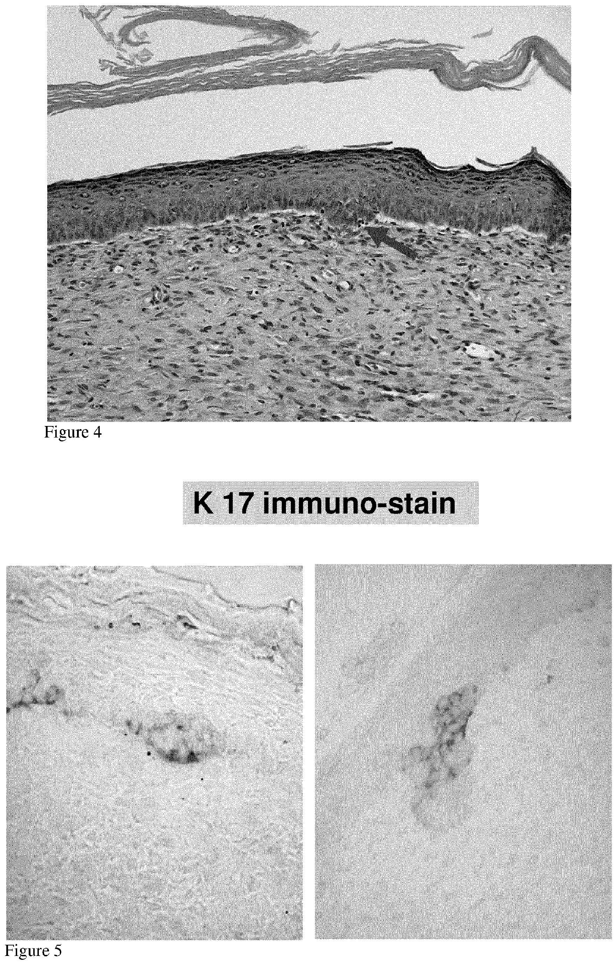

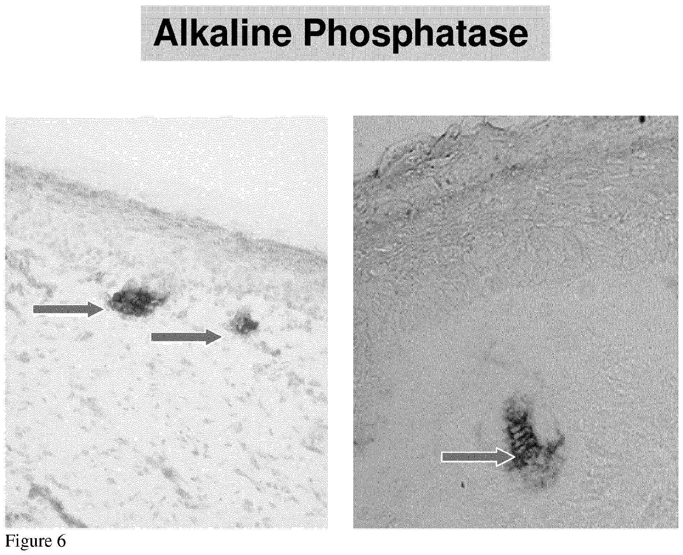

To test whether wounding could induce HF formation, punch wounds or excisional wounds were induced in mice. Both types of wounds exhibited contraction and re-epithelialization following wound induction; however, unlike the mice receiving punch wounds, the mice receiving excisional wounds also exhibited scar formation within 10 days of wound induction (Figure 3, left panel). No follicles were evident at this time point (Figure 3, right panel). 12 days after wound induction, hair germs, with similar morphology to fetal hair germs, were observed in the wound site, following BrdU pulse labeling (Figure 4). Several markers were used to verify that the observed structures were HF. The structures exhibited staining with anti-keratin 17 (K 17), an HF marker (Figure 5), and staining with anti-alkaline phosphatase at the 12 day time point verified that the structures had dermal papilli containing fibroblasts, as expected for HF (Figure 6; HF at earlier and later stages are depicted in the left and right panels, respectively).

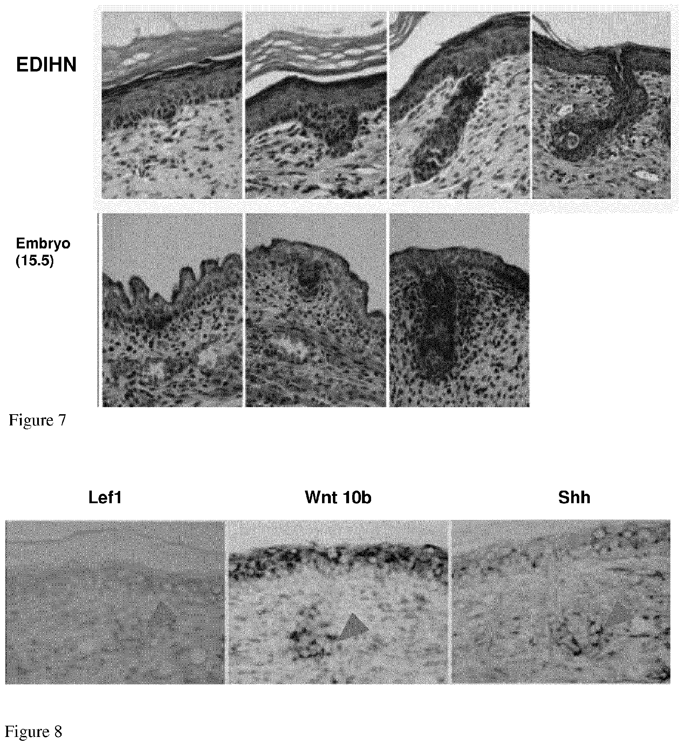

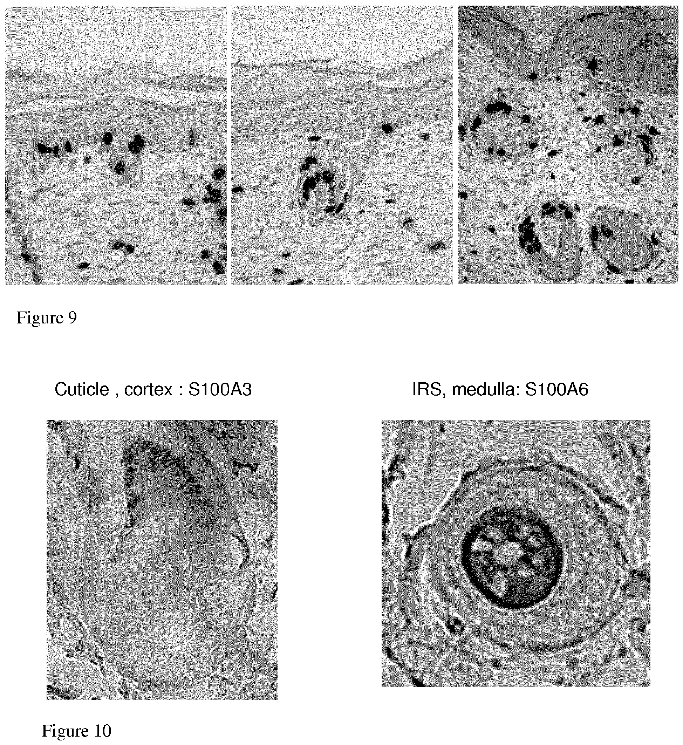

The HF generated by wound induction were further characterized by morphological comparison to embryonic HF, following BrdU staining; a clear correspondence in morphology was observed at various stages (Figure 7). In addition, several markers of embryonic HF development, namely Lef1, wingless/ int (Wnt) 10b, and sonic hedgehog (Shh), were also induced in the epidermal disruption-induced HF neogenesis (EDIHN) (Figure 8). Additional BrdU staining (Figure 9) and staining for HF markers S100A3 and S100A6 (Figure 10; left panel: tissue section parallel to HF axis; right panel: cross-sectional view of follicle) provided further verification that the development of the EDIHN follicles closely paralleled embryonic HF development.

These findings provide further evidence that disruption of the epidermis causes generation of new HF, and that this generation of new HF can occur (b) in adult subjects and (c) during telogen (21-day-old mice are in the first telogen stage of the hair cycle).

### EXAMPLE 3

### EDIHN-INDUCED HAIR FOLLICLES GENERATE HAIRS

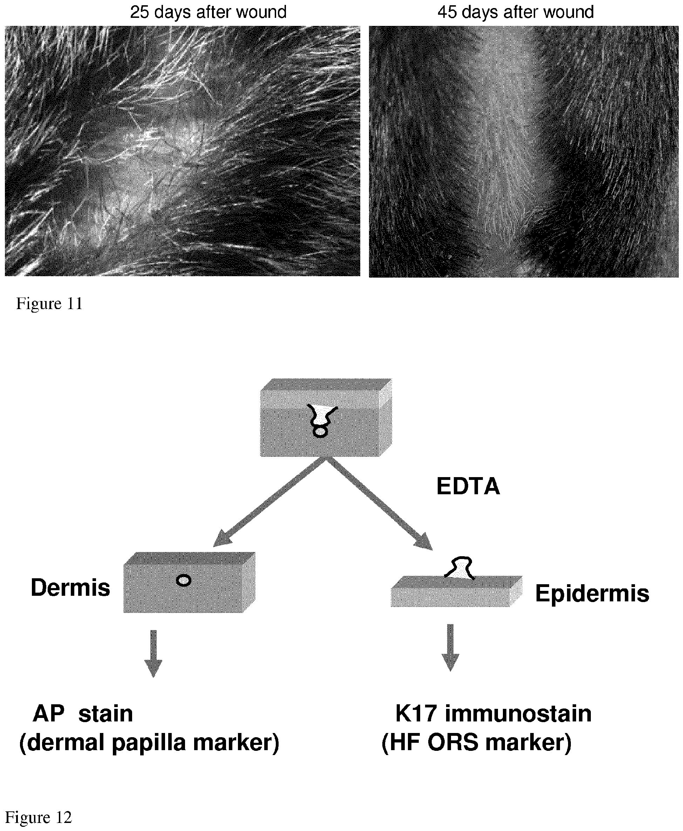

At 25 and 45 days after wound induction, wound sites contained new hairs (Figure 11, left and right panels, respectively). New hairs appeared to lack pigmentation, except when the wnt pathway was inhibited, using Dkk-1 (Dickkopf-1) during the first nine days after wounding (see Example 10).

These findings indicate that EDIHN-induced HF function normally; i.e. are capable of generating hairs.

### EXAMPLE 4

### EDIHN HAIR FOLLICLES RETAIN THE ABILITY TO ENTER INTO CYCLICAL HAIR GROWTH

### MATERIALS AND EXPERIMENTAL METHODS

### BrdU labeling

50 mg/kg bodyweight BrdU (Sigma) was injected twice per day for 3 days beginning 20 days after wounding. BrdU was detected 40 days after wounding (17 day chase).

### Whole mounting and immunofluorescence

HF whole mounts were obtained by incubating fresh skin with EDTA (20mM in PBS) at 37 °C overnight, then separating the epidermis and dermis. Epidermis was then fixed in 10% formalin for 10 min, room temperature (RT). Dermis was fixed in acetone overnight, RT. After rinsing with PBS, whole mounts were stained with antibodies for immunohistochemistry (schematically depicted in Figure 12) and were imaged using a Leica confocal microscope.

### RESULTS

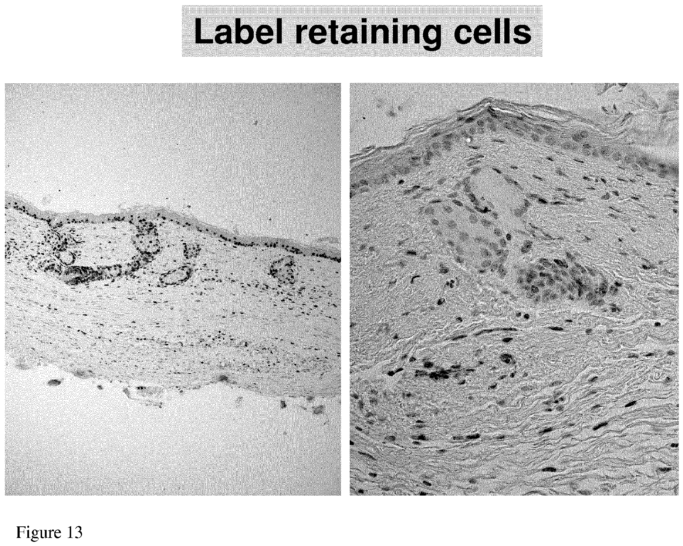

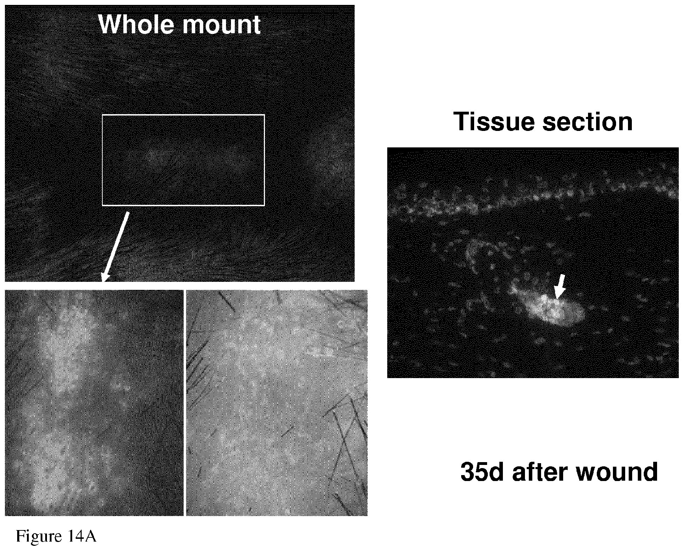

To determine whether EDIHN-induced HF contain normal levels of HF stem cells, mouse skin was examined for the presence of label-retaining cells at 21 days after wound induction. Retention of BrdU during a long chase period is, under these conditions, one of the hallmarks of HF stem cells. Normal numbers and placement of label-retaining cells (in the bulge of the HF) were observed (Figure 13). To verify that the label-retaining cells were HF stem cells, K15-eGFP mice were utilized. In these mice, eGFP (enhanced green fluorescent protein) is expressed from the K15 promoter; thus, expression of eGFP identifies HF stem cells. As depicted in Figure 14A, eGFP-expressing cells were observed in in tissue sections (right side) of newly formed hair follicles 35 days following wound induction. eGFP-expressing cells were also seen in the epidermis whole mounts (bottom, far left panel) indicating the conversion of epidermal cells into cells with hair follicle stem cell characteristics, ([bottom, second from left] panel is same as [bottom, far left] panel but viewed under white light) This finding shows that the observed label-retaining cells exhibited HF stem cell properties.

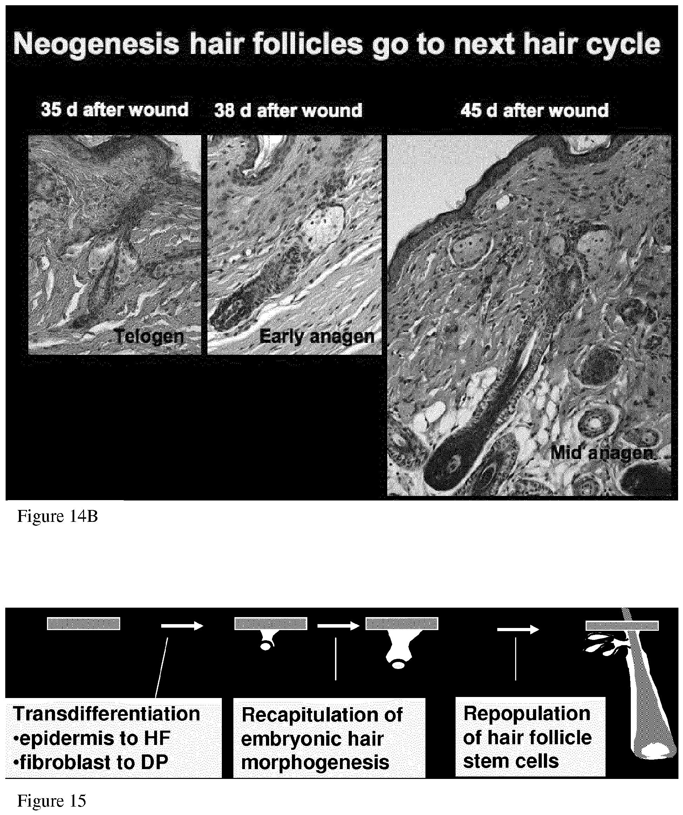

To determine whether EDIHN-induced HF cycle normally, mounts were prepared from additional mice at 35, 38 and 45 days after wounding. As depicted in Figure 14B, the EDIHN-induced HF entered the resting phase, telogen, and then re-entered a new anagen stage.

In summary, the findings of this Example show that EDIHN-induced HF contain HF stem cells, as do embryonically generated HF. The presence of the HF stem cells shows that EDIHN-induced HF retain the ability to enter into cyclical hair growth in the same manner as embryonically generated HF. The findings also show that wounding induces epidermal cells to assume a hair follicle stem cell state (expressing K15-eGFP). This model is shown schematically in Figure 15. The findings of Examples 2, 3, and 4 show that EDIHN-induced HF are fully functional and thus able to restore hair growth to a subject in need.

### EXAMPLE 5

### EDIHN-INDUCES NEW HAIR FOLLICLES IN MICE AT THE TELOGEN STAGE OF THE HAIR CYCLE

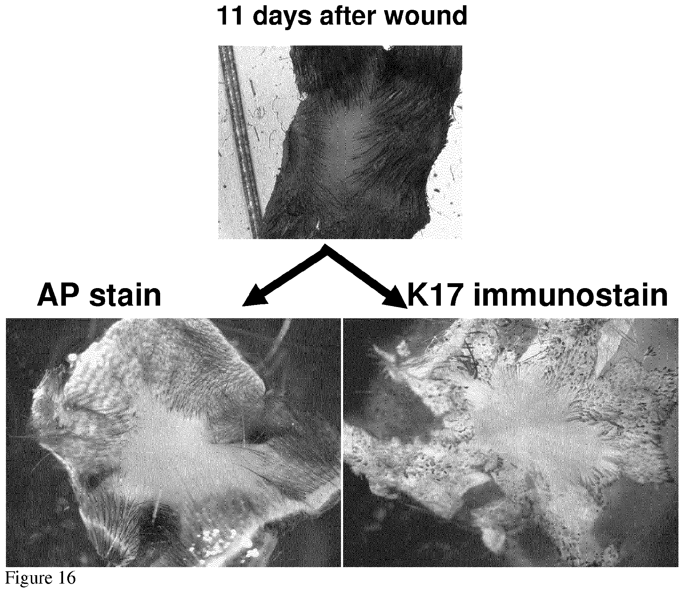

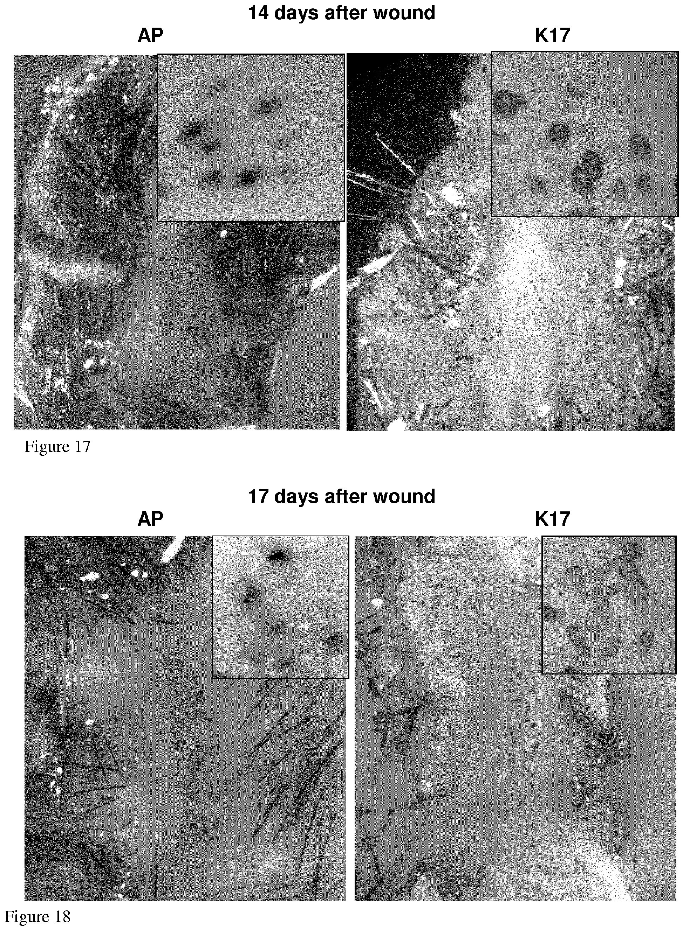

To determine whether EDIHN was induced new hair follicles in mice wounded at the telogen stage of the hair cycle, 21-day-old mice were subjected to EDIHN using a 1-cm excisional wound, as described in Example 2. Skin was then examined by whole-mount assay for indications of new HF. As depicted in Figure 16, after 11 days, new HF were not evident by macroscopic examination (top panel), AP staining of the dermis (bottom left panel), or K17 staining of the epidermis (bottom right panel). After 14 days, as depicted in Figure 17, dermal papilla cells were detected in the dermis (left panel) and HF stem cells in the epidermis (right panel), demonstrating that new follicles were being formed. After 17 days, the new follicles were more developed, as shown by examination of the dermis and epidermis (Figure 18, left and right panels, respectively). This method induced formation of an average of 49 new follicles in the wound, a number that was consistent over three separate experiments, as depicted in Table 1.

**Table 1. Results of three separate experiments performed on 21-day-old mice.**

| Sample | Expt 1 | Expt 2 | Expt 3 | | |
|---|---|---|---|---|---|
| 1 | 24 | 70 | 55 | | |
| 2 | 29 | 52 | 25 | | |
| 3 | 27 | 85 | 53 | | |
| 4 | 102 | 25 | 80 | | |
| 5 | 53 | 27 | 23 | Avg of expts | Std dev of expts |
| Average | 47 | 51.8 | 47.2 | 48.67 | 2.71 |
| Std dev | 32.8 | 26.3 | 23.7 | | |

The findings of this Example demonstrate that EDIHN is capable of inducing formation of new HF in mice at the telogen stage of the hair cycle, despite that fact that these mice do not contain HF at the anagen stage during wounding.

### EXAMPLE 6

### IN ADULT MICE. INDUCTION OF ANAGEN INCREASES THE EFFICIENCY OF EDIHN

The experiment described in Example 5 was repeated with mice of different ages, and therefore at different stages of the hair cycle. To ensure that wound scarring occurred, larger wounds were in induced in the older mice. As depicted in Table 2, adult mice at telogen, such as 8-week-old mice, exhibited lower efficiencies of HF formation by EDIHN.

**Table 2. Efficiency of HF formation by EDIHN in adult mice at various stages of the hair cycle.**

| Age | Wound size | Days after wound | Mice exhibiting EDIHN | Hair cycle |
|---|---|---|---|---|
| 3 wk | 1 cm | 20 | 25/25 (100%) | Telogen |
| 4 wk | 1 cm | 20 | 5/5 (100%) | Early anagen |
| 5 wk | 1 cm | 20 | 1/2 (50%) | Anagen |
| 8 wk | 1.5 cm | 30 | 16/35 (46%) | Telogen |
| 14 wk | 1.5 cm | 30 | 1/2 (50%) | N/A^{∗} |
| 20 wk | 1.5 cm | 30 | 2/2 (100%) | N/A^{∗} |

| | | | | |
|---|---|---|---|---|
| ^{∗} The second telogen lasts approximately 40 days in mice. Thus, 14-week-old and 20- week-old mice contained a mixture of telogen and anagen HF. | | | | |

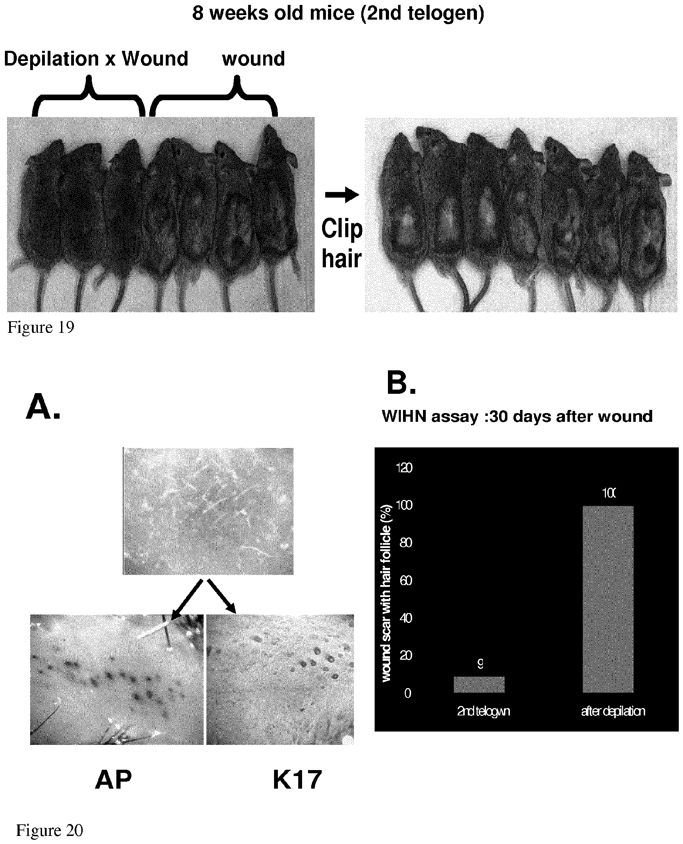

To determine whether experimental induction of anagen increased the efficiency of EDIHN, 8-week-old mice were depilated several days prior to wound induction. As depicted in Figure 19, the wounds closed similarly whether or not they were preceded by depilation. As depicted in Figure 20A-B, the depilated mice exhibited enhanced EDIHN relative to the non-depilated mice depicted in the previous Example by a factor of 11-fold.

The findings of this Example demonstrate that anagen induction enhances EDIHN. In addition, these findings show that EDIHN is capable of not only forming new HF, but also of activating anagen in pre-existing HF in the telogen stage.

### EXAMPLE 7

### EDIHN-INDUCES NEW HAIR FOLLICLES IN HUMAN SKIN MATERIALS AND EXPERIMENTAL METHODS

### Grafting

Discarded human adult scalp from the preauricular area obtained from plastic surgery was grafted onto immunodeficient (scid) mice. The graft was bandaged and allowed to heal, then was used in the wound healing study 3 months after grafting.

### RESULTS

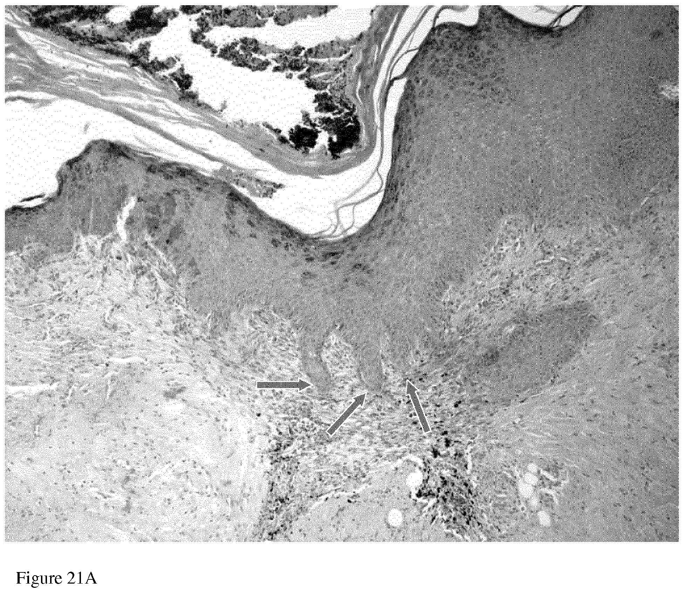

To determine whether human skin responded to EDIHN as did mouse skin, human skin was grafted onto SCID (immuno-deficient) mice and subjected to depilation by plucking and wound induction three days later. Seven days following wound induction, formation of new HF was observed in the human skin (Figure 21A; arrows indicate new HF) by hematoxylin and eosin staining of paraffin embedded tissue sections.

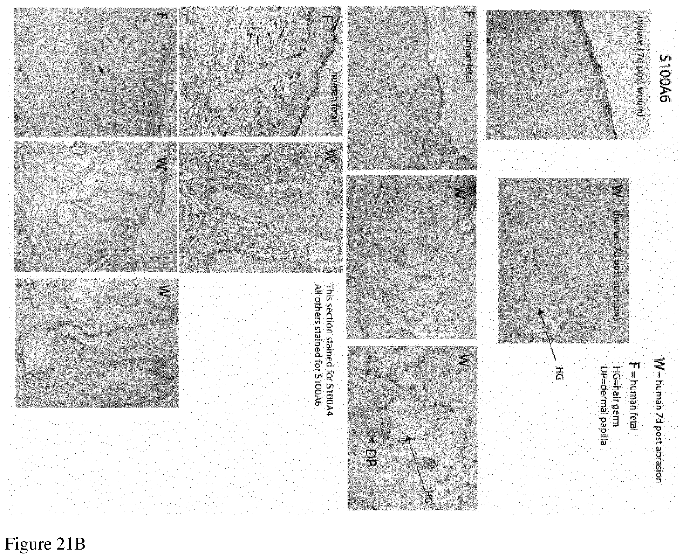

In additional experiments, adult human skin was grafted onto mice, abraded, and examined at 7 days post-abrasion. New HF were generated in the human skin, which mimicked normal hair follicle formation during fetal development, as evidenced by staining for S100A6 or S100A4 (Figure 21B).

The results of this Example show that EDIHN can be used to generate hair growth in human skin as for mouse skin.

### EXAMPLE 8

### MOLECULAR PATHWAYS ACTIVATED DURING HF STEM CELL ACTIVATION MATERIALS AND EXPERIMENTAL METHODS

### Isolation and activation of HF stem cells

K15-eGFP mice were depliated in order to induce formation of new HF. Activated hair follicle stem cells were isolated from K15-eGFP mice using fluorescence-activated cell sorting (FACS) two days after depilation and 5 µg (micrograms) total RNA from the cell population was isolated, reverse-transcribed and hybridized to an Affymetrix (Santa Clara, California) array MG_U74v2 chip. Scanned chip images were analyzed using Affymetrix Microarray Suite 5.0 and GeneSpring software (Silicon Genetics) to detect fold-change differences between activated HF stem cells (HFSCs) and non-activated (telogen) HFSCs. Values were normalized before computing fold-changes and differences between non-activated "bs-line" and activated ("expt") samples.

### RESULTS

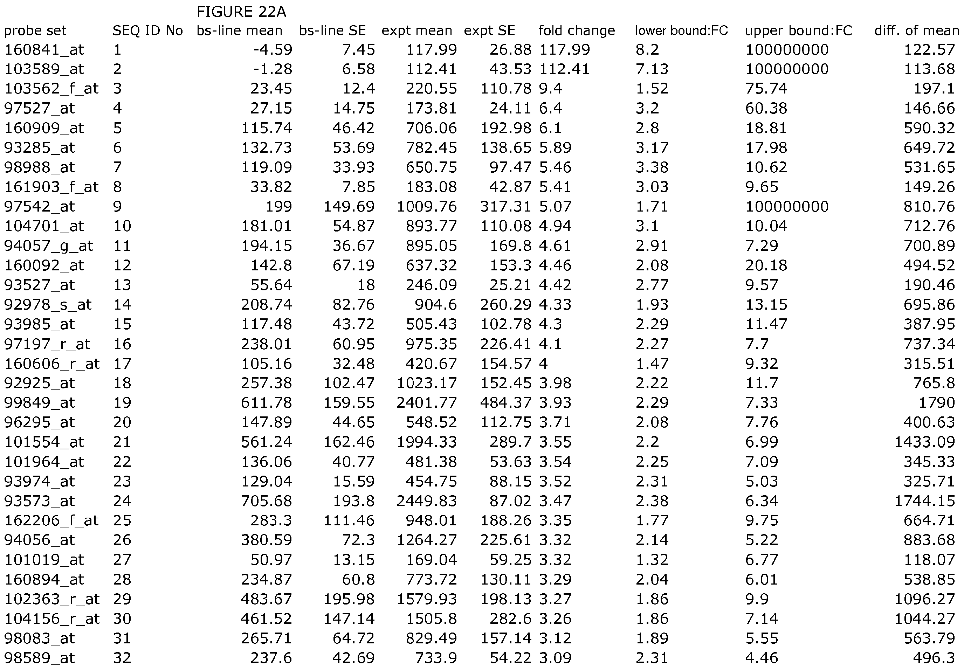

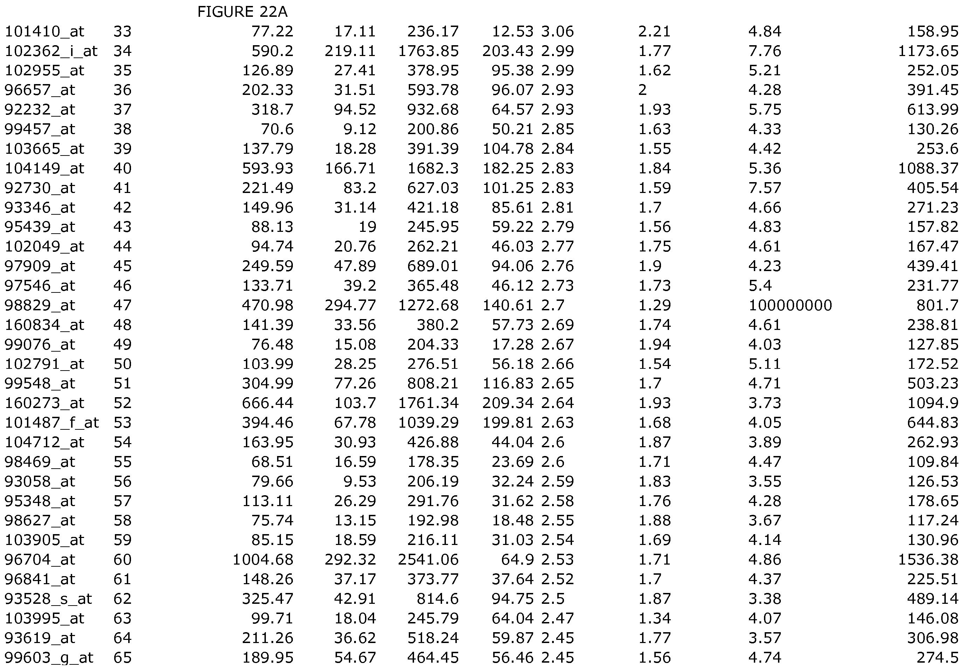

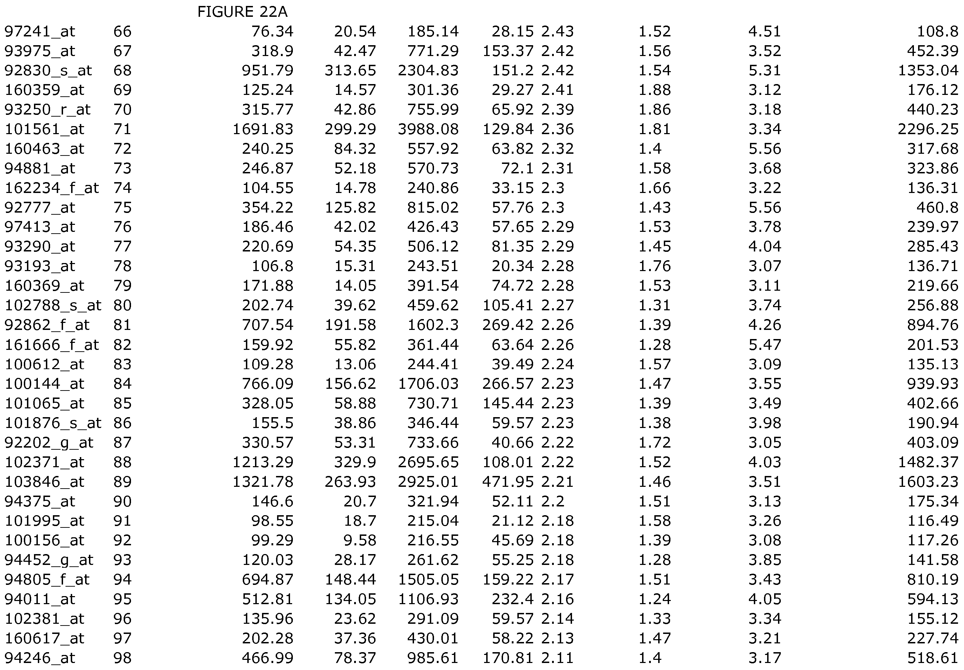

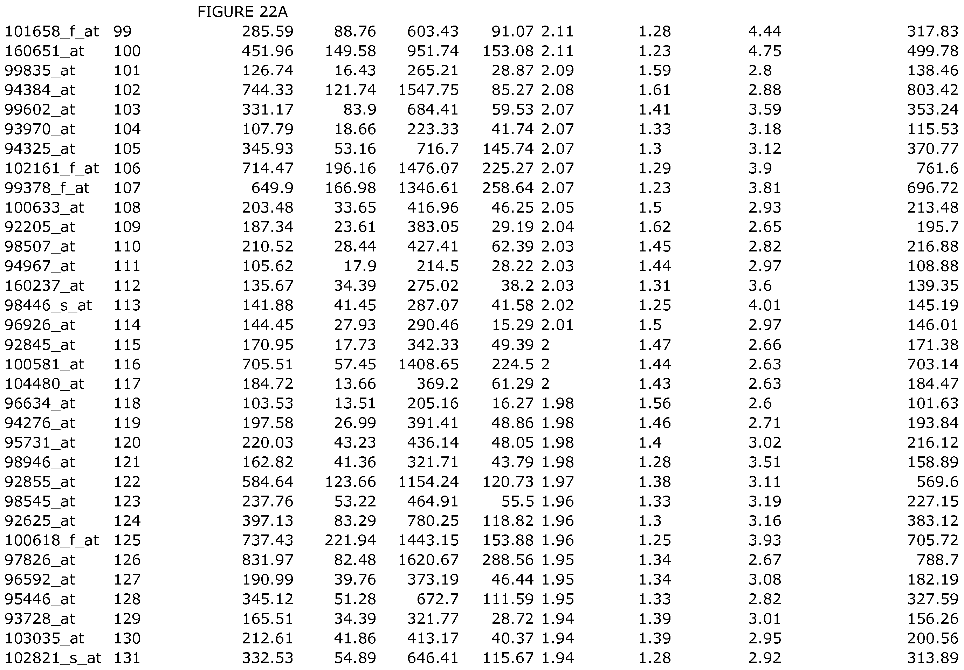

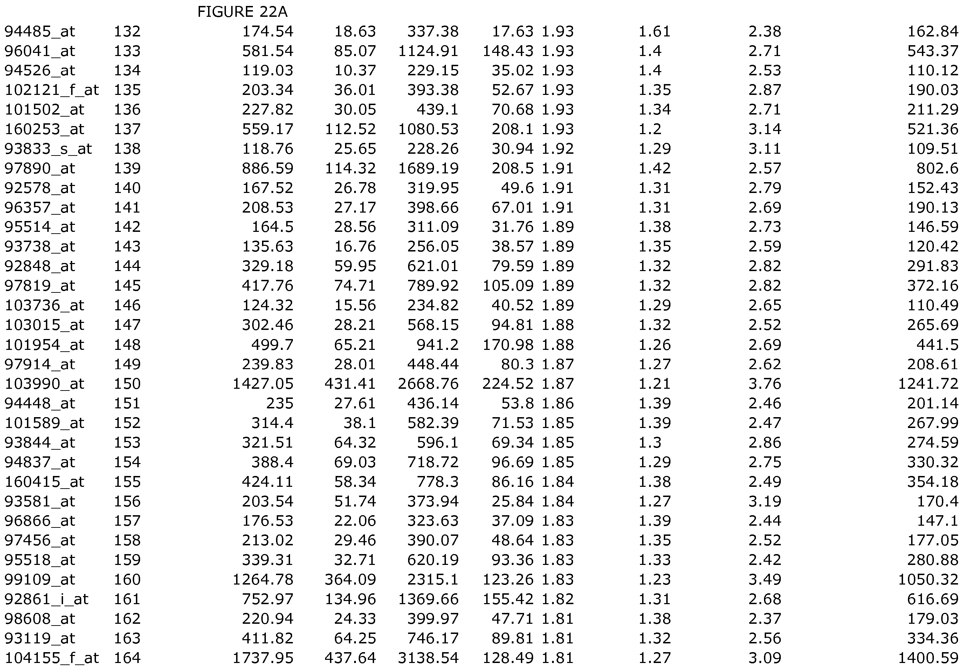

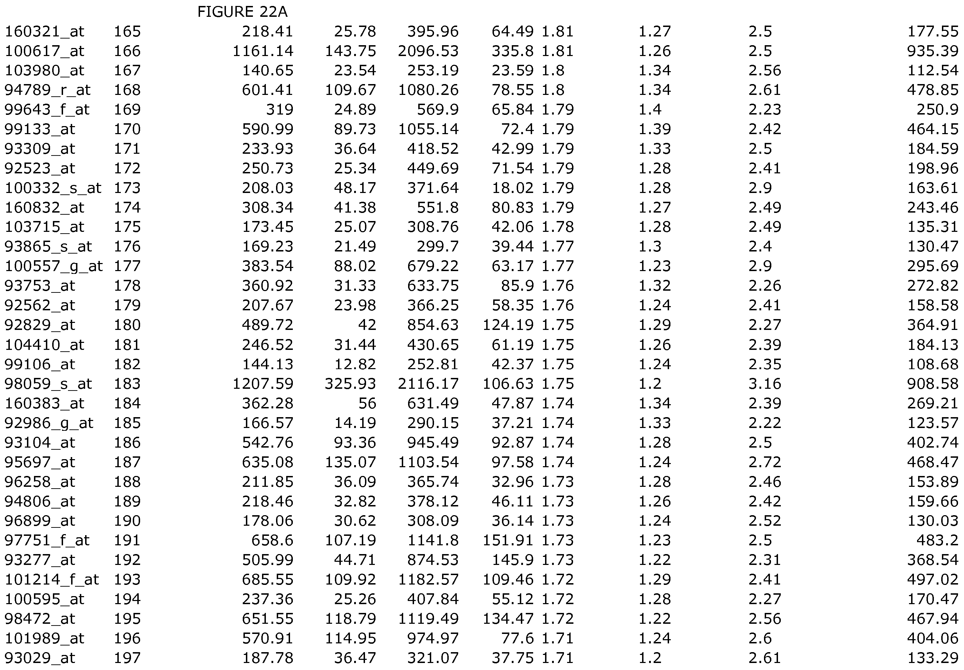

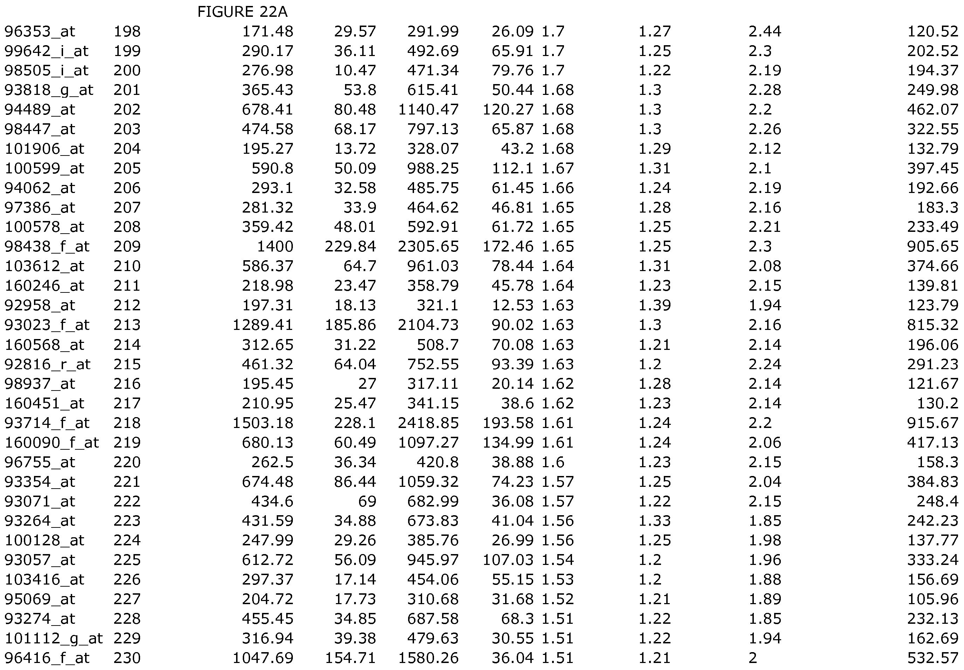

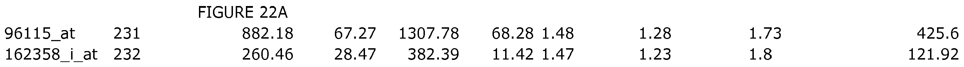

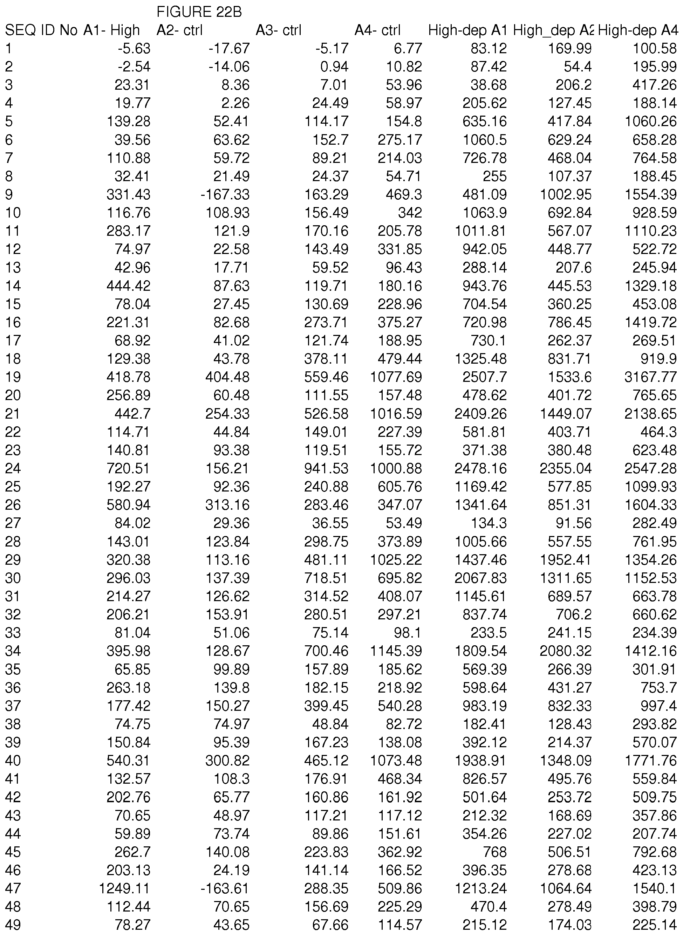

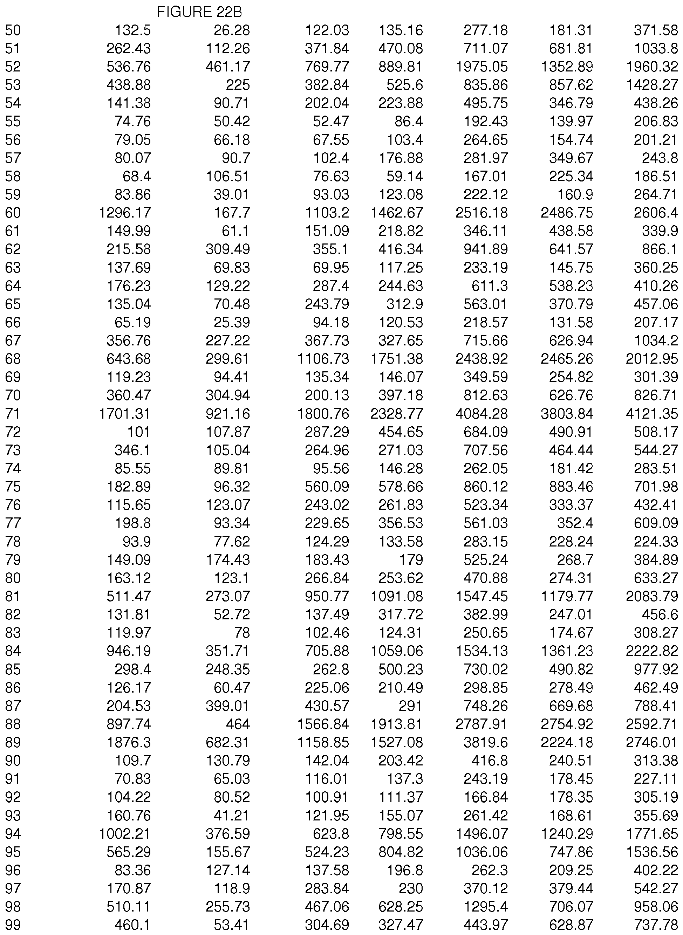

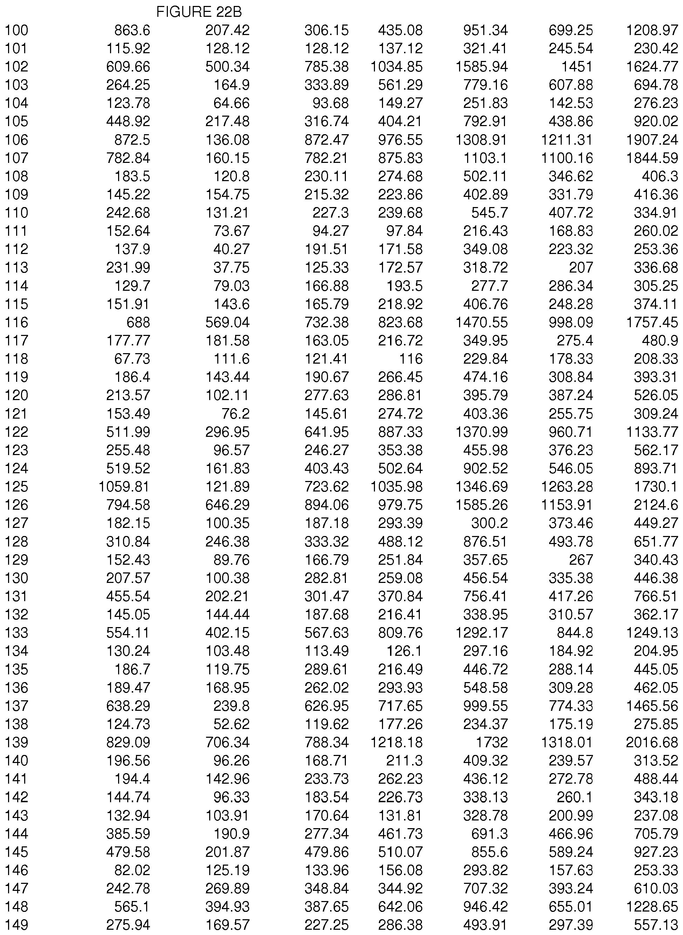

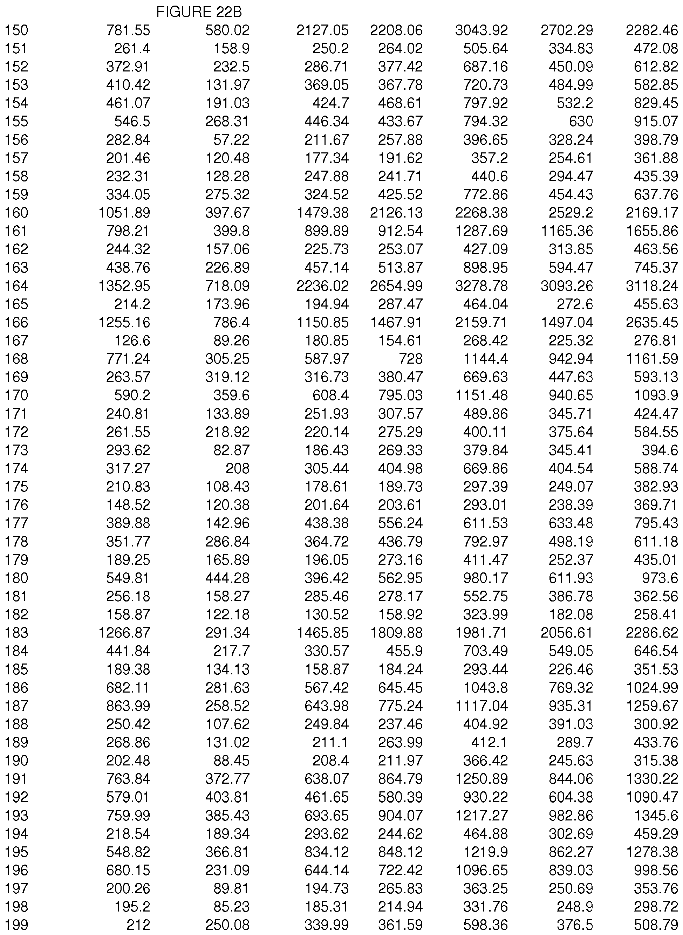

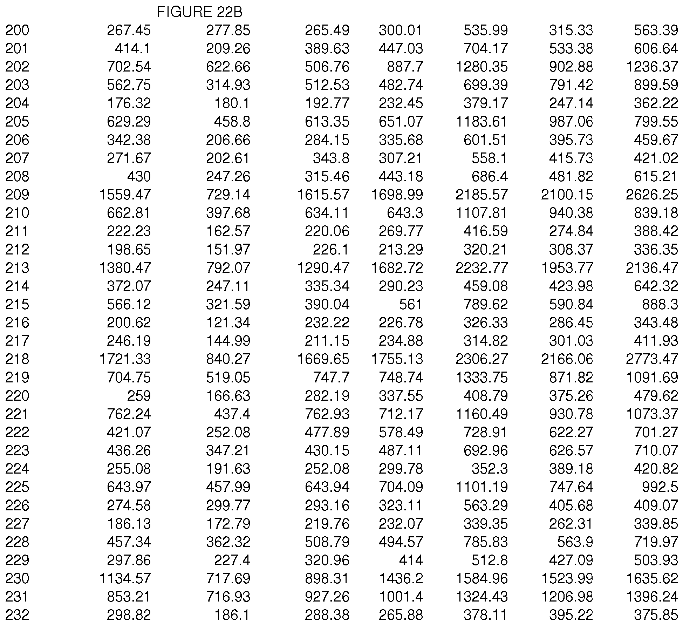

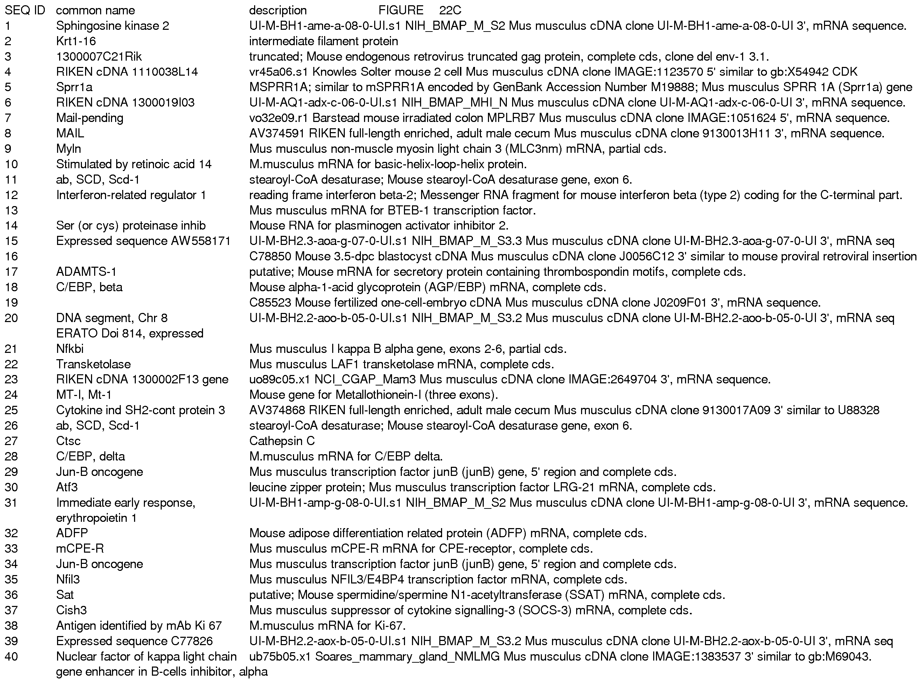

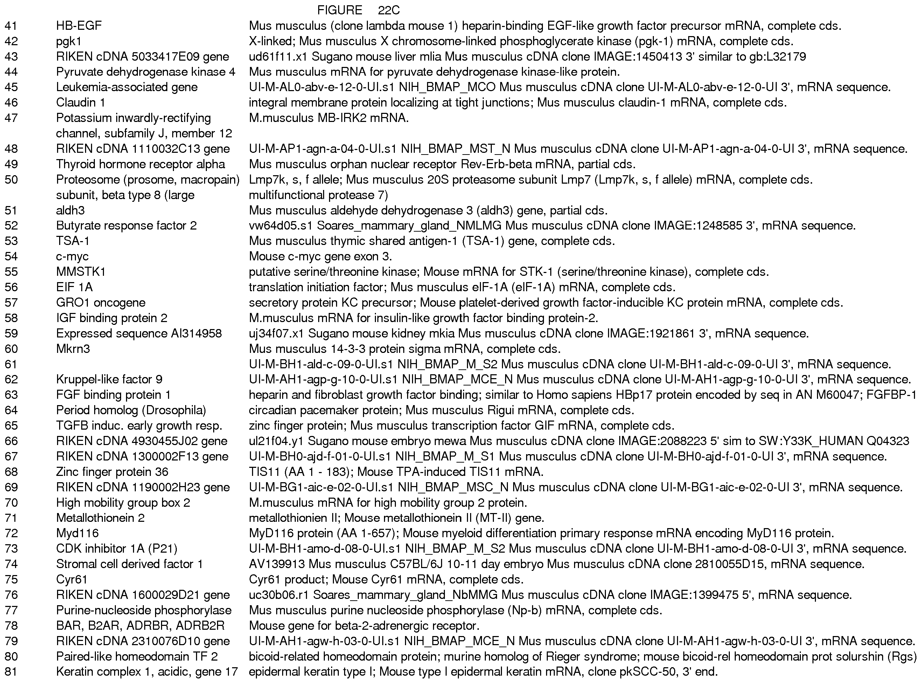

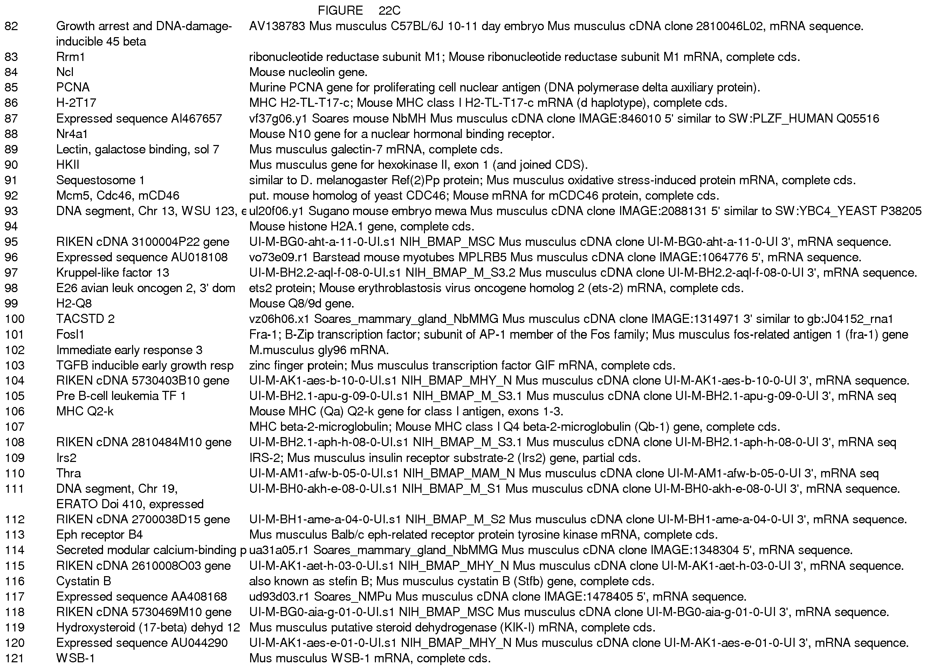

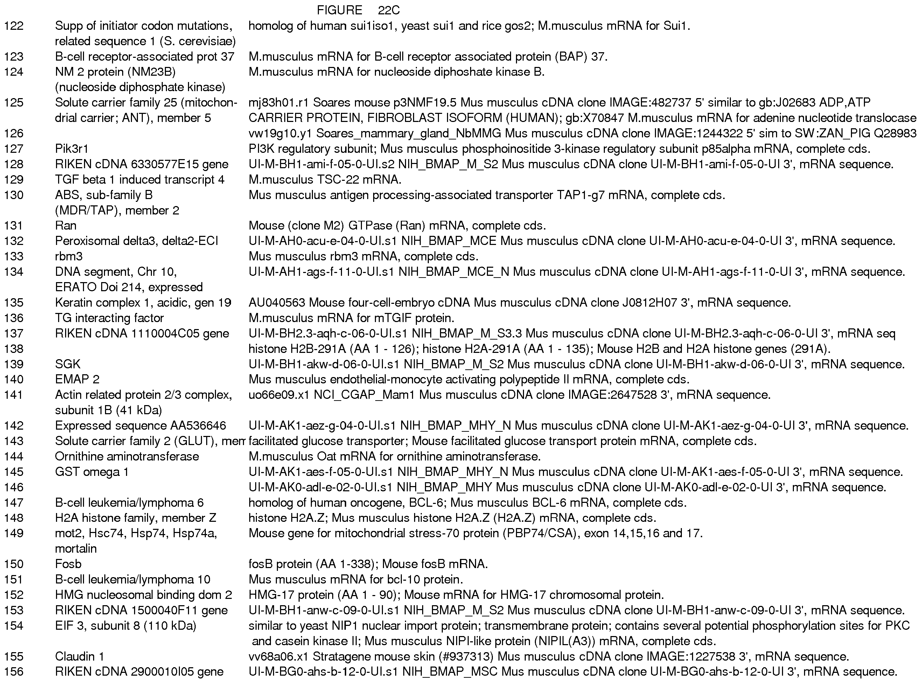

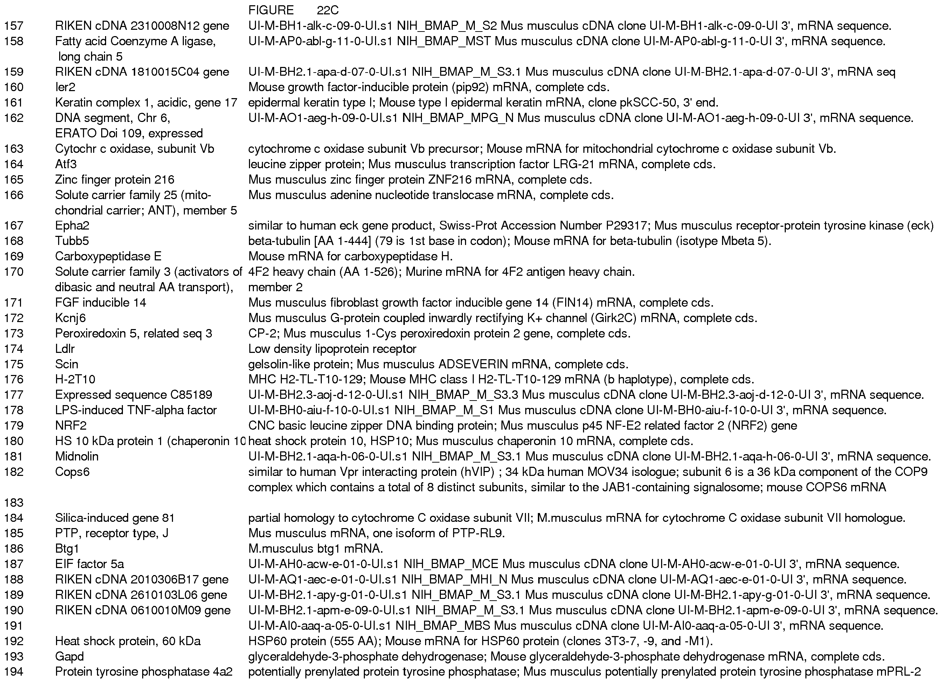

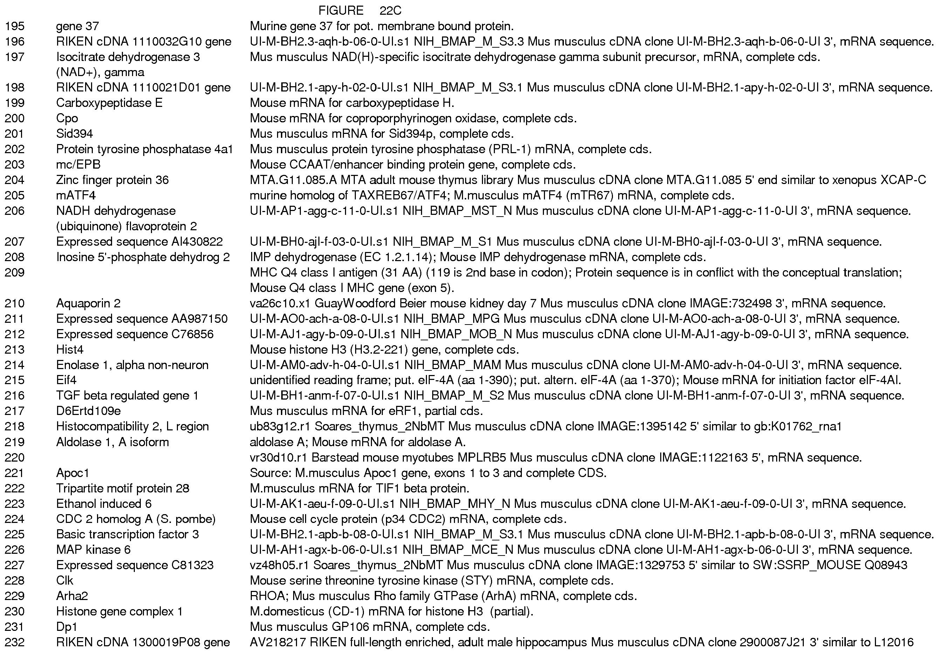

To identify molecular pathways up-regulated during HF stem cell activation, activated HF stem cells were isolated, and the gene expression patterns of the cells were analyzed to detect up-regulated transcripts. The transcripts depicted in Table 3 were up-regulated at least 2-fold in the activated HF stem cells relative to the cells prior to activation. In some cases, the sequence in Table 3 is a genomic sequence that contains the sequence of the transcript. Data pertaining to the up-regulation of the transcripts and further information about them is provided in Figure 22.

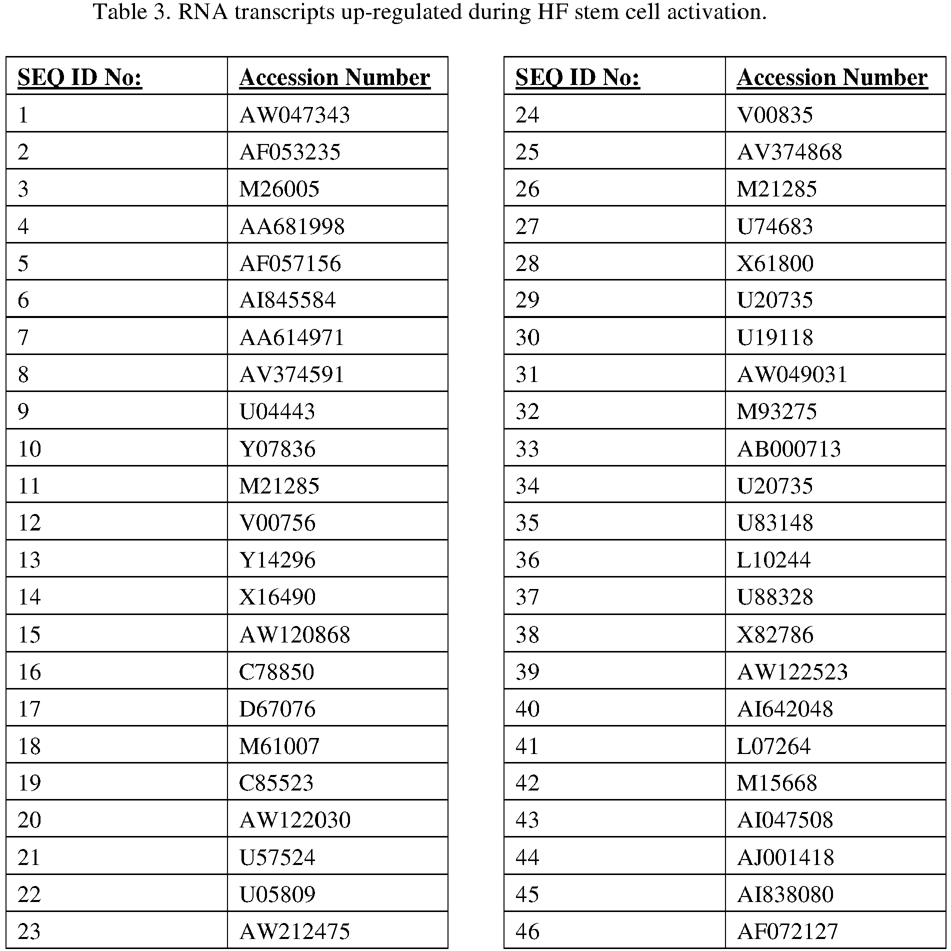

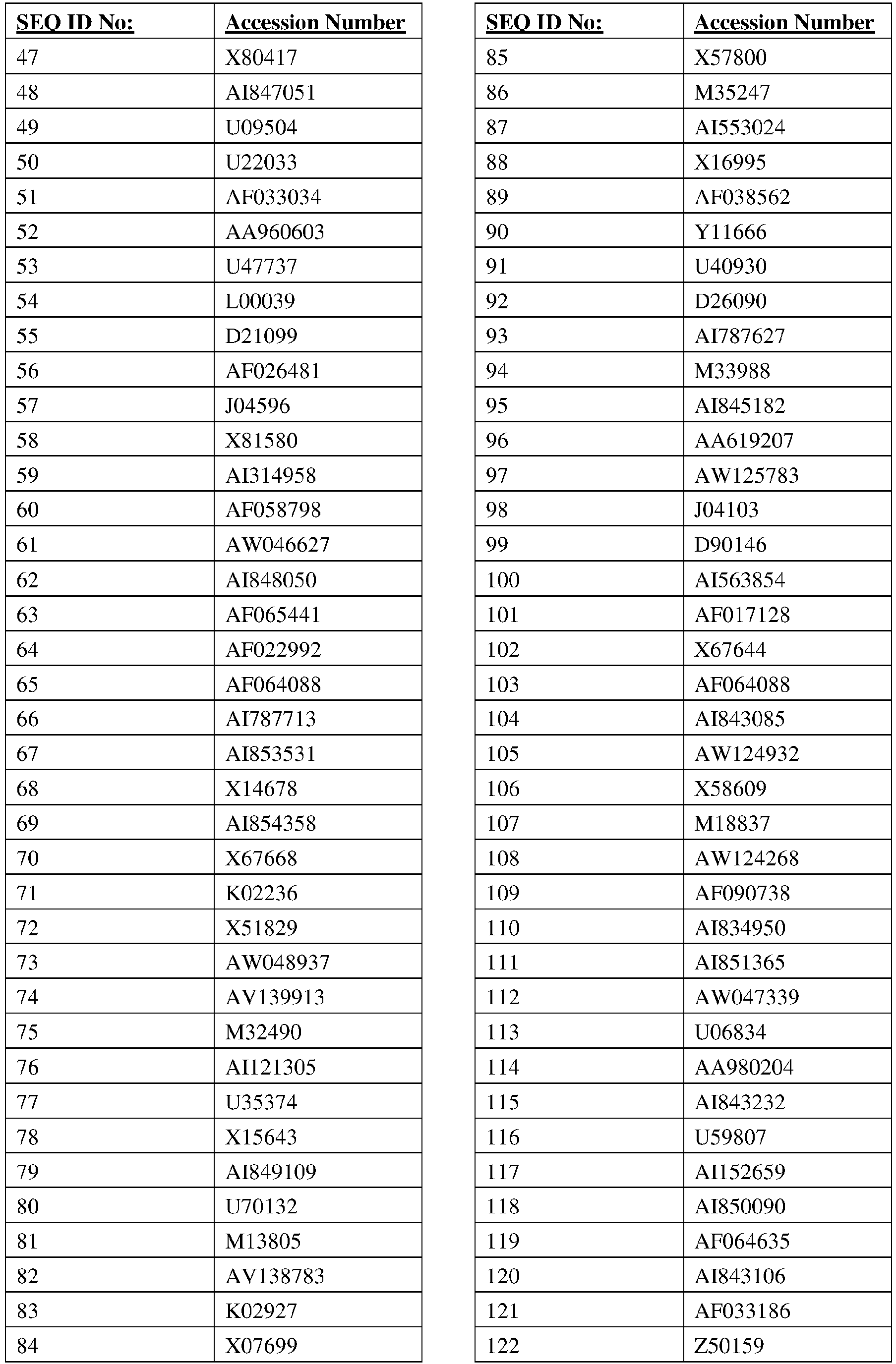

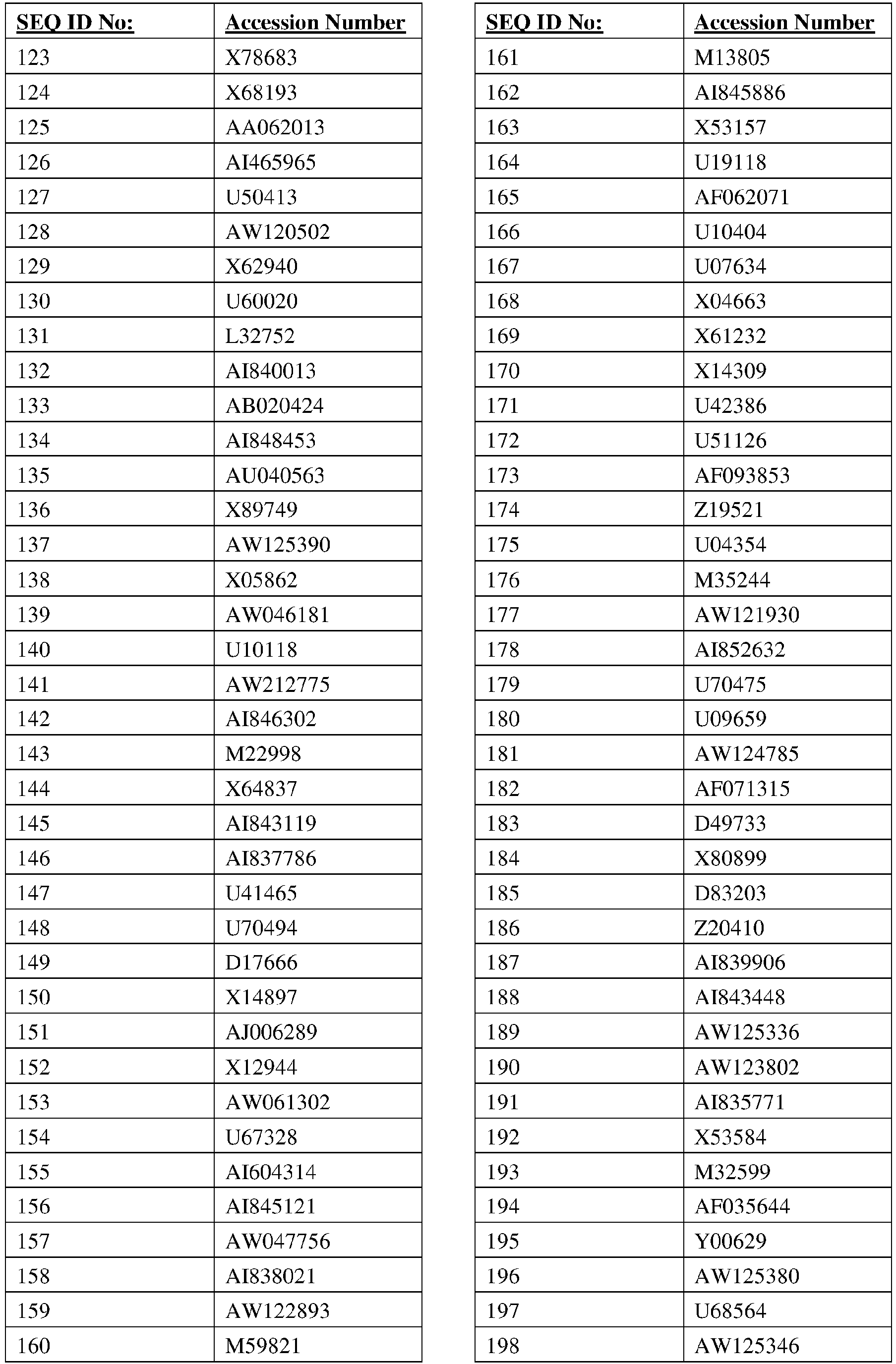

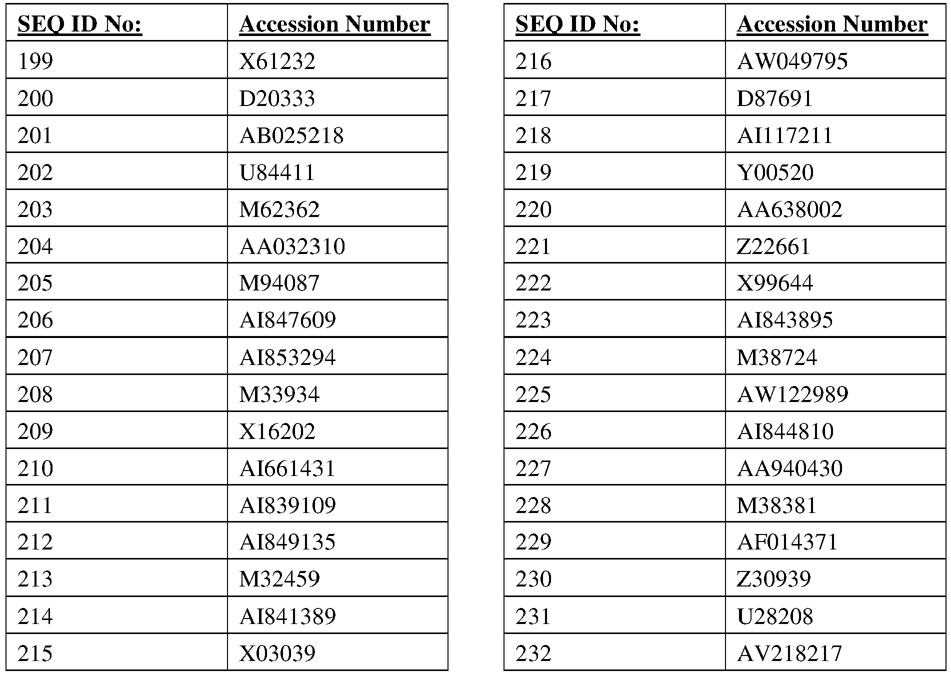

Thus, the transcripts identified in this Example, the proteins they encode, and the pathways in which the proteins participate contribute significantly to HF stem cell activation. Accordingly, anagen can be induced by activation of these transcripts, proteins, and pathways.

### EXAMPLE 9

### MOLECULAR PATHWAYS ACTIVATED DURING INDUCTION OF EPIDERMAL CELLS TO DIFFERENTIATE INTO HF STEM CELLS

The gene expression pattern of HF stem cells was analyzed as described in Example 8 and compared to non-bulge basal keratinocytes. 157 genes were differentially expressed in the HF stem cells, as assessed by microarray analysis and quantitative polymerase chain reaction (qPCR). A group of selected genes with increased expression in HF stem cells is depicted in Table 4. A group of selected genes with decreased expression in HF stem cells is depicted in Table 5.

Table 4. Genes up-regulated in HF stem cells. Numbers in parentheses are the fold increase as determined by quantitative real time PCR.

| **Gene Name/ Protein name** **GenBank Accession Number** | **Fold Increase** | **SEQ ID No.** |
|---|---|---|
| Cell surface proteins | | |
| Cd34/Cd 34 antigen AI847784, AI173145 | 43 (189) | 233 |

| Calcium -related | | |
|---|---|---|
| S100a4/S100A4 (mts) X15986 | 35 (144) | 234 |

| Transcription Factors and related genes | | |
|---|---|---|
| Id2 helix-loop-helix protein AF077861 | 11 (25) | 235 |
| Id4 AJ001972 | 4 (12) | 236 |
| Peg3/Paternally expressed gene 3 zinc finger protein AF038939 | 12 | 237 |

| Growth Factors, Receptors and Related genes | | |
|---|---|---|
| Fz2/Frizzled 2 A W123618 | 9 (17) | 238 |
| Dkk3/Dickkopf 3 AJ243964 | 6 (22) | 239 |
| Sfrp1/Secreted frizzled-related protein 1 U88566 | 6 | 240 |
| Dab2/Disabled homolog 2 U18869 | 15 | 241 |
| Cktsf1b1/Gremlin, Cysteine knot superfamily 1, BMP antagonist 1 AF045801 | 12 (12) | 242 |
| Fgfr1/Fibroblast growth factor receptor 1 U22324 | 10 | 243 |
| Fgf1/Fibroblast growth factor 1 M30641 | 10 | 244 |
| Gpr49/G protein-coupled receptor 49 FEX AF110818 | 64 (377) | 245 |
| Igfbp5/insulin-like growth factor binding protein 5 L12447 | 37 | 246 |
| Myoc/trabecular meshwork induced gluco-corticoid protein AF041335 | 111 | 247 |
| Itm2a/E25 putative Integral membrane protein 2A L38971 | 30 | 248 |
| Eps8/epidermal growth factor receptor pathway substrate 8 L21671 | 15 | 249 |
| Fyn/Fyn proto-oncogene M27266 | 10 | 250 |

| Structurally-related | | |
|---|---|---|
| Co16a1/Procol-lagen, type VI, alpha 1 X66405 | 36 | 251 |
| Tnc/Tenascin C AV230686, X56304 | 17 | 252 |
| Krt2-6a/ Keratin complex 2, basic, gene 6a (keratin 6a) K02108 | 10 | 253 |

| Channel-related | | |
|---|---|---|
| Potassium channel, subfamily K, member 2 AI849601 | 14 | 254 |
| Skd3/Suppressor of K+ transport defect 3 AI837887 | 4 | 255 |
| Clic4/Chloride intracellular channel 4 (mito-chondrial) AI845237 | 3 | 256 |
| Col18a1/Endostatin (alpha 1(XVIII) collagen) L22545 | 5 | 257 |

**Table 5. Genes down-regulated in HF stem cells.**

| **Gene Name/protein** **Accession Number** | **Fold Decrease** | **SEQ ID No.** |
|---|---|---|
| Growth Factors, Receptors and Downstream genes | | |
| GNA-14 Mouse G protein alpha subunit (GNA-14) M80631 | 32 | 258 |
| Ly6/Lympho-cyte antigen 6 complex X04653 | 12 | 259 |
| Bmp4/Bone morphogenetic protein 4 L47480 | 11 | 260 |
| IL1r2/Inter-leukin 1 receptor, type II AV223216, X59769 | 11 | 261 |
| Wnt3a/wing-less-related MMTV integration site 3A X56842 | 4 | 262 |
| Il12rb2/Interleukin 12 receptor, beta 2 U64199 | 3 | 263 |
| Wnt10a/Wing-less-related MMTV integration site 10a U61969 | 3 | 264 |
| Ifngr2/Inter-feron-gamma receptor precursor M28233 | 3 | 265 |
| Fgfbp1/Fibroblast growth factor binding protein 1 AF065441 | 3 | 266 |

| Transcription Factors and Related Genes | | |
|---|---|---|
| Klf5/Kruppel-like factor 5 AA611766 | 5 | 267 |
| Gata3/GATA binding protein 3 X55123 | 4 | 268 |
| Stimulated by retinoic acid 14, basic-helix-loop-helix protein Y07836 | 3 | 269 |

| Cell Cycle Related | | |
|---|---|---|
| Mki67/antigen identified by monoclonal antibody Ki67 X82786 | 4 | 270 |
| Cks2/CDC28 protein kinase regulatory subunit 2, sim to cdk regulatory subunit 2 AA681998 | 4 | 271 |
| Ccng2/ Cyclin G2 U95826 | 3 | 272 |
| Prc1/Protein regulator of cytokinesis 1 DNA segment, Chr 7 AA856349 | 3 | 273 |

Thus, the transcripts identified in this Example, the proteins they encode, and the pathways in which the proteins participate, contribute significantly to induction of epidermal cells to differentiate into HF stem cells. Activation of the transcripts, proteins, and pathways depicted in Table 4 is thus a method for enhancing EDIHN. In addition, inhibition of the transcripts, proteins, and pathways depicted in Table 4 is thus a method for preventing EDIHN and eliminating hair follicles. In addition, inhibition of the transcripts, proteins, and pathways depicted in Table 5 is a method for enhancing EDIHN. In addition, activation of the transcripts, proteins, and pathways depicted in Table 5 is thus a method for enhancing EDIHN.

### REFERENCE EXAMPLE 10

### EXPRESSION OF WNT-1 INHIBITORS DURING THE FIRST NINE DAYS AFTER WOUNDING CAUSES PIGMENTATION OF NEW HF

### MATERIALS AND EXPERIMENTAL METHODS

In this Example, doubly transgenic mice expressing both tetO-Dkkland K5-rtTA were utilized. When these mice are fed chow formulated with 1 g/kg doxycycline (BioServ, Laurel, MD), they express Dkk1, under the control of the K5 promoter, in the basal epidermis. The control mice also received doxycycline, but they were K5-rtTA negative and thus did not express Dkk1.

### RESULTS

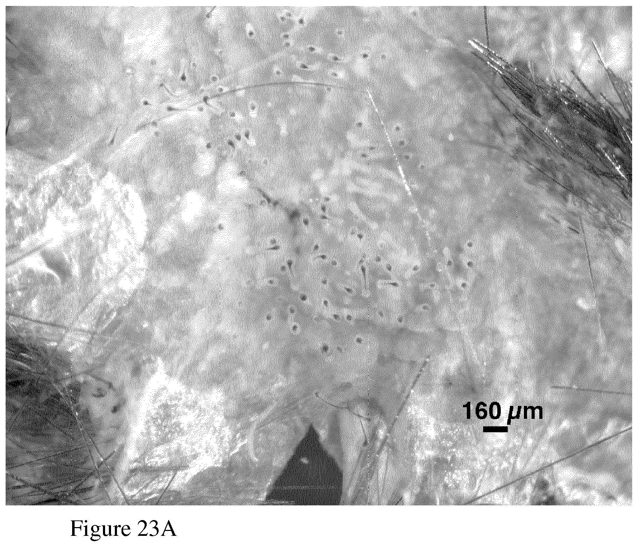

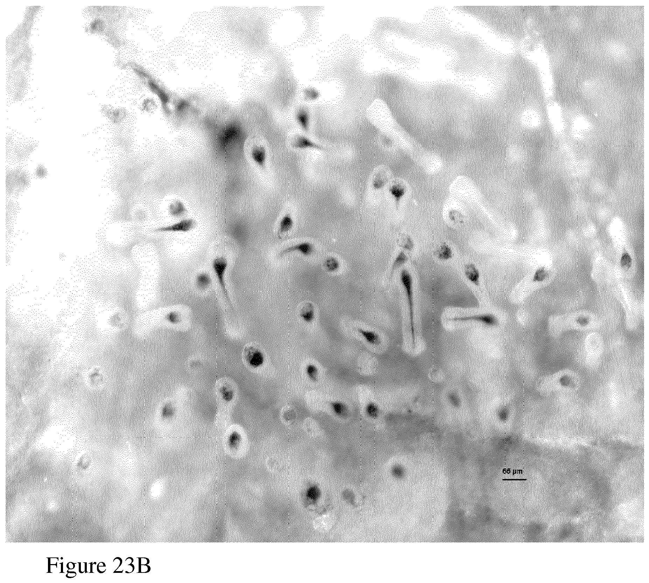

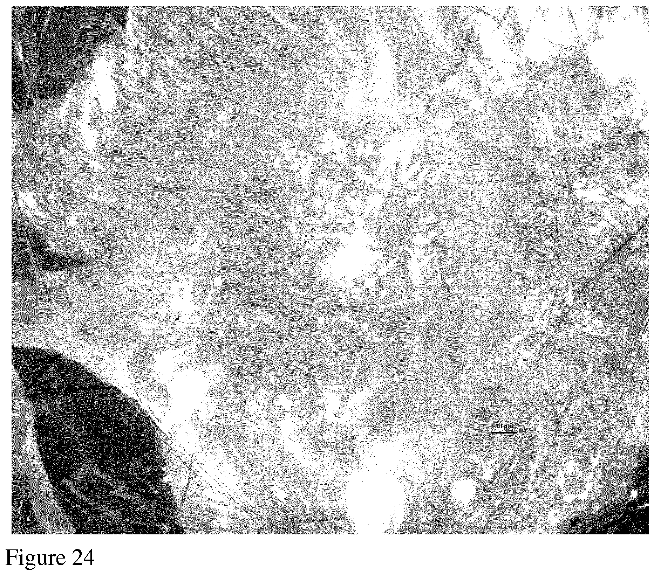

A 1 cm² wound was induced on the lower back of the doubly transgenic mice at 21 days or 50 days old. Mice were placed on doxycycline-containing chow immediately after wounding to induce Dkk1 expression, and then doxycycline was discontinued after completion of the re-epithelialization at 9 days after wounding. Dkk1 expression inhibits Wnt activity, which in turn induces follicle pigmentation. At 22 days after wounding, pigmented HF were observed in the excised skin after preparing the epidermal sheet (Figure 23A-B). Control mice lacked pigmented HF (Figure 24).

In other experiments, continued expression of Dkk1 after the 9-day period inhibited formation of new HF.

The findings of this Example show that pigmented HF can be produced by suppressing expression of Wnt1 or by inducing expression of Dkk1 during the period of re-epithelialization, then inducing expression of Wnt1. In addition, the findings of this Example show that factors that inhibit neonatal hair follicle formation (e.g. Dkk1) also inhibit EDIHN, thus further supporting the notion that hair follicles formed by EDIHN are similar to normal hair follicles.

### REFERENCE EXAMPLE 11

### INHIBITION OF EDIHN BY EPIDERMAL GROWTH FACTOR INJECTION

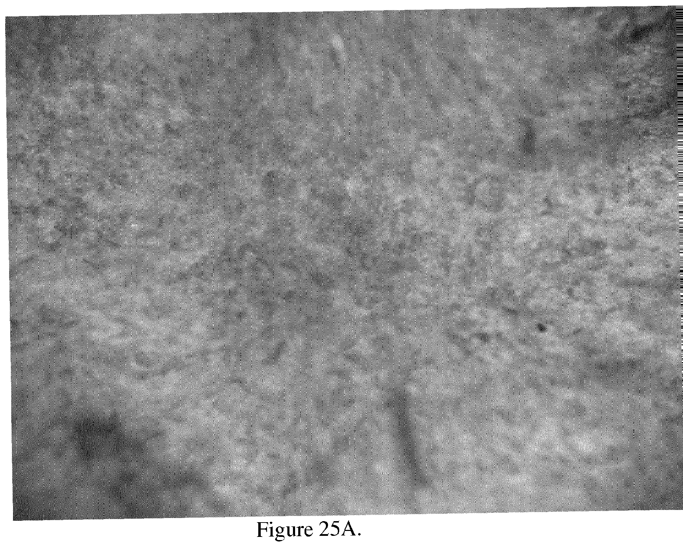

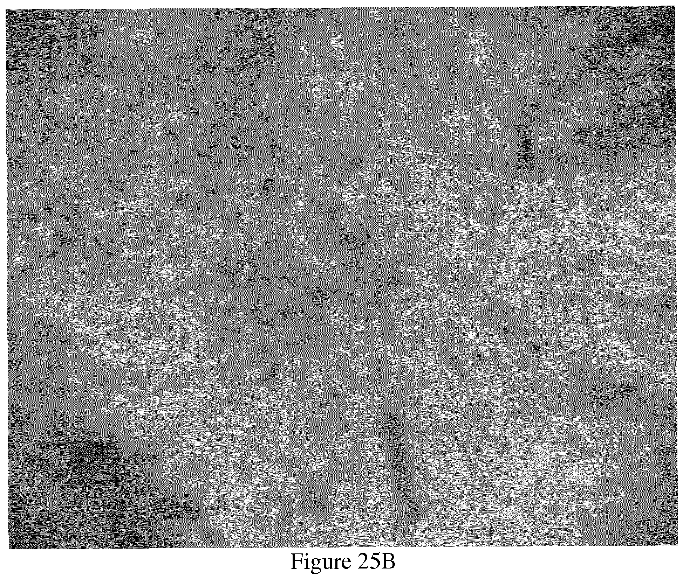

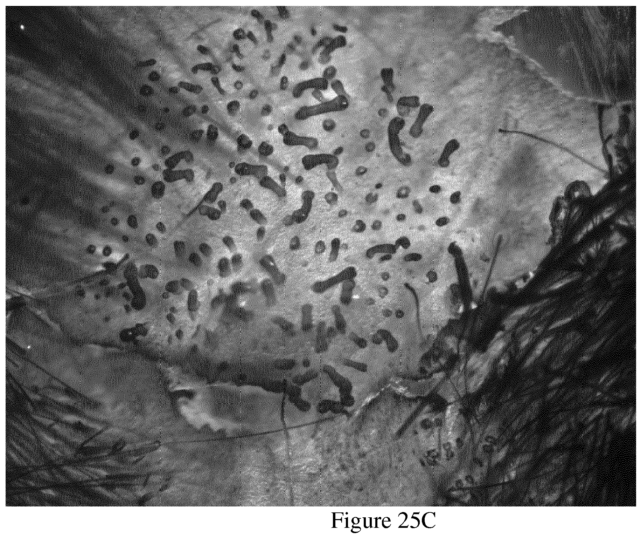

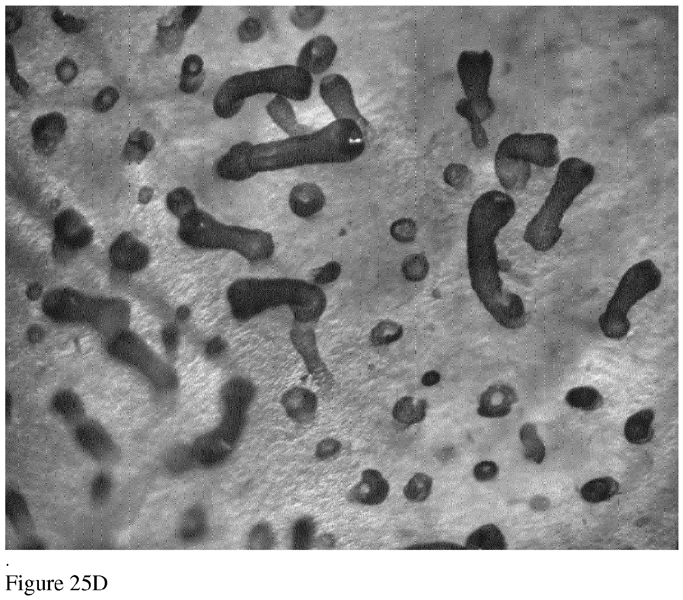

21 day-old mice were wounded as described in previous Examples. Starting from day 11 after wounding, a time point corresponding to the point at which the wound had recently reepithelialized, 10 µL of 1 µg/ml EGF was injected into the wound bed. EGF was injected once per day after this point for a total of 5 days. Three days later, the skin was collected, and whole-mount EDIHN assays were performed. EGF prevented HF formation as assessed by gross morphology. In addition, whole mounts of control and treated skin were analyzed with anti-K17 antibody immunostaining. All mice injected with EGF (n=4) exhibited no new HF formation (Figures 25 A-B), while control mice (n=2) had many new HF, as expected. (Figures 25 C-D).

In an additional experiment, recombinant EGF (1 microgram (mcg)/microliter (mcl)) was injected at days 11, 13 and 15 after wounding. Skin was collected at 18 days after wounding and stained for K17 and alkaline phosphotase. Once again, administration of EGF inhibited EDIHN.

The findings of this Example show that EGF inhibits HF formation. Thus, inhibiting EGF, EGFR, or one of the pathways in which they participate increases EDIHN-induced HF formation.

### REFERENCE EXAMPLE 12

### ENHANCEMENT OF EDIHN BY INHIBITION OF EGF RECEPTOR

To determine the effect of administration of EGF receptor inhibitors on DIHN, the inhibitor AG1478 (150 µM in 10 µL volume) was administered as a single injection 11 days after incisional wounding (1 cm²) to the middle of the wound near the skin surface. EGF receptor inhibitor administration led to generation of more and larger hair follicles compared with control mice that were wounded only (Figure 26A). As shown in Figure 26B, large hair follicles developed in the wounded area in the AG1478-injected mice. Left panel: epidermis stained for K17, with three large hair follicles next to each other. Right panel: dermis stained for AP with large coalescing dermal papilla areas.

The findings of this Example confirm the results of the previous Example, and show that more and larger HF can be generated when EDIHN comprises, or is followed by, administration of EGFR inhibitors, or with compounds with a similar mechanism of action; e.g. Hedgehog protein and androgen antagonists.

### REFERENCE EXAMPLE 13

### ENHANCEMENT OF EDIHN BY EXPRESSION OF A β-CATENIN ACTIVATOR

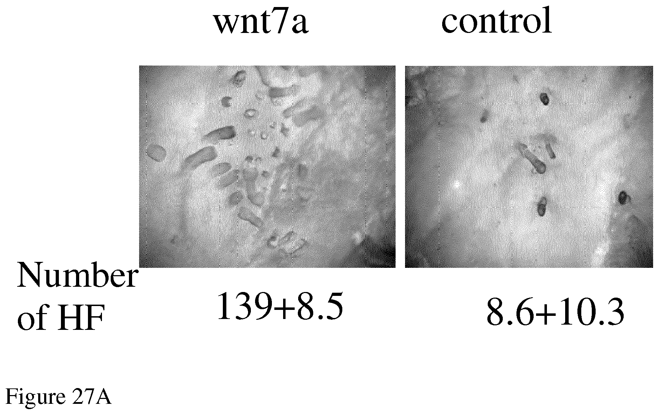

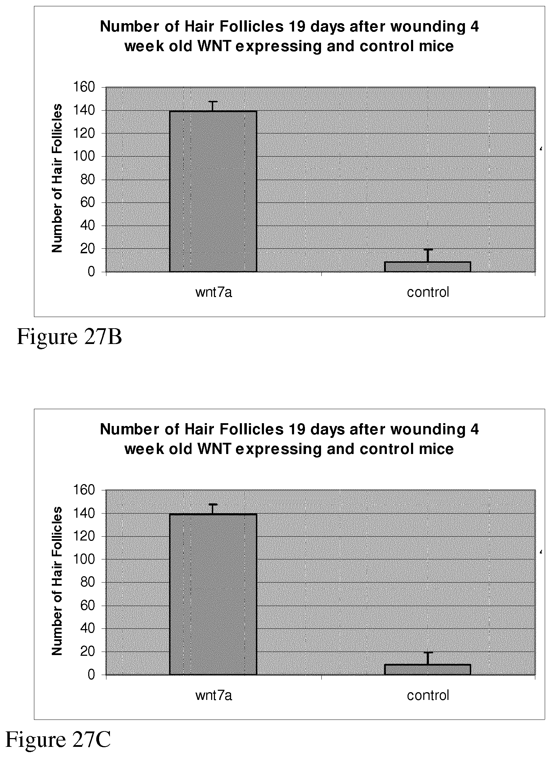

To determine the effect of administration of β-catenin activators on EDIHN, K14-Wnt7 transgenic mice, which overexpress the β-catenin pathway activator, Wnt7, in the epidermis, were subjected to EDIHN, then HF formation was measured 19 days after wounding. In each of 2 separate experiments, with 4 week old and 3 week old mice, the transgenic mice developed significantly larger numbers of HF compared to control, non-transgenic littermate mice (Figure 27 A-C).

Thus, administration of β-catenin activators leads to an increase in EDIHN. The findings of Examples 11-13 show that new HF can be generated by (a) disrupting the epidermis; and (b) administering a factor that promotes a differentiation of an uncommitted epidermal cell into a HF cell.

### REFERENCE EXAMPLE 14

### ENHANCEMENT OF EDIHN BY ADMINISTRATION OF FGF

To determine the effect of fibroblast growth factor (FGF) on EDIHN, recombinant FGF is administered 11 days after incisional wounding, as described in Example 11. FGF administration enhances HF formation, showing that new HF can be generated by (a) disrupting the epidermis; and (b) administering FGF, a nucleotide encoding FGF, or a factor that increases signaling by FGF.

### REFERENCE EXAMPLE 15

### ENHANCEMENT OF EDIHN BY ADMINISTRATION OF EDAR

To determine the effect of fibroblast growth factor (FGF) on EDIHN, K14-Eda-A1 transgenic mice, which overexpress (ectodysplasin-A1) Eda-A1 in the epidermis, are subjected to EDIHN, then HF formation is measured 19 days after wounding as described in Example 13. The transgenic mice developed significantly larger numbers of HF compared to control, non-transgenic littermate mice, showing that new HF can be generated by (a) disrupting the epidermis; and (b) administering a factor that enhances signaling by ectodysplasin.

### EXAMPLE 16

### ENHANCEMENT OF EDIHN BY ADMINISTRATION OF MINOXIDIL

To determine the effect of minoxidil on EDIHN, minoxidil is administered 11 days after incisional wounding, as described in Example 11. Minoxidil administration enhances HF formation, showing that new HF can be generated by (a) disrupting the epidermis; and (b) administering a minoxidil.

### REFERENCE EXAMPLE 17

### REMOVAL OF HF BY ABRASION AND ADMINISTRATION OF EGF

Hair-bearing regions of the epidermis of mice is abraded, as described in Example 1, then administering recombinant EGF, as described in Example 1. This method prevents hair re-growth in the abraded areas, showing that hair can be removed by (a) disrupting the epidermal layer; and (b) administering EGF, a nucleotide encoding EGF, or a factor that increases signaling by EGF.

## Claims

1. Use of minoxidil for the preparation of a pharmaceutical composition for treating baldness in a scalp, eyebrow or scarred region of a subject, wherein the treating comprises:
(a) disrupting an epidermis of the scalp, eyebrow or scarred region, wherein said disrupting is perforating the epidermis or epidermal layer; and
(b) contacting the scalp, eyebrow or scarred region with the pharmaceutical composition.

2. Minoxidil for use in treating baldness in a scalp, eyebrow or scarred region of a subject, wherein the treating comprises:
(a) disrupting an epidermis of the scalp, eyebrow or scarred region, wherein said disrupting is perforating the epidermis or epidermal layer; and
(b) contacting the scalp, eyebrow or scarred region with the minoxidil.

3. The use of claim 1 or the minoxidil for use according to claim 2, wherein the subject has androgenetic alopecia (AGA) or another disease or disorder comprising balding.

4. A non-therapeutic method for generating a hair follicle in a scalp, eyebrow or scarred region of a subject, the method comprising:
(a) disrupting an epidermis of the scalp, eyebrow or scarred region, wherein said disrupting is perforating the epidermis or epidermal layer; and
(b) contacting the scalp, eyebrow or scarred region with minoxidil,
thereby generating a hair follicle in the scalp, eyebrow or scarred region of the subject.

5. The use of claims 1 or 3, the minoxidil for use according to claims 2 or 3, or the method of claim 4, wherein the disruption is performed with a tool that comprises sandpaper, a laser, such as a Fraxel laser, a CO2 laser or an excimer laser, a felt wheel, a microdermabrasion device, a light-based method, irradiation with visible light, irradiation with infrared light, irradiation with ultraviolet radiation, orthovoltage radiation, x-ray radiation, a surgical tool, a dermal biopsy punch, or wherein the disruption is performed with a burn treatment or chemical treatment.

6. The use of claims 1 or 3, the minoxidil for use according to claims 2 or 3, or the method of claim 4, wherein the disruption comprises perforating the epidermis and dermis of the area of the balding scalp.

7. The use of claims 1 or 3, the minoxidil for use according to claims 2 or 3, or the method of claim 4, wherein the disruption comprises removal of basal and supra-basal layers of epidermis with a felt wheel.

8. The use of claims 1 or 3, the minoxidil for use according to claims 2 or 3, or the method of claim 4, wherein the treating further comprises contacting the area of the balding scalp with a precursor cell, an inductive cell, or a hair follicle or a portion thereof, or with one or more of the following compounds: finasteride, dutasteride, Fluridil^{®}, spironolactone, cyproterone acetate, bicalutamide, flutamide, nilutamide, an inhibitor of an androgen receptor, an androgen antagonist or an antiandrogen.

9. The use, the minoxidil for use, or the method of claim 8, wherein the compound is formulated to be administered as a cream, gel, lotion, emulsion, suspension, oil, nonaqueous solution, aqueous solution, or drop, or by direct injection, or is encapsulated in a liposome.

10. The use, the minoxidil for use, or the method of claim 8, wherein the disrupting step is performed between 3-12 days, 4-12 days, 5-12 days, 4-11 days, 6-11 days, 6-10 days, 6-9 days, 6-8 days, 7-8 days, 5-11 days, 5- 10 days, 7- 10 days, or about 1 week prior to the further contacting step.

11. The use of claims 1 or 3, the minoxidil for use according to claims 2 or 3, or the method of claim 4, wherein the disrupting step is performed between 3-12 days, 4-12 days, 5-12 days, 4-11 days, 6-11 days, 6-10 days, 6-9 days, 6-8 days, 7-8 days, 5-11 days, 5- 10 days, 7- 10 days, or about 1 week prior to the contacting step.

12. The use of claims 1 or 3, the minoxidil for use according to claims 2 or 3, or the method of claim 4, wherein the area of the balding scalp is subjected to depilation or administration of a retinoid prior to wounding.

13. The use, the minoxidil for use, or the method of claim 12, wherein the depilation is performed by plucking, waxing, abrasion, laser, electrolysis, or administration of thioglycolic acid on the area of the balding scalp.

14. The use of claims 1 or 3, the minoxidil for use according to claims 2 or 3, or the method of claim 4, wherein the wounded area of the balding scalp is not surgically closed, or not contacted with a bandage, dressing, or ointment that facilitates wound healing for a period of time following the wounding step.

15. The use, the minoxidil for use, or the method of claim 14, wherein the period of time is within about 2 days, about 3 days, about 4 days, about 5 days, about 7 days, about 10 days, about 11 days, about 14 days, about 17 days or about 3 weeks.

16. The use of claims 1 or 3, the minoxidil for use according to claims 2 or 3, or the method of claim 4, wherein the treatment or the method results in differentiation of an uncommitted epidermal cell into a hair follicle cell.

## Patentansprüche

1. Anwendung von Minoxidil zur Herstellung einer pharmazeutischen Zusammensetzung zum Behandeln von Haarausfall von einer Kopfhaut, Augenbraue oder vernarbten Region eines Subjekts, wobei das Behandeln wie folgt umfasst:
(a) Stören einer Epidermis der Kopfhaut, Augenbraue oder vernarbten Region, wobei das Stören ein Perforieren der Epidermis oder Epidermis-Schicht ist; und
(b) Kontaktieren der Kopfhaut, Augenbraue oder vernarbten Region mit der pharmazeutischen Zusammensetzung.

2. Minoxidil zur Anwendung beim Behandeln von Haarausfall von einer Kopfhaut, Augenbraue oder vernarbten Region eines Subjekts, wobei das Behandeln wie folgt umfasst:
(a) Stören einer Epidermis der Kopfhaut, Augenbraue oder vernarbten Region, wobei das Stören ein Perforieren der Epidermis oder Epidermis-Schicht ist; und
(b) Kontaktieren der Kopfhaut, Augenbraue oder vernarbten Region mit Minoxidil.

3. Anwendung nach Anspruch 1 oder Minoxidil zur Anwendung nach Anspruch 2, wobei das Subjekt androgenetische Alopezie oder eine andere Krankheit oder ein anderes Leiden hat, das Haarausfall umfasst.

4. Nicht-therapeutisches Verfahren zum Generieren eines Haarfollikels in einer Kopfhaut, Augenbraue oder vernarbten Region eines Subjekts, wobei das Verfahren wie folgt umfasst:
(a) Stören einer Epidermis der Kopfhaut, Augenbraue oder vernarbten Region, wobei das Stören ein Perforieren der Epidermis oder Epidermis-Schicht ist; und
(b) Kontaktieren der Kopfhaut, Augenbraue oder vernarbten Region mit Minoxidil, wodurch ein Haarfollikel in der Kopfhaut, Augenbraue oder vernarbten Region des Subjekts generiert wird.

5. Anwendung nach Anspruch 1 oder 3, Minoxidil zur Anwendung nach Anspruch 2 oder 3 oder Verfahren nach Anspruch 4, wobei die Störung mit einem Werkzeug durchgeführt wird, das Sandpapier umfasst, mit einem Laser, wie etwa einen Fraxel-Laser, einen CO2-Laser oder einem Excimer-Laser, mit einer Filzscheibe, einem Peeling-Gerät, einem lichtbasierten Verfahren, das Bestrahlung mit VIS-Licht, Bestrahlung mit IR-Licht, Bestrahlung mit UV-Strahlen, Orthovolt-Bestrahlung und Röntgenstrahlen umfasst sowie mit einem chirurgischen Instrument, einer Hautbiopsie-Stanze oder wobei die Störung mit einer Brandbehandlung oder einer chemischen Behandlung durchgeführt wird.

6. Anwendung nach Anspruch 1 oder 3, Minoxidil zur Anwendung nach Anspruch 2 oder 3 oder Verfahren nach Anspruch 4, wobei die Störung ein Perforieren der Epidermis und Dermis in der Region der kahl werdenden Kopfhaut umfasst.

7. Anwendung nach Anspruch 1 oder 3, Minoxidil zur Anwendung nach Anspruch 2 oder 3 oder Verfahren nach Anspruch 4, wobei die Störung eine Entfernung von basalen und suprabasalen Schichten der Epidermis mit einer Filzscheibe umfasst.

8. Anwendung nach Anspruch 1 oder 3, Minoxidil zur Anwendung nach Anspruch 2 oder 3 oder Verfahren nach Anspruch 4, wobei das Behandeln ferner ein Kontaktieren der Region der kahl werdenden Kopfhaut mit einer Vorläuferzelle, einer Induktivzelle oder einem Haarfollikel oder einem Anteil hiervon oder mit einer oder mehreren der folgenden Verbindungen umfasst: Finasterid, Dutasterid, Fluridil^{®}, Spironolacton, Cyproteronacetat, Bicalutamid, Flutamid, Nilutamid, einem Hemmer eines Androgenrezeptors, einem Androgenantagonisten oder einem Antiandrogen.

9. Anwendung, Minoxidil zur Anwendung oder Verfahren nach Anspruch 8, wobei die Verbindung dazu formuliert ist, um als Creme, Gel, Lotion, Emulsion, Suspension, Öl, nicht-wässrige Lösung, wässrige Lösung oder als Tropfen oder durch eine direkte Injektion oder als in einem Liposom verkapselt verabreicht wird.

10. Anwendung, Minoxidil zur Anwendung oder Verfahren nach Anspruch 8, wobei der Störungsschritt an zwischen 3-12 Tagen, 4-12 Tagen, 5-12 Tagen, 4-11 Tagen, 6-11 Tagen, 6-10 Tagen, 6-9 Tagen, 6-8 Tagen, 7-8 Tagen, 5-11 Tagen, 5-10 Tagen, 7-10 Tagen oder etwa 1 Woche vor dem weiteren Kontaktierungsschritt durchgeführt wird.

11. Anwendung nach Anspruch 1 oder 3, Minoxidil zur Anwendung nach Anspruche 2 oder 3 oder Verfahren nach Anspruch 4, wobei der Störungsschritt an zwischen 3-12 Tagen, 4-12 Tagen, 5-12 Tagen, 4-11 Tagen, 6-11 Tagen, 6-10 Tagen, 6-9 Tagen, 6-8 Tagen, 7-8 Tagen, 5-11 Tagen, 5-10 Tagen, 7-10 Tagen oder etwa 1 Woche vor dem Kontaktierungsschritt durchgeführt wird.

12. Anwendung nach Anspruch 1 oder 3, Minoxidil zur Anwendung nach Anspruch 2 oder 3 oder Verfahren nach Anspruch 4, wobei der Region der kahl werdenden Kopfhaut vor der Verwundung einer Enthaarung oder Verabreichung eines Retinoids ausgesetzt wird.

13. Anwendung, Minoxidil zur Anwendung oder Verfahren nach Anspruch12, wobei die Enthaarung durch Auszupfen, Wachsen, Peeling, Laseranwendung, Elektrolyse oder Verabreichung von Thioglycolsäure auf die Region der kahl werdenden Kopfhaut durchgeführt wird.

14. Anwendung nach Anspruch 1 oder 3, Minoxidil zur Anwendung nach Anspruch 2 oder 3 oder Verfahren nach Anspruch 4, wobei der Wundbereich der kahl werdenden Kopfhaut weder chirurgisch geschlossen wird noch mit einer Bandage, einem Verband oder einer Salbe in Kontakt gebracht wird, wodurch eine Wundheilung während einen Zeitraums nach dem Verwundungsschritt ermöglicht wird.

15. Anwendung, Minoxidil zur Anwendung oder Verfahren nach Anspruch 14, wobei der Zeitraum innerhalb etwa 2 Tagen, etwa 3 Tagen, etwa 4 Tagen, etwa 5 Tagen, etwa 7 Tagen, etwa 10 Tagen, etwa 11 Tagen, etwa 14 Tagen, etwa 17 Tagen oder etwa 3 Wochen ist.

16. Anwendung nach Anspruch 1 oder 3 oder Minoxidil zur Anwendung nach Anspruch 2 oder 3 oder Verfahren nach Anspruch 4, wobei die Behandlung oder das Verfahren in einer Differenzierung einer nicht festgelegten Epidermiszelle in eine Haarfollikelzelle resultiert.

## Revendications

1. Utilisation de minoxidil pour la préparation d'une composition pharmaceutique pour le traitement de la calvitie du cuir chevelu, des sourcils ou de la région cicatrisée d'un sujet, dans laquelle le traitement comprend :
(a) la rupture d'un épiderme du cuir chevelu, des sourcils ou de la région cicatrisée, ladite rupture perforant l'épiderme ou la couche épidermique ; et
(b) la mise en contact du cuir chevelu, des sourcils ou de la région cicatrisée avec la composition pharmaceutique.

2. Minoxidil pour une utilisation dans le traitement de la calvitie du cuir chevelu, des sourcils ou de la région cicatrisée d'un sujet, dans lequel le traitement comprend :
(a) la rupture d'un épiderme du cuir chevelu, des sourcils ou de la région cicatrisée, ladite rupture étant la perforation de l'épiderme ou de la couche épidermique ; et
(b) la mise en contact du cuir chevelu, des sourcils ou de la région cicatrisée avec le minoxidil.

3. Utilisation selon la revendication 1 ou utilisation de minoxidil selon la revendication 2, dans laquelle le sujet souffre d'alopécie androgénétique (AGA) ou d'une autre maladie ou trouble comprenant la calvitie.

4. Méthode non thérapeutique pour générer un follicule pileux dans un cuir chevelu, un sourcil ou une région cicatrisée d'un sujet, la méthode comprenant :
(a) la rupture d'un épiderme du cuir chevelu, d'un sourcil ou d'une région cicatrisée, ladite rupture consistant à perforer l'épiderme ou couche épidermique ; et
(b) la mise en contact du cuir chevelu, des sourcils ou de la région cicatrisée avec du minoxidil, générant ainsi un follicule pileux dans le cuir chevelu, les sourcils ou la région cicatrisée du sujet.

5. Utilisation selon les revendications 1 ou 3, utilisation de minoxidil selon les revendications 2 ou 3, ou méthode selon la revendication 4, dans lequel la rupture est effectuée avec un outil qui comprend du papier de verre, un laser, tel qu'un laser Fraxel, un laser CO₂ ou laser excimer, une roue en feutre, un dispositif de microdermabrasion, une méthode basée sur la lumière, une irradiation par de la lumière visible, une irradiation par de la lumière infrarouge, une irradiation par rayonnement ultraviolet, un rayonnement orthovoltage, un rayonnement X, un outil chirurgical, un poinçon de biopsie, ou dans lequel la rupture est effectuée avec un traitement de brûlure ou un traitement chimique.

6. Utilisation selon les revendications 1 ou 3, utilisation de minoxidil selon les revendications 2 ou 3, ou méthode selon la revendication 4, dans lequel la rupture comprend la perforation de l'épiderme et du derme de la zone du cuir chevelu dégarni.

7. Utilisation selon les revendications 1 ou 3, utilisation de minoxidil selon les revendications 2 ou 3, ou méthode selon la revendication 4, dans lequel la rupture comprend l'élimination des couches basale et supra et basale de l'épiderme avec une roue en feutre.

8. Utilisation selon les revendications 1 ou 3, utilisation de minoxidil selon les revendications 2 ou 3, ou méthode selon la revendication 4, dans laquelle le traitement comprend en outre la mise en contact de la zone du cuir chevelu dégarni avec une cellule précurseur, une cellule inductive, ou un follicule pileux ou une partie de celui-ci, ou avec un ou plusieurs des composés suivants : finastéride, dutastéride, Fluridil^{®}, spironolactone, acétate de cyprotérone, bicalutamide, flutamide, nilutamide, un inhibiteur d'un récepteur aux androgènes, un antagoniste des androgènes ou un antiandrogène.

9. Utilisation, utilisation de minoxidil ou méthode selon la revendication 8, dans laquelle le composé est formulé pour être administré sous forme de crème, de gel, de lotion, d'émulsion, de suspension, d'huile, de solution non aqueuse, de solution aqueuse ou de goutte, ou par injection directe, ou est encapsulé dans un liposome.

10. Utilisation, utilisation de minoxidil ou méthode selon la revendication 8, dans laquelle l'étape de rupture est réalisée entre 3 et 12 jours, 4 et 12 jours, 5 et 12 jours, 4 et 11 jours, 6 et 11 jours, 6 et 10 jours, 6 et 9 jours, 6 et 8 jours, 7 et 8 jours, 5 et 11 jours, 5 et 10 jours, 7 et 10 jours, ou environ 1 semaine avant l'étape suivante de contact.

11. Utilisation selon les revendications 1 ou 3, utilisation de minoxidil selon les revendications 2 ou 3, ou méthode selon la revendication 4, dans lequel l'étape de rupture est réalisée entre 3 et 12 jours, 4 et 12 jours, 5 et 12 jours, 4 et 11 jours, 6 et 11 jours, 6 et 10 jours, 6 et 9 jours, 6 et 8 jours, 7 et 8 jours, 5 et 11 jours, 5 et 10 jours, 7 et 10 jours, ou environ 1 semaine avant l'étape de mise en contact.

12. Utilisation selon les revendications 1 ou 3, utilisation de minoxidil selon les revendications 2 ou 3, ou méthode selon la revendication 4, dans laquelle la zone du cuir chevelu dégarni est soumise à une épilation ou à l'administration d'un rétinoïde avant lésion.

13. Utilisation, utilisation du minoxidil, ou méthode selon la revendication 12, dans laquelle l'épilation est réalisée par arrachage, à la cire, par abrasion, au laser, par électrolyse, ou administration d'acide thioglycolique sur la zone du cuir chevelu dégarni.

14. Utilisation selon les revendications 1 ou 3, utilisation de minoxidil selon les revendications 2 ou 3, ou méthode selon la revendication 4, dans lequel la zone lésée du cuir chevelu dégarni n'est pas fermée chirurgicalement, ou n'est pas mise en contact avec un bandage, pansement, ou une pommade qui facilite la cicatrisation des plaies pendant une période de temps suivant l'étape de lésion.

15. Utilisation, utilisation de minoxidil ou méthode selon la revendication 14, dans laquelle la période de temps est d'environ 2 jours, d'environ 3 jours, d'environ 4 jours, d'environ 5 jours, d'environ 7 jours, d'environ 10 jours, d'environ 11 jours, d'environ 14 jours, d'environ 17 jours ou d'environ 3 semaines.

16. Utilisation selon les revendications 1 ou 3, utilisation de minoxidil selon les revendications 2 ou 3, ou méthode selon la revendication 4, dans laquelle le traitement ou la méthode entraîne la différenciation d'une cellule épidermique non différenciée en une cellule folliculaire pileuse.
